(19) **Europäisches Patentamt / European Patent Office / Office européen des brevets**

(11) **EP 1 565 574 B1**

(12) # EUROPEAN PATENT SPECIFICATION

(45) Date of publication and mention
of the grant of the patent:
**03.03.2010 Bulletin 2010/09**

(21) Application number: **03777005.4**

(22) Date of filing: **21.11.2003**

(51) Int Cl.:
*C12Q 1/68* (2006.01)

(86) International application number:
**PCT/GB2003/005102**

(87) International publication number:
**WO 2004/046382 (03.06.2004 Gazette 2004/23)**

(54) **PRODUCT AND METHOD**

VERFAHREN UND PRODUKTE

PRODUIT ET PROCEDE

(84) Designated Contracting States:
**AT BE BG CH CY CZ DE DK EE ES FI FR GB GR
HU IE IT LI LU MC NL PT RO SE SI SK TR**

(30) Priority: **21.11.2002 GB 0227238**

(43) Date of publication of application:
**24.08.2005 Bulletin 2005/34**

(73) Proprietor: **DiaGenic AS
0663 Oslo (NO)**

(72) Inventors:
• **SHARMA, Praveen
  0370 Oslo (NO)**
• **SAHNI, Narinder, Singh
  N-0196 Oslo (NO)**
• **LÖNNEBORG, Anders
  N-1430 Aas (NO)**

(74) Representative: **Jones, Elizabeth Louise
Dehns
St Bride's House
10 Salisbury Square
London
EC4Y 8JD (GB)**

(56) References cited:
WO-A-02/59271          US-A1- 2002 022 222
US-A1- 2002 169 560

• SHERLOCK G: "Analysis of large-scale gene expression data" CURRENT OPINION IN IMMUNOLOGY, CURRENT BIOLOGY LTD, XX, vol. 12, no. 2, 1 April 2000 (2000-04-01), pages 201-205, XP004257642 ISSN: 0952-7915

• HEYER LAURIE J ET AL: "Exploring expression data: Identification and analysis of coexpressed genes" GENOME RESEARCH, COLD SPRING HARBOR LABORATORY PRESS, US, vol. 9, no. 11, November 1999 (1999-11), pages 1106-1115, XP002149915 ISSN: 1088-9051

• HARDY A J ET AL: "Double-case diagnositc for outliers identification" CHEMOMETRICS AND INTELLIGENT LABORATORY SYSTEMS, ELSEVIER SCIENCE PUBLISHERS B.V. AMSTERDAM, NL, vol. 34, no. 1, 1 August 1996 (1996-08-01), pages 117-129, XP004037935 ISSN: 0169-7439

• ALIZADEH A A ET AL: "DISTINCT TYPES OF DIFFUSE LARGE B-CELL LYMPHOMA IDENTIFIED BY GENE EXPRESSION PROFILING" NATURE, MACMILLAN JOURNALS LTD. LONDON, GB, vol. 403, 3 February 2000 (2000-02-03), pages 503-512, XP002943414 ISSN: 0028-0836 cited in the application

• GOLUB T R ET AL: "Molecular classification of cancer: Class discovery and class prediction by gene expression monitoring" SCIENCE, AMERICAN ASSOCIATION FOR THE ADVANCEMENT OF SCIENCE,, US, vol. 286, no. 5439, 15 October 1999 (1999-10-15), pages 531-537, XP002207658 ISSN: 0036-8075 cited in the application

• WASSERMAN W W ET AL: "Identification of regulatory regions which confer muscle-specific gene expression" JOURNAL OF MOLECULAR BIOLOGY, LONDON, GB, vol. 278, no. 1, 24 April 1998 (1998-04-24), pages 167-181, XP004453985 ISSN: 0022-2836

- **BRO R ET AL: "On the difference between low-rank and subspace approximation: improved model for multi-linear PLS regression" CHEMOMETRICS AND INTELLIGENT LABORATORY SYSTEMS, ELSEVIER SCIENCE PUBLISHERS B.V. AMSTERDAM, NL, vol. 58, no. 1, 28 September 2001 (2001-09-28), pages 3-13, XP004318243 ISSN: 0169-7439**
- **DATABASE GEO [Online] NCBI 11 March 2002 'Affymetrix GeneChip Human Genome U133 Array Set HG-U133A', XP002254749**

- **WHITNEY ET AL: "Individuality and variation in gene expression patterns in human blood" PROC. NATL. ACAD.SCI, USA, vol. 100, no. 4, pages 1896-1901, XP002371125**

Remarks:

The file contains technical information submitted after the application was filed and not included in this specification

**Description**

The present invention relates to oligonucleotide probes, for use in assessing gene transcript levels in a cell, which may be used in analytical techniques, particularly diagnostic techniques. Conveniently the probes are provided in kit form. Different sets of probes may be used in techniques to prepare gene expression patterns and identify, diagnose or monitor different states, such as diseases, conditions or stages thereof. Also provided are methods of identifying suitable probes and their use in methods of the invention.

The identification of quick and easy methods of sample analysis for, for example, diagnostic applications, remains the goal of many researchers. End users seek methods which are cost effective, produce statistically significant results and which may be implemented routinely without the need for highly skilled individuals.

The analysis of gene expression within cells has been used to provide information on the state of those cells and importantly the state of the individual from which the cells are derived. The relative expression of various genes in a cell has been identified as reflecting a particular state within a body. For example, cancer cells are known to exhibit altered expression of various proteins and the transcripts or the expressed proteins may therefore be used as markers of that disease state.

Thus biopsy tissue may be analysed for the presence of these markers and cells originating from the site of the disease may be identified in other tissues or fluids of the body by the presence of the markers. Furthermore, products of the altered expression may be released into the blood stream and these products may be analysed. In addition cells which have contacted disease cells may be affected by their direct contact with those cells resulting in altered gene expression and their expression or products of expression may be similarly analysed.

However, there are some limitations with these methods. For example, the use of specific tumour markers for identifying cancer suffers from a variety of defects, such as lack of specificity or sensitivity, association of the marker with disease states besides the specific type of cancer, and difficulty of detection in asymptomatic individuals.

In addition to the analysis of one or two marker transcripts or proteins, more recently, gene expression patterns have been analysed. Affymetrix have described an array set of oligonucleotides covering more than 39000 transcripts which would allow such an analysis (Affymetrix GeneChip Human Genome U133 Assay Set HG-U133A, Database GEO NCBI, Accession No. GPL96). Most of the work involving large-scale gene expression analysis with implications in disease diagnosis has involved clinical samples originating from diseased tissues or cells. For example, several recent publications, which demonstrate that gene expression data can be used to distinguish between similar cancer types, have used clinical samples from diseased tissues or cells (Alon et al. 1999, PNAS, 96, p6745-6750; Golub et al. 1999, Science, 286, p531-537; Alizadeh et al, 2000, Nature, 403, p503-511; Bittner et al., 2000, Nature, 406, p536-540). WO 02/059271 discloses genes which are differentially expressed in tumour biopsies compared to normal tissues and their use in diagnostic methods.

However, these methods have relied on analysis of a sample containing diseased cells or products of those cells or cells which have been contacted by disease cells. Analysis of such samples relies on knowledge of the presence of a disease and its location, which may be difficult in asymptomatic patients. Furthermore, samples can not always be taken from the disease site, e.g. in diseases of the brain.

In a finding of great significance, the present inventors identified the previously untapped potential of all cells within a body to provide information relating to the state of the organism from which the cells were derived. WO98/49342 describes the analysis of the gene expression of cells distant from the site of disease, e.g. peripheral blood collected distant from a cancer site.

This finding is based on the premise that the different parts of an organism's body exist in dynamic interaction with each other. When a disease affects one part of the body, other parts of the body are also affected. The interaction results from a wide spectrum of biochemical signals that are released from the diseased area, affecting other areas in the body. Although, the nature of the biochemical and physiological changes induced by the released signals can vary in the different body parts, the changes can be measured at the level of gene expression and used for diagnostic purposes.

The physiological state of a cell in an organism is determined by the pattern with which genes are expressed in it. The pattern depends upon the internal and external biological stimuli to which said cell is exposed, and any change either in the extent or in the nature of these stimuli can lead to a change in the pattern with which the different genes are expressed in the cell. There is a growing understanding that by analysing the systemic changes in gene expression patterns in cells in biological samples, it is possible to provide information on the type and nature of the biological stimuli that are acting on them. Thus, for example, by monitoring the expression of a large number of genes in cells in a test sample, it is possible to determine whether their genes are expressed with a pattern characteristic for a particular disease, condition or stage thereof. Measuring changes in gene activities in cells, e.g. from tissue or body fluids is therefore emerging as a powerful tool for disease diagnosis.

Such methods have various advantages. Often, obtaining clinical samples from certain areas in the body that is diseased can be difficult and may involve undesirable invasions in the body, for example biopsy is often used to obtain samples for cancer. In some cases, such as in Alzheimer's disease the diseased brain specimen can only be obtained post-mortem. Furthermore, the tissue specimens which are obtained are often heterogeneous and may contain a mixture of both diseased and non-diseased cells, making the analysis of generated gene expression data both complex and difficult.

It has been suggested that a pool of tumour tissues that appear to be pathogenetically homogeneous with respect to morphological appearances of the tumour may well be highly heterogeneous at the molecular level (Alizadeh, 2000, supra), and in fact might contain tumours representing essentially different diseases (Alizadeh, 2000, supra; Golub, 1999, supra). For the purpose of identifying a disease, condition, or a stage thereof, any method that does not require clinical samples to originate directly from diseased tissues or cells is highly desirable since clinical samples representing a homogeneous mixture of cell types can be obtained from an easily accessible region in the body.

Whitney et al. (2003, PNAS, Vol. 100(4), p 1896-1901) have analysed the individuality and variation in gene expression patterns in normal blood to provide a database against which disease-associated gene expression patterns can be compared. Methods of analysing complex data sets are reviewed by Sherlock et al. (2000, Current Opinion in Immunology, Vol. 12, p 201-205).

We have now identified a set of probes of surprising utility for identifying one or more diseases. Thus, we now describe probes and sets of probes derived from cells which are not disease cells and which have not contacted disease cells, which correspond to genes which exhibit altered expression in normal versus disease individuals, for use in methods of identifying, diagnosing or monitoring certain conditions, particularly diseases or stages thereof.

A set of oligonucleotide probes which correspond to genes in a cell whose expression is affected in a pattern characteristic of a particular disease, condition or stage thereof, wherein said genes are systemically affected by said disease, condition or stage thereof, is disclosed. Preferably said genes are metabolic or house-keeping genes and preferably are constitutively moderately or highly expressed. Preferably the genes are moderately or highly expressed in the cells of the sample but not in cells from disease cells or in cells having contacted such disease cells.

Such probes, particularly when isolated from cells distant to the site of disease, do not rely on the development of disease to clinically recognizable levels and allow detection of a disease or condition or stage thereof very early after the onset of said disease or condition, even years before other subjective or objective symptoms appear.

As used herein "systemically" affected genes refers to genes whose expression is affected in the body without direct contact with a disease cell or disease site and the cells under investigation are not disease cells.

"Contact" as referred to herein refers to cells coming into close proximity with one another such that the direct effect of one cell on the other may be observed, e.g. an immune response, wherein these responses are not mediated by secondary molecules released from the first cell over a large distance to affect the second cell. Preferably contact refers to physical contact, or contact that is as close as is sterically possible, conveniently, cells which contact one another are found in the same unit volume, for example within 1cm$^3$.

A "disease cell" is a cell manifesting phenotypic changes and is present at the disease site at some time during its life-span, e.g. a tumour cell at the tumour site or which has disseminated from the tumour, or a brain cell in the case of brain disorders such as Alzheimer's disease.

"Metabolic" or "house-keeping" genes refer to those genes responsible for expressing products involved in cell division and maintenance, e.g. non-immune function related genes.

"Moderately or highly" expressed genes refers to those present in resting cells in a copy number of more than 30-100 copies/cell (assuming an average $3 \times 10^5$ mRNA molecules in a cell).

Specific probes having the above described properties are provided herein.

The present description discloses a set of oligonucleotide probes, wherein said set comprises at least 10 oligonucleotides selected from:

> an oligonucleotide as described in Table 1 or derived from a sequence described in Table 1, or an oligonucleotide with a complementary sequence,
> or a functionally equivalent oligonucleotide.

"Table 1" as referred to herein refers to Table 1a and/or Table 1b. Table 1b contains reference to additional clones and sequences as disclosed herein. Similarly Tables 2 and 4 comprise 2 parts, a and b.

Also disclosed are one or more oligonucleotide probes, wherein each oligonucleotide probe is selected from the oligonucleotides listed in Table 1, or derived from a sequence described in Table 1, or a complementary sequence thereof. The use of such probes in products and methods as described hereinafter are also disclosed.

As referred to herein an "oligonucleotide" is a nucleic acid molecule having at least 6 monomers in the polymeric structure, ie. nucleotides or modified forms thereof. The nucleic acid molecule may be DNA, RNA or PNA (peptide nucleic acid) or hybrids thereof or modified versions thereof, e.g. chemically modified forms, e.g. LNA (Locked Nucleic acid), by methylation or made up of modified or non-natural bases during synthesis, providing they retain their ability to bind to complementary sequences. Such oligonucleotides are used to probe target sequences and are thus referred to herein also as oligonucleotide probes or simply as probes.

An "oligonucleotide derived from a sequence described in Table 1" (or any other table) refers to a part of a sequence disclosed in that Table (e.g. Table 1-4), which satisfies the requirements of the oligonucleotide probes as described herein, e.g. in length and function. Preferably said parts have the size described hereinafter.

Preferably the oligonucleotide probes forming said set are at least 15 bases in length to allow binding of target molecules. Especially preferably said oligonucleotide probes are from 20 to 200 bases in length, e.g. from 30 to 150 bases, preferably 50-100 bases in length.

As referred to herein the term "complementary sequences" refers to sequences with consecutive complementary bases (ie. T:A, G:C) and which complementary sequences are therefore able to bind to one another through their complementarity.

Reference to "10 oligonucleotides" refers to 10 different oligonucleotides. Whilst a Table 1 oligonucleotide, a Table 1 derived oligonucleotide and their functional equivalent are considered different oligonucleotides, complementary oligonucleotides are not considered different. Preferably however, the at least 10 oligonucleotides are 10 different Table 1 oligonucleotides (or Table 1 derived oligonucleotides or their functional equivalents). Thus said 10 different oligonucleotides are preferably able to bind to 10 different transcripts.

Preferably said oligonucleotides are as described in Table 1 or are derived from a sequence described in Table 1. Especially preferably said oligonucleotides are as described in Table 2 or Table 4 or are derived from a sequence described in either of those tables. Especially preferably the oligonucleotide (or the oligonucleotide derived therefrom) has a high occurrence as defined in Table 3, especially preferably >40%, e.g. >80 or >90, e.g. 100%.

A "set" as described refers to a collection of unique oligonucleotide probes (ie. having a distinct sequence) and preferably consists of less than 1000 oligonucleotide probes, especially less than 500 probes, e.g. preferably from 10 to 500, e.g. 10 to 100, 200 or 300, especially preferably 20 to 100, e.g. 30 to 100 probes. In some cases less than 10 probes may be used, e.g. from 2 to 9 probes, e.g. 5 to 9 probes.

It will be appreciated that increasing the number of probes will prevent the possibility of poor analysis, e.g. misdiagnosis by comparison to other diseases which could similarly alter the expression of the particular genes in question. Other oligonucleotide probes not described herein may also be present, particularly if they aid the ultimate use of the set of oligonucleotide probes. However, preferably said set consists only of said Table 1 oligonucleotides, Table 1 derived oligonucleotides, complementary sequences or functionally equivalent oligonucleotides, or a sub-set thereof (e.g. of the size as described above), preferably a sub-set for which sequences are provided herein (see Table 1 and its footnote). Especially preferably said set consists only of said Table 1 oligonucleotides, Table 1 derived oligonucleotides, or complementary sequences thereof, or a sub-set thereof.

Thus in a first aspect the present invention provides a set of less than 1000 oligonucleotide probes, wherein said set comprises the oligonucleotides as described in Table 2b for which sequences are provided having the sequences as set forth in sequence No. 61, 77, 93, 108, 110, 192, 250, 308, 309, 310, 321, 327, 338, 339, 360, 361, 364, 365, 368, 378, 380, 381, 382, 384, 390, 391, 397, 398, 401, 403, 406, 411, 412, 413, 414, 415, 416, 418, 421, 423, 424, 428, 434, 436, 438, 441, 442, 450, 452, 453, 458, 460, 463, 464, 469, 471, 473, 474, 475, 476, 477, 478, 479, 482, 483, 485, 487, 488, 489, 492, 493, 494, 495, 503, 504, 505, 506, 507, 508, 509, 510, 512, 513, 515, 518, 519, 521, 523, 524, 526, 527, 529, 530, 532, 534, 560, 562, 564, 565, 566, 567, 568, 570, 571, 572, 575, 576, 578, 579, 580, 583, 585, 589, 591, 592, 593, 594, 596, 598, 600, 601, 605, 607, 610, 612, 613, 614, 615, 617, 618, 619, 622, 624, 628, 629, 630, 631, 632, 633, 634, 635, 636, 637, 638, 639, 643, 644, 645, 649, 651, 656, 658, 660, 661, 663, 665, 672, 673, 675, 679, 682, 683, 684, 685, 687, 688, 689, 691, 693, 696, 697, 699, 701, 702, 705, 706, 707, 708, 709, 711, 714, 718, 720, 721, 722, 724, 726, 736, 739, 747, 757, 758, 764, 766, 768, 773, 776, 782, 785, 796, 801, 808, 814, 817, 821, 825, 833, 837, 839, 849, 860, 864, 865, 867, 869, 870, 871, 873, 875, 876, 878, 879, 881, 885, 887, 889, 891, 892, 893, 895, 897, 899, 903, 904, 905, 906, 907, 908, 910, 911, 912, 915, 917, 926, 938, 939, 947, 949, 1028, 1056, 1071, 1074, 1081, 1083, 1084, 1099, 1109, 1118, 1125, 1139, 1148, 1160, 1165, 1172, 1178, 1180, 1181, 1182, 1183, 1185, 1186, 1188, 1189, 1190, 1192, 1193, 1195, 1196, 1197, 1198, 1199, 1200, 1201, 1202, 1203, 1204, 1205, 1207, 1208, 1209, 1210, 1211, 1212, 1213, 1214, 1215, 1216, 1217, 1218, 1219, 1220, 1221, 1224, 1226, 1228, 1230, 1231, 1239, 1331, 1332, 1335, 1336, 1337, 1338, 1344, 1348, 1351, 1352, 1353, 1355, 1360, 1361, 1364, 1365, 1366, 1368, 1369, 1370, 1371, 1372, 1374, 1378, 1380, 1382, 1387, 1389, 1390, 1391, 1392, 1394, 1395, 1396, 1397, 1399, 1440, 1448, 1453, 1456, 1460, 1495 and g6 or a set in which one or more of said oligonucleotides is substituted, wherein each oligonucleotide which is substituted is substituted by a part of said oligonucleotide which part is 15-200 bases in length, or by an oligonucleotide with a complementary sequence to said oligonucleotide.

In a further aspect the invention provides a set of less than 1000 oligonucleotide probes, wherein said set comprises the oligonucleotides as described in Table 4b for which sequences are provided having the sequences as set forth in sequence No. 299, 300, 302, 304, 306, 308, 309, 310, 311, 313, 314, 315, 316, 321, 322, 323, 324, 325, 326, 327, 328, 330, 331, 335, 337, 338, 339, 360, 361, 363, 364, 365, 366, 368, 369, 370, 371, 373, 374, 378, 380, 381, 382, 383, 384, 386, 387, 388, 389, 390, 391, 394, 395, 396, 397, 398, 399, 400, 401, 402, 403, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 438, 441, 442, 446, 447, 448, 450, 452, 453, 454, 458, 459, 460, 461, 462, 463, 464, 469, 471, 472, 473, 474, 475, 476, 477, 478, 479, 481, 482, 483, 484, 485, 487, 488, 489, 490, 491, 492, 493, 494, 495, 496, 497, 499, 500, 502, 503, 504, 505, 506, 507, 508, 509, 510, 512, 513, 515, 518, 519, 521, 523, 524, 525, 526, 527, 529, 530, 532, 533, 534, 560, 561, 562, 563, 564, 565, 566, 567, 568, 570, 571, 572, 574, 575, 576, 577, 578, 579, 580, 581, 582, 583, 585, 586, 587, 588, 589, 590, 591, 592, 593, 594, 595, 596, 597, 598, 599, 600, 601, 602, 603, 605, 606, 607, 609, 610, 611, 612, 613, 614, 615, 617, 618, 619, 621, 622, 624, 625, 626, 627, 628, 629, 630, 631, 632, 634, 635, 636, 637, 638, 639, 641, 642, 643, 644, 645, 646, 647, 648, 649, 650, 651, 652, 653, 654, 655, 656, 657, 658, 660, 661, 663, 665, 666, 669, 670, 671, 672, 673, 674, 675, 676, 679, 682, 683, 684, 685, 686, 687, 688, 689, 690, 691, 692, 693, 694, 696, 697, 698, 699, 700, 701, 702, 703, 704, 705, 706, 707, 708, 709, 710, 711, 713, 714, 717, 718, 719, 720, 721, 722, 724, 726, 727, 728, 870, 871, 873, 878, 879, 883, 885, 887, 889, 890, 892, 893, 895, 896, 897, 898, 899, 900, 903, 904, 905, 906, 907, 908, 910, 911, 912, 913, 914, 915, 1178, 1180, 1181, 1182, 1183, 1185, 1186, 1188, 1189, 1190, 1191, 1193, 1200, 1332, 1336, 1337, 1348, 1351, 1353, 1355, 1359, 1361, 1364, 1365, 1366, 1367, 1368, 1369, 1370, 1372, 1374, 1382, 1387, 1389, 1390, 1391, 1397, 1399, 1440, 1447, 1448, 1449, 1450, 1453, 1454, 1490, 1491, 1492, 1493, 1494 and 1495

or a set in which one or more of said oligonucleotides is substituted, wherein each oligonucleotide which is substituted is substituted by a part of said oligonucleotide which part is 15-200 bases in length, or by an oligonucleotide with a complementary sequence to said oligonucleotide.

Multiple copies of each unique oligonucleotide probe, e.g. 10 or more copies, may be present in each set, but constitute only a single probe.

A set of oligonucleotide probes, which may preferably be immobilized on a solid support or have means for such immobilization, comprises the at least 10 oligonucleotide probes selected from those described hereinbefore. Especially preferably said probes are selected from those having high occurrence as described in Table 3 and as mentioned above. As mentioned above, these 10 probes must be unique and have different sequences. Having said this however, two separate probes may be used which recognize the same gene but reflect different splicing events. However oligonucleotide probes which are complementary to, and bind to distinct genes are preferred.

As described herein a "functionally equivalent" oligonucleotide to those described in Table 1 or derived therefrom refers to an oligonucleotide which is capable of identifying the same gene as an oligonucleotide of Table 1 or derived therefrom, ie. it can bind to the same mRNA molecule (or DNA) transcribed from a gene (target nucleic acid molecule) as the Table 1 oligonucleotide or the Table 1 derived oligonucleotide (or its complementary sequence). Preferably said functionally equivalent oligonucleotide is capable of recognizing, ie. binding to the same splicing product as a Table 1 oligonucleotide or a Table 1 derived oligonucleotide. Preferably said mRNA molecule is the full length mRNA molecule which corresponds to the Table 1 oligonucleotide or the Table 1 derived oligonucleotide.

As referred to herein "capable of binding" or "binding" refers to the ability to hybridize under conditions described hereinafter.

Alternatively expressed, functionally equivalent oligonucleotides (or complementary sequences) have sequence identity or will hybridize, as described hereinafter, to a region of the target molecule to which molecule a Table 1 oligonucleotide or a Table 1 derived oligonucleotide or a complementary oligonucleotide binds. Preferably, functionally equivalent oligonucleotides (or their complementary sequences) hybridize to one of the mRNA sequences which corresponds to a Table 1 oligonucleotide or a Table 1 derived oligonucleotide under the conditions described hereinafter or has sequence identity to a part of one of the mRNA sequences which corresponds to a Table 1 oligonucleotide or a Table 1 derived oligonucleotide. A "part" in this context refers to a stretch of at least 5, e.g. at least 10 or 20 bases, such as from 5 to 100, e.g. 10 to 50 or 15 to 30 bases.

Particularly preferably, the functionally equivalent oligonucleotide binds to all or a part of the region of a target nucleic acid molecule (mRNA or cDNA) to which the Table 1 oligonucleotide or Table 1 derived oligonucleotide binds. A "target" nucleic acid molecule is the gene transcript or related product e.g. mRNA, or cDNA, or amplified product thereof. Said "region" of said target molecule to which said Table 1 oligonucleotide or Table 1 derived oligonucleotide binds is the stretch over which complementarity exists. At its largest this region is the whole length of the Table 1 oligonucleotide or Table 1 derived oligonucleotide, but may be shorter if the entire Table 1 sequence or Table 1 derived oligonucleotide is not complementary to a region of the target sequence.

Preferably said part of said region of said target molecule is a stretch of at least 5, e.g. at least 10 or 20 bases, such as from 5 to 100, e.g. 10 to 50 or 15 to 30 bases. This may for example be achieved by said functionally equivalent oligonucleotide having several identical bases to the bases of the Table 1 oligonucleotide or the Table 1 derived oligonucleotide. These bases may be identical over consecutive stretches, e.g. in a part of the functionally equivalent oligonucleotide, or may be present non-consecutively, but provide sufficient complementarity to allow binding to the target sequence.

Thus, preferably, said functionally equivalent oligonucleotide hybridizes under conditions of high stringency to a Table 1 oligonucleotide or a Table 1 derived oligonucleotide or the complementary sequence thereof. Alternatively expressed, said functionally equivalent oligonucleotide exhibits high sequence identity to all or part of a Table 1 oligonucleotide. Preferably said functionally equivalent oligonucleotide has at least 70% sequence identity, preferably at least 80%, e.g. at least 90, 95, 98 or 99%, to all of a Table 1 oligonucleotide or a part thereof. As used in this context, a "part" refers to a stretch of at least 5, e.g. at least 10 or 20 bases, such as from 5 to 100, e.g. 10 to 50 or 15 to 30 bases, in said Table 1 oligonucleotide. Especially preferably when sequence identity to only a part of said Table 1 oligonucleotide is present, the sequence identity is high, e.g. at least 80% as described above.

Functionally equivalent oligonucleotides which satisfy the above stated functional requirements include those which are derived from the Table 1 oligonucleotides and also those which have been modified by single or multiple nucleotide base (or equivalent) substitution, addition and/or deletion, but which nonetheless retain functional activity, e.g. bind to the same target molecule as the Table 1 oligonucleotide or the Table 1 derived oligonucleotide from which they are further derived or modified. Preferably said modification is of from 1 to 50, e.g. from 10 to 30, preferably from 1 to 5 bases. Especially preferably only minor modifications are present, e.g. variations in less than 10 bases, e.g. less than 5 base changes.

Within the meaning of "addition" equivalents are included oligonucleotides containing additional sequences which are complementary to the consecutive stretch of bases on the target molecule to which the Table 1 oligonucleotide or the Table 1 derived oligonucleotide binds. Alternatively the addition may comprise a different, unrelated sequence, which may for example confer a further property, e.g. to provide a means for immobilization such as a linker to bind the oligonucleotide probe to a solid support.

Particularly preferred are naturally occurring equivalents such as biological variants, e.g. allelic, geographical or allotypic variants, e.g. oligonucleotides which correspond to a genetic variant, for example as present in a different species.

Functional equivalents include oligonucleotides with modified bases, e.g. using non-naturally occurring bases. Such derivatives may be prepared during synthesis or by post production modification.

"Hybridizing" sequences which bind under conditions of low stringency are those which bind under non-stringent conditions (for example, 6x SSC/50% formamide at room temperature) and remain bound when washed under conditions of low stringency (2 X SSC, room temperature, more preferably 2 X SSC, 42˚C). Hybridizing under high stringency refers to the above conditions in which washing is performed at 2 X SSC, 65˚C (where SSC = 0.15M NaCl, 0.015M sodium citrate, pH 7.2).

"Sequence identity" as referred to herein refers to the value obtained when assessed using ClustalW (Thompson et al., 1994, Nucl. Acids Res., 22, p4673-4680) with the following parameters:

Pairwise alignment parameters - Method: accurate, Matrix: IUB, Gap open penalty: 15.00, Gap extension penalty: 6.66;

Multiple alignment parameters - Matrix: IUB, Gap open penalty: 15.00, % identity for delay: 30, Negative matrix: no, Gap extension penalty: 6.66, DNA transitions weighting: 0.5.

Sequence identity at a particular base is intended to include identical bases which have simply been derivatized.

Also disclosed are polypeptides encoded by the mRNA sequence to which a Table 1 oligonucleotide or a Table 1 derived oligonucleotide binds. Additionally disclosed are antibodies which bind to any of said polypeptides.

As described above, conveniently said set of oligonucleotide probes may be immobilized on one or more solid supports. Single or preferably multiple copies of each unique probe are attached to said solid supports, e.g. 10 or more, e.g. at least 100 copies of each unique probe are present. In a further aspect of the invention the set of probes comprising the Table 2b or 4b probes are immobilized on one or more solid supports.

One or more unique oligonucleotide probes may be associated with separate solid supports which together form a set of probes immobilized on multiple solid support, e.g. one or more unique probes may be immobilized on multiple beads, membranes, filters, biochips etc. which together form a set of probes, which together form modules of the kit described hereinafter. The solid support of the different modules are conveniently physically associated although the signals associated with each probe (generated as described hereinafter) must be separately determinable.

Alternatively, the probes may be immobilized on discrete portions of the same solid support, e.g. each unique oligonucleotide probe, e.g. in multiple copies, may be immobilized to a distinct and discrete portion or region of a single filter or membrane, e.g. to generate an array.

A combination of such techniques may also be used, e.g. several solid supports may be used which each immobilize several unique probes.

The expression "solid support" shall mean any solid material able to bind oligonucleotides by hydrophobic, ionic or covalent bridges.

"Immobilization" as used herein refers to reversible or irreversible association of the probes to said solid support by virtue of such binding. If reversible, the probes remain associated with the solid support for a time sufficient for methods as disclosed herein to be carried out.

Numerous solid supports suitable as immobilizing moieties, are well known in the art and widely described in the literature and generally speaking, the solid support may be any of the well-known supports or matrices which are currently widely used or proposed for immobilization, separation etc. in chemical or biochemical procedures. Such materials include, but are not limited to, any synthetic organic polymer such as polystyrene, polyvinylchloride, polyethylene; or nitrocellulose and cellulose acetate; or tosyl activated surfaces; or glass or nylon or any surface carrying a group suited for covalent coupling of nucleic acids. The immobilizing moieties may take the form of particles, sheets, gels, filters, membranes, microfibre strips, tubes or plates, fibres or capillaries, made for example of a polymeric material e.g. agarose, cellulose, alginate, teflon, latex or polystyrene or magnetic beads. Solid supports allowing the presentation of an array, preferably in a single dimension are preferred, e.g. sheets, filters, membranes, plates or biochips.

Attachment of the nucleic acid molecules to the solid support may be performed directly or indirectly. For example if a filter is used, attachment may be performed by UV-induced crosslinking. Alternatively, attachment may be performed indirectly by the use of an attachment moiety carried on the oligonucleotide probes and/or solid support. Thus for example, a pair of affinity binding partners may be used, such as avidin, streptavidin or biotin, DNA or DNA binding protein (e.g. either the lac I repressor protein or the lac operator sequence to which it binds), antibodies (which may be mono- or polyclonal), antibody fragments or the epitopes or haptens of antibodies. In these cases, one partner of the binding pair is attached to (or is inherently part of) the solid support and the other partner is attached to (or is inherently part of) the nucleic acid molecules.

As used herein an "affinity binding pair" refers to two components which recognize and bind to one another specifically (ie. in preference to binding to other molecules). Such binding pairs when bound together form a complex.

Attachment of appropriate functional groups to the solid support may be performed by methods well known in the art, which include for example, attachment through hydroxyl, carboxyl, aldehyde or amino groups which may be provided by treating the solid support to provide suitable surface coatings. Solid supports presenting appropriate moieties for attachment of the binding partner may be produced by routine methods known in the art.

Attachment of appropriate functional groups to the oligonucleotide probes disclosed herein may be performed by ligation or introduced during synthesis or amplification, for example using primers carrying an appropriate moiety, such as biotin or a particular sequence for capture.

Conveniently, the set of probes described hereinbefore is provided in kit form.

Thus viewed from a further aspect the present invention provides a kit comprising a set of oligonucleotide probes of the invention as described hereinbefore immobilized on one or more solid supports.

Preferably, said probes are immobilized on a single solid support and each unique probe is attached to a different region of said solid support. However, when attached to multiple solid supports, said multiple solid supports form the modules which make up the kit. Especially preferably said solid support is a sheet, filter, membrane, plate or biochip.

Optionally the kit may also contain information relating to the signals generated by normal or diseased samples (as discussed in more detail hereinafter in relation to the use of the kits), standardizing materials, e.g. mRNA or cDNA from normal and/or diseased samples for comparative purposes, labels for incorporation into cDNA, adapters for introducing nucleic acid sequences for amplification purposes, primers for amplification and/or appropriate enzymes, buffers and solutions. Optionally said kit may also contain a package insert describing how the method as described herein should be performed, optionally providing standard graphs, data or software for interpretation of results obtained when performing the methods described herein.

The use of kits of the invention to prepare a standard diagnostic gene transcript pattern as described hereinafter forms a further aspect of the invention.

The set of probes as described herein have various uses. Principally however they are used to assess the gene expression state of a test cell to provide information relating to the organism from which said cell is derived. Thus the probes are useful in diagnosing, identifying or monitoring a disease or condition or stage thereof in an organism.

Thus also disclosed herein is the use of a set of oligonucleotide probes or a kit as described hereinbefore to determine the gene expression pattern of a cell which pattern reflects the level of gene expression of genes to which said oligonucleotide probes bind, comprising at least the steps of:

   a) isolating mRNA from said cell, which may optionally be reverse transcribed to cDNA;
   b) hybridizing the mRNA or cDNA of step (a) to a set of oligonucleotide probes or a kit as defined herein; and
   c) assessing the amount of mRNA or cDNA hybridizing to each of said probes to produce said pattern. In a use according to the invention, the set or kit is a set or kit according to the invention.

The mRNA and cDNA as referred to in this method, and the methods hereinafter, encompass derivatives or copies of said molecules, e.g. copies of such molecules such as those produced by amplification or the preparation of complementary strands, but which retain the identity of the mRNA sequence, ie. would hybridize to the direct transcript (or its complementary sequence) by virtue of precise complementarity, or sequence identity, over at least a region of said molecule. It will be appreciated that complementarity will not exist over the entire region where techniques have been used which may truncate the transcript or introduce new sequences, e.g. by primer amplification. For convenience, said mRNA or cDNA is preferably amplified prior to step b). As with the oligonucleotides described herein said molecules may be modified, e.g. by using non-natural bases during synthesis providing complementarity remains. Such molecules may also carry additional moieties such as signalling or immobilizing means.

The various steps involved in the method of preparing such a pattern are described in more detail hereinafter.

As used herein "gene expression" refers to transcription of a particular gene to produce a specific mRNA product (ie. a particular splicing product). The level of gene expression may be determined by assessing the level of transcribed mRNA molecules or cDNA molecules reverse transcribed from the mRNA molecules or products derived from those molecules, e.g. by amplification.

The "pattern" created by this technique refers to information which, for example, may be represented in tabular or graphical form and conveys information about the signal associated with two or more oligonucleotides. Preferably said pattern is expressed as an array of numbers relating to the expression level associated with each probe.

Preferably, said pattern is established using the following linear model:

$$y = Xb + f \hspace{3cm} \text{Equation 1}$$

wherein, **X** is the matrix of gene expression data and **y** is the response variable, **b** is the regression coefficient vector and **f** the estimated residual vector. Although many different methods can be used to establish the relationship provided in equation 1, especially preferably the partial Least Squares Regression (PLSR) method is used for establishing the relationship in equation 1.

The probes are thus used to generate a pattern which reflects the gene expression of a cell at the time of its isolation. The pattern of expression is characteristic of the circumstances under which that cells finds itself and depends on the influences to which the cell has been exposed. Thus, a characteristic gene transcript pattern standard or fingerprint (standard probe pattern) for cells from an individual with a particular disease or condition may be prepared and used for comparison to transcript patterns of test cells. This has clear applications in diagnosing, monitoring or identifying whether an organism is suffering from a particular disease, condition or stage thereof.

The standard pattern is prepared by determining the extent of binding of total mRNA (or cDNA or related product), from cells from a sample of one or more organisms with the disease or condition or stage thereof, to the probes. This reflects the level of transcripts which are present which correspond to each unique probe. The amount of nucleic acid material which binds to the different probes is assessed and this information together forms the gene transcript pattern standard of that disease or condition or stage thereof. Each such standard pattern is characteristic of the disease, condition or stage thereof.

A method is therefore disclosed for preparing a standard gene transcript pattern characteristic of a disease or condition or stage thereof in an organism comprising at least the steps of:

a) isolating mRNA from the cells of a sample of one or more organisms having the disease or condition or stage thereof, which may optionally be reverse transcribed to cDNA;
b) hybridizing the mRNA or cDNA of step (a) to a set of oligonucleotides or a kit as described hereinbefore specific for said disease or condition or stage thereof in an organism and sample thereof corresponding to the organism and sample thereof under investigation; and
c) assessing the amount of mRNA or cDNA hybridizing to each of said probes to produce a characteristic pattern reflecting the level of gene expression of genes to which said oligonucleotides bind, in the sample with the disease, condition or stage thereof.

In relation to the invention, the present invention provides such a method using blood samples to prepare a standard transcript pattern characteristic of breast cancer or Alzheimer's disease or a stage thereof. Thus in a preferred aspect the invention provides a method of preparing a standard gene transcript pattern characteristic of breast cancer or Alzheimer's disease or a stage thereof in an organism comprising at least the steps of:

a) isolating mRNA from the cells of a blood sample of one or more organisms having breast cancer or Alzheimer's disease or a stage thereof, which may optionally be reverse transcribed to cDNA;
b) hybridizing the mRNA or cDNA of step (a) from an organism with breast cancer or a stage thereof to a set of oligonucleotides or a kit of the invention as described hereinbefore specific for breast cancer or a stage thereof in an organism and sample thereof corresponding to the organism and sample thereof under investigation or hybridizing the mRNA or cDNA of step (a) from an organism with Alzheimer's disease or a stage thereof to a set of oligonucleotides or a kit of the invention as described hereinbefore specific for Alzheimer's disease or a stage thereof in an organism and sample thereof corresponding to the organism and sample thereof under investigation; and
c) assessing the amount of mRNA or cDNA hybridizing to each of said probes to produce a characteristic pattern reflecting the level of gene expression of genes to which said oligonucleotides bind, in the sample with breast cancer or Alzheimer's disease or a stage thereof, wherein oligonucleotides specific for breast cancer or a stage thereof are as set forth in Table 2b and oligonucleotides specific for Alzheimer's disease or a stage thereof are as set forth in Table 4b.

For convenience, said oligonucleotides are preferably immobilized on one or more solid supports.

The standard pattern for a great number of diseases or conditions and different stages thereof using particular probes may be accumulated in databases and be made available to laboratories on request.

"Disease" samples and organisms as referred to herein refer to organisms (or samples from the same) with an underlying pathological disturbance relative to a normal organism (or sample), in a symptomatic or asymptomatic organism, which may result, for example, from infection or an acquired or congenital genetic imperfection. Such organisms are known to have, or which exhibit, the disease or condition or stage thereof under study.

A "condition" refers to a state of the mind or body of an organism which has not occurred through disease, e.g. the presence of an agent in the body such as a toxin, drug or pollutant, or pregnancy.

"Stages" thereof refer to different stages of the disease or condition which may or may not exhibit particular physiological or metabolic changes, but do exhibit changes at the genetic level which may be detected as altered gene expression. It will be appreciated that during the course of a disease or condition the expression of different transcripts may vary. Thus at different stages, altered expression may not be exhibited for particular transcripts compared to "normal" samples. However, combining information from several transcripts which exhibit altered expression at one or more stages through the course of the disease or condition can be used to provide a characteristic pattern which is indicative of a particular stage of the disease or condition. Thus for example different stages in cancer, e.g. pre-stage I, stage I, stage II, II or IV can be identified.

"Normal" as used herein refers to organisms or samples which are used for comparative purposes. Preferably, these are "normal" in the sense that they do not exhibit any indication of, or are not believed to have, any disease or condition that would affect gene expression, particularly in respect of the disease for which they are to be used as the normal standard. However, it will be appreciated that different stages of a disease or condition may be compared and in such cases, the "normal" sample may correspond to the earlier stage of the disease or condition.

As used herein a "sample" refers to any material obtained from the organism, e.g. human or non-human animal under investigation which contains cells and includes, tissues, body fluid or body waste or in the case of prokaryotic organisms, the organism itself. "Body fluids" include blood, saliva, spinal fluid, semen, lymph. "Body waste" includes urine, expectorated matter (pulmonary patients), faeces etc. "Tissue samples" include tissue obtained by biopsy, by surgical interventions or by other means e.g. placenta. Preferably however, the samples which are examined are from areas of the body not apparently affected by the disease or condition. The cells in such samples are not disease cells, e.g. cancer cells, have not been in contact with such disease cells and do not originate from the site of the disease or condition. The "site of disease" is considered to be that area of the body which manifests the disease in a way which may be objectively determined, e.g. a tumour or area of inflammation. Thus for example peripheral blood may be used for the diagnosis of non-haematopoietic cancers, and the blood does not require the presence of malignant or disseminated cells from the cancer in the blood. Similarly in diseases of the brain, in which no diseased cells are found in the blood due to the blood:brain barrier, peripheral blood may still be used in the methods of the invention. For performance of methods of the invention, blood samples are used.

It will however be appreciated that the method of preparing the standard transcription pattern and other methods disclosed herein are also applicable for use on living parts of eukaryotic organisms such as cell lines and organ cultures and explants.

As used herein, reference to "corresponding" sample etc. refers to cells preferably from the same tissue, body fluid or body waste, but also includes cells from tissue, body fluid or body waste which are sufficiently similar for the purposes of preparing the standard or test pattern. When used in reference to genes "corresponding" to the probes, this refers to genes which are related by sequence (which may be complementary) to the probes although the probes may reflect different splicing products of expression.

"Assessing" as used herein refers to both quantitative and qualitative assessment which may be determined in absolute or relative terms.

The methods described herein and particularly methods of the invention may be put into practice as follows. To prepare a standard transcript pattern for a particular disease, condition or stage thereof, sample mRNA is extracted from the cells of tissues, body fluid or body waste according to known techniques (see for example Sambrook et. al. (1989), Molecular Cloning : A laboratory manual, 2nd Ed., Cold Spring Harbor Laboratory Press, Cold Spring Harbor, N.Y.) from a diseased individual or organism.

Owing to the difficulties in working with RNA, the RNA is preferably reverse transcribed at this stage to form first strand cDNA. Cloning of the cDNA or selection from, or using, a cDNA library is not however necessary in this or other methods disclosed herein. Preferably, the complementary strands of the first strand cDNAs are synthesized, ie.

second strand cDNAs, but this will depend on which relative strands are present in the oligonucleotide probes. The RNA may however alternatively be used directly without reverse transcription and may be labelled if so required.

Preferably the cDNA strands are amplified by known amplification techniques such as the polymerase chain reaction (PCR) by the use of appropriate primers. Alternatively, the cDNA strands may be cloned with a vector, used to transform a bacteria such as E. coli which may then be grown to multiply the nucleic acid molecules. When the sequence of the cDNAs are not known, primers may be directed to regions of the nucleic acid molecules which have been introduced. Thus for example, adapters may be ligated to the cDNA molecules and primers directed to these portions for amplification of the cDNA molecules. Alternatively, in the case of eukaryotic samples, advantage may be taken of the polyA tail and cap of the RNA to prepare appropriate primers.

To produce the standard diagnostic gene transcript pattern or fingerprint for a particular disease or condition or stage thereof, the above described oligonucleotide probes are used to probe mRNA or cDNA of the diseased sample to produce a signal for hybridization to each particular oligonucleotide probe species, ie. each unique probe. A standard control gene transcript pattern may also be prepared if desired using mRNA or cDNA from a normal sample. Thus, mRNA or cDNA is brought into contact with the oligonucleotide probe under appropriate conditions to allow hybridization.

When multiple samples are probed, this may be performed consecutively using the same probes, e.g. on one or more solid supports, ie. on probe kit modules, or by simultaneously hybridizing to corresponding probes, e.g. the modules of a corresponding probe kit.

To identify when hybridization occurs and obtain an indication of the number of transcripts/cDNA molecules which become bound to the oligonucleotide probes, it is necessary to identify a signal produced when the transcripts (or related molecules) hybridize (e.g. by detection of double stranded nucleic acid molecules or detection of the number of molecules which become bound, after removing unbound molecules, e.g. by washing).

In order to achieve a signal, either or both components which hybridize (ie. the probe and the transcript) carry or form a signalling means or a part thereof. This "signalling means" is any moiety capable of direct or indirect detection by the generation or presence of a signal. The signal may be any detectable physical characteristic such as conferred by radiation emission, scattering or absorption properties, magnetic properties, or other physical properties such as charge, size or binding properties of existing molecules (e.g. labels) or molecules which may be generated (e.g. gas emission etc.). Techniques are preferred which allow signal amplification, e.g. which produce multiple signal events from a single active binding site, e.g. by the catalytic action of enzymes to produce multiple detectable products.

Conveniently the signalling means may be a label which itself provides a detectable signal. Conveniently this may be achieved by the use of a radioactive or other label which may be incorporated during cDNA production, the preparation of complementary cDNA strands, during amplification of the target mRNA/cDNA or added directly to target nucleic acid molecules.

Appropriate labels are those which directly or indirectly allow detection or measurement of the presence of the transcripts/cDNA. Such labels include for example radiolabels, chemical labels, for example chromophores or fluorophores (e.g. dyes such as fluorescein and rhodamine), or reagents of high electron density such as ferritin, haemocyanin or colloidal gold. Alternatively, the label may be an enzyme, for example peroxidase or alkaline phosphatase, wherein the presence of the enzyme is visualized by its interaction with a suitable entity, for example a substrate. The label may also form part of a signalling pair wherein the other member of the pair is found on, or in close proximity to, the oligonucleotide probe to which the transcript/cDNA binds, for example, a fluorescent compound and a quench fluorescent substrate may be used. A label may also be provided on a different entity, such as an antibody, which recognizes a peptide moiety attached to the transcripts/cDNA, for example attached to a base used during synthesis or amplification.

A signal may be achieved by the introduction of a label before, during or after the hybridization step. Alternatively, the presence of hybridizing transcripts may be identified by other physical properties, such as their absorbance, and in which case the signalling means is the complex itself.

The amount of signal associated with each oligonucleotide probe is then assessed. The assessment may be quantitative or qualitative and may be based on binding of a single transcript species (or related cDNA or other products) to each probe, or binding of multiple transcript species to multiple copies of each unique probe. It will be appreciated that quantitative results will provide further information for the transcript fingerprint of the disease which is compiled. This data may be expressed as absolute values (in the case of macroarrays) or may be determined relative to a particular standard or reference e.g. a normal control sample.

Furthermore it will be appreciated that the standard diagnostic gene pattern transcript may be prepared using one or more disease samples (and normal samples if used) to perform the hybridization step to obtain patterns not biased towards a particular individual's variations in gene expression.

The use of the probes to prepare standard patterns and the standard diagnostic gene transcript patterns thus produced for the purpose of identification or diagnosis or monitoring of a particular disease or condition or stage thereof in a particular organism forms a further disclosure of the description.

Once a standard diagnostic fingerprint or pattern has been determined for a particular disease or condition using the selected oligonucleotide probes, this information can be used to identify the presence, absence or extent or stage of that disease or condition in a different test organism or individual.

To examine the gene expression pattern of a test sample, a test sample of tissue, body fluid or body waste containing cells, corresponding to the sample used for the preparation of the standard pattern, is obtained from a patient or the organism to be studied. A test gene transcript pattern is then prepared as described hereinbefore as for the standard pattern.

A method is therefore disclosed for preparing a test gene transcript pattern comprising at least the steps of:

a) isolating mRNA from the cells of a sample of said test organism, which may optionally be reverse transcribed to cDNA;

b) hybridizing the mRNA or cDNA of step (a) to a set of oligonucleotides or a kit as described hereinbefore specific for a disease or condition or stage thereof in an organism and sample thereof corresponding to the organism and sample thereof under investigation; and

c) assessing the amount of mRNA or cDNA hybridizing to each of said probes to produce said pattern reflecting the level of gene expression of genes to which said oligonucleotides bind, in said test sample.

In relation to the invention, the present invention provides such a method using blood samples to prepare a test transcript pattern by binding to oligonucleotides specific for breast cancer or Alzheimer's disease or a stage thereof. Thus in a further preferred aspect the invention provides a method of preparing a test gene transcript pattern comprising at least the steps of:

a) isolating mRNA from the cells of a blood sample of said test organism, which may optionally be reverse transcribed to cDNA;

b) hybridizing the mRNA or cDNA of step (a) to a set of oligonucleotides or a kit of the invention as described hereinbefore specific for breast cancer or a stage thereof in an organism and sample thereof corresponding to the organism and sample thereof under investigation or to a set of oligonucleotides or a kit or the invention as described hereinbefore specific for Alzheimer's disease or a stage thereof in an organism and sample thereof corresponding to the organism and sample thereof under investigation; and

c) assessing the amount of mRNA or cDNA hybridizing to each of said probes to produce said pattern reflecting the level of gene expression of genes to which said oligonucleotides bind, in said test sample, wherein oligonucleotides specific for breast cancer or a stage thereof are as set forth in Table 2b and oligonucleotides specific for Alzheimer's disease or a stage thereof are as set forth in Table 4b.

This test pattern may then be compared to one or more standard patterns to assess whether the sample contains cells having the disease, condition or stage thereof.

A further method is therefore disclosed for diagnosing or identifying or monitoring a disease or condition or stage thereof in an organism, comprising the steps of:

a) isolating mRNA from the cells of a sample of said organism, which may optionally be reverse transcribed to cDNA;

b) hybridizing the mRNA or cDNA of step (a) to a set of oligonucleotides or a kit as described hereinbefore specific for said disease or condition or stage thereof in an organism and sample thereof corresponding to the organism and sample thereof under investigation;

c) assessing the amount of mRNA or cDNA hybridizing to each of said probes to produce a characteristic pattern reflecting the level of gene expression of genes to which said oligonucleotides bind, in said sample; and

d) comparing said pattern to a standard diagnostic pattern prepared according to the method disclosed herein using a sample from an organism corresponding to the organism and sample under investigation to determine the presence of said disease or condition or a stage thereof in the organism under investigation.

The method up to and including step c) is the preparation of a test pattern as described above.

In relation to the invention, the present invention provides such a method using blood samples to prepare a test transcript pattern by binding to oligonucleotides specific for breast cancer or Alzheimer's disease or a stage thereof, for comparison to a standard diagnostic pattern prepared as described hereinbefore.

Thus in a yet further preferred aspect the invention provides a method of diagnosing or identifying or monitoring breast cancer or Alzheimer's disease or a stage thereof in an organism, comprising the steps of:

a) isolating mRNA from the cells of a blood sample of said organism, which may optionally be reverse transcribed to cDNA;

b) hybridizing the mRNA or cDNA of step (a) to a set of oligonucleotides of the invention as described hereinbefore specific for breast cancer or a stage thereof in an organism and sample thereof corresponding to the organism and sample thereof under investigation or to a set of oligonucleotides or a kit of the invention as described hereinbefore specific for Alzheimer's disease or a stage thereof in an organism and sample thereof corresponding to the organism and sample thereof under investigation;

c) assessing the amount of mRNA or cDNA hybridizing to each of said probes to produce a characteristic pattern reflecting the level of gene expression of genes to which said oligonucleotides bind in said sample; and

d) comparing said pattern to a standard diagnostic pattern prepared as described in accordance with the invention hereinbefore described using a sample from an organism corresponding to the organism and sample under investigation to determine the degree of correlation indicative of the presence of breast cancer or Alzheimer's disease or a stage thereof in the organism under investigation, wherein oligonucleotides specific for breast cancer or a stage thereof are as set forth in Table 2b and oligonucleotides specific for Alzheimer's disease or a stage thereof are as set forth in Table 4b.

As referred to herein, "diagnosis" refers to determination of the presence or existence of a disease or condition or stage thereof in an organism. "Monitoring" refers to establishing the extent of a disease or condition, particularly when an individual is known to be suffering from a disease or condition, for example to monitor the effects of treatment or the development of a disease or condition, e.g. to determine the suitability of a treatment or provide a prognosis.

The presence of the disease or condition or stage thereof may be determined by determining the degree of correlation between the standard and test samples' patterns. This necessarily takes into account the range of values which are obtained for normal and diseased samples. Although this can be established by obtaining standard deviations for several representative samples binding to the probes to develop the standard, it will be appreciated that single samples may be sufficient to generate the standard pattern to identify a disease if the test sample exhibits close enough correlation to that standard. Conveniently, the presence, absence, or extent of a disease or condition or stage thereof in a test sample can be predicted by inserting the data relating to the expression level of informative probes in test sample into the standard diagnostic probe pattern established according to equation 1.

Data generated using the above mentioned methods may be analysed using various techniques from the most basic visual representation (e.g. relating to intensity) to more complex data manipulation to identify underlying patterns which reflect the interrelationship of the level of expression of each gene to which the various probes bind, which may be quantified and expressed mathematically. Conveniently, the raw data thus generated may be manipulated by the data processing and statistical methods described hereinafter, particularly normalizing and standardizing the data and fitting the data to a classification model to determine whether said test data reflects the pattern of a particular disease, condition or stage thereof.

The methods described herein may be used to identify, monitor or diagnose a disease, condition or ailment or its stage or progression, for which the oligonucleotide probes are informative. "Informative" probes as described herein, are those which reflect genes which have altered expression in the diseases or conditions in question, or particular stages thereof. Probes as described herein may not be sufficiently informative for diagnostic purposes when used alone, but are informative when used as one of several probes to provide a characteristic pattern, e.g. in a set as described hereinbefore.

Preferably said probes correspond to genes which are systemically affected by said disease, condition or stage thereof. Especially preferably said genes, from which transcripts are derived which bind to probes, are metabolic or house-keeping genes and preferably are moderately or highly expressed. The advantage of using probes directed to moderately or highly expressed genes is that smaller clinical samples are required for generating the necessary gene expression data set, e.g. less than 1ml blood samples.

Furthermore, it has been found that such genes which are already being actively transcribed tend to be more prone to being influenced, in a positive or negative way, by new stimuli. In addition, since transcripts are already being produced at levels which are generally detectable, small changes in those levels are readily detectable as for example, a certain detectable threshold does not need to be reached.

Preferably, the set of probes disclosed herein are informative for a variety of different diseases, conditions or stages thereof. A sub-set of the probes disclosed herein may be used for diagnosis, identification or monitoring a particular disease, condition or stage thereof.

Thus the probes may be used to diagnose or identify or monitor any condition, ailment, disease or reaction that leads to the relative increase or decrease in the activity of informative genes of any or all eukaryotic or prokaryotic organisms regardless of whether these changes have been caused by the influence of bacteria, virus, prions, parasites, fungi, radiation, natural or artificial toxins, drugs or allergens, including mental conditions due to stress, neurosis, psychosis or deteriorations due to the ageing of the organism, and conditions or diseases of unknown cause, providing a sub-set of the probes as described herein are informative for said disease or condition or stage thereof.

Such diseases include those which result in metabolic or physiological changes, such as fever-associated diseases such as influenza or malaria. Other diseases which may be detected include for example yellow fever, sexually transmitted diseases such as gonorrhea, fibromyalgia, candida-related complex, cancer (for example of the stomach, lung, breast, prostate gland, bowel, skin, colon, ovary etc), Alzheimer's disease, disease caused by retroviruses such as HIV, senile dementia, multiple sclerosis and Creutzfeldt-Jakob disease to mention a few.

The methods disclosed herein may also be used to identify patients with psychiatric or psychosomatic diseases such as schizophrenia and eating disorders. Of particular importance is the use of this method to detect diseases, conditions, or stages thereof, which are not readily detectable by known diagnostic methods, such as HIV which is generally not detectable using known techniques 1 to 4 months following infection. Conditions which may be identified include for example drug abuse, such as the use of narcotics, alcohol, steroids or performance enhancing drugs.

Preferably said disease to be identified or monitored is a cancer or a degenerative brain disorder (such as Alzheimer's or Parkinson's disease).

In particular, a set of oligonucleotide probes, wherein said set comprises at least 10 oligonucleotides selected from:

an oligonucleotide as described in Table 4 or an oligonucleotide derived therefrom or an oligonucleotide with a complementary sequence, or a functionally equivalent oligonucleotide,

may be used for diagnosis or identification or monitoring the progression of Alzheimer's disease. Similarly Table 2 probes and Table 2 derived probes and their functional equivalents may be used to diagnose, identify or monitor the progression of breast cancer. Especially preferably the probes used for breast cancer analysis are selected based on their occurrence as set forth in Table 3 and as described hereinbefore.

The diagnostic method may be used alone as an alternative to other diagnostic techniques or in addition to such techniques. For example, methods as disclosed herein may be used as an alternative or additive diagnostic measure to diagnosis using imaging techniques such as Magnetic Resonance Imagine (MRI), ultrasound imaging, nuclear imaging or X-ray imaging, for example in the identification and/or diagnosis of tumours.

The methods disclosed herein may be performed on cells from prokaryotic or eukaryotic organisms which may be any eukaryotic organisms such as human beings, other mammals and animals, birds, insects, fish and plants, and any prokaryotic organism such as a bacteria.

Preferred non-human animals on which the methods disclosed herein may be conducted include, but are not limited to mammals, particularly primates, domestic animals, livestock and laboratory animals. Thus preferred animals for diagnosis include mice, rats, guinea pigs, cats, dogs, pigs, cows, goats, sheep, horses. Particularly preferably the disease state or condition of humans is diagnosed, identified or monitored.

As described above, the sample under study may be any convenient sample which may be obtained from an organism. Preferably however, as mentioned above, the sample is obtained from a site distant to the site of disease and the cells in such samples are not disease cells, have not been in contact with such cells and do not originate from the site of the disease or condition. In such cases, although preferably absent, the sample may contain cells which do not fulfil these criteria. However, since the probes disclosed herein are concerned with transcripts whose expression is altered in cells which do satisfy these criteria, the probes are specifically directed to detecting changes in transcript levels in those cells even if in the presence of other, background cells.

It has been found that the cells from such samples show significant and informative variations in the gene expression of a large number of genes. Thus, the same probe (or several probes) may be found to be informative in determinations regarding two or more diseases, conditions or stages thereof by virtue of the particular level of transcripts binding to that probe or the interrelationship of the extent of binding to that probe relative to other probes. As a consequence, it is possible to use a relatively small number of probes for screening for multiple disorders or diseases. This has consequences with regard to the selection of probes, discussed in relation to random identification of probes hereinafter, but also for the use of a single set of probes for more than one diagnosis. Table 9 discloses probes which are informative for both Alzheimer's and breast cancer.

Thus, also disclosed are sets of probes for diagnosing, identifying or monitoring two or more diseases, conditions or stages thereof, wherein at least one of said probes is suitable for said diagnosing, identifying or monitoring at least two of said diseases, conditions or stages thereof, and kits and methods of using the same. Preferably at least 5 probes, e.g. from 5 to 15 probes, are used in at least two diagnoses.

Thus, also disclosed is a method of diagnosis or identification or monitoring as described hereinbefore for the diagnosis, identification or monitoring of two or more diseases, conditions or stages thereof in an organism, wherein said test pattern produced in step c) of the diagnostic method is compared in step d) to at least two standard diagnostic patterns prepared as described previously, wherein each standard diagnostic pattern is a pattern generated for a different disease or condition or stage thereof.

Whilst in a preferred aspect the methods of assessment concern the development of a gene transcript pattern from a test sample and comparison of the same to a standard pattern, the elevation or depression of expression of certain markers may also be examined by examining the products of expression and the level of those products. Thus a standard pattern in relation to the expressed product may be generated.

In such methods the levels of expression of a set of polypeptides encoded by the gene to which an oligonucleotide of Table 1 or a Table 1 derived oligonucleotide, binds, are analysed.
Various diagnostic methods may be used to assess the amount of polypeptides (or fragments thereof) which are present. The presence or concentration of polypeptides may be examined, for example by the use of a binding partner to said polypeptide (e.g. an antibody), which may be immobilized, to separate said polypeptide from the sample and the amount of polypeptide may then be determined.

"Fragments" of the polypeptides refers to a domain or region of said polypeptide, e.g. an antigenic fragment, which is recognizable as being derived from said polypeptide to allow binding of a specific binding partner. Preferably such a fragment comprises a significant portion of said polypeptide and corresponds to a product of normal post-synthesis processing.

Thus also disclosed herein is a method of preparing a standard gene transcript pattern characteristic of a disease or condition or stage thereof in an organism comprising at least the steps of:

a) releasing target polypeptides from a sample of one or more organisms having the disease or condition or stage thereof;
b) contacting said target polypeptides with one or more binding partners, wherein each binding partner is specific to a marker polypeptide (or a fragment thereof) encoded by the gene to which an oligonucleotide of Table 1 (or derived from a sequence described in Table 1) binds, to allow binding of said binding partners to said target polypeptides, wherein said marker polypeptides are specific for said disease or condition thereof in an organism and sample thereof corresponding to the organism and sample thereof under investigation; and
c) assessing the target polypeptide binding to said binding partners to produce a characteristic pattern reflecting the level of gene expression of genes which express said marker polypeptides, in the sample with the disease, condition or stage thereof.

As used herein "target polypeptides" refer to those polypeptides present in a sample which are to be detected and "marker polypeptides" are polypeptides which are encoded by the genes to which Table 1 oligonucleotides or Table 1 derived oligonucleotides bind. The target and marker polypeptides are identical or at least have areas of high similarity, e.g. epitopic regions to allow recognition and binding of the binding partner.

"Release" of the target polypeptides refers to appropriate treatment of a sample to provide the polypeptides in a form accessible for binding of the binding partners, e.g. by lysis of cells where these are present. The samples used in this case need not necessarily comprise cells as the target polypeptides may be released from cells into the surrounding tissue or fluid, and this tissue or fluid may be analysed, e.g. urine or blood. Preferably however the preferred samples as described herein are used. "Binding partners" comprise the separate entities which together make an affinity binding pair as described above, wherein one partner of the binding pair is the target or marker polypeptide and the other partner binds specifically to that polypeptide, e.g. an antibody.

Various arrangements may be envisaged for detecting the amount of binding pairs which form. In its simplest form, a sandwich type assay e.g. an immunoassay such as an ELISA, may be used in which an antibody specific to the polypeptide and carrying a label (as described elsewhere herein) may be bound to the binding pair (e.g. the first antibody: polypeptide pair) and the amount of label detected.

Other methods as described herein may be similarly modified for analysis of the protein product of expression rather than the gene transcript and related nucleic acid molecules.

Thus, the present description discloses a method of preparing a test gene transcript pattern comprising at least the steps of:

a) releasing target polypeptides from a sample of said test organism;
b) contacting said target polypeptides with one or more binding partners, wherein each binding partner is specific to a marker polypeptide (or a fragment thereof) encoded by the gene to which an oligonucleotide of Table 1 (or derived from a sequence described in Table 1) binds, to allow binding of said binding partners to said target polypeptides, wherein said marker polypeptides are specific for said disease or condition thereof in an organism and sample thereof corresponding to the organism and sample thereof under investigation; and
c) assessing the target polypeptide binding to said binding partners to produce a characteristic pattern reflecting the level of gene expression of genes which express said marker polypeptides, in said test sample.

Also disclosed is a method of diagnosing or identifying or monitoring a disease or condition or stage thereof in an organism comprising the steps of:

a) releasing target polypeptides from a sample of said organism;
b) contacting said target polypeptides with one or more binding partners, wherein each binding partner is specific to a marker polypeptide (or a fragment thereof) encoded by the gene to which an oligonucleotide of Table 1 (or derived from a sequence described in Table 1) binds, to allow binding of said binding partners to said target polypeptides, wherein said marker polypeptides are specific for said disease or condition thereof in an organism and sample thereof corresponding to the organism and sample thereof under investigation; and

c) assessing the target polypeptide binding to said binding partners to produce a characteristic pattern reflecting the level of gene expression of genes which express said marker polypeptides in said sample; and

d) comparing said pattern to a standard diagnostic pattern prepared as described hereinbefore using a sample from an organism corresponding to the organism and sample under investigation to determine the degree of correlation indicative of the presence of said disease or condition or a stage thereof in the organism under investigation.

The methods of generating standard and test patterns and diagnostic techniques rely on the use of informative oligonucleotide probes to generate the gene expression data. In some cases it will be necessary to select these informative probes for a particular method, e.g. to diagnose a particular disease, from a selection of available probes, e.g. the probes described hereinbefore (the Table 1 oligonucleotides, the Table 1 derived oligonucleotides, their complementary sequences and functionally equivalent oligonucleotides). The following methodology describes a convenient method for identifying such informative probes, or more particularly how to select a suitable sub-set of probes from the probes described herein.

Probes for the analysis of a particular disease or condition or stage thereof, may be identified in a number of ways known in the prior art, including by differential expression or by library subtraction (see for example WO98/49342). As described hereinafter, in view of the high information content of most transcripts, as a starting point one may also simply analyse a random sub-set of mRNA or cDNA species and pick the most informative probes from that sub-set. The following method describes the use of immobilized oligonucleotide probes (e.g. the probes as described hereinbefore) to which mRNA (or related molecules) from different samples is bound to identify which probes are the most informative to identify a particular type of sample, e.g. a disease sample.

The immobilized probes can be derived from various unrelated or related organisms; the only requirement is that the immobilized probes should bind specifically to their homologous counterparts in test organisms. Probes can also be derived from commercially available or public databases and immobilized on solid supports or, as mentioned above, they can be randomly picked and isolated from a cDNA library and immobilized on a solid support.

The length of the probes immobilised on the solid support should be long enough to allow for specific binding to the target sequences. The immobilised probes can be in the form of DNA, RNA or their modified products or PNAs (peptide nucleic acids). Preferably, the probes immobilised should bind specifically to their homologous counterparts representing highly and moderately expressed genes in test organisms. Conveniently the probes which are used are the probes described herein.

The gene expression pattern of cells in biological samples can be generated using prior art techniques such as microarray or macroarray as described below or using methods described herein. Several technologies have now been developed for monitoring the expression level of a large number of genes simultaneously in biological samples, such as, high-density oligoarrays (Lockhart et al., 1996, Nat. Biotech., 14, p1675-1680), cDNA microarrays (Schena et al, 1995, Science, 270, p467-470) and cDNA macroarrays (Maier E et al., 1994, Nucl. Acids Res., 22, p3423-3424; Bernard et al., 1996, Nucl. Acids Res., 24, p1435-1442).

In high-density oligoarrays and cDNA microarrays hundreds and thousands of probe oligonucleotides or cDNAs, are spotted onto glass slides or nylon membranes, or synthesized on biochips. The mRNA isolated from the test and reference samples are labelled by reverse transcription with a red or green fluorescent dye, mixed, and hybridised to the microarray. After washing, the bound fluorescent dyes are detected by a laser, producing two images, one for each dye. The resulting ratio of the red and green spots on the two images provides the information about the changes in expression levels of genes in the test and reference samples. Alternatively, single channel or multiple channel microarray studies can also be performed.

In cDNA macroarray, different cDNAs are spotted on a solid support such as nylon membranes in excess in relation to the amount of test mRNA that can hybridise to each spot. mRNA isolated from test samples is radiolabelled by reverse transcription and hybridised to the immobilised probe cDNA. After washing, the signals associated with labels hybridising specifically to immobilised probe cDNA are detected and quantified. The data obtained in macroarray contains information about the relative levels of transcripts present in the test samples. Whilst macroarrays are only suitable to monitor the expression of a limited number of genes, microarrays can be used to monitor the expression of several thousand genes simultaneously and is, therefore, a preferred choice for large-scale gene expression studies.

A macroarray technique for generating the gene expression data set has been used to illustrate the probe identification method described herein. For this purpose, mRNA is isolated from samples of interest and used to prepare labelled target molecules, e.g. mRNA or cDNA as described above. The labelled target molecules are then hybridised to probes immobilised on the solid support. Various solid supports can be used for the purpose, as described previously. Following hybridization, unbound target molecules are removed and signals from target molecules hybridizing to immobilised probes quantified. If radio labelling is performed, PhosphoImager can be used to generate an image file that can be used to generate a raw data set. Depending on the nature of label chosen for labelling the target molecules, other instruments can also be used, for example, when fluorescence is used for labelling, a FluoroImager can be used to generate an image file from the hybridised target molecules.

The raw data corresponding to mean intensity, median intensity, or volume of the signals in each spot can be acquired from the image file using commercially available software for image analysis. However, the acquired data needs to be corrected for background signals and normalized prior to analysis, since, several factors can affect the quality and quantity of the hybridising signals. For example, variations in the quality and quantity of mRNA isolated from sample to sample, subtle variations in the efficiency of labelling target molecules during each reaction, and variations in the amount of unspecific binding between different macroarrays can all contribute to noise in the acquired data set that must be corrected for prior to analysis.

Background correction can be performed in several ways. The lowest pixel intensity within a spot can be used for background subtraction or the mean or median of the line of pixels around the spots' outline can be used for the purpose. One can also define an area representing the background intensity based on the signals generated from negative controls and use the average intensity of this area for background subtraction.

The background corrected data can then be transformed for stabilizing the variance in the data structure and normalized for the differences in probe intensity. Several transformation techniques have been described in the literature and a brief overview can be found in Cui, Kerr and Churchill http://www.jax.org/research/churchill/research/expression/Cui-Transform.pdf). Normalization can be performed by dividing the intensity of each spot with the collective intensity, average intensity or median intensity of all the spots in a macroarray or a group of spots in a macroarray in order to obtain the relative intensity of signals hybridising to immobilised probes in a macroarray. Several methods have been described for normalizing gene expression data (Richmond and Somerville, 2000, Current Opin. Plant Biol., 3, p108-116; Finkelstein et al., 2001, In "Methods of Microarray Data Analysis. Papers from CAMDA, Eds. Lin & Johnsom, Kluwer Academic, p57-68; Yang et al., 2001, In "Optical Technologies and Informatics", Eds. Bittner, Chen, Dorsel & Dougherty, Proceedings of SPIE, 4266, p141-152; Dudoit et al, 2000, J. Am. Stat. Ass., 97, p77-87; Alter et al 2000, supra; Newton et al., 2001, J. Comp. Biol., 8, p37-52). Generally, a scaling factor or function is first calculated to correct the intensity effect and then used for normalising the intensities. The use of external controls has also been suggested for improved normalization.

One other major challenge encountered in large-scale gene expression analysis is that of standardization of data collected from experiments performed at different times. We have observed that gene expression data for samples acquired in the same experiment can be efficiently compared following background correction and normalization. However, the data from samples acquired in experiments performed at different times requires further standardization prior to analysis. This is because subtle differences in experimental parameters between different experiments, for example, differences in the quality and quantity of mRNA extracted at different times, differences in time used for target molecule labelling, hybridization time or exposure time, can affect the measured values. Also, factors such as the nature of the sequence of transcripts under investigation (their GC content) and their amount in relation to the each other determines how they are affected by subtle variations in the experimental processes. They determine, for example, how efficiently first strand cDNAs, corresponding to a particular transcript, are transcribed and labelled during first strand synthesis, or how efficiently the corresponding labelled target molecules bind to their complementary sequences during hybridization. Batch to batch difference in the printing process is also a major factor for variation in the generated expression data.

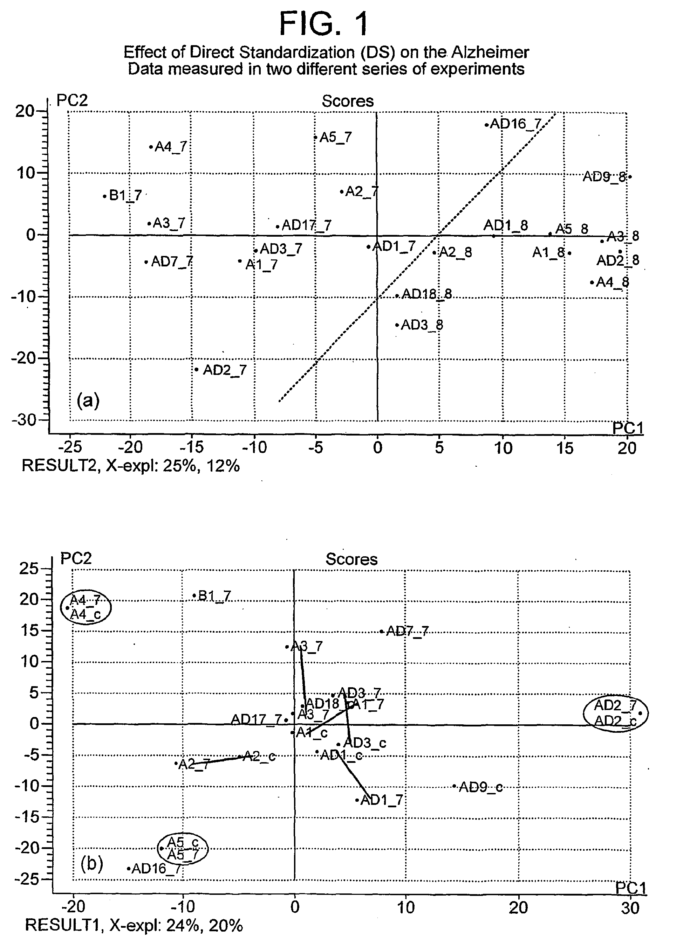

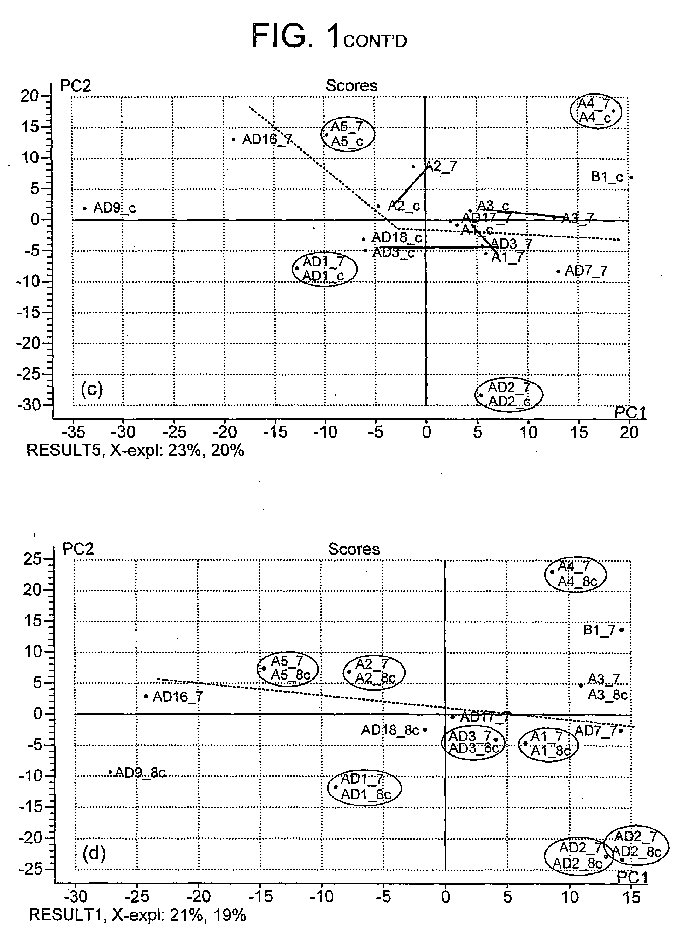

Failure to properly address and rectify for these influences leads to situations where the differences between the experimental series may overshadow the main information of interest contained in the gene expression data set, i.e. the differences within the combined data from the different experimental series. Figure 1 provides one such example showing a classification based on Principal Component Analysis (PCA) of combined data from two experimental series where the main goal is to distinguish between Alzheimer/non-Alzheimer patients.

PCA (also known as singular value decomposition) is a technique for studying interdependencies and underlying relationships of a set of variables. The data are modelled in terms of a few significant factors or principal components (PC's), plus residuals. The PC's contain the main phenomena and define the systematic variability present in the data, while the residuals represent the variability interpreted as noise. Details on PCA can be found in Jollife (1986, Principal Component Analysis, Springer-Verlag, NY), and Jackson (1991, A User's Guide to Principal Components, Wiley, NY). The results of Figure 1 show that two clusters are formed representing the data from two experimental series rather than the Alzheimer/non-Alzheimer differentiation. There were eight samples in common between the two series of experiments, which ideally should have fallen on top of, or in near proximity to, each other if appropriately standardized.

We have now found that gene expression data between different experiments can be efficiently standardized by including a subset of samples from one experimental series in the next experimental series and using a direct standardization method (DS), originally described by Wang and Kowalski (Anal. Chem., 1991, 63, p2750 and J. Chemometrics, 1991, 5, p129-145). Although the method of DS is well known in the field of analytical chemistry, it remains undescribed and unused in the field of gene expression data analysis.

In DS, the secondary data representing for example experimental series 2 (secondary measurements, $R_2$) are corrected to match the data measured on the primary measurements representing data from series 1 ($R_1$), while the calibration model remains unchanged. In DS, response matrices for both experimental series are related to each other by a transformation matrix F, i.e.

$$R_1 = R_2 F \qquad (1)$$

Where F is a square matrix dimensioned gene by gene. From (1), the transformation matrix is calculated as:

$$F = R_2{}^+ R_1 \qquad (2)$$

The transformation matrix F in equation (2) is calculated using a relatively small subset of samples which are measured on both the master primary and the secondary series of data.

Finally, the response of the unknown sample measured on the secondary series $r^T{}_{2,un}$, is standardized to the response vector $\hat{r}^T{}_{1,un}$ expected from the primary series

$$\hat{r}^T{}_{1,un} = r T_{21,un} \hat{F} \qquad (3)$$

From the preceding equation it can be seen that the column i of the transformation matrix contains the multiplication factors for a set of genes measured in the secondary series to obtain the intensity at spot i of the corrected series.

The number of samples that are repeated in the experimental series, $R_1$ and $R_2$, should be equal to their ranks, which in this case is equal to the number of principal components retained for explaining the variation in the $R_1$ and $R_2$. For example, if three principal components are retained for explaining the variation in the data set, a minimum of three samples should be repeated between $R_1$ and $R_2$. The samples that should be repeated between different series should ideally be those that exhibit high leverages in the gene expression pattern. At times, two samples may suffice, while at other times, more than two samples should be ideally be included for good representativity. In some cases, the samples selected can be the same in all the experimental series to be compared (reference samples), while in other cases, representative samples can be selected sequentially by analyzing the expression pattern after each experiment. The selected samples with high leverages are then included in the next experimental series. The results of using Direct Standardization are shown in Figure 1.

Another approach for normalizing and standardizing the gene expression data set is to hybridize each DNA array with target molecules prepared from a test sample and an equal amount of labelled target molecules prepared from representative reference samples. In order to measure the intensity of labelled target molecules hybridizing to the immobilized probes it is necessary that the labelled molecules are prepared from test and reference samples using different labels, for example, different fluorescent dyes can be used for preparing the labelled material. The labelled molecules prepared from reference samples can be added to the hybridization solution together with the labelled material prepared from test samples. A data file from each array representing the expression pattern of different genes in the test sample and reference samples can then be obtained, normalized and standardized by the direct standardization method as described above. An instant advantage of including the differentially labelled target molecules from reference samples during hybridization is that it enables an efficient comparison of new test samples to the data sets already stored in a database.

Monitoring the expression of a large number of genes in several samples leads to the generation of a large amount of data that is too complex to be easily interpreted. Several unsupervised and supervised multivariate data analysis techniques have already been shown to be useful in extracting meaningful biological information from these large data sets. Cluster analysis is by far the most commonly used technique for gene expression analysis, and has been performed to identify genes that are regulated in a similar manner, and or identifying new/unknown tumour classes using gene expression profiles (Eisen et al., 1998, PNAS, 95, p14863-14868, Alizadeh et al. 2000, supra, Perou et al. 2000, Nature, 406, p747-752; Ross et al, 2000, Nature Genetics, 24(3), p227-235; Herwig et al., 1999, Genome Res., 9, p1093-1105; Tamayo et al, 1999, Science, PNAS, 96, p2907-2912).

In the clustering method, genes are grouped into functional categories (clusters) based on their expression profile, satisfying two criteria: homogeneity- the genes in the same cluster are highly similar in expression to each other; and *separation* - genes in different clusters have low similarity in expression to each other.

Examples of various clustering techniques that have been used for gene expression analysis include hierarchical clustering (Eisen et al., 1998, supra; Alizadeh et al. 2000, supra; Perou et al. 2000, supra; Ross et al, 2000, supra), K-means clustering (Herwig et al., 1999, supra; Tavazoie et al, 1999, Nature Genetics, 22(3), p. 281-285), gene shaving (Hastie et al., 2000, Genome Biology, 1(2), research 0003.1-0003.21), block clustering (Tibshirani et al., 1999, Tech repot Univ Stanford.) Plaid model (Lazzeroni, 2002, Stat. Sinica, 12, p61-86), and self-organizing maps (Tamayo et al. 1999, supra). Also, related methods of multivariate statistical analysis, such as those using the singular value decomposition (Alter et al., 2000, PNAS, 97(18), p10101-10106; Ross et al. 2000, supra) or multidimensional scaling can be effective at reducing the dimensions of the objects under study.

However, methods such as cluster analysis and singular value decomposition are purely exploratory and only provide a broad overview of the internal structure present in the data. They are unsupervised approaches in which the available information concerning the nature of the class under investigation is not used in the analysis. Often, the nature of the biological perturbation to which a particular sample has been subjected is known. For example, it is sometimes known whether the sample whose gene expression pattern is being analysed derives from a diseased or healthy individual. In such instances, discriminant analysis can be used for classifying samples into various groups based on their gene expression data.

In such an analysis one builds the classifier by training the data that is capable of discriminating between member and non-members of a given class. The trained classifier can then be used to predict the class of unknown samples. Examples of discrimination methods that have been described in the literature include Support Vector Machines (Brown et al, 2000, PNAS, 97, p262-267), Nearest Neighbour (Dudoit et al., 2000, supra), Classification trees (Dudoit et al., 2000, supra), Voted classification (Dudoit et al., 2000, supra), Weighted Gene voting (Golub et al. 1999, supra), and Bayesian classification (Keller et al. 2000, Tec report Univ of Washington). Also a technique in which PLS (Partial Least Square) regression analysis is first used to reduce the dimensions in the gene expression data set followed by classification using logistic discriminant analysis and quadratic discriminant analysis (LD and QDA) has recently been described (Nguyen & Rocke, 2002, Bioinformatics, 18, p39-50 and 1216-1226).

A challenge that gene expression data poses to classical discriminatory methods is that the number of genes whose expression are being analysed is very large compared to the number of samples being analysed. However in most cases only a small fraction of these genes are informative in discriminant analysis problems. Moreover, there is a danger that the noise from irrelevant genes can mask or distort the information from the informative genes. Several methods have been suggested in literature to identify and select genes that are informative in microarray studies, for example, t-statistics (Dudoit et al, 2002, J. Am. Stat. Ass., 97, p77-87), analysis of variance (Kerr et al., 2000, PNAS, 98, p8961-8965), Neighbourhood analysis (Golub et al, 1999, supra), Ratio of between groups to within groups sum of squares (Dudoit et al., 2002, supra), Non parametric scoring (Park et al., 2002, Pacific Symposium on Biocomputing, p52-63) and Likelihood selection (Keller et al., 2000, supra).

In the methods described herein the gene expression data that has been normalized and standardized is analysed by using Partial Least Squares Regression (PLSR). Although PLSR is primarily a method used for regression analysis of continuous data (see Appendix A), it can also be utilized as a method for model building and discriminant analysis using a dummy response matrix based on a binary coding. The class assignment is based on a simple dichotomous distinction such as breast cancer (class 1) / healthy (class 2), or a multiple distinction based on multiple disease diagnosis such as breast cancer (class 1) / Alzheimer (class 2) / healthy (class 3). The list of diseases for classification can be increased depending upon the samples available corresponding to other diseases or conditions or stages thereof.

PLSR applied as a classification method is referred to as PLS-DA (DA standing for Discriminant analysis). PLS-DA is an extension of the PLSR algorithm in which the Y-matrix is a dummy matrix containing $n$ rows (corresponding to the number of samples) and $K$ columns (corresponding to the number of classes). The Y-matrix is constructed by inserting 1 in the $k$th column and -1 in all the other columns if the corresponding ith object of X belongs to class $k$. By regressing Y onto X, classification of a new sample is achieved by selecting the group corresponding to the largest component of the fitted, $\hat{y}(x) = (\hat{y}_1(x), \hat{y}_2(x), ..., \hat{y}_k(x))$. Thus, in a -1/1 response matrix, a prediction value below 0 means that the sample belongs to the class designated as -1, while a prediction value above 0 implies that the sample belongs to the class designated as 1.

An advantage of PLSR-DA is that the results obtained can be easily represented in the form of two different plots, the score and loading plots. Score plots represent a projection of the samples onto the principal components and shows the distribution of the samples in the classification model and their relationship to one another. Loading plots display correlations between the variables present in the data set.

It is usually recommended to use PLS-DA as a starting point for the classification problem due to its ability to handle collinear data, and the property of PLSR as a dimension reduction technique. Once this purpose has been satisfied, it is possible to use other methods such as Linear discriminant analysis, LDA, that has been shown to be effective in extracting further information, Indahl et al. (1999, Chem. and Intell. Lab. Syst., 49, p19-31). This approach is based on first decomposing the data using PLS-DA, and then using the scores vectors (instead of the original variables) as input to LDA. Further details on LDA can be found in Duda and Hart (Classification and Scene Analysis, 1973, Wiley, USA).

The next step following model building is of model validation. This step is considered to be amongst the most important aspects of multivariate analysis, and tests the "goodness" of the calibration model which has been built. In this work, a cross validation approach has been used for validation. In this approach, one or a few samples are kept out in each segment while the model is built using a full cross-validation on the basis of the remaining data. The samples left out are then used for prediction/classification. Repeating the simple cross-validation process several times holding different samples out for each cross-validation leads to a so-called double cross-validation procedure. This approach has been shown to work well with a limited amount of data, as is the case in some of the Examples described here. Also, since the cross validation step is repeated several times the dangers of model bias and overfitting are reduced.

Once a calibration model has been built and validated, genes exhibiting an expression pattern that is most relevant for describing the desired information in the model can be selected by techniques described in the prior art for variable selection, as mentioned elsewhere. Variable selection will help in reducing the final model complexity, provide a parsimonious model, and thus lead to a reliable model that can be used for prediction. Moreover, use of fewer genes for the purpose of providing diagnosis will reduce the cost of the diagnostic product. In this way informative probes which would bind to the genes of relevance may be identified.

We have found that after a calibration model has been built, statistical techniques like Jackknife (Effron, 1982, The Jackknife, the Bootstrap and other resampling plans. Society for Industrial and Applied mathematics, Philadelphia, USA), based on resampling methodology, can be efficiently used to select or confirm significant variables (informative probes).

The approximate uncertainty variance of the PLS regression coefficients B can be estimated by:

$$S^2B = \sum_{m=1}^{M} ((B-B_m)\,g)^2$$

where

$S^2B$ = estimated uncertainty variance of B;

B = the regression coefficient at the cross validated rank A using all the N objects;

$B_m$ = the regression coefficient at the rank A using all objects except the object(s) left out in cross validation segment m; and

g = scaling coefficient (here: g=1).

In our approach, Jackknife has been implemented together with cross-validation. For each variable the difference between the B-coefficients $B_i$ in a cross-validated sub-model and $B_{tot}$ for the total model is first calculated. The sum of the squares of the differences is then calculated in all sub-models to obtain an expression of the variance of the $B_i$ estimate for a variable. The significance of the estimate of $B_i$ is calculated using the t-test. Thus, the resulting regression coefficients can be presented with uncertainty limits that correspond to 2 Standard Deviations, and from that significant variables are detected.

No further details as to the implementation or use of this step are provided here since this has been implemented in commercially available software, The Unscrambler, CAMO ASA, Norway. Also, details on variable selection using Jackknife can be found in Westad & Martens (2000, J. Near Inf. Spectr., 8, p117-124).

The following approach can be used to select informative probes from a gene expression data set:

a) keep out one unique sample (including its repetitions if present in the data set) per cross validation segment;

b) build a calibration model (cross validated segment) on the remaining samples using PLSR-DA;

c) select the significant genes for the model in step b) using the Jackknife criterion;

d) repeat the above 3 steps until all the unique samples in the data set are kept out once (as described in step a). For example, if 75 unique samples are present in the data set, 75 different calibration models are built resulting in a collection of 75 different sets of significant probes;

e) select the most significant variables using the frequency of occurrence criterion in the generated sets of significant probes in step d). For example, a set of probes appearing in all sets (100%) are more informative than probes appearing in only 50% of the generated sets in step d).

Once the informative probes for a disease have been selected, a final model is made and validated. The two most commonly used ways of validating the model are cross-validation (CV) and test set validation. In ' cross-validation, the data is divided into k subsets. The model is then trained k times, each time leaving out one of the subsets from training, but using only the omitted subset to compute error criterion, RMSEP (Root Mean Square Error of Prediction). If k equals the sample size, this is called "leave-one-out" cross-validation. The idea of leaving one or a few samples out per validation segment is valid only in cases where the covariance between the various experiments is zero. Thus, one sample at-a-time approach can not be justified in situations containing replicates since keeping only one of the replicates out will introduce a systematic bias in our analysis. The correct approach in this case will be to leave out all replicates of the same samples at a time since that would satisfy assumptions of zero covariance between the CV-segments.

The second approach for model validation is to use a separate test-set for validating the calibration model. This requires running a separate set of experiments to be used as a test set. This is the preferred approach given that real test data are available.

The final model is then used to identify a disease, condition or stage thereof in test samples. For this purpose, expression data of selected informative genes is generated from test samples and then the final model is used to determine whether a sample belongs to a diseased or non-diseased class or has a condition or stage thereof.

Thus, also disclosed herein is a method of identifying probes useful for diagnosing or identifying or monitoring a disease or condition or stage thereof in an organism, comprising the steps of:

a) immobilizing a set of oligonucleotide probes, preferably as described hereinbefore, on a solid support;
b) isolating mRNA from a sample of a normal organism (normal sample), which may optionally be reverse transcribed to cDNA;
c) isolating mRNA from a sample from an organism, corresponding to the sample and organism of step (b), which is known to have said disease or condition or a stage thereof (diseased sample), which may optionally be reverse transcribed to cDNA;
d) hybridizing the mRNA or cDNA of steps (b) and (c) to said set of immobilized oligonucleotide probes of step (a); and
e) assessing the amount of mRNA or cDNA hybridizing to each of said oligonucleotide probes to determine the level of gene expression of genes to which said oligonucleotide probes bind in said normal and diseased samples to generate a gene expression data set for each sample;
f) normalizing and standardizing said data set of step (e);
g) constructing a calibration model for classification, preferably using the statistical techniques Partial Least Squares Discriminant Analysis (PLS-DA) and Linear Discriminant Analysis (LDA);
h) performing JackKnife analysis and identifying those oligonucleotide probes which are required for classification of said disease and normal samples into their respective groups.

Specifically, in a further aspect the present invention provides a method of identifying probes useful for diagnosing or identifying or monitoring breast cancer or Alzheimer's disease or a stage thereof in an organism, comprising the steps of:

a) immobilizing a set of oligonucleotide probes of the invention as described hereinbefore specific for breast cancer or Alzheimer's disease on a solid support;
b) isolating mRNA from a blood sample of a normal organism (normal sample), which may optionally be reverse transcribed to cDNA;
c) isolating mRNA from a sample from an organism, corresponding to the sample and organism of step (b), which is known to have breast cancer or Alzheimer's disease or a stage thereof (diseased sample), which may optionally be reverse transcribed to cDNA;
d) hybridizing the mRNA or cDNA of steps (b) and (c) from said organism with breast cancer or Alzheimer's disease to said set of immobilized oligonucleotide probes of step (a) for breast cancer or Alzheimer's disease, respectively; and
e) assessing the amount of mRNA or cDNA hybridizing to each of said oligonucleotide probes to determine the level of gene expression of genes to which said oligonucleotide probes bind in said normal and diseased samples to generate a gene expression data set for each sample;
f) normalizing and standardizing said data set of step (e);
g) constructing a calibration model for classification, preferably using the statistical techniques Partial Least Squares Discriminant Analysis (PLS-DA) and Linear Discriminant Analysis (LDA);
h) performing JackKnife analysis and identifying those oligonucleotide probes which are required for classification of said disease and normal samples into their respective groups, wherein oligonucleotides specific for breast cancer or a stage thereof are as set forth in Table 2b and oligonucleotides specific for Alzheimer's disease or a stage thereof are as set forth in Table 4b.

Preferably a model for classification purposes is generated by using the data relating to the probes identified according to the above described method. Preferably the sample is as described previously. Preferably the oligonucleotides which are immobilized in step (a) are randomly selected as described below or are the probes as described hereinbefore. Such oligonucleotides may be of considerable length, e.g. if using cDNA (which is encompassed within the scope of the term "oligonucleotide"). The identification of such cDNA molecules as useful probes allows the development of shorter oligonucleotides which reflect the specificity of the cDNA molecules but are easier to manufacture and manipulate.

The above described model may then be used to generate and analyse data of test samples and thus may be used for the diagnostic methods as described hereinbefore. In such methods the data generated from the test sample provides the gene expression data set and this is normalized and standardized as described above. This is then fitted to the calibration model described above to provide classification.

The method described herein can also be used to simultaneously select informative probes for several related and unrelated diseases or conditions. Depending upon which diseases or conditions have been included in the calibration or training set, informative probes can be selected for the said diseases or conditions. The informative probes selected for one disease or condition may or may not be similar to the informative probes selected for another disease or condition of interest. It is the pattern with which the selected genes are expressed in relation to each other during a disease, condition, or stage thereof, that determines whether or not they are informative for the disease, condition or stage thereof.

In other words, informative genes are selected based on how their expression correlates with the expression of other selected informative genes under the influence of responses generated by the disease, condition or stage thereof under investigation. In examples 1 and 2 provided hereinafter, 139 informative probes were selected for breast cancer diagnosis and 182 probes were selected for Alzheimer's disease diagnosis by training the gene expression data set of genes representing 1435 or 758 randomly picked cDNA clones for breast cancer/non breast cancer samples, or Alzheimer/non-Alzheimer samples, respectively. Among the probes selected for breast cancer and Alzheimer, about 10 probes were informative both for breast cancer and Alzheimer disease diagnosis.

For the purpose of isolating informative probes or identifying several related and unrelated diseases, conditions and stages thereof simultaneously, the gene expression data set must contain the information on how genes are expressed when the subject has a particular disease, condition or stage thereof under investigation. The data set is generated from a set of healthy or diseased samples, where a particular sample may contain the information of only one disease, condition or stages thereof or may also contain information about multiple diseases, conditions or stages thereof. For example, if the isolation of informative probes for Alzheimer disease, breast cancer and diabetes is sought, whole blood samples can be obtained from an Alzheimer patient who has breast cancer and diabetes. Hence, the method also teaches an efficient experimental design to reduce the number of samples required for isolating informative probes by selecting samples representing more than one disease, condition or stage thereof.

As mentioned previously, in view of the high information content of most transcripts, the identification and selection of informative probes for use in diagnosing, monitoring or identifying a particular disease, condition or stage thereof may be dramatically simplified. Thus the pool of genes from which a selection may be made to identify informative probes may be radically reduced.

Unlike, in prior art technologies where informative probes are selected from a population of thousands of genes that are being expressed in a cell, like in microarray, in the method described herein, the informative probes are selected from a limited number of randomly obtained genes. For example, from a population of 1435 cDNA clones, randomly picked from a human whole blood cDNA library, we were able to select 139 informative probes for breast cancer diagnosis (see Example 1 and Table 2).

Thus in a preferred aspect of the above mentioned method of identifying probes useful for diagnosing or identifying or monitoring a disease or condition or stage thereof in an organism, said set of oligonucleotides which are immobilized in step (a) are randomly selected from a larger set of oligonucleotides, e.g. from a cDNA library or other oligonucleotide pool, which may be, but is preferably not selected from the set provided herein. Preferably said larger set comprises oligonucleotides which correspond to moderately or highly expressed genes. Thus preferably in methods described herein, the set of oligonucleotides described herein are replaced with a set of oligonucleotides which are randomly selected, e.g. from commercially available oligonucleotide or cDNA libraries.

As referred to herein "random" refers to selection which is not biased based on the extent of information carried by the transcripts in relation to the disease, condition or organism under study, ie. without bias towards their likely utility as informative probes. Whilst a random selection may be made from a pool of transcripts (or related products) which have been biased, e.g. to highly or moderately expressed transcripts, preferably random selection is made from a pool of transcripts not biased or selected by a sequence-based criterion. The larger set may therefore contain oligonucleotides corresponding to highly and moderately expressed genes, or alternatively, may be enriched for those corresponding to the highly and moderately expressed genes.

Random selection from highly and moderately expressed genes can be achieved in a wide variety of ways. A strategy used in this work, but not limiting in itself involves randomly picking a significant number of cDNA clones from a cDNA library constructed from a biological specimen under investigation. Since, in a cDNA library, the cDNA clones corresponding to transcripts present in high or moderate amount are more frequently present than transcripts corresponding to cDNA present in low amount, the former will tend to be picked up more frequently than the later. A pool of cDNA enriched for those corresponding to highly and moderately expressed genes can be isolated by this approach.

To identify genes that are expressed in high or moderate amount among the isolated population for use in methods described herein, the information about the relative level of their transcripts in samples of interest can be generated using several prior art techniques. Both non-sequence based methods, such as differential display or RNA fingerprinting, and sequence-based methods such as microarrays or macroarrays can be used for the purpose. Alternatively, specific primer sequences for highly and moderately expressed genes can be designed and methods such as quantitative RT-CR can be used to determine the levels of highly and moderately expressed genes. Hence, a skilled practitioner may use a variety of techniques which are known in the art for determining the relative level of mRNA in a biological sample.

Especially preferably the sample for the isolation of mRNA in the above described method is as described previously and is preferably not from the site of disease and the cells in said sample are not disease, cells and have not contacted disease cells.

The following examples are given by way of illustration only in which the Figures referred to are as follows:

Figure 1 shows the effect of Direct Standardization (DS) on the Alzheimer data measured in two different series of experiments in which AD denotes Alzheimer's samples and A,B are non-Alzheimer's samples. The samples in both series have been labelled systematically as (xx_7/xx_8), whereas the corrected samples from series 8 (in b,c,d) have been labelled as (xx_c), thus, for example, AD2-7 denotes Alzheimer disease sample number 2 in experiment series 7. The circled spots represent the samples chosen as the transfer samples. The connecting lines in figures b,c,d show the proximity of the replicated samples after applying DS. The dashed lines in figures a,c,d represent the decision boundary separating the classes. These lines have not been drawn on the basis of any statistical criteria, but serve the purpose of visually separating the classes. All the four figures show scores plot (PC1-PC2) from PCA analysis based on (a) non-standardized data, (b) scores plot after direct standardization using 3 transfer samples, (c) scores plot after direct standardization using 4 transfer sample, (d) scores plot after direct standardization using 8 transfer samples;

Figure 2 shows the projection of normal (including benign) and breast cancer samples onto a classification model generated by PLSR-DA using the data of 44 informative genes, in which PC is the principal components and N and C are normal and breast cancer samples, respectively;

Figure 3 shows the projection of individuals with and without Alzheimer's disease onto a classification model generated by PLSR-DA using 182 informative genes;

Figures 4, 6 and 8 show projection plots as Figure 2 in which the classification model is generated using 719, 111 and 345 cDNAs, respectively, wherein PC is the principal components, N denotes normal and B denotes breast cancer samples;

Figures 5, 7 and 9 show prediction plots based on 3 principal components using the data of 719, 111 and 345 cDNAs, respectively;

Figure 10 shows a projection plot as Figure 3 in which the classification model is generated using 520 cDNAs; and

Figure 11 is the prediction plot corresponding to Figure 10.

Example 1: Diagnosis of Breast Cancer

Methods

Whole blood was obtained from the arms of breast cancer patients and patients with benign tumours (Ullevål and Haukland hospitals in Norway). All of the patients with breast cancer had a malignant tumour of the breast (disease samples). Healthy blood was collected from the above two hospitals, or collected at a Health station at Ås, Norway or at DiaGenic AS, Norway, from the arms of female donors with no reported signs of breast cancer. The blood from healthy individuals or with benign tumours comprise the normal samples. The blood was either collected in tubes containing EDTA and stored immediately at -80˚C or was collected in PAXgene tubes and stored for 12-24 hours at room temperature before finally storing them at -80˚C before use. Further details of the breast cancer and benign tumour patients from which blood was taken is provided in Table 5. mRNA was isolated from the blood of the 29 breast cancer patients and 46 normal donors and used to prepare labelled probes by reverse transcribing in the presence of $\alpha^{33}$P-dATP. The first strand cDNA of the normal and diseased samples was bound, separately to 1435 cDNA clones immobilized on a solid support (nylon membrane). These cDNA clones were randomly picked, without any prior knowledge of their gene sequences, from a cDNA library constructed using whole blood of 550 healthy individuals (Clontech, Palo Alto, USA). These methods were conducted as follows.

For amplification of inserts, bacterial clones were grown in microtiter plates containing 150 $\mu$l LB with 50 $\mu$g/ml carbenicillin, and incubated overnight with agitation at 37˚C. To lyse the cells, 5 $\mu$l of each culture were diluted with 50 $\mu$l H2O and incubated for 12 min. at 95˚C. Of this mixture, 2 $\mu$l were subjected to a PCR reaction using 20 pmoles of M13 forward and reverse primer in presence of 1.5 mM MgCl$_2$. PCR reactions were performed with the following cycling protocol: 4 min. at 95˚C, followed by 25 cycles of 1 min. at 94˚C, 1 min. at 60˚C and 3 min. at 72˚C either in a RoboCycler®

Temperature Cycler (Stratagene, La Jolla, USA) or DNA Engine Dyad Peltier Thermal Cycler (MJ Research Inc., Waltham, USA). The amplified products were denatured by incubating with NaOH (0.2 M, final concentration) for 30 min. and spotted onto Hybond-N+ membranes (Amersham Pharmacia Biotech, Little Chalfont, UK), using MicroGrid II workstation according to the manufacturer's instructions (BioRobotics Ltd, Cambridge England). The immobilized cDNAs were fixed using a UV cross-linker (Hoefer Scientific Instruments, San Francisco, USA).

In addition to the 1435 cDNAs, the printed arrays also contained controls for assessing background level, consistency and sensitivity of the assay. These were spotted at multiple positions and included controls such as PCR mix (without any insert); positive and negative controls of SpotReportTM 10 array validation system (Stratagene, La Jolla, USA) and cDNAs corresponding to constitutively expressed genes such as b-actin, g-actin, GAPDH, HOD and cyclophilin. Also, oligonucleotides corresponding to SIX1, b-tubulin, TRP-2, MDM2, Myosin Light C, CD44, Maspin, Laminin, and SRP 19 were included to detect disseminated cancer cells.

The total RNA from blood collected in EDTA tubes was purified using Trizol LS Reagent protocol (Invitrogen/ Life Technologies). From blood contained in PAXgene tubes, the total RNA was purified according to the supplier's instructions (PreAnalytiX, Hombrechtikon, Switzerland). Contaminating DNA was removed from the isolated RNA by DNAase I treatment using DNA-free kit (Ambion, Inc. Austin, USA). RNA quality was determined visually by inspecting the integrity of 28S and 18S ribosomal bands following agarose gel electrophoresis. The concentration and purity of extracted RNA was determined by measuring the absorbance at 260 nm and 280 nm. mRNA was isolated from the total RNA using Dynabeads as per the supplier's instructions (Dynal AS, Oslo, Norway).

Labelling and hybridization experiments were performed in batches. The number of samples assayed in each batch varied from six to nine. In the case of samples that were assayed more than once (replicates), aliquots derived from the same mRNA pool were used for probe synthesis. For probe synthesis, aliquots of mRNA corresponding to 4-5 $\mu$g of total RNA were mixed together with oligodT$_{25NV}$ (0.5 $\mu$g/ml) and mRNA spikes of SpotReport™ 10 array validation system (10 pg; Spike 2, 1 pg), heated to 70˚C to remove secondary structures, and then chilled on ice. Probes were prepared in 35$\mu$l reaction mixes by reverse transcription in the presence of 50$\mu$Ci [$\alpha^{33}$P] dATP, 3.5 $\mu$M dATP, 0.6 mM each of dCTP, dTTP, dGTP, 200 units of SuperScript reverse transcriptase (Invitrogen, LifeTechnologies) and 0.1 M DTT, labelling for 1.5 hr at 42˚C. Following synthesis, the enzyme was deactivated for 10 min. at 70˚C and mRNA removed by incubating the reaction mix for 20 min. at 37˚C in 4 units of Ribo H (Promega, Madison USA). Unincorporated nucleotides were removed using ProbeQuant G 50 Columns (Amersham Biosciences, Piscataway, USA).

Prior to hybridization, the membranes were equilibrated in 4 x SSC for 2 hr at room temperature and prehybridized overnight at 65˚C in 10 ml prehybridisation solution (4 x SSC, 0.1 M NaH$_2$PO$_4$, 1 mM EDTA, 8% dextran sulphate, 10 x denhardt's solution, 1% SDS). Freshly prepared probes were added to 5 ml of the same prehybridisation solution, and hybridization continued overnight at 65˚C. The membranes were washed at 65˚C at increasing stringency (2 x 30 min. each in 2 x SSC, 0.1% SDS; 1 x SSC, 0.1% SDS; 0.1 x SSC, 0.1% SDS) to remove unspecific signals.

The amount of labelled first strand cDNA binding to each spot was assessed and quantified using a PhosphoImager to generate a gene expression data set. The data was generated using Phoretix software version 3 (Non Linear Dynamics, England). Background subtraction was performed on the generated data by subtracting the median of the line of pixels around each spot outline from the total intensity obtained from the respective spots.

The background-subtracted data was then normalized and transformed by selecting out 50 lowest and 50 maximum signals from each membrane. This step was to exclude genes that were expressed with a high degree of variance. Since the genes varied from membrane to membrane, the expression data from 497 genes were removed from the data set. The values for the remaining 938 genes were then normalised by using different approaches such as external controls, dividing each spot by the median intensity of the observed signal in the respective membrane, range normalizing the data from each membrane, and then log transforming the data obtained.

The processed data obtained above was then used to isolate the informative probes by:

a) keeping one unique sample (including all repetitions of the selected sample) out per cross validation segment;
b) building a calibration model (cross validated) on the remaining samples using PLSR-DA;
c) selecting the set of significant genes for the model in step b using the Jackknife criterion;
d) repeating steps a), b) and c) until all the unique samples were kept out once (hence, in all 75 different calibration models were built (after repeating step b) 75 times), resulting in 75 different sets of significant probes (after repeating step c) 75 times));
e) selecting significant variables using the frequency of occurrence criterion amongst the 75 different sets of significant probes.

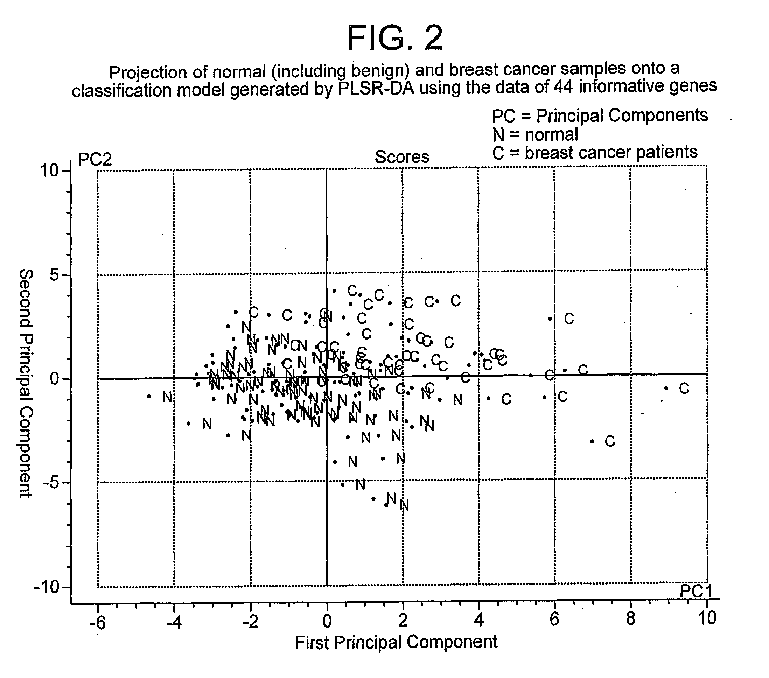

The selected informative probes based on occurrence criterion were used to construct a classification model. The result of the classification model based on probes appearing in at least 90% of the generated sets after the step of isolating informative probes as described above is shown in Figure 2 in which it is seen that the expression pattern of these genes was able to classify most women with breast cancer and women with no breast cancer into distinct groups.

In this figure PC1 and PC2 indicate the two principal components statistically derived from the data which best define the systemic variability present in the data. This allows each sample, and the data from each of the informative probes to which the sample's labelled first strand cDNA was bound, to be represented on the classification model as a single point which is a projection of the sample onto the principal components - the score plot.

The ability of the generated model, based on isolated informative probes, to predict future samples was determined by the double cross-validation approach. The performance of the diagnostic test for breast cancer based on the occurrence criterion is presented in Table 6.

Correct prediction of most breast cancer cells was achieved. These included all three samples obtained from women with ductal carcinoma in situ (DCIS), 11/15 samples obtained from women with stage I breast cancer, all five samples obtained from women with stage II breast cancer, and one of two samples obtained from women with stage III breast cancer. Interestingly, two correctly predicted stage I samples were obtained from women having a tumour size of <5 mm in diameter.

The model also correctly predicted the class of most non-cancer samples (41/46), including those that were obtained from women with non-cancerous breast abnormalities.

Confirmation that the gene transcripts are not from cells which are disseminated disease cells has been confirmed by several lines of evidences. Firstly, the informative genes were expressed constitutively at high or moderate levels in blood cells of women irrespective of whether they had cancer or not. Secondly, in the assay described in this Example, in order to identify transcripts, at least 720 disseminated cells in blood samples would be required. Since, the average number of disseminated cells present in blood during different stages of breast cancer is much lower (organ confined breast cancer, 0.8 cells per ml; invasive breast cancer spread to lymph nodes only, 2.4 cells per ml; and metastatic breast cancer, 6 cells per ml; SD>100%) (29), we believe that the signals being detected originated from peripheral blood cells and could not have originated from disseminated cells. Thirdly, we were not able to detect any signal from the eight cancer markers known to have elevated expression in malignant cancer cells, including cancer cells that are disseminated in the blood.

Example 2: Diagnosis of Alzheimer's disease

Similar experiments were conducted with samples from Alzheimer's patients. In this method 7 patients diagnosed with Alzheimer's Disease at the Memory Clinic at Ullevål University Hospital were used in the trial. The patients were confirmed as having Alzheimer's disease based on the following criteria:

* A standardized interview with a care-giver using IQCODE, an ADL scale and a scale measuring behaviour of the patient (Green scale).
* Neuropsychological evaluation using MMSE, Clock drawing test, Trailmaking test A and B (TMT A and B), Kendrick object learning test (visual memory test), part of the Wechsler battery and Benton test.
* A psychiatric evaluation using scales for detection of depression, MADRS for interviewing the patient and Cornell scale for interviewing the care-giver.
* A physical examination.
* Laboratory tests of blood samples to rule out other diseases.
* CT scan of the brain.
* SPECT of the brain.

The mean age of the patients was 72.3 with an age range of 69-76. The mean MMSE score was 22.0 (the maximum score attainable being 30).

Six age-matched individuals without diagnosed Alzheimer's disease were used as a control. All had been tested with MMSE and had a minimum score of 28 (mean: 28.4). The mean age of the normal control group was 73.0 and the age range 66-81. A sample from a 16-year old individual, with a consequent minimal chance of having Alzheimer's disease, was also included as an additional control.

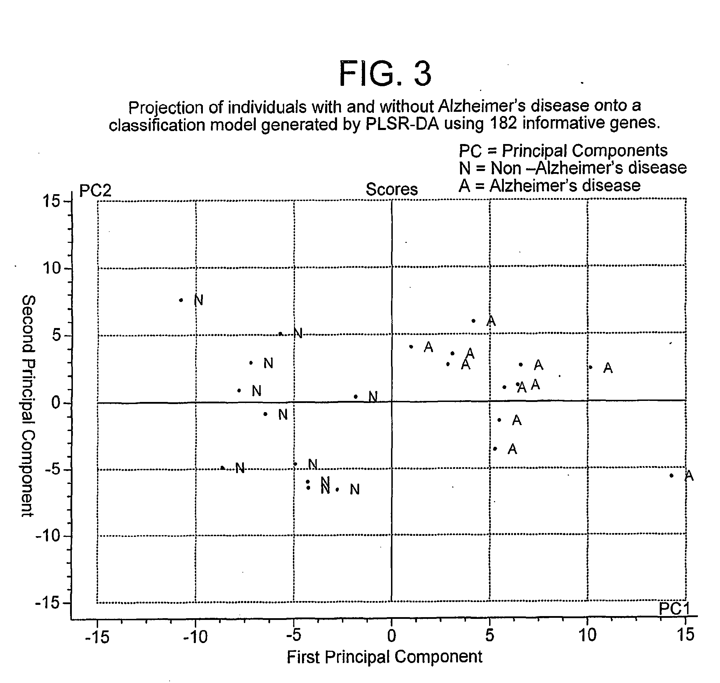

Using the methods described above (except that hybridization to 758 rather than 1435 cDNA clones was performed), informative probes were selected based on occurrence criterion and used to construct a classification model. The results of the classification model based on probes appearing at least once in the generated sets after the method to isolate informative probes as described above is shown in Figure 3 in which it will be seen that the expression pattern of these genes was able to classify individuals with or without Alzheimer's disease into distinct groups. In this Figure PC1 and PC2 indicate the 2 principal components statistically derived from the data which define the systematic variability present in the data. This allows each sample, and the data from each of the informative probes to which the samples' cDNA was bound, to be represented on the classification model as a single point which is a projection of the sample onto the principal components - the score plot.

The ability of the generated model, based on isolated informative probes, to predict future samples was determined by the double cross-validation. The performance of the diagnostic test for Alzheimer's disease is presented in Table 7.

Appendix A

Partial Least Squares regression (PLSR)

Let a multivariate regression model be defined as:

$$Y = XB + F$$

where
X a $NxP$ matrix with $N$ predictor variables (genes);
Y ($NxJ$) being the $J$ predicted variables. In our case Y represents a matrix containing dummy variables;
B is a matrix of regression coefficients; and
F is a $NxJ$ matrix of residuals.
The structure of the PLSR model can be written as:

$$X = TP^T + E_A,$$

and

$$Y = TQ^T + F_A,$$

where
where
T ($NxA$) is a matrix of score vectors which are linear combinations of the x-variables;
P ($PxA$) is a matrix with the x-loading vectors $p_a$ as columns;
Q ($JxA$) is a matrix with the y-loading vectors $q_a$ as columns;
$E_a$ ($NxP$) is the matrix for X after A factors; and
$F_a$ ($NxJ$) is the matrix for Y after A factors.
The criterion in PLSR is to maximize the explained covariance of [X,Y]. This is achieved by the loading weights vector $w_{a+1}$, which is the first eigenvector of $E_a^T F_a F_a^T E_a$ ($E_a$ and $F_a$ are the deflated X and Y after a factors or PLS components).
The regression coefficients are given by:

$$B = W(P^T W)^{-1} Q^T$$

A PLSR model with full rank, i.e. maximum number of components, is equivalent to the MLR solutions. Further details on PLSR can be found in Marteus & Naes, 1989, Multivariate Calibration, John Wiley & Sons, Inc., USA and Kowalski & Seasholtz, 1991, supra.

Example 3: Validation of Example 1 diagnosis of breast cancer

The results in Example 1 were validated by using the informative probes identified in Example 1 on new beast cancer and control samples.

Methods

The methods, essentially as described in Example 1, were used. Blood was taken from patients as described in Table 8. However, blood was collected in PAXgene tubes and the first strand labelled cDNAs were hybridized to 719 cDNAs spotted on nylon membranes along with other controls as described in Example 1. After background subtraction using control spots, the data of each membrane was normalized using the inter quantile range. The data was analysed as described in Example 1 and the model validated by cross validation.

The 719 cDNAs which were spotted are a subset of the cDNAs spotted in Example 1 and include 111 cDNAs described in Table 2 and which were found to be informative in Example 1.

Results

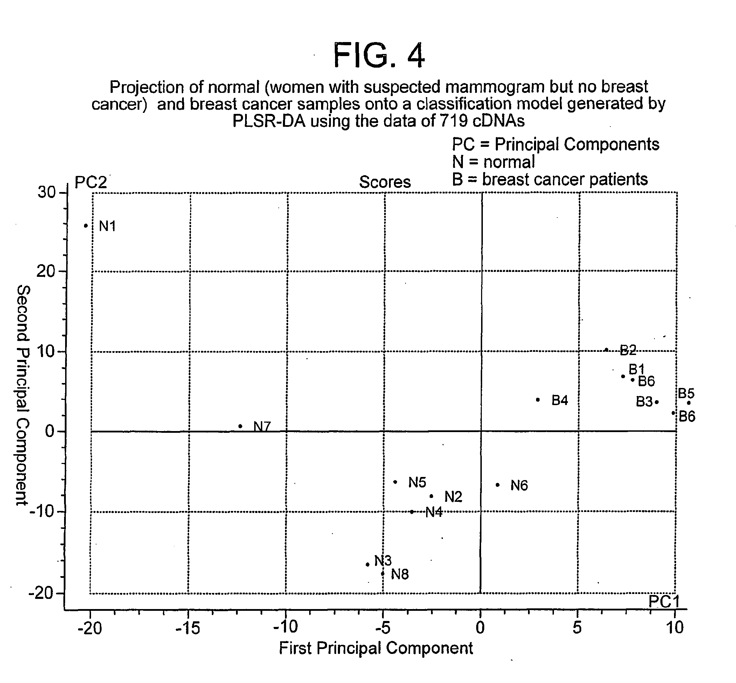

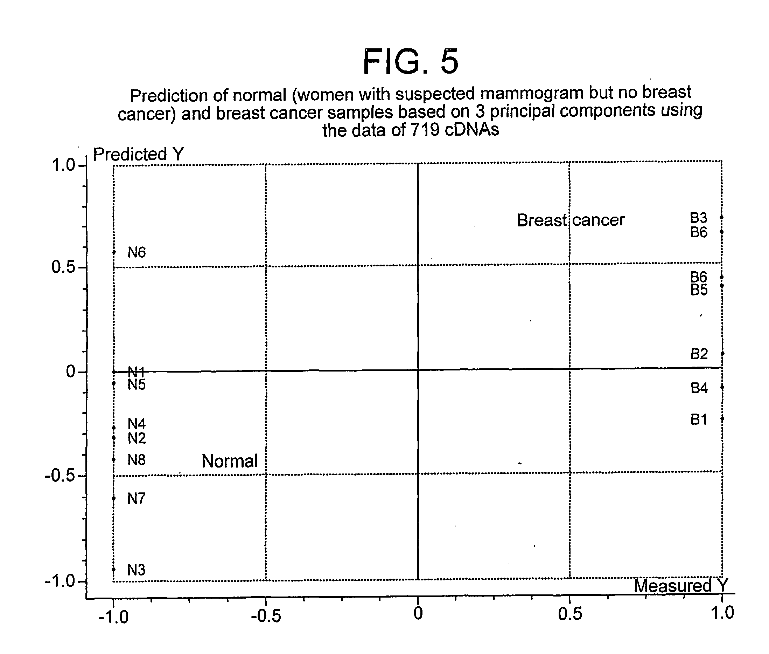

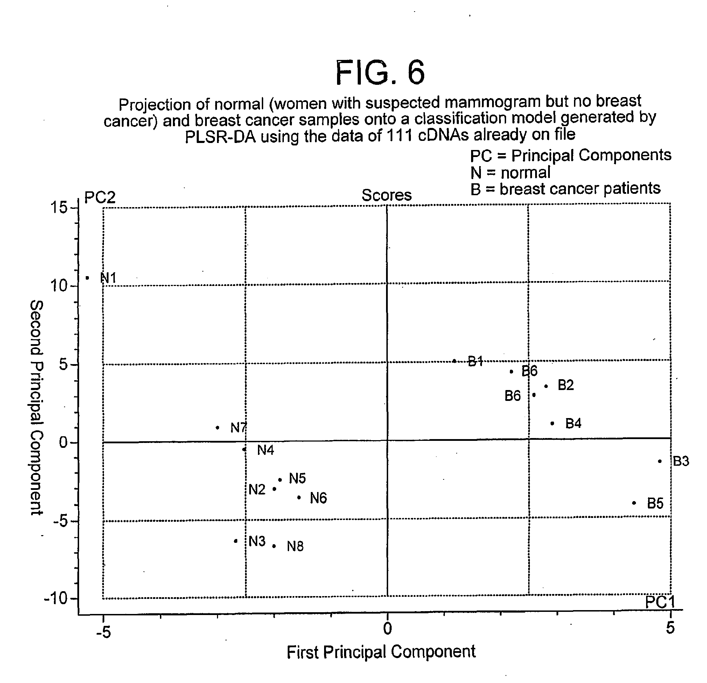

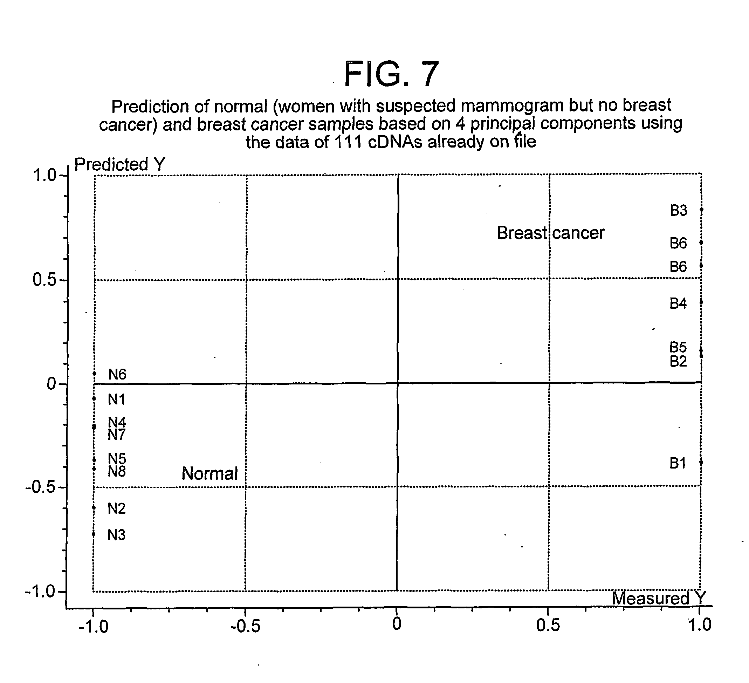

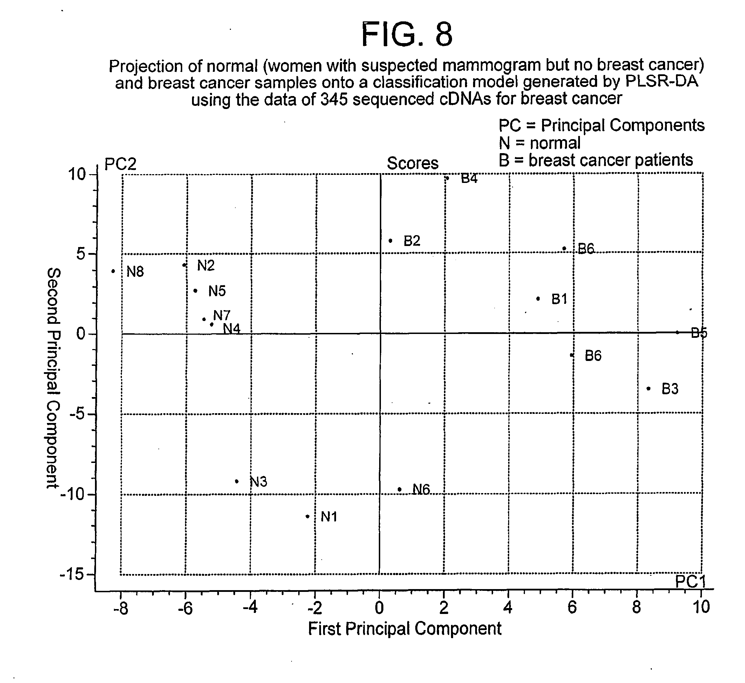

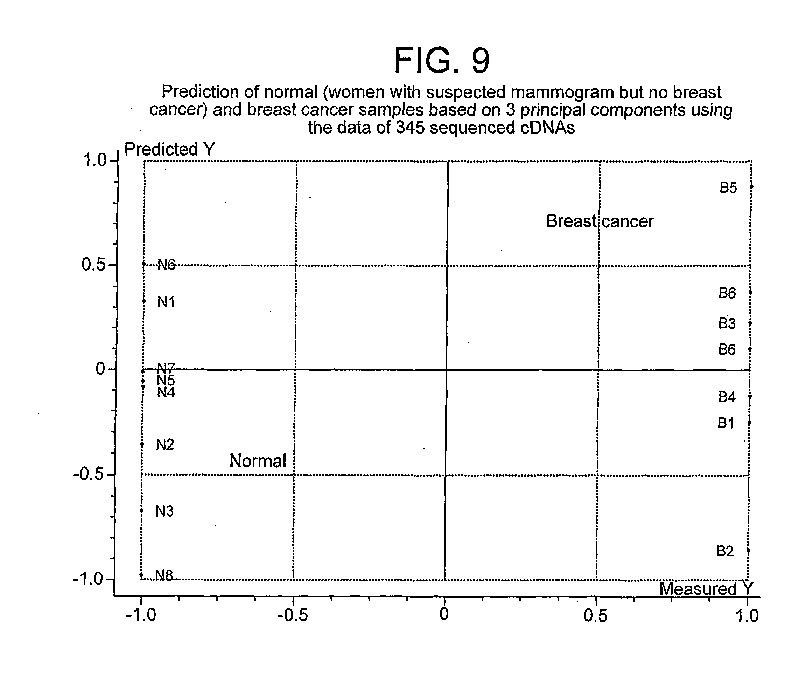

The results are shown in Figures 4 to 9. Figures 4, 6 and 8 are projection plots similar to Figure 2 and show the projection of normal and breast cancer patients' samples onto a classification model generated using all 719 cDNA. Figure 6 is similar but uses a classification model generated with the 111 probes common to Example 1. Figure 8 uses the 345 sequences of the 719 for which sequence information is provided herein. In each case classification of normal and breast cancer groups was possible. Figures 5, 7 and 9 show prediction plots which reflect the ability of the generated models to correctly diagnose breast cancer. In the 3 prediction plots shown, the disease samples appear on the x axis at +1 and the non-disease samples appear at -1. The y axis represents the predicted class membership. During prediction, if the prediction is correct, disease samples should fall above zero and non-disease samples should fall below zero. In each case almost all samples are correctly predicted.

Example 4: Validation of Example 2, diagnosis of Alzheimers

The results in Example 2 were validated by using the informative probes identified in Example 2 on new Alzheimer's patient samples.

Methods

The methods, essentially as described in Example 2, were used. Twelve female patients diagnosed with Alzheimer's disease at the Memory Clinic at Ullevål University Hospital who were confirmed as having Alzheimer's disease based on the criteria of Example 2 were used in the trial. The mean age of the patients was 72.3 with an age range of 66-83. The mean MMSE score was 22.0 (the maximum score attainable being 30).

Sixteen age-matched female individuals without diagnosed Alzheimer's disease were used as the normal control group. All had been tested with MMSE and had a minimum score of 29. The mean age of the normal control group was 74.0 and the age range 66-86.

After transfer of the blood to PAXgene tubes, total mRNA was isolated from the blood of the Alzheimer's disease and from the control group donors according to the manufacturers's instructions (PreAnalytiX, Hombrechtikon, Switzerland). The isolated mRNA was labelled during reverse transcription in the presence of $\alpha^{33}$P-dATP, yielding a labelled first strand cDNA. Hybridization was performed as described previously onto 730 cDNA clones picked from a cDNA library from whole blood of 550 healthy individuals without knowledge of the gene sequence of the random cDNA clones.

Results

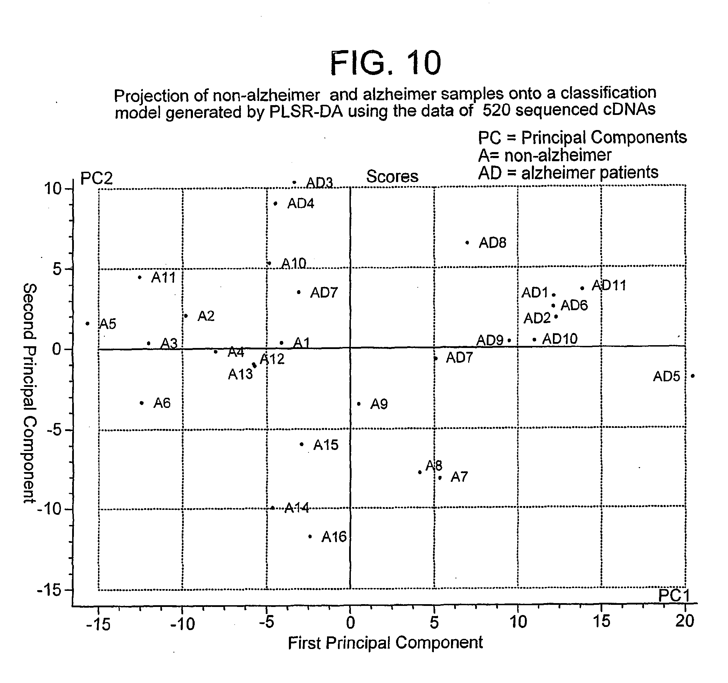

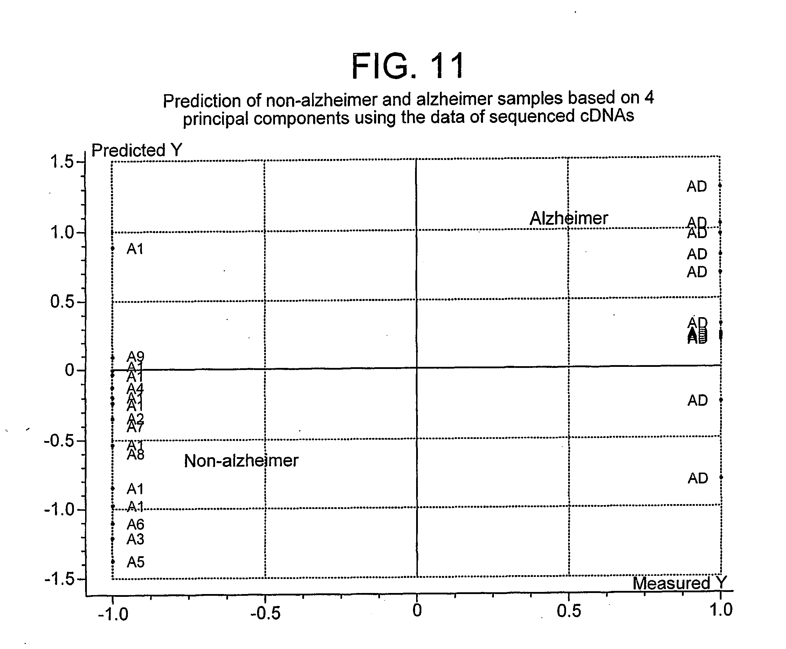

The results are shown in Figures 10 and 11. Figure 10 is a projection plot generated using 520 probes which have been sequenced. Figure 11 is a prediction plot and shows correct prediction of almost all samples.

Table 1a

| List of probes informative for disease diagnosis | | | |
|---|---|---|---|
| | **Clone ID** | **Sequence ID** | **No. of nucleotides** |
| 1 | I-01 | - | - |
| 2 | I-02 | - | - |
| 3 | I-13 | - | - |
| 4 | I-21 | - | - |
| 5 | I-24 | 308 | 373 |
| 6 | I-28 | 310 | 564 |

(continued)

| | Clone ID | Sequence ID | No. of nucleotides |
|---|---|---|---|
| | **List of probes informative for disease diagnosis** | | |
| 7 | I-30 | 1180 | 622 |
| 8 | I-34 | 313 | 554 |
| 9 | I-37 | - | - |
| 10 | I-42 | - | - |
| 11 | I-52 | - | - |
| 12 | I-54 | 1181 | 155 |
| 13 | I-68 | 326 | 554 |
| 14 | I-71 | - | - |
| 15 | I-72 | - | - |
| 16 | I-86 | - | - |
| 17 | I-95 | - | - |
| 18 | II-03 | 361 | 622 |
| 19 | II-05 | 363 | 628 |
| 20 | II-06 | 364 | 528 |
| 21 | II-10 | 368 | 329 |
| 22 | II-24 | 381 | 534 |
| 23 | II-25 | 382 | 444 |
| 24 | II-26 | 383 | 566 |
| 26 | II-33 | 390 | 523 |
| 26 | II-34 | 391 | 566 |
| 27 | II-41 | 397 | 534 |
| 28 | II-42 | 398 | 612 |
| 29 | II-41 | - | - |
| 30 | II-57 | 411 | 505 |
| 31 | II-61 | 415 | 596 |
| 32 | II-69 | 423 | 387 |
| 33 | II-70 | 424 | 420 |
| 34 | II-75 | 429 | 535 |
| 35 | II-83 | - | - |
| 36 | II-84 | 438 | 577 |
| 37 | II-87 | 441 | 552 |
| 38 | II-88 | 442 | 606 |
| 39 | II-90 | - | - |
| 40 | II-94 | 448 | 329 |
| 41 | III-02 | 453 | 747 |
| 42 | III-05 | - | - |
| 43 | III-06 | 458 | 682 |

(continued)

| | Clone ID | Sequence ID | No. of nucleotides |
|---|---|---|---|
| List of probes informative for disease diagnosis | | | |
| 44 | III-08 | 460 | 536 |
| 45 | III-10 | - | - |
| 48 | III-13 | 464 | 615 |
| 47 | III-15 | - | - |
| 48 | III-17 | - | - |
| 49 | III-20 | 1183 | 479 |
| 50 | III-23 | 473 | 694 |
| 51 | III-26 | 476 | 476 |
| 52 | III-35 | 485 | 551 |
| 53 | III-39 | 467 | 224 |
| 54 | III-40 | 488 | 349 |
| 55 | III-43 | 490 | 382 |
| 56 | III-44 | 491 | 382 |
| 57 | III-53 | 500 | 390 |
| 68 | III-56 | 503 | 109 |
| 59 | III-57 | 604 | 374 |
| 60 | III-60 | - | - |
| 61 | III-60 | - | - |
| 62 | III-61 | 507 | 521 |
| 63 | III-63 | 509 | 676 |
| 64 | III-68 | - | - |
| 65 | III-74 | 518 | 502 |
| 66 | III-80 | 523 | 585 |
| 67 | III-82 | - | - |
| 68 | III-85 | 526 | 516 |
| 69 | III-89 | 630 | 660 |
| 70 | III-92 | - | - |
| 71 | III-96 | - | - |
| 72 | IV-14 | 694 | 545 |
| 73 | IV-15 | 1185 | 628 |
| 74 | IV-23 | - | - |
| 76 | IV-26 | 1186 | 494 |
| 75 | IV-26 | - | - |
| 77 | IV-29 | - | - |
| 78 | IV-31 | 687 | 268 |
| 79 | IV-32 | 688 | 569 |
| 80 | IV-34 | - | - |

(continued)

| | Clone ID | Sequence ID | No. of nucleotides |
|---|---|---|---|
| | | List of probes informative for disease diagnosis | | |
| 81 | IV-35 | - | - |
| 82 | IV-41 | - | - |
| 83 | IV-45 | - | - |
| 84 | IV-53 | 61 | 362 |
| 85 | IV-62 | - | - |
| 86 | IV-69 | 192 | 286 |
| 87 | IV-80 | 701 | 679 |
| 88 | IV-82 | - | - |
| 89 | IV-93 | - | - |
| 90 | IX-10 | 736 | 641 |
| 91 | IX-12 | - | - |
| 92 | IX-38 | 757 | 683 |
| 93 | IX-39 | 758 | 424 |
| 94 | IX-42 | - | - |
| 95 | IX-48 | 764 | 626 |
| 96 | IX-77 | 785 | 556 |
| 97 | V-01 | - | - |
| 98 | V-02 | - | - |
| 99 | V-03 | 706 | 496 |
| 100 | V-04 | 707 | 397 |
| 101 | V-06 | - | - |
| 102 | V-07 | 708 | 293 |
| 103 | V-11 | 1188 | 599 |
| 104 | V-12 | 711 | 498 |
| 105 | V-15 | - | - |
| 106 | V-17 | - | - |
| 107 | V-21 | - | - |
| 108 | V-25 | - | - |
| 109 | V-32 | - | - |
| 110 | V-35 | - | - |
| 111 | V-39 | - | - |
| 112 | V-42 | - | - |
| 113 | V-43 | - | - |
| 114 | V-47 | - | - |
| 115 | V-49 | - | - |
| 116 | V-52 | - | - |
| 117 | V-54 | - | - |

(continued)

| | Clone ID | Sequence ID | No. of nucleotides |
|---|---|---|---|
| List of probes informative for disease diagnosis | | | |
| 118 | V-55 | 77 | 412 |
| 119 | V-58 | - | - |
| 120 | V-59 | - | - |
| 121 | V-65 | - | - |
| 122 | V-68 | - | - |
| 123 | V-71 | - | - |
| 124 | V-75 | - | - |
| 125 | V-79 | - | - |
| 126 | V-80 | 726 | 260 |
| 127 | V-90 | - | - |
| 128 | V-91 | - | - |
| 129 | V-92 | - | - |
| 130 | V-94 | - | - |
| 131 | VI-02 | - | - |
| 132 | VI-04 | 865 | 122 |
| 133 | VI-07 | 93 | 405 |
| 134 | VI-09 | - | - |
| 135 | VI-10 | - | - |
| 136 | VI-12 | 869 | 667 |
| 137 | VI-14 | 871 | 642 |
| 138 | VI-17 | - | - |
| 139 | VI-20 | 876 | 115 |
| 140 | VI-21 | - | - |
| 141 | VI-23 | 878 | 634 |
| 142 | VI-34 | - | - |
| 143 | VI-41 | - | - |
| 144 | VI-42 | - | - |
| 145 | VI-43 | - | - |
| 146 | VI-44 | - | - |
| 147 | VI-48 | 891 | 626 |
| 148 | VI-49 | - | - |
| 149 | VI-50 | 893 | 685 |
| 150 | VI-52 | - | - |
| 151 | VI-53 | 895 | 660 |
| 152 | VI-56 | 897 | 609 |
| 163 | VI-65 | - | - |
| 154 | VI-70 | 108 | 550 |

(continued)

| | Clone ID | Sequence ID | No. of nucleotides |
|---|---|---|---|
| | | List of probes informative for disease diagnosis | |
| 155 | VI-71 | - | - |
| 156 | VI-72 | - | - |
| 157 | VI-74 | 905 | 655 |
| 158 | VI-76 | 907 | 582 |
| 159 | VI-78 | - | - |
| 160 | VI-79 | - | - |
| 161 | VI-84 | - | - |
| 162 | VI-87 | 911 | 595 |
| 163 | VI-88 | 912 | 651 |
| 164 | VI-90 | - | - |
| 165 | VI-93 | - | - |
| 166 | VI-95 | 915 | 230 |
| 167 | VI-96 | - | - |
| 168 | VII-02 | - | - |
| 169 | VII-03 | 1196 | 412 |
| 170 | VII-06 | - | - |
| 171 | VII-10 | - | - |
| 172 | VIII-11 | - | - |
| 173 | VII-15 | 1199 | 439 |
| 174 | VII-19 | 562 | 580 |
| 175 | VII-21 | 564 | 671 |
| 176 | VII-25 | - | - |
| 177 | VII-32 | 571 | 457 |
| 178 | VII-36 | 676 | 209 |
| 179 | VII-39 | 576 | 541 |
| 180 | VII-42 | 579 | 502 |
| 181 | VII-43 | 680 | 316 |
| 182 | VII-46 | 583 | 631 |
| 183 | VII-47 | 1200 | 526 |
| 184 | VII-48 | 1201 | 613 |
| 185 | VII-69 | 693 | 565 |
| 186 | VII-80 | - | - |
| 187 | VII-63 | 595 | 98 |
| 188 | VII-66 | 598 | 362 |
| 189 | VII-67 | - | - |
| 180 | VII-72 | 600 | 595 |
| 191 | VII-73 | 601 | 522 |

(continued)

| | Clone ID | Sequence ID | No. of nucleotides |
|---|---|---|---|
| | List of probes informative for disease diagnosis | | |
| 192 | VII-75 | - | - |
| 183 | VII-76 | 603 | 624 |
| 194 | VII-77 | 1203 | 692 |
| 195 | VII-80 | 605 | 338 |
| 196 | VII-B1 | 606 | 656 |
| 197 | VII-83 | - | - |
| 198 | VII-86 | - | - |
| 199 | VII-88 | - | - |
| 200 | VII-90 | 612 | 576 |
| 201 | VII-91 | 613 | 341 |
| 202 | VII-93 | 615 | 379 |
| 203 . | VIII-01 | - | - |
| 204 | VIII-02 | - | - |
| 205 | VIII-03 | - | - |
| 206 | VIII-06 | - | - |
| 207 | VIII-09 | 618 | 698 |
| 208 | VIII-10 | - | - |
| 209 | VIII-15 | - | - |
| 210 | VIII-20 | 628 | 419 |
| 211 | VIII-22 | - | - |
| 212 | VIII-26 | - | - |
| 213 | VIII-28 | 634 | 511 |
| 214 | VIII-29 | 635 | 592 |
| 215 | VIII-30 | 636 | 572 |
| 216 | VIII-31 | 637 | 482 |
| 217 | VIII-32 | 638 | 545 |
| 218 | VIII-33 | 639 | 624 |
| 219 | VIII-39 | - | - |
| 220 | VIII-41 | 645 | 649 |
| 221 | VIII-42 | 646 | 600 |
| 222 | VIII-44 | - | - |
| 223 | VIII-46 | 649 | 425 |
| 224 | VIII-48 | 651 | 251 |
| 225 | VIII-58 | - | - |
| 226 | VIII-64 | 663 | 627 |
| 227 | VIII-65 | - | - |
| 228 | VIII-66 | 665 | 345 |

(continued)

| | Clone ID | Sequence ID | No. of nucleotides |
|---|---|---|---|
| | | | List of probes informative for disease diagnosis |
| 229 | VIII-67 | 666 | 252 |
| 230 | VIII-74 | - | - |
| 231 | VIII-76 | 675 | 591 |
| 232 | VIII-76 | - | - |
| 233 | VIII-82 | - | - |
| 234 | VIII-83 | - | - |
| 235 | VIII-85 | - | - |
| 236 | VIII-87 | - | - |
| 237 | VIII-91 | - | - |
| 238 | VIII-92 | - | - |
| 239 | VIII-93 | - | - |
| 240 | VIII-95 | - | - |
| 241 | X-04 | - | - |
| 242 | X-07 | 808 | 641 |
| 243 | X-15 | 814 | 132 |
| 244 | X-29 | 821 | 370 |
| 245 | X-34 | - | - |
| 246 | X-35 | - | - |
| 247 | X-54 | 837 | 603 |
| 248 | X-56 | 839 | 71 |
| 249 | X-68 | 1207 | 642 |
| 250 | X-72 | 849 | 622 |
| 251 | X-94 | 860 | 601 |
| 252 | XI-07 | - | - |
| 263 | XI-13 | 1209 | 620 |
| 254 | XI-50 | - | - |
| 255 | XI-58 | - | - |
| 256 | XI-81 | 1212 | 374 |
| 257 | XII-07 | 1213 | 667 |
| 258 | XII-17 | - | - |
| 259 | XII-26 | - | - |
| 220 | XII-27 | - | - |
| 261 | XII-31 | - | - |
| 262 | XII-32 | - | - |
| 263 | XII-35 | 1214 | 620 |
| 264 | XII-36 | - | - |
| 265 | XII-52 | - | - |

(continued)

| | Clone ID | Sequence ID | No. of nucleotides |
|---|---|---|---|
| | List of probes informative for disease diagnosis | | |
| 266 | XII-59 | 1216 | 484 |
| 267 | XIII-19 | 1219 | 659 |
| 268 | XIII-29 | - | - |
| 269 | XIII-52 | 939 | 513 |
| 270 | XIII-62 | - | - |
| 271 | XIII-84 | - | - |
| 272 | XIII-92 | 1221 | 741 |
| 273 | XV-18 | - | - |
| 274 | XV-22 | 1099 | 561 |
| 275 | XV-24 | - | - |
| 276 | XV-25 | 1224 | 485 |
| 277 | XV-28 | - | - |
| 278 | XV-34 | - | - |
| 279 | XV-42 | - | - |
| 280 | XV-68 | - | - |
| 281 | XV-74 | - | - |
| 282 | XV-93 | - | - |
| 283 | XV-94 | - | - |
| 284 | XV-96 | - | - |
| 285 | XVI-36 | 1056 | 435 |
| 286 | XVI-53 | 1230 | 741 |
| 287 | XVI-59 | - | - |
| 288 | XVI-66 | 1074 | 689 |
| 269 | XVI-76 | 1083 | 198 |
| 290 | XVI-77 | 1084 | 198 |
| 291 | XVII-07 | - | - |
| 292 | XVII-08 | - | - |
| 293 | XVII-17 | - | - |
| 294 | XVII-28 | - | - |
| 295 | XVII-29 | - | - |
| 296 | XVII-31 | 1139 | 503 |
| 297 | XVII-36 | - | - |
| 298 | XVII-39 | - | - |
| 299 | XVII-40 | 1231 | 203 |
| 300 | XVII-48 | 1148 | 587 |
| 301 | XVII-55 | - | - |
| 302 | XVII-50 | - | - |

(continued)

| | Clone ID | Sequence ID | No. of nucleotides |
|---|---|---|---|
| | | List of probes informative for disease diagnosis | |
| 303 | XVII-67 | - | - |
| 304 | XVII-72 | - | - |
| 365 | XVII-76 | 1160 | 650 |
| 306 | XVII-82 | - | - |
| 307 | XVII-87 | 1165 | 502 |
| 308 | XVII-95 | 1172 | 648 |

**Table 1b**

| List of sequences of probes informative for disease diagnosis Please see the note at the bottom | |
|---|---|
| **Clone ID** | **Sequence ID** |
| I-09 | 298 |
| I-10 | 299 |
| I-13 | 1331 |
| I-14 | 1178 |
| I-15 | 300 |
| I-16 | 301 |
| I-17 | 302 |
| I-19 | 304 |
| I-20 | 305 |
| I-22 | 306 |
| I-23 | 307 |
| I-24 | 308 |
| I-25 | 309 |
| I-28 | 310 |
| I-30 | 1180 |
| I-31 | 311 |
| I-32 | 312 |
| I-34 | 313 |
| I-37 | 1440 |
| I-38 | 314 |
| I-39 | 315 |
| I-40 | 316 |
| I-42 | 1332 |
| I-44 | 317 |
| I-45 | 318 |
| I-46 | 319 |

(continued)

| List of sequences of probes informative for disease diagnosis<br>Please see the note at the bottom | |
| --- | --- |
| **Clone ID** | **Sequence ID** |
| I-47 | 320 |
| I-48 | 321 |
| I-49 | 322 |
| I-53 | 323 |
| I-54 | 1181 |
| I-56 | 324 |
| I-57 | 325 |
| I-58 | 326 |
| I-60 | 327 |
| I-64 | 328 |
| I-67 | 330 |
| I-69 | 331 |
| I-71 | 332 |
| I-72 | 333 |
| I-73 | 334 |
| I-77 | 335 |
| I-79 | 336 |
| I-80 | 337 |
| I-81 | 338 |
| I-82 | 339 |
| I-86 | 1336 |
| I-88 | 1182 |
| 1-95 | 1337 |
| II-02 | 360 |
| II-03 | 361 |
| II-05 | 363 |
| II-06 | 364 |
| II-07 | 365 |
| II-08 | 366 |
| II-09 | 367 |
| II-10 | 368 |
| II-11 | 369 |
| II-12 | 370 |
| II-13 | 371 |
| II-14 | 372 |
| II-15 | 373 |

(continued)

| List of sequences of probes informative for disease diagnosis<br>Please see the note at the bottom | |
| --- | --- |
| **Clone ID** | **Sequence ID** |
| II-16 | 374 |
| II-17 | 375 |
| II-18 | 376 |
| II-20 | 377 |
| II-21 | 378 |
| II-22 | 379 |
| II-23 | 380 |
| II-24 | 381 |
| II-25 | 382 |
| II-26 | 383 |
| II-27 | 384 |
| II-28 | 385 |
| II-29 | 386 |
| II-30 | 387 |
| II-31 | 388 |
| II-32 | 389 |
| II-33 | 390 |
| II-34 | 391 |
| II-35 | 392 |
| II-37 | 393 |
| II-38 | 394 |
| II-39 | 395 |
| II-40 | 396 |
| II-41 | 397 |
| II-42 | 398 |
| II-43 | 399 |
| II-44 | 400 |
| II-46 | 401 |
| II-47 | 402 |
| II-48 | 403 |
| II-49 | 404 |
| II-50 | 405 |
| II-52 | 406 |
| II-53 | 407 |
| II-54 | 408 |
| II-55 | 409 |

(continued)

| List of sequences of probes informative for disease diagnosis<br>Please see the note at the bottom | |
| --- | --- |
| **Clone ID** | **Sequence ID** |
| II-56 | 410 |
| II-57 | 411 |
| II-58 | 412 |
| II-59 | 413 |
| II-60 | 414 |
| II-61 | 415 |
| II-62 | 416 |
| II-63 | 417 |
| II-64 | 418 |
| II-65 | 419 |
| II-66 | 420 |
| II-67 | 421 |
| II-68 | 422 |
| II-69 | 423 |
| II-70 | 424 |
| II-71 | 425 |
| II-72 | 426 |
| II-73 | 427 |
| II-74 | 428 |
| II-75 | 429 |
| II-76 | 430 |
| II-77 | 431 |
| II-78 | 432 |
| II-79 | 433 |
| II-80 | 434 |
| II-81 | 435 |
| II-82 | 436 |
| II-83 | 437 |
| II-84 | 438 |
| II-85 | 439 |
| II-86 | 440 |
| II-87 | 441 |
| II-88 | 442 |
| II-89 | 443 |
| II-90 | 444 |
| II-91 | 445 |

(continued)

| List of sequences of probes informative for disease diagnosis Please see the note at the bottom | |
|---|---|
| **Clone ID** | **Sequence ID** |
| II-92 | 446 |
| II-93 | 447 |
| II-94 | 448 |
| II-95 | 449 |
| II-96 | 450 |
| III-01 | 452 |
| III-02 | 453 |
| III-03 | 454 |
| III-04 | 455 |
| III-05 | 457 |
| III-06 | 458 |
| III-07 | 459 |
| III-08 | 460 |
| III-09 | 461 |
| III-11 | 462 |
| III-12 | 463 |
| III-13 | 464 |
| III-14 | 465 |
| III-15 | 466 |
| III-16 | 467 |
| III-17 | 468 |
| III-18 | 469 |
| III-19 | 470 |
| III-20 | 1183 |
| III-21 | 471 |
| III-22 | 472 |
| III-23 | 473 |
| III-24 | 474 |
| III-25 | 475 |
| III-26 | 476 |
| III-27 | 477 |
| III-28 | 478 |
| III-29 | 479 |
| III-31 | 481 |
| III-32 | 482 |
| III-33 | 483 |

(continued)

| List of sequences of probes informative for disease diagnosis<br>Please see the note at the bottom | |
| --- | --- |
| **Clone ID** | **Sequence ID** |
| III-34 | 484 |
| III-35 | 485 |
| III-37 | 486 |
| III-39 | 487 |
| III-40 | 488 |
| III-42 | 489 |
| III-43 | 490 |
| III-44 | 491 |
| III-45 | 492 |
| III-46 | 493 |
| III-47 | 494 |
| III-48 | 495 |
| III-49 | 496 |
| III-50 | 497 |
| III-51 | 498 |
| III-52 | 499 |
| III-53 | 500 |
| III-54 | 501 |
| III-55 | 502 |
| III-56 | 503 |
| III-57 | 504 |
| III-58 | 505 |
| III-59 | 506 |
| III-61 | 507 |
| III-62 | 508 |
| III-63 | 509 |
| III-64 | 510 |
| III-65 | 511 |
| III-66 | 512 |
| III-67 | 513 |
| III-69 | 514 |
| III-70 | 515 |
| III-71 | 516 |
| III-73 | 517 |
| III-74 | 518 |
| III-75 | 519 |

(continued)

| List of sequences of probes informative for disease diagnosis<br>Please see the note at the bottom | |
|---|---|
| **Clone ID** | **Sequence ID** |
| III-77 | 520 |
| III-78 | 521 |
| III-79 | 522 |
| III-80 | 523 |
| III-81 | 524 |
| III-82 | 1348 |
| III-83 | 525 |
| III-85 | 526 |
| III-86 | 527 |
| III-87 | 528 |
| III-88 | 529 |
| III-89 | 530 |
| III-91 | 531 |
| III-92 | 1351 |
| III-93 | 532 |
| III-94 | 533 |
| III-95 | 534 |
| III-96 | 535 |
| IV-02 | 681 |
| IV-04 | 682 |
| IV-13 | 683 |
| IV-14 | 684 |
| IV-15 | 1185 |
| IV-17 | 685 |
| IV-23 | 1353 |
| IV-26 | 1186 |
| IV-28 | 686 |
| IV-31 | 687 |
| IV-32 | 688 |
| IV-35 | 1355 |
| IV-37 | g6 |
| IV-38 | 689 |
| IV-40 | 690 |
| IV-42 | 691 |
| IV-43 | 1239 |
| IV-44 | 692 |

(continued)

| List of sequences of probes informative for disease diagnosis<br>Please see the note at the bottom | |
| --- | --- |
| **Clone ID** | **Sequence ID** |
| IV-47 | 693 |
| IV-53 | 61 |
| IV-55 | 694 |
| IV-56 | 695 |
| IV-61 | 696 |
| IV-64 | 697 |
| IV-65 | 698 |
| IV-69 | 192 |
| IV-72 | 699 |
| IV-73 | 700 |
| IV-80 | 701 |
| IV-82 | 196 |
| IV-85 | 702 |
| IV-93 | 703 |
| IV-95 | 704 |
| IV-96 | 705 |
| IX-10 | 736 |
| IX-12 | 738 |
| IX-13 | 739 |
| IX-24 | 747 |
| IX-38 | 757 |
| IX-39 | 758 |
| IX-48 | 764 |
| IX-50 | 766 |
| IX-56 | 768 |
| IX-62 | 773 |
| IX-65 | 776 |
| IX-72 | 782 |
| IX-77 | 785 |
| IX-91 | 796 |
| IX-96 | 801 |
| V-01 | 1361 |
| V-03 | 706 |
| V-04 | 707 |
| V-07 | 708 |
| V-08 | 709 |

(continued)

| List of sequences of probes informative for disease diagnosis<br>Please see the note at the bottom | |
|---|---|
| **Clone ID** | **Sequence ID** |
| V-09 | 710 |
| V-11 | 1188 |
| V1-16 | 873 |
| V1-19 | 875 |
| V-12 | 711 |
| V-17 | 1364 |
| V-18 | 712 |
| V-20 | 713 |
| V-24 | 714 |
| V-25 | 1365 |
| V-28 | 1189 |
| V-35 | 1366 |
| V-37 | 716 |
| V-38 | 1190 |
| V-39 | 1109 |
| V-40 | 717 |
| V-41 | 718 |
| V-47 | 1368 |
| V-48 | 719 |
| V-49 | 1369 |
| V-55 | 77 |
| V-57 | 720 |
| V-58 | 1370 |
| V-61 | 721 |
| V-64 | 722 |
| V-65 | 723 |
| V-68 | 1448 |
| V-71 | 1495 |
| V-74 | 724 |
| V-75 | 1372 |
| V-80 | 726 |
| V-81 | 727 |
| V-87 | 728 |
| V-90 | 1374 |
| VI-02 | 340 |
| VI-03 | 341 |

(continued)

| List of sequences of probes informative for disease diagnosis | |
|---|---|
| Please see the note at the bottom | |
| **Clone ID** | **Sequence ID** |
| VI-04 | 342 |
| VI-06 | 343 |
| VI-07 | 344 |
| VI-08 | 345 |
| VI-09 | 346 |
| VI-11 | 347 |
| VI-12 | 869 |
| VI-13 | 870 |
| VI-14 | 871 |
| VI-16 | 873 |
| VI-18 | 348 |
| VI-19 | 349 |
| VI-20 | 350 |
| VI-21 | 351 |
| VI-22 | 352 |
| VI-23 | 878 |
| VI-24 | 879 |
| VI-25 | 353 |
| VI-26 | 354 |
| VI-27 | 355 |
| VI-31 | 356 |
| VI-32 | 885 |
| VI-33 | 357 |
| VI-35 | 358 |
| VI-39 | 887 |
| VI-43 | 1382 |
| VI-44 | 1193 |
| VI-45 | 889 |
| VI-48 | 359 |
| VI-49 | 892 |
| VI-50 | 893 |
| VI-53 | 895 |
| VI-55 | 897 |
| VI-58 | 899 |
| VI-66 | 903 |
| VI-67 | 904 |

(continued)

| List of sequences of probes informative for disease diagnosis<br>Please see the note at the bottom | |
| --- | --- |
| **Clone ID** | **Sequence ID** |
| VI-70 | 108 |
| VI-71 | 1387 |
| VI-74 | 905 |
| VI-75 | 906 |
| VI-76 | 907 |
| VI-77 | 110 |
| VI-79 | 1389 |
| VI-80 | 908 |
| VI-85 | 910 |
| VI-87 | 911 |
| VI-88 | 912 |
| VI-90 | 1390 |
| VI-93 | 1391 |
| VI-95 | 915 |
| VI-96 | 1392 |
| VII-02 | 547 |
| VII-03 | 548 |
| VII-04 | 549 |
| VII-05 | 550 |
| VII-O6 | 551 |
| VII-07 | 552 |
| VII-08 | 553 |
| VII-09 | 554 |
| VII-10 | 555 |
| VII-11 | 556 |
| VII-12 | 557 |
| VII-14 | 558 |
| VII-15 | 559 |
| VII-17 | 560 |
| VII-18 | 561 |
| VII-19 | 562 |
| VII-20 | 563 |
| VII-21 | 564 |
| VII-22 | 565 |
| VII-23 | 566 |
| VII-24 | 567 |

(continued)

| List of sequences of probes informative for disease diagnosis Please see the note at the bottom | |
| --- | --- |
| **Clone ID** | **Sequence ID** |
| VII-25 | 1397 |
| VII-26 | 250 |
| VII-27 | 568 |
| VII-28 | 569 |
| VII-29 | 570 |
| VII-32 | 571 |
| VII-33 | 572 |
| VII-34 | 573 |
| VII-35 | 574 |
| VII-36 | 575 |
| VII-39 | 576 |
| VII-40 | 577 |
| VII-41 | 578 |
| VII-42 | 579 |
| VII-43 | 580 |
| VII-44 | 581 |
| VII-45 | 582 |
| VII-46 | 583 |
| VII-47 | 1200 |
| VII-48 | 584 |
| VII-49 | . 585 |
| VII-50 | 586 |
| VII-52 | 587 |
| VII-53 | 588 |
| VII-54 | 589 |
| VII-55 | 590 |
| VII-57 | 591 |
| VII-58 | 592 |
| VII-59 | 593 |
| VII-62 | 594 |
| VII-63 | 595 |
| VII-64 | 596 |
| VII-65 | 597 |
| VII-66 | 598 |
| VII-67 | 1399 |
| VII-71 | 599 |

(continued)

| List of sequences of probes informative for disease diagnosis<br>Please see the note at the bottom | |
|---|---|
| **Clone ID** | **Sequence ID** |
| VII-72 | 600 |
| VII-73 | 601 |
| VII-74 | 602 |
| VII-76 | 603 |
| VII-77 | 604 |
| VII-80 | 605 |
| VII-81 | 606 |
| VII-82 | 607 |
| VII-83 | 608 |
| VII-84 | 609 |
| VII-86 | 1453 |
| VII-87 | 610 |
| VII-89 | 611 |
| VII-90 | 612 |
| VII-91 | 613 |
| VII-92 | 614 |
| VII-93 | 615 |
| VII-94 | 616 |
| VII-96 | 617 |
| VIII-09 | 618 |
| VIII-10 | 619 |
| VIII-11 | 620 |
| VIII-12 | 621 |
| VIII-13 | 622 |
| VIII-15 | 623 |
| VIII-16 | 624 |
| VIII-17 | 625 |
| VIII-18 | 626 |
| VIII-19 | 627 |
| VIII-20 | 628 |
| VIII-21 | 629 |
| VIII-22 | 1455 |
| VIII-23 | 630 |
| VIII-24 | 631 |
| VIII-25 | 632 |
| VIII-26 | 1456 |

(continued)

| List of sequences of probes informative for disease diagnosis Please see the note at the bottom | |
|---|---|
| **Clone ID** | **Sequence ID** |
| VIII-27 | 633 |
| VIII-28 | 634 |
| VIII-29 | 635 |
| VIII-30 | 636 |
| VIII-31 | 637 |
| VIII-32 | 638 |
| VIII-33 | 639 |
| VIII-34 | 640 |
| VIII-36 | 641 |
| VIII-37 | 642 |
| VIII-38 | 643 |
| VIII-40 | 644 |
| VIII-41 | 645 |
| VIII-42 | 646 |
| VIII-43 | 647 |
| VIII-45 | 648 |
| VIII-46 | 649 |
| VIII-47 | 650 |
| VIII-48 | 651 |
| VIII-50 | 652 |
| VIII-51 | 653 |
| VIII-53 | 654 |
| VIII-54 | 655 |
| VIII-55 | 656 |
| VIII-56 | 657 |
| VIII-57 | 658 |
| VIII-58 | 659 |
| VIII-59 | 660 |
| VIII-60 | 661 |
| VIII-61 | 662 |
| VIII-64 | 663 |
| VIII-65 | 664 |
| VIII-66 | 665 |
| VIII-67 | 666 |
| VIII-68 | 667 |
| VIII-69 | 668 |

(continued)

| List of sequences of probes informative for disease diagnosis Please see the note at the bottom | |
|---|---|
| **Clone ID** | **Sequence ID** |
| VIII-70 | 669 |
| VIII-71 | 670 |
| VIII-72 | 671 |
| VIII-73 | 672 |
| VIII-74 | 673 |
| VIII-75 | 674 |
| VIII-76 | 675 |
| VIII-77 | 676 |
| VIII-78 | 677 |
| VIII-79 | 678 |
| VIII-80 | 679 |
| X-07 | 808 |
| X-15 | 814 |
| X-20 | 817 |
| X-29 | 821 |
| X-34 | 825 |
| X-46 | 833 |
| X-54 | 837 |
| X-56 | 839 |
| X-68 | 1207 |
| X-72 | 849 |
| X-73 | 1208 |
| X-94 | 860 |
| XI-13 | 1209 |
| XI-37 | 1460 |
| XI-43 | 1210 |
| XI-67 | 1211 |
| XI-81 | 1212 |
| XII-07 | 1213 |
| XII-35 | 1214 |
| XII-36 | 1215 |
| XII-59 | 1216 |
| XII-65 | 1028 |
| XII-92 | 1217 |
| XIII-03 | 917 |
| XIII-04 | 1218 |

(continued)

| List of sequences of probes informative for disease diagnosis<br>Please see the note at the bottom | |
|---|---|
| **Clone ID** | **Sequence ID** |
| XIII-19 | 1219 |
| XIII-24 | 926 |
| XIII-51 | 938 |
| XIII-52 | 939 |
| XIII-67 | 947 |
| XIII-69 | 949 |
| XIII-88 | 1220 |
| XIII-92 | 1221 |
| XV-22 | 1099 |
| XV-24 | 1101 |
| XV-25 | 1224 |
| XV-42 | 1108 |
| XV-62 | 1226 |
| XV-64 | 1118 |
| XV-84 | 1125 |
| XVI-19 | 1228 |
| XVI-36 | 1056 |
| XVI-53 | 1230 |
| XVI-60 | 1071 |
| XVI-66 | 1074 |
| XVI-74 | 1081 |
| XVI-76 | 1083 |
| XVI-77 | 1084 |
| XVII-31 | 1139 |
| XVII-40 | 1231 |
| XVII-48 | 1148 |
| XVII-76 | 1160 |
| XVII-87 | 1165 |

(continued)

| List of sequences of probes informative for disease diagnosis<br>Please see the note at the bottom | |
| --- | --- |
| **Clone ID** | **Sequence ID** |
| XVII-95 | 1172 |
| **Note**<br>**Sequences not available for sequence IDs in Table 1, and corresponding sequence Ids in Table 2 and 4.**<br>298,301,305,307,312,317,318,319,320,332,333,334,336,340,341,342,343,344,345,346,347,348,349,350,351,352,353,354,355,356,357,358,359,367,372,375,376,377,379,385,392,393,404,437,439,440,443,444,445,449,455,457,465,466,467,468,470,486,498,501,511,514,516,517,520,522,528,531,535,547,548,549,550,551,552,553,554,555,556,557,558,559,573,584,604,608,616,620,623,640,659,662,664,667,668,673,677,678,679,681,695,702,712,716,825,886,894,902,909,916,1101,1108,1109,1177,1187,1193,1204,1220,1239,1255,1256,1342,1347,1354,1357,1362,1363,1364,1373,1375,1379,1403,1404,1405,1406,1413 | |

Table 2a

| List of informative probes for diagnosis of breast cancer | | | | |
| --- | --- | --- | --- | --- |
| **Clone ID** | **Sequence ID** | | **Clone ID** | **Sequence ID** |
| I-24 | 308 | | VI-72 | - |
| I-28 | 310 | | VI-78 | - |
| I-30 | 1180 | | VI-84 | - |
| I-52 | - | | VII-03 | 1196 |
| I-54 | 1181 | | VII-15 | 1199 |
| II-41 | 397 | | VII-32 | 571 |
| II-70 | 424 | | VII-39 | 576 |
| II-87 | 441 | | VII-47 | 1200 |
| III-06 | 458 | | VII-48 | 1201 |
| III-20 | 1183 | | VII-60 | - |
| III-40 | 488 | | VII-73 | 601 |
| III-67 | 504 | | VII-77 | 1203 |
| III-60 | - | | VII-90 | 612 |
| III-61 | 507 | | VIII-20 | 628 |
| III-89 | 530 | | VIII-29 | 635 |
| IV-14 | 684 | | VIII-30 | 636 |
| IV-15 | 1185 | | VIII-31 | 637 |
| IV-26 | 1186 | | VIII-39 | - |
| IV-32 | 688 | | VIII-44 | - |
| IV-41 | - | | VIII-46 | 649 |
| IV-53 | 61 | | VIII-48 | 651 |
| IV-62 | - | | VIII-66 | 665 |
| IV-69 | 192 | | VIII-74 | - |
| IV-80 | 701 | | VIII-76 | 675 |

(continued)

| List of informative probes for diagnosis of breast cancer | | | | |
|---|---|---|---|---|
| **Clone ID** | **Sequence ID** | | **Clone ID** | **Sequence ID** |
| IV-82 | 196 | | X-04 | - |
| IX-10 | 736 | | X-07 | 808 |
| IX-12 | - | | X-15 | 814 |
| IX-38 | 757 | | X-29 | 821 |
| IX-39 | 758 | | X-34 | - |
| IX-42 | - | | X-35 | - |
| IX-48 | 764 | | X-54 | 837 |
| IX-77 | 785 | | X-56 | 839 |
| V-11 | 1188 | | X-68 | 1207 |
| V-32 | - | | X-72 | 849 |
| V-39 | - | | X-94 860 | - |
| V-55 | 77 | | XI-07 | - |
| V-80 | 726 | | XI-13 | 1209 |
| V-94 | - | | XI-50 | - |
| VI-07 | 93 | | XI-58 | - |
| VI-34 | - | | XI-81 | 1212 |
| VI-41 | - | | XII-07 | 1213 |
| VI-48 | 891 | | XII-17 | - |
| VI-49 | - | | XII-26 | - |
| VI-52 | - | | XII-27 | - |
| VI-55 | 897 | | XII-31 | - |
| VI-65 | - | | XII-32 | - |
| VI-70 | 108 | | XII-35 | 1214 |
| XII-36 | - | | | |
| XII-52 | - | | | |
| XII-59 | 1216 | | | |
| XIII-19 | 1219 | | | |
| XIII-29 | - | | | |
| XIII-52 | 939 | | | |
| XIII-62 | - | | | |
| XIII-84 | - | | | |
| XIII-92 | 1221 | | | |
| XV-18 | - | | | |
| XV-22 | 1099 | | | |
| XV-24 | - | | | |
| XV-25 | 1224 | | | |
| XV-28 | - | | | |

(continued)

| List of informative probes for diagnosis of breast cancer | | | | |
|---|---|---|---|---|
| **Clone ID** | **Sequence ID** | | **Clone ID** | **Sequence ID** |
| XV-34 | - | | | |
| XV-42 | - | | | |
| XV-68 | - | | | |
| XV-74 | - | | | |
| XV-93 | - | | | |
| XV-94 | - | | | |
| XV-96 | - | | | |
| XVI-36 | 1056 | | | |
| XVI-53 | 1230 | | | |
| XVI-59 | - | | | |
| XVI-68 | 1074 | | | |
| XVI-76 | 1083 | | | |
| XVI-77 | 1084 | | | |
| XVII-07 | - | | | |
| XVII-08 | - | | | |
| XVII-17 | - | | | |
| XVII-28 | - | | | |
| XVII-29 | - | | | |
| XVII-31 | 1139 | | | |
| XVII-36 | - | | | |
| XVII-39 | - | | | |
| XVII-40 | 1231 | | | |
| XVII-48 | 1148 | | | |
| XVII-55 | - | | | |
| XVII-58 | - | | | |
| XVII-67 | - | | | |
| XVII-72 | - | | | |
| XVII-76 | 1160 | | | |
| XVII-82 | - | | | |
| XVII-87 | 1165 | | | |
| XVII-95 | 1172 | | | |
| | | | | |

**Table 2b**

| List of sequences of probes informative for breast cancer<br>Please see the note at the bottom of Table 1. Some sequences are missing. ||
| :--- | :--- |
| Clone ID | Sequence ID |
| I-13 | 1331 |
| I-14 | 1178 |
| I-24 | 308 |
| I-25 | 309 |
| I-28 | 310 |
| I-30 | 1180 |
| I-37 | 1440 |
| I-42 | 1332 |
| I-48 | 321 |
| I-54 | 1181 |
| I-60 | 327 |
| I-72 | 1335 |
| I-81 | 338 |
| I-82 | 339 |
| I-86 | 1336 |
| I-88 | 1182 |
| I-95 | 1337 |
| II-02 | 360 |
| II-03 | 361 |
| II-06 | 364 |
| II-07 | 365 |
| II-10 | 368 |
| II-21 | 378 |
| II-23 | 380 |
| II-24 | 381 |
| II-25 | 382 |
| II-27 | 384 |
| II-33 | 390 |
| II-34 | 391 |
| II-41 | 397 |
| II-42 | 398 |
| II-46 | 401 |
| II-47 | 1338 |
| II-48 | 403 |
| II-52 | 406 |
| II-57 | 411 |

(continued)

| List of sequences of probes informative for breast cancer | |
| --- | --- |
| Please see the note at the bottom of Table 1. Some sequences are missing. | |
| Clone ID | Sequence ID |
| II-58 | 412 |
| II-59 | 413 |
| II-60 | 414 |
| II-61 | 415 |
| II-62 | 416 |
| II-64 | 418 |
| II-67 | 421 |
| II-69 | 423 |
| II-70 | 424 |
| II-74 | 428 |
| II-80 | 434 |
| II-82 | 436 |
| II-84 | 438 |
| II-87 | 441 |
| II-88 | 442 |
| II-96 | 450 |
| III-01 | 452 |
| III-02 | 453 |
| III-06 | 458 |
| III-08 | 460 |
| III-12 | 463 |
| III-13 | 464 |
| III-17 | 1344 |
| III-18 | 469 |
| III-20 | 1183 |
| III-21 | 471 |
| III-23 | 473 |
| III-24 | 474 |
| III-25 | 475 |
| III-26 | 476 |
| III-27 | 477 |
| III-28 | 478 |
| III-29 | 479 |
| III-32 | 482 |
| III-33 | 483 |
| III-35 | 485 |

(continued)

| List of sequences of probes informative for breast cancer | |
| --- | --- |
| Please see the note at the bottom of Table 1. Some sequences are missing. | |
| Clone ID | Sequence ID |
| III-39 | 487 |
| III-40 | 488 |
| III-42 | 489 |
| III-45 | 492 |
| III-46 | 493 |
| III-47 | 494 |
| III-48 | 495 |
| III-56 | 503 |
| III-57 | 504 |
| III-58 | 505 |
| III-59 | 506 |
| III-61 | 507 |
| III-62 | 508 |
| III-63 | 509 |
| III-64 | 510 |
| III-66 | 512 |
| III-67 | 513 |
| III-70 | 515 |
| III-74 | 518 |
| III-75 | 519 |
| III-78 | 521 |
| III-80 | 523 |
| III-81 | 524 |
| III-82 | 1348 |
| III-85 | 526 |
| III-86 | 527 |
| III-88 | 529 |
| III-89 | 530 |
| III-92 | 1351 |
| III-93 | 532 |
| III-95 | 534 |
| III-96 | 1352 |
| IV-04 | 682 |
| IV-13 | 683 |
| IV-14 | 684 |
| IV-15 | 1185 |

(continued)

| List of sequences of probes informative for breast cancer | |
|---|---|
| Please see the note at the bottom of Table 1. Some sequences are missing. | |
| Clone ID | Sequence ID |
| IV-17 | 685 |
| IV-23 | 1353 |
| IV-26 | 1186 |
| IV-31 | 687 |
| IV-32 | 688 |
| IV-35 | 1355 |
| IV-37 | g6 |
| IV-38 | 689 |
| IV-42 | 691 |
| IV-43 | 1239 |
| IV-47 | 693 |
| IV-53 | 61 |
| IV-61 | 696 |
| IV-64 | 697 |
| IV-69 | 192 |
| IV-72 | 699 |
| IV-80 | 701 |
| IV-82 | 196 |
| IV-85 | 702 |
| IV-93 | 1360 |
| IV-96 | 705 |
| IX-10 | 736 |
| IX-12 | 738 |
| IX-13 | 739 |
| IX-24 | 747 |
| IX-38 | 757 |
| IX-39 | 758 |
| IX-48 | 764 |
| IX-50 | 766 |
| IX-56 | 768 |
| IX-62 | 773 |
| IX-65 | 776 |
| IX-72 | 782 |
| IX-77 | 785 |
| IX-91 | 796 |
| IX-96 | 801 |

(continued)

| List of sequences of probes informative for breast cancer | |
| --- | --- |
| Please see the note at the bottom of Table 1. Some sequences are missing. | |
| Clone ID | Sequence ID |
| V-01 | 1361 |
| V-03 | 706 |
| V-04 | 707 |
| V-07 | 708 |
| V-08 | 709 |
| V-11 | 1188 |
| V-12 | 711 |
| V-17 | 1364 |
| V-24 | 714 |
| V-25 | 1365 |
| V-28 | 1189 |
| V-35 | 1366 |
| V-38 | 1190 |
| V-39 | 1109 |
| V-41 | 718 |
| V-47 | 1368 |
| V-49 | 1369 |
| V-55 | 77 |
| V-57 | 720 |
| V-58 | 1370 |
| V-61 | 721 |
| V-64 | 722 |
| V-65 | 1371 |
| V-68 | 1448 |
| V-71 | 1495 |
| V-74 | 724 |
| V-75 | 1372 |
| V-80 | 726 |
| V-90 | 1374 |
| VI-03 | 864 |
| VI-04 | 865 |
| VI-07 | 93 |
| VI-08 | 867 |
| VI-09 | 1378 |
| VI-12 | 869 |
| VI-13 | 870 |

(continued)

| List of sequences of probes informative for breast cancer | |
|---|---|
| Please see the note at the bottom of Table 1. Some sequences are missing. | |
| Clone ID | Sequence ID |
| VI-14 | 871 |
| VI-16 | 873 |
| VI-19 | 875 |
| VI-20 | 876 |
| VI-21 | 1380 |
| VI-23 | 878 |
| VI-24 | 879 |
| VI-25 | 1192 |
| VI-26 | 881 |
| VI-32 | 885 |
| VI-39 | 887 |
| VI-43 | 1382 |
| VI-44 | 1193 |
| VI-45 | 889 |
| VI-48 | 891 |
| VI-49 | 892 |
| VI-50 | 893 |
| VI-53 | 895 |
| VI-55 | 897 |
| VI-58 | 899 |
| VI-66 | 903 |
| VI-67 | 904 |
| VI-70 | 108 |
| VI-71 | 1387 |
| VI-74 | 905 |
| VI-75 | 906 |
| VI-76 | 907 |
| VI-77 | 110 |
| VI-79 | 1389 |
| VI-80 | 908 |
| VI-85 | 910 |
| VI-87 | 911 |
| VI-88 | 912 |
| VI-90 | 1390 |
| VI-93 | 1391 |
| VI-95 | 915 |

(continued)

| List of sequences of probes informative for breast cancer | |
| --- | --- |
| Please see the note at the bottom of Table 1. Some sequences are missing. | |
| Clone ID | Sequence ID |
| VI-96 | 1392 |
| VII-02 | 1195 |
| VII-03 | 1196 |
| VII-06 | 1394 |
| VII-08 | 1197 |
| VII-09 | 1198 |
| VII-10 | 1395 |
| VII-11 | 1396 |
| VII-15 | 1199 |
| VII-17 | 560 |
| VII-19 | 562 |
| VII-21 | 564 |
| VII-22 | 565 |
| VII-23 | 566 |
| VII-24 | 567 |
| VII-25 | 1397 |
| VII-26 | 250 |
| VII-27 | 568 |
| VII-29 | 570 |
| VII-32 | 571 |
| VII-33 | 572 |
| VII-36 | 575 |
| VII-39 | 576 |
| VII-41 | 578 |
| VII-42 | 579 |
| VII-43 | 580 |
| VII-46 | 583 |
| VII-47 | 1200 |
| VII-48 | 1201 |
| VII-49 | 585 |
| VII-54 | 589 |
| VII-57 | 591 |
| VII-58 | 592 |
| VII-59 | 593 |
| VII-62 | 594 |
| VII-63 | 1202 |

(continued)

| List of sequences of probes informative for breast cancer Please see the note at the bottom of Table 1. Some sequences are missing. | |
|---|---|
| Clone ID | Sequence ID |
| VII-64 | 596 |
| VII-66 | 598 |
| VII-67 | 1399 |
| VII-72 | 600 |
| VII-73 | 601 |
| VII-77 | 1203 |
| VII-80 | 605 |
| VII-82 | 607 |
| VII-86 | 1453 |
| VII-87 | 610 |
| VII-90 | 612 |
| VII-91 | 613 |
| VII-92 | 614 |
| VII-93 | 615 |
| VII-96 | 617 |
| VIII-09 | 618 |
| VIII-10 | 619 |
| VIII-13 | 622 |
| VIII-16 | 624 |
| VIII-20 | 628 |
| VIII-21 | 629 |
| VIII-22 | 1455 |
| VIII-23 | 630 |
| VIII-24 | 631 |
| VIII-25 | 632 |
| VIII-26 | 1456 |
| VIII-27 | 633 |
| VIII-28 | 634 |
| VIII-29 | 635 |
| VIII-30 | 636 |
| VIII-31 | 637 |
| VIII-32 | 638 |
| VIII-33 | 639 |
| VIII-34 | 1204 |
| VIII-38 | 643 |
| VIII-40 | 644 |

(continued)

| List of sequences of probes informative for breast cancer | |
|---|---|
| Please see the note at the bottom of Table 1. Some sequences are missing. | |
| Clone ID | Sequence ID |
| VIII-41 | 645 |
| VIII-46 | 649 |
| VIII-48 | 651 |
| VIII-55 | 656 |
| VIII-57 | 658 |
| VIII-59 | 660 |
| VIII-60 | 661 |
| VIII-61 | 1205 |
| VIII-64 | 663 |
| VIII-66 | 665 |
| VIII-73 | 672 |
| VIII-74 | 673 |
| VIII-76 | 675 |
| VIII-80 | 679 |
| X-07 | 808 |
| X-15 | 814 |
| X-20 | 817 |
| X-29 | 821 |
| X-34 | 825 |
| X-46 | 833 |
| X-54 | 837 |
| X-56 | 839 |
| X-68 | 1207 |
| X-72 | 849 |
| X-73 | 1208 |
| X-94 | 860 |
| XI-13 | 1209 |
| XI-37 | 1460 |
| XI-43 | 1210 |
| XI-67 | 1211 |
| XI-81 | 1212 |
| XII-07 | 1213 |
| XII-35 | 1214 |
| XII-36 | 1215 |
| XII-59 | 1216 |
| XII-65 | 1028 |

(continued)

| List of sequences of probes informative for breast cancer<br>Please see the note at the bottom of Table 1. Some sequences are missing. | |
|---|---|
| Clone ID | Sequence ID |
| XII-92 | 1217 |
| XIII-03 | 917 |
| XIII-04 | 1218 |
| XIII-19 | 1219 |
| XIII-24 | 926 |
| XIII-51 | 938 |
| XIII-52 | 939 |
| XIII-67 | 947 |
| XIII-69 | 949 |
| XIII-88 | 1220 |
| XIII-92 | 1221 |
| XV-22 | 1099 |
| XV-24 | 1101 |
| XV-25 | 1224 |
| XV-42 | 1108 |
| XV-62 | 1226 |
| XV-64 | 1118 |
| XV-84 | 1125 |
| XVI-19 | 1228 |
| XVI-36 | 1056 |
| XVI-53 | 1230 |
| XVI-60 | 1071 |
| XVI-66 | 1074 |
| XVI-74 | 1081 |
| XVI-76 | 1083 |
| XVI-77 | 1084 |
| XVII-31 | 1139 |
| XVII-40 | 1231 |
| XVII-48 | 1148 |
| XVII-76 | 1160 |
| XVII-87 | 1165 |
| XVII-95 | 1172 |

Table 3

| Occurrence* | Clone ID |
|---|---|
| List of informative probes (Clone ID) sel ected for breast cancer diagnosis based on their occurrence criterion during variable selection | |
| 100% | XI-8,XVI-66,VIII-66,XVI-59,VII-03,XIII-19,XII-35,X-35,XI-50,XII-26,IV-53,XIII-29,XIII-62,I-30,III-06,XV-22,XV-94,VII-15,VII-39,IX-39,XVB-39,III-40,VII-32 |
| 90% | I-52,VI-65,VI-34,IV-62,XV-34,XVII-58,V-11,VI-78,XII-36,XIII-92,VIII-29,XVI-53,XVI-77,XI-13,XIII-84,IV-14,XII-31,V-80,VII-48,XVII-29,XVII-72 |
| 80% | III-60,VIII-74,IX-12,X-04,XIII-52,VIII-30,IX-38 |
| 70% | VI-49,X-29,VIII-48 |
| 60% | IV-82,IX-10,VI-52,X-68,VII-77 |
| 50% | IV-15 |
| 40% | XV-28,II-70,V-55 |
| 30% | XVII-17,XVIII-67 |
| 20% | XI-58,XVI-36,VIII-39,VIII-44,III-61,IV-69,XV-68,X-72 |
| 10% | IX-42,IX-77,X-94,XV-96,XVII-55 |
| 5% | XII-59,XVI-76,I-54,XV-18,V-94,X-54,VI-07,VII-47,XVII-31,XVII-87,XVII-48 |
| In at least one model | II-41,VI-41,III-57,III-89,VII-73,XV-25,IV-26,X-34,IV-41,VII-90,XV-42,XVII-82,XII-27,VIII-120,I-28,VII-60,VIII-76,III-20,VI-84,XI-07,XVII-28,XII-17,XVII-36,XII-52 , XVII-76,VIII-46,VI-70,XV-74,XV-93,VIII-31,II-87,V-39 , VT-55,X-07,X-15,XII-07,XVII-07,XVII-08,XVII-95,I-24,IV-32,V-32,VI-48,VI-72,IV-80,IX-48,X-56,XV-24,XII-32,XYII-40 |
| *100% = Genes appearing in all the 75 cross validated models; 90% = Additional genes appearing in at least 68 out of 75 cross validated models; 5% = Additional genes appearing in at least 4 out of 75 cross validated models and so on. | |

Table 4a

| List of informative probes for diagnosis of Alzheimer disease | | | | |
|---|---|---|---|---|
| Clone ID | Sequence ID | | Clone ID | Sequence ID |
| I-01 | - | | III-60 | - |
| 1-02 | - | | III-83 | 509 |
| I-13 | - | | III-68 | - |
| I-21 | - | | III-74 | 518 |
| I-34 | 313 | | III-80 | 523 |
| I-37 | - | | III-82 | - |
| I-42 | - | | III-85 | 526 |
| I-58 | 326 | | III-92 | - |
| I-71 | - | | III-96 | - |
| I-72 | - | | IV-23 | - |
| I-86 | - | | IV-26 | - |

(continued)

| List of informative probes for diagnosis of Alzheimer disease | | | | |
|---|---|---|---|---|
| Clone ID | Sequence ID | | Clone ID | Sequence ID |
| I-95 | - | | IV-29 | - |
| II-03 | 361 | | IV-31 | 687 |
| II-05 | 363 | | IV-34 | - |
| II-06 | 364 | | IV-35 | - |
| II-10 | 368 | | IV-45 | - |
| II-24 | 381 | | IV-80 | 701 |
| II-25 | 382 | | IV-82 | - |
| II-26 | 383 | | IV-93 | - |
| II-33 | 390 | | V-01 | - |
| II-34 | 391 | | V-02 | - |
| II-42 | 398 | | V-03 | 706 |
| II-47 | - | | V-04 | 707 |
| II-67 | 411 | | V-06 | - |
| II-61 | 415 | | V-07 | 708 |
| II-69 | 423 | | V-12 | 711 |
| II-75 | 429 | | V-15 | - |
| II-83 | - | | V-17 | - |
| II-84 | 438 | | V-21 | - |
| II-88 | 442 | | V-25 | - |
| II-90 | - | | V-35 | - |
| II-84 | 448 | | V-42 | - |
| III-02 | 453 | | V-43 | - |
| III-05 | - | | V-47 | - |
| III-06 | 458 | | V-49 | - |
| III-08 | 460 | | V-52 | - |
| III-10 | - | | V-54 | - |
| III-13 | 464 | | V-58 | - |
| III-15 | - | | V-59 | - |
| III-17 | - | | V-65 | - |
| III-23 | 473 | | V-68 | - |
| III-26 | 476 | | V-71 | - |
| III-35 | 485 | | V-75 | - |
| III-39 | 487 | | V-79 | - |
| III-43 | 490 | | V-80 | 726 |
| III-44 | 491 | | V-90 | - |
| III-53 | 500 | | V-91 | - |
| III-56 | 603 | | V-92 | - |

(continued)

| List of informative probes for diagnosis of Alzheimer disease | | | | |
|---|---|---|---|---|
| **Clone ID** | **Sequence ID** | | **Clone ID** | **Sequence ID** |
| VI-02 | - | | VII-91 | 613 |
| VI-04 | 865 | | VII-93 | 615 |
| VI-09 | - | | VIII-01 | - |
| VI-10 | - | | VIII-02 | - |
| VI-12 | 869 | | VIII-03 | - |
| VI-14 | 871 | | VIII-08 | - |
| VI-17 | - | | VIII-09 | 618 |
| VI-20 | 876 | | VIII-10 | - |
| VI-21 | - | | VIII-15 | - |
| VI-23 | 876 | | VIII-22 | - |
| VI-41 | - | | VIII-26 | - |
| VI-42 | - | | VIII-28 | 634 |
| VI-43 | - | | VIII-30 | 636 |
| VI-44 | - | | VIII-32 | 638 |
| VI-48 | 891 | | VIII-33 | 639 |
| VI-49 | - | | VIII-41 | 645 |
| VI-50 | 893 | | VIII-42 | 646 |
| VI-53 | 895 | | VIII-48 | 651 |
| VI-71 | - | | VIII-68 | - |
| VI-74 | 905 | | VIII-64 | 663 |
| VI-76 | 907 | | VIII-65 | - |
| VI-78 | - | | VIII-67 | 666 |
| VI-79 | - | | VIII-78 | - |
| VI-87 | 911 | | VIII-62 | - |
| VI-88 | 912 | | VIII-83 | - |
| VI-90 | - | | VIII-85 | - |
| VI-93 | - | | VIII-87 | - |
| VI-95 | 916 | | VIII-91 | - |
| VI-96 | - | | VIII-92 | - |
| VII-02 | - | | VIII-93 | - |
| VII-03 | - | | VIII-95 | - |

(continued)

| List of informative probes for diagnosis of Alzheimer disease | | | | |
| --- | --- | --- | --- | --- |
| **Clone ID** | **Sequence ID** | | **Clone ID** | **Sequence ID** |
| VII-06 | - | | | |
| VII-10 | - | | | |
| VII-11 | - | | | |
| VII-19 | 562 | | | |
| VII-21 | 664 | | | |
| VII-25 | - | | | |
| VII-36 | 575 | | | |
| VII-42 | 579 | | | |
| VII-43 | 580 | | | |
| VII-46 | 583 | | | |
| VII-59 | 593 | | | |
| VII-63 | 595 | | | |
| VII-66 | 598 | | | |
| VII-67 | - | | | |
| VII-72 | 600 | | | |
| VII-73 | 601 | | | |
| VII-75 | - | | | |
| VI-02 | - | | | |
| VI-04 | 866 | | | |
| VI-09 | - | | | |
| VI-10 | - | | | |
| VI-12 | 873 | | | |
| VI-14 | 975 | | | |
| VI-17 | - | | | |

**Table 4b**

| List of sequences of probes informative for Alzheimer disease Please see note to Table 1 | |
| --- | --- |
| **Clone ID** | **Sequence ID** |
| I-09 | 298 |
| I-10 | 299 |
| I-15 | 300 |
| I-16 | 301 |
| I-17 | 302 |
| I-19 | 304 |
| I-20 | 305 |
| I-22 | 306 |

(continued)

| List of sequences of probes informative for Alzheimer disease Please see note to Table 1 | |
|:---:|:---:|
| **Clone ID** | **Sequence ID** |
| 1-23 | 307 |
| I-24 | 308 |
| I-25 | 309 |
| I-28 | 310 |
| I-31 | 311 |
| I-32 | 312 |
| I-34 | 313 |
| I-38 | 314 |
| I-39 | 315 |
| I-40 | 316 |
| I-44 | 317 |
| I-45 | 318 |
| I-46 | 319 |
| I-47 | 320 |
| I-48 | 321 |
| I-49 | 322 |
| I-53 | 323 |
| I-56 | 324 |
| I-57 | 325 |
| I-58 | 326 |
| I-60 | 327 |
| I-64 | 328 |
| I-67 | 330 |
| I-69 | 331 |
| I-71 | 332 |
| I-72 | 333 |
| I-73 | 334 |
| I-77 | 335 |
| I-79 | 336 |
| I-80 | 337 |
| I-81 | 338 |
| I-82 | 339 |
| VI-02 | 340 |
| VI-03 | 341 |
| VI-04 | 342 |
| VI-06 | 343 |

(continued)

| List of sequences of probes informative for Alzheimer disease<br>Please see note to Table 1 | |
| --- | --- |
| **Clone ID** | **Sequence ID** |
| VI-07 | 344 |
| VI-08 | 345 |
| VI-09 | 346 |
| VI-11 | 347 |
| VI-18 | 348 |
| VI-19 | 349 |
| VI-20 | 350 |
| VI-21 | 351 |
| VI-22 | 352 |
| VI-25 | 353 |
| VI-26 | 354 |
| VI-27 | 355 |
| VI-31 | 356 |
| VI-33 | 357 |
| VI-35 | 358 |
| VI-48 | 359 |
| II-02 | 360 |
| II-03 | 361 |
| 11-05 | 363 |
| II-06 | 364 |
| II-07 | 365 |
| 11-08 | 366 |
| II-09 | 367 |
| II-10 | 368 |
| II-11 | 369 |
| II-12 | 370 |
| II-13 | 371 |
| II-14 | 372 |
| II-15 | 373 |
| II-16 | 374 |
| II-17 | 375 |
| II-18 | 376 |
| II-20 | 377 |
| II-21 | 378 |
| II-22 | 379 |
| II-23 | 380 |

(continued)

| List of sequences of probes informative for Alzheimer disease<br>Please see note to Table 1 | |
|:---:|:---:|
| **Clone ID** | **Sequence ID** |
| II-24 | 381 |
| II-25 | 382 |
| II-26 | 383 |
| II-27 | 384 |
| II-28 | 385 |
| II-29 | 386 |
| II-30 | 387 |
| II-31 | 388 |
| II-32 | 389 |
| II-33 | 390 |
| II-34 | 391 |
| II-35 | 392 |
| II-37 | 393 |
| II-38 | 394 |
| II-39 | 395 |
| II-40 | 396 |
| II-41 | 397 |
| II-42 | 398 |
| II-43 | 399 |
| II-44 | 400 |
| II-46 | 401 |
| II-47 | 402 |
| II-48 | 403 |
| II-49 | 404 |
| II-50 | 405 |
| II-52 | 406 |
| II-53 | 407 |
| II-54 | 408 |
| II-55 | 409 |
| II-56 | 410 |
| II-57 | 411 |
| II-58 | 412 |
| II-59 | 413 |
| II-60 | 414 |
| II-61 | 415 |
| II-62 | 416 |

(continued)

(continued)

| List of sequences of probes informative for Alzheimer disease Please see note to Table 1 | |
|---|---|
| **Clone ID** | **Sequence ID** |
| II-63 | 417 |
| II-64 | 418 |
| II-65 | 419 |
| II-66 | 420 |
| II-67 | 421 |
| II-68 | 422 |
| II-69 | 423 |
| II-70 | 424 |
| II-71 | 425 |
| 11-72 | 426 |
| II-73 | 427 |
| II-74 | 428 |
| II-75 | 429 |
| II-76 | 430 |
| II-77 | 431 |
| II-78 | 432 |
| II-79 | 433 |
| II-80 | 434 |
| II-81 | 435 |
| II-82 | 436 |
| II-83 | 437 |
| II-84 | 438 |
| II-85 | 439 |
| II-86 | 440 |
| II-87 | 441 |
| II-88 | 442 |
| II-89 | 443 |
| II-90 | 444 |
| II-91 | 445 |
| II-92 | 446 |
| II-93 | 447 |
| II-94 | 448 |
| II-95 | 449 |
| II-96 | 450 |
| III-01 | 452 |
| III-02 | 453 |

(continued)

| List of sequences of probes informative for Alzheimer disease Please see note to Table 1 | |
|---|---|
| **Clone ID** | **Sequence ID** |
| III-03 | 454 |
| III-04 | 455 |
| III-05 | 457 |
| III-06 | 458 |
| III-07 | 459 |
| III-08 | 460 |
| III-09 | 461 |
| III-11 | 462 |
| III-12 | 463 |
| III-13 | 464 |
| III-14 | 465 |
| III-15 | 466 |
| III-16 | 467 |
| III-17 | 468 |
| III-18 | 469 |
| III-19 | 470 |
| III-21 | 471 |
| III-22 | 472 |
| III-23 | 473 |
| III-24 | 474 |
| III-25 | 475 |
| III-26 | 476 |
| III-27 | 477 |
| III-28 | 478 |
| III-29 | 479 |
| III-31 | 481 |
| III-32 | 482 |
| III-33 | 483 |
| III-34 | 484 |
| III-35 | 485 |
| III-37 | 486 |
| III-39 | 487 |
| III-40 | 488 |
| III-42 | 489 |
| III-43 | 490 |
| III-44 | 491 |

(continued)

| List of sequences of probes informative for Alzheimer disease Please see note to Table 1 | |
|---|---|
| **Clone ID** | **Sequence ID** |
| III-45 | 492 |
| III-46 | 493 |
| III-47 | 494 |
| III-48 | 495 |
| III-49 | 496 |
| III-50 | 497 |
| III-51 | 498 |
| III-52 | 499 |
| III-53 | 500 |
| III-54 | 501 |
| III-55 | 502 |
| III-56 | 503 |
| III-57 | 504 |
| III-58 | 505 |
| III-59 | 506 |
| III-61 | 507 |
| III-62 | 508 |
| III-63 | 509 |
| III-64 | 510 |
| III-65 | 511 |
| III-66 | 512 |
| III-67 | 513 |
| III-69 | 514 |
| III-70 | 515 |
| III-71 | 516 |
| III-73 | 517 |
| III-74 | 518 |
| III-75 | 519 |
| III-77 | 520 |
| III-78 | 521 |
| III-79 | 522 |
| III-80 | 523 |
| III-81 | 524 |
| III-83 | 525 |
| III-85 | 526 |
| III-86 | 527 |

(continued)

| List of sequences of probes informative for Alzheimer disease Please see note to Table 1 | |
| --- | --- |
| **Clone ID** | **Sequence ID** |
| III-87 | 528 |
| III-88 | 529 |
| III-89 | 530 |
| III-91 | 531 |
| III-93 | 532 |
| III-94 | 533 |
| III-95 | 534 |
| III-96 | 535 |
| VII-02 | 547 |
| VII-03 | 548 |
| VII-04 | 549 |
| VII-05 | 550 |
| VII-06 | 551 |
| VII-07 | 552 |
| VII-08 | 553 |
| VII-09 | 554 |
| VII-10 | 555 |
| VII-11 | 556 |
| VII-12 | 557 |
| VII-14 | 558 |
| VII-15 | 559 |
| VII-17 | 560 |
| VII-18 | 561 |
| VII-19 | 562 |
| VII-20 | 563 |
| VII-21 | 564 |
| VII-22 | 565 |
| VII-23 | 566 |
| VII-24 | 567 |
| VII-27 | 568 |
| VII-28 | 569 |
| VII-29 | 570 |
| VII-32 | 571 |
| VII-33 | 572 |
| VII-34 | 573 |
| VII-35 | 574 |

(continued)

| List of sequences of probes informative for Alzheimer disease<br>Please see note to Table 1 | |
| --- | --- |
| **Clone ID** | **Sequence ID** |
| VII-36 | 575 |
| VII-39 | 576 |
| VII-40 | 577 |
| VII-41 | 578 |
| VII-42 | 579 |
| VII-43 | 580 |
| VII-44 | 581 |
| VII-45 | 582 |
| VII-46 | 583 |
| VII-48 | 584 |
| VII-49 | 585 |
| VII-50 | 586 |
| VII-52 | 587 |
| VII-53 | 588 |
| VII-54 | 589 |
| VII-55 | 590 |
| VII-57 | 591 |
| VII-58 | 592 |
| VII-59 | 593 |
| VII-62 | 594 |
| VII-63 | 595 |
| VII-64 | 596 |
| VII-65 | 597 |
| VII-66 | 598 |
| VII-71 | 599 |
| VII-72 | 600 |
| VII-73 | 601 |
| VII-74 | 602 |
| VII-76 | 603 |
| VII-77 | 604 |
| VII-80 | 605 |
| VII-81 | 606 |
| VII-82 | 607 |
| VII-83 | 608 |
| VII-84 | 609 |
| VII-87 | 610 |

(continued)

| List of sequences of probes informative for Alzheimer disease<br>Please see note to Table 1 | |
|---|---|
| **Clone ID** | **Sequence ID** |
| VII-89 | 611 |
| VII-90 | 612 |
| VII-91 | 613 |
| VII-92 | 614 |
| VII-93 | 615 |
| VII-94 | 616 |
| VII-96 | 617 |
| VIII-09 | 618 |
| VIII-10 | 619 |
| VIII-11 | 620 |
| VIII-12 | 621 |
| VIII-13 | 622 |
| VIII-15 | 623 |
| VIII-16 | 624 |
| VIII-17 | 625 |
| VIII-18 | 626 |
| VIII-19 | 627 |
| VIII-20 | 628 |
| VIII-21 | 629 |
| VIII-23 | 630 |
| VIII-24 | 631 |
| VIII-25 | 632 |
| VIII-28 | 634 |
| VIII-29 | 635 |
| VIII-30 | 636 |
| VIII-31 | 637 |
| VIII-32 | 638 |
| VIII-33 | 639 |
| VIII-34 | 640 |
| VIII-36 | 641 |
| VIII-37 | 642 |
| VIII-38 | 643 |
| VIII-40 | 644 |
| VIII-41 | 645 |
| VIII-42 | 646 |
| VIII-43 | 647 |

(continued)

| List of sequences of probes informative for Alzheimer disease<br>Please see note to Table 1 | |
| --- | --- |
| **Clone ID** | **Sequence ID** |
| VIII-45 | 648 |
| VIII-46 | 649 |
| VIII-47 | 650 |
| VIII-48 | 651 |
| VIII-50 | 652 |
| VIII-51 | 653 |
| VIII-53 | 654 |
| VIII-54 | 655 |
| VIII-55 | 656 |
| VIII-56 | 657 |
| VIII-57 | 658 |
| VIII-58 | 659 |
| VIII-59 | 660 |
| VIII-60 | 661 |
| VIII-61 | 662 |
| VIII-64 | 663 |
| VIII-65 | 664 |
| VIII-66 | 665 |
| VIII-67 | 666 |
| VIII-68 | 667 |
| VIII-69 | 668 |
| VIII-70 | 669 |
| VIII-71 | 670 |
| VIII-72 | 671 |
| VIII-73 | 672 |
| VIII-74 | 673 |
| VIII-75 | 674 |
| VIII-76 | 675 |
| VIII-77 | 676 |
| VIII-78 | 677 |
| VIII-79 | 678 |
| VIII-80 | 679 |
| IV-02 | 681 |
| IV-04 | 682 |
| IV-13 | 683 |
| IV-14 | 684 |

(continued)

| List of sequences of probes informative for Alzheimer disease Please see note to Table 1 | |
|---|---|
| **Clone ID** | **Sequence ID** |
| IV-17 | 685 |
| IV-28 | 686 |
| IV-31 | 687 |
| IV-32 | 688 |
| IV-38 | 689 |
| IV-40 | 690 |
| IV-42 | 691 |
| IV-44 | 692 |
| IV-47 | 693 |
| IV-55 | 694 |
| IV-56 | 695 |
| IV-61 | 696 |
| IV-64 | 697 |
| IV-65 | 698 |
| IV-72 | 699 |
| IV-73 | 700 |
| IV-80 | 701 |
| IV-85 | 702 |
| IV-93 | 703 |
| IV-95 | 704 |
| IV-96 | 705 |
| V-03 | 706 |
| V-04 | 707 |
| IV-07 | 708 |
| V-08 | 709 |
| V-09 | 710 |
| V-12 | 711 |
| V-18 | 712 |
| V-20 | 713 |
| V-24 | 714 |
| V-37 | 716 |
| V-40 | 717 |
| V-41 | 718 |
| V-48 | 719 |
| V-57 | 720 |
| V-61 | 721 |

(continued)

(continued)

| List of sequences of probes informative for Alzheimer disease<br>Please see note to Table 1 | |
| --- | --- |
| Clone ID | Sequence ID |
| V-64 | 722 |
| V-65 | 723 |
| V-74 | 724 |
| V-80 | 726 |
| V-81 | 727 |
| V-87 | 728 |
| | |
| VI-13 | 870 |
| IV-14 | 871 |
| VI-16 | 873 |
| VI-23 | 878 |
| VI-24 | 879 |
| VI-28 | 883 |
| VI-32 | 885 |
| VI-38 | 886 |
| VI-39 | 887 |
| VI-45 | 889 |
| VI-46 | 890 |
| VI-49 | 892 |
| VI-50 | 893 |
| VI-52 | 894 |
| VI-53 | 895 |
| VI-54 | 896 |
| VI-55 | 897 |
| VI-57 | 898 |
| VI-58 | 899 |
| VI-63 | 900 |
| VI-65 | 902 |
| VI-66 | 903 |
| VI-67 | 904 |
| VI-74 | 905 |
| VI-75 | 906 |
| VI-76 | 907 |
| VI-80 | 908 |
| VI-81 | 909 |
| VI-85 | 910 |

(continued)

| List of sequences of probes informative for Alzheimer disease<br>Please see note to Table 1 | |
| --- | --- |
| **Clone ID** | **Sequence ID** |
| VI-87 | 911 |
| VI-88 | 912 |
| VI-91 | 913 |
| VI-94 | 914 |
| VI-95 | 915 |
| VI-96 | 916 |
| I-13 | 1177 |
| I-14 | 1178 |
| I-30 | 1180 |
| I-54 | 1181 |
| I-88 | 1182 |
| III-20 | 1183 |
| IV-15 | 1185 |
| IV-26 | 1186 |
| IV-62 | 1187 |
| V-11 | 1188 |
| V-28 | 1189 |
| V-38 | 1190 |
| V-45 | 1191 |
| VI-44 | 1193 |
| VII-47 | 1200 |
| I-42 | 1332 |
| I-52 | 1333 |
| I-86 | 1336 |
| I-95 | 1337 |
| III-10 | 1342 |
| III-60 | 1347 |
| III-82 | 1348 |
| III-92 | 1351 |
| IV-23 | 1353 |
| IV-34 | 1354 |
| IV-35 | 1355 |
| IV-41 | 1356 |
| IV-45 | 1357 |
| IV-82 | 1359 |
| V-01 | 1361 |

(continued)

| List of sequences of probes informative for Alzheimer disease Please see note to Table 1 | |
|---|---|
| **Clone ID** | **Sequence ID** |
| V-02 | 1362 |
| V-06 | 1363 |
| V-17 | 1364 |
| V-25 | 1365 |
| V-35 | 1366 |
| V-42 | 1367 |
| V-47 | 1368 |
| V-49 | 1369 |
| V-58 | 1370 |
| V-75 | 1372 |
| V-79 | 1373 |
| V-90 | 1374 |
| V-91 | 1375 |
| V-94 | 1376 |
| VI-10 | 1379 |
| VI-41 | 1381 |
| VI-43 | 1382 |
| VI-71 | 1387 |
| VI-72 | 1388 |
| VI-79 | 1389 |
| VI-90 | 1390 |
| VI-93 | 1391 |
| VII-25 | 1397 |
| VII-60 | 1398 |
| VII-67 | 1399 |
| VIII-22 | 1403 |
| VIII-26 | 1404 |
| VIII-39 | 1405 |
| VIII-44 | 1406 |
| I-37 | 1440 |
| V-32 | 1445 |
| V-52 | 1447 |
| V-68 | 1448 |
| V-92 | 1449 |
| VI-42 | 1450 |
| VI-78 | 1452 |

(continued)

(continued)

| List of sequences of probes informative for Alzheimer disease<br>Please see note to Table 1 | |
|---|---|
| **Clone ID** | **Sequence ID** |
| VII-86 | 1453 |
| VII-88 | 1454 |
| IV-29 | 1490 |
| V-15 | 1491 |
| V-39 | 1492 |
| V-54 | 1493 |
| V-59 | 1494 |
| V-71 | 1495 |

Table 5

**Samples**

| Diagnosis | No. of women |
|---|---|
| Normal /Benign | 42* |
| DCIS | 3 |
| Invasive cancer | 26 |

\* From one woman, whole blood was collected at weeks 1,2,3,4,5 following menstruation. Hence, the number of unique normal/benign samples tested in the experiment is 75

Information about women with breast cancer

| Sample | AGE | Stage | Cancer type | Size hist. (mm) | Nodes |
|---|---|---|---|---|---|
| 1 | 51 | II | IDC | 20 | 1/7 |
| 2 | 84 | II | IDC | 22 | 2/2 |
| 3 | 50 | I | DCIS+ 1 IDC | >50 DCIS; 5 x 14 | 0/7 |
| 4 | 47 | I | IDC | 15 | 0 |
| 5 | 69 | III | ILC g.2 + tubular adenocarcinoma | 50 + 3 | 1 av 12 + 1 av 7 |
| 6 | 50 | II | IDC | 24 | 0 |
| 7 | 65 | I | IDC | 15 | 0 |
| 8 | 63 | II | IDC | 23 | 0 |
| 9 | 55 | I | IDC + DCIS | 4 | 0 av 1 |
| 10 | 52 | 0 | DCIS + small colloid carcinoma foci | 50 + 3 | 0 |
| 11 | 60 | II | IDC | 24 | 0 |
| 12 | 54 | I | IDC | 11 | 0 |
| 13 | | 0 | DCIS | 20 | 0 |
| 14 | 49 | 0 | DCIS | 9 | 0 |
| 15 | 48 | I | IDC | 4 | 0 |
| 16 | 56 | I | IDC | 4 | 0 |
| 17 | 68 | I | IDC | 14 | 0 |
| 18 | 68 | I | IDC | 7 | 0 |
| 19 | 63 | I | IDC | 10 | 0 |
| 20 | 45 | I | IDC | 19 | 1 |
| 21 | 57 | III | IDC | 60 | 8/20 |
| 22 | 55 | II | IDC/DCIS | 35 +55 | 0 |
| 23 | 71 | I | IDC/extensive DCIS | 8 | 0 |
| 24 | 56 | I | IDC | 9 | ? |

| 25 | 66 | II | IDC | 26 | 0 |
| 26 | 66 | I | IDC | 15 | ? |
| 27 | 61 | I | IDC | 9 | ? |
| 28 | ? | ? | ? | ? | ? |
| 29 | 65 | I | IDC | 11 | 0 |

Other diseases /conditions present in the women tested

*Other diseases /conditions present in the women tested*

| Disease/condition |
| --- |
| Diabetes |
| Asthma |
| Ulcerous colitis |
| Hemochromatose |
| Crohn's disease |
| Fibromyalgia |
| Psoraiasis |
| Atopic eczema |
| Rheumatism |
| Allergies |

*Prior history of cancer in the women tested*

| Cancer type | No. of women |
| --- | --- |
| Breast | 3 |
| Colon | 2 |
| Stomach | 1 |
| Skin | 1 |

# Table 6

Number of samples tested by double cross validation and success of the diagnostic test for breast cancer based on selected informative genes

Number of samples tested by double cross validation

| Number of unique samples tested | 75 |
|---|---|
| Number of unique non cancer samples tested | 46 |
| Number of cancer samples tested | 29 |

Success of the diagnostic test for breast cancer based on selected informative genes

| Occurrence in percentage[*] | Number of informative probes | Specificity | Sensitivity | Accuracy | False Positive rate | False negative rate | Total error rate |
|---|---|---|---|---|---|---|---|
| 100.00 | 23 | 84.78 | 75.86 | 81.33 | 15.22 | 24.14 | 18.67 |
| 90.00 | 44 | 91.30 | 79.31 | 86.67 | 8.70 | 20.69 | 13.33 |
| 80.00 | 51 | 86.96 | 79.31 | 84.00 | 13.04 | 20.69 | 16.00 |
| 70.00 | 54 | 89.13 | 75.86 | 84.00 | 10.87 | 24.14 | 16.00 |
| 60.00 | 58 | 89.13 | 75.86 | 84.00 | 10.87 | 24.14 | 16.00 |
| 50.00 | 59 | 89.13 | 75.86 | 84.00 | 10.87 | 24.14 | 16.00 |
| 40.00 | 63 | 89.13 | 75.86 | 84.00 | 10.87 | 24.14 | 16.00 |
| 30.00 | 66 | 86.96 | 75.86 | 82.67 | 13.04 | 24.14 | 17.33 |
| 20.00 | 74 | 89.13 | 75.86 | 84.00 | 10.87 | 24.14 | 16.00 |
| 10.00 | 79 | 89.13 | 75.86 | 84.00 | 10.87 | 24.14 | 16.00 |
| 5.00 | 90 | 86.96 | 79.31 | 84.00 | 13.04 | 20.69 | 16.00 |
| 1.33 | 139 | 84.78 | 72.41 | 80.00 | 15.22 | 27.59 | 20.00 |

[*]100% = Genes appearing in all the 75 cross validated models; 90% = Genes appearing in at least 68 out of 75 cross validated models; 5% = Genes appearing in at least 4 out of 75 cross validated models; and so on.

# Table 7

Double cross-validation and details of the success of the diagnostic test for Alzheimer disease
based on the expression 182 informative genes

## Validation Result

| | |
|---|---|
| Total number of samples tested | 14 |
| Number of Alzheimer's disease samples tested | 7 |
| Number of Alzheimer,s disease samples incorrectly predicted | 1 |
| Number of non_Alzheimer's disease samples tested | 7 |
| Number of non-Alzheimer's disease samples incorrectly predicted | 0 |

## Success of diagnostic test

| Performance | Description | % |
|---|---|---|
| Accuracy | Percentage of the total number of predictions that were correct | 92.9 |
| Sensitivity | Percentage of positive cases that were correctly identified | 85.7 |
| Specificity | Percentage of negatives cases that were correctly predicted | 100 |
| False positive rate | Percentage of negatives cases that were incorrectly classified as positive | 0.0 |
| False negative rate | Percentage of positives cases that were incorrectly classified as negative | 14.3 |
| Total error rate | Percentage of the total cases incorrectly predicted | 7.1 |

## Table 8

**Some relevant features of the blood donors.** B, Female donors with breast cancer N, Female donors with suspected mammogram but no breast cancer; IDC, invasive ductal carcinoma; DCIS, ductal carcinoma in situ; na, not available nd, not determined; ++, no degradation of mRNA and no ribosomal contamination in the sample, +, no degradation of mRNA but ribosomal contamination in the sample.

| | | AGE | Cancer type /breast abnormality | Size Hist. (mm) | mRNA Quality |
|---|---|---|---|---|---|
| 1 | B1 | na | IDC | 5 | ++ |
| 2 | B2 | 49 | DCIS | 8 | nd |
| 3 | B3 | 54 | IDC | 18 | ++ |
| 4 | B4 | 59 | IDC | 12 | + |
| 5 | B5 | 61 | DCIS+micro invasive cancer | 15+1.5 | ++ |
| 6 | B6 | 55 | IDC | 12+17 | nd |
| 7 | B6 | | IDC | 12+17 | nd |
| 8 | N1 | 45 | Fibroadenoma | - | nd |
| 9 | N2 | 52 | na | - | + |

(continued)

| | | | Cancer type /breast abnormality | Size Hist. (mm) | mRNA Quality |
|---|---|---|---|---|---|
| | | *AGE* | | | |
| 10 | N3 | 55 | Cyst | - | ++ |
| 11 | N4 | 54 | na | - | ++ |
| 12 | N5 | 51 | Benign ductal epitelhelium | - | nd |
| 13 | N6 | 57 | Benign | - | nd |
| 14 | N7 | 50 | na | - | ++ |
| 15 | N8 | 52 | na | - | + |

Some relevant features of the blood donors. B, Female donors with breast cancer N, Female donors with suspected mammogram but no breast cancer; IDC, invasive ductal carcinoma; DCIS, ductal carcinoma in situ; na, not available nd, not determined; ++, no degradation of mRNA and no ribosomal contamination in the sample, +, no degradation of mRNA but ribosomal contamination in the sample.

**Table 9**

| List of sequence of probes informative for both alzheimer and breast cancer disease | |
|---|---|
| **Clone ID** | **Sequence ID** |
| I-24 | 308 |
| I-25 | 309 |
| I-28 | 310 |
| I-48 | 321 |
| I-60 | 327 |
| I-72 | 333 |
| I-81 | 338 |
| 1-82 | 339 |
| II-02 | 360 |
| II-03 | 361 |
| II-06 | 364 |
| II-07 | 365 |
| II-10 | 368 |
| II-21 | 378 |
| II-23 | 380 |
| II-24 | 381 |
| II-25 | 382 |
| II-27 | 384 |
| II-33 | 390 |
| II-34 | 391 |
| II-41 | 397 |
| II-42 | 398 |

(continued)

| List of sequence of probes informative for both alzheimer and breast cancer disease | |
| --- | --- |
| **Clone ID** | **Sequence ID** |
| II-46 | 401 |
| II-47 | 402 |
| II-48 | 403 |
| II-52 | 406 |
| II-57 | 411 |
| II-58 | 412 |
| II-59 | 413 |
| II-60 | 414 |
| II-61 | 415 |
| II-62 | 416 |
| II-64 | 418 |
| 11-67 | 421 |
| II-69 | 423 |
| II-70 | 424 |
| 11-74 | 428 |
| II-80 | 434 |
| II-82 | 436 |
| II-84 | 438 |
| II-87 | 441 |
| II-88 | 442 |
| II-96 | 450 |
| III-01 | 452 |
| III-02 | 453 |
| III-06 | 458 |
| III-08 | 460 |
| III-12 | 463 |
| III-13 | 464 |
| III-17 | 468 |
| III-18 | 469 |
| III-21 | 471 |
| III-23 | 473 |
| III-24 | 474 |
| III-25 | 475 |
| III-26 | 476 |
| III-27 | 477 |
| III-28 | 478 |
| III-29 | 479 |

(continued)

| List of sequence of probes informative for both alzheimer and breast cancer disease ||
| Clone ID | Sequence ID |
| --- | --- |
| III-32 | 482 |
| III-33 | 483 |
| III-35 | 485 |
| III-39 | 487 |
| III-40 | 488 |
| III-42 | 489 |
| III-45 | 492 |
| III-46 | 493 |
| III-47 | 494 |
| III-48 | 495 |
| III-56 | 503 |
| III-57 | 504 |
| III-58 | 505 |
| III-59 | 506 |
| III-61 | 507 |
| III-62 | 508 |
| III-63 | 509 |
| III-64 | 510 |
| III-66 | 512 |
| III-67 | 513 |
| III-70 | 515 |
| III-74 | 518 |
| III-75 | 519 |
| III-78 | 521 |
| III-80 | 523 |
| III-81 | 524 |
| III-85 | 526 |
| III-86 | 527 |
| III-88 | 529 |
| III-89 | 530 |
| III-93 | 532 |
| III-95 | 534 |
| III-96 | 535 |
| IV-04 | 682 |
| IV-13 | 683 |
| IV-14 | 684 |
| IV-17 | 685 |

(continued)

| List of sequence of probes informative for both alzheimer and breast cancer disease ||
| Clone ID | Sequence ID |
| --- | --- |
| IV-31 | 687 |
| IV-32 | 688 |
| IV-38 | 689 |
| IV-42 | 691 |
| IV-47 | 693 |
| IV-61 | 696 |
| IV-64 | 697 |
| IV-72 | 699 |
| IV-80 | 701 |
| IV-85 | 702 |
| IV-93 | 703 |
| IV-96 | 705 |
| V-03 | 706 |
| V-04 | 707 |
| V-07 | 708 |
| V-08 | 709 |
| V-12 | 711 |
| V-24 | 714 |
| V-41 | 718 |
| V-57 | 720 |
| V-61 | 721 |
| V-64 | 722 |
| V-65 | 723 |
| V-74 | 724 |
| V-80 | 726 |
| VI-03 | 341 |
| VI-04 | 342 |
| VI-07 | 344 |
| VI-08 | 345 |
| VI-09 | 346 |
| VI-12 | 869 |
| VI-14 | 871 |
| VI-19 | 349 |
| VI-20 | 350 |
| VI-21 | 351 |
| VI-23 | 878 |
| VI-25 | 353 |

(continued)

(continued)

| List of sequence of probes informative for both alzheimer and breast cancer disease | |
|---|---|
| **Clone ID** | **Sequence ID** |
| VI-26 | 354 |
| VI-48 | 359 |
| VI-50 | 893 |
| VI-53 | 895 |
| VI-74 | 905 |
| VI-76 | 907 |
| VI-87 | 911 |
| VI-88 | 912 |
| VI-95 | 915 |
| VII-02 | 547 |
| VII-03 | 548 |
| VII-06 | 551 |
| VII-08 | 553 |
| VII-09 | 554 |
| VII-10 | 555 |
| VII-11 | 556 |
| VII-15 | 559 |
| VII-17 | 560 |
| VII-19 | 562 |
| VII-21 | 564 |
| VII-22 | 565 |
| VII-23 | 566 |
| VII-24 | 567 |
| VII-27 | 568 |
| VII-29 | 570 |
| VII-32 | 571 |
| VII-33 | 572 |
| VII-36 | 575 |
| VII-39 | 576 |
| VII-41 | 578 |
| VII-42 | 579 |
| VII-43 | 580 |
| VII-46 | 583 |
| VII-48 | 584 |
| VII-49 | 585 |
| VII-54 | 589 |
| VII-57 | 591 |

(continued)

| List of sequence of probes informative for both alzheimer and breast cancer disease | |
| --- | --- |
| Clone ID | Sequence ID |
| VII-58 | 592 |
| VII-59 | 593 |
| VII-62 | 594 |
| VII-63 | 595 |
| VII-64 | 596 |
| VII-66 | 598 |
| VII-72 | 600 |
| VII-73 | 601 |
| VII-77 | 604 |
| VII-80 | 605 |
| VII-82 | 607 |
| VII-87 | 610 |
| VII-90 | 612 |
| VII-91 | 613 |
| VII-92 | 614 |
| VII-93 | 615 |
| VII-96 | 617 |
| VIII-09 | 618 |
| VIII-10 | 619 |
| VIII-13 | 622 |
| VIII-16 | 624 |
| VIII-20 | 628 |
| VIII-21 | 629 |
| VIII-23 | 630 |
| VIII-24 | 631 |
| VIII-25 | 632 |
| VIII-28 | 634 |
| VIII-29 | 635 |
| VIII-30 | 636 |
| VIII-31 | 637 |
| VIII-32 | 638 |
| VIII-33 | 639 |
| VIII-34 | 640 |
| VIII-38 | 643 |
| VIII-40 | 644 |
| VIII-41 | 645 |
| VIII-46 | 649 |

(continued)

| List of sequence of probes informative for both alzheimer and breast cancer disease | |
|---|---|
| **Clone ID** | **Sequence ID** |
| VIII-48 | 651 |
| VIII-55 | 656 |
| VIII-57 | 658 |
| VIII-59 | 660 |
| VIII-60 | 661 |
| VIII-61 | 662 |
| VIII-64 | 663 |
| VIII-66 | 665 |
| VIII-73 | 672 |
| VIII-74 | 673 |
| VIII-76 | 675 |
| VIII-80 | 679 |

Nucleotide sequences

```
Sequence ID - 93                        nt:      405
GGATCCTGTGGCCCACAGAGCTGCCCCAGCAGACGCTCCGCCCCACCCGGTGATGG
AGCCCCGGGGGGACAATCGTGCCTGGGGAGGAGCAGGGTACAGCCCATTCCCCCAG
CCCTGGCTGACCTGGCCTAGCAGTTTGGCCCTGCTGGCCTTAGCAGGGAGACAGGG
GAGCAAAGAACGCCAAGCCGGAGGCCCGAGGCCAGCCGGCCTCTCGAGAGCCAGAG
CAGCAGTTGAATGTAATGCTGGGGACAGGCATGCTGCCGCCAGTAGGGCGGGGACC
CGGACAGCCAGGTGACTACCAGTCCTGGGGACACACTCACCATAAACACATCCCCA
GGCAGGACAGATCGGGGAAGGGGTGTGTACCAGGCTATGATTTCTCTTGCATTAAA
ATGTATTATTATT
```

Sequence ID - 108                         nt:        550

GGCTTTGACAGAGTGCAAGACGATGACTTGCAAAATGTCGCATCTGGAACGCAACA
TAGANACCATCATCAACACCTTCCACCAATACTCTGTGAAGCTGGGGCACCCAGAC
ACCCTGAACCAGGGGGAATTCAAAGAGCTGGTGCGAAAAGATCTGCAAAATTTTCT
CAAGAAGGAGAATAAGAATGAAAAGGTCATAGAACACATCATGGAGGACCTGGACA
CAAATGCAGACAAGCAGCTGAGCTTCGAGGAGTTCATCATGCTGATGGCGAGGCTA
ACCTGGGCCTCCCACGAGAAGATGCACGAGGGTGACGAGGGCCCTGGCCACCACCA
TAAGCCAGGCCTCGGGGAGGGCACCCCCTAAGACCACAGTGGCCAAGATCACAGTG
GCCACGGCCACGGCCACAGTCATGGTGGCCACGGCCACAGCCACTAATCAGGAGGC
CAGGCCACCCTGCCTNTACCCAACCAGGGCCCCGGGGCCTGTTATGTCAAACTGTC
TTGGCTGTGGGGCTAGGGGCTGGGGCCAAATAAAGTCTCTTTCTCC


Sequence ID 110

ACGAAGACAGACATCTGTGGAATGATTCACATCCTCTCAAGTTAGGAGGATGGAGG
CCTGCTTCATTAAGAAGCTGGGGGTAGGGTGGGGGTGGGGAGAACACTTAACAACA
TGGGGACCAGTCAGGGGAATCCCCTTATTTCTGTTTTGCATATGAGGAACCCTAGA
GCAGCCAGGTGAGGCTCTCTAGTTTAATAAAAATCATGGAAAGACTCTTAATGCAG
ACTCTTCTTAAGTGTTAATAGGGATTTTTTCAGCTTATTTTGGTTGCAGTTTCCAA
TTTTTAAAAATGTTGAGGTAATCTTTCCCACCTTCCCAAACCTAATTCTTGTAGAT
GCATTAGTGTTGAACCAATGCTTTCTCATGTCTCAATTCTTTGTATATGCATTCTT
TTCAGATGTATTAAACAAACAAAAACCCTTC


Sequence ID - 192                         nt:        286
CCGGTAATAGAATAGAAAAGGGAGAGTGTCTTCATGCAATGTGGCATCCTGGATTG
GGTCTCGNNACAAAAACAGGACATTAGTGGGAAAATTGGAAATCTGAAAAAAGTCT


GAATTTTAGTTAATATACCAATTTCAGTCTCTTGGTTTTGACAGATGTACCATGGT
GATGTAAGATGTTGACCTTGGGGTAGGCTGGGTGAAGGGTATACAGGAACTCTTTG
TACTATCTCTGCAACTTCTCTGTAAATCTAGTATCATTCCAAAATAAAAGTTTATT
TAATTT

Sequence ID 250

GTGGAAGTGACATCGTCTTTAAACCCTGCGTGGCAATCCCTGACGCACCGCCGTGA
TGCCCAGGGAAGACAGGGCGACCTGGAAGTCCAACTACTTCCTTAAGATCATCCAA
CTATTGGATGATTATCCGAAATGTTTCATTGTGGGAGCAGACAATGTGGGCTCCAA
GCAGATGCAGCAGATCCGCATGTCCCTTCGCGGGAAGGCTGTGGTGCTGATGGGCA
AGAACACCATGATGCGCAAGGCCATCCGAGGGCACCTGGAAAACAACCCAGCTCTG
GAGAAACTGCTGCCTCATATCCGGGGGAATGTGGGCTTTGTGTTCACCAAGGAGGA
CCTCACTGAGATCAGGGACATGTTGCTGGCCAATAAGGTGCCAGCTGCTGCCCGTG
CTGGTGCCATTGCCCCATGTGAAGTCACTGTGCCAGCCCAGAACACTGGTCTCGGG
CCCGAGAAGACCTCCTTTTTCCAGGCTTTAGGTATCACCACTAAAATCTCCAGGGG
CACCATTGAAATCCTGAGTGATGTGCACTGATCAAGACTGG

Sequence ID 299

CAGCGCAGGGGCTTCTGCTGAGGGGGCAGGCGGAGCTTGAGGAAACCGCAGATAAG
TTTTTTTCTCTTTGAAAGATAGAGATTGNTACAACTACTTAAAAAATATAGTCAAT
AGGTTACTAAGATATTGCTTAGCGTTAAGTTTTTAACGTAATTTTAATAGCTTAAG
ATTTTAAGAGAAAATATGAAGACTTAGAAGAGTAGCATGAGGAAGGAAAAGATAAA
AGGTTTCTAAAACATGACGGAGGTTGAGATGAAGCTTCTTCATGGAGTAAAAAATG
TATTTAAAAGAAAATTGAGAGAAAGGACTACAGAGCCCCGAATTAATACCAATAGA
AGGGCAATGCTTTTAGATTAAAATGAAGGTGACTTAAACAGCTTAAAGTTTAGTTT
AAAAGTTGTAGGTGATTAAAATAATTTGAAGGCGATCTTTTAAAAAGAGATTAAAC
CGAAGGTGATTAAAAGACCTTGAAATCCATGACGCANGGAGAATTGCGCATTTAAA
GCCTAGTTACGCATTTACTAAACGCAGACGAAAATGGGAAGATTAATTGGGAGTGG
TAGGATGAAACAATTTTGGAGAAGATAGAAG

Sequence ID 300

CTCAAAGGAGAAAAAAAACCTTGTAAAAAAAGCAAAAATGACAACAGAAAAACAAT
CTTATTCCGAGCATTCCAGTAACTTTTTTGTGTATGTACTTAGCTGTACTATAAGT
AGTTGGTTTGTATGAGATGGTTAAAAAGGCCAAAGATAAAAGGTTTCTTTTTTTTT
CCTTTTTTGTCTATGAAGTTGCTGTTTATTTTTTTTTGGCCTGTTTGATGTATGTGT
GAAACAATGTTGTCCAACAATAAACAGGAATTTTATTTTGCTGAGTTGTTCTAAAA
AAAAAAAAAAAAAAAAAA

Sequence ID 302

AGTAGAGACGGGGTTTCACTGTGTTAGCCAGGATGGTCTCGATCTCCTGACCTCGT
GATCCGGCCACCTCGGCCTCCCGAAAGTGCTGGGATTACAGGCGTGAGCCACGGCG
CCCAGCCCCAGCCTGTCACTTAAACTGATAAACGACAGATTAACAGTAGAAAAATT
TTATTTTGCATACATAATGAGGCTTCACAAAGAGAAGTGAAAACCCAAGTAGGAG
TTTAGGGCTGGGGGCTTATATACCATTTAACAAGGGGTGATAAATTGTAAGAGAAT
AG


Sequence ID 304

TCCTTGGTTTCGATTTGTGGCAACAATCCAGTCTTTTTGTTTTTTTCAGGGATACC
ATATGTAACAGGTGCCATTGTTACTGTAACTTTTCACACATGCCTTCAGTTTGATG
TCAAAGTCATCATTTAGTGTAAACAGCAAGTTATCTGTTAGGCTGCACATCATGAA
CTTTACTTTTAGAAAGTCTTATCTTTTATGCCACAGAAATAGCATTTGGCTATTAG
TCATGGATGGCAAAGAAATTAATTTTGAGTTGTTTGGATAAAAATGTTTCAGTTGA
CTGTAGTGTGTATTGAGAGACACTGCCAGTAAACAAACTCTCTTGGTAGGTGGAAA
TCCCCTAGAAGTTACAGAAAATTGGGAGGAGGTGAACTTAATTAAATAACTTGAAT
TGTTTAGACATATTCAGAGCTTCTTATGACCTTGAAGAAATCACCCAACTTCAAAA
GACCTCGGTTTCTTCATTTGTAAAATTAGGGAGTTTGACTAGATGTGTAAATCTAG
TTGTTAGTTAACTTCTAAGATGTAAAAACCCTCTTGTTTAACAAAAACCTACAAGA
TCAAGTTGCTTATCTGAAATCTTTATGAATCAACACTAGTCACTAAGTCTAGCTCG
ACC


Sequence ID 306

CTTTTCCTCCCGCTGTCCCCCACGGAGGGGACTGCTCTCCCCCGCTGCATCCTTTC
TGTGAGGTACCTTACCCACCTCAGCACCTGAGAGGGTGAAATAGAATTCTAACCTC
GACATTCGGGAAGTGTTTTTGAGAAGTCTCGGTCGGTAAGGGAAGTCTTCCAAGTC
CGTGCAGCACTAACGTATTGGCACCTGCCTCCTCTTCGGCCACCCCCCAGATGAGG
CAGCTGTGACTGTGTCAAGGGAAGCCACGACTCTGACCATAGTCTTCTCTCAGCTT
CCACTGCCGTCTCCACAGGAAACCCAGAAGTTCTGTGAACAAGTCCATGCTGCCAT
CAAGGCATTTATTGCAGTGTACTATTTGCTTCCAAAGGATCAGGCCCTGAGAACAA
TGACCTTATTTCCTACAACAGTGTCTGGGTTGCGTGCCAGCAGATGCCTCAGATAC
CAAGAGATAACAAAGCTGCAGCTCTTTTGATGCTGACCAAGAATGTGGATTTTGTG
AAGGATGCNCATGAANAAATGGACNAGCTGTG

Sequence ID - 308                  nt:      373

AAGTGGGTCTTGCCATCCCTGAACTGNAATCATCCCTAACATATTCATACCTGTTT
TCATTTTAAAAGTTGGGTCAGTTTTTTTATTAGTACATGTATTTCTATCCTACTGA


TTTATTTGCTATATCATCTAATTTAGTTTGAATATTCCATAATTTACTTAATTAGT
CCTGTATGGAGACCTAGCTCTTCTCAGTGTCTACTATTATAAACAATGCTACAGTG
AATATTGGTGNATAAATCCATACNCACCACGTACATATCTTAAGTTCTGGAAGAGA
TATTGCTAAACCAGAAGATAACCTGCATTTAAAATTTGACTGCTAGGGNCAGGGNC
ACATTTAATTAAATTAGAACAANGAATGCATAATGNC


Sequence ID 309

CCGGAATCGCGGCCGCGTCGACGAAAATATGTGCCCTGGCCAACTCCACAGGACTA
GTTCTAGGCAATCTGAAGGAAACCAGAAAATGTGAATTTCTCTTCCCTCAAAAAGC
TATACTGAAGTAGTATTTAATATTCAAGTACTTGTAAATTTGCAGAACAGTACTTT
TTAATTTGACCCATGAATTCTATTTAAATTTGTCACTTAATATTTAGCCAAGAAGC
AAACCATCTAAAAAGATTTCTGGTTTATTTCTCCAACTCCTAATAAATAGGGTCAC
ATATTTTTTAACTTTTTTTCTAATTTGAAAAGTAATACAGGCATATGGTATTTTAAA
AATGAAACAACACAAAGGGATATGTTTTGAAAAGTGGTCTTGCCATCCCTGAACTG
TAATCATCCCTAACATATTCATACCTGTTTTCATTTTAAAAGTTGGGTCAGTTTTT
TTATTAGTACATGTATTTCTATCCTACTGATTTATTTGCTATATCATCTAATTTAG
TTTGAATATTCCATAATTTACTTAATTAGTCCTGTATGGAGACCTAGCTCTTCTCA
GTGTCTACTATTATAAACAATGCTACAGTGAATATTGGTGNATAAATCCTACACAC
CACGTAACATATCTTAAGTTCCTGGAAGAGATATTGCTAAACCAGAAGATAACCTG
CATTTAAAATTTGACTGCTAGGGTCAGGGTCACATTTAAATTAAATTAGAACAAGG
AATGCATAATGTCTTCGATAGCAATCTATTCAAGGTGCACCGTGGTCACAAAGGAA
AGCAAAACTGTC


98

Sequence ID - 310 nt:564
CCTGGNCAGAGGCCTCTATCCTGTANTGATAATTGCCATCAAAATTGTCAAAAANG
ATTTAATTTCTATGGGNAATAGTCCTTTTCTTAGCTTCTGCCNNTCACTTGCTTAT
TTTTTGTGTGGGAATGGGGTTGGATAAACCAATGAACTTTATTATAAACAAATCCC
ACCTATATCTANCAAATTTATATTTTCGGTGAAATACAGATATTTGCCTTTCTGGA
GTANTATAGAAGCTGTCAATATGTATCTACTGTACAGTACTAAATAGTATTCATTT
ATGAAATGAGTAGTGTTTGGGTGGCTGGGGTTAAGGAAAAATGAGACTTGGAATTG
TAGCTTTTATCCAAGTTTTGAGTATAAATAGGGTTTTGTTTTGTTTTTTTTAACCT
AAAAACTGAAATGCCATATAGAAAAACAGCATTGTTTTTACAGTTTGTAGTAAGTA
ACTTTTTAAAGATTTTATCAAAAAGAATTTTGTCTATNGTGAGTAAAAGAAGTTCT
AATAATGGCCTAATCACTGCATTTTTAAAAAACAAAGTTCAACACAAATGACATTT
GTTT


Sequence ID 311


CCTCTCCTCCATCTAAAGGCAACATTCCTTACCCATTAGTCTCAGAAATTGTCTTA
AGCAACAGCCCCAAATGCTGGCTGCCCCCGGCCAAGCATTGGGGCCGCCATCCTGC
CTGGCACTGGCTGATGGGCACCTCTGTTGGTTCCATCAGCCAGAGCTCTGCCAAAG
GCCCCGCAGTCCCTCTCCCAGGAGGACCCTAGAGGCAATTAAATGATGTCCTGTTC
CATTGG


Sequence ID - 313                          nt:        554
CCCGGAATCGCGGCCCGCGTCGACAACAAACCTGCATGTTCTGCACATGTATCCAG
GAACTTAAAAAAAAAAAAAGATAGTTTGTGTGTCTTAATTGAATAATAGTAGATTT
ATAGATTAAAGATCTATGGGTTTTTAATATGGATTANAAATCTGTGGGTTTTTGAT
ATGGATTANAAATCTGTGGGTTTTTAATATGGATTGGAAATCTGTGGGTTTTTAAT
ATGGATTAAAAAACATCTGTGGGTTTTTAATATGGATTAAACATCTGTGGGTTTTT
AATATGGATTAAACATCTGGGTTTTTAATATGGATTAAACATCTGTGGGTTTTTAA
TATGGGTTAAAAATCAAAGAAAATGAACTATTTGCTCCAGTGCAGGAAAATACAG
GCAATACTGGATACAATTAGATGGTCAGGAGCGATAACCCGGTTGCCATTGTTTGA
AGAAGAGAATAAGGNGCTAGCATTCCTATCCGTAGATAATTTGACAGCTAGGAAAT
AGGGGGAGTCTTCTATGTAGTTAGTGAAGGCTAAATGAACTATTATATGC

Sequence ID 314

CTTTTCCTCCCGCTGTCCCCCACGGAGGGGACTGCTCTCCCCCGCTGCATCCTTTC
TGTGAGGTACCTTACCCACCTCAGCACCTGAGAGGGTGAAATAGAATTCTAACCTC
GACATTCGGGAAGTGTTTTTGAGAAGTCTCGGTCGGTAAGGGAAGTCTTCCAAGTC
CGTGCAGCACTAACGTATTGGCACCTGCCTCCTCTTCGGCCACCCCCCAGATGAGG
CAGCTGTGACTGTGTCAAGGGAAGCCACGACTCTGACCATAGTCTTCTCTCAGCTT
CCACTGCCGTCTCCACAGGAAACCCAGAAGTTCTGTGAACAAGTCCATGCTGCCAT
CAAGGCATTTATTGCAGTGTACTATTTGCTTCCAAAGGATCAGGCCCTGAGAACAA
TGACCTTATTTCCTACAACAGTGTCTGGGTTGCGTGCCAGCAGATGCCTCAGATAC
CAAGAGATAACAAAGCTGCAGCTCTTTTGATGCTGACCAAGAATGTGGATTTTGTG
AAGGATGCACATGAAGAAATGGAGCAGGCTGTGGAAGAATGTGACCCTTACTCTGG
CCTCTTGAATGATACTGAGGAGAACAACTCTGACAACCACAATCATGAGG


Sequence ID 315

TGGTACAGATACAAACTGGACTCTCAGGACAAAACGACACCAGCCAAACCAGCAGC
CCCTCAGCATCCAGCAGCATGAGCGGAGGCATTTTCCTTTTCTTCGTGGCCAATGC
CATAATCCACCTCTTCTGCTTCAGTTGAGGTGACACGTCTCAGCCTTAGCCCTGTG
CCCCCTGAAACAGCTGCCACCATCACTCGCAAGAGAATCCCCTCCATCTTTGGGAG
GGGTTGATGCCAGACATCACCAGGTTGTAGAAGTTGACAGGCAGTGCCATGGGGGC


AACAGCCAAAATAGGGGGGTAATGATGTACGGGCCAAGCACTGCCCAGCTGGGGGT
CAATAAAGTTACCCTTGTACTTG


Sequence ID 316

CGCCACTTATCCAGTGAACCACTATCACGAAAAAAACTCTACCTCTCTATACTAAT
CTCCCTACAAATCTCCTTAATTATAACATTCACAGCCACAGAACTAATCATATTAA
AAAAAAAAAAAAAAAAAAAAAAAAAAAAAAAAAAAAAAAAA

Sequence ID 321

```
CAGAACAGTACTTTTTAATTTGACCCATGAATTCTATTTAAATTTGTCACTTAATA
TTTAGCCAAGAAGCAAACCATCTAAAAAGATTTCTGGTTTATTTCTCCAACTCCTA
ATAAATAGGGTCACATATTTTTTAACTTTTTTTCTAATTTGAAAAGTAATACAGGCA
TATGGTATTTTAAAAATGAAACAACACAAAGGGATATGTTTTGAAAAGTGGTTCTT
GCCATCCCTGAACTGTAATCATCCCTAACATATTCATACCTGTTTTCATTTTAAAA
GTTGGGTCAGTTTTTTTATTAGTACATGTATTTCTATCCTACTGATTTATTTGCTA
TATCATCTAATTTAGTTTGAATATTCCATAATTTACTTAATTAGTCCTGTATGGAG
ACCTAGCTCTTCTCAGTGTCTACTATTATAAACAATGCTACAGTGAATATTGGTGT
ATAAATCCATACACACCACGTAACATATCTTAAGTTCCTGGAAGAGATATTGCTAA
ACCAGAAGATAACCTGCATTTAAAATTTTGACTGCTAGGGTCAGGGTCACATTTAA
ATTAAATTAGAACAAGGAATGCATAATGTCTTCGATAGCAATCTATTCCAGGTGCA
CCGTGGTCACAAAGGAAAGCAAAACTGTCAATAACTTTCTTCTCA
```

Sequence ID 322

```
TAGCATTTGGCCTTTTAAAACATTTGTTTATTTTTTTTTCTGAGAATGGCTAACACA
CTTTATTGAGGTTCGAAATTAATAAAGAAAATAAAAGAAATGTATCTTCATTCATT
CTGTATGTTAGTGTTTTAATTACCCTTAGAATATATGGATAAAAAATACTATTCTT
TGTCTTGGAGAAGGTAAGAGTCTAGTTAGATGAATAAGGGTTATCTATGTAGAACA
ACTAGAGAATGAGAAGAGAGCTTATGAGATTGAGTACTACGTTATGCAGTAGAGTA
GCACGTCATCTGCTACTGAGTATGGTGTGATAACATTGTGTAACAGGAAAGTATGA
TCAATATCTACTTAAAATTAAGGACAATATTAGCACTACATTGCTTTATTTTAAAG
TAAAAATTAGAGAACTAAACACAAGCATTGTAAGTACAATAAAAGCTGATCTTTCT
AGTTAAGCAGAATAATACATGTTCAAGCATCTGCTAAATCATTAAATATAAGAATA
TAGGGGTTTTCTATAATCTTATTTTCTTTGGAAGAGTACCTCATTTTCAAGANGAG
AAGTTTCTAATTGCCACTTCTTTAAAAATAAAACAGGGTTTTAATGTTCCCAGCAC
AAAAATTAATATCTCTTCAAAAAGTCTCTTGTGATTAAGTTTGAATCCCTTGTCAT
ACTGCTTCTAATATTGACACTGACCTCCTTAGGTATTTTTCAGGGGTTATAATCTT
TTCTTAAGGTATCTTTTTTCAAGAATTGGATACCTTGGGCTT
```

Sequence ID 323

CGCGTCGACTTTTAAAGTCATCTCTATAGGAAGGTGCTGGGCAGGGATCCCAGAGA
AAGAAAGGGTCCAAGACTCCATTAACTGCCCTGGATGAAGGGCACTGCTACAGCAG
CTAGTACCAGAGACTCTCCTATCTCACGGTTGAGGCAGACCCAGGATAGAATAGAG
AATAAAAGGAATGCTTATAGGAAACAATTTTGTATGGAATGCTAGATGGCCAAGCC
TCAGCCTTTGGTCCAGTGCAACCCTTGCCTCGCTTGTCAACAGTGAAAAATTAGTT
TGGTTAGAAGAACCATCTGGAAACACACCAGCTTCTGCTACCTTCATGCTCATTGT
TAAAAAAGATTAACCAGTGTGAACATTCTGATCTGTTAATTCCAGGGACTGTTTT
CTTTCCAATGGACTGTTTGTTGGTAGAATAACCCCCAAAAGCTCAAAGCTAAAATG
CATCATCAGTCCTAGTCGGCAGTTCCTTAAGAATGGACTGGCGGCGTGGTTGAGCT
GATATGGAAAAGCTGCACCTTCCTGCAGAAGATCAACTGACCTGCTATCCCACCCC
AAATTCAACCTGAGGTATATTTCAGTGAAGCAGGTAGCTGTGCTTCTCAAAGCAGA
GAAGCAGTTTTAAGAACCAAAAAGGTAGAGGAAATCTA

Sequence ID 324

GTTTGTTACAGGCAGAATTGGATAGATACAGCCCTACAAATGTATATGCCCTCCCC
TGAAAAAAATTGGATGAAAATCTGCACAGCAAAGTGAAACACACAGATAATAGGAA
CAAAATGTAGTTCCCATGTGCCAAACAAAATAAATGAAATCTCTGCATGTTTGCAG
CATATCTGCCTTTTGGGAATGTAATCAAGGNATAATCTTTGGCTAGTGTTATGTGC
CTGTATTTTTTTAAAATGGTACACCAGAAAAGGACTGGCAGTCTACTTCTACCATA
GTTAAACTTCACCCTCTTTAATTTCACAACATATTCTTTGGAAGCAGGAAGAAATG
CTCATAAAGAGGATCAGACCTTCTTTCCCGTGAAACCAGTATTTGGCGCCATATAT
AAGCCTGGTTAAATTGGTCATCTAAAGCTGTCAAATAAGACATTCTGTGAAAGGTA
AACATCGAAACTGGTTATAAGTAAAACCATCAAGCCAACAACAGGGTCTTGAGATA
ACCTTTGAAGCTTATTGTCTGGCCTGCACCAGAAGATGTCTGCATTACTCATTGCT
AAAAATGTGTACACAGAACTGCACTAGGATTAATTGGTTCAAGAAGAAATTTAAAC
TTACGTTTGGGTTTCCATACAGCACTCTATTGAATACATGCATCTGAATTTAAGTT
GCAA

Sequence ID 325

GACCAGTAATGGCTTTTAAGAGTCCATTTTGTCATTGTCTCCCTAGTTAATTACAG
GTGGGGGATCTTTTGCCTCTATTCTCTTCATATTGAAATGAATCATACTCATGTTT
TGTGGAACTCCTTAAAGTTGTAGCTGTCATGATCAGATTTTTTTTATATTTCCTCA
GCTTAACTCTGCTACTTGATTTACAGTGACCCATAACCTACTCATCCTTGGTTTAT
AGTGACACATAATCTTATCTCTTTATAGAACCTTAAATTTTATCATTATTTTCGCT
TAGAATACAGCATTTCTTTGCTTCTGTTGCTGGTTTGACTTAAGAAATAAGGCAGT
AACTCTGATCAATCAATTATCCATAAGGAAGGGCTTTTCATGGGTTCTATTAATTT

GTTAGTACCCTAAGTATATCTGAAAAATATGTCTATTGAGAGAAGATTTTGGCATT
CCAGATGGTATAGTCTATATATATTTAAAGTTTTGAATTTGCTTATATATACTCAG
CTTTCTTTTTCTAGCATTTTTGCATTTACCTGTTAATTGAAGTATACCCCCCACAT
ATAAAGTTCCTCTTAAAGACACTGGACTCTTTCTGGGGGGCTAAAATA

Sequence ID - 326                    nt:     554

CCCGGAATCGCGGCCCGCGTCGACAACAAACCTGCATGTTCTGCACATGTATCCAG
GAACTTAAAAAAAAAAAAAGATAGTTTGTGTGTCTTAATTGAATAATAGTAGATTT
ATAGATTAAAGATCTATGGGTTTTTAATATGGATTANAAATCTGTGGGTTTTTGAT
ATGGATTANAAATCTGTGGGTTTTTAATATGGATTGGAAATCTGTGGGTTTTTAAT
ATGGATTAAAAAACATCTGTGGGTTTTTAATATGGATTAAACATCTGTGGGTTTTT
AATATGGATTAAACATCTGGGTTTTTAATATGGATTAAACATCTGTGGGTTTTTAA
TATGGGTTAAAAATCAAAAGAAAATGAACTATTTGCTCCAGTGCAGGAAAATACAG
GCAATACTGGATACAATTAGATGGTCAGGAGCGATAACCCGGTTGCCATTGTTTGA
AGAAGAGAATAAGGNGCTAGCATTCCTATCCGTAGATAATTTGACAGCTAGGAAAT
AGGGGGAGTCTTCTATGTAGTTAGTGAAGGCTAAATGAACTATTATATGC

Sequence ID 327

CGGCTACCGACAGAAGGACTATTTCATCGCCACCCAGGGGCCACTGGCACACACGG
TTGAGGACTTCTGGAGGATGATCTGGGAGGGGAAGTCCCACACTATCGTGATGCTG
ACGGAGGTGCAGGAGAGAGAGCAGGATAAATGCTACCAGTATTGGCCAACCGAGGG
CTCAGTTACTCATGGAGAAATAACGATTGAGATAAAGAATGATACCCTTTCAGAAG
CCATCAGTATACGAGACTTTCTGGTCACTCTCAATCAGCCCCAGGCCCGCCAGGAG
GAGCAGGTCCGAGTAGTGCGCCAGTTTCACTTCCACGGCTGGCCTGAGATCGGGAT
TCCCGCCGAGGGCAAAGGCATGATTGACCTCATCGCAGCCGTGCAGAAGCANCAGC
AGCAGACAGGCAACCACCCCATCACCGTGCACTGCAGTGCCGGAGCTGGGCGAACA
GGTACATTCATAGCCCTCAGCAACATTTTGGAGCGAGTAAAAGCCGAGGGACTTTT
ANATGTATTTCAAGCTGTGAAGAGTTTACGACTTCAGAGACCACATATGGTGCAAC
CCTGGAACAGTATGAAATGTGCTACAAAGTGGTACAAGATTTATTGATATATTTCT
GATTATGCTAATTTCAATGAAGATCCTGCCTTAAATATTTTTTAATTTAATGGCAN
AT

Sequence ID 328

CAAGACTCCATCTCAAAAAAAAAAAAAAAATCTACAGTGCTGAGTATATAAAATTAT
TAACACATTTCACAACAATATGTGTTTGTGGAGTTAAATATTTTTTGTCTTTAAAA
CAGGTAATTTTAGTGCATACTTAATTTGATGATTAAATATGGTAGAATTAAGCATT
TTAAATGTTAATGTTTGTTACATTGTTCAAGAAATAAGTAGAAATATATTCCTTTG

TTTTTTATTTAAATTTTTGTTCCTCTGTAAACTAAAAGAACACGAAGTAATTGGTC
ACAATTACTGGTGTTTAACTGCCAAATATGGGTAAATAAGGGAAAATTTTGTTTAA
TATTTAGTCCTTCTGAGATGGCTTGAATATTTGAATTTTGTTGTACGTCTATACTG
GGTAGTCACAAGTCTTATAAACACTTTAGAGGAAAGATGGATTTCAGTCTGTATTT
TTAAACATCATTTATTTAAATCTGGTGCTGAAAAATAAGAAAAAAATTAAACTGC
ATTCTGCTGTTCTTCTTTANAAGCATTCCTGCGTAAATACTGCTGTAATACTGTCA
TGCAAAGTGTATCCTTTCTTGTCGTATCCTTTTTGGGGCAGTGGTTTTT

Sequence ID 330
GCGGGAATCGCGGCCCGCGTCGACCTCAAAGGAGAAAAAAAACCTTGTAAAAAAAG
CAAAAATGACAACAGAAAAACAATCTTATTCCGAGCATTCCAGTAACTTTTTTGTG
TATGTACTTAGCTGTACTATAAGTAGTTGGTTTGTATGAGATGGTTAAAAAGGCCA
AAGATAAAAGGTTTCTTTTTTTTTTCCTTTTTTTGTCTATGAAGTTGCTGTTTATTTT
TTTTGGCCTGTTTGATGTATGTGTGAAACAATGTTGTCCAACAATAAACAGGAATT
TTATTTTGCTGAGTTGTTCTAAAAAAAAAAAAAAAAAAAAAAAAAAAAAAAAAAAA
AAAAAAAAAAAAAATTTTAAAATTTTTAAAATAAAACCCTTGGTTAT


Sequence ID 331
GCCGCGTCGACCTGCATGAGCCACAGTTTCTTGACTGGAGGCCATCAACCCTCTTG
GTTGAGGCCTTGTTCTGAGCCCTGACATGTGCTTGGGCACTGGTGGGCCTGGGCTT
CTGAGGTGGCCTCCTGCCCTGATCAGGGACCCTCCCCGCTTTCCTGGGCCTCTCAG
TTGAACAAAGCAGCAAAACAAAGGCAGTTTTATATGAAAGATTANAAGCCTGGAAT
AATCAGGCTTTTTAAATGATGTAATTCCCACTGTAATAGCATAGGGATTTTGGAAG
CAGCTGCTGGTGGCTTGGGACATCANTGGGGCCAAGGGTTCTCTGTCCCTGGTTCA
ACTGTGATTTGGCTTTCCCGTGTCTTTCCTGGTGATGCCTTGTTTGGGGTTCTGTG
GGTTTGGGTGGGAAGAGGGCCATCTGCCTGAATGTAACCTGCTAGCTCTCCGAAGC
CCTGCGGGCCTGGCTTGTGTGAGCGTGTGGACAGTGGTGGCCGCGCTGTGCCTGCT
CGTGTTGCCTACATGTCCCTGGCTTGTTGAGGCGCTGCTTCAACCTGCACCCCTCC
TTGTCTCATAGATGCTCCTTTTGACCTTTTCAAAATTAATATGGATGGGAAAGCTC
CTATGCCTTTTGGCTTCCTGGTAGAAGGCGGGATGCCCAAGGGTCTGCCTGGGTGT
GGATTGGATGCTTGGGGTGTGGGGGTTGGAAACTGTCTTGTGGCCCACTTGGGCCC
C


Sequence ID 335
CCCGCGTCGACTTTTAAAGTCATCTCTATAGGAAGGTGCTGGGCAGGGATCCCAGA
GAAAGAAAGGGTCCAAGACTCCATTAACTGCCCTGGATGAAGGGCACTGCTACAGC
AGCTAGTACCAGAGACTCTCCTATCTCACGGTTGAGGCAGACCCAGGATAGAATAG

AGAATAAAAGGAATGCTTATAGGAAACAATTTTGTATGGAATGCTAGATGGCCAAG
CCTCAGCCTTTGGTCCAGTGCAACCCTTGCCTCGCTTGTCAACAGTGAAAAATTAG
TTTGGTTAGAAGAACCATCTGGAAACACACCAGCTTCTGCTACCTTCATGCTCATT
GTTAAAAAAGATTAACCAGTGTGAACATTCTGATCTGTTAATTCCAGGGACTGTT
TTCTTTCCAATGGACTGTTTGTTGGTAGAATAACCCCCAAAAGCTCAAAGCTAAAA
TGCATCATCAGTCCTAGTCGGCAGTTCCTTAAGAATGGACTGGCGGCGTGGGTGAG
CTGATTTGGAAAACTGCCCTTCTGCAAAAAACACTGGCCTGCTTTCCA


Sequence ID 337

CAAGACTCCATCTCAAAAAAAAAAAAAAAATCTACAGTGCTGAGTATATAAAATTAT
TAACACATTTCACAACAATATGTGTTTGTGGAGTTAAATATTTTTTGTCTTTAAAA
CAGGTAATTTTAGTGCATACTTAATTTGATGATTAAATATGGTAGAATTAAGCATT
TTAAATGTTAATGTTTGTTACATTGTTCAAGAAATAAGTAGAAATATATTCCTTTG
TTTTTTATTTAAATTTTTGTTCCTCTGTAAACTAAAAGAACACGAAGTAATTGGTC
ACAATTACTGGTGTTTAACTGCCAAATATGGGTAAATAAGGGAAAATTTTGTTTAA
TATTTAGTCCTTCTGAGATGGCTTGAATATTTGAATTTTGTTGTACGTCTATACTG
GGTAGTCACAAGTCTTATAAACACTTTAGAGGAAAGATGGATTTCAGTCTGTATTT
TTAAACATCATTTATTTTAAATCTGGTGCTGAAAAATAAGAAAAAAATTAAACTGC
ATTCTGCTGTTCTTCTTTAGAAGCATTCCTGCGTAAATACTGCTGTAATACTGTCA
TGCAAAGTGTATCCTTTCTTGTCGTATCCTTTTTGGGGCAGTGGTT


Sequence ID 338

CTGGACTGCATGACCAGATCTGATGGGTGAGACTCAGGTGGCATGGAAGAGCCGAA
AGAGGATACCATATGTGGGTGCCGGGGGGGATAGGTGAGAAGTACTAGAAGGCGGA
ATGGAAGGACACTTCTGCTCAGCTCTGTGACACGGGCAGGGACCCTGCAGGGCTCA
GGTCCTTTAACACAGCAGCTTCATTCTAACACCAGCAGCGTTGGAACACACGTACA
AGTATGCAGACTAAGCTCTTGCTTGGCTGATACGGCTTTTTGGGTTTTTAGAGAAC
ATGCATATATGTTCTCATTCATGGTACATGAACTCAGAAGCCTTACTGCCTATTTT
TGTTAATACTTCTGGGCAAACATTACCACTTACAACTCACACCAGTTAGAAATCAT
TTGTAAAATGTTATTTAATAAAGCCAAAGAACTAAATCATATTTATTTTCCAAGGN
TTTCTAAGATCTCTGAAACTAATGAGGTTTTTTAAATCCCCATTAAGTACTCATCA
CTGCTAGTAAAAGCAGTTGTCTTTACCTTTAATTCCAGTGAGTCCCCTTAAATTTA
TTTTTTATTATCTTTGGCTACATTGCCTTAGACAAAATGTGGTCACCCTAATTTAA
NGGATAAAATTCACATCCTCACAGATTTCTTATTAAGAGGGTCTAANCCTTGAATA
ATCANCAGTGGAAATGGAAGTCTTCTTTACTGGNTTTNATCCTTTCCCTTTTTTAT
CCCATG

Sequence ID 339

TTTTTTTTTAAATAAAGCTGTCGGCACTCAAGGGTAATTTCATATCAGTGTGNTCT
ACAAGCTGGGGGAAAATGAGTTCTAATTGTCANAGCTACCAAATCCTTCACCTTTA
GCATAAAGGTTTAAAGATATCACAAAGATGCCAAGTGATTAATAATGTTTTAAACC
ACCCCTTTTTCTGTCTGAAAAAACAACTAAAACAATATTACAACAGTATAGTTACA
GAAGGGTTCTATTTTCATATGTTTTATGCACACTGTGCCTCAAAGGTACTATTTAA
ATATATATACTTTTGAGGGGGTGGCTAATGCAGAAACACCCAAGACCTAAGGAAGA
TACAACCCCATTTCTAGGTGTGAGGTCTAAATGCTTCACACACCCACTTGTGACCT
TTTTTCATGAAGAATCATAACACTGTGCAGTGAGAAACAGTGGCAAAGCAATACTG
AAAGCATTTTAAATTATTTACTAGGTTAAAAGGGTGAACTGATACTTTAAATACAT
CAAATTTCATCAT

Sequence ID 360

GCAAGTGAGAGCCGGACGGGCACTGGGCGACTCTGTGCCTCGCTGAGGAAAAATAA
CTAAACATGGGCAAAGGAGATCCTAAGAAGCCGAGAGGCAAAATGTCATCATATGC
ATTTTTTGTGCAAACTTGTCGGGAGGAGCATAAGAAGAAGCACCCAGATGCTTCAG
TCAACTTCTCAGAGTTTTCTAAGAAGTGCTCAGAGAGGTGGAAGACCATGTCTGCT
AAAGAGAAAGGAAAATTTGAAGATATGGCAAAAGCGGACAAGGCCCGTTATGAAAG
AGAAATGAAAACCTATATCCCTCCCAAAGGGGAGACAAAAAAGAAGTTCAAGGATC
CCAATGCACCCAAGAGGCCTCCTTCGGCCTTCTTCCTCTTCTGCTCTGAGTATCGC
CCAAAAATCAAAGGAGAACATCCTGGCCTGTCCATTGGTGATGTTGCGAAGAAACT
GGGAGAGATGTGGAATAACACTGCTGCAGATGACAAGCAGCCTTATGAAAAGAAGG
CTGCGAAGCTGAAGGAAAAATACGAAAAGGTA

Sequence ID - 361                    nt:      622

CTGTNATNGAATCTGCTTGTNACTNAAATGCTAAACTCAATTCTGTAATTCAATAG

GTGCACCTNTCTGAGAAACATANNAGACAATGAGGAAAAGGATTCANCATTCCGTG

GAATTTGTACCATGATCAGTGTGAATCCCANTGGCGTAATCCAAGTAAGATGTTCA

CAAAGATTTGTTTTTAATGTCTAATTAATAAAATTTTAAAGGAAGAAACATTCTAA

TACTTTAATTATAAAAAGTTAACTATTTTCAAAGGTATCAAAATACAGTTAAACCT

TTAAAATGTATATTTCTTAATATCTTGAAATTGTAATGCCTTTTTTTTTTTCCTAAA

TTTTTTTTGTCATGAAATGAGATAGTAACAGCAGATTGGGACAACAAGGTTATATT

CTTGTCTTGAATCAGGCCATGGCTTCTTTCATCCAAATTTCAGACCTCATTTATTT

ACTTTGTCCCTGCCTCCCATCCCTGGATATCANGTTTGTGGATATCTACAGTTAAT

AGAGTGACCAAATAGTAGGAATACTGTCTCTCTATTCTGAATAAAATACTTTGAAT

CAGATTTAGAAATAATGAATAAAATACAAATCACCATTGAAATTGCTCTAATTTTG

AGAGCT


Sequence ID - 363                    nt:      628

ATCACNTGAGGCAAGAGTTTGAGCCAGCCTAGCTAACATGGTGAAACCCCATCTCT

ACAAAAATATAAAAATTAGCCTGGGTGGTGATGGGCACCTGTAACCCCAGCTACTC

GGGAGGCTGAGGTAGGAGAATCACTTGAACCCGGGAGATGGAGGTTGCAGTGAGCC

AAGATCGTGCCACTGCACTCCAGCCTGTGTGACAGAACAAGACTCTGTCTCAAAAA

AAAATAATAATAATAATAATAATAAAAAGGAATAACATAGCTAGGAATAAATTTAA

TCAAAGAGGTGAAAGACTTATACACTTAAAACTACAAAAAAAAAATCACTGAAGGA

ATTATAGACCCAAATAAAAATAAATAAAAGACATTCTGTGTTTTAGGGAAAGAAG

ACTTAATATTGTTAAGATGTCAATACTACCCAAAGTGATCTACAGATTCAACATAA

TCCCTATCAAAATTCCAACAGCCTACTTTGTAGAAATGGAAAAGCCAATTTTCAAA

TTCAGATGGAATTGCGAGGGGTTCTGAATAACAAAAACAATCTTGGGGAAAAAAAA

CAAAAAACAAAGTCAAAGAACTCACACTTCTCTATTTATAAATTTACTACAAAGTT

ATAGTAATCAAA

Sequence ID - 364            nt:      528

```
TGAACATCCAGCCATGTCATTTCTTCCATTCCTGCCCTGGAGTAAAGTAGATTTAC
TGAGCTGATGACTTGTGTGCATTTGTACATTGCAACCTTAGCTTACCTCTTGAAGC
ATGTAGAGCATTCATCACCCACCATTCATTCACTGCCTACTCCCACCACAGCTGTT
TCGTGGTCTGTCTGCTCCCTGTGCCACCCCCACCCCATCAGGTGGGCCTTTTGCAA
GTGATGAAGTCACCTGTGGGGGAAGAGCTTTCCTTTCCTCTCCTCAACTCAGAAGG
CCTCTTCCTCTTGCTCAAGAGGGTGCTGCTGCTTTCTGCCTCCTTCCCCGGCCGGC
CTCCATCCCAGTTCACCTTTTCAGAAATGGCCCCTCAGTCAACTCTTCCCTTTTCT
CCTGGCTTTTTATTTCTCCCAGTCTCTTAAGAGTATCCTTAGCTTTAAAAACAATA
ACACAGAGGATGGGTGCAGTGGCTCATGCCTGTAATCCCAGCACTTTGGAGCCTGG
GGCGGGCGGATCACTTGAGGNCA
```

Sequence ID 365

```
GTCCCGGAATCGCGGCCGCGTCGACCTTTTCTATGCCTGCTATATAAACAGTACCT
TGCAAGATGTCCTGTCTGATATCCACAAAGGGGTATTGTCAACCCCAAGTTCAGAC
AGCTTTGTATTCTTCTGTCCCTGGATACATGAATTACTGCCATCTTTACACAGCGC
CCTAAAATACCAACGCGAAGTTACCTGCTCAGCTTGAAGCTGCGCTGTACCCTGGA
ACCAGCACTTCTGCTGAATGACTCAGGATGAAGCCTCGACTTCTCCTTCCCATCCC
ATGCCCAGACCCCAGTGGCTCCTTTCCCAATCTGATCCAGTGACTTTAAGTCCAGC
TGTTGCAACCTGGGCATGAGGAGGAGTGCAAGATGGCTTTGTCCTACCTGGAAAGA
GGCTTTCTGGA
```

Sequence ID 366

```
CACCATTTACACACAGTGGGTCCTTGAATAGCATCGTTTTATTCAATGTCATTTTG
TTATAACATTGAGAAAAAAATTGATTCCCGGCTGGGGCCACTGTCTGTGCACCGT
```

Sequence ID - 368                          nt:       329
GAAAGATCTAAAATCGACACCCTAACATCACAATTAAAAGAACTAGAGAAGCAAGA
GCAAATTCAAAAGCTAGCAGAAGGCAAGAAATAACTAAGATCAGAGCAGAGCTGAA
AGAGATAGAGACACAAAAAACCATTCAAAAAAAAACAATGAATCCAGGAGTTTTTT
TTTTAAAAAGATCAACAGAATTGACAGACTGCTAGCAAGACTAATAAAGAAGAGAG
AAGCATCAAATAGACTCAATAAAAAATGATAAAGGGGATATCACCACCAATCCCAC
AGAAATACAAACTACCATCAGAGAACACTATAAACACCTCTATGCAAAT


Sequence ID 369
GAAAGATCTAAAATCGACACCCTAACATCACAATTAAAAGAACTAGAGAAGCAAGA
GCAAATTCAAAAGCTAGCAGAAGGCAAGAAATAACTAAGATCAGAGCAGAGCTGAA
AGAGATAGAGACACAAAAAACCATTCAAAAAAAAACAATGAATCCAGGAGTTTTTT
TTTTAAAAAGATCAACAGAATTGACAGACTGCTAGCAAGACTAATAAAGAAGAGAG
AAGCATCAAATAGACTCAATAAAAAATGATAAAGGGGATATCACCACCAATCCCAC
AGAAATACAAACTACCATCAGAGAACACTATAAACACCTCTATGCAAATAAACTAG
AAAAT


Sequence ID 370
GAAAGATCTAAAATCGACACCCTAACATCACAATTAAAAGAACTAGAGAAGCAAGA
GCAAATTCAAAAGCTAGCAGAAGGCAAGAAATAACTAAGATCAGAGCAGAGCTGAA
AGAGATAGAGACACAAAAAACCATTCAAAAAAAAACAATGAATCCAGGAGTTTTTT
TTTTAAAAAGATCAACA


Sequence ID 371
GCCCGGAATCGCGGCCGCGTCGACGTAAGCTCGGCTGAATCCACGGTTCAAGAACA
GGAAAGAAGGCCAAGGCATAGGGAGTGGGGCAGTTGGGTGAATATTAGTACCTTTC
CCTCAGNTNCATTAATTACCCCTGCCTACTCTGCACAAAAGGATNTAACAACAGTT
TCCTTTTTAATGGCCAGGTACAGCTGCTTATATGGANGGGCATTTNTNAATGATAT
CCTTNATCACTGTCTTAATCATCACATNCTTAAAACAATCACTTTATTGTGTTAAG
GAAGATAAAAATGGCTGGGTTCAATTTCCGTTCTGGAAGAAATCGANTNAAAAGGT
AACCATTTAATAATGCANAGGGCANTTTCACTGCAGACCCTAATACTGGAAATTTT
TAAAAACAAATGAAAAACTTCTACTTTTTCTTCTAAGCTTACTTAACCACCCAAAT
TTTCCAGCCACATATCTTCCTAGTCTACAACTGCCTTTAACTTTAAGAGATGCTCA
AAAAAATGTAAATTCTCAAATACATTCTTATTACAATTACTGCTAACCT

Sequence ID 373

CCAGTGTGCTGGGATTACAGGCATGAGCCCTGCACCCAGCCTCTTAAACTGATCAT
ATGATATTGGTTCTCAACCAAGGGTGACTTTGCCCCCAGAGGATACTTGGCAATGT
CTGGAGATACTCAGTTGTCATGACTTGGACAGGTGCTACTGTCACCCAGTGGGTAG
AGGTCAGGGATGGTGCTAAACATAGGACAGCTGTCAAGAGAAAAGAATGTACCCAG
CCCCAAATGTCAGTAGGGCTGAGGTTGAGAAACCCAGCTGTAGCTGACGTGTGAAG
GACAGACTGGCCTGGAAGTGTGTTTTCTGCCCCTTTCCACCCCTGCATATTAGTTA
AGGCCAAAGGAAAAAAGGAATGCAGGAAATGCCCGTTAAAAATCTTCAAAACAATA
TAAAATGATCAATTCCACTAAAACCCTTTACACATTTAAGTATAAAGGTATTGGTA
GGAAAATTTGTTATTCACTGCTTTTCTCAGTGTCATGAAATAATTATTTCTGCTGT
CAGTTT


Sequence ID 374

AAAAAAAAAATCACTGAAGGAATTATAGACCCAAATAAAAATAAATAAAAAGACAT
TCTGTGTTTTAGGGAAAGAAGACTTAATATTGTTAAGATGTCAATACTACCCAAAG
TGATCTACAGATTCAACATAATCCCTATCAAAATTCCAACAGCCTACTTTGTAGAA
ATGGAAAAGCCAATTTTCAAATTCAGATGGAATTGCGAGGGGGTTCTGAATAACAAA
AACAATCTTGGGGAAAAAAAACAAAAAACAAAGTCAAAGAACTCACACTTCTCTAT
TTATAATTTACTACAAAGTTATAGTAATCAAAGTCGACGCGGCCGCGATTCCGGG


Sequence ID 378

CGACTGCGGCTCTTCCTCGGGCAGCGGAAGCGGCGCGGCGGTCGGAGAAGTGGCCT
AAAACTTCGGCGTTGGGTGAAAGAAAATGGCCCGAACCAAGCAGACTGCTCGTAAG
TCCACCGGTGGGAAAGCCCCCCGCAAACAGCTGGCCACGAAAGCCGCCAGGAAAAG
CGCTCCCTCTACCGGCGGGGTGAAGAAGCCTCATCGCTACAGGCCCGGGACCGTGG
CGCTTCGAGAGATTCGTCGTTATCAGAAGTCGACCGAGCTGCTCATCCGGAAGCTG
CCCTTCCAGAGGTTGGTGAGGGANATCGCCCAGG

Sequence ID 380

GCAATTTAATTTTTAATAACAAAGATACTGTATTTTAACATGGTGAAATATACTTG
GCTAAGTCCAGATTAAAAAAAAAAAGTATCTAGCCCAACAGTACAATTATACAGCT
TTGTACAGAACATTCCATAGATCAACAGAAAATACATTTGAGCGCAAAAATAAAAA
ATATTTAAGGAGAATCTCTAAGCAGCATTTTATTTCTGCAAAAGACATATCTTGTC
TGATTAAATATCTACAAGTGCTTTTCCTTTCAAAAATACATATATTCTTAATAGAC
TAAGTCATTAACAATGACCTGGTAATTCTTTCACTTCAATTTGAATGATTTATAAG
CTAAATCTTCAACCACAAAAAGGTTTTTATTTGTATTAAGATGTTACCACTTTTGA

CAAAAAGCTTAAAATATTTTATATTTCAAAGGAAAATTAGCAACATAACTTTACAA
TATATTCTATGATATTTTGATTGTGAGGGCTACTCTATTTAAAACTGATGATCTCT
GTTGTGTTGCTCAGATGCAGGAAAGCAGCAAAA

Sequence ID - 381                              nt:      534

GACTTANATCTAAATGGACCACATTCTCTACTTAAAAAAATGCTATTAACCATGTG
ATCTTCTCAGTCATGAGGTAATCTGGTGACTACCCTTCCTCAAAGCCAGTTGGGAT
ATTCTTTGAATAGAGTAAAACAGTGTTTCTAGGCTGGGAGACACCAGACATAGTTG
AGGACAGAGGTGCTAGAAAATAGGAAGTTTAAAAGCATGTGCGGTGATGCTCAGAG
GAGGTAAACCCCACCCTCATGCTCATAGCTTCCAATCATTTTCTCTAGTTCTTAAC
TCTTAAATGTGAGAAATGCTTGAAGATTCTAGTCATCTGAAGAAAGTCTCTTTATT
AAAGATTTTCATAAAAGAGACCAAAGCAGACAAACAGAAAAAGACATCTTGGGGAA
AAAAACAAGGATAATGGGAAGAGAAGGAAAGTTTTAAAAATTATCAATATCCTCAG
GGGGACAAAATATTATATCCTATAAAGACAGATTTTTATTTTTTAAAAAAATAGAA
AGCAAAACAAGCTCCTAAAAATAAAGTTTG

Sequence ID - 382                              nt:      444

GTTAAGGAAGTCAGCACTTACATTAAGAAAATTGGCTACAACCCCGACACAGTAGC
ATTTGTGCCAATTTCTGGTTGGAATGGTGACAACATGCTGGAGCCAAGTGCTAACA
TGCCTTGGTTCAAGGGATGGAAAGTCACCCGTAAGGATGGCAATGCCAGTGGAACC
ACGCTGCTTGAGGCTCTGGACTGCATCCTACCACCAACTCGTCCAACTGACAAGCC
CTTGCGCCTGCCTCTCCAGGATGTCTACAAAATTGGTGGTATTGGTACTGTTCCTG
TTGGCCGAGTGGAGACTGGTGTTCTCAAACCCGGTATGGTGGTCACCTTTGCTCCA
GTCAACGTTACAACGGAAGTAAAATCTGTCGAAATGCACCATGAAGCTTTGAGTGA
AGCTTTTCCTGGGGACAATGTGGGCTTCAATGTCAAGAATGTGTCTGTCAAG

Sequence ID - 383                    nt:        566

CTTTGAAGAACTTTGCCAAATACTTTCTTACCAATCTCATGAGGAGAGGGAACATG

CTGAGAAACTGATGAAGCTGCAGAACCAACGAGGTGGCCGAATCTTCCTTCAGGAT

ATCAAGAAACCAGACTGTGATGACTGGGAGAGCGGGCTGAATGCAATGGAGTGTGC

ATTACATTTGGAAAAAAATGTGAATCAGTCACTACTGGAACTGCACAAACTGGCCA

CTGACAAAAATGACCCCCATTTGTGTGACTTCATTGAGACACATTACCTGAATGAG

CAGGTGAAAGCCATCAAAGAATTGGGTGACCACGTGACCAACTTGCGCAAGATGGG

AGCGCCCGAATCTGGCTTGGCGGAATATCTCTTTGACAAGCACACCCTGGGAGACA

GTGATAATGAAAGCTAAGCCTCGGGCTAATTTCCCCATAGCCGTGGGGTGACTTCC

CTGGTCACCAAGGCAGTGCATGCATGTTGGGGTTTCCTTTACCTTTTCTATAAGTT

GTACCAAAACATCCACTTAAGTTCTTTGATTTGTCCATTCCTTCAAATAAAGAAAT


TTGGTA


Sequence ID 384

TTTTGGGGTTTATATATAAGCCTGGTTCTTGCTGAAACTGCTTATGTTGATAACCA

GTTAGTGAGTTCCTCTCTATTGACTTGCTGGGAAGTTTATAGAGACATTTTTTATG

CATTCAGAGATTTCAGTACAAATCTTGAAAAAGGGACATTTAGGCCGGGCGCGGTG

GCTCACATCTGTAACCCTAGCACTCTGGGAGGCTGAGGTGGGTGGATCATGAAGTC

AAGAGATAGAGACCATCCTGGCAAAAATTAGCTGGGCGTGGTGGGGTGCGCCCGTA

GTCCCAGCTACTCGGGAGGCTGAGGCAGGAGAATTGCTTGAGCCCGGGAGGCGGAG

GTTTCATTGAGCCGAGATAGTGCCACTGCACTCCAGCCTGGACAACAGAGCGAGAC

TGTGTCTT

Sequence ID 386

CTAAGGGTTTAAAGATGGAAAGAGGCATTGATGAACAGCTGGGGAAGGAGTAGTTT
GAGGTAGATGTGCAGATGGAATGAAGAGAAGGTCTCAAGAAGAGGGTGGAGCCAAA
GAGGGCTGCAGATTTAGAAGGCTAAAGTCTTTAGATGGCTTTGGATAGCCTGTTGT
ATCTTGGACCATGCAGGTTACAGTGGAGCATGGAGTGGGGACAGAAGTGGAGGAAG
GAACCAGGGAACATGGAGTGAGAAGCTAAAGGAAAGTGATGCAGTAGATACATGGC
TCTAAAGTACTCAGGACTTTCAGAGGCTTAAACATAGGGTGACCAACTATCCCACT
ATGCCTGATACTAAGGGCATTCCCTGGATGTGGACCTTTCATTCCCCAAATTAGGA
AAGTCTTGGGCATACCAAGACAAGTTGGCCACCCTACTCAAAAGTATGTAAGCTAA
CATATCTGTTCTCTAAGAGGTTAAAGCTGGATGGGGATACCAGATGTATGTACGTG
ATGCAGTTAAACAGCAATACAAGGGGGCAAGTCTACCTGATCGGCCAATTCAATGG
GA                                                       .

Sequence ID 387

GAAGCCAAACCAAAGGAGCTTCTACTTCATGATGCCATTTATGTAAAGTTCAGGCA
GAGAAAATCAGTGGTTTAAGAAGTTAGAATAATGATTATCTTTGGAGGGATTGCAA
CTGGAAGAAGTCATGATTGGGATTTCTGGGTCCTAATAGTGCTCTGTGTCTTGATC
TGAGTGCCGACTACATGAGTGGTTAGGTTTGCAAAATTCATTGAGTTATGCACTTA
ATGGTGTTGTCTTATTAGAGCTGATGGAGGAGAGAGGGCTTCAATTTGCACAACTG
AGTAATCAGCTAGGCCCAGTCACTAGGTGAACAACTTACTGCTCCAATCAGCCTTA
GAGCAGGAATCAAACTCATGTCTCAGAAAAGTTATTAATTCAGCTTGTCTTGGGAC
TTCCTTCAGAGTCACTCTTGAATAGCTGAAATAGTAAATGTTAAATCTGTGGATGC
AAGTGTGTAAATTATTTTAGTCATCAGCTCTAATAAGATGGCCTTTGGGGAAATGA
GTATAAGGTCACGAAAATGAAATGGCAAGAAGGAGGTCTACTATTTCTTCTGTAAT
ACTGATTTTTACCCCATCAGGGTCAGTCCCCAGAGGTTGTAAATGTGAAGCTTG-T

CTTTTTCTTTAATAA

Sequence ID 388

```
CTTTGGACACTAGGAAAAAACCTTGTAGAGAGAGTAAAAAATTTAACACCCATAGT
AGGCCTAAAAGCAGCCACCAATTAAGAAAGCGTTCAAGCTCAACACCCACTACCTA
AAAAATCCCAAACATATAACTGAACTCCTCACACCCAATTGGACCAATCTATCACC
CTATAGAAGAACTAATGTTAGTATAAGTAACATGAAAACATTCTCCTCCGCATAAG
CCTGCGTCAGATTAAAACACTGAACTGACAATTAACAGCCCAATATCTACAATCAA
CCAACAAGTCATTATTACCCTCACTGTCAACCCAACACAGGCATGCTCATAAGGAA
AGGTTAAAAAAAGTAAAAGGAACTCGGCAAATCTTACCCCGCCTGTTTACCAAAAA
CATCACCTCTAGCATCACCAGTATTAGAGGCACCGCCTGCCCAGTGACACATGTTT
AACGGCCGCGGTACCCTAACCGTGCAAAGGTAGCATAATCACTTGTTCCTTAATTA
GGGACCTGTATGAATGGCTCCACGAGGGTTCAGCTGTCTCTTACTTTTAACCAGTG
AAATTGACCTGCCCGTGAAGAGGCGGGCATAACACAGCAAGACGAGAAGACCCTAT
GGAGCTTTAATTTATTAATGCAAACAGTCCTAACAAACCCCAGGTCCTAAACTCCA
AACCTGCATTAAA
```

Sequence ID 389

```
CGACCCGGAATTCGCGGCCGCGTCGACTGAGTTCTTGACAAGAGTGTTTTTCCCTT
CCCGTCACAGAGTGGGCCCAACGACCTACGGCACTTTGACCCCGAGTTTACCGAAG
AGCCTGTCCCCAACTCCATTGGCAAGTCCCCTGACAGCGTCCTCGTCACAGCCAGC
GTCAAGGAAGCTGCCGAGGCTTTCCTAGGCTTTTCCTATGCGCCTCCCACGGACTC
TTTCCTCTGAACCCTGTTAGGGCTTGGTTTTAAAGGATTTTATGTGTGTTTCCGAA
TGTTTTAGTTAGCCTTTTGGTGGAGCCGCCAGCTGACAGGACATCTTACAAGAGAA
TTTGCACATCTCTGGAAGCTTAGCAATCTTATTGCACACTGTTCGCTGGAAGCTTT
TTGAAGAGCACATTCTCCTCAGTGAGCTCATGAGGTTTTCATTTTTATTCTTCCTT
CCAACGTGGTGCTATCTCTGAAACGAGCGTTAGAGTGCCGCCTTAGACGGAGGCAG
GAGTTTCGTTAGAAAGCGGACGCTGTTCT
```

Sequence ID - 390                          nt:      523

```
GAATCCCTAGAAAAAGAGAATTCCCAACTTGATGAGGAAAACTTAGAACTGCGAAG
GAATGTAGAATCTTTGAAGTGTGCAAGCATGAAAATGGCTCAGCTACAGCTAGAAA
ACAAAGAACTGGAAAGTGAAAAAGAGCAACTTAAGAAGGGTTTGGAGCTCCTGAAA
GCATCTTTCAAGAAAACAGAACGCTTAGAAGTTAGCTACCAGGGTTTAGATATAGA
AAATCAAAGACTGCAAAAAACTTTAGAGAACAGCAATAAAAAAATCCAGCAATTAG
AGAGTGAACTACAAGACTTAGAGATGGAAAATCAAACATTGCAGAAAAACCTAGAA
GAACTAAAAATATCTAGCAAAAGACTAGAACAGCTGGAAAAAGAAAATAAATCATT
```

AGAGCAAGAGACTTCTCAACTGGAAAAGGATAAGAAACAATTGGAGAAGGAAAATA
AGAGACTCCGACANCAAGCAGAAATTAAAGATCCACATTTGAAGAAAATAATGTGA
AGATTGGAAATTTGGAAAA

Sequence ID - 391                                nt:       566
CTTTGAAGAACTTTGCCAAATACTTTCTTACCAATCTCATGAGGAGAGGGAACATG
CTGAGAAACTGATGAAGCTGCAGAACCAACGAGGTGGCCGAATCTTCCTTCAGGAT
ATCAAGAAACCAGACTGTGATGACTGGGAGAGCGGGCTGAATGCAATGGAGTGTGC
ATTACATTTGGAAAAAAATGTGAATCAGTCACTACTGGAACTGCACAAACTGGCCA
CTGACAAAAATGACCCCCATTTGTGTGACTTCATTGAGACACATTACCTGAATGAG
CAGGTGAAAGCCATCAAAGAATTGGGTGACCACGTGACCAACTTGCGCAAGATGGG
AGCGCCCGAATCTGGCTTGGCGGAATATCTCTTTGACAAGCACACCCTGGGAGACA
GTGATAATGAAAGCTAAGCCTCGGGCTAATTTCCCCATAGCCGTGGGGTGACTTCC
CTGGTCACCAAGGCAGTGCATGCATGTTGGGGTTTCCTTTACCTTTTCTATAAGTT
GTACCAAAACATCCACTTAAGTTCTTTGATTTGTCCATTCCTTCAAATAAAGAAAT
TTGGTA

Sequence ID 394
GACCCGGAATCGCGGCCGCGTCGACCATTTTAGCCAAGGTGCCTCTATAGGGGTCA
AGACATCATGTGCCCAGACCTAAGGTCAGGAATGTCATATTTTTCTGTTAAAATCA
TTTTATTTCTGTGTATCTTACCTTTAAATCATTGTGGTTTACTCTGAGATTCTGTA
GTCCTAATATTGTATCATTGTGCTGTCTGCAAAACAACTTGAATCTATTTTGTTTG
CATCTTTTGTTACATGTAACGCAGCTGTACTTTATGTTCTTTGCAACTGTTTCCAT
TATGAGAACGCTGTGCTATTTACAAGGTTACATTTTTCTTGGCCAGGCGAGGTGGT
CATGCCTGTGATCCCAGCACTTTGGGAGGCCAAGGTGGGCGGATCACTTGAGGTAA
AGAGTTGAGACCAGCCTGGCTAGCATGGCGAAGCCCAGTCTCTACTAAAAATACAA
AAATTGGCCGGGTGAAATTAGCCGGGCGTGGTGGTGTGTGCTTGTAATCCCAGCTA
CTCGGGAGGCTGAGGCAGGAGAATCGCTTGAATCCGGGAGGCAGAGGTTGCAGTGA
GCCAAGATCANGCCACTGCACTCCACCTCGGGGTCAAGAGCGAAACTCTGTCTCAA

Sequence ID 395

```
CCGTTTTAGTCAGGATGGTCTCGATCTCCTGACCTCGTGATCCGCCTGCCTCGGCC
TCCCAAAGTGCTGGGATTACAGGCGTGAGCCACCGCGCCCGGCGTAAATCAGGTTT
TTTAAATGTTTGCCAAACCTTATCACTGACTTTTATAACAAAATTATTTACTATAA
TCATTAGGGAATATTTAAGTTCTGCTAATACTTAAAATTGCAGAGTGCTAAAACCA
GCAGTGAGTTTAGAATCAAGCTAAGCTTTATTGTTGCTACTATTTGAGGCATATTA
GTTGACTGGTGTTCATATGCAAGGCAGTCTACTGGGTGCAACAAGGGTTAGAAGGA
```

```
TATTTTTAAAAAACTGACCCTATTCTCAGGATGAAAATAATACACTAGTAATAGTC
TGCTCTGTTGGTTAACTCCTCGTAAGGAGGTCAATTAAAATGCTGTAGTGTTGCAA
GGGAAGGAGAGGAAGAATCATATTCCTTCACTAGCAGGATCAAGAAAGCTTTTATA
GAAATATACAAAATCTTCACTTCTTGAAGGATTGGTAAAATTTAATAGCCAACATT
GGGCACTTATTCATTCTCTGAGTAAATATTTATTGCAT
```

Sequence ID 396

```
CTTAAATCTAAATGGACCACATTCTCTACTTAAAAAAATGCTATTAACCATGTGAT
CTTCTCAGTCATGAGGTAATCTGGTGACTACCCTTCCTCAAAGCCAGTTGGGATAT
TCTTTGAATAGAGTAAAACAGTGTTTCTAGGCTGGGAGACACCAGACATAGTTGAG
GACAGAGGTGCTAGAAAATAGGAAGTTTAAAAGCATGTGCGGTGATGCTCAGAGGA
GGTAAACCCCACCCTCATGCTCATAGCTTCCAATCATTTTCTCTAGTTCTTAACTC
TTAAATGTGAGAAATGCTTGAAGATTACTAGTCATCTGAAGAAAGTCTCTTTATTA
AAGATTTTCATAAAAGAGACCAAAGCAGACAAACAGAAAAAGACATCTTGGGGAAA
AAAACAAGGATAATGGGAAGAGAAGGAAAGTTTTAAAAATTATCAATATCCTCAGG
GGGACAAAATATTATATCCTATAAAGACAGATTTTTATTTTTTAAAAAAATAGAAA
GCAAAACAAGCTCCTAAAAA
```

Sequence ID - 397                          nt:        534

GACCCGGAATCGCGGCCGCGTCGACGGAAGCTCCTGCCCCTCCTAAAGCTGAAGCC
AAAGCGAAGGCTTTAAAGGCCAAGAAGGCAGTGTTGAAAGGTGTCCACAGCCACAA
AAAGAAGGAGATCCGCACGTCACCCACCTTCCGGCGGCCGAAGACACTGCGACTCC
GGAGACAGCCCAAATATCCTCGGAAGAGCGCTCCCAGGAGAAACAAGCTTGACCAC
TATGCTATCATCAAGTTTCCGCTGACCACTGAGTCTGCCATGAAGAAGATAGAAGA
CAACAACACACTTGTGTTCATTGTGGATGTTAAAGCCAACAAGCACCAGATTAAAC
AGGCTGTGAAGAAGCTGTATGACATTGATGTGGCCAAGGTCAACACCCTGATTCGG
CCTGATGGAGAGAAGAAGGCATATGTTCGACTGGCTCCTGATTACGATGCTTTGGA
TGTTGCCAACAAAATTGGGATCATTTAAACTGAGTCCAGCTGCCTAATTCTGAATA
TATATATATATATATATCTTTTCACCATAA


Sequence ID - 398                          nt:        512

GGGGAGCCCCCTCTTCCCTCAGTTGTTCCTACTCAGACTGTTGCACTCTAAACCTA
GGGAGGTTGAAGAATGAGACCCTTAGGTTTTAACACGAATCCTGACACCACCATCT
ATAGGGTCCCAACTTGGTTATTGTAGGCAACCTTCCCTCTCTCCTTGGTGAAGAAC
ATCCCAAGCCAGAAAGAAGTTAACTACAGTGTTTTCCTTTGCACCGATCCCCACCC
CAATTCAATCCCGGAAGGGACTTACTTAGGAAACCCTTCTTTACTAGATATCCTGG
CCCCCTGGGCTTGTGAACACCTCCTAGCCACATCACTACAGTACAGTGAGTGACCC


CAGCCTCCTGCCTACCCCAAGATGCCCCTCCCCACCCTGACCGTGCTAACTGTGTG
TACATATATATTCTACATATATGTATATTAAAACTGCACTGCCATGTCTGCCCTTT
TTTGTGGTGTCTAGCATTAACTTATTGTCTAGGCCAAAGCGGGGGTGGGAGGGGAA
TGCCACAG

Sequence ID 399

TTTTGGCATTACTTAATCCAATTATAAAAACTGAATTTTTAAAAAACAGCACTTGT
TTTTTCTTCCAAGATTAATTTGAATTTTTTTATGGACATTAGAAAACATTGCAGTT
TAGTCATAATCAAAAATAAATCTTGAGGCTGGTAGAGCAGCTTTGTTGCTGTTTAT
ATTTTTATTGCTTACTGGATTTCAGTGTTACCTAGTGCCATCAGTTTGGTATTTTG
CCACCTTGCACATTCAGTGATGTTTGATTTTTCTTTTTCCTTTTTTTCATATTACT
TTTAAATCCTGAATAGTTTGTGGCAGCTGGAGATCACCTAGTCCACCACTGTCCAA
CATGGCAATGGTAAGTAATATTGAGTAAAGAATAGAAAATTAGTAAAATGCATGGC
TTCAGAATTATAGCAATTTGCAAAATAGGTTAATGGATGAAAATTAGAATGACCAG
TTTAACTTTCCCCCCAGCAGATTCTTCTGTTAAACAATGCCCCTTCAAAATAAAGG
AAGAACAAGTGGGTGTTATACCTATGTTATTTGGCTATGTTAGCACAATATGATGG
ACTAATTTGAGAAAAAGCATTTACTTCCTTTACTATTACTTCTTTTCTTTATAGGG
CTAAGTCTGCCTTCTGGGTCTTTGAA


Sequence ID 400

GAAGAAGCGCGAAGAGCCGTTAGTCATGCCGGTGTGGTGGCGGCGGCGGAGACTGC
GGGCCCGTAGCTGGGCTCTGCGAGGTGCAAGAAAGCCTTTGAGGTGAAGGTGTATG
AAAGTCATCATAACAGATGTTTTCCAAAAACTTGTAGAAGGTTGTGAAAAAACTAC
TAGGATCACGCGGCATGTATTGAGCATATAGGTTGCTGTAGATGAATGTTCTTAGC
TGTCATGTTTAAAAATACTTCTGCTTCGTTACCTCAAGTGTGGCATGCAGCATTTT
GGAAGGAAAATTGAAGACGTGTTCAAGAAAACATGAACAGAAGCAAATGATGAAAA
TGAGCATTTTACTTGATGTTGATAACATCACAATAAATTATGGAGAAAAATACATA
TTTGGCTAACTTTTAATTGCTGAACAATAAAGTGTTTTCTTTTAAATCNAAAAA


Sequence ID 401

GAAGCCAAACCAAAGGGAGCTTCTACTTCATGATGCCATTTATGTAAAGTTCAGGC
AGAGAAAATCAGTGGTTTAAGAAGTTAGAATAATGATTATCTTTGGAGGGATTGCA
ACTGGAAGAAGTCATGATTGGGATTTCTGGGTCCTAATAGTGCTCTGTGTCTTGAT
CTGAGTGCCGACTACATGAGTGGTTAGGTTTGCAAAATTCATTGAGTTATGCACTT
AATGGTGTTGTCTTATTAGAGCTGATGGAGGAGAGAGGGCTTCAATTTGCACAACT
GAGTAATCAGCTAGGCCCAGTCACTAGGTGAACAACTTACTGCTACCAATCAGCCT
TAGAGCAGGAATCAAACTCATGTCTCAGAAAAGTTATTAATTCAGCTTGTCTTGGG

ACTTCCTTCAGAGTCACTCTTGAATAGCTGAAATAGTAAATGTTAAATCTGTGGAT
GCAAGTGTGTAAATTATTTTAGTCATCAGCTCTAATAAGATGGCCTTTGGGGAAAT
GAGTATAAGGTCACGAAAATGAAATGGCAAGAAGGAGGTCTACTATTTCTTCTGTA
ATACTGATTTTTACCCCATCAGGGTCAGTCCCCAAAGGTTGTAAATGTGAAGCTTG
GTCTTTTTCTTTA


Sequence ID 402
GACCCTATTCTCAGGATGAAAATAATACACTAGTAATAGTCTGCTCTGTTGGTTAA
CTCCTCGTAAGGAGGTACAATTAAAATGCTGTAGTGTTGCAAGGGAAGGAGAGGAA
GAATCATATTCCTTCACTAGCAGGATCAAGAAAGCTTTTATAGAAATATACAAAAT
CTTCACTTCTTGAAGGATTGGTAAAATTTAATAGCCAACATTGGGCACTTATTCAT
TCTCTGAGTAAATATTTATTGCATGCTTATCTTGTATCAACATTGNGATGAAAGCN
CAAGAATGAAAGAGGAGGGAGAATGTTTANAGAATAAGGCTGAAACACAGATTTTG
TAGGGAGCGTAGGGGAGACTGANAAAACAG


Sequence ID 403
AAGACACCTGATAGATTGTCTTGTATTATTTTTCCTTTGCCTTCTTACAATCTCAG
TGATTAGAATTGGGCTGAAAACAATACATCAAATTCTCAGCAAAATCCTTATGGGT
TGCTGGATACCGAGGGTTTTTAAGATCTTTAGACTTCACTATATAGAACAAATGTT
GAATGGGAATTTTCTTTATTTCTATANCGTTTNG


Sequence ID 405
CCCGGAATCGCGGCCGCGTCGACGATGAGCATTTTTTCATGTGTCTTTTGGCTGCA
TAAATGTCTTCTTTTGAGAAGTGTCGGTTCATATCCTTTGCCCACTTTTTGATGGG
GTTGTTTTTTTCTTGTAAATTTGTTTGAGTTCATTGTAGATTCTGGATATTAGCCC
TTTGTCAGATGAGTAGGTTGCGAAAATTTTCTCCCATTTTGTAGGTTGCCTGTTCA
CTCTGATGGTAGTTTCATTTGCTGTGCAGAAGCTCTTTAGTTTAATTAGATCCCAT
TTGTCAATTTTGGCTTTTGTTGCCATTGCTTTTGGTGTTTTAGACTTGAAGTCCTT
GCCCATGCCTATGTCCTGAATGGTAATGCCTAGGTTTTCTTCTAGGGTTTTGATGG
TTTTAGGTCTAACGTTTCAGTCTTTAATCCATCTTTTAAAAGTCTCTTCACAGTAC
ATGAGTAGTAGTGACACCAATAATGTCAGAGCAGGGAACTCCCAGGTTCTGCCCAT
CCACAAAAACAACAAATAAGCTGGCAAAAACTTTAAGAATCAACTTTTGCAGATCT
CTGAAATCTAGTCAAAACTTAAACAGAGGAAAGATTAATAAAGACNGGCTGCCTGA
GATAACACTAACACACAC

Sequence ID 406

CATCAAATAAATAAATAAATAAATTTTAAAAGTCACAGCATTGAATTTTTAAATGT

TTGGGATGATAAAGCACCTGCTTATCATGAAGCTANAGAAATTCAATGACACGTTT
GCCAGGGTCTTTGCTAGTGATGTTGGAACAAGTCTGTAATGCTGATGAAACATCAC
TGTTCGGGCATTATTGCCCCAGAAAGACACTGACTGCAGCTGATGAAACAGCCCTT
CCAAGAATTAAGGATGCCAAAGACCAAATAACTGTGCTGAGATATACTTACGCAGC
AGGCATGCATAAGTGTAAACTTGCTGTTATAAGCAAAAGCTTGCGTTCTCACTGTT
TTCAAGGAGTGAATTTCATACCAATCCATTATTATGCTAATAAAAAGGCATGGATC
ACCAGGGACATCTTTTCAGATTGGTTTCACAAACATTTTGTACCAGCAGCTTGTGC
TTACTGCAGGGAAGCTGACTGGATGATGACTGCAAGATTTTGTTATATCTTAACAA
CTGTTGTGCTCATCCTCCAGCTGAAATTCTCATCAAAAATAATGTTTATGGCTCAC
ACCTGTAATCTCAACACTTTGGGAGGATTGCCTGACCCAGGAGTTCAAGCCCACCC
TGGGCAACACAGCAAGACCCAACCTNTC

Sequence ID 407

TTTTAAAAATCATAAAACGTTTCTTACAAAAGAGCATTACATTNTGCACACTGCTC
TGAACAGATGCCAGGGACATGTGGACTATTGTTACTTTTCCTCCCTGTCCCACCCC
CCAAATGTTACAGTGACCACAAAGCAAGGTGTTCACAATAATTACATGGGGGGAAT
TTTTTAAACCACCAACAATAACGAAAAATAAAATCCACTCACTCTGCTGCTGTTTC
AAAATTTCAATGTTAGTTTTTGCACGCCCTTCCCCCCCCCAACCCTGTTTGTAAGG
AACTAAAACATTACATCTGGTGAACAGCAAAGATTTCACTACACCTCAAATGCAGA
ACACCTATGAAGCAGAGGAATGTTGGCTTTTTAAACAGAAGCAGATAAAAAAAAAA
GATGCAGGACTCCTTCAGTTCTTCACTAGTCTTAGAAAAACTTTCCAGAATACTGC
TTCACACTATAAAAAAGAAAAAATATCTTGCATTAGAATCCTTCAACATCTGCATA
CTGCTTCACACTGTTCGTTTCTAGGAGCACTTTGTCACAGGACACTTCTGCTTATA
TTTCTTTAATCAGAACTTAGTTGGATGGGCCGGGCATGGTGGCTCACGCCTGTAAT
CCCAGCACTTTGGGAGGCCGAGG-GGGTGGATCACC

Sequence ID 408

CCATCTCCAAATTTAGTATTCATTCTGTTTAGCATATTATCAGTTGCCATCTATTT
GTTTTAACTGATTACTTGAATCTGATTAAACATCACAGAAATGGGCTTTGATAAGA
ACAATATTGAATAAGAAATTTTAAATAACAAAACAGCTTATAGAAAAATTCAGCAT
AACTTTTCCATCACCTTCACCACCCTTGCCTTTTATTATCCTGTCCTGTATCACTG
CTTTCTGTTAGCAGTGTTGTGTGAGTTAGGATTTGGGCAGGAAAGCAAAAGCAACC
ACCCGTCATTTTCCCAGAATGAAGGGTTTGACGTAGGATGTAGACTTTGTATAGTA
GTTGGGAGAGCTGTGGGAGTGAAGGTCAGGGATGTCACCTACAGAAGTCAGGGAAT
CTGCCACCAGAGATCCTGCATCAGAAACAGCCAACAGCGTGCTTCTGAAGAACTAG
TGGGGAAGTGGCTATAATTCTTAGGAATCCCAGCAAGTCCGCACCACTGTCTCAGT
CTACAGCAGTGGAGAAAGGGGTTTCCAGGAGCTCTCTGGAAAGTTCCTGCCCACAC


TTTGCAACAATCTTCAGAGGATAATGGGCTTCTCTTCCAGCTTCCACACCCAACAA
GAGTGCCTTTCATCGGCCAACTCTAACCTGGAACCCTATGGCAGAGGGGATTTAGG
AGACAGTTTGTNATGTCTGTGGAATGCAAATGAANANGTANCAATGCTTANTTGAC
AGCGGNCATACACAAATNTNGAAA



Sequence ID 409

GATCCGTNGACT



Sequence ID 410

CTCTTCCCAGCCCCTGAGCCCAGCCCCTTCCCAAGTGGTGCCAGACAAAAAACTAC
ATGGCCCTTTCGTGTCTTGGGGGTGGAAAGGGAGGGATGAATTGGGGTGATAGAAC
CCTGGTGAATTCAGAGTAATCTTTCTTTAGAAAACTGGTGTTTTCTAAAGAAACAG
GATAGGAGTTTAGAGAAGGCACCAAAGCTTTCACTTTGGTTTGGCACCAGTTTCTA
ACCATCTGTTTTTTCTACCCTAGCTATCTTTTATTGGTAAAATATAAATGTATAAT
TATGTTTGTAGAGCTTTACCAAGGAGTTTCCCTCCTTTTTTGTTTGTTGATTAGCA
AATTTTTGATTCTCCATTTTCCAAAAGTAAGAGACTCCAGCATGGCCTTCTGTTTG
CCCCGCAGTAAAGTAACTTCCATATAAAATGGTATTTGAAAGTGAGAGTTCATGAC
AACAGACCGTTTTCCATTTCATCTGTATTTTATCTCCGTGACTCCACTTGTGGGTT
T

Sequence ID - 411             nt:     505

```
TGGAGCTGAAAAATTCCTATTACCTAGGGGCATCACAACGCATTGCATTTCGCCCG
TGTTTGGGATGATGCTGGTGTAAACCTACTATGCTGCCAGTCATGTAAAAGTATAG
CACACACAATTAGTAGGTAATGCTTGCAAATAATAATGAAAGACTCTGCTACTGGT
TTATGTATTTACTATGCTATACTTTTTGTCATTACTTTAGAGTGTACTCCTACTTT
TTTTTTTTTTTTTTTTTGAGATGGAGTTTCACTCTTGTCCTGTAGGCTGGAGCGAAN
TGGCGCGATCTCGGCTTACTGCAACCTCCACCTCCTGGGTTCAAGCGATTCTCCTG
CCTCANCTTCCCAGAGTAGCTGAGATTACAGGCATGCACCGCCACGCACGGGTAAT
TTTGTATTTTTGGTAGAGACAGGGTTTCACCATGTTGGCCAGGCTGGTCACCAACT
CCTGACCTCAGGTGACCCGCCTCCTCACCTCCAGAGTGTTGGGATTACAGGNGTGA
G
```

Sequence ID 412

```
ATAAAAATTAGCTGGGGGTGATGGGCCCTGTACCCCAGCTACTCGGGAGGTGAGGT
AGGAGAATCACTTGAACCCGGGAGATGGAGGTTGCAGTGAGCCAAGATCGTGCCAC
TGCACTCCAGCCTGTGTGACAGAACAAGACTCTGTCTCAAAAAAAAATAATAATAA
TAATAATAATAAAAAGGAATAACATAGCTAGGAATAAATTTAATCAAAGAGGTGAA
```

```
AGACTTATACACTTAAAACTACAAAAAAAAAATCACTGAAGGAATTATAGACCCAA
ATAAAAATAAATAAAAAGACATTCTGTGTTTTAGGGAAAGAAGACTTAATATTGTT
AAGATGTCAATACTACCCAAAGTGATCTACAGATTCAACATAATCCCTATCAAAAT
TCCAACAGCCTACTTTGTAGAAATGGAAAAGCCAATTTTCAAATTCAGATGGAATT
GCGAGGGGTTCTGAATAACAAAACACAATCTTGGGGAAAAAAAACAAAAAACAAAG
TCAAAGAACTCACACTTCTCTATTTATAATTTACTACAAAGTTATAGNATCAAAGT
CGACGCGCCGCGATCCGGGC
```

Sequence ID 413

CACAGTACTCCATTTTGGGGTCCAAACTGTAATGCTCAAAATAATAAATGCTTACA
CGAAAATTATTTATTGAGAATATTCATATAAAAATTACCTAAAGCAAAGTAAAAAA
AGTAAAATCAAGGTGGTATATTTGAAGTGAATGGTGATTGGAAATTTTTAGCTGTA
ACAAAAAGAAAGAAACAACTTTTTTTAAAGCCTCATTCTCTTTTCTTTCAAAATG
TACCTTATTCCCACACACTCTTGGGCTGACCTTTATTTTATCAATAAGCTCAATAT
TACTTTGTTTAAAATAAGATGCTTCAGCAAAAGTCATTCTCTCTTTAACCATATAA
TTTAAAAACTCCTCTTCACGATTGATAGCAAAATCAGAAACGTTAGGGCACCAGTG
AGTTGAAAAAACTGGTCTTAAGTTGGAAAAACTATTATTAATAATATTATCCTATC
CATCCATATCTATTGAAATTGTCAGGTCCATAATTTCATTTTAATTAATTATAGGA
AAGAAGAAAAGATAATACCCATTTGTTCTAT


Sequence ID 414

CTCAGACTCTTTCTGCCCTAATGGCCATTACTATCCAGTCTGTATTGCTACAAGGG
ACCCACTGGTACCCCTTTTAGATTCTATCAAAAGGAACAGGGTTTTCCTAGAGGCA
GGCAGCCTGGTGGTATGGCACAGCAGAAGCTTACTGCTAATGAAATGGGAACCTCC
CCCTCCCTTGTGGTTTCAGCACAGAACCTGAATGCCAGGAAAAATTCCTGGGCCAA
GAAGCTAAAGCTAAAGAAACCTTCCTTTTTTCAACGTTTTTTTTTCTTTCAAACTG
TAGGGTCACTTTTGATTGAGGCAAAGGGGTCCTACTGTAAGTGGAAAAGACTCACT
CCCCTAACATAAGTTTTCACTGTGGTGGGATGGTGCCGCCCGATATGCTTGATATG
CTTTTCCTTCCACATGTTAAGCTAGGAAACCTAACAGGATGTCAGCAGGGCAGTTA
ACTCTGGACTCANAGCCCTCAAGGGCATGTGGCANAACCTCATGGCATNCAAGACC
A


Sequence ID - 415                               nt:      596

GTATAATTGATTCTTTTGAACCTAAAGTATAAGACTTCACGATTAGAAAAAAATTA
TCCAAAGACTAATGTAATTAAGTGAGGAAAAGGTGCTGGAGGAACTGGATAACCAC
ATGGAAATGTATGAACCATGACCTCTATGTCACATACTATATATAAAACTTAATTT
GAGGTGTATCACAGAGCTAACTGTGGGGGCTAAAACGTTGAAGCCTTTGGATGGCC

GCACAAGAGATGTCTGCATTCATAACCTTGGGGAGGGTATGAACATTTCTTGGTAA
CATGCAAAAAGCACTAACTGTAAAAGAGAACAGTTGGTCAGTTGAATTTCATGAAA
CATTGTAAACTTCTGCTAAACAACTGACACCATTAAGAATGTGGAAAAAGGCTGGG
CACAGTGGCTCATGCCTATAATCCCAGCATTTTGGGAGGCCGGGGCGGGAGAATCA
CTTGAGGCCAGGAGTTTGAAACCAGCCTGGGCAACATGGCAAGACCCCGACTCTAC
AAAAATATTTTTAAAAATTAGTTGGGTGTGGTGATGCACTCCTGTAGTCCTAGCTG
CCAGGANGCTAAGGNGGAAGGATCACTTAACCCTGG


Sequence ID 416

CTGGTGGCGGCGGTCGTGCGGACGCAAACATGCAGATCTTTGTGAAGACCCTCACT
GGCAAAACCATCACCCTTGAGGTCGAGCCCAGTGACACCATTGAGAATGTCAAAGC
CAAAATTCAAGACAAGGAGGGTATCCCACCTGACCAGCAGCGTCTGATATTTGCCG
GCAAACAGCTGGAGGATGGCCGCACTCTCTCAGACTACAACATCCAGAAAGAGTCC
ACCCTGCACCTGGTGTTGCGCCTGCGAGGTGGCATTATTGAGCCTTCTCTCCGCCA
GCTTGCCCAGAAATACAACTGCGACAAGATGATCTGCCGCAAGTGCTATGCTCGCC
TTCACCCTCGTGCTGTCAACTGCCGCAAGAAGAAGTGTGGTCACACCAACAACCTG
CGTCCCAAGAAGAAGGTCAAATAAGGTTGTTCTTTCCTTGAAGGGCAGCCTCCTGC
CCAGGCCCCGTGGCCCTGGAGCCTCAATAAAGTGTCCCTTTCATTGACTGGAGCAG


Sequence ID 417

GCAGGGGCTTCTGCTGAGGGGGCAGGCGGAGCTTGAGGAAACCCGCAGATAAGTTT
TTTTCTCTTTGAAAGATAGAGATTAATACAACTACTTAAAAAATATAGTCAATAGG
TTACTAAGATATTGCTTAGCGTTAAGTTTTTAACGTAATTTTAATAGCTTAAGATT
TTAAGAGAAAATATGAAGACTTAGAAGAGTAGCATGAGGAAGGAAAAGATAAAAGG
TTTCTAAAACATGACGGAGGTTGAGATGAAGCTTCTTCATGGAGTAAAAAATGTAT
TTAAAAGAAAATTGAGAGAAAGGACTACAGAGCCCCGAATTAATACCAATAGAAGG
GCAATGCTTTTAGATTAAAATGAAGGTGACTTAAACAGCTTAAAGTTTAGTTTAAA
AGTTGTAGGTGATTAAAATAATTTGAAGGCGATCTTTTAAAAAGAGATTAAACCGA
AGGTGATTAAAAGACCTTGAAATCCATGACGCAGGGAGAATTGCGTCATTTAAAGC
CTAGTTAACGCATTTACTAAACGCAGACCAAAATGGAAAGATTAATTGGGAGTGGT
AGGA

Sequence ID 418

CCCGGAATCGCGGCCGCGTCGACGGGAGGTGATAGCATTGCTTTCGTGTAAATTAT
GTAATGCAAAATTTTTTTAATCTTCGCCTTAATACTTTTTTATTTTGTTTTATTTT
GAATGATGAGCCTTCGTGCCCCCCCTTCCCCCTTTTTTGTCCCCCAACTTGAGATG
TATGAAGGCTTTTGGTCTCCCTGGGAGTGGGTGGAGGCAGCCAGGGCTTACCTGTA

CACTGACTTGAGACCAGTTGAATAAAAGTGCACACCTTATAAAAAA


Sequence ID 419

CCCGGAATCGCGGCCGCGTCGACGGGAGGTGATAGCATTGCTTTCGTGTAAATTAT
GTAATGCAAAATTTTTTTAATCTTCGCCTTAATACTTTTTTATTTTGTTTTATTTT
GAATGATGAGCCTTCGTGCCCCCCCTTCCCCCTTTTTTGTCCCCCAACTTGAGATG
TATGAAGGCTTTTGGTCTCCCTGGGAGTGGGTGGAGGCAGCCAGGGCTTACCTGTA
CACTGACTTGAGACCAGTTGAATAAAAGTGCACACCTTATAAAA


Sequence ID 420

CTTCATTTGAAATGGTTGAATCTGCTGTGTAATAAAGTGGTTCAACCATGATTAGG
AACTGAAATTTAGTAGAAGAGGGAAAAGGAGTTAATGTAACAAATTATTTTAGCTA
CAAACCCCGGTAATAGAGCACTTGGGGGATGGGATGGGGTGGGTTGGTGAGACAAT
CAGAATGGTAAATTGATTAAATGCTCCTAACCCTGTAATTTTGTGCATAGAGCACC
CTATGCTGTGGAAATAACTGTTCTTAGATTTCATTGTAACTGGACTGTTCAGGTTG
CCCAGAGGGAAAGAACATTCCTAATTCTAATAAAATAAACTTTTATTTTGTTTA

Sequence ID 421

```
TGTCATTGAATCTGCTTGTTACTTAAATGCTAAACTCAATTCTGTAATTCAATAGG
TGCACCTCTCTGAGAAACATAAGAGACAATGAGGAAAAGGATTCAGCATTCCGTGG
AATTTGTACCATGATCAGTGTGAATCCCAGTGGCGTAATCCAAGTAAGATGTTCAC
AAAGATTTGTTTTTAATGTCTAATTAATAAAATTTTAAAGGAAGAAACATTCTAAT
ACTTTAATTATAAAAAGTTAACTATTTTCAAAGGTATCAAAATACAGTTAAACCTT
TAAAATGTATATTTCTTAATATCTTGAAATTGTAATGCCTTTTTTTTTTCCTAAAT
TTTTTTTGTCATGAAATGAGATAGTAACAGCAGATTGGGACAACAAGGTTATATTC
TTGTCTTGAATCAGGCCATGGCTTCTTTCATCCAAATTTCAGACCTCATTTATTTA
CTTTGTCCCTGCCTCCCATCCCTGGATATCAGTTTGTGGATATCTACAGTTAATAG
AGTGACCAAATAGTAGGAATACTGTCTCTCTATTCTGAATAAAATCTTTGAATCAG
ATTTAGAAATAATGAATAAAATACAAATCAGCCATTGAAATTGCTCTAATTTTGAG
AGCTTATGATTTATTCATCTTTGGTTTCCAAGTTCAAGTTATATGTAGACATTTTA
ATT
```

Sequence ID 422

```
GCTTCCTAGGTGAGGTCACGAGGAAACCTGCTGGCCAAGTGACCTGGCAGGGTGTG
GCCAGTGTGGCCAGGGCCGCCGAGCCTGCTTTCCTTCCCTGCAGCAGGAACCCTTC
TGGGGCTGTGATCCTGCGATGGTGCCTGGGTGGGAGTGGGGGTGGGGGGCGGGATG
GTCTCCCTACCTGCCAGCTTCTTGGTTTGAGGTGAGGACAGCCCCGGAAGCTCANA
```

```
CTTGGCTCCTGTCCATGTACTTGGGGCCATGAGCTCTGCAGGGACCTTGGAAAGAN
AGAGACGGGTGGTGTANGGCANGGGAAGGCATTGTCTTCAAACAGGAAAAAGCTGA
NAATGGAAACAGGCGAAACTTACCAAGTGTAACATCACCTGGAACTGAAGGAGGGT
GGGAAGGTTTTAATTATTTTAAAAATAGAGATGGGGTCTCACTATGTTGCCCAGGC
TGGTCTCAAACTACTGGGCTCAAGTGAACCTCCTTCT
```

Sequence ID - 423                              nt:        387
```
TGTTTCTCNAGGGCGAGAGGCTGTCTTANAGCACCATTCTCTGGCCCTNGTCCCAT
GAGAAGGAACCGCACTCAGGAGCCACACTCTCCCACTNCCCTTGCCCANAAGACTC
ACAGAGGGCACGGAGCTGGCTGTGGTGAGAGGAGGTCCANCAAATTCCTGTCTGCA
NAAGGGTTCTGAACACCACCGCCTGGCAGCGTGCTGGAGGAGGGATTCCTCTTTTC
CTCACAGCAATTCTGACCAGAAACCTGTCAAATCAGGAATGGCTAAAATAAGACCA
GGGTATGAATGACCATCAGCCACAGTAAAACCAAGGCACAGCTCTCCTGAGCCCAC
CCAAGCTGCTGTGGCCCAGACTGGTGACATCACCTCAGGGCAAAAAAAAA
```

Sequence ID - 424                               nt:        420

CGCAGAATGGCTCCCGCAAAGAAGGGTGGCGAGAAGAAAAAGGGCCGTTCTGCCAT
CAACGAAGTGGTAACCCGAGAATACACCATCAACATTCACAAGCGCATCCATGGAG
TGGGCTTCAAGAAGCGTGCACCTCGGGCACTCAAAGAGATTCGGAAATTTGCCATG
AAGGAGATGGGAACTCCAGATGTGCGCATTGACACCAGGCTCAACAAAGCTGTCTG
GGCCAAAGGAATAAGGAATGTGCCATACCGAATCCGTGTGCGGCTGTCCAGAAAAC
GTAATGAGGATGAAGATTCACCAAATAAGCTATATACTTTGGTTACCTATGTACCT
GTTACCACTTTCAAAAATCTACAGACAGTCAATGTGGATGAGAACTAATCGCTGAT
CGTCAGATCAAATAAAGTTATAAAATTG


Sequence ID 425

GGAAACTGATGCCAGTCAGAAACTCAGATCAAATGAAGGGGTGAAGAGAACCAGAA
TTGATCTCTCTGTAGGAGAATATAAATGACTTTTTTAAAGTACATATTTTCTGTGA
AAGACAGTTTTTTGTTTAATGCAAAAATGTTAACAATGTTTATATCATGTAGAAGT
AAAAGATCGTGAAACAGCACAGAGAACAGTAGTAAGACAGATTGAATTGCACTGTT
GTAAGATGATGAACTTACAATATTAAGTGAAGGTAGACTGTGATAGATTAAGGATA
TATATTGTAATCCCTAGAGCAATTGTCAAAGTGGTACAGGTAAAAAGCCAATAGAG
GTGATAAAATGGAATACTAAAAAATATCAGATGAATAATAAAGAAGACAGGAAATG
AGGAACAGTGGAACAGAATGAATAAAAAACAAGACCATTAACTTAATCATTAATAA
TTACTTTAAATGGGTTAAACATTATGGTTATAAGGCAGAGATTTTCAGACTAGATA
AAAGAGCAAGCTCCACTATATACTGTCTACAAGAGATATACTTTAAAGTGTATATT
ATATTTAAATATAAAGATTTGGAATAAATAAACCTAAGAATAAGCTTACTAGGGAA


GTGAAAGATCTGTACAACAAGAATTACAAAACACTGCTGAACGAAATCATAGGTGA
CCA

Sequence ID 426

GTCCCGGAATCGCGGCCGCGTCGACGTTTCCTCAAAATTTATCTTCCTGTTAATGT
CAGGCATGTATCTCCTTAGCTTGCCACAAATAACTATATATACCACAGACCTTCCT
TTGTAGGGCTAACAGTGTTGCATTGTAAGTGGAGGCCTCATAGATACCTGGCCTTT
TCCTACCTTATTCCAAAGATGGTTGCATCTTATAAATAATGTCATTCTTCAGCAAA
TGGTATGGAAATGAGATTGTAATGTCATTATTTCCTCTTTAAATAATCAGGACAAC
TCATGATACAAAGAGCTCTTCTCTATAAAAGGTGGGACTTTTTTTTTTAGTAATAG
CAAAAATAAAATTGTACCTCCTTAATCTTCTACAGAAAGATGGATTTCATTTTCAA
CATTAAGAGGTAGTTTTAAGAAGCAGTAGAAGTCAGCCTGGGCAGCATGGTGAAAC
CCCGTCTCTACAAAAAGTTAGCTGGGCTTAGTAGTTGCAATCCCAGCTACTCTGG
AGGCTGAGGTTGGAGATCATCTGANCCTGGGGAGGTCNAGGCTGCAATGATACANT
GAGCCCTGATTGTGCCACTCCACCTGGTTGCAGA

Sequence ID 427

TTCCAATCTTCGTGTTCACTTTAAGAACACTCGTGAAACTGCTCAGGCCATCAAGG
GTATGCATATACGAAAAGCCACGAAGTATCTGAAAGATGTCACTTTACAGAAACAG
TGTGTACCATTCCGACGTTACAATGGTGGAGTTGGCAGGTGTGCGCAGGCCAAGCA
ATGGGGCTGGACACAAGGTCGGTGGCCCAAAAAGAGTGCTGAATTTTTGCTGCACA
TGCTTAAAAACGCAGAGAGTAATGCTGAACTTAAGGGTTTAGATGTAGATTCTCTG
GTCATTGAGCATATCCAAGTGAACAAAGCACCTAAGATGCGCCGCCGGACCTACAG
AGCTCATGGTCGGATTAACCCATACATGAGCTCTCCCTGCCACATTGAGATGATCC
TTACGGAAAAGGAACAGATTGTTCCTAAACCAGAAGAGGAGGTTGCCCAGAAGAAA
AAGATATCCCAGAAGAAACTGAAGAAACAAAAACTTATGGCACGGGAGTAAATTCA
GCATTAAAATAAATGTAATTAAAAGG

Sequence ID 428

TGCAGGATCCGTCGACTCTAGATAACATGGCTAGAAAAGAGAATGAAAAAGTTGGA
ATTTTTAATTGCCATGGTATGGGGGGTAATCAGGTTTTCTCTTATACTGCCAACAA
AGAAATTAGAACAGATGACCTTTGCTTGGATGTTTCCAAACTTAATGGCCCAGTTA
CAATGCTCAAATGCCACCACCTAAAAGGCAACCAACTCTGGGAGTATGACCCAGTG
AAATTAACCCTGCAGCATGTGAACAGTAATCAGTGCCTGGATAAAGCCACAGAAGA
GGATAGCCAGGTGCCCAGCATTAGAGACTGCAATGGAAGTCGGTCCCAGCAGTGGC
TTCTTCGAAACGTCACCCTGCCAGAAATATTCTGAGACCAAATTT

Sequence ID - 429                         nt:      535
CACAGTACTCCATTTTGGGGTCCAAACTGTAATGCTCAAAATAATAAATGCTTACA
CGAAAATTATTTATTGAGAATATTCATATAAAAATTACCTAAAGCAAAGTAAAAAA
AGTAAAATCAAGGTGGTATATTTGAAGTGAATGGTGATTGGAAATTTTTAGCTGTA
ACAAAAAGAAAGAAACAACTTTTTTTAAAGCCTCATTCTCTTTTCTTTCAAAATG
TACCTTATTCCCACACACTCTTGGGCTGACCTTTATTTTATCAATAAGCTCAATAT
TACTTTGTTTAAAATAAGATGCTTCAGCAAAAGTCATTCTCTCTTTAACCATATAA
TTTAAAAACTCCTCTTCACGATTGATAGCAAAATCAGAAACGTTAGGGCACCAGTG
AGTTGAAAAAACTGGTCTTAAGTTGGAAAAACTATTATTAATAATATTATCCTATC
CATCCATATCTATTGAAATTGTCAGGTCCATAATTTCATTTTAATTAATTATAGGA
AAGAAGAAAGATAATACCCATTTGTTCTAT


Sequence ID 430
CAGGGGCTTCTGCTGAGGGGGCAGGCGGAGCTTGAGGAAACCGCAGATAAGTTTTT
TTCTCTTTGAAAGATAGAGATTAATACAACTACTTAAAAAATATAGTCAATAGGTT
ACTAAGATATTGCTTAGCGTTAAGTTTTTAACGTAATTTTAATAGCTTAAGATTTT
AAGAGAAAATATGAAGACTTAGAAGAGTAGCATGAGGAAGGAAAAGATAAAAGGTT
TCTAAAACATGACGGAGGTTGAGATGAAGCTTCTTCATGGAGTAAAAAATGTATTT
AAAAGAAAATTGAGAGAAAGGACTACAGAGCCCCGAATTAATACCAATAGAAGGGC
AATGCTTTTAGATTAAAATGAAGGTGACTTAAACAGCTTAAAGTTTAGTTTAAAAG
TTGTAGGTGATTAAAATAATTTGAAGGCGATCTTTTAAAAAGAGATTAAACCGAAG
GTGATTAAAAGACCTTGAAATCCATGACGCAGGGAGAATTGCGTCATTTAAAGCCT
AGTTAACGCATTTACTAAACGCAGACGAAAATGGAAAGATTAATTGGGAGTGGTAG
GATGAAACAATTTGGAGAAGATAGAAGTTTGAAGTGGAAAACTGGAAGACAGAAGT
ACC


Sequence ID 431
CGCTGGGTGCCTGCAGCGCCTCCCTTGTCTCATATGGTGTGTCCAGCACTCTATTG
TTGTAAACTGTTGNTTTGNCTGACCTAAATTNTCTTTACTAAACANATTTAATAGT
TNAAAAAAAAAAAANANCA

Sequence ID 432

TTTTAAAGTCATCTCTATAGGAAGGTGCTGGGCAGGGATCCCAGAGAAAGAAAGGG
TCCAAGACTCCATTAACTGCCCTGGATGAAGGGCACTGCTACAGCAGCTAGTACCA
GAGACTCTCCTATCTCACGGTTGAGGCAGACCCAGGATAGAATAGAGAATAAAAGG
AATGCTTATAGGAAACAATTTTGTATGGAATGCTAGATGGCCAAGCCTCAGCCTTT
GGTCCAGTGCAACCCTTGCCTCGCTTGTCAACAGTGAAAAATTAGTTTGGTTAGAA

GAACCATCTGGAAACACACCAGCTTCTGCTACCTTCATGCTCATTGTTAAAAAAAG
ATTAACCAGTGTGAACATTCTGATCTGTTAATTCCAGGGACTGTTTTCTTTCCAAT
GGACTGTTTGTTGGTAGAATAACCCCCAAAAGCTCAAAGCTAAAATGCATCATCAG
TCCTAGTCGGCAGTTCCTTAAGAATGGACTGGCGGCGTGGTTGAGCTGATATGGAA
AAGCTGCACCTTCCTGCAGAAGATCAACTGACCTGCTATCCCACCCCAAATTTCAA
CCTGAGGTATATTTCAATGAAGGCAGGTAGCTGTGCTTCTCAGAGCA

Sequence ID 433

TCCCGGAATCGCGGCCGCGTCGACCCGCCGCCGAGGATTCAGCAGCCTCCCCCTTG
AGCCCCCTCGCTTCCCGACGTTCCGTTCCCCCCTGCCCGCCTTCTCCCGCCACCGC
CGCCGCCGCCTTCCGCAGGCCGTTTCCACCGAGGAAAAGGAATCGTATCGTATGTC
CGCTATCCAGAACCTCCACTCTTTCGACCCCTTTGCTGATGCAAGTAAGGGTGATG
ACCTGCTTCCTGCTGGCACTGAGGATTATATCCATATAAGAATTCAACAGAGAAAC
GGCAGGAAGACCCTTACTACTGTCCAAGGGATCGCTGATGATTACGATAAAAAGAA
ACTAGTGAAGGCGTTTAAGAAAAAGTTTGCCTGCAATGGTACTGTAATTGAGCATC
CGGAATATGGAGAAGTAATTCAGCTACAGGGTGACCAACGCAAGAACATATGCCAG
TTCCTCGTAGAGATTGGACTGGCTAAGGACGATCAGCTGAAGGTTCATGGGTTTTA
AGTGCTTGTGGCTCACTGAAGCTTAAGTGAGGATTTCCTTGCAATGAGTAGAATTT
CCCTTCCTCCCTTGTCACAGGTTTAAAAACCTCACAGCTTGTATAATGTAACCATT
TGGGGTCCGCTTTTAACTTGGACTAGTGTAACTNCTTCATGCAATAAACTGAAAAG
ACCATGCTGCTANTC

Sequence ID 434

TTCGGACGCAAGAAGACAGCGACAGCTGTGGCGCACTGCAAACGCGGCAATGGTCT
CATCAAGGTGAACGGGCGGCCCCTGGAGATGATTGAGCCGCGCACGCTACAGTACA
AGCTGCTGGAGCCAGTTCTGCTTCTCGGCAAGGAGCGATTTGCTGGTGTAGACATC
CGTGTCCGTGTAAAGGGTGGTGGTCACGTGGCCCANATTTATGCTATCCGTCAGTC
CATCTCCAAAGCCCTGGTGGCCTATTACCANAAATATGTGGATGAGGCTTCCAAGA
AGGAGATCAAAGACATCCTCATCCAGTATGACCGGACCCTGCTGGTAGCTGACCCT
CGTCGCTGCGAGTCCAAAAAGTTTGGAGGCCCTGGTGCCCGCGCTCGCTACCAGAA
ATCCTACCGATAAGCCCATCGTGACTCAAAACTCACTTGTATAATAAACAGTTTTT
GAGGGATTTTAAAA

Sequence ID 435

CTGCAATGTGCAATAGTTGCACCACTGCACTCCAGCCTGGGTGACAGAGTGAGAAC
CTATCTCTTAAAAAAAAAAAAAAAAAAAAAAGGAAGAAGAGACATGAGAGGGCCCAAGT
CACTTGCTCACTCACTTTCCGTGTACATGTACCAAGAAAGGCCATGTGGGAAAGA

GCAAGAAGGCAGCCGCCTTCAAGACAGGAAGAGAGCCCTCACCAGAAACTGAGCCA
GAACCTTGGAATTCCAGCCTCCANAACTGTGAGAAAAGAATTTTCTGTTGTTTCAG
TCCCCCACACTATGGCATTTTGTTACGGCAGCCTGAGCTAATACTCCTACTTTGTC
CTGCATTTACTTGGTCTTCCAGTTAGTTTTTTAGACTTTGGGAATCAGAGCAGTCA
GTTGTCAGATTTTAGCTTACAGTTGTCCTACCTGTGCAACTGAAATTTCTTCCATT
TTAAACCAGAGCAGAGTTTTAGAGTCAAAAGAAACCAGATCTTTTAGTGCAGAAGC
TTTCCACTGTATTANAAGTGAGGAAGTTGGT

Sequence ID 436

AAAAAAACTCCAGAGAAGTTTATAGAAAGAGATGACATGTAAACCCTGCTGAAAAA
TAGTTTCATTTGTTAGAATATAATTGTCTTCCACTAAAAAAAGAAAAAAAAAAGCA
TTTAAGGCTCTAAGATCTCTTGAAGTACCACTTTTCCTGAATCCCAGAGTTTTTAT
GTGCATTATTTTTATGCGTTTGTAGTTTGATATGTTGTATTTATAAGTAGTTTTAG
CTTTCCATTATGAATTCTTCTTTGACCCATGAGTTATTTAGGTAAGTGTTTAAAAA
TTTACAATAGTTTATATATGCAAATATTATGTTGTTAGAGTTGGTTTTCATGTCAT
TTTTACATATACAGGGGCAGTTTCCCCAACTAAATTGTATATTCCTTAAAGCAGCA
CTCTTAAATTTTATTTCTGTGTCAATTTCTTGNCTGTGTTTCCTGGCATGGAATAC
ATGGCATAAAATTTGTTATGTAATTAAATGAAATATTATTATACTTTCTATTTTTT
AGAAAAAA

Sequence ID - 438                                    nt:      577

GTCGACAGGGATGACATAACTATTAGTGGCAGGTTAGTTGTTGGTCACTTTCAACT
CTGGGTTCAAGCGATTCTCCTACCTCAGCCTCCCGAGTAGCTGGGATTACAGGCAT
GCACCGCCACACCTAATTTTCTATTCTTAGTAGAGACGGGGTTTCTCCCTGTTGGT
CAGGCTGGTCTCGAACTCCCGACCTCAGGTGATCTGCCTGCCTCAGTCTCCCAAAG
TCCTGGAACCACAGACATGAGCCACCACGCCTGGCCCCTTTTAAAATATTTCTGCT
CATTGATGATGCACCCAGTCACCCAAGTGCTCTGATGGAGATGTATAAGGAGATGA
ATGCTGTTTTCATGGCTGCTAATACAACATTCATTCTGCAACCCCCAAATCAAGAA
GTAATTTTGACTTTCAAGTCTTATTATTTAAGAAATATATTTTGCAAGACTATAGC
TGCCATAGACCGTGATTCCTCTGATGGATCAGACAAACTAAAATGAAAACCTCCTG
CAACGTATTCATCATTCTAGATCCCTGAGGAATCGCCACACTGACTTNCACAATGG
GTGAACTGGGTTACAGT

Sequence ID - 441                                    nt:      552

AAACAAAATTATTCTCTGAGAGGGAAAGGACATTTGAGGGAAACATCAAATTTCCC
CATAAATAAATGAATGGAGTTTGCAGGAAGGTGAGGGTGAGCAGAGATGTGTGTGG

133

ACATCTCTGACCATCCATCGCTGTATTCAAATGGATTGTTTTATTCCATTCTGGTC
TCAGGCATGACCACGTCCAGTGAAGACATTTGAGGCAGCACATCTCAGGACCCAGG
CAATAGACTGGCCCCAACTCAGGCTGGACTAAGGTGTGATTAATTCTTTGTTTTTT
GTGTGGAACAGCTCACCTTGTCAGACAGCCTCAGGGCATCTCTGAGACACAGGGGC
AGAAAATGACATTCATCTTTTGAGTCCTCATCCATGGAGTGCTGTGTTTGGGGGGC
TGCATCTGCTGAAGCGAGAACCCCATTCTGCCACCCCACCAGGATGCCCATTCTCC
AGGACTTCTCCAACTTACTATTAGACTAAACCAGAACAAGCAACAAACTGTATTTA
TGCAAGCAAAATTGATGAGAAATTATATTCAAATAAAGCAAAAATTA


Sequence ID - 442                           nt:      606
TCGTGCCACTGCACTCCAGCCTGGACGACAGAGTGAGACTCCATCTCAAAATAAAT
AAATAAATAAATAAATAAATAAATAAATAAAAAAATAAAAAATACTTCTGCTATGA
AAAACCTAGTTGGTATTTTTGCTTATTTAATACTATAGAAATATGGTGATCTCATC
TTTAATAGAGTGCTTTTAAGGTCCCCAGTGATAATCTCCTAAAATCATGAACTTTA
AGAATTTATAATGTTAATATGAGGAAATGAAATCTGGATTATCTCACCACATATTA
TATAATTCATTAGTGACAGAGCAAGAACTCCAGGTCACCTGTCTATTCCATGTTTT
TCCTATCTGCCTTTAAATGTTGAGATACTACCCTTATCTCATGTGAATGGAGAAAC
TGCCTAAAATGCTAAAACTGACTCAGAGGCACCCAGACATAAGTGAAGTGTGATTA
GAAAATCCTGGTCAGTTGAGTCTTAGCCAAATGTGTACCTACTGTGTCTGCCTCTA
TCAAGTCAATGAAAACATGATCTGAGAACTGTAAGTCCATTTATGGAAAGGGTTGA
TTTANAGATATTTTGAACTTNCAGTGATGAGCCCCTTCTCAAATAG


Sequence ID 446
CGGACTCCTGTGCTAATTGTCAGCTTACATATCATTGTATAGAGACTGTTTATTCT
GTACCAAACTGATTTCAAAAGTACTACATNGAAAATAAACCGGTGACTGTTTTTCT
TCATAAAGTTCTGCGTTTGGCATCTTCACTCTTTCCAAAATGTATCTGTACATCAN
AAATGTCACTATTCCAAGTGTCTTTTTAGTGTGGCTTTAGTATGGCTTCCTTTTAA
TATTGNACATACATTGNATCTTTGTTTTATGGNAATAAGTAATAAAAATGTAGACT
TCATATTTTGTACAAAATGTCCTATGTACAGAATAAAAAAGTTCATAGAAACAGCC
NANAA

Sequence ID 447

AGGCCGAGGCAGGCAGATCNCNTGAGGTCAAGAGTTTGAGACCAGCNTAGCTAACA
TGGTGAAACCCCATCTCTACAAAAATATA-AAAATTAGCCTGG-GTGGTGATGGGC
ACCTGTAACCCCAGCTACTCGGGAGGCTGAGGTAGGAGAATCACTTGAACCCGGGA
GATGGAGGTTGCAGTGAGCCAAGATCGTGCCACTGCACTCCAGCCTGTGTGACAGA
ACAAGACTCTGTCTCAAAAAAAAATAATAATAATAATAATAATAAAAGGAATAAC


ATAGCTAGGAATAAATTTAATCAAAGAGGTGAAAGACTTATACACTTAAAACTACA
AAAAAAAAATCACTGAAGGAATTATAGACCCAAATAAAAATAAATAAAAGACATT
CTGTGTTTTAGGGAAAGAAGACTTAATATTGTTAAGATGTCAATACTACCCAAAGT
GATCTACAGATTCAACATAATCCCTATCAAAATTCCAACAGCCTACTTTGTAGAAA
TGGAAAAGCCAATTTTCAAATTCAGATGGAATTGCGAGGGGTTNTGAATAACAAAA
CACNATCTTGGGGAAAAAAAACAAAAAACAAAGTCAAAGAACTCACACTTCTNTAT
TTATAAATTTACTACAAAGTTATAGTAATCNAA


Sequence ID - 448                     nt:      329
TACGCACACGAGAACATGCCTCTCGCAAAGGATCTCCTTCATCCCTCTCCAGAAGA
GGAGAAGAGGAAACACAAGAAGAAACGCCTGGTGCAGAGCCCCAATTCCTACTTCA
TGGATGTGAAATGCCCAGGATGCTATAAAATCACCACGGTCTTTAGCCATGCACAA
ACGGTAGTTTTGTGTGTTGGCTGCTCCACTGTCCTCTGCCAGCCTACAGGAGGAAA
AGCAAGGCTTACAGAAGGATGTTCCTTCAGGAGGAAGCAGCACTAAAAGCACTCTG
AGTCAAGATGAGTGGGAAACCATCTCAATAAACACATTTTGGGTTAAAA


Sequence ID 450

GAGCAGTGGCATGATCACACCTTACTGCGGCCTCCAACCCCTGAGCTTAAGTGATT
CTCCCGCATTATCCTCCTGAGTAGCTGAGACTACAGGTGCATGCCACCATACACTA
CTAAATTTGGGTCGGGTGGTGGTGGTGATTTTTTAATATTTTTGTAGAGACAGGGT
CTCACTGTGATGCCCAGGCTGGTCTTGAACTCCTGGGCTCAAGCAGTCACCCACCT
CAGCCTCCCAAAGCACTGGGATTACAGGTGTGAGCCACCACACTGGCCAGCTTTGT
TTTGTTTTGATGACTAAGCTGCTCTTGCTAAAAGGGCTTCTCTCTGAACTTCCCTA
CCTTTCTTCTGTTTCCCTGGGCTAGGGCTCCATGTTGGCAGTCCTACTCCCAATTA
ACCTGGGGCTGTCTGGTTAACCTTTATAAGATCTGCAGTCATTGGGAGACCCGGGG
ACCAGGAATATTGTTGTTGAGGGAGCTACCCTGGAAAGTGGATGGGTGGCCAAAGG


135

Sequence ID 452

TTTGGCTTTGCCTCTAGGCATTAGATGTTATCTTTGGAGGCATCCTTCTATGAGCA
TTCATTTTTGGACCAAGCCTGGATTTACAATTCTATTACTGGCCCAGACTTCATTT
CTATCCAATTTCATTCCACTGTGCTATAGTTTACAACATATAATTTGACTTATAAA
TAATTCCTGACTATGGGTTTAAAGACTGAAAATGGATCAATAGAAACTTTGAAAAT
GTTAACATCTTGATTGCTTTTCTCAGTGTAGAAATGGACAATGTTTAGCTTAAAAA
CTGCATGTTTTTAATGAGATACGGGGTTGAAAGACTTATTCCTGGAATTTATTGTT
CTGGAGAAAGCCTGTTGCTATCTGCCATACCTTGGTTTACTTTGTGCAAAATGAGC
TTCTTTTTAAGTAATGAGCTCTTTCCATGTTCAGCTTAAATTGCTGTCTTAGACAC
TTCATCAGGGTTCCCTGCTCTGCCTCATTCCCCCTTTTGCTCACTTGCAGCCTTTG

ACATAATCCTGGGAGGCAATTGGCATCATACATATTTTGCTTTGTAATCTCCTGCT
TTGATTCTGACTGGGACCCAGC

Sequence ID - 453                                    nt:      747

GGATCTAAGACCAGCCTGGCAGCCACCAGATGGTGATTCTAGTCCTGGCTCAGTCA
GTAATAGGTCACTGACCCCAGAGAAATCAATTCAGCCTCCCCAGGTCCTTGGATTT
CTTTCTGTGAAAATGAAAGCATAGGTAGGAATTTCCCATGGAACAGCTAGCAGAGG
AGAAATATTAAAAGTCAGGAGACTCATGCTATAGTTTTCATACTTCATTACAACAA
TGTTGTTTAGGACAAGTGAGTTAACCTGTTAGCTTCCTCTATATAAAATGGAAAGT
CATTAAAAACCTACATAGCAGGGTTCTTGTGAAGATCAAGTGATAATGTAGGAAGC
ATGTACAAATGTCACATTCTGCCGTCACGTAATGGTCCTCACAGCTTGAGGTAGCA
TTTAGCATGTGTCATGATTTAGTACAAGGGTTGGCAAACTGTTGCTCTTGGATTAA
GTCTGGCTCATTGCCTGTTTTTTCAAAGAAAAAAATTGTATATGTGTGTATATATGT
TATATATAGGTACACACACATATGTGCTATATATAGCATATATACACACATAATAT
ATAAACATGTACATATATAGCATTATATATATACCGTGTATAATATCTCCAGTCCT
CATGACCAGCCATGCTTGTTCATTTACATTTGCATACTCTATGATTGCTTTCATGC
AACAATGGCAGAGTTGAGTGATTGTTTTGCACAGANACTGTATGGCCCACTAAACC
TAAAATATTAATCTCTGCC

Sequence ID 454

CTCCTGCCGGGCTCGTGGCGGCTTCTGTCCGCTCCGCGGAGGGAAGCGCCTTCCCC
ACAGGACATCAATGCAAGCTTGAATAAGAAAAACAAATTCTTCCTCCTAAGCCATG
GCATATCAGTTATACAGAAATACTACTTTGGGAAACAGTCTTCAGGAGAGCCTAGA
TGAGCTCATACAGTCTCAACAGATCACCCCCCAACTTGCCCTTCAAGTTCTACTTC
AGTTTGATAAGGCTATAAATGCAGCACTGGCTCAGAGGGTCAGGAACAGAGTCAAT
TTCAGGGGCTCTCTAAATACGTACAGATTCTGCGATAATGTGTGGACTTTTGTACT
GAATGATGTTGAATTCAGAGAGGTGACAGAACTTATTAAAGTGGATAAAGTGAAAA
TTGTAGCCTGTGATGGTAAAAATACTGGCTCCAATACTACAGAATGAATAGAAAAA
ATATGACTTTTTTACACCATCTTCTGTTATTCATTGCTTTTGAAGAGAAGCATAGA
AGAGACTTTTTATTTATT


Sequence ID - 458                    nt:       682

TGCCACTGAAGATCCTGGTGTCGCCATGGGCCGCCGCCCCGCCCGTTGTTACCGGT
ATTGTAAGAACAAGCCGTACCCAAAGTCTCGCTTCTGCCGAGGTGTCCCTGATGCC
AAGATTCGCATTTTTGACCTGGGGCGGAAAAAGGCAAAAGTGGATGAGTTTCCGCT
TTGTGGCCACATGGTGTCAGATGAATATGAGCAGCTGTCCTCTGAAGCCCTGGAGG
CTGCCCGAATTTGTGCCAATAAGTACATGGTAAAAGTTGTGGCAAAGATGGCTTC


CATATCCGGGTGCGGCTCCACCCCTTCCACGTCATCCGCATCAACAAGATGTTGTC
CTGTGCTGGGGCTGACAGGCTCCAAACAGGCATGCGAGGTGCCTTTGGAAAGCCCC
AGGGCACTGTGGCCAGGGTTCACATTGGCCAAGTTATCATGTCCATCCGCACCAAG
CTGCAGAACAAGGAGCATGTGATTGAGGCCCTGCGCAGGGCCAAGTTCAAGTTTCT
GGCCGCAGAAGATCCACATCTCAAAGAAGTGGGGCTTCACCAAGTTCAATGCTGAT
GAATTTGAAGACATGGTGGCTGAAAAGCGGCTCATCCCANATGGCTGTGGGGTCAA
GTACATCCCCAATCGTGGCCCTCTGGACAAGTGGCGGCCCTGCACTCATGAAGGCT
TTCAATGTGC

137

Sequence ID 459

TCCCGGAATCGCGGCCGCGTCGACCTTGTCCTTGAGCGTCAACCTTCTTTCCCTGA
AGTGGCTGGGGTTCCTGTTTCCTTCTTTGATTGACAACTTGTGTTAACCCTCGCAC
ATCTCTGGGCCAATTTTTGCTTGTAAGTCTTTCCGGAGACCCCTGGAATTTAAATC
ATTAGCACCGCGCCCTTCCCCGAAGAGTCTTCGAAGGGTTGCCGCTTTTCGGTGGC
GCAGTTCTCGCGAGAAGGTGACTTTCTTTCTCGGTATTTCCTGGTTTCCAGAATCC
TTAGCGCGAGGCGGAAAAAATATTTCTCCCAGCTTGTGTTGATGCCGCGATTTTGA
CTGAGACTTCTTCCCACGATTTCTGTTTTTGCTTCTCCAAGGAAAATGGCAGCTCC
CGAGCAGCCGCTTGCGATATCAAGGGGATGCACGAGCTCCTCCTCGCTTTCCCCGC
CTCGGGGCGACCGAACCCTTCTGGTCAGGCACCTGCCGGCTGAGCTTACTGCTGAG
GAGAAAGAGGACTTGCTGAAGTACTTCGGGGCTCAGTCTGTGCGGGTCCTGTCAGA
TAAGGGGCGACTGAAACATACAGCTTTTGCCACATTCCCTAATGAAAAAGCAGCTN
TAAAGGCATTGACAAACTNCATCAACTGAAACTTTTAGTCATACTTTAATCG


Sequence ID - 460                         nt:      536
CAGAGATCAAAATAGGCCTTACACAGTGCGACGCGAATTTAAAAGATTACCCCATT
CAGGTGTATGGATTTTGCAGTATTAAAGATGCTGCCTGGAATAGGTCATTATCTTC
TCCAAGTACTCTGTTAAGTCAATGAGTCACATAGAGTATAAGGTTTATTATCTGCT
TTTCTTTCATTAAATAAATCTTTATTGAATTTCTACTACATTAAAAAACCAAACCA
AAACAAAACAAACAAAAAAACACTTCCCTGAGCCATAAAGGAGAAGGTAGTTTTG
ACTGGAACCTTGAAGGATGGGTAAACTTTCAGCAGATAAAGATTGAGAGAAGACCT
TCCAGGTAGAGAAAGCAGTGTGGGCACAGGCAAAGATGGAAGAACACACGTGGCTG
TGGGAAACACAGCTAGAAGCCAGTGCGGATAGAGAGTAGGCTATGATGTGCAAAGG
TTANACACTGGGAGAGACAGGTCCATGAGAGTAGCTTGGACTAACACAGGGAGGGT
TTGGAATCCCAACTGGGGAACCTANAAATCAA


Sequence ID 461

TAGGAGGCTTATTCACTGATTTCCCCTATTCTCAGGCTACACCCTAGACCAAACCT

ACGCCAAAATCCATTTCACTATCATATTCATCGGCGTAAATCTAACTTTCTTCCCA
CAACACTTTCTCGGCCTATCCGGAATGCCCCGACGTTACTCGGACTACCCCGATGC
ATACACCACATGAAACATCCTATCATCTGTAGGCTCATTCATTTCTCTAACAGCAG
TAATATTAATAATTTTCATGATTTGAGAAGCCTTCGCTTCGAAGCGAAAAGTCCTA
ATAGTAGAAGAACCCTCCATAAACCTGGAGTGACTATATGGATGCCCCCCACCCTA
CCACACATTCGAAGAACCCGTATACATAAAAT

Sequence ID 462

TCTTTATCAAGTTGAGAAAGTTCCTCCCCTCTATTCCTAGTTTGCTAAGAGTCCTT
CTATCCTATTTCTTAATGGTTTAGTAGATGACTCTGTGGTACTTTGAAGGTTGTTT
GCAGAATTTCCATGCCATAGGCAATTTACCTTTCCTTGACATTTGAAGGATTGATG
TTGGTGCCAAGTATAGAATCTTCACAGAGTCCTCCTGTAGCTTCTAAAGGTTTAGC
TTGAAAATGTTAATTGCTTAACGCTAGTAAGTGAGTGAAAAGCTGGGGATAAATT
TTGTATCTTGCTTATATTTCAGTTCCCACCTCTGTCCNGACNAAACCCCCATATAT
AA

Sequence ID 463

TAGTTTACATATCCCAACCTTTAAAAATATTCCTCTTATTAGCTTTATATTCACTT
TATAGAAGTTGAGTTTTAATTAAAATTCTTGGCATCCTGAAGTATGTCACATAGCA
TGTGCTCCTTATAAATATGTTGATATCTCAGAAGACAGCATCCCGGTTTTCATTTT
ATAAAGTACCATACTTAAGAATGCTGTAATACTTATCTTTTATAACATGTTTCCTT
CGCTTTGCTTGNCTTTTATGNCATCAGTTTTAACTGTTTACTTCATTTAACAGNTT
ACATCATNCAACAGTTTACTTCATTAAACAGTAGGTGGAAAAATAGATGCCAGTCT
ATGAAAATCTTCCCATCTATATCAAAATACTTTCAAGGATATACTTT

Sequence ID - 464        nt:     615

CGACTTTCAACCATCAAGTGAGGAATACCTTCACATAACTGAGCCTCCCTCTTTAT

CTCCTGACACAAAATTAGAACCTTCAGAAGATGATGGTAAACCTGAGTTATTAGAA

GAAATGGAAGCTTCTCCCACAGAACTTATTGCTGTGGAAGGAACTGAGATTCTCCA

AGATTTCCAAAACAAAACCTATGGTCAAGTTTCTGGAGAAGCAATCAAGATGTTTC

CCACCATTAAAACACCTGAGGCTGGAACTGTTATTACAACTGCCGATGAAATTGAA

TTAGAAGGTGCTACACAGTGGCCACACTCTACTTCTGCTTCTGCCACCTATGGGGT

CGAGGCAGGTGTGGTGCCTTGGCTAAGTCCACAGACTTCTGAGAGGCCCACGCTTT

CTTCTTCTCCAGAAATAAACCCTGAAACTCAAGCAGCTTTAATCAGAGGGCAGGAT

TCCACGATAGCAGCATCAGAACAGCAAGTGGCAGCGAGAATTCTTGATTCCAATGA

TCAGGCAACAGTAAACCCTGTGGAATTTAATACTGAGGGTGCAACACCCCATTTTC

CCTTCTGGAGACTTCTAATGAAACANATTTCCTGATTGGCATTAATGAANAGTCA

Sequence ID 469

GATTTTTAAAAATACATATAGCAAAAATATTACAGGGTCAGGGGAGACAATTAGAA

TGATATAATTCAAAGTGGATTAAAAAAAAAACTGTCACCCAGAATACAATACCCAG

CAAAGTTGTCCTTCATAAATGAAAGAAAAATNAAATCTTTNCCNAACNA

Sequence ID 471

TCCCGGGAATCTGCAGGATCCGTCGACT

Sequence ID 472

GACAGTGCCCAGGGCTCTGATATGTCTNTCACANCTTGNAAAGTGTGAGACAGCTG

CCTTGTGTGGGACTGAAAGGCAAGATTTGTTCCTGCCCTTCCCTTTGTGACTTGAA

GAACCCTGACTTTGTTTCTGCAAAGGCACCTGCATGTGTCTGTGTTCTTGTAGGCA

TAATGTGAGGAGGTGGGGANACCACCCCACCCCCATGTCCACCATGACCCTCTTNC

CACNCTNACCTGTGCTCCCTCCCCAATCATNTTT

Sequence ID - 473                                      nt:        694

TGGGCTTTGGGCTGGCTGCAGTCTGTCTGAGGGCGGCCGAAGTGGCTGGCTCATTT
AAGATGAGGCTTCTGCTGCTTCTCCTAGNGGCGGCGTCTGCGATGGTCCGGAGCGA
GGCCTCGGCCAATCTGGGCGGCGTGCCCAGCAAGAGATTAAAGATGCAGTACGCCA
CGGGGCCGCTGCTCAAGTTCCAGATTTGTGTTTCCTGAGGTTATAGGCGGGTGTTT
GAGGAGTACATGCGGGTTATTAGCCAGCGGTACCCAGACATCCGCATTGAAGGAGA
GAATTACCTCCCTCAACCAATATATAGACACATAGCATCTTTCCTGTCAGTCTTCA
AACTAGTATTAATAGGCTTAATAATTGTTGGCAAGGATCCTTTTGCTTTCTTTGGC
ATGCAAGCTCCTAGCATCTGGCAGTGGGGCCAAGAAAATAAGGTTTATGCATGTAT
GATGGTTTTCTTCTTGAGCAACATGATTGAGAACCAGTGTATGTCAACAGGTGCAT
TTGAGATAACTTTAAATGATGTACCTGTGTGGTCTAAGCTGGAATCTGGTCACCTT
CCATCCATGCAACAACTTGTTCAAATTCTTGACAATGAAATGAAACTCAATGTGCA
TATGGGATTCAATCCCCACCATCGATCATAGCACCCCCTATCAGCACTGNAAACTC
TTTTGCATTAAGGGATCATTGC

Sequence ID 474

GGCAGCGCGGGGAGCCCGTCGGCGCCGGCGGGCGGGCCGGTTTCGAAGTTGATGCA
ATCGGTTTAAACATGGCTGAACGCGTGTGTACACGGGACTGACGCAACCCACGTGT
AACTGTCAGCCGGGCCCTGAGTAATCGCTTAAAGATGTTCCTACGGGCTTGTTGCT
GTTGATGTTTTGTTTTGTTTTGTTTTTTGGTCTTTTTTTGTATTATAAAAAATAAT
CTATTTCTATGAGAAAAGAGGCGTCTGTATATTTTGGGAATCTTTTCCGTTTCAAG
CATTAAGAACACTTTTAATAAACTTTTTTTTGATAATGGTTAAAAAAAAAAAAAAAA

A

Sequence ID 475

CATAATAAAAAACAATCAACAAACAGGGAATGGAAAGAAACTTCCTCAGCATGGTG
AAGGCCACATATGAAAATCCCACAGCTAACATCATACTCAATGATGAAAGACTGAA
AGCTTTTCTCCTGAGATCAGGAACAAGACAAAGATGTCACCTTTTGTCACTTCTAT
TCAACTCATTATTGGAAGTTTTTGCCAGAGCAATTAGGTAAG

Sequence ID - 476                    nt:      476

CAGAATCTTTTCATAGGCTGAATGTTGCTCCACAATGTGTCCTTTGACTATCTCTG
GCTAATTATTATTTTAATCTCTTCTCAGCTTTTCCAAGAACATAACGTTAACCAAA
GATCTTAGGCCATTCACAACTCTTTTGTAAAAATTAATGTGGATGTGAAACGAGGC
AACAAATCCTGAAGTAGAAAGTTATTCCTGGCCAGGCACGGTGGCTCACGCCTGTA
ATCCTGGCACTTTGGGAGGCCGAGGTGGGTGGATCATGAGGACAGGAGATCGAGAC
CATCCTGGCCAACATGATGAAACCCCATCTCTACTAAAATACAAAAAATTAGCTGG
GCATGGTGACGCGTGCCTGTAGTCCCAGTTACTCGGGAGGCTGAGGCAGGGGAATT
GCTTGAACCTCGGAGGTGGGAGGTTGCAGTGTGCCGAGATCACGCTACTGCACTCC
AGCCTGGCAACAGAGCAAGACTCCATCT


Sequence ID 477

AAACAGAAAGTTTCTTCTAAAGGCATGATTCAGTTAAGTCATTCTTAAGTGTTAAA
AAATTGTGAAAAATGTGCCTGTAATCCCAACACTTTGGGAGGCCGAGGCAGGCAGA
TCACGAGGTCAGGAGATCAAGACCATCCTGGCTAACAAGGTGAAACCCCGTCTCTA
CGAAAAATACCAAAAACATTAGCCGGGCGTGGTTGTGGGCGCCTGTAGTCCCAGCT
ACTTGAGAGGCTGAGGCAGGAGAATG


Sequence ID 478

TTCTTGGGATATTGATGACTACTGTCTGAGAGGTGCTGTGGGGAGATTTTCAGGAT
TGTGTGGTCTTTGAGGGGGGTGTTTTTTTAAGACAACATTGACCACTGTCCACTGT
CCACATGATCATTGTAAAATTGCAATGCCGCATGCTAGTTGGTTACATAAGACATA
ATTCCAGTGATTGAAGGTGGTTACACTGTATGGTGGTGTGTTCAAGATGGCACTGG
CATCTTTGAGCAGAGCCTGGCTATGCAGCATCATTTGAGTTTTTTAAACACCCTAN
AGGTCTGGTTGTTGTTGCTGTTGTCCTTTCCTGTGAAAGTCACAANANAAGTTACA
GTCCAGGTGAACCTGGAGTTTATAGGTTGGTTTTGTTTCTGNTATATATATATATA
TATATATTTTTTTTTTTTTTTTAACATTTACCTGTAGTGCTGTAGCTGTTGATACTA
TCACCTGCATGCTATTTCTAGTGAGTGCTAAATACAGTATGGTCCAATGACAATAA


CAGCCCATGGTACTGCCAG

Sequence ID 479

CATCAGTCTGTTATCCATGCTGACTTTCCGAAGACTTGCAGCTACTGCATTGATAT
CTTTCCTGCCAATAAGCAAAGTGTTGAACACTTCACAAAATATTTTACTGAGGCAG
GCTTGAAAGAGCTTTCAGAATATGTTCGGAATCAGCAAACCATCGGAGCTCGTAAG
GAGCTCCAGAAAGAACTTCAAGAACAGATGTCCCGTGGTGATCCATTTAAGGATAT
AATTTTATATGTCAAGGAGGAGATGAAAAAAAACAACATCCCAGAGCCAGTTGTCA
TCGGAATAGTCTGGTCAAGTGTAATGAGCACTGTGGAATGGAACAAAAAAGAGGAG
CTTGTAGCAGAGCAAGCCATCAAGCACTTGAAGCAATACAGCCCTCTACTTGCTGC
CTTTACTACTCAAGGTCAGTCTGAGCTGACTCTGTTACTGAAGATTAGGGAGTATT
GCTATGACAACATTCATTTCATGAAAGCCTTCCANAAAA


Sequence ID 481

CACACTTTCATGATAAAAACAGAACCTAGGAATGAAAAGAAATTATAGCAACATAA
TAAAGACCATATATGAGAAGCCCACAGCTAACATACTGTATGGTGAAAAACTGAAA
GCTCTTCCTCTAAGATCAGGAACAAGGCAAGGATGCCCATTCTTGCCACTTCTATC
GAACGTAGTACTGGAAGCCCTAGCCAGAACAACTAGGCAATAGAAAGAAATTAAAG
GCATCCATNTCAGAAAGGAAGAANCAAAATGCTGTCTGTTTAANATGACA


Sequence ID 482

TTTCTATANAAAAAAATTTTTTAAAATAATTGTAAAGTTAGATTTAAAATTGTAAA
ATATAAAATCACAAAGGAATGTACCCAATAAAATGTAAATGCNCCATAAAAAAAAA
AAAAAAAAAAAAAAAAAA


Sequence ID 483

CGNTAACGTGCAATCCGCCGCACGCCAGCAAACTGGACAAACTCCGGGATCTCATC
GAAGCGATTGAGCACCAGTACCAGAGTAATACCGGACTGATGTAACGAGGCGAGTC
GCTCATCCAGCTTGCTGACGTGAGGCAACATCCAGGCCATCGAACGGNTCATCAAG
AATCAACAAGTCAGGCTCCGACATCAGCGCCTGACACAGCAGGGTTTTTCGCGTCT
CGCCAGTGGAAAGGTATTTAAAGCGTCNGTCGAGGAGGGCGGTAATACCGAACTGC
TGCGCCAGTTGCATGCAACGCGGTGCATCCTTTACTTCATCCTGAATGATCTCAGC
CGTAGTGCGTCCGGTGCCATCTTCGCCAGGGCCGAGCATATCGGTGTTATTCCGCT
GCCATTCGTCGCTGACGAGTTTTTGCAATTGCTCGAAGGAGAGACGAGTGATGTGG
GAAAACTGGCTTTGCCGTTCACCTTTCAAAAGCGGGAAGTTCCCCCGCCAGCGCGC
GGGCCAGGGCCCGAT

Sequence ID 484

TTTTTTTTTTTTATTCTATTAAAAAATGTTNNTGAAAAAAGATACTTAAATTTTAA
AGATAACTNAATTCCTAANGATTTAAAATAATCCAAGCAGAGATGAAAGANCAAAT
GCAAATGCNTAAAAAGACCCCANAGCATTGTTAGCAAAAAGCAAATATAGTTAGCC
AAGCATATATATNTCATAAAAGCAATAANAAGGCNTAAAGCAAGTTTGGGGAGAGC
TTATTTAAAACTTGTAAAAATCATTTGAATTTTTAAAAGTTTTCAAAC


Sequence ID - 485                                   nt:       551

TTTGGAACACAAAGTTCCCTTTTTTAGAAGAATAGGTATTGAGCCCTTGAGCGTGGG
TAGAAAGATAGAGACAGAGTGATTTGCAAAATAATGGAGGATCATATTTATATATG
AATTTTCACTTATTTGAACTTTCAGATATCANCTTNAAAANCTTTGGTTTAAGTAA
AGTNTNTTAATGAGACTCCTTGGATGAAAGTAACCAAAACCAGTAAAAATAAGGTA
ATAAGGATGTAATAGTTTCTTATGGACACTCAACAGCTAGAATGCAGTTAGTCTCA
GAAAAGAATTAGAACAAATAACTGGAAGGCCATCAGGAGTCCAAAACCATCACTCT
TTTATATTTTATATTTTATTTTTCTCTCTTCANATGAGCATTCTCTTTCTATGTCC
ATATGGTANAAGGCGGCAGCTCCATAGATTATGGCTTCAGATGTTACAGTTCCGCT
NAATGCAGGGACAGACTTGCTATCTTTCAGTCCCCTTACATATCCTGGGGAGAGAG
CAAATGATTGACTGGCTTGAGTCAGGTGCCCGTTCCCTTTCCAATCT


Sequence ID - 487 nt:224

GTTTGNTTGTGACCATCTGTACTTGTAATTTCTTTACNTTCATTGGTATGAAAAAT
ATGTTCTTAGAAGCANGAAAAAGAATTCAGNTTTGCTTTGTATACTAAATTAAATG
CTGTAATTTTGATAAAATGAAAAATCTGCTTTATTTGCAACAATTGGTTTCTTCCT
TGACGTCAGCCTCACTCTTGGACTTTGGTATTCAGCCNGNCACCCCTGGGAATTCC


Sequence ID - 488                                   nt:       349

GTGCCTCCCTGTGTGAGTAGCCTAAGGTGCATTGAAAAAGACTGGGATGTGTTTTA
TTTTTTTGTATTAGATAGCATTAACCTTACTGTTGAAGTATTTTTGGTGGAGTATT
AGTGACAAGCCATTGAGTCTTAAGCCTTACGGCTTCCTATAAAATCACTAATTTCG
TGTGTGTTTGTGTGTAGGTTACGTTATATATAGGATTCGTGTTCGCCGTGGTGGCC
GAAAACGCCCAGTTCCTAAGGGTGCAACTTACGGCAAGCCTGTCCATCATGGTGTT
AACCAGCTAAAGTTTGCTCGAAGCCTTCAGTCCGTTGCAGAGGANCGAGCTGGACN
CCCTGGGGGGCTC

Sequence ID 489

TTAACAGCTGCATAGAGTTTTAAAAGTACATTATATTTTGTCAGACAAGTAAAATA

TCTGTTTTTCACGCAAAAAAAGCCATGAAATACGTAATTTTTTAAAGACAAAAAAT

CATCTTTTGAGTTTGCTCTTTGGTTTTTCTTCATTCCTTTTGAGGATTGGGAAAAC

AGAAAGATTCTTTGATTTGGGTAATGAAGAGGTAATTTGGGACAGTGTGGTGGTAC

CAGGAAGAAAGAGGATTGGAAAGGCCAGTACTGTTTTAGTTGCTCGGCACTGTTGG

TTTTGTTTTAATGTGGTTGCCCTGTCCACTACATGGTTCTATCAGTAGTGTAATCC

ATTTTCAATGTAAAGCTCTTTTAGTTTTTGTCATAGACATAAATTAATATTTTGAG

AGGCATCCCTCACCTGTTCATTTCTTCTGTGTTGAAATGAAGTACTTAAAATTACC

GTTATACATGAACTTTGTGGACTGTAAGATTTGTTATATATGTTCAAATGCCTTTT

AGCTGGCTTTTTAATTAATATGCCTGTTTTGAGTGCTTAATACAATGTAATGNGGA

TTGTAAATCATACCTATTTTAAATCATTCCTTCCTGTATATTTGNACTCAGAGAGC

CTTATTTTATTCTTCCAGC

Sequence ID - 491                          nt:      382

TTTTCTTAGAACTTTATTTTTTCTGGCCAGGCGCAGTGGCTCACACCTGTAATCCC

AGCACTTTGGGAGGCCAAGGCAGGTCGATCACCTGAGGTCAGGAGCTCAAGACCAG

CCTGGCCAACATGGTGAAACCCTGTCTCTACTAAAAATACAAAAATTAGCTGGGCG

TGGTGGCGCATGCCTGTAATCCCANCTACTCAGGAGGCTGAGGCAGGAGAATTGTT

TGAACCCGGGAGGCGGAGGTTGCANTGAGCCGAGATTGCGCCACTGCACTCCAGCC

TGGGCAACAGAGCGAAACTCCATCTCAAAAAAAAAAAAAAAAAAACAACCTTTATTT

TTTCTGATTTTAAAAGTAATAACTAGTTTGTAGAAACATTAAAAGT

Sequence ID 492

ACCCTAAACATAACTTAAAATTTGTTNGGAATTTGAAAGTACAGAATTTTCCTGTA

ATTGAGACTNTTTAAACTTTTGTGGTTGGAGAAGGTATTCTATTTTTTGAAAATAT

CTGTAAGTTTTATCTAAATAGTAAACTCTAAGTATTCTTCCCCTTTACTTACAGCC

ACCCTGGGAATCTGAGACTAGAGAAAATAAAGTTTGTCTCTTGTTCTAAGGAGGGT

CTGGTTTAGAAATCTGATTTAGACATAGAAAAATTGCAAGAAGCTTGAGGTGATTG

GAAGATACGATTTTGTTATCAAAGNATGTTTCTGTTTTATAGATTTTATTCATCTA

CAACTCCTTATTAATATATTTAAGAAGTCATTAACCCACCATTGATTACTTGATAT

AAAAGGAGAANCGGTGGTAAAAGGTGAAATANAATTTTTAATTTTTTTTTTTTTAA

GTTTAGGATTTTTTTTTTAAATTCTAAGAGTTTCTGTCATTTGGGGACAATCAGAA

Sequence ID 493

TGGGAATCATAATTNGTTAACTGAAGCTNATAAGATGAGAGCATTCANAGAGAAAA
GAACGGAAAGATTGAATATCAGTTTCCCTTCTTTAAAAAAATTGTGGATATGTGAT
CTAGCTTCTTGAGCATCACAGTGACTGATTGGCTCGTGGTAATTGATCGCTATGCT
GACAATCTTATCTCCACCTATGTCATTCAATTTTCTAAGAGGCAAAATCCTTAATC
AGGAGGAGAGTTTAGCTCTAGCTAAATTTCCCTTGTCCAGCATGCTCCTGCTCCCC

CAACTTGTGGAAACAGCTAAAGGATTGGACTAGGAGCANAAGTTTGGAATGGTTAA
AATGTAGCAACATGTGTTTCCTGAAACAAAATTCCACTATAATAAAAAAAGCATTT
GAATGCTCCCTTGTAATTCTGTTGGAGCTTGTTGCCTTTTTTATGACACAACCATA
ATCAGTGATAGACAGTAGCATAAAGAAGCAAGAGCAAAGCAATTAAGTAATAATAG
CACTACAAAAATGTGTGCTGTACTTACCAAACACGACATTTATGAATTATTANATA
GGAATAAGGGGATGGT

Sequence ID 494

GACCCAGCCATCTAAATAAGTTRTACATGTTGCGTATTTTTTTGTTAGGGACTTAT
CTTCCGAAGAGGAAAGGTTTATGAAACCTAAAGTAACAATGATAGCTTGGAATCAA
AATGATAGCATTGTTGGCACAGCTGTGAATGATCATGTCCTCAAAGTGTGGAATTC
TTACACTGGACAACTGCTTCATAACTTAATGGGACATGCTGATGAAGTATTTGTTC
TGGAGACACATCCCTTTGATTCCAGAATTATGTTATCTGCAGGACATGATGGCAGC
ATATTTATATGGGATATTACAAAAGGTACCAAGATGAAACATTATTTTAATATGGT
AAGTGAAGTGAGATGTACCTTGATACATGCTTGATAATTTGTTTAGAGTATTTGGG
TTATGCGGCTTACCCAGAAATTGATCTGCTTGTTTTGGCAGTTTGTTTTTACAAAT
CAACATATTCAAAGCCTGCTAAATATTAGACAGCTACATGTATATACGTACATACA
TGAA

Sequence ID 495
TTTC

Sequence ID 496

```
CTCGCTGGCGGGAGGCCACGGGCTTTCCACAGCGCGGGGGAACGGGAGGCTGCAGG
ATGGTCAAGCTGACGGCGGAGCTGATCGAGCAGGCGGCGCAGTACACCAACGCGGT
GCGCGACCGGGAGCTGGACCTCCGGGGGTGATCTGGACCCTCTGGCATCTCTCAAA
TCGCTGACTTACCTAAGTATCCTAAGAAATCCGGTAACCAATAAGAAGCATTACAG
ATTGTATGTGATTTATAAAGTTCCGCAAGTCATAGTACTGGATTTCCAGAAAGTGA
AACTAAAATTTTAATCCAGGTGCTGGTTTGCCAACTGACAAAAGAAAGGTGGGCC
ATCTCCAGGGGATGTAAAAGCAATCAAGAATGCCATAGCAAATGCTTNAACTCTGG
CTGAAGTGGANAGGCTGAANGGGTTGCTGCAGTCTGGTC
```


Sequence ID 497

```
GAAGACCTCACATCTGAGAGCTCATCTGCGTTGGCATTCTGGAGAACGCCCTTTTG
TTTGTAACTGGATGTACTGTGGTAAAAGATTTACTCGAAGTGATGAATTACAGAGG
CACAGAAGAACACATACAGGTGAGAAGAAATTTGTTTGTCCAGAATGTTCAAAACG
```


```
CTTTATGANAAGTGACCACCTTGCCAAACATATTAAAACACACCAGAATAAAAAAG
GTATTCACTCTANCAGTACAGTGCTGGCATCTGTGGAAGCTGCGCGAGATGATACT
TTGATTACTGCAGGAGGAACAACGCTTATCCTTGCAAATATTCAACAAGGTTCTGT
TTCAGGGATAGGAACTGTTAATACTTCCGCCACCAGCAATCAAGATATCCTTACCA
ACACTGAAATACCTTTACAGCTTGTCACAGTTTCTGGAAATGAGACAATGGGAGTA
AATATTACACAAATACTTATTCATTGNGGTTATTTTTATACAGTAGTGAGAAGAAT
ATTGTTCCTAAGTTCTTAGATATCTTTTTTTGGATGTGCAAAAATTTTTGGATTGA
CAGTAACTTGGGTATACATGACACTGAAATGCCTTACTTTGGATGA
```

Sequence ID 499

TGCCTGCGGGCCAGGACCTCGCCCAGCCCATGTTCATCCAGTCAGCCAACCAGCCC

TCCGANGGGCAGGCCCCCCAGGTGACCGGCGACTGAGGGCCTGAGCTGGCAAGGCC

AAGGACACCCAACACAATTTTTGCCATACAGCCCCAGGCAATGGGCACAGCCTTCC

TCCCCANAGGACCCGGCCGACCTCAGCGCCTCCTGCAGGCTAGGACACTGGTGCAC

TACACCCCATGCCTGGGGGCCGAGATTCTCCAGCAGAAAGATGCAATATTTTTTGT

TTCCTTTTTTTCCATTTTTTTTCTCTAAGGAATCAATATTTCAATATGTTGAGTGTG

TGTCCAATGCTATGAAATTAAAATATTAAATAACATATTTATGGCATTTTCTTGAA

GAGTGTGGTTGAAGAAATATTTCTCCTTTTGTTTTTCTTTTTTTTTTGNTTGNTAC

TGCCACTTCTTTTTAGGAGCAAATCTCCCCAGGGGTGTACGGNATTTCTTGACTCT

GGGAACAGCTGCTACCCCCAAGACTTGCCACGTTGTTCTGCCCTCAAATGGAATTA

AGTG


Sequence ID - 500                              nt:        390

GGAATATGGTCAGGATCTTCTCCATACTGTCTTCAAGAATGGCAAGGTGACAAAAA

GCTATTCATTTGATGAAATAAGAAAAAATGCACAGCTGAATATTGAACTGGAAGCA

GCACATCATTAGGCTTTATGACTGGGTGTGTGTTGTGTGTATGTAATACATAATGT

TTATTGTACANATGTGTGGGGTTTGTGTTTTATGATACATTACAGCCAAATTATTT

GTTGGTTNATGGACATACTGCCCTTTCATTTTTTTCTTTTCCAGTGTTTAGGTGAT

CTCAAATTAAGAAATGCATTTAACCATGTAAAANATGANTGCTAAAGTCAGCTTTT

TAGGGCCCTTTGCCAATAGGTANTCATTCAATCTGGTATTGATCTTTTCACAAA


Sequence ID 502

ACCCGCCATCTTCCAGTAATTCGCCAAAATGACGAACACAAAGGGAAAGAGGAGAG

GCACCCGATATATGTTCTCTAGGCCTTTTANAAAACATGGAGTTGTTCCTTTGGCC

ACATATATGCGAATCTATAAGAAAGGTGATATTGTAGACATCAAGGGAATGGGTAC

TGTTCAAAAAGGAATGCCCCACAAGTGTTACCATGGCAAAACTGGAAGAGTCTACA

ATGTTACCCAGCATGCTGTTGGCATTGTTGTAAACAAACAAGTTAAGGGCAAGATT


CTTGCCAAGAGAATTAATGTGCGTATTGAGCACATTAAGCACTCTAAGAGCCGAGA

TAGCTTCCTGAAACGTGTGAAGGAAAATGATCAGAAAAAGAAAGAAGCCAAAGAGA

AAGGTACCTGGGTTCAACTAAAGCGCCAGCCTGCTCCACCCAGAGAAGCACACTTT

GTGAGAACCAATGGGAAGGAGCCTGAGCTGCTGGAACCTATTCCCTATGAATTCAT

GGCATAATAGGTGTTAAAAAAAAAAAATAAAGGACCTCTGGG

Sequence ID - 503                              nt:      109

ACATTTTCCGGNCCTTTTGCCATACACAGTTACAGAGATCAGTCAAATCCATACCA
CCACTGAGATCTCATTTATTGCCACAGATGCACAAAATAAATAACCCAAAATC


Sequence ID - 504                              nt:      374

CCAGCAACGACCCATACCTCAGACCCGACGGCCCGGAGCGGAGCGCGCCCTGCCCT
GGCGCAGCCAGAGCCGCCGGGTGCCCGCTGCAGTTTCTTGGGACATAGGAGCGCAA
AGAAGCTACAGCCTGGACTTACCACCACTAAACTGCGAGAGAAGCTAAACGTGTTT
ATTTTCCCTTAAATTATTTTTGTAATGGTAGCTTTTTCTACATCTTACTCCTGTTG
ATGCAGCTAAGGTACATTTGTAAAAAGAAAAAAAACCAGACTTTTCANACAAACCC
TTTGTATTGTANATAAGAGGAAAAGACTGAGCATGCTCACTTTTTTATATTAATTT
TTACAGTATTTGTAAGAATAAAGCANCATTTGAAATCG


Sequence ID 505

GTACAGGAGGTAAATTGGATACCCCATCTAAGGGGATCTGTGAGACCAGGTAGTTA
TTTGGAATGAAAGAGTAAGATATTAAACCAGCCAGCATGTCAACAGGTGGGTGATA
GTCTTGTTCTCACAGACAACAGATGGCCATCATCTTAAAACAACATTTATGTTAAC
CAGCAGATAAGGGACTCCTGCATTGTCAGTGGACTTTGAGCCTGAGTTTTTCTACT
TGCATAGGTGAAAGTGGACTGCAATGCTAGTATAAATGCCGTATGATGACTAGTAC
CCCTTAGGGAGCTCCAGTTTGCCTTCCTGGGGAACCACAGACCCCAAGTGTAATTT
CCTGAGGACAGCCCGACTTCT


Sequence ID 506

GTTACTGTGAGCCTGTCAGTAGTGGGTACCAATCTTTTGTGACATATTGTCATGCT
GAGGTGNGACACCTGCTGCACTCATCTGATGTAAAACCATCCCANAGCTGGCGAGA
GGATGGAGCTGGGTGGAAACTGCTTTGCACTATCGTTTGCTTGGTGTTTGTTTTTA
ACGCACAACTTGCTTGTACAGTAAACTGTCTTCTGTACTATTTAACTGTAAAATGG
AATTTTGACTGATTTGTTACAATAATATAACTCTGAGATGTGTGAAAAAAAAAAAA
AAAAAAAAAAAA

Sequence ID - 507                                        nt:      521

CTGCGGTGGAGCCGCCACCAAAATGCAGATTTTCGTGAAAACCCTTACGGGGAAGA
CCATCACCCTCGAGGTTGAACCCTCGGATACGATAGAAAATGTAAAGGCCAAGATC
CAGGATAAGGAAGGAATTCCTCCTGATCAGCAGAGACTGATCTTTGCTGGCAAGCA
GCTGGAAGATGGACGTACTTTGTCTGACTACAATATTCAAAAGGAGTCTACTCTTC
ATCTTGTGTTGAGACTTCGTGGTGGTGCTAAGAAAAGGAAGAAGAAGTCTTACACC
ACTCCCAAGAAGAATAAGCACAAGAGAAAGAAGGTTAAGCTGGCTGTCCTGAAATA
TTATAAGGTGGATGAGAATGGCAAAATTAGTCGCCTTCGTCGAGAGTGCCCTTCTG
ATGAATGTGGTGCTGGGGTGTTTATGGCAAGTCACTTTGACAGACATTATTGTGGC
AAATGTTGTCTGACTTACTGTTTCAACAAACCAGAAGACAAGTAACTGTATGAGTT
AATAAAAGACATGAACT


Sequence ID 508

AAGCTCATGATTTTAAATGTATTTTTTCTAATAAACTATACTCCCATTTAAAAATCA
CCAATACCTTAATGTTTCAATTATATAAGCTAATTAAAAATAAAGGCTGGGCGTGG
TGGCTCACTTTGGAAGACCGAGGCAGGCAGATCACCTGAGGTCAGGAGTTCGAGAC
CAGCCTGCCCAACATGGAGAAACCCCATCTCTACTAAAAATACAAAATTAGCCAGG
CATGGTGGCACATGCCCGTAATCCCAGCTACTGGGGAAGCTGAGGCAGGAGAATCA
CTTGAACCTGGGAGGCAGGGGCTGCAGTGAGCCGAGATCATGCCATTGCACTCCAG
TCTGGGCAACAATAGTGGAACTCCATCTCAAAAATAATAAAAAAAATAAAATAAAA
ATAAAATTCAAACCTAAAATAGATGCTCTACTTCAGGAGTGGGCAAATTAATCACC
TGCATCCTTTTTTTGGGCTTTC


Sequence ID - 509                                        nt:      575

TTTTTTTCTAAATGGNGATTACTAATATATGTGGAGACTATTAATCTCTTTTCTGT
TGCCATTAGTTCATTTTTCCCCAAAAGCCAATACATGTTCATTACAAAAATGAATT
ATAAAATATAAGTTAAAAGAAAAACATAAAACCCTACAATCTTACCCACCCAGACA
ACTACTATTAATACCTTAGTATTAACATATACACATCATGTATATGTATAAATTTA
TCTTAAACAAAAATAAAATTATTCTTTACATATTGTTTTAAAACCTATTTATCTGG
CCAGGTGCCGTGGCTCACGCTTGTAATCCCAGCACTTTGGGAGGCTGAGGCACGTG
GATCACCTGAGGTCAGGAATTCGAGACCAGCCCAGCCAACATGGTGAAACCCTGTC
TCTAATGGTTTAAATACCAAAAAATTAGCTGGGCATGGTGGCACATGCCTGTAATA
TCAGCTAACATGGGAGGCTGAGGCAGGAGAATCACTTGAACCANGGAGGGGGAGGT
TGCAGTGAGCCGAAATCACACCACTTCACTGCAGCCTGGGCAACAAAGCAAGACTG
TCTCAAAAAGAAAAA

Sequence ID 510

CACTGTCATTCCCAGGAGGCTTTGGAGTCAGAACTGGATTCAAATTCTGACTNTAT
GTTGTGTGACTTGGGCCAATAGCTTCTTTNTGTGCCTCAGTTTCTTTAGCTGTAAA
TANACGGGTAGGTCACCCCTTACCCCATAGGTTATGGGGAAAGTTACAGAAAATGG
TCAGCTGGGCNCAGTGGCTCAAGCCTGTGGTCCCAGCNCCTTGGGAGGCCAAGGTG
AGCAGATTGCTTGAGCCCAGGAGTTTGACACCAGTNTGGCAACGTGACGAAACCCT
ATCNCTGTGAAAAATACAAAAAATTAGCCAGGCATGGTGGTGTGTGTCTGTGGTTC
CAGCTGCTTGAGAGTTTGAAGTGGGAGGATCACCTGAGCCCAGAAGGTCGAGGCTG
CAGTGAGCTGTGATCGCGTCACTGCACTCCAGCCTGGC-GACAGAGTGAGA-CCCC
T-TTGAAAAAAAAAAAAAAAAAAAT


Sequence ID 512

GTGAGCGGTGGTGGTTTATTCTTCCGTGGAGTTAAGGGCTCCGTGGACATCTCAGG
TCTTCAGGGTCTTCCATCTGGAACTATATAAAGTTCAGAAAACATGTCTCGAAGAT
ATGACTCCAGGACCACTATATTTTCTCCAGAAGGTCGCTTATACCAAGTTGAATAT
GCCATGGAAGCTATTGGACATGCAGGCACCTGTTTGGGAATTTTAGCAAATGATGG
TGTTTTGCTTGCAGCAGAGAGACNCAACATCCACAAGCTTCTTGATGAAGTCTTTT
TTTCTGAAAAAATTTATAAACTCAATGAGGACATGGCTTGCAGTGTGGCAGGCATA
ACTTCTGATGCTAATGTTCTGACTAATGAACTAAGGCTCATTGCTCAAAGGTATTT
ATTACAGTATCAGGAGCCAATACCTTGTGAGCAGTTGGTTACAGCGCTGTGTGATA
TCAAACAAGCTTATACACAATTTGGAGGAAAACGTCCCTTTGGTGTTTCATTGCTG
TACATTGGCTGGGATAAGCACTATGGCTTTCAGCTCTATCAGAGTGACCCTAGTGG
AAATTCGGGGGATGGGAAGGCCACATGCATTGGAAATAATANCGCTGCAGCTGTGT
CAATGTTGAAACAAG

Sequence ID 513

TTTTTTTTTTATAAACTCCAATCATTTCCAGAGCTACTTAGCTCAGCATCTTTTTT
TTCCACGCTCTTAAGTTGTGTTTATACATTTTTGATACAGTTAGATTGTTTTTGTC
ACATTCTTCATTCTATCCTGGGATCCCCCAACCACCTAAGTGGATTTTTTGATAAT
TTGCATGCTTTAAGGATAACTCTTCATTCTGNAAAGGGCTATGGGTTTTGGCAAAT
GCAGAGTCATGTATCCAAGATTACAATATCGCACAGAAGAGTTTCATCACTATATA
AAACTCACCAGTCTTCCTCCTATTCAACCATCTCCATGCCTTCTTCCCAGCCCTAA
CTCCTTAAAACCACTCATATCTTTACTATTGCTATAGTATTGCCTCTTCCACCATG
TCATATAAATGGAAACATACAGTATTAGTCTTCTCAAACTAGTTTCTTTTACCTAA
CAACATGCATTTAAGATTCATAGTGTCTTTTAATGACTTGATAGATTATTTCTTTG
TAGCTGAATAATATTGCATCTTATAGATGTAACCGTTTGTATATCCATATTTTCTC
ACAGCCTATGACTTGNCTTTTGATTCTCTGAACAGGCCATTCACAAAGCAGAAGTT
TTAATTTTTATAAAGCTAATGNATCAACTT


Sequence ID 515

CCTGGATGACAGCATATCTGTTTATAGCTCAGTTTACTGAATACTTTAAGCCCACT
GTTGAAACCTGCT


Sequence ID - 518                              nt:      502

GATGCATGTCCAGCATAGGCAGGATTGCTCGGTGGTGAGAAGGTTAGGTCCGGCTC
AGACTGAATAAGAAGAGATAAAATTTGCCTTAAAACTTACCTGGCAGTGGCTTTGC
TGCACGGTCTGAAACCACCTGTTCCCACCCTCTTGACCGAAATTTCCTTGTGACAC
AGAGAAGGGCAAAGGTCTGAGCCCAGAGTTGACGGAGGGAGTATTTCAGGGTTCAC
TTCAGGGGCTCCCAAAGCGACAAGATCGTTAGGGAGAGAGGCCCAGGGTGGGGACT
GGGAATTTAAGGAGAGCTGGGAACGGATCCCTTAGGTTCAGGAAGCTTCTGTGCAA
GCTGCGAGGATGGCTTGGGCCGAAGGGTTGCTCTGCCCGCCGCGCTAGCTGTGAGC
TGAGCAAAGCCCTGGGCTCACAGCACCCCAAAAGCCTGTGGCTTCAGTCCTGCGTC
TGCACCACACATTCAAAAGGATCGTTTTGTTTTGTTTTTAAAGAAAGGTGANAT

Sequence ID 519

CTGCGATNGAGTTTTGAGAGGAAGGANTAAAGTNCTCATCTCNGACGGTGAGAAAG
ATCATNACTAAGGAAACGCAGGGTTGGAAGCAGTGCTGANTGTCCAGTTGAGTTTC
ATGANCAAACATTTGCTGTGGGACCAGTTTTCATGGNGGTTTGTCATTTTGTCCAG
CTGCCTGGAGCTGCTTGGTTGAAGGCACAGAATAATCAGGATTAATTGTTNAACTT
GTATGAATTTCTTTATTTTAAAATAGGAATAATATCTGCCTTGGGAGCAAGTTGTA
AGAGTTAACTGAAAGCTTNAGGAAAAACTTTCCCTTGCTATTTAAGTAGGGCTTTA
CAAGTTACAATTCTATCACAGTTTTAAGATTATAAAC


Sequence ID 521

GCGGCGCANCTGCGGATCCANAAGGNCATAAACGANCNGAACCTGCCCAANNCGTG
TGATATCACCTTCTNAGATCCAGACNACCTCCTCAACTTCAAGCTGGTCATCTGTC
CTGATGAGGGCTTCNACAAGAGTGGGAAGTTTGTCTCAAAAAA


Sequence ID - 523                    nt:      585

GATTTACTGTGGGAATTTGCTCATGCAATTATGGAAACCTAGAAGTCCCATAATAT
GCCATCTTCAAGCTGGAATCCCAGGAAAGCAGGTGGTGTAATTCTGAGATTGAAGT
CTTGAGAACCGGGGGAGTCAATGGTGTAACTCCCAATCTAGGGCTTAAGGCCCAAG
GACCAGGGCTGCTGGTGTGCAGATGCAAATCCTGGAGTTCAAAGGATTGAGAACCA
GGAGCTCTGGTGTCTGAGGGCAGTAGAAGATGGATGTTCCAGCTCAAGAAGGGAAA
GTAAGAATCCGTCCTTCCTCCACTTTTTTGTTCTATTCAGATGAGCCCTCAATGGA
CTGAACGATGCTCACCCACACTGTGAGGGCTGGTCTTCTTTATTCAATCCACTGAC


TTAAGTGCTGATCTCTTCTGGAAACACCTTCACAGACACACCCAGAAATAATGTTC
TACCAGCCATGGGCCTGTTACTTAGCCCAGTCAAGTTGACACAGAAAATTAGCTAT
CACAACATCTGTGTGTGTATATACATATGTATTTGCATGTGTGTGTATATATGGNG
TATATATATTCATGTGTGTGTATAT

Sequence ID 524

CTTTTGCCAGTAGGCCCCCTGAGTAGGTTCCTCTATCTTTTGGCATGACCCCAGAA

GTCTTTGATAACTTCCTTGCTTTCTGATGTGACAAGACATCCAGGGCCAGATTGTC

CATATCCTGCCCCGGATGCACGATGCACTGTTTCTCCAAGAATCCCTGTGTCCTTT

GCTGATGATGCCATGATTTTAAGTTCTCTAATATAGTTTTATCTCTTTGTTTCAGA

TAATGCTTTTGTGTTCTCACATGTCCTGCTCTCTCTCTCTCTCATTTTGGTGTT

GATCAGTCTTTCCATAAGATTGTTTATTTCACTAGTCCTTCATTCTTCTTTTTTCT

AAATTTACTCTTCTTGACTAGTATCCTGTCACTTCTGAGGACTCATATTTTTGCAA

CTTGAAAATTATTCTTATTTATTTAAGTATATGTTNCTGAAACTCTCATTAGACAC

ATTTTG


Sequence ID 525

GTTAAAAAAAGTAAAAGGAACTCGGCAAATCTTACCCCGCCTGTTTACCAAAAACA

TCACCTGGTAGCATCACCAGTATTAGAGGCACCGCCTGCCCAGTGACACATGTTTA

ACGGCCGCGGTACCCTAACCGTGCAAAGGTAGCATAATCACTTGTTCCTTAAATAG

GGACCTGTATGAATGGCTCCACNAGGGTTCANCTGTCTCTTACTTTTAACCAGTGA

AATTGACCTGCCCGTGAAGAGGCGGGCATAACACAGCTGAAAAAAAAAAAAAAAAA

AAAAAAAAAAAAAAAAAAAAAAAAAAAAAAAAAAAAAAAAAAAAAAAAAAAAAAAA

AAAAAATTTT


Sequence ID - 526                          nt:      516

CTTTTCATGGTCTCTTGTTCATTAATCATCTAAAATCCAAGCNCAGAGAATTCAAT

TTTAGATGGTCTCCAGAGCAGAATTTGATGTATAATCTTAATTACAAATCATAGAT

AATTAATATTGNTTACAAAATCANAATACGATTAGAGGTAGGGATCCTGCACACAC

CCTATTTTCCTCCCCAGTGTTCTGACCGAGAGACTAATTAATAATTCAAGGAACTT

ACAGTGAATGANAACCCATGGTTTTGCTTAATTATCAGAACAGCTAGATCTGAGAA

CAGCTGTCTCCCACATGGATAGACACTTATTCCACCCATTTGCAGGTAGAATAGCT

GGCAATAATAAGTCCTTCCCATTGGATATGTTGAAAGGTGCCTGCCATGGCATAGT

TGCCACAAGAGAGGAAGAAATGGACACAAATGTAGGCTGTTTTCAGGGCANAGGGA

AGGTGGGAGGAAACCAANTTGCTGGTTTTCACACACCCTCTGGGGAACACCCATGC

ACCTATGANATG

Sequence ID 527

GACAAAAGCTGAGAGAATTTTTTTCTTGAATATTTGCACTAAAAGATAGGTTAAAA
TTCTTCAGGCTGAAGAGAGCATACCAGGTGGAGATTTGGATCTACAAAAAGGAAGG
AAGATTTGGAAATGGATTTGGCACCATTGACTCAATTTCCAGAACAAGAAAGCAGG
GACAGTTTTGGGAAGCTCAAGACACACTGCCCATGAGCAGCAATTTGGACCTCCTG
CTGCATCCACTGTGCATCAAACACACACTGTACAGACAAAGACTCCCAGGAAAAGA
AGTATAAACATGGACTAACACAGAGATGGGCAAACTACAGCCTGTGACCCAGCCAC
CTGTTTATGTAGAATCCAAAGTAAGAATCTTTAACTTACACATAAACTT


Sequence 529; 660nt

GACAGCAGAGCACACAAGCTTNTAGGACAAGAGCCAGGAAGAAACCACCGGAAGGA
ACCATCTCACTGTGTGTAAACATGACTTCCAAGCTGGCCGTGGCTCTCTTGGCAGC
CTTCCTGATTTCTGCAGCTCTGTGTGAAGGTGCAGTTTTGCCAAGGAGTGCTAAAG
AACTTAGATGTCAGTGCATAAAGACATACTCCAAACCTTTCCACCCCAAATTTATC
AAAGAACTGAGAGTGATTGAGAGTGGACCACACTGCGCCAACACAGAAATTATTGT
AAAGCTTTCTGATGGAAGANAGCTCTGTCTGGACCCCAAGGAAAACTGGGTGCANA
GGGTTGTGGANAAGTTTTTGAAGAGGGCTGAGAATTCATAAAAAAATTCATTCTCT
GTGGTATCCAAGAATCAGTGAAGATGCCAGTGAAACTTCAAGCAAATCTACTTCAA
CACTTCATGTATTGTGTGGGTCTGTTGTAGGGTTGCCAGATGCAATACAAGATTCC
TGGTTAAATTTGAATTTCAGTAAACAATGAATAGTTTTTCATTGTACCATGAAATA
TCCAGAACATACTTATATGTAAAGTATTATTTATTTGAATCTACAAAAAACAACAA
ATAATTTTTAGATATAAGGATTTTCCTGGATATTGCACGGGAGA


Sequence ID 529

GACAGCAGAGCACACAAGCTTNTAGGACAAGAGCCAGGAAGAAACCACCGGAAGGA
ACCATCTCACTGTGTGTAAACATGACTTCCAAGCTGGCCGTGGCTCTCTTGGCAGC
CTTCCTGATTTCTGCAGCTCTGTGTGAAGGTGCAGTTTTGCCAAGGAGTGCTAAAG
AACTTAGATGTCAGTGCATAAAGACATACTCCAAACCTTTCCACCCCAAATTTATC
AAAGAACTGAGAGTGATTGAGAGTGGACCACACTGCGCCAACACAGAAATTATTGT
AAAGCTTTCTGATGGAAGANAGCTCTGTCTGGACCCCAAGGAAAACTGGGTGCANA
GGGTTGTGGANAAGTTTTTGAAGAGGGCTGAGAATTCATAAAAAAATTCATTCTCT
GTGGTATCCAAGAATCAGTGAAGATGCCAGTGAAACTTCAAGCAAATCTACTTCAA
CACTTCATGTATTGTGTGGGTCTGTTGTAGGGTTGCCAGATGCAATACAAGATTCC
TGGTTAAATTTGAATTTCAGTAAACAATGAATAGTTTTTCATTGT

Sequence ID - 530                    nt:      660

GACAGCAGAGCACACAAGCTTNTAGGACAAGAGCCAGGAAGAAACCACCGGAAGGA


ACCATCTCACTGTGTGTAAACATGACTTCCAAGCTGGCCGTGGCTCTCTTGGCAGC

CTTCCTGATTTCTGCAGCTCTGTGTGAAGGTGCAGTTTTGCCAAGGAGTGCTAAAG

AACTTAGATGTCAGTGCATAAAGACATACTCCAAACCTTTCCACCCCAAATTTATC

AAAGAACTGAGAGTGATTGAGAGTGGACCACACTGCGCCAACACAGAAATTATTGT

AAAGCTTTCTGATGGAAGANAGCTCTGTCTGGACCCCAAGGAAAACTGGGTGCANA

GGGTTGTGGANAAGTTTTTGAAGAGGGCTGAGAATTCATAAAAAAATTCATTCTCT

GTGGTATCCAAGAATCAGTGAAGATGCCAGTGAAACTTCAAGCAAATCTACTTCAA

CACTTCATGTATTGTGTGGGTCTGTTGTAGGGTTGCCAGATGCAATACAAGATTCC

TGGTTAAATTTGAATTTCAGTAAACAATGAATAGTTTTTCATTGTACCATGAAATA

TCCAGAACATACTTATATGTAAAGTATTATTTATTTGAATCTACAAAAAACAACAA

ATAATTTTTAGATATAAGGATTTTCCTGGATATTGCACGGGAGA


Sequence ID 532

GAATTGTGATAGTTCAGCTTGAATGTCTCTTAGAGGGTGGGCTTTTGTTGATGAGG

GAGGGGAAACTTTTTTTTTTTCTATAGACTTTTTTCANATAACATCTTCTGAGTCA

TAACCAGCCTGGCAGTATGATGGCCTANATGCAGAGAAAACAGCTCCTTGGTGAAT

TGATAAGTAAAGGCAGAAAAGATTATATGTCATACCTCCATTGGGGAATAAGCATA

ACCCTGAGATTCTTACTACTGATGAGAACATTATCTGCATATGCCAAAAAATTTTA

AGCAAATGAAAGCTACCAATTTAAAGTTACGGAATCTACCATTTTAAAGTTAATTG

CTTGTCAAGCTATAACCACAAAAATAATGAATTGATGAGAAATACAATGAAGAGGC

AATGTCCATCTCAAAATACTGCTTTTACAAAAGCAGAATAAAAGCGAAAGAAATG

AAAATGTTACACTACATTAATCCTGGAATAAAAGAAGCCGAAATAAATGAGAGATG

AGTTGGGATCAAGTGGGATTGANGANGCTGTGCTGTGT


156

Sequence ID 533

CTTGAACCTCGGAGGCAGAGGTTGCAGTGAGCCGAGATCACGCCACTGCACTCCAG

CCTCGGGGACAGAGCAAGACTCCATCTCAAAACACACACACACACACACACACACA

CACACACACACAAAACAGATATACACTGAACACAGCACAAGTGGGACATAAGAG

ATTTAAAAGGGTTAGAGATGTAAAATGGATCTAGGAATGGAAACCATAAGGNGGGA

TTTATCAACTGGATTCTGCANAATGCTGTTAAGGCCAGATGTTAGCAGGTGTTACA

TAAAAAAGGGATACCATGAGCAAAAGTATTTGAACATGGGCAATGGTTGAAACAAG

TTTAAACAGATTATNTTTATTACCAAATCTCTCAAACCTTTAATATGCTATAAACA

TTGTGAAACAATAAAAAAACTTTCCAAAA


Sequence ID 534

GGGAAGGGAGCTATGAGTGTGTGTGTTGTGTATGGACTCACTCCCAGGTTCACCTG

GCCACAGGTGCACCCTTCCCACACCCTTTACATTCCCCAGAGCCAAGGGAGTTTAA


GTTTGCAGTTACAGGCCAGTTCTCCAGCTCTCCATCTTANAGAGACAGGTCACCTT

GCAGGCCTGCTTGCAGGAAATGAATCCAGCAGCCAACTCGAATCCCCCTAGGGCTC

AGGCACTGAGGGCCTGGGGACAGTGGAGCATATGGGTGGGAGACAGATGGAGGGTA

CCCTATTTACAACTGAGTCAGCCAAGCCACTGATGGGAATATACAGATTTAGGTGC

TAAACCGTTTATTTTCCACGGATGAGTCACAATCTGAAGAATCAAACTTCCATCCT

GAAAATCTATATGTTTCAAAACCACTTGCCATCCTGTTAGATTGCCAGTTCCTGGG

ACCAGGCCTCANACTGTGAAAGTA


Sequence ID 560

GGCGGAGGTTGCAGTGAGCTGAGATGGCGCCATTGCTCTCCCAGCCTGGGTGACAA

GAGCAAAACTCCGTCTCAAAAAAAAAAAAAAAAAAAAAAAAAGCAATTTACTTAAAAAC

ATACAAACACAGAGACAAGTATTTTTGAGAAACAAATACCTTTTTCATTTTTTATA

CCAATGTAACAATAATCCATTAAACACACCTTTACTAACTGTTTTCTAGGAGTCTG

ATATGATGAGGAAATAGGTAAACCTTTAATAGCCAGTACTAAATTAGAGTGGCACA

ACTTTCACTGGGAAAAAAGATGGGTATTTTACTTTTCTGTTTTAGAAAAGTGGCTT

GACAACAGTATGCTTATGTCTTAGAGTTTGAAATTCAAGTTCTTGAACATTATTAA

TGGCTACAATCATTCATACCCACATTGGGCTGTATTCTTGATGAATCCAAAGTGAT

TTTCACCTCAACTCTGAATTTCATTCTCCTCTTTTGAATATAATACAACCATCTCA

CTAGAGGAAGCATTTCAGTCTTTTCTGATTGGAGATTCATTATTGTTTTAGATAAT

GTTTTCATTTGCTTATGGGTATATAAAAAATTTTATCTTAAAAATATTTCCTCTCA

TTTAGCTAGCAACATTGTTTTC

Sequence ID 561

CTCAGGGTGATCTCTGAACCCAAACTTGCCCCAAAGAAGGTTGCTCTGTCCTCTCC
ACATCCCCATCTCCTCCCTAGGGCCTTGTTGGGGAGAGGCTCCTCCATCTTTCCCA
AGTCACACCATCGTTTCCTACGTGGTCTGGACAAGAGCAAGAGCACACCTTGTCCC
CACCTTCTCCAGAGCAGCCAGAACCCACCTCAGGTGCCTTCCCCATCCGGTGCAGT
TAAGGCACTTCTGCCAGCACCATGGTATGAGCACTAGACTTGGAGTTAAGATTTGA
GAGCCCCCTCTGTCACTGTGGAAGCTTGAGCATGTTGCTTGATCTCTCTGAACCTT
GTGTTTCTCATCTGTGAAAGGTGATAATGTGGGGCTGCTGTGAGATTTAAAGGACA
TAATGCACCTACGGTCCAAGCACTGCCTGGAATACAGCANAAGCTCAACAGATACT
GGACAACCCATCCCCTTAGTAGAGGCACTAACCATGTGACCCAAGGCAAAAGTGCT
TAAAAAAA

Sequence ID - 562                    nt:     580

ATTGCATGCAAGTTTGCTGAGCTGAAGGAAAAGATTGATCGCCGTTCTGGTAAAAA
GCTGGAAGATGGCCCTAAATTCTTGAAGTCTGGTGATGCTGCCATTGTTGATATGG

TTCCTGGCAAGCCCATGTGTGTTGAGAGCTTCTCAGACTATCCACCTTTGGGTCGC
TTTGCTGTTCGTGATATGAGACAGACAGTTGCGGTGGGTGTCATCAAAGCACTGGA
CAAGAAGGCTGCTGGAGCTGGCAAGGTCACCAAGTCTGCCCAGAAAGCTCAGAAGG
CTAAATGAATATTATCCCTAATACCTGCCACCCCACTCTTAATCAGTGGTGGAAGA
ACGGTCTCAGAACTGTTTGTTTCAATTGGCCATTTAAGTTTAGTAGTAAAAGACTG
GTTAATGATAACAATGCATCGTAAAACCTTCAGAAGGAAAGGAGAATGTTTTGTGG
ACCACTTTGGTTTTCTTTTTTGCGTGTGGCAGTTTTAAGTTATTAGTTTTTAAAAT
CAGTACTTTTTAATGGAAACAACTTGACCAAAAATTTGTCACAGAATTTTGAGACC
CATTAAAAAAGTTAAATGAG

Sequence ID 563

GCAACCTGCACAACCCCGCCCTGTTCGAGGGCCGGAGCCCTGCCGTGTGGGAGCTG
GCCGAGGAGTATCTGGACATCGTGCGGGAGCACCCCTGCCCCCTGTCCTACGTCCG
GGCCCACCTCTTCAAGCTGTGGCACCACACGCTGCAGGTGCACCAGGAGCTGCGAG
AGGAGCTGGCCAAGGTGAANACCCTGGAGGGCATCGCTGCTGTGAGCCAGGAGCTG
AAGCTGCGGTGTCAGGAGGAGATATCCAGGCAGGAGGGAGCGAAGCCCACCGGCGA
CTTGCCCTTCCACTGGATCTGCCAGCCCTACATCCGGCCGGGGCCCAGGGAGGGGA
GCAAGGAGAAGGCAGGTGCGCGCAGCAAGCGGGCCCTGGAGGAAGAGGAGGGTGGC
ACGGAGGTCCTGTCCAAGAACAAGCAAAAGAAGCAGCTGAGGAACCCCCACAAGAC
CTTCGACCCCTCTCTGAACCAAAATATGCAAAGTGTGACCAGTGTGGAAACCCAAA
GGGCAACAGATGTGTGTTCAGCCTGTGCCGCGGNTTG


Sequence ID - 564                               nt:      671
GGAATAGAATTTTAAATAGTAATAACTGCTTGTTTTTTTTGTGCAAGTACTTTTAT
ACATAAGATAAACAAAAACCTTACCACCAAACATACCAAAATGCACCTCTTTCATA
AGTGAGTTACTAAGATTTCTATACCTGGAATATCATGTATGTTTCATTTACTGGAT
GTTTACATTTTAGGAAGGAAAATAGTTTTGTTTATTTAAACAACTGAATACTTATA
AACTGTTGTTCCTGGAAGTTATTTATTCCATAAAAAATTTGTTCTTTTGTCATGAA
TTTATAATTCCTAAATGAAGACCAGAAAGTACAAATTGCTGGGAGGAAGAATAGGC
TTTATTAATCAACTGATGTCTTGATTTTTCTAAATGGGAAGATTGCTTTATTTTTA
ACACTAATTATGGGAGCAGATTCTTAGCAAACTTCTTTGGAAAAGTTAATGTTATG
ATGTGCATTAGGCTGCCCCATCGTGTATATAAATGAAGCAGATTTGATTTTTGTAT
TCTTACGTTTCTCTGCTTTGTAGTTGTGGCTGTACTTAAAGAAATACAGAATTTCA
TATATTTAAAAATGTTTAAAATGTGACCCACAGACATTGTAAATGGATTNAAAACT
AACATGAAAAATATTCAACCTAAAAGAATTCTTAACTTCACAAGTGTTTTACTTC


Sequence ID 565

CTTGGTTCCGCGTTCCCTGCACAAAATGCCCGGCGAAGCCACAGAAACCGTCCCTG
CTACAGAGCAGGAGTTGCCGCAGCCCCAGGCTGAGACAGGGTCTGGAACAGAATCT
GACAGTGATGAATCAGTACCAGAGCTTGAAGAACAGGATTCCACCCAGGCAACCAC
ACAACAAGCCCAGCTGGCGGCAGCAGCTGAAATCGATGAAGAACCAGTCAGTAAAG
CAAAACAGAGTCGGAGTGAAAAGAAGGCACGGAAGGCTATGTCCAAACTGGGTCTT
CGGCAGGTTACAGGAGTTACTAGAGTCACTATCCGGAAATCTAAGAATATCCTCTT
TGTCATCACAAAACCAGATGTCTACAAGAGCCCTGCTTCAGATACTTACATAGTTT
TTGGGGAAGCCAAGATCGAAGATTTATCCCAGCAAGCACAACTAGCAGCTGCTGAG
AAATTCAAAGTTCAAGGTGAAGCTGTCTCAAACATTCAAGAAAACACACAGACTCC
AACTGTACAAGAGGAGAGTGAAGAGGAAGAGGTCGATGAAACAGGTGTAGAAGTTA
AGGACATAGAATTTGGTCATTGTCACAAAGCAAATGTGTCGAGAGCA


Sequence ID 566
GTCACCAAGAGCTTGTTGTCAGGTTTTCACTTGCTATTCGCAGAGATTTTTTTTAA
AGGCACTATTTGTAGTGTTAAAAGGGTGAATTTATCANAAGGCATAATAATCATAA
ATGTGTATATGCCTAATAATAGAACTTTAAAAGGCATGAAGCAACACTCAAAAGGA
TTAAAGGGAGATCATCTCACCCCCTTCTTACCAATTGATAGAATGATCTGATGAAA
ACAGTAAAATAACAACAGATCTGAACACTGTCAACCATCTTGACAAATACTTATGC
CTAGTGTTCCATTATTGGAACACTAAACATGTGGAATGATTTATATCCTACTGCTC
AAGGTCATCACCAAGGTCTAATTGTAAAATTTCAAAAAATTGCAACCTCAGGCATA
AATGGGTTAATCGACATTTATAGCACACACATGCAACATGTACCAGAGATTCCTTC
TTTTCTATGAACATGGTACTTCCACCAAGATAGACCACATTGTGAACTATAAAACA
AATCTAAAAACATTTGAAATGAAGGAAATTATATAAAATATGTTCTCTTGATCTCA
ATGAAATTAAATTAATACTATAT


Sequence ID 567
CTCATGGCGGCCAATGTAGGCCCAAAACTTCCTCAAGTCAAACTCTCCAGGCCCAC
CTTCTGCTTCCCGGTGGCATCAACAGGCCCAGCTTTGACTTGAGAACAGCCTCTGC
AGGCCCTGCTCTTGCCTCCCAGGGGCTTTTTCCAGGCCCAGCTCTTGCCTCATGGC
AGCTGCCCCAGGCCAAATTTCTGCCTGCCTGCCAGCAGCCTCAACAGGCACAGCTC
CTCCCTCACAGTGGCCCATTTAGGCCCAACTCATGACTGTGAGGCCATTTCCAGGC
CTAGTGCCTGCCTCGTGGCTGACTCTTGAAGCCCAAAACTTCCTCAAATCAGCCTT
TTGCCCAACTTCTGTCTACTGTCGGACTCTACAGGTCAGCCTCTGCCTCACAGTGG
ACCCTCCAGACCCAGATGGTGTCTNCTGTGGCATCCTCAGGCGAAGCTCCTGCCTT
TCGGCAGCCTCTCCAGGCCCAGCTCCTCCTGCTCCAGCCTTCTCTCCAGGCTCTGA
ACTTTCTCAGGTCTCCCTCTGTTGTCCAAGGCTGGAGTGTAGTAG

Sequence ID 568

TATATATGTAATGCCCTTAACCTAGTGTTTGGCATGATCGTTGCTGAAAGGGAAGC

TTGTGGGTACAGTGTCCCCTCAGAAGCCAAAGCCCAGGGAAGGTCGCCTGCCCAGG

TCAGGCTCCCAGCGAGTTTGTCTGGGGAGGGGCCATTCATACCTCCAGGTCAGGAC

AGAGGCTCGGGCTGAGGGAACCCTACACAGGTCCTGGAAGCAGATCCTTCCTGCCT

AAGCCAGCAGGACAGCTCAACAGGAAGCATCTTCCAGCCACGGGAGGAGAGGCAGC

ACCTTTTTTGGAACCATACAGAGCTAAGAATGGTGGTACAAGTAATAGATTCTGTA

CTGGCAACCCCACTTGGTGGAGCAAGTTCTAGGAAAAGGGGGCTGTCCTTGAGTCA

GCCATGGGGTCAGCCACACAGTCACCGCAGCTGCTCTTTGGCACCGGGCGCTGGAA

AGACCTAGGATGACACAGCCTGGAAAGAGCTTGGGAAAAGCTCATCTTCCACAGAA

CTACCTGCTATACCAGCCAGGGCAGGTGCTTATTCCCACAACAGCCCTCTGTTGTA

GGCGGCAGTGCCATCCTGAANGTGCCGTGGTACCTTCTGAANACCCAGCTGAGGGC

CTGTAATGGCACTTGCATGCCACATGGNACACCCTTTCCCGGTTAA


Sequence ID 570

ACCGCGGCCGCGTNAANAAAAAAAAAAAAAAAGAATTCCACTTGATCAACTTAATTCC

TTNTCTTTATCTTCCCTCCCTCACTTCCCTTTTCTCCCACCCTCTTTTCCAAGCTG

TTTCGCTTTGCAATATATTACTGGTAATGAGTTGCAGGATAATGCAGTCATAACTT

GTTTTCTCCTAAGTATTTGAGTTCAAAACTCCTGTATCTAAAGAAATACGGTTGGG

GTCATTAATAAAGAAAATCTTTCTATCTTAAAAAAAAAAAAAAAAAAAAAAAAAAAA

AAAAAAAAAAAAAAAAAAAAAAAAAAAAAAAAAAAAAAAAAAAAAAAAAAAAAAAAA

AAA


Sequence ID - 571                                    nt:        457

TTAGAGAGGTGAGGATCTGGTATTTCCTGGACTAAATTCCCCTTGGGGAAGACGAA

GGGATGCTGCAGTTCCAAAAGAGAAGGACTCTTCCAGAGTCATCTACCTGAGTCCC

AAAGCTCCCTGTCCTGAAAGCCACAGACAATATGGTCCCAAATGACTGACTGCACC

TTCTGTGCCTCAGCCGTTCTTGACATCAAGAATCTTCTGTTCCACATCCACACAGC

CAATACAATTAGTCAAACCACTGTTATTAACAGATGTAGCAACATGAGAAACGCTT

ATGTTACAGGTTACATGAGAGCAATCATGTAAGTCTATATGACTTCAGAAATGTTA

AAATAGACTAACCTCTAACAACAAATTAAAAGTGATTGTTTCAAGGTGATGCAATT

ATTGATGACCTATTTTATTTTTCTATAATGATCATATATTACCTTTGTAATAAAAC

ATTTTTCCC

Sequence ID 572

CGTCTATTTGNGTTTCTTCTCACAATTGGTAAGTTCTCTGTATTGATTGATGGCTA

AGTTTGATTAGTGTTTTTCTCTAGTTGGTAATTATATTCTAGTATTTTATCATCTT
ATTGTTTACTCAACTNAAAGTGNCACAGAAGAGTTGCCAGGTTTCTCTTTGATATG
AGATCTCTNNTTGATTTGGAATGCAAATCANAAGTGTCATGTTTTGAATAAAGGGA
CCAGATGACTTATAGGTATTCTTTCTCTAAATATAACTAAGGTAAGATTTTTGTTT
TGAGGTACTTAATCTATATAAGTGGTAAAGAATTTACTTGAATTTCTCCAAATTCT
CATGTCTAAAGTCTGATTGATTAAATTCATTCTTGGTATTTCATTTTGAAAAGAAT
GTAGCTTTAGCAAACCTCTTTGTATAAATGCAGTGGGATTAAGGTCATTTAAAAAA
TTGTTATATCATTGTATTTTTAAAATTTACCAGTTTTATTTTTCTTTTTACCCTTT
AGCCCGGCCTCAGAAAGTGTGTTTGTGTCCATTTCTCCCAGCGCACCCTCTGCATA
TCTCTACCCACTTGTCATAATTCAGCATCCAGCAGAGGAAAACAAAGTGTTGCGTA
CAGTTCCTCTACTAGCAGCATGCCTCCCCCAGGACAAGTGTA

Sequence ID 574

TTATTGCTGACATAAAAATGGTGCACATCGGCCAGGGCCCAGGATGAATCAGCCAA
TCTGCACCATTTATACATGGAACTGGAGAACATTGTGCCAATAATCATTTAATATA
TGCCAAATCTTACACGTCTACTCTAAACTGCTCTAATGAAGTTTCAGTGACCTTGA
GGGCTAAAGATTGTTCTTCTGGGTAAGAGCTCTTGGGCTGGTTTTTTCANAGCAGAG
TTCTTGTTGTGGGTAGACTGTGACTAGGTTCACAGCCTTTGTGGAACATTCCGTAT
AACGGCATTGTGGAAGCAATAACTAGTTCCTATGAAAGAACCAGAGCTGGGAAGAT
GGCTGGGAAGCCAGGCCAAAGTGGGGGCAACAGCTTGCTTCTCTTTCTCTTCTCAC
CCTCAGTTTGTATGGGAAAATGGAGATGTCCTCTCCACTTTATCCCACGATATCTA
AATG

Sequence ID - 575                               nt:      209
CAGGATATCGAGACCATCCCAGACAGCATGGTGAAACTCCGTCTCTACTGGAATAC
AAAAAGTTAGCCGTGTGTGGTGGCACGCGCCTCTAATCCCAGCTATTCGGGAGGCT
TAGGCAGGAGAATTACTTGAACCCGGGAGGCGAAGGTTGCAGTGAGCTGAGATCGC
ACCATTGCACTCCACCCTGG-CGACAGAGCAAGACTCCGTCT

Sequence ID - 576                    nt:        541

CAGCCAACCCAGAAGGAGCCAGTCTACAACTATGCCTGATCCTCCTCATGGCAGGC

CACGAAGCATTGCTGCCATGTGTTGAATTATAAAACCCACATTGCTTTTTGAACCC

TGTTGCGGGTAAAAATAACCAAATTATCAGTCCTTGGAAACCCAGGCAATCAAGTG

AGTACAAGGTAAAGATAAGTATGGTTTAGAGGAGAAATTATGTTCCTGAACTGGTG

TCCTTTGATGGCAGCGTCAGCCTTGCTAAGTCAGAGTAGAGGGAGCAGTGACCTTA

ATAAGCTTTGGTGAGCATCATGTGCACGCGTGGGTGGGAGTCCCTTTCACTGATGC

TTTTAAAAGTGCTTTTGCAGACCCTGGAAGGGATCCTCCACACATATGAGGTGTGG

GACAGGTAGGCCAGAGAGGATTAGCCCTGCTTTCGAGACTAGAAATCTACAGTCCT

GAAGGAGCAGTAATTAATTGGTACACCTGTCAGGGCCAGCCCCCAGGTCTCCTGGC

TTTTTCCAGGTTTTCTGTCTCACATGATTTTGCTTTT

Sequence ID 577

CTTTAATTTTTCAAGTGTTTAAAAAACAATTTTATACTTAAGCCAGCCTTGAAGAT

AAGCACAAAATTTACCAGTTTACATTTAAAAAACAAACAAAAAACGACAACAACTC

AAGCACCCGCTCTGTGCATAGCACTATTCTAGGTGCAATAAAAGGGAATCTTAACC

TTAGAAATATGAGTTCACTTTCTGGAATTGTATTATCTCCTTTTCCAGAGAGTAAA

AATAAATAAAATCACCATTGTTTACTACAGATCTGCCCCAAACCACATCTGGTTCA

CAGAAAGGCTAATTTCTGCCAAATTAAAGATGTAATGAACTCAGTTCCTGCTTTCC

CAAAAACACGAAAGCAGAATTCCTTTTCACTGAAAAAAATAAACAGTTTTCCATGC

AAGGGCAGTTTGCTTCTAATAAGTATTTTTTAAAAAATTTTTTTTTCCTCTAGCTT

TTCTTTAAATTTTCTTCCTCTAATATTGCCTTTTCTTGTACAAGGCAGACCAGGTA

TCTTTTTATGCTGTTTTTCCTTTACTAAGAAAGTATTGCATCTTGAAGACAAACC

ATTTCCCAGAGTAGTGATAAAAAATAACACTAAAAAAACTTTAAAGGTGAGTCACT

TCATCACCTTGATGAAGTAAAAAA

Sequence ID 578

GGAAAAAATATTTCCACTTAGATATTTTACATGGTTTTGTTTAAAATTACCATTAC

TTGTTTTTTAAAAACACATGACCACATATGTATATGTATATCTACCTAAACATTGT

ATCATGGTTTCAGTATGTTATTCATGTATTACTGGGAGATGCTACCAAGAAACCAA

CCCAAAGAAAATTCTGGAAAATACATTTCTATTTATAGAATAAATGTTTCATTTAT

ATAAAAGCAAAAGAACTTAGAGTTCTAATAAATGGGATGTCTAATAAATTATGAAG

TTACTGATTTGAATATATTATATTTTTATAACTTCCTTGCCAAAGTCCTGATTTAG

TACATTAGAGAACCTGTGTTTCCTCTCTCCTCTACCATTCATCTCTCTTCCATACA

GTCATTTGGGCTTTTTACTCAAAGAGAATCAAGAAATAATAAGGTATAACAAGCTT

GGCAAAGTGTTGGCTTTTTAAAAAAAAATTTTTTTAATCTCTAGCAGTTTGGTAAT

TTAGCAGCATCATTTATTTGGGATTCTTTTATCTGATTTCAACAGTGAAAAACATC

CCTATGATAAAGCCTAATGACCCATTTCCAAAGATGGAATTGCCCTTCCTAGAAA

ATATGACGGAGAAAGT


Sequence ID - 579                                    nt:        502

CGAATAGCCAAGTGGTCTGACAAGATCGAGAGTAATGAGGCCCATACTTTAGTACA

GTCTTGAATGGCCAGATGGTGCTGGGCATACCCCAACCAGAGATATGTAAGTCTTT

ATGTTGTCAAAATTTCCCAGAAACATGAATTTCCCACTAAGATTCATTAAGGAAAA

CTAGAATGAAAACAAAAACGTTCCTTGTATAATATTCATTANAAAGAAATGAAGAA


GGCCGGGCATGGTGGCTCACGCCTGTAATCCCAGCACTTTGAGAGGCCAAGGTAGG

CAGATCATGAGGTCAGGAGTTTGAGACCAGCCTGGCCAACATAGTGAAATCCCGTC

TCTACCAAAAATACAAAAAAATTAGCCGGGCATGGTGGCACACACCTGTCATCCCA

GCTACTCAGGAGGCTGAGGCAGGAGAATTGCTTGAACCTGGGAGGTGGAGGTTGCA

GTGAGCTGAGATTGCACCACTGTACTACAGCCTAGGTGACAGTGCAAGACTCTG


Sequence ID - 580                                    nt:        316

CCTATGCCAAACTAAAGAAAGCTTGCCTGGCCTACAGGCCTAAAGGTTCAAATGNG

GATTAAAAAAACACAGTAGTCACATAAAATGTCTGCTGGCTGGCTGGAATTCCATC

ACCTACAATTTACCTGCTTTCAAAAACTGTGTTCAACATTGAGAAAACAGAAAACC

ACTTATCTTGAGCTTAATATGGGCTTCTTTTTCCTTAACTGTAGAACACTTACTGA

AATATCAAATCAATGGTTAGGATATGTATCCTAGGCAGGCCTAAACCATTAACACT

TGGTTTAAGCAACTTTGTATAATTNACCTCCTAAAT

Sequence ID 581

```
CTTCATGAGTGCCCGGTTGCCCAAGTCAAAAACCTGGGAGTGATATAAACTCCCCA
CACATCCAGTCAGTCACTCATCAACTCTATTGATTCTG-CTGCTAAATATATCTCA
ATTGTATTAACTTAAACATATGCATAATACATCTTCTTCTTCACTGCATTTTTGTG
GGCTGCACTTACCTTTCAGGTAACAACAACACTGGCCCCTCTTGCCCTTCTAGTCA
GAAGTGCCAAAATGATGAGAGCTAGCCATGACAAACCCACAGCCAACATTACACTG
AATGTGCAAAACTGGAAGGGCATCCAAACAGAGGAGG
```

Sequence ID 582

```
TAGAATTCTCGCCTGCCTTGGCTTCTCCCTCTAGTTGTTCCTTCTCTGTCTTCTGT
GGGCTTCTTATTGTCTGCTCACTCCTTCTTCAGTGTCCTCTCATGGGCTTCCTTCC
CTTCTCAGCTGATGCCATCACCTGGGGAATCACAGTTACTCAGCAGCACTGGGGCC
TCTCTATCTCTATGCTGGTCATGCCTATGTGTGAGCTGCAGACCCAGTGGAATTTC
CATTTGTGCATCCCATGCCCAGCCCACCCTCCACCAGCCTCGAATGCAGCTGTTCA
GCCCTACCCCAGTCCTCAGAAAAGTTCCTCTCCCTGGATCCTCTTTTTCCTTCATG
AGTGCCCGGTTGCCCAAGTCAAAAACCTGGGAGTGATATAAACTCCCCACACATCC
AGTCAGTCACTCATCAACTCTATTGATTCTGTCTGCTAAATATATCTCAATTGTAT
TAACTTAAACATATGCATAATACATCTTCTTCTTCACTGCATTTTTGTGGGCTGCA
CTTACCTTTCAGGTAACAACAACACTGGCCCCTCTTGCCCTTCTAGTCAGAAGTGC
CAAAATGATGAGAGCTAGCCATGACAAACCCACAGCCAACATTACACTGAATGTGC
AAAACTGGAAGGGCATCCAAACAGAGGA
```

Sequence ID - 583               nt:     631

CTGAGGTGGGAGGATTCCACTCTCACCCATTTCTTCTTTCATTTTCAGTTTCTCCA
GTTAGTAACTGAAGATGTTCTTTGAGTAATTAAGTGAGTGAGAAAATTTTTAAGTG
AGAAATCTATAAAAAGAACCATGTTAACATAAATATTTCAGTCCTTACAAGTTGGT
ATTGACTTTTCTCATTGGTAATCTGACTGATTTAATACTGCTCATTCCAATATCTG
GTGATGTAATTCTGGTTATGAATCCTTGTATTAATAACACCTCCTGGGAGGTTTTT
TTTCCCCAACATTACATTCAGAATATTAGAGCTGAAAATACCTTTTTTAAGGTTAT
CAGGAGGAGGGAGCTTATGTTTAATGTGGTGGATAAAACTTAACTGCTGGTTAATA
CAATTGTTATTCAGGTGAAATTCCCTAAACTTTTCACGTGCAAAGTTTTGTATGTA
TACAGACATTTGGGGAAAAGTTTTATCATCCCTAAAACCGGTTACTGTCCAGAAAA
TGATAAGAATCCCTGGGTTCCAAATCCTTCATAAGGTATTTATTCATTTATTTATT
CAACACATTTACTCAATGCCTCCGCTCTGCTGCAACTACACTGACATTCTGCTTCT
AATCTAACCGAAAAT


Sequence ID 585

TTTCAAATTGTACAATAACACAAACAACTTTGTTAAGGCCATGTTTTATTTGCTGA
TTAATGGACAAAAGGCAATGTAATTTATTTTCAAGTATTTTCTTGAAAGTCTGTGC
TCATAAAAATCATGAAAAGTTGGAAAGACTGTTAAATCACTGAAACTTCAAATATA
TCTTACACAATCTTGTTTGTACAAAAATACAAGTTAAATATAAACATAAAGCAATC
ATGGTAATTTTATGCAAATCTGTTTTATGTGATCATCAGTTATATATAAAAGTTTC
TCAGTTCTGTTATTTGTGAAAAGATCAATACCAGATTGAATGACTACCTATTGGCA
AAGGGCCCTAAAAAGCTTACTTTAGCACTCATCTTTTACATGGTTAAATGCATTTC
CTAATTTGAGATCACCTAAACACTGGAAAAGAAAAAAAATGAAAGGGCAGTATGTC
CATAAACCAACAAATAATTTGGCTGTAATGTATCATAAAACACAAACCCCACACAT
CTGTACAATAAACATTATGTATTACATACACACAACACACACCCAGTCATAAAGCC
TAATGATGTGCTGCTTCCAGTTCAATATTCAGCTGTGCATTTTTTCTTATTTCATC
AAATGAATAGCTTTTTGTCACC


Sequence ID 586

GTAAACTGTTCTCTCCGAGGGAAAAAATGGAAGTTATCCTCACAGTTCACTGCCGT
GGTATTTCTTCTGTCCCATGCTTTGCATGACTGCCATGGTACAGCCTTGTTTCAAA
CTGTTCACTGTGATCTGTGGGTCTTTGAGTTTCAGTGAGTTTGCTGAAATGTCGAA
GAAGTAGTTCCAAACTTCAATGTTCAATGAAATTTTTGTTCAAGTTTGAAATGGAG
AGAGCAGCTTTAAAAGGTACTAAGCCTTTTACAAATTGGTGAGTACTGGCACATGA
GAT

Sequence ID 587

TTTTTTTTTTTCCTTAAAAGGTAACCCCTAAACACAGCTAAAACTATGCCATCAGC

TGACTCCAAGGNACACACAGTCCTGTATCTGGAACTACTGAGTGGCAGGCATCTTT
CTCTGCCTCTGACAGTGGAGTCCCCATCACTGCAGAGCATAGCCAAAGGAGTCAAA
GGTCTCAGCGGGTCACTGCCTTATCAACCCTCACCAGTCCCTTATGTTTTTTAATA
TTTTATAATCTTGACATGACACCAAGATGCTTTAATAAAAAAGCACCTCTAACTCG
GTCTTGTATTCACTTACCTTGAGCCTGGGACTTCTCTAGGCTCCTGAGGCAAAAAC
AGGTAGAGGGGAGATGGTGGAACATAAAACACAATTTTGCTTGGCACCCACCTTGG
CGTCTGTCCCCATGACCAGGTCTTTCAATTCGATGATTTTGTCATTGATGGAGGAG
CGATATCGTTTCTCAATGATATTATGGGTTGTCCGCCTTTCTCCTTCTTTGGGGGG
CTCAAGCTGCTTGACTCCCCCAGGTACCTGCTTAATGGGGCACTTTCTCTTGCCCC
ATCATTACAGGCATTGTGGTCAGAATGGTCCCACTGCTGCCCACCAGGGTCTA

Sequence ID 588

CTAGTCTTTTCATAGTCTGCATAGAGTCTGGCCATTACCATCAGTTTTTAAGATGT
CCATATTGTGGCCGGGCGCGGTGGCTCACGCCTGGTAGTCCCAGCACTTTGGGAGG
CTGAGGCAGGTGGATCATGAGGTCAGGAGATCGAGACCATCCTGGCTAACACGGTG
AAACCCGTCTCTACTAAAAAAAATATTAAAAAATTGGCCAGGCCTGGTGGTGGGCG
CCTGTGGTCCCGGCTGCTTGGGAGGCTGAGGCAGGANAATGGTGTGAACCCGGAAG
TCGGAGGTTGCAGTGAGCCAAGATTGCACCTGGGCAACACAGCGAGACTCCGTCTC
AAAAAAAAAAAAAA

Sequence ID 589

CAATTATTTATTACCTTTCCATTTGTTCGCCTGATGATGTGACAATGCATGGTCTT
TGTGCATGCTGCTAGACACTTTTCTTTCCCAGCCGAAAAGTCTATTATGTAATTTT
TACATTCATAATTTTAATGTGGATGATCAGGATTAAATCAAGATATATATCTGGAA
CCTCTTATAAATGGAGCACTTAGAAATTTGTTGTTCTGCACTTAACCTAGAGAGAG
AAAAAATGCTTTTCTTTGTGAAAAATCTGAATTCCTGTCCTGACCTTCTGTGATGT
GGAAACCCTAGGCTCTGAGACACACTCTCTGGTGTCTGAGACAGAACCAAAGCAAT
AACGTTGTGATGCCCACAGGCCTGGAGCCAGCTAGCGACCTTGTGCCGCCCAGCTG
TCCATGGCCCGTGCAGAGCAGAGGACAGTGAGTGTCTGCACTGAGAACCTTAAACC
ACAGTTGAACATACCCACACCTGTTTGTCTTAAGCTATAGTGTAAAAACAAAGTTT
GGGCTCTGAAAATTTAACTGAAAAGATTTCCTTGTT

Sequence ID 590

GTGGCAGCAGGCGCAGCCCAGCCTCGAAATGCAGAACGACGCCGGCGAGTTCGTGG
ACCTGTACGTGCCGCGGAAATGCTCCGCTAGCAATCGCATCATCGGTGCCAAGGAC
CACGCATCCATCCAGATGAACGTGGCCGAGGTTGACAAGGTCACAGGCAGGTTTAA
TGGCCAGTTTAAAACTTATGCTATCTGCGGGGCCATTCGTAGGATGGGTGAGTCAG


ATGATTCCATTCTCCGATTGGCCAAGGCCGATGGCATCGTCTCAAAGAACTTTTGA
CTGGAGAGAATCACAGATGTGGAATATTTGTCATAAATAAATAATGAAAACCTAAA


Sequence ID 591

CAGCAGCAGAAATGTTTGCAAGATAGGCCAAAATGAGTACAAAAGGTCTGTCTTCC
ATCAGACCCAGTGATGCTGCGACTCACACGCTTCAATTCAAGACCTGACCGCTAGT
AGGGAGGTTTATTCANATCGCTGGCAGCCTCGGCTGAGCAGATGCACAGAGGGGAT
CACTGTGCAGTGGGACCACCCTCACTGGCCTTCTGCAGCAGGGTTCTGGGATGTTT
TCAGTGGTCAAAATACTCTGTTTAGAGCAAGGGCTCAGAAAACAGAAATACTGTCA
TGGAGGTGCTGAACACAGGGAAGGTCTGGTACATATTGGAAATTATGAGCAGAACA
AATACTCAACTAAATGCACAAGTATAAAGTGTAGCCATGT


Sequence ID 592

TACTCAATGAAAAACCATGATAATTCTTTGTATATAAAATAAACATTTGAAAAAAA
AAAAAAA

Sequence ID - 593                           nt:        565

CAGGATCAAGGTGAAAAGGAGAACCCCATGCGGGAACTTCGCATCCGCAAACTCTG
TCTCAACATCTGTGTTGGGGAGAGTGGAGACAGACTGACGCGAGCAGCCAAGGTGT
TGGAGCAGCTCACAGGGCAGACCCCTGTGTTTTCCAAAGCTAGATACACTGTCAGA
TCCTTTGGCATCCGGAGAAATGAAAAGATTGCTGTCCACTGCACAGTTCGAGGGGC
CAAGGCAGAAGAAATCTTGGAGAAGGGTCTAAAGGTGCGGGAGTATGAGTTAAGAA
AAAACAACTTCTCAGATACTGGAAACTTTGGTTTTGGGATCCAGGAACACATCGAT
CTGGGTATCAAATATGACCCAAGCATTGGTATCTACGGCCTGGACTTCTATGTGGT
GCTGGGTAGGCCAGGTTTCAGCATCGCAGACAAGAAGCGCAGGACAGGCTGCATTG
GGGCCAAACACAGAATCAGCAAAGAGGAGGCCATGCGCTGGTTCCAGCAGAAGTAT
GATGGGATCATCCTTCCTGGCAAATAAATTCCCGTTTCTATCCAAAAGAGCAATAA
AAAGT


Sequence ID 594

CAGAAGAGTAAGCAAATCTCAAAGCAGCGAAAGGGAAGAAACTAAAAAAGGTAGAG
CAGAAATAAGAGAAAATAGAGAAGAGAACAATTGAGAAAAATAATTGAAACCAAAA
GGTGGTTCTTTGAAAAGCCTAACAAAATGGACACATCTTTAGTTAGAGTGACCAAG
AAAAAAGGGCAGTGACTCAGATTACTTCATTCAAGAGTGAAAGAGGGCACATCACT
ACCAATTTACAGAAATAAAAAGGATTATGAGGAAATACTACAGATAATTGATGACA
TTAACTTAGAAGAATATATTTCAAGAAAGACACAAACTACTGAAACCGACTCAAGA
AGAAACAGAAAATCTGAACAGACCTATAAAAAATAGAGATTTAATTGATATTCAGA


AAGTTTCCCAAAAAGAAAAGCACTGGCCAAGATGACTTCACTGGTGAATTCTATCA
AGTGTCAAAGATGAATTACTGACATTCATTCACACTCCTTTAAGAAATAGAAGAGG
GGACATCACTTTTCAAAGCATCGACATTCTAATCATTAGTCCCTTGGTTTCCTGCT
CCCAAAGCCAGGTGATGTATCACAAAAAAACCCCTACAGACCCACTGGGCACAATG
GCTTTATGCCTAT


Sequence ID - 595                           nt:        98

CTTTGCTCGAATNGTCAGATAAGGATTCTGTGAANGGAGATGAGATTTCCATCCAT
GCTGACTTTGANAATACATGTTCCCGAATTGGGGNCCCCAAA

Sequence ID 596

CTCAAGTGTTCCCTCAGCTTAGGCTTTGTTTAAATGATCCCACCCAGGGGCGATGG
TAGGGAACAACAGGGTCACTAAACTATTTGGCTGGCTACAACTCTGGGAAATGGTA
AGACAGGGAAAGGCCATGTTGTTCATTCCCTTGTGCAGATCTAGGGAGAACCGCAG
AGAGAACAGTTAGCATTTCTTGTTCAATGAATTATCCTATTAAGAACACTGGATGT


Sequence ID 597

CGGNCGCGGTCGACGCTACTCCTACCTATCTCCCCTTTTATACTAATAATCTTATA
AAAAAAAAAAAAANAAAAAAAAAA


Sequence ID - 598                              nt:      362

GGCATGTGCCTGTAGTCCTAGTTGCTGAGGTAAGAGGATTGCTTGAGCCCAAGAGT
TCAAGGCTGCAACAAGCTTTGATTGCGCCACTGCACTCCANCCTTGGCGACAGACT
AAAACGCTGTCTCAAAAAAAAAACAAAAACGACNAAAAAAAAACAAAACAGAAAAA
ATTAACTTAGGCAATGACAGTCCCTGGCAAATGCTGGGAGGGAGGCAACANTGGTC
AAGGAAGGTAACCCTGAANCAGGACTTGTAAAGCAAATAANATTGGGAGGCCAAGG
TGGGTGGATCACNAGGTCAGGAGTTCGAGACCAACCTGGCCAACATAGTGAAACCC
CGTCTTTCTAAAAATACAAAAAAATT


Sequence ID 599

GACAAAAGAACCATTTGGATACATAGGTATGGTCTGAGCTATGATATCAATTGGCT
TCCTAGGGTTTATCGTGTGAGCACACCATATATTTACAGTAGGAATAGACGTAGAC
ACACGAGCATATTTCACCTCCGCTACCATAATCATCGCTATCCCCACCGGCGTCAA
AGTATTTAGCTGACTCGCCACACTCCACGGAAGCAATATGAAATGATCTGCTGCAG
TGCTCTGAGCCCTAGGATTCATCTTTCTTTTCACCGTAGGTGGCCTGACTGGCATT
GTATTAGCAAACTCATCACTAGACATCGTACTACACGACACGTACTACGTTGTAGC
TCACTTCCACTATGTCCTATCAATAGGAGCTGTATTTGCCATCATAGGAGGCTTCA


TTCACTGATTTCCCCTATTCTCAGGCTACACCCTAGACCAAACCTACGCCAAAATC
CATTTCACTATCATATTCATCGGCGTAAATCTAACTTTCTTCCCACAACACTTTCT
CGGCCTGTCCGGAATGCCCCGACGTTACTCGGACTACCCCGATGCATACACCACAT
GAAACATCCTATCATCTGGAG

Sequence ID - 600           nt:     595

TTCAAATTCTTGNTAANAGTCTTTGTTCTGAATTTTACTTTGTCTGTTATTCCTAT
AGCCTTTCCAATTTTTCTTTCGCTTGGATTTTACGTGATAAGTTTTTTTCCCCCATTT
TACTTTTANCAACTCTATATTTTTTAGTTGAGGTTGGGTTTCTTGTAAACAGCATA
TAATTTGGGTTTTTTAATCCAATCTGAAAATTAATGTCCTTAATTTTGTGTTTATA
CCATTTACACATAATGTACTCATATATAAGGTTTAACTGAAACCTACTATCTTGCT
AGTTGTGCTCTACTTGAATTTTTTTTTAGTATTCTGTTTTAATTGACCAACATTTG
ACTGTATCTCTTTGTGTAATTCTTTTACAGGTTGCTGTAGGCATGACAATATATAC
ACTTAACTTTTCTCAGTACACTGAGAGTTGAAATTGTAGTACTTCGAGGAAAACAT
AGAAAACTTGCAATGATATCGGTTACATTTTACCACCTCCATATGTTGCAATTATT
AAATGTATTAGATCTGCCTACCTCGAAAACCCATCAGTCTTTTAACTTTGCTCTCA
ATGGTGATTCATATTTTTAAAAAAACTTGAGGCAA


Sequence ID - 601           nt:     522

TCGACCGGGTTTGGAGCAGTGCCTTGTTTGCTGTGCAGCGGATACTCTACAGGTAC
ATTTCCTTTTTGGAACCAAAAGGGAGGGATTTGACAATATTGATGGTAGATCTTTT
TTCTTTAGCAAGAATTAAGGATTTTGGTGGGTGGGGGGAGGCTTCTGTGGGGACCA
AGACAATGTACTGTCAGTCAGGATTTAAGTCGAACTACCTCATCCCTTGCCCCAGA
GAACAGTTGATCGTGTTTTAAACCAAAAGGTGCGGAATGGAGAGAGGGAGGCGGTG
CATTGCAGCTTCCGATAGAGCTTTTTATTTTTGGATATCAGGAACCAATTTTGAAG
ATTTCTTAAGAAAGTCATTTACATCAGGGACATGAAGAGCAAAGTAGGTATTTTTG
GTCAGTACTTGAATTTGATAGGCTTTATGCAAACAACTCTCCCTCTGCTGGAGTCT
GGCAAGTTTGCTTTTCACTGGACGCTAATTCAAGTGCCATACAAAACTAAAATAAN
AGTTTTACTTATAACACA


Sequence ID 602

CAGAAATCGCAATTGAAGACCAGATTTGTCAAGGTTTGAAACTGACATTTGATACT
ACCTTCTCACCAAACACAGGAAAGAAAAGTGGTAAAATCAAGTCTTCTTACAAGAG
GGAGTGTATAAACCTTGGTTGTGATGTTGACTTTGATTTTGCTGGACCTGCAATCC
ATGGTTCAGCTGTCTTTGGTTATGAGGGCTGGCTTGCTGGCTACCAGATGACCTTT
GACAGTGCCAAATCAAAGCTGACAAGGAATAACTTTGCAGTGGGCTACAGGACTGG
GGACTTCCAGCTACACACTAATGTCAATGATGGGACAGAATTTGGAGGATCAATTT

ATCAGAAAGTTTGTGAAGATCTTGACACTTCAGTAAACCTTGCTTGGACATCAGGT
ACCAACTGCACTCGTTTTGGCATTGCAGCTAAATATCAGTTGGATCCCACTGCTTC
CATTTCTGCAAAAGTCAACAACTCTAGCTTAATTGGAGTAGGCTATACTCAGACTC
TGAGGCCTGGTGTGAAGCTTACACTCTCTGCTCTGGTAGATGGGAAGAGCATTAAT
GCTGGAGGCCACAAGGTTGGGCTCG


Sequence ID - 603                          nt:       624
GACACACGAGCATATTTCACCTCCGCTACCATAATCATCGCTATCCCCACCGGCGT
CAAAGTATTTAGCTGACTCGCCACACTCCACGGAAGCAATATGAAATGATCTGCTG
CAGTGCTCTGAGCCCTAGGATTCATCTTTCTTTTCACCGTAGGTGGCCTGACTGGC
ATTGTATTAGCAAACTCATCACTAGACATCGTACTACACGACACGTACTACGTTGT
AGCCCACTTCCACTATGTCCTATCAATAGGAGCTGTATTTGCCATCATAGGAGGCT
TCATTCACTGATTTCCCCTATTCTCAGGCTACACCCTAGACCAAACCTACGCCAAA
ATCCATTTCACTATCATATTCATCGGCGTAAATCTAACTTTCTTCCCACAACACTT
TCTCGGCCTATCCGGAATGCCCCGACGTTACTCGGACTACCCCGATGCATACACCA
CATGAAACATCCTATCATCTGTAGGCTCATTCATTTCTCTAACAGCAGTAATATTA
ATAATTTTCATGATTTGAGAAGCCTTCGCTTCGAAGCGAAAAGTCCTAATAGTAGA
AGAACCCTCCATAAACCTGGAGTGACTATATGGATGCCCCCCACCCTACCACACAT
TCGAAGAA


Sequence ID - 605                     nt:       338
ACCTGAGGCCTCGGTGGGGCCAGTGCGACGCTGGCTTAAGGAGCTGGAGGGGTTCC
TAATACACATTTAATTCAGTTTCTCTTCCCTAAGAGGCTGCCGGAGTTGGGGCCTC
CTCCAGCAGAGACCCTCGGACCCCTGCAGGGCCTGGACTTGGGGTGAACAGGGCTT
CAGTCAGCGCAAGTATTCCATTTGCATTTGGTAATTTTTCATGCCACCTATTTATG
AATATATAAATCTTTATACCAAATCTATTTTTTAAAACATGGAAAAGTTGCCTTTA
TGGAAACTTGGCAGAGCCAGAGTGTACACATTCCTAAACCATTAAACAGATTTCTA
TA

Sequence ID - 606                    nt:        556

```
GGATAATGATACCTCTGACCTTTCTTCCTTTTGGGAAGTACTTGAGTGTGCAGCTG
CATGAGGCCTCAGCAGGAGAGAGATTTTAGGTCCAAGAAGCTATACCAGTAGGACA
AGGCAGGAAAATACTACACTTTCAGGATCAAGCCCCTCTGACTCTCATTTGGAAAC
TGGATGTTTGCTAAGCACCTGCTTCTTAAGGATGCCGAGGGATTTAATGATACTCC
CAGAAACCTGGAGAGATTAATGGGGCCTATGGAGAAGTGCTCTGAACTCAGTGTTG
GGACTTGAATAAAATTAACCATTGTCATGTTTTCAGAACAACTAAGCTGTTTTATA
TTTCATGTGCATGAAAGCCCTAGAACTAAGTTGTGTTATTTCCAGAAATGAAATAG
```

```
ATCCCACAGTTAGATGATGTGGCCATTAGGAAGTACCAAATTTATAAAAATCACTG
GAGGTCTGTCTGAGCAGTACCTAATAAAATATAGTATACTGAAAGTGAACAGATCT
TTGTCTCTTTCTTTGGCTGCTTGATACTTTATCTGTGTCTGCCGGACAGTGC
```

Sequence ID 607

```
CAATAAAAGCAGGTTAACCTCAATGATAGCAGTTAAAATGTTCTATCTTATGTATT
TCTTTTAAGTATTACCATTATGGTGCTACTGAGCGTTTTCTTTTGGTAAAAAGAAA
AATGCCATGGGCTGCAGTCTTCTTCCATCACTTTTCCCTACCAGGTCCATTAATAT
GCTTATAACACTAGTGCCAGTTATTTTATTTGATAATGCTTATGGTATTTGTATAT
TTGTTTGCATTCCAATTTTGTTTAATAATGAGTGTGTAAACTGCATACGTTAAATA
AATGTAAATACTAATGTACTGCTGC
```

Sequence ID 609

```
TTTTATTACCCAAGTTTTAACCTCTGTCTGGTGATTTGTTGTTGTTGTTGTTGTNG
TTGTTGTTGAAGTTCAGGCTGCATGTGGGATAGGTTTGCTCAGGCATACTTCTTAG
GAAGTAGTCACTTGCATGACTGTTTTTGGGATAACTCTTTGAGTATTTGGAGAGGT
CTATTGTAACTTCTGAAAGGCATTGTTTTTACGTATGAATGTTCTAAAATTCATTC
TAAATGGTCATGAAAAGAAAAGGATTCACATTTTAGAATGGCAATAGTCCCTGAGG
ACTATTATGTCTTTTAGATTTCCTGTGGGTTTCTAGGAATGTTAGTGTAACTTANA
TTTCCACCTACCTGATTTCTGGATGTGCCTATTGGAACTTGCTGAGATCTTTTTTTT
TTCCTTAACATGTTGTCCCCTTGACCCGTACTTCGAAACTAAACATATTATTTTAT
TTGCTTACACTTCAGGAGGCAATTGGCAGACACCAGGCCAACAGTCT
```

Sequence ID 610

GCTCTGACCCCAGTTGGAAATGTATCTGTACTTTGTCCGGCTTCCACTCAAGGACC
ATTTATGACATTGCTTGGTGTCAGCTGACAGGGGCTCTGGCCACAGCTTGTGGGGA
TGACGCGATCCGCGTGTTTCAGGAGGATCCCAACTCGGATCCACAGCAGCCCACCT
TCTCCCTGACAGCCCACTTGCATCAGGCCCATTCCCAGGATGTCAACTGTGTGGCC
TGGAACCCCAAGGAGCCAGGGCTACTGGCCTCCTGCAGTGATGATGGGGAGGTGGC
CTTCTGGAAGTATCAGCGGCCTGAAGGCCTCTGAGCTACCTCGACTTTGGACAGAG
TAATGACTCCCCAGAAAACGTCATATAAGACTTTACCAGCCCCTGAGAGGACCAGG
AGGAGCATCCTTGACCTTCATTTAACTTGGCTCACTTCTCTTCANACTTGGGTAGA
AGTGCAGAGCCACAAAATTGCTTTCCTTCCCCGCCTTTGACATGAGGCCTTCAGTA
AAG


Sequence ID 611

TGCAGGATCCGTCGACT


Sequence ID - 612                                    nt:       576

GAGAAATATAAGATTATGTATAGATCAAATCTACCTCTATTTGGTGTCCTGAAAGA
GATGAGGAGAATGGGACAAACTTGGAAAGCTTATTTCAAGATAACATTCCTGAGAA
CTTCCCCAATCTTGCTAGAGAGGCCAACATTAAAATTCAGTAAATGCTGAAAACTC
CAGTAAGATATTTCTTAAGAAAATTATTCCCAAGATATATACTCATCAAATTATCT
AAGGTCAAATGAAGGAAAAAATTTTATAGGCAGCTAGAGAGAAATGTCAGGTCACC
TACAAAGAGAATGGCATAAGACAAAAAGTAGAACTCCCAGCAGAAACTCTAAAAGC
CAGAAGAGATTAGGGGCCAATATTTAACATTCTGAAAGAAATTCCAACAAGGAATT
TCATATCCAGCCAAACTAAGCTTCATAATTGAAGGAGAAATAAGATATTTTCCAGA
CAAGCAAATGCTGATGAAATCCATCACCACCAGACCTGCCTTATAAGAGCTCCTGA
GGGAAGCACTAAATATTGAAAGGGAAGAACTTTATGAACCATTTCAAAAACACATT
TAAGTNCACAAAGCAG

Sequence ID - 613                          nt:        341

CCTTATTTTACAGGTGAAAAACCACGAATCAGATAGATTTTTATTTGCCCAAGTCA
CATAATATTAAGAACAGGCCAAGTGTGGTGGCTCATGTCTGTAATCTGAGCACTTT
GGGAGGCTAAGGCGGGTGGATTTCCTGAGCCTAGGAGTTTGAGATCAGCCTGGGCA
ACATGGCGAAACCTCATCTCTACAAAACATACAAAAATTAGTCAGTGTGGTGGTGA
GAGCCTGTAGTCCTGGCTACTCGTGAGGCTGAGGTGGGAGCATCACCTGAGCCTGG
GAAGTCGAGGCTGCAGTGGCAACAGAATGGGTAACCTGGACATCAGAGTGAGACCC
TGTCT


Sequence ID 614

CTCACACCTGTAATTCCATTACTTTGGAAGGCTGAGAGAGGAGGATCAGTGGAGCC
CAGGAGTTTGAGACCAGCCTGGGCAATATAGGGAGACCCTGTCTCTACAAAAATGA
AATAGCCAGGCGAGGTGGCATGTGCCTGTGGTCCCAGCTACTTGGGAGACTGAGGT
GGAAGGCTGCCTTGAGCCCAGGAGTTCCAGGCTGCAGTGAGCCATCATTATGCCAC
TGCACTCCAACCTGGGAGACAGAGTGAGAGAGACCCTGTCTCAAACAAACAAACCC
AAAATAGGCCAGGCACAGTGACTCATGCCTGTAATCCCAGCACTTTGGGAGGCTGA
AATAGGCGGATCATTTGAGGTCAGGAGTTCAAATTCAAGACCAGCCCGGCCAACAT
GGCAAAACCACATCTCTACTACAAATAAAAAATTAGTTGGGTGTGGNGGAGCATTC
CTGTAATCACAGCTATTCAGGAGGCTGAGGCATGANAACCGCTTCA


Sequence ID - 615                          nt:        379

TAAATTTAAAACATTTTAATTAGCTGGCATGATGGCATGCACCTGTAGTCCTACCT
ACTTGGGAGGCCAAGGCAGGAAGATTGCTTGAGCCCAGGAGTTTGAGCTTACTGTG
AGCTGTGATCACACCACTGCACTCCAGCCTGGGTGACAAAGGAAGACCGTATTTCT


AAAAAATAAAAAATACAAATACAACTACAAACTAGCACTAGACCAACAGTGACTAT
GTACCATGAACTGAGGAATATTATTAATTCCACCATTTGCATCTGAGGTTAACAAT
ATGTCAATGACTTAAATAACATCATATCTCTGAGAGTAATTTCTCCTATATTTCCA
TGACAAATGTTAGATAATTTTCCATTTTTTCCATTCAACAAAA

Sequence ID 617

TTTTCAGGCATGTCAGAGAAGGGAGGACTCACTAGAATTAGCAAACAAAACCACCC
TGACATCCTCCTTCAGGAACACGGGGAGCAGAGGCCAAAGCACTAAGGGGAGGGCG
CATACCCGAGACGATTGTATGAAGAAAATATGGAGGAACTGTTACATGTTCGGTAC
TAAGTCATTTTCAGGGGATTGAAAGACTATTGCTGGATTTCATGATGCTGACTGGC
GTTAGCTGATTAACCCATGTAAATAGGCACTTAAATAGAAGCAGGAAAGGGAGACA
AAGACTGGCTTCTGGACTTCCTCCCTGATCCCCACTCTTACTCATCACCTGCAGTG
GCCAGAATTAGGGACTCAGAATCAAACCAGTGTAAGGCAGTGCTGGCTGCCATTGC
CTGGTCACATTGAAATTGGTGGCTTCATT

Sequence ID - 618                                        nt:        598
GATTAACTTTCATTTTAAGCTCTTCTCTACTAATTCTGTTCGTATGTTTATTCATT
TTGCGTTGATCATATTTTGTACACCAGGCACTCTTCTCAGTTTTATATGTGTGTTA
ATTTACTCCTTTCAAGAGCCCTATGATACATGAATTTATCTCCATTTTATAGATGA
GGAAATTAAGACCTAGAGTTACTGAACTTGCCCAAGGTTATACAGCTGATGGGTAG
GGCCAGAACTTTGCCTCAGAGAATCTGAATTTCCAAAAAATAACCTAAAAGAGAAA
TTTAAGTACTAATTAGTAAGCAAAGAAATGCACATTTAAGGAAGACAGTGCACATT
TAAGGAAGACAGTAACCTTTTATCTATTAGAGAAAAACACACATTCTGTCTTTAAC
ACACACATAAATCTTATATTGGCAGGGATTTTCTTTATTCAGCAATTATTTATTGG
TTGTCTGCTTTGTGGTACACATAAATGCTGGGGATAAACACTTAATAAAATATACT
TCCTTCTCTTGAATATCTTGCACTTTAAGTGGGAAGGTAAGTCAACAGAGTAGAGG
TGATATATCCAAGTGATAGACTGTTTCATTGCCAGTAG

Sequence ID 619

GTTGCCTGAGAGTGACCTTTGCATCTGCCTGTCCAGCCAGCATGGAACCAAAGCGG
ATCAGAGAGGGCTACCTTGTGAAGAAGGGGAGCGTGTTCAATACGTGGAAACCCAT
GTGGGTTGTATTGTTAGAAGATGGAATTGAATTCTATAAGAAGAAAGTGACAACA
GCCCCAAAGGAATGATCCCGCTGAAAGGGAGCACTCTGACTAGCCCTTGTCAAGAC
TTTGGCAAAAGGATGTTTGTGTTTAAGATCACTATGACCAAACAGCAGGACCACTT
CTTCCAGGCAGCCTTCCTGGAGGAGAGAGATGCCTGGGTTCGGGATATCAATAAGG
CCATTAAATGCATTGAAGGAGGCCAGAAATTTGCCAGGAAATCTACCAGGAGGTCC
ATTCGACTGCCAGAAACCATTGACTTAGGTGCCTTATATTTGTCCATGAAAGACAC

TGAAAAAGGAATAAAAGAACTGAAT

Sequence ID 621

TGGTACTGAACCTACGAGTACACCGACTACGGCGGACTAATCTTCAACTCCTACAT
ACTTCCCCCATTATTCCTAGAACCAGGCGACCTGCGACTCCTTGACGTTGACAATC
GAGTAGTACTCCCGATTGAAGCCCCCATTCGTATAATAATTACATCACAAGACGTC
TTGCACTCATGAGCTGTCCCCACATTAGGCTTAAAAACAGATGCAATTCCCGGACG
TCTAAACCAAACCACTTTCACCGCTACACGACCGGGGGTATACTACGGTCAATGCT
CTGAAATCTGTGGAGCAAACCACAGTTTCATGCCCATCGTCCTAGAATTAATTCCC
CTAAAAATCTTTGAAATAGGGCCCGTATTTACCCTATAGCACCCCTCTACCCCCT

Sequence ID 622

TTTTTCTTGTTTTTGTGTGTCTACCTTGGCATATACTAAAGGAAGGTGTGTATTCA
TTTATTACATGATATCTCTGGGTTATAATTATTTACATATATGAATTTGAAAGAAA
GATTGAGAGGGATATGTGTGACCTTTGTTTCATTATGATCATTTACATGACTAAAG
ATAAAGATCATATGTCTGATTTTCAGTTTAATGGCAAGTTACTTAAAATAAATGAA
ATATGTTTTTATTGTTTTCGTGGGTTTGATGCTTTGTGTTTTATTTCAAGTAACTT
GAGAATGCATTGTGTTTGGTACTGTTTTTTATGAATATCATTAAAAATTTATTTAA
GGAGAGAGTAATTTTGCAATAATATTTTTGATTTATTTGAAAATAAAATTCAAGAT
AAATGAAATAATTGAAATTTTCTAAAGAAGGAATTGAATATATTTTTACATTTGAA
TGAACTAAGGATTAACTGAACCATTTATATATAGTACTTTCAGAACTGAATGTCTT
AAATGATAAAGCTCTAATTGGTTAAAGTGACTTTCTTTCAAGTCAAAGAACCCAGA
AACTGAATAGATGATCTAACTACTGCCACTGAGGTTTTGGATTAGTGAGTATAAAT
TT

Sequence ID 624

TGCAGGATCCGTCGACT

Sequence ID 625

GACAATCAGAGCAGATCTTGGGCTTCTGTGGCTCATCTCAGCCCTTTATAACTGGC
CTGAGAAGAGGGTTTATCTACTTGTGCAAGTGGCCCAGAAATCTCACTCGTACATG
AGGCTTTGGAACATCCTTGCAAAGGTACGCTGAAAGCAAATTGCTGTTTTCCTGGT
GGTTCTGCACGTTTCCTAACTTTTATCATAGTTTGATTTTCATTATTTAAGAAAAA
ATAAAAAATCCAAAGACCATAAGATGGCATTAGATTTTTTACCATTAAATTATTAA
TGCCTATTTGGTGCTCATAAAGATTAATCATGTCACGCATGTTTCCAATCTTTCTT
TTGCAGTATATTATTTTCTAAAAATTGTTACATGCAAATTTAAACCAAGATTTATC
AGTA

Sequence ID 626

TTGGAAGAAATAAACCAAGGCAGAAAAATTTTAAATGGCCAAAATAAATTGTATTG
CTAACTTAGATGGCCACAGATGGGGGCAGGGGTGGAGAGAGGAGAAATTGAAAACN
CCACAAAGACCCCGCAATGGCTAGAACTTGAAATCTCTGGATATTGCAACAATAGC
AGCCTCCTTAAGTCAGCAAAAAGATAAAGATTGATCCAATGTTCTATATTACAGAA
CAGAGCAGATTGTCAATATAGCAAATAAAGTTACCGTTGAGTGGACTGCGCTGTNT
AAGCTGCTTGGTTGGCCTTAAGTGCCGACAATTAAGAGATGAAGGCAATGAGAACT
GAAACAAACATTTAAGTTCAAGACCCAGTTTACTGACACTGGGACTATTACTATAT
CTCTTTGGGCCTCAGTTTACTTATCTGTAACATTAAGAGGTTGGATTACATGATGT
CTCACGATTCTTTTTTTTTATTTAGAGATGGGGTTTTGCTCTGTTGCCCAGGCTGG
AGTGCAGTGGCATGATCATAGCTCACAGCAG


Sequence ID 627

CCAGCCTGTCACTGGCCTGGCCAAGGAGGAGAGACAGGCCAGGGATTCTGGTCCTA
ACTCTACTGGCCACACTGTGTGGCCTGAGACCCCCCTTTCCCTCCCAAGCCCCTGC
CTCCGCATCTGCGTGGTGAAGGCCATTGGCCCTCATCGGTGGATCTGCGTTTCCTC
GGGCCTACACTGTCTAGGATTGTGCGGGGCTGGTGAGAGAACAAGATCTCTTCCGT
GTTCAAGGCAGACTTCCTGCCCCCTGCACCCTGCTCTCTCCCAGGCCTTGAGGTCA
GTGTGAGCCCCAAGGGCAAGAACACTTCTGGAAGGGAGAGTGGATTTGGCTGGGCC
ATCTGGATGGAAGGTAAAAAAAAGAAAATCCCTTGAAAGGAGATTGAGGGAAGTTT


Sequence ID - 628                          nt:        419
AAGAGAAAGGACTCAGTGTGTGATCCGGTTTCTTTTTTGCTCGCCCCTGTTTTTTGT
AGAATCTCTTCATGCTTGACATACCTACCAGTATTATTCCCGACGACACATATACA
TATGAGAATATACCTTATTTATTTTTGTGTAGGTGTCTGCCTTCACAAATGTCATT
GTCTACTCCTAGAAGAACCAAATACCTCAATTTTTGTTTTTGAGTACTGTACTATC
CTGTAAATATATCTTAAGCAGGTTTGTTTTCAGCACTGATGGAAAATACCAGTGTT
GGGTTTTTTTTTAGTTGCCAACAGTTGTATGTTTGCTGATTATTTATGACCTGAAA
TAATATATTTCTTCTTCTAAGAAGACATTTTGTTACATAAGGATGACTTTTTTATA
CAATGGGAATAAATTATGGCATTTTTT

Sequence ID 629

CTGAGAGTCACTGTGTTTTTAGCCAAATCTAAGGGAGAAAATGAATATTGATAGCA

GCATGCTGTAGCCAGCTCCTTAAAGGAAGGATGGTGCCTGGTACAGAGTTAGAGTT .

AGTGCTTCAGTAAATAATGAATGTGTGCTAGGTAGGTTCTGCTGGGTAGGCTGCAT

GCATTGACCAATTTATTCCTCCTTGTTTCAAAACAGGATTTAAGGGCACTTATATA

TATATATTTTTTAGTTTTTTTAATGTAAATGAGAGAATAAAGATATATATATATGT


CTATATATGTATATATGTATATATATGTCTATATGTCTATATGTATATATGTCTAT

ATGTATATATGTGTGTGTGTATATATATATATATATATATAAGTTTTCTGTTGCTA

GCATAACAAACTACCAGAAACTTAGCAACTGAAACAACATGAATTTATCTTACGGT

TCTATAGTTCAGAAGTCTAACGTGTCACTGGGATGAAATCCAGGTTTCAACAGGAC

TGGGTTCCCTTCTAGCTCATTCAGCTACCTGGCTCATTCAGGTTGTNGGCAGAATA

TACTTCCATGAAACTGTAGGGCTGAGACCCCGTTCCTTCCTGGCTATCATCTGAAA

ACTTTC


Sequence ID 630

AGGCGCAGCCCAGCCTCGAAATGCAGAACGACGCCGGCGAGTTCGTGGACCTGTAC

GTGCCGCGGAAATGCTCCGCTAGCAATCGCATCATCGGTGCCAAGGACCACGCATC

CATCCAGATGAACGTGGCCGAGGTTGACAAGGTCACAGGCAGGTTTAATGGCCAGT

TTAAAACTTATGCTATCTGCGGGGCCATTCGTAGGATGGGTGAGTCAGATGATTCC

ATTCTCCGATTGGCCAAGGCCGATGGCATCGTCTCAAAGAACTTTTGACTGGAGAG

AATCACAGATGTGGAATATTTGTCATAAATAAATAATGAAAACCTAAAAAAAAAAA

AAAAAAAAAAAAA


Sequence ID 631

TNCACTCACACACTCCCAAACCTTAACAAACACATACATGTGCAGCCAACCCAATG

GGCCAGCCTCTTTTATGCTCCTCACATGTTTCCTTTAACTGGAATACCCATGACAG

·CTCCCTACATAGTTACTTGTAAACTCCTCCTCTCTGTATAAGTTTTCCTGAATTTT

TTTGATAAAATTAAGTTGTGCCACCCCTTTATGCTCTCTTANAACTTTGTTCTGTT

CTCATGGCTGTTCTGCAACGAATCTCATTGTGTTCTCCTACTCAATTACATTCCTG

CGTCTCCCACTAGATGGCAGACTCTTTGAGAGTAGGAGATTCCCTTGTTATCTCTG

GATCCCTGGCACTTGCAGAAAGCCTGTTACGTAATAATTGCTCAACAATTAGTTTT

TAAATAAATGAATTATTTTTAAAACGCCAAAATTACAATGATTGTGCATTAAGTGA

AAGATGACCATCTAAAAACATAAAGCCATGCTTCATGACATTGGC

Sequence ID 632

GACCATTCAGGGAAATTTTATAAAAAATGCAGATACTGTCTTGAGCAGATCGAAAT
GCCGATGAGGTGGATGCAATTTCCTTTTGTGCAAGCAGTGCACGGTGCCCCCCCCT
CGGGTGTCCGTGCTGTGCCTTAGCTTCCCCAGGTGCCGGGACTCACACCTGCTAGG
GGCTGGGCAAGGCCCCGGCTCTGCTTTCTCTGAAGGGCTTGTCCAAGTTCATTGCC
CTGTTACAGGTGGTCAAGACGTCCGGCCGCCTTGACCCAGGCTACCCTTAGCCAAT
ATCCTCTGCCCCTGGGTGGTTGGTGGCTGGGCCTCAGGGTGGGCAACGTTAGGGGT
TTGGCGAAAGCCCGCCCCATGGGATTGAGGGACGGGGCTGCACTCCAACCGTCTGC
ACCTGCTCTTCCCCCACCCCTGTGGGACCTCATCTTCACGTGCCATGTGTGCTGAA

GGCCCAGGGCCCAGCAGGGGGCAGTGGCACCTGTTGACGGAAAAGCCGAGGTGCTT
ACCAATGGACCTTCTGGCCCGCCCTCCCCTGTACTTGTCGGGCATTCAGGGCCCCG
ACCTGTGCCTACCCGCA

Sequence ID 633

CAACTGTGTTCACTAGCAACCTCAAACAGACACCATGGTGCACCTGACTCCTGAGG
AGAAGTCTGCCGTTACTGCCCTGTGGGGCAAGGTGAACGTGGATGAAGTTGGTGGT
GAGGCCCTGGGCAGGCTGCTGGTGGTCTACCCTTGGACCCAGAGGTTCTTTGAGTC
CTTTGGGGATCTGTCCACTCCTGATGCTGTTATGGGCAACCCTAAGGTGAAGGCTC
ATGGCAAGAAAGTGCTCGGTGCCTTTAGTGATGGCCTGGCTCACCTGGACAACCTC
AAGGGCACCTTTGCCACACTGAGTGAGCTGCACTGTGACAAGCTGCACGTGGATCC
TGAGAACTTCAGGCTCCTGGGCAACGTGCTGGTCTGTGTGCTGGCCCATCACTTTG
GCAAAGAATTCACCCCACCAGTGCAGGCTGCCTATCANAAAGTGGTGGCTGGTGTG
GGCTAATGCCTGGCCCCACAAGTATCACTAAGCTCGCTTTCTTGCTGTCCAATTTC
TATTAAAGGTTCCTTTGTTCCCTAAGTCCAACTACTAAACTGGGGGATATTATGAA
GGGCCTTG

Sequence ID - 634          nt:      511

TTTTTTAATTTCACCAAAATTTGTTGACGTCCCTTGATTTGCTGATAGGGACAATA
ATTAAATATTTTCCACTTGTTTTTATAAAAACTGTAATGGTGATTTGTTTAACAGA
TGTTGACTTAGCACCTTCTCTCTTTTTTTTTTTTTTTTTTTGAGTTGGAGTCTTGC
TCTGTCACCCAGCTGGAGTGCAGTGGCACGATTTCGGCTCACTGCAACCTCCGCCT
CCCAGGTTCGGGCGCTTCTCCTGCCTCAGCCTCCCANATAGTTGGGATTACAGGTG
CATGCCGCCACNCCTAGCTAATGTTTTTTGTATCTTGGTANANATGGNGTTTCACC
TTGTTGCCCATGCCGCTCTTGAACTCCTTGGCCTCCCAAAGTGTTAGGATTACAGG
CGTGAGCCACTGTGCCTGGCCCCAATTTANCACCTTACTGGGTGCTGAGGCTGTGA
GCCATAGTAGAATGCATGTGATCCAGGGCCTTGCTGAATTCATGGGCTAATAGGGA
GCCTGAC

Sequence ID - 635          nt:      592

TGAGCGTTGGGCTGTAGGTCGCTGTGCTGTGTGATCCCCCAGAGCCATGCCCGAGA
TAGTGGATACCTGTTCGTTGGCCTCTCCGGCTTCCGTCTGCCGGACCAAGCACCTG
CACCTGCGCTGCAGCGTCGACTTTACTCGCCGGACGCTGACCGGGACTGCTGCTCT
CACGGTCCAGTCTCAGGAGGACAATCTGCGCAGCCTGGTTTTGGATACAAAGGACC
TTACAATAGAAAAAGTAGTGATCAATGGACAAGAAGTCAAATATGCTCTTGGAGAA
AGACAAAGTTACAAGGGATCGCCAATGGAAATCTCTCTTCCTATCGCTTTGAGCAA
AAATCAAGAAATTGTTATAGAAATTTCTTTTGAGACCTCTCCAAAATCTTCTGCTC

TCCAGTGGCTCACTCCTGAACAGACTTCTGGGAAGGAACACCCATATCTCTTTAGT
CAGTGCCAGGCCATCCACTGCAGAGCAATCCTTCCTTGTCAGGACACTCCTTCTGN
GAAATTAACCTATACTGCAGAGGTGTCTGTCCCTAAAGAACTGGTGGCACTTATGA
GTGCTATTCGTGATGGAGAAACACCTGACCCA

Sequence ID - 636                              nt:      572

CTTANAAGAGTTGCTCATTCACACCCACGCCCTTGCCCAAGGCTGGCCCACTCAGA
GCGAAACTTAACTTTTGTCTGGATGGGAAGAGAAGTAAGTCTACCCCGAGGTTGCC
ATGTTGAAGAGTGAGAGGTCCAAGTGATTCTGTGCATTGAAACCAAGACACCCCAC
CCAGAACACTTCTTCCCTCCCTCAGCCCAAACCAAAGGCTGGGGTTCTCATCTCCA
AGTGGCTGTTCTCCAACTTTCCCAAGCCGCTTGCATTCCCCAGACTGGACTACTGT
GGCGGTTAGGTTAGATTTGAAGACGGGGCCCAGGCTGGGTATGAACGGGTGCAGCC
CTCTTCTCCTCTTCCCCCCCACATCTCTCATGAGAGAGGTAGTGGCATTTCCTTCT
CAGGGAGCTTCAATGGGAAAGGTCTCGAAAGCTTCAGGAGGAGCAGAATACCAACG
CAGGGGGATGGCTGTAACGATCTCACCGTCTCCTAACCTCAGTCCCTTTTTTGAGA
GTGAATGGTGGAGGGTGGGAAAGGGACCCAAATTTGTAGATCTCTTTGTCTGGGGG
AGGGGAANGATG


Sequence ID - 637                              nt:      482

TTAAAACAGGCGCAGGGGTAAAAATGAGAATGAATCTGAAAAAAGAGAGTTGGTGT
TTAAAGAGGATGGACAAGAGTATGCTCAGGTAATCAAAATGTTGGGAAATGGACGA
TTGGAAGCATTGTGTTTTGATGGTGTAAAGAGGTTATGCCATATCAGAGGGAAATT
GAGAAAAAAGGTTTGGATAAATACATCAGACATTATATTGGTTGGTCTACGGGACT
ATCAGGATAACAAAGCTGATGTAATTTTAAAGTACAATGCAGATGAAGCTAGAAGC
CTGAAGGCATATGGCGAGCTTCCAGAACATGCTAAAATCAATGAAACAGACACATT
TGGTCCTGGAGATGATGATGAAATCCAGTTTGACGATATTGGAGATGATGATGAAG
ACATTGATGATATCTAAATTGAACCAAGTGTTTTTACATGACAAGTTCTCTGAGGA
TGGTTCTACAGTTGGGATTTTGGCCATCATCAAC


Sequence ID - 638                              nt:      545

TTTGAAGGCAAAGAGGGATTAATCTGTGCTGGCATCATGTAAGGAGACTTGATAGA
TAAGAAAAAGCTTTACCTAAGTTTTGAAGAATAGGTTTTTCATAATGGAAAATTTA
AGGGAAAAATCTCCAAAAAAGTGCTACTCAAGTTTTATCCATTTGTATTTCCAACA
CAGCCTAGGACAGTACCTGCACATAGTAGGTGATTAATAAAAATTTAGAAAGCATT
AATACTAAAGAGGAAAAATAGCAATGGCAAGAAAACACATGTAGGGAACACATGTA
GCCAAAAAATAATATATAATCAGAGAAATAATAGGACTTCTGGAAAAAAAAGATGA
GATCAGATTGGTTAGGATCTTTACTAACATGACAAGAGCATGAATTTTTTTTTCTGT

AGATAATAAGTATGAAAGAATTTTAGCTTAAAAATTAGCATAATTTGGATCCACAT
ATGCAAATCAATGAATGTAATTCATAATATAAACAGAACTAAACACAAAAACCACG
TGATTATCTCAATAGACACAGAAAAGGCCTTCAAAAAAATT


Sequence ID - 639                          nt:     624
GACACACGAGCATATTTCACCTCCGCTACCATAATCATCGCTATCCCCACCGGCGT
CAAAGTATTTAGCTGACTCGCCACACTCCACGGAAGCAATATGAAATGATCTGCTG
CAGTGCTCTGAGCCCTAGGATTCATCTTTCTTTTCACCGTAGGTGGCCTGACTGGC
ATTGTATTAGCAAACTCATCACTAGACATCGTACTACACGACACGTACTACGTTGT
AGCCCACTTCCACTATGTCCTATCAATAGGAGCTGTATTTGCCATCATAGGAGGCT
TCATTCACTGATTTCCCCTATTCTCAGGCTACACCCTAGACCAAACCTACGCCAAA
ATCCATTTCACTATCATATTCATCGGCGTAAATCTAACTTTCTTCCCACAACACTT
TCTCGGCCTATCCGGAATGCCCCGACGTTACTCGGACTACCCCGATGCATACACCA
CATGAAACATCCTATCATCTGTAGGCTCATTCATTTCTCTAACAGCAGTAATATTA
ATAATTTTCATGATTTGAGAAGCCTTCGCTTCGAAGCGAAAAGTCCTAATAGTAGA
AGAACCCTCCATAAACCTGGAGTGACTATATGGATGCCCCCCACCCTACCACACAT
TCGAAGAA


Sequence ID 641
CAAGATGACAAAGAAAAGAAGGAACAATGGTCGTGCCAAAAAGGGCCGCGGCCACG
TGCAGCCTATTCGCTGCACTAACTGTGCCCGATGCGTGCCCAAGGACAAGGCCATT
AAGAAATTCGTCATTCGAAACATAGTGGAGGCCGCAGCAGTCAGGGACATTTCTGA
AGCGAGCGTCTTCGATGCCTATGTGCTTCCCAAGCTGTATGTGAAGCTACATTACT
GTGTGAGTTGTGCAATTCACAGCAAAGTAGTCAGGAATCGATCTCGTGAAGCCCGC
AAGGACCGAACACCCCCACCCCGATTTAGACCTGCGGGTGCTGCCCCACGTCCCCC
ACCAAAGCCCATGTAAGGAGCTGAGTTCTTAAAGACTGAAGACAGGCTATTCTCTG
GAGAAAAATAAAATGGAAATTGTACTTAA

Sequence ID 642

TGCTTGGCCCTCTACCTCCTGCCCTCTTCCTGTTCATCTCCCAACCACTGCACTCT
TGATTTTTATACCACACAGAAGGTAAGAAAATTCTAGGAACCCTAAGGATCAATCC
TCTCCATTTTCACTCAAATGCCTGGGGCCCAGCTCTGCAATGACTGACTCCAGGGC
CTCTTTCCTCACTGCCAGCATAGAAGTCAGGGGAGCCAGCTGGGCCCTGCGGTCAG
GAAGGTTCTCATTTTTGGAGCATTCCCTGAGCCCAGATCATAGGAGCAGCTGTCCC
TGGTGGGACACAGGAGTCATGACTCCTACCCTCCACCCTCCACACCCACCAGGCAT
TTAGCAGTCTGTCCTATGCAAGACAGATGAATTCTCAGCCAGGATACCTCAAGGCA
GGCAAAGGTGAGTGGAGGGAAAATTCACAAACATTCAGGGTGTGTGGTGCTGGCAT


CACCATGGCCAAATCCAAGAGGTCTTCCTGGAAGAGGGCCCAAACTGGAACCAAAA
GAATGCTGTCAGCAGTTGGAATAGAGCTGTGAATT


Sequence ID 643

CTTTCCAAGAGGAATCCTCGGCAGATAAACTGGACTGTCCTCTACAGAAGGAAGCA
CAAAAAGGGACAGTCGGAAGAAATTCAAAAGAAAAGAACCCGCCGAGCAGTCAAAT
TCCAGAGGGCCATTACTGGTGCATCTCTTGCTGATATAATGGCCAAGAGGAATCAG
AAACCTGAAGTTAGAAAGGCTCAACGAGAACAAGCTATCAGGGCTGCTAAGGAAGC
AAAAAAGGCTAAGCAAGCATCTAAAAAGACTGCAATGGCTGCTGCTAAGGCACCTA
CAAAGGCAGCACCTAAGCAAAAGATTGTGAAGCCTGTGAAAGTTTCAGCTCCCCGA
GTTGGTGGAAAACGCTAAACTGGCAGATTAGATTTTTAAATAAAGATTGGATTATA
ACTCT

Sequence ID 644

CTTTGATAGAGAAGAAAATTCTCCTAGGATACAAGAGCCTCAACATTTTAAAGATT
TTCTGCATCTCAAAAGCGTAGGCTCCTTGCTGGGCAAGGTGAGCCTCTGTGAGTCC
TCATAGGACCGAGCAAATCTGATTCACCCCAGAAAATCCAATATCGAAGCTGAGCT
TTGGCCTGAGCGGGTTCCATTTCCTCCCCAGATCCTATTTAGGAAGTGTCTCCTGA
CAACCTCCAAAAGGTGCTAACATGCAACGTTCTGAAGGGTTATTGCTCAAAAACAA
GATTTTCCTTGTGGTCAAGACTCTGCGAGCCTCGAACACGATGAATCCGCTCGAAT
GGGCTTGGGCTTTGCCCGGGTGGCGCACGCTCACACGCTGGAAGCACAGCTTTGAC
GATCTCCACACACGCACAGGCACACACGCCACAGATGATGCCGGCTCATTCTCAGG
GGGTGTCTAAGTTCTGCTTTAAATATTTACCCCCTAATTGTACAAACAATAGGGGC
ATGAGCCTGGTACTCGATAAATGGGGACTTNCTTAAAA


Sequence ID - 645                          nt:      649
CTACAGCCTGGGCAGCGCGCTGCGCCCCAGCACCAGCCGCAGCCTCTACGCCTCGT
CCCCGGGCGGCGTGTATGCCACGCGCTCCTCTGCCGTGCGCCTGCGGAGCAGCGTG
CCCGGGGTGCGGCTCCTGCAGGACTCGGTGGACTTCTCGCTGGCCGACGCCATCAA
CACCGAGTTCAAGAACACCCGCACCAACGAGAAGGTGGAGCTGCAGGAGCTGAATG
ACCGCTTCGCCAACTACATCGACAAGGTGCGCTTCCTGGAGCAGCAGAATAAGATC
CTGCTGGCCGAGCTCGAGCAGCTCAAGGGCCAAGGCAAGTCGCGCCTGGGGGACCT
CTACGAGGAGGAGATGCGGGAGCTGCGCCGGCAGGTGGACCAGCTAACCAACGACA
AAGCCCGCGTCGAGGTGGAGCGCGACAACCTGGCCGAGGACATCATGCGCCTCCGG
GAGAAATTGCAGGAGGAGATGCTTCAGAGAGAGGAAGCCGAAAACACCCTGCAATC
TTTCAGACAGGAAATCCAGGAGCTGCAGGCTCAGATTCAGGAACAGCATGTCCAAA


TCGATGTGGATGTTTCCAAGCCTGACCTCACGGCTGCCTTGCGTGACGTACGTANC
AATATGAAAGTGTGGCTGCCAAAAACCTTGCAG

Sequence ID - 646                         nt:       600

```
GAGATGTCTCGCTCCGTGGCCTTAGCTGTGCTCGCGCTACTCTCTCTTTCTGGCCT
GGAGGCTATCCAGCGTACTCCAAAGATTCAGGTTTACTCACGTCATCCAGCAGAGA
ATGGAAAGTCAAATTTCCTGAATTGCTATGTGTCTGGGTTTCATCCATCCGACATT
GAAGTTGACTTACTGAAGAATGGAGAGAGAATTGAAAAAGTGGAGCATTCAGACTT
GTCTTTCAGCAAGGACTGGTCTTTCTATCTCTTGTACTACACTGAATTCACCCCCA
CTGAAAAAGATGAGTATGCCTGCCGTGTGAACCATGTGACTTTGTCACAGCCCAAG
ATAGTTAAGTGGGATCGAGACATGTAAGCAGCATCATGGAGGTTTGAAGATGCCGC
ATTTGGATTGGATGAATTCCAAATTCTGCTTGCTTGCTTTTTAATATTGATATGCT
TATACACTTACACTTTATGCACAAATGTAGGGTTATAATAATGTTAACATGGACA
TGATCTTCTTTATAATTCTACTTTGAGTGCTGTCTCCATGTTTGATGTATCTGAGC
AGGGTGCTCCACAGGTAGCTCTAGGAGGGCTGGCAACTTA
```

Sequence ID 647

```
CGAATGTGCAGGTTTGTTACATAGGTATATATATGCCATGATGGAAATATTTATTT
TTTTAAGCGTAATTTTGCCAAATAATAAAAACAGAAGGAAATTGAGATTAGAGGGA
GGTGTTTAAAGAGAGGTTATAGAGTAGAAGATTTGATGCTGGAGAGGTTAAGGTGC
AATAAGAATTTAGGGAGAAATGTTGTTCATTATTGGAGGGTAAATGATGTGGTGCC
TGAGGTCTGTACGTTACCTCTTAACAATTTCTGTCCTTCAGATGGAAACTCTTTAA
CTTCTCGTAAAAGTCATATACCTATATAATAAAGCTACTGATTTCCAAAAA
```

Sequence ID 648

```
AAAAAAAAAAAAAAAAAAAAAAAAAAAAAAAAAAAAAAAAAAAA
```

Sequence ID - 649                         nt:       425

```
CAAAAAAACGAAGAAAAGTGACGACAGTCTGAGGGACTTATGGGAGATCATCAAGT
GAACCACTATATGTGTAATGTAAGTCTTGGAATGAGAAGAGAGAAGGAGAAGGAGG
AGAGAGCTTATTTGTAGAAATAATGGCTGAAAACATCCCAAACTTTCCTTTTTTTG
AGGAAAGAAATAGGCATACAAGTTCAAGAAACTCAAGGAACTCCAGAGAGGACAAT
TCTAAAGACACCCCCTCTAACATACATTATAATCAAATTGTCAAAAGTAAAATACA
AAGAGAATCTTTTAAATTGACAAGAGAAAAGCAGCTGGTCACGTTCAAGGGAGTTC
TATAAGAATTTCAGCAGATTTCTCAGCAGAAACCTTGCAGGCCAACAGGCAGTGGG
ATGATACATTCAAAGTGCAAAAAAAAAAAAAAA
```

Sequence ID 650

CGAGAGTTTACCAGTNGCCTAATAATGCAATAAAAAATGCTTTGAGATAGCTAACN

GCCCATAAAACAAACTCAAATTGCTTATAAAGTTTCTTCCCATGTTCCCATTTGAT

GAAAAGTCTTACATCACATATAACTGGGAAGCAGGGGTCCCTCCTCAATTTTCAGA

CATTTTGAAAGGATGACAGTTCTGTTTGTTAGATGAGTAAACCTCTATATTCATAA

GTTCTAAAATCCTTCATTATGAGGGATTCAAAGTATTTATAAAAACACTGCCCTCT

AAAAATTTCCTCAGATCTGAAGTATGGNCTTGGNCCTGAATATACAGTGTTATCCT

ATGTTTAAAAGGGTGATCCAGACATGAGACGCAACTAGTTGGTGCATAAGAAGGCC

CCACTTGGCTATTTCATATCTACCTACAATTGACCAAAAAAAATTTTTTAGGCCAG

CAATTATTATTTAGCTTCGCTCTTTCTAGTGCAAGAAACTGCAGGCTGGATCAGTA

GTTCAACAGCTAAACAGTCATAAAATAGTCATTGGCATGTTAAATTTCTTTCAATG

CTTCAAAGATAAATTCCAATTCTATTTACTTATTCATTGNGACNGNATTACTAAAC

AGGTAAGGATGGGAATA

Sequence ID - 651                          nt:      251

CTTTGGGAGGCCGAGGCGGGCGGATCACTTGAGGTCAGGGGTTCGAGACCAGTCTG

GCCAACATGGTGAAACCCCAACTCTACTAAAAATACAAAAGTTAGCCAAGTGTGGT

GGCAAGTGCCTGTAATCCCAGCTACTCGGGAGGCTGAGACAGGAGAATCACTTTGA

ACCTGGGAGGCGGAGGTTGCAGTGAGCCAAGATCGTGCCACTGCACTTCAGCCTGG

GCAACAGAGCAAGATTCCGTCCATCTC

Sequence ID 652

CTTTCTTCAGCCTTGCAGACACCTAAACATCATGTAATTACCTAAGGAATTCCCAA

GTGCCTCTTCCAGGTTATACGTGTAAATAGCTGTTTTTATGCAAGATTAGTTAGAT

ACTGCTCTTTACAGGATGAGTGGTGTTGTCTTTGGCTGGGGGGGNCTTAAATGTGT

TTCTAATGTGTGTGTCAAATAATTACCTGTTAAACAGACTGCCAATCTGGCTGAAG

CCAATGCTTCTGAAGAAGATAAAATTAAAGCAATGATGTCGCAATCTGGCCATGAA

TACGACCCAATCAATTACATGAAGAAACCTCTAGGTCCACCACCTCCATCTTACAC

GTGTTTCCGTTGTGGTAAACCTGGACATTATATTAAGAATTGCCCAACAAATGGGG

ATAAAAACTTTGAATCTGGTCCTAGGATTAAAAAGAGCACTGGAATTCCCAGAAGT

TTCATGATGGAAGTGAAAGATCCTAATATGAAAGGTGCAATGCTTACCAACACTGG

AAAATATGCAATCCAACTATAGATGCAGAAGCATATGCAATTGGGAAGAAAGAGAA

ACCTCCTTNTTACCAGAGAGCCATCTTNTTTCT

Sequence ID 653
GTTGTGACTCGTTGGCATGTGATCTGAAGTTCCTGCCCTGCAGCTGACGAGCCAGT
GTTTCAATAATTAAAAACAACTCAACTCACTGTCCTCCTGCCTTGAATTTGATCAT

TGCGCTTTGCATGTATGTATCACAATACCACATGTACCCCATAAATATGTACAAAG
ATTATGTGTCAATAAAAAACAAAAATTAAAATCCCAATTTTTA

Sequence ID 654
GTTGCTAGTAGCGGCAGGAAGATGTCAGGCTCACTTTCCTCTGATTCCCGAAATGG
GGGGAACCTCTAACCATAAAGGAATGGTAGAACAGTCCATTCCTCGGATCAGAGAA
AAATGCAGACATGGTGTCACCTGGATTTTTTTCTGCCCATGAATGTTGCCAGTCAG
TACCTGTCCTCCTTGTTTCTCTATTTTTGGTTATGAATGTTGGGGTTACCACCTGC
ATTTAGGGGAAAATTGTGTTCTG

Sequence ID 655
GTCCCCGGGAATCGCGGCCGCGTCGACGGTTTATTTTCAGTGCTTGAAGATACATT
CACAAATACTTGGTTTGGGAAGACACCGTTTAATTTTAAGTTAACTTGCATGTTGT
AAATGCGTTTTATGTTTAAATAAAGAGGAAAATTTTTTGAAAAAAAAAAAAAAAAAA
AAAAAAAAAAAAAAAAAAAAAAAAAAAAAAAAAAAAAAAAAAAAAAAAAAAAAAAAA
AAAAAAAAAAAAAATTTTT

Sequence ID 656
TAGAGGCCTGAATAGGTAGACAATGGCAGCAGCGTTTTTAATCACAGTCCTATTCA
TGCCCTAATTCGGGAGTGATGATTAAAGGACATTAGAGGGAGCACTTTGACATCTG
ATCCTTTGAACTGACGTCTGTGCAGGCTGCACTCCATAGAGCTCACTTGGCCAAAC
TGATTTCCTTAAATAAAGTGCTGTGATTTCCAATGTAGGAAATATTACATTAGAGC
CTATTGAAATGATTAGGAATTGAGGAGCTTTTCTTTAGGTGGGAATGTGGTGTATG
CTGTATACTCACAAAGTGAGATCATTAATATTGCATGTACTACTTTGAATATCAG
GGACCACAGAGAAATAGCATGAGAAACGCCTTCCTGCAGTCATGCACTTAAAATGA
ATATGAACAAAAATGTGGAACTCTGCTGTCATAGCTCTCCG

Sequence ID 657

GGGGCTTCTGCTGAGGGGGCAGGCGGAGCTTGAGGAAACCGCAGATAAGTTTTTTT
CTCTTTGAAAGATAGAGATTAATACAACTCTTAAAAAATATAGTCAATAGGTTACT
AAGATATTGCTTAGCGTTAAGTTTTTAACGTAATTTTAATAGCTTAAGATTTTAAG
AGAAAATATGAAGACTTAGAAGAGTAGCATGAGGAAGGAAAAGATAAAAGGTTTCT
AAAACATGACGGAGGTTGAGATGAAGCTTCTTCATGGAGTAAAAAATGTATTTAAA
AGAAAATTGAGAGAAAGGACTACAGAGCCCCGAATTAATACCAATAGAAGGGCAAT
GCTTTTAGATTAAAATGAAGGTGACTTAAACAGCTTAAAGTTTA


Sequence ID 658


GACCTTTGAGAAAATTAATTTAAATCCTAGAACTTTGGGTGAACCGAAGAAATTTA
TAATATTTGTTTAGTTAATAACAGATAAAAAGGAAAGATTCAAGCCTATTGGATGA
GAATTTGTACATTATTTTAGAGCTAATAATAATGGTTTTCAGTTTAGTGAGGATTT
AAAAAATGTTTTTGAATCAAACTTTTTTTCTTTATAATCCTTTTTAACTAACTCAG
GAAATAAGGTATTATGAAATCCACACACTGTTACCTCCTTAAAGTATGAGGATACT
TCCCACTGTTTGGTCCACTAGTGGCTGATTATTTTGTTTGTGGATTATTTGTAATT
TTCTTTTTAATTCTTCCTTAAAGAGCATGGCATTTGGAGTCACAGACCTATATTTG
AATCCTGTCATTTACTAGCGTTTTGACCTTGAACAATTATGCTCAGAGTCTCAGTT
TTTTCTTGTAAAGTGATGATGATACTACTTAACTCACAGGGTTGTAGTGAAGATCA
AATGAGATCATGTCTGTANAACACCCTGCCCGGCACTCAATAAGTATTAATAGGAA
CCCATATACCTC


Sequence ID 660

TGTTTTTATTTTTTAAAAGGTATAAACACCAAAAAAAAAATTAACATTGTATGAAG
ATGGAAAATAAGAAGATGCACTTTCTGTAACTTTGTCTAAGGATTTAAATTACTAA
CTTATGAACTCCAATTTGAATTGAACTTAACTATCGGCTTTCTTACTGGTAAAATT
ATATGGTTTATTTTAAATGCGTACATATTGACCAATGGCCTCTGAAAAAGCACATT
TTAGATACTGAAATTGAAGGAAAGAAAATGCATCTTCAAACATTTTTTGGAATCTC
ACCACATATACTTTGTTANATTTGTGTATTGTAGGGTGTTTGTTTTGTATTTTTGT
ATTGTATATGAACTTTTTTTAAATGTGACAGTTAAACACATCTTTAAAAGCATAGT
CACAGACAAAAGCATACAGTATAAAAATTTCCTTGAAAACTCCTACAATATTATAT
TTGGAGGCAGCTTCAGACTGTTTTATTGG

Seqeunce ID 661

CTCTGGCACACATTAGTTCCTCTTATATTACATTGATATAAGCAAGTCATATGGAT
TTATCTGAGTGTAAGGAGAGCTGGAAAAAATAGTTTCTAGCAGGTCAGCCACCTCC
CAGTGAGGGCTGCATACCATAGAAGGGGAGAATGAATTTTGGGAAAACAGGTAATT
ATCTCTGTCACAGAAGGGGATGAAAAGTATGGTAGTTACNCAAGTTANACATCTGT
ATGGAAAATACCACTTGGTTCTACAAATGNGG


Sequence ID - 663                    nt:      627
GCCTCCCGGGTTCAGGGATTTCTCCTGCCTCAGCCTCCTGAGTGGCTGCATTGCAG
GCACCTGCCACCACGCCTTGCAAATTTTTGTGTTTTTAGTGGAGATGGGGTTTTGC
CATGTTGGCCAGGCTGGTCTCGGACTCCTGACCTCAGGTGATCCGCCCGCCTCAGC
CTCCCAGAGGGCTGGGATTACAGGCGTGAGCCACTGTGCCTGGCCCCAAGTTTTGC
ATCTTTTAATGCCCTCTGAACAAATACATAGAGAAAACTCTCAGAACAATTAAAAC
CTGCAGAGCAACAGTGTCCTCCATGTCTTAGGTTTCAAGTTTGCCTCTAAAATTCT


AATCCATATTTTTCTACTTCTCAGATAATTTATGTGTGTGTACTCTTCCTAGACGT
ACAAGAGACTTTTTAATGCTAAATATTTGTCAGTGCTTAACAAAAACTCAATTTCA
CATTACTCATATTGTTTTTGTTTTAATTGAATGTGAATTAAATTTTTATTAGTTAT
TTGATTTGGAATGTTATGTATGCCATTAACACTATTAGGGGAATCTCTAGCATTTC
TGTATTTTTAAAGAATTTGATTCTTTTGTANATTCTGCCTGTGTGGCATTTTAAAC
ATGTGTGACAT


Sequence ID - 665                    nt:      345
ACCGGCGACATGGCCAAACGTACCAAGAAAGTCGGGATCGTCGGTAAATACGGGAC
CCGCTATGGGGCCTCCCTCCGGAAAATGGTGAAGAAAATTGAAATCAGCCAGCACG
CCAAGTACACTTGCTCTTTCTGTGGCAAAACCAAGATGAAGAGACGAGCTGTGGGG
ATCTGGCACTGTGGTTCCTGCATGAAGACAGTGGCTGGCGGTGCCTGGACGTACAA
TACCACTTCCGCTGTCACGGTAAAGTCCGCCATCAGAAGACTGAAGGAGTTGAAAG
ACCAGTAGACGCTCCTCTACTCTTTGAGACATCACTGGCCTATAATAAATGGGTTA
ATTTATGTA

Sequence ID - 666                     nt:      252

```
ATAATTCAGAACTTCTTCATATGCTCGAGTCTCCAGAGTCACTCCGTTCTAAGGTT
GATGAAGCTGTAGCTGTACTACAAGCCCACCAAGCTAAAGAGGCTGCCCAGAAAGC
AGTTAACAGTGCCACCGGTGTTCCAACTGTTTAAAATTGATCAGGGACCATGAAAA
GAAACTTGTGCTTCACCGAAGAAAAATATCTAAACATCGAAAAACTTAAATATTAT
GGAAAAAAAACATTGCAAAATATAAAAT
```

Sequence ID 669

```
TTACTTTTAACCAGNGAAATTGACCTGCCCGTGAANAGGCGGGCNTGACACAGCAA
GACGAGAAGACCCTATGGAGCTTTAATTTATTAATGCAAACGGTACCTAACAAACC
CACAGGTCCTAAACTACCAAACCTGCATTAAAAATTTCGGTTGGGGCGACCTCGGA
GCAGAACCCAACCTCCGAGCAGTACATGCTAAGACTTCACCAGTCAAAGCGAACTA
CTATACTCAATTGATCCAATAACTTGACCAACGGAACAAGTTACCCTAGGGATAAC
AGCGCAATCCTATT
```

Sequence ID 670

```
GGCTGATTCCTGAGCTATAAAAGCATAATTGCTTTATATTTTGGATCATTTTTTAC
TGGGGGCGGACTTGGGGGGGGGTTGCATACAAAGATAACATATATATCCAACTTTCT
GAAATGAAATGTTTTTAGATTACTTTTTCAACTGTAAATAATGTACATTTAATGTC
ACAAGAAAAAAATGTCTTCTGCAAATTTTCTAGTATAACAGAAATTTTTGTAGATG
AAAAAAATCATTATGTTTAGAGGTCTAATGCTATGTTTTCATATTACAGAGTGAAT
```

```
TTGTATTTAAACAAAAATTTAAATTTTGGAATCCTCTAAACATTTTTGTATCTTTA
ATTGGTTTATTATTAAATAAATCATATAAAAATT
```

Sequence ID 671

```
CAGGAAGTCACCTGGGATTGGCTGCCTCACCCACTCACAGTGCCATCCCTGCCCCA
GGCCTCCCAGTGGCAATTCCAAACCTGGGTCCCTCCCTGAGCTCTCTGCCTTCTGC
TCTGTCTTTAATGCTACCAATGGGTATTGGGGATCGAGGGGTGATGTGTGGGTTAC
CTGAAAGAAACTACACCCTACCTCCACCACCTTACCCTCACCTGGAGAGCAGTTAT
TTCAGAACCATTCTACCTGGCATTTTATCTTATTTAGCTGACAGACCACCTCCACA
GTACATCCACCCTAACTCTATAAATGTTGATGGTAATACAGCATTATCTATCACCA
ATAACCCTTCAGCACTA
```

Sequence ID 672

CAGGAAGTCACCTGGGATTGGCTGCCTCACCCACTCACAGTGCCATCCCTGCCCCA
GGCCTCCCAGTGGCAATTCCAAACCTGGGTCCCTCCCTGAGCTCTCTGCCTTCTGC
TCTGTCTTTAATGCTACCAATGGGTATTGGGGATCGAGGGGTGATGTGTGGGTTAC
CTGAAAGAAACTACACCCTACCTCCACCACCTTACCCTCACCTGGAGAGCAGTTAT
TTCANAACCATTCTACCTGGCATTTTATCTTATTTAGCTGACAGACCACCTCCACA
GTACATCCACCCTAACTCTATAAATGTTGATGGTAATACAGCATTATCTATCACCA
ATAACCCTTCAGCACTAGATCCCTATCAGTCCAATGGAAATGTTGGATTANAACCA
GGCATTGTTTCAATANACTCTCGCTCTGTGAACACACATGG

Sequence ID 673

GGGTTTTCTTTCGGAAGCGCGCCTTGTGTTGGTACCCGGGAATTCGCGGCCGCGTC
GACTGCTAAACAGAATACTGCTATTTTGAGAGAGTCAAGACTCTTTCTTAAGGGCC
AAGAAAGCCACNTGNNCCCTNGGNCTAATCTGGCTGAGTAGTCAGTTATAAAAGCC
NTAATNGCTTNNTNTTTGGNNTCNTTTTTTNNCNGGGGNCGGNCTTGGGGGGGGGTTG
CNTCCAAAGATANCATNTNTTTCCAACTTTNTNAANNNAANNGTTTTAAAATCCCT
TTTCCNCCNGAAAANANNGCCCTTTAAGNGCCNCAAAAAAAAANNGTNTTCTGCAN
NTTTTCTANTATNACAAANNTTTTNGTAGAANAAAAATTTTTTTTTAGNGGCTACC
CTTTNTTTNTTANNCANNGGAGTTTNTTTTTACAAAAAAAAAANATTGGGNCCCCT
CCACAACCTTGGGTCTNTAATNGGGGGGGTTTTTAAATAAANCNTNTNTAAATCCCC
CNNNNNNNNNCNNNNNNNNNCCNNNNNNNNNNNNNNNCCCNNNNAAAAAATTTTTNC
TCCCCCNCCCTTTTTCTTCCTGCCGGCCCCAATTTAAGCCCNGGCGCTTGGGGCAA
ATCCCCCTTTAGNGGGGGGGTTTANAAAAACCNGGGGCGGGGNTTTAAAACCNCGG
GGNNNGGGGAA

Sequence ID 674

ACCTCTAGCATCACCAGTATTAGAGGCACCGCCTGCCCAGTGACACATG-TTTAAC
GGCCGCGGTACCCTAACCGTGCAAAGGTAGCATAATCACTTGTTCCTTAATTAGGG
ACCTGTNTGAATGGCTCCACNAGGGTTCACTTGTCTCTTACTTTTAACCAGTGAAA
TTGACCTGCC

Sequence ID - 675                            nt:        591

GTATAGAAAATAATGTCCCCAGNGCATAGAAAAAATGAGTCTCTGGGCCAGTGAAT
ACAAAACATCATGTCGAGAATCATTGGAAGATATACAGAGTTCGTATTTCAGCTTT
GTTTATCCTTCCTGTTAAGAGCCTCTGAGTTTTTAGTTTTAAAAGGATGAAAAGCT
TATGCAACATGCTCAGCAGGAGCTTCATCAACGATATATGTCAGATCTAAAGGTAT
ATTTTCATTCTGTAATTATGTTACATAAAAGCAATGTAAATCAGAATAAATATGTT
AGACCAGAATAAAATTAATTATATTCTGGTCTTCAAAGGACACACAGAACAGATAT
CAGCAGAATCACTTAATACTTCATAGAACAAAAATCACTCAAAACCTGTTTATAAC
CAAAGAATTCATGAAAAGAAAGCCTTTGCCATTTGTCTTAGAAAGTTATTTTTTA
AAAAAAAATCATACTTACTATTAGTATCTATGGAAGTATATGTAACAATTTTTATG
TAAAGGTCATCTTTCTGTGATAGTGAAAAAATATGTCTTTACTAAGTTGAAATGAA
TACTTTCTGNCTTTGCTAATGGATAGTTATT


Sequence ID 676

CTCAATTCTACTAAAAAGCCCCCCAAGAAAAGCGAATGAGAAAACAGAGTCATCCT
CTGCACAGCAAGTAGCAGTGTCACGCCTTAGCGCTTCCAGCTCCAGCTCAGATTCC
AGCTCCTCCTCTTCCTCGTCGTCGTCTTCAGACACCAGTGATTCAGACTCAGGCTA
AGGGGTCAGGCCAGATGGGGCAGGAAGGCTNCGCAGGACCGGACCCCTAGACCACC
CTGCCCCACCTGCCCCTTCCCCCTTTGCTGTGACACTTCTTCATCTCACCCCCCCC
TGCCCCCCTCTAGGAGAGCTGGCTCTGCAGTGGGGGAGGGATGCAGGGA


Sequence ID 679

GNANCNTTTCCTNTCGNAAANCGCGCCTTGTGTTGGTACCCGGGAATTCGCGGCCG
CGTCGACAAAAAAAAAAAAAAAAAAAAAAAAAAAAAAANTNTAGACTCGANCAAGCTT
ATGCANGCNTGCGGCCGCAATTCGAGCTCGGCCGACTTGGCCAATTCGCCCTATAG
NGAGTCGTATTACAATTCACTGGCCGTCGTTTTACAACGTCGNGACTGGGAAAACC
CTGGCGTTACCCAACTTAATCGCCTTGCAGCACATCCCCCTTTCGCCAGCTGGCGT
AATANCGAANAGGCCCGCACCGATCGCCCTTCCCAACAGTTGCGCAGCCTGAATGG
CGAANGGAAATTGTAAGCGTTAATATTTTGTTAAAATTCGCGTTAAATTTTTGTTA
AATCAGCTCATTTTTTAACCAATAGGCCGAAATCGGCAAAATCCCTTATAAATCAA
AAGAATAGACCGAGATAGGGTTGAGNGTTGTTCCAGTTTGGAACAANAGTCCACTN

TTAAAGAACGNGGACTCCAACGTCAAAGGGCGAAAAACCGTCTATCAGGGCGATGG
CCCACTACGTGAACCATCNCCCTAATCAAGTTTTTTGGGGTCGAGGNGCCGTAAAG
CACTAAATCGGAACCCTAAAGGGAGCCCCCGATTTAAAGCTTGACGGGGAAAGCCC
GGCGAACGTGGCGAAA


Sequence ID 682
CACCTGCAGTCCAAGTACATCGGCACGGGCCACGCCGACACCACCAAGTGGGAGTG
GCTGGTGAACCAACACCGCGACTCGTACTGCTCCTACATGGGCCACTTCGACCTTC
TCAACTACTTCGCCATTGCGGAGAATGAGAGCAAAGCGCGAGTCCGCTTCAACTTG
ATGGAAAAGATGCTTCAGCCTTGTGGACCGCCAGCCGACAAGCCCGAGGAAAACTG
AAACTTTGCTTAACNACCGAATGGNGGGGANCTTTTCCAACGNTTTT


Sequence ID 683
TTGGTTTCATACTGNTGGGGNTTGAATGNTCCCTNCAACACTNATGTTGANACTTA
ATCCCTAATGNGGCAATACTGAAAGGTGGGGCCTTTGAGATGTGATTGGATCGTAA
GGCTGTGCCTTCATTCATGGGTTAATGGATTAATGGGTTATCACAGGAATGGGACT
GGTGGCTTTATAAGAAGAGGAAAAGAGAACTGAGCTTGCATGCCC


Sequence ID - 684                          nt:       545
GTGGAAGNGACATCGTCTTTAAACCCTGCGTGGCAATCCCTGACGCACCGCCGTGA
TGCCCANGGAAGACAGGGCGACCTGGAAGTCCAACTACTTCCTTAAGATCATCCAA
CTATTGGATGATTATCCGAAATGTTTCATTGTGGGAGCAGACAATGTGGGCTCCAA
GCAGATGCAGCAGATCCGCATGTCCCTTCNCGGGAAGGCTGTGGTGCTGATGGGCA
AGAACACCATGATGCGCAAGGCCATCCGAGGGCACCTGGAAAACAACCCAGCTCTG
GAGAAACTGCTGCCTCATATCCGGGGGAATGTGGGCTTTGTGTTCACCAAGGAGGA
CCTCACTGANATCAGGGACATGTTGCTGGCCAATAAGGTGCCAGCTGCTGCCCGTG
CTGGTGCCATTGCCCCATGTGAAGTCACTGTGCCAGCCCAGAACACTGGTCTCGGG
CCCGATAAGACCTCCTTTTTCCAGGCTTTAGGTATCACCACTAAAATCTCCAGGGG
CACCATTGAAATCCTGAGTGATGTGCACTGATCAAGACTGG

Sequence ID 685

GGAAAGGGCCATTTTATTGCCTAAAACCACCTGGNTTTTNAGGTAACAGTTCCAAC
ATGTCCTTTTTTGAATAGCTGTTCTAATTATTATATATTCAGCTGATTAATAGGAG
TACTTGATAGGTGGACTGTGTCAGGTAGCCTCAGGCAATCCTACTTCAACAAGCTG
TCAGGGAGCCATGCCATGCTTCTTTATGACATAGGTGAATTTGATAGGCTCACTAG
CAGAACATGGGATCACAAGGTGGAACCNTTCCNTTT


Sequence ID 686

GACCCCTTCCTTACACCTTATACAAAAAAACTGAAACTGGACCCCTTCCTTACACC
TTATACAAAAATTAACTCAATTTTATTATGTTGTATTAAATTAAGTTGGGTTTAAT
TAAGATGGATTAAAGACTTAATTATAAGACCTAAAACCATAAAAACCCTAGAAGAA
AACCTAGGCCATACCATTCAGGACACGGGTATGGGCAAAGACTTCATAACTAAAAC
ACCAAAAGCAATGGCAACGAAGTCCAAATAGACAAATTGGACCTGATTAAACTAAA
GAGCTTCAGCACAGCAGAAGAGACTATCGTCAGAGTGAACAGGCAACCCACAGAAT
GGAAGAAAATTCTTGCAATCTATCCATCTGACAAGGGGCTAATATCCAAAATCTAC
AAAGAACTTAAACAAATTTACAAGGAAAAACACAAACAACCCCATCAAAAAGTGGG
CTAAGGATGTGAACAGACACTTCTCAAAAGAAAACATTTATGCAGCCAACAAACAT
GAAAAAAGTTCATCATCACTGCTCATTAGAGACATGCAAATCAAAACCACAATGA
GATCCCATCCCACACCAGTTAGAATGGCAATCATTAAAAATGT


Sequence ID - 687                            nt:      268

TTTATGTGTTTTTGCTTGGGGGGCGCTGGGCCTAGCCCAGAGTAGTGCTTGCTCCC
CCTGCCTTGTCCCACCAGGGAGGCAGCAGACTCAGGCCCTCCATGGTCCTCTTTGT
CATTTTGTTGACATGCATTCCTCCTTTTGTCATCTTGTTGGGGGGAGGGGATTAAC
CAAAGGCCACCCTGACTTTGTTTTTGTGGACACACAATAAAAGCCCCGTTTATTTG
TAAAAAAAAAAAAAAAAAAAAAAAAAAAAAAAAAAAAAAAAAAAAA

Sequence ID - 688                              nt:        569

CTTTAGCCAGCCTGATCAGAAAAAAACAAAAGAAGAGGAAAGACGTAGATTACCAA
CATCAAGAATGTGAGTTATGATATCACTACAGACTCTCCAGGTATTAAAAGCATAA
TTAGAGAATGATATGAGCAGCTATATGCAAATAAGTTCAACATTGGACAAATGGAC
AAATTTCTTGAAAGATAAATTATGAAATTTCATTCTGAAAGAACTACATGACCTTA
ATTGTCTTACATCTATTAAATAAGTGGAAATTGTAGTTTAGAAACTTTCCCACAAA
GAAAACTCTAGGCCCAGATGGCATCAAAATAATATTCAGATGAATGAAATGGAGAA
AGGATAGCCTTTTCAACAAATGGTGGTGGAACAATTGGATTTCCATATGCAAAAAA
ATAGAGATGGACGCAGAGGTGTGTGCTTAGGAGGCTGAGGTGAGAGGATTGTTTGA
GGCCAGCCTGGGCAACATAGCAAGACCCCATTTCAAAAACAAAAATAAAGAACTTG
TAGCCTTACCTTGTGCCATATTATGAAAATGTATCATAGGCTTAAATGTGAAACGT
AAAACAAAA


Sequence ID 689

CGCAGGGGCTTCTGCTGAGGGGGCAGGCGGAGCTTGAGGAAACCGCAGATAAGTTT
TTTTCTCTTTGAAAGATAGAGATTAATACAACTACTTAAAAAATATAGTCAATAGG
TTACTAAGATATTGCTTAGCGTTAAGTTTTTAACGTAATTTTAATAGCTTAAGATT


TTAAGAGAAAATATGAAGACTTAGAAGAGTAGCATGAGGAAGGAAAAGATAAAAGG
TTTCTAAAACATGACGGAGGTTGAGATGAAGCTTCTTCATGGAGTAAAAAATGTAT
TTAAAAGAAAATTGAGAGAAAGGACTACAGAGCCCCGAATTAATACTAATAGAAGG
GCAATGCTTTTAGATTAAAATGAAGGTGACTTAAACAGCTTAAAGTTTAGTTTAAA
AGTTGTAGGTGATTAAAATAATTTGAAGGCGATCTTTTAAAAAGAGATTAAACCGA
AGGTGATTAAAAGACCTTGAAATCCATGACGCAGGGAGAATTGC


Sequence ID 690

CGAAAAGCAAATATAACTTGCCACTAACCAAGATCACCTCTGCAAAAAGAAATGAA
AACAACTTTTGGCAGGATTCTGTTTCATCTGACAGAATTCAGAAGCAGGAAAAAAA
GCCTTTTAAAAATACCGAGAACATTAAAAATTCGCATTTGAAGAAATCAGCATTTC
TAACTGAAGTGAGCCAAAAGGAAAATTATGCTGGGGCAAAGTTTAGTGATCCACCT
TCTCCTAGTGTTCTTCCAAAGCCTCCTAGTCACTGGATGGGAAGCACTGTTGAAAA
TTCCAACCAAAACAGGGAGCTGATGGCAGTACACTTAAAAACGCTCCTCAAAGTTC
AAACTTAGATTTCAGATTT

Sequence ID 691

CCGGTCTCTACACAATATATAGAAATCTGGGCATGGTGGTGCCTGGCTGTAGTCTC
AGCTACCTAGTTGGGTGAGGTGGGAGAGTCGCTTGAGTCCTGGAGGTTGAGGCTGT
AGTGAGCCAGGGCTGCACCACTGCATTCCAGCCTGGGTAACAGAGTGAGACCCTGT
CTCAAAAAGAAAAAAAAAAATTGCTAATTTTAACAAATCACAAAACTGACTCAGGC
AAGTTGTCTGACTCAAAAGCCCTTGAAAAACCATCAAAGACAGTAGAATGTTAACT
GGTCATTTACGTAAAATAGTGTTCATTAAATTTTTGGTTCATTTAGGATAATCATT
TTAAATGAGACTGTATTTGAGACTGTATACACATACATATACATGTTTACACACAT
ATACGTACAATATATGTACATTCTATCTAAAGATCATACATGTGTGTACATATAT
GTTTTTAAAAGTCAAACTGACATATTAATGGAAACAGTGCTTACATCTCTGGTAGT
GATTTTCTATTAGCAGCAGCCCTACATATGCTGCGTCTCTGAACAGCATGTCAGTG
CCATGACTGTCTAAACATGCAAATATGACTGACAGACTCTTGAGACAGCTTTCACC
TTG


Sequence ID 692

AATTCGNGGCCGCGTCNNCCTANGAGGCACCAGGAAATCCCGCGGGGTGGCCCATG
CAGACCAGGCGCACGTGGCTCATGGGGCANAATTGCCAAGGACAGCTCACGACAGT
GCCACCTTCTCACCATTCCAGCCAAGGAGAGATGTGACGTTGGAACTGCTCTGGCA
CTTCTGTCAAGCCTCCCCCGCCCCAATTGCCTTGAGATCTCTGCTCTTTGTCAGAG
ATTTGCAAAGACTCACGTTTTTGTTGTTTTCTCATCATTCCATTGTGATACTAAGA
AACTAAGAAGCTTAATGAAAAGAAATAAAATGCCTATGTTGTTGTTCT


Sequence ID 693

CTAGAACCCATGACTCCTAGGTCTTATACTGCAACCACAGTATCAGCAAATAATCT
TTCATAAGGGGATTATTCTCTGATTAACAGGAAATACAGGAATTTAATTTGTGAAC
ACGCTAGGTAGAAGCAGAAACCCAAATCCAAATCCAAATTTAAACATTTAAAATTC
ATTCTATAACTAAGATCTAACAGTCATTTTCTTCCCAGTAAGAAATAACCAAAGCA
TGCTAAAAATCACTGGACTAAATTGGTGTCAAAACTGCCACATTGCCAGGCATGGG
GGGGTCATACTTGTAATCCCAGCACTTTGGGAGGCCGAGGTGGGAAAATTGCTTGA
GGCCAGGAGTTCGAAACCAGCCTGGGCAACACAGTGAGACCCCATCTCCACAAAAA
AAAAAAATTAAAAAACAAAACAAAACATTAGCTGGGCATGGTGGTACACGCCTGTA
GTCCCAGCTACTCAGGAGCCTGAAGTGAGAGGATCACTGAAGCCCAGGAGGTAGAG
CTATGACTGTAGTGAGCTATGACTGTGCCACTACACTCCACCTGGGTGACAGGGGA
CTC

Sequence ID 694

CGACTTCCATTTGTATTAATGGAATACTAAGTCCCTCTGTGATTTCTGAACCAAGC
TATTCCTAGGCCTGAGTTTTATTTTGTTGACACAGAAATAAATTANAAGGCCAAGC
GTGGTGGCATGTGCCTGTAGTCCTAGTTGCTGAGGTAAGAGGATTGCTTGAGCCCA
GGAGTTCAAGGCTGCAGCAAGCTTTGATTGCGCCACTGCACTCCAGCCTTGGCGAC
AGACTAAGACGCTGTCTCAAAAAAAAACAAAAA

Sequence ID 696

GGTTATCAATGAGATTAAGAGACAACTAGAGTAAAAACAAAAGAAAAGAAAAGAAA
NGAAAACAACAGAAGCTCTATTAACTGACCTCTAACCAATACAACAGGTTAACTGA
TGTTCTCCATTCTGTATATAAAAATCCCAGTGGACACCCACAACACAGGCTTCAGG
CTTGTAGGACACTTTCTAGTTCATCTGAGCACTTTTGTTCTCAGCAGTTGAGCTGT
ATACTTAGCAACATTTGGTGCTTCCAAACCCATTTGTGCCTGTAGCACTTACTATT
GAAATACATAATTTAATTAAATATTATATAAAGGAATGGAATACGAGTTGGACAAG
AAAAAGAGTTAAATCTGAAGGTTAGGTAAAAAGAGCAACTTCTTTTCTCTGTTTTG
CAGGTTGGCAAAATCATTTAAAAACAATTGGAAGTATTATATGTTCTGCATTAAGT
TGTCATTTTACTTAAAAACTAGGCATCAAAGATGATGCATAATAAATTTAGTGTAT
GCAAGAATGACTGCTTGGGACCTCAATATATGAATTCTTAATCCAAGGAAAGTCCT
TGGCCTTACATTTAAAAGTCGGCAAATAAGTGTACGTTCATT

Sequence ID 697

GAACATTTAAAAATAATGCAAATAAGGCTGGGCGTGGGGGCTCACACCTGTAATCC
CAGCACTTTGGGAGGCCGAGGCAGGCAGATCACGAGGTCAGGAGATTGAGACCATC
CTGGCTAACACAGTGAAACCCTGTCTCTACTTAAAAAATAAAAAAATTAGCCAGGC

GTGGTGGTGGGCGCCTGTAGTCCCAGCTACTCAGGAGGCTGAGGCAGGAGAATGGT
GTGAACCCGGGAGGCGGAGCTTGCANTGAGCTGAGATCGTGCCACTGCACTCCAGC
CTGAGCGACAGAGCGAGACTCTGTCT

Sequence ID 698
TCATTAGAATCCAAGCTTTGAAAATTTCTGATTAATGCTCATGTATTTCTTTATCT
TTGTTTTTCCTTGTGAAGAAAGACTTTCACCACTGTCTGAGTGATGATGCTGTTGA
TAAGGATGATGTCGATGACTACTATATTGCATCTCTCAGGAACAGCTGATGGGAAG
GGAGGGGCTGCTGAGTTCCCTTGTTCTAGCTAGCAGCACGCTCCTCANAGAGGGGG
CCGAGTTACAGACAGCAGCCGCATTCTCATGCAAAATTAGTTTTAAACTGCTAGTG
TGGGCATCGGTACCTTTTGCCTGGGTGATACCGAAGAATTGTTGAGGATTTAGTAT
GCTCCGTAGAGACAGTTCAGCCAGTCATTTCTGCATTGGAGAGACTTCTCATACTT
TCTTTGAAGACTCATAGAAAGCTGGAT


Sequence ID 699
ATTAAGGTTTGTNCCCAACAAGAATAGATGTAATTAGAAAAAANTGNCTTCCTTAC
CTATTGCCTCTGATNTTTACTTGCTTAAATTTTTTTTATTGNAAATCCAGAAAAAG
NGGATTTAGAGAACAACACTAACTCCCACCTAATCTATGACAGANATGTACAANAN
AGTACCTGTGAAAAATGTGAAAGNATNTGAAAAATGTAACCTTTGGCAGCCTGAGC
ATAGTCAACCAGAAAAACTATCTGAATTAAAATAATTGGTCCATAGGTACTATTTT
ATTTGGTCCATAAGGATTATTTTTTCAACTTTTTTTTCAAGTGTATTATTATGTCA
TTTCCCACGTAGGTTACTGATACCTGAAGACTTTTTNCACCTTTAACCTTNCTCGT
TGAGGAGCTTTGTANTCTAATAAAAGAGAAATATAAGTAAATGTTAGATATATGGG
NGGATAATGGTAACTATGTGCTTAAAGAGGTATAAAAGAAGGGTAGGGAGCAGATA
AGACAAAGGAAGGGCTATATTATAANGAAGAATATTCCAAGTAGGGAAGAGAAAAA
GATATGTTATCCATATAATATTTTATGTGCAGTAGAGAACATGTTCTATAGAANAG
ACAGAAGATG


Sequence ID 700
CTTGAGCCCAGGAATTCCAGCCTGGGCAATATAGTAAGACTCCGTCTCTACAAAAG
ATACAAAAATTAGCCAGATGTGGTGGTGCGTGCCTGTAGTTCCAGATACTGGAAAG
ACTGAGGCAGGAGGATTGCTTGAGCATGGGAAGTTGAGGCTGCAATGAGCTGTGAT
TACGCCACTACACTCCAGCCTGGGCAACAGAGTAAGATCTTGTCTCAAAAAAAAAA
TTGAATTCAGCTAAAAATAATAAAATTTTAAAATAATTTTAAAAAGCCCTCAACAG
CTTTGTTTTTCTCTCCTTGCCAGCTTCTCTGCAGCCTATAGCCTGCAGGCTGGCTG
CTGCGAGCCAGGACAAGCGGTGGGAAATGCAATCACAGCGTGAAATCTCTGTGTTC
AGAGACACGCAGGAAGCAGGTGAACCATGAAGGGCCAACACATGCCCCCAGTTAGC

AGGGTGTAGAGACCGGGGCAGGGCTTTCTTCTTCCTTCTGGGTTATAAATATCCAT
GTCCTGCCATTTGAAGCTGCAAGTGGCACACATGGATGCTGGACAGGCGCTCGCAC
TTTCTGGGCAGGGCANGGGGCTCAAAGGCAGGACAGCTGGGCAAAAGCACCTTGCG
TGGGCCC


Sequence ID - 701                              nt:      579
CTTTGGAGCTTCTGTCTGTGCTGTGGACCTCAATGCAGATGGCTTCTCAGATCTGC
TCGTGGGAGCACCCATGCAGAGCACCATCAGAGAGGAAGGAAGAGTGTTTGTGTAC
ATCAACTCTGGCTCGGGAGCAGTAATGAATGCAATGGAAACAAACCTCGTTGGAAG
TGACAAATATGCTGCAAGATTTGGGGAATCTATAGTTAATCTTGGCGACATTGACA
ATGATGGCTTTGAAGGTAATTAAAATTATCAAATTGGTGCTTGATTTCTGCTTTTA
AAATGGTTTATGGAAGAAAATATGATTAAAGTTTTGTATTGTTTTCCTTCCTATAG
AAGATGGAGCCAGAATGGCATGCTAAGTTTTTTCTTTTCTTTAGTGTTATATATGA
CTTCTCCTCAATTGTCACCCATTGATCTTTACCACTGTTAATAATGGATGATATTC
AAAATACCTTATTTCAGTGATTCTAAGGCACCATTGATTAGAAACTGCATTATTAT
TTATGTGTCCCTAAAAGCTACCTATTAAGCTGTTACACCCACCATTTTTCTGTTAA
GAAAATCCTGATTTCAGAA


Sequence ID 702
GTNNTCCTCTCGGAACGCGCCTTNTGTAGCCAGGTGCTACCAGACCNAATACACGG
TTGTTCCAGCTTGCGCATTCACCGATGGCGTAGATATCCGGATCGGAAGTCTGGCA
GGAATCATTAATGACAATACCCCCACGCGGAGCAACGTCCAGACCACACTGGGTTG
CCAGCTTATCGCGCGGACGGATACCGGTAGAGAAGACGATAAAGTCGACTTCCAGT
TCGCTGCCGTCGGCAAAACGCATGGTTTTACGCGCTTCAACACCTTCCTGCACAAT
CTCAAGGGTGTTTTTGCTGGTGTGAACGCGCACGCCCATACTTTCGATTTTGCGAC
GCAGCTGCTCGCCGCCCATCTGATCAAGCTGTTCTGCCATCAGCATAGGGGCAAAT
TCGATAAC
GTGGGTTTCAATACCTAAGTTTTTTCAGCGCGCCTGCGGCTTCCAGACCTAACAGGC
CGCAATTCGAGCTCGGCCGACTTGGCCAATTCGCCCTATAGTGAGTCGTATTACAA
TTCACTGGCCGTCGTTTTACAACGTCGTGACTGGGAAAACCCTGGCGTTACCCAAC
TTAATCGCCTTGCAGCACATCCCCCTTTCGCCAGCTGGCGTAATAGCGAAAGAGGC
CCGCACCCGATCGCCCTTTCCAACAGTTGCGCACCTGAATGGCGAATGGAAATTGT
AAGCGTTAATATTTTGTTAAAATTCGCGT

Sequence ID 703
CTGCGCAGACCAGACTTCGCTCGTACTCGTGCGCCTCGCTTCGCTTTTCCTCCGCA
ACCATGTCTGACAAACCCGATATGGCTGAGATCGAGAAATTCGATAAGTCGAAACT

GAAGAAGACAGAGACGCAAGAGAAAAATCCACTGCCTTCCAAAGAAACGATTGAAC
AGGAGAAGCAAGCAGGCGAATCGTAATGAGGCGTGCGCCGCCAATATGCACTGTAC
ATTCCACAAGCATTGCCTTCTTATTTTACTTCTTTTAGCTGTTTAACTTTGTAAGA
TGCAAAGAGGTTGGATCAAGTTTAAATGACTGTGCTGCCCCTTTCACATCAAAGAA
CTACTGACAACGAAGGCCGCGCCTGCCTTTCCCATCTGTCTATCTATCTGGCTGGC
AGGGAAGGAAAGAACTTGCATGTTGGTGAAGGAAGAAGTGGGGTGGAAGAAGTGGG
GGTGGGACGACAGTGAAATCTAA

Sequence ID 704
CTTGTATTCAAGAACTACTGTAATGCATTAGTGGTCTGGCTTCATTTTGTATGATG
CCAGATCCTTAATTTACCCAGCACAATCATTTCAGTAGTTTCCTATGGCTCCTGCA
AAAATGCAAACAGAAACCACCACAGGAACAGCCCCTTGCTGCCTCCTGTTGCTGAG
GTAGTAGTCGCTAAAGAAAATTGAAGGCTCCTTACAATCTATATTTGAAAACTAGA
ACTTCTGTAGAAACACACAGATCCCGATCTTAGAAGTTGTACAGGACAATCTGGTA
AAACTGACATAATTGTGATTTATTAACATGAATTAAAATGCCCAACCAGTGCTTCA
GTGTGACAGTATATTTAAAATAAAAAAGAAATTAAAGGTCATATACTGTACTACTT
TCACAAAGATCCACAGTTTTGCAAAAGACTTGTCATATGTACAATGCTATATATCA
AATGAGAAAAGCTGTAAGCAATTATATACGCAAAAGAAATGGCAGTA

Sequence ID 705
TTCCAGTCCTTTCATTTAGTATAAAAGAAATACTGAACAAGCCAGTGGGATGGAAT
TGAAAGAACTAATCATGAGGACTCTGTCCTGACACAGGTCCTCAAAGCTAGCAGAG
ATACGCAGACATTGTGGCATCTGGGTAGAAGAATACTGTATTGTGTGTGCAGTGCA
CAGTGTGTGGTGTGTGCACACTCATTCCTTCTGCTCTTGGGCACAGGCAGTGGGTG
TAGAGGTAACCAGTAGCTTTGAGAAGCTACATGTAGCTCACCAGTGGTTTTCTCTA
AGGAATCACAAAGGTAAACTACCCAACCACATGCCACGTAATATTTCAGCCATTCA
GAGGAAACTGTTTTCTCTTTATTTGCTTATATGTTAATATGGTTTTTAAATTGGTA
ACTTTTATATAGTATGGTAACAGTATGTTAATACACACATACATATGCACACATGC
TTTGGGTCCTTCCATAATACTTTTATATTTGTAAATCAATGTTTTTGGAGCAATCC
CAAGTTTAAGGGAAATATTTTTGTAAA

Sequence ID - 706                     nt:     496

```
CAACCCTCTCTCCTCAGCGCTTCTTCTTTCTTGGTTTGATCCTGACTGCTGTCATG
GCGTGCCCTCTGGAGAAGGCCCTGGATGTGATGGTGTCCACCTTCCACAAGTACTC
GGGCAAAGAGGGTGACAAGTTCAAGCTCAACAAGTCAGAACTAAAGGAGCTGCTGA
CCCGGGAGCTGCCCAGCTTCTTGGGGAAAAGGACAGATGAAGCTGCTTTCCAGAAG
CTGATGAGCAACTTGGACAGCAACAGGGACAACGAGGTGGACTTCCAAGAGTACTG

TGTCTTCCTGTCCTGCATCGCCATGATGTGTAACGAATTCTTTGAAGGCTTCCCAG
ATAAGCAGCCCAGGAAGAAATGAAAACTCCTCTGATGTGGTTGGGGGGTCTGCCAG
CTGGGGCCCTCCCTGTCGCCAGTGGGCACTTTTTTTTTTCCACCCTGGCTCCTTCA
ACACGTGCTTGATGCTGAGCAAAGTTCAATAAAGATTTTGGGAAGTTT
```

Sequence ID - 707                     nt:     397

```
CGGATGTGGTGGCAGGCGCCTCTAGTCCCAGCTACTCGGCAGGCTGAGGTAGGAGA
ATGGCTTGAACCCAGGAGGTGGAGCTGACAGTGAGCCGAGATCGCGCCACTGCACT
CCAGCCTGGGCGGCAGAGCGAGACTCCATCTCAAAAAAAAAAAAAAAAAAAAAATAGA
CTTTGAGACCAGCCTGACCAACATAGTGAAACCCGTCACTACTAAAAATACAAAAA
TTACCCGGGCGTGGTGACGGGCGCCTGTAATCCCAGCTACTTGGGAGGCTGAGACA
GGAGAATCACTTGAACCAGGGAGGCGGAGGTTGTAGTGAACTGAAATCGTGCCCCT
GCACTCCAGCCTGGGTAACAAGAGCGAAACTCCGTCTCAAAAATAAATAAATAAAT
AAAAT
```

Sequence ID - 708                     nt:     293

```
CCAGCTTTTTATGGTGTTTAATCTAATACACTTAAGCTGCAGTCCCAAAATTAGGG
GTCCTTCAGTCTTGGAGACTATAAGGGAGCCTCTGCACCCAGGGAAAATGTTACCC
TTTACAGGGGGGAAGGGTAAACCAGTAGGGAATACAGTACAATCCCAACCCTACTG
GGAGGGGCGGGAGGGAGGTGTTGCCGTCACTGTATTAAGTCGATGTTGGGAAACGT
TTTAACATCTGGAGCCTTTGTGGGTGGAAATATGTCTCCAGTTACAACTCCGCAGT
GGATGTGAAGAAG
```

Sequence ID 709

GGAAGCTACAATGATTTTGGGAATTACAACAATCAGTCTTCAAATTTTGGACCCAT
GAAGGGAGGAAATTTTGGAGGCAGAAGCTCTGGCCCCTATGGCGGTGGAGGCCAAT
ACTTTGCAAAACCACGAAACCAAGGTGGCTATGGCGGTTCCAGCAGCAGCAGTAGC
TATGGCAGTGGCAGAAGATTTTAATTAGGAAACAAAGCTTANCAGGAGAGGAGAGC
CAGAGAAGTGACAGGGAAGCTACAGGTTACAACAGATTTGTGAACTCAGCCAAGCA
CAGTGGTGGCAGGGCCTAGCTGCTACAAAGAAGACATGTTTTAGACAAATACTCAT
GTGTATGGGCAAAAAACTCGAGGACTGTATTTGTGACTAATTGTATAACAGGTTAT
TTTAGTTTCTGTTCTGTGGAAAGTGTAAAGCATTCCAACAAAGGGGTTTTAATGTA
NATT

Sequence ID 710

TGGATTCCCGTCGTAACTTAAAGGGAAACTTTCACAATGTCCGGAGCCCTTGATGT
CCTGCAAATGAAGGAGGAGGATGTCCTTAAGTTCCTTGCAGCAGGAACCCACTTAG

GTGGCACCAATCTTGACTTCCAGATGGAACAGTACATCTATAAAAGGAAAAGTGAT
GGCATCTATATCATAAATCTCAAGAGGACCTGGGAGAAGCTTCTGCTGGCAGCTCG
TGCAATTGTTGCCATTGAAAACCCTGCTGATGTCAGTGTTATATCCTCCAGGAATA
CTGGCCAGAGGGCTGTGCTGAAGTTTGCTGCTGCCACTGGAGCCACTCCAATTGCT
GGCCGCTTCACTCCTGGAACCTTCACTAACCAGATCCAGGCAGCCTTCCGGGAGCC
ACGGCTTCTTGTGGTTACTGACCCCAGGGCTGACCACCAGCCTCTCACGGAGGCAT
CTTATGTTAACCTACCTACCATTGCCCTGTGT

Sequence ID - 711                              nt:      498

GTGGTACATATACACAAAGGAAAACTATGTAGCCATTAAAAGAAAAGGAACTCCTA
TCATTTGTAACAACATAAATAAATCTGGAGGAGATTAGGCTAAGGTGAAATAAGCC
AGGCACAAAAAGACAACTACCATATGATCTTACTTATACGTGTGTGGAATCTAAAA
AGGTGGAATTTACAGAAGCAGAGAGTAGAATGGTGATTACCAGAGGCTGGGGAGTG
AGGGCAGGAGGTTGGAGAAATGTTGGTCAAAGGATACAAAGTTTCAGTTATACAGG
ATGAATAAGTTCAAGAGATCTATTGTACAACGTGGTGGCTATAGTTGATAACAATG
TATTGTGTTCTTGAAAAATGCTGAGAGAGTAGATTTTAAGTGTTCTCACCACAAAA
CATAAGTATGTGAGGTAATGCATGTGTTAATTANCTTAATTTAGACATTTCATAAT
GTATTATACATATTTCAAAACCACGTTGTACATGAGAAAGATACACAATT

Sequence ID 713

GCCCAGTCGACCCATGTTCTCCTTTCTACACCAGCATTAGACGCTGTCTTCACAGA
TTTGGAAATCCTGGCTGCCATTTTTGCAGCTGCCATCCATGACGTTGATCATCCTG
GAGTCTCCAATCAGTTTCTCATCAACACAAATTCAGAACTTGCTTTGATGTATAAT
GATGAATCTGTGTTGGAAAATCATCACCTTGCTGTGGGTTTCAAACTGCTGCAAGA
AGAACACTGTGACATCTTCATGAATCTCACCAAGAAGCAGCGTCAGACACTCAGGA
AGATGGTTATTGACATGGTGTTAGCAACTGATATGTCTAAACATATGAGCCTGCTG
GCAGACCTGAAGACAATGGTAGAAACGAAGAAAGTTACAAGTTCAGGCGTTCTTCT
CCTAGACAACTATACCCGATCGCATTCAGGTCCTTCGCAACATGGTCACTGTGCAG
ACCTGAGCAACCCCACCAAGTCCTTG


Sequence ID 714

CTGTAACAGAGATTCCTTTTTTTCAATAATCTTAATTCAAAAGCATTATTAGACTTG
AAAGGGTTTGATAATCTCCCAGTCCTTAGTAAAGATTGAGAGAGGCTGGAGCAGTT
TTCAGTTTTAAATGAGTCTGCAGTTAATATCAAATGTGAGTTTGGGACTGCCTGGC
AACATTTATATTTCTTATTCAGAACCCTTGATGAGACTATTTTTAAACATACTAGT
CTGCTGATAGAAAGCACTATACATCCTATTGTTTCTTTCTTTCCAAAATCAGCCTT
CTGTCTGTAACAAAAATGTACTTTATAGAGATGGAGGAAAAGGTCTAATACTACAT


AGCCTTAAGTGTTTCTGTCATTGTTCAAGTGTATTTTCTGTAACAGAAACATATTT
GGAATGTTTTTCTTTTCCCCTTATAAATTGTAATTCCTGAAATACTGCTGCTTTAA
AAAGTCCCACTGTCAGATTATATTATCTAACAATTGAATATTGNAAATATACTTGG
CTTACCTCTCAATAAAAGGGTCTTTTCTATT


Sequence ID 717

TCCACCCACCTTGACCTCCCAAAGTGCTGGGATTATAGGCGTGAGCCACCTCGCCC
AGCCCGATACTAGGACTTATGCAGAAAAAACCTTGACATGGAGGAAAGTAAGATCT
AAATAAATACTGTATTCATAGATTAAAAGACTCAGCATAATAAATATACCATTTCT
CCCCAGATTGATGTACAGATTTAACACAATTCCTATCAAGATCCCAGCAAGATTTT
TGTAGATATGTAAAAGATTATTCAAAAATGTAAAAGGAAGGACAAAGGACTAGAAT
AGATAAAACAAAATGGAGAAAGATTTAATAGGAATCACTGTAACTGATTTTAAGAC
ATACAGAACAATAATAGAAACTGCTTGTATTAGTCCATTTTCACGCTGCTGATAAA
GACATACCTGAGATTGGCAATTACAAAGGAAAGANGTTTATTGGCTTACAGTTCCC
ATGGCTGGGGAGGCCT


204

Sequence ID 718

CTCCTCTGGGTTGAAACCCGGGCGCCGCCAAGATGCCGGCTTACCACTCTTCTCTC
ATGGATCCTGATACCAAACTCATCGGAAACATGGCACTGTTGCCTATCAGAAGTCA
ATTCAAAGGACCTGCCCCCAGAGAGACAAAAGATACAGATATTGTGGATGAAGCCA
TCTATTACTTCAAGGCCAATGTCTTCTTCAAAAACTATGAAATTAAGAATGAAGCT
GATAGGACCTTGATATATATAACTCTCTACATTTCTGAATGTCTGAAGAAACTGCA
AAAGTGCAATTCCAAAAGCCAAGGTGAGAAAGAAATGTATACGCTGGGAATCACTA
ATTTTCCCATTCCTGGAGAGCCTGGTTTTCCACTTAACGCAATTTATGCCAAACCT
GCAAACAAACAGGAAGATGAAGTGATGAGAGCCTATTTACAACAGCTAAGGCAAGA
GACTGGACTGAGACTTTGTGAGAAAGTTTTCGACCCTCAGAATGATAAACCCAGC
AAGTGGNGGGCTTGCTTTGTGAAGAGACAGTTCATGAACAANAGTCTTTCAGGACC
TGGACAGTGAAGGGAGCCCGGGCAGCCA

Sequence ID 719

CGNGGCCGCGTNAACTTTTGATCGTCAGCTGGGGCTGGCAGGCACCTAAATGGGAA
GGGTGATAGCAGTGTGTTGGGGGGAGTTTAGGGAACGGTCCTCTACCGATAGAGGC
AGCANCTCATTGGAATTTCCTCCTGAAGTTGTCTTGCCCCTTGAATCCTGCAGGAA
GGCTGGCAAATGGCCATTTCCCTTCCACTTGAATAGAGACCCATAACTCAAGTATC
TGCCCTTAAGACACCACAGGACTGTTCTTCGCGGGCCCTGCCCCTGGATTTGGGAG
AGGCAGTCCANCTCACCCAACTAGGCTCTGCANGGGGACCANGAGGGATGGGTTGT
GTCCACAGGACCAGCCAGACTGATGAGGGATGCGGCAAGCATATTCTCACCACCTT

CTTTCACGTTTACAACANACCAGCNTTCCCTGTGTGGCAGGGGTTACATTGGTCAC
CGAGGACCTANAATCATGGAGTGCTCTGGGGATCCGGGCTTGGA

Sequence ID 720

TCAGTGTTGAATTTTGTCAGACACTTTCTCTGCATCAATTGGTATGACCATGTGAT
TTTTTTTCTGTAGCCTGTTAATATGGTTAATTTTCAAATATTGAGCTGATTAATTT
TCAAATATTGAGCTCTCCTTGCATCTCTGGAATAAGTACCACTTGGTCGTGGTATA
TATTTCTTTTAATATATTGCTGAATTCTGTTTGATCATGTTTTCTTAAAGACTTTC
GTGTCTGTTTTCATGATAGATACTGGTCTATAGTTTTGTTGTAATATCTTGGTTTG
ATTTTGATATCAGGATAATGCTACCTTAATAGAATGAATTGGAGCCAAGTATGGTG
GCAAATGCCTATAGTCCTAGCTACTCAGGAGGCTGAGGTGGTGGGGACTGCTTGAC
CCANGAGTTCAAATCTAGCTTGGGCAATGTAGCAAGAC

Sequence ID 721

TAGAAGGAATGACTATTCATGTCCAAAGTGAATGGTTTTGTGCAGTGAACAACACA
TGGCGAGGTACTAACTGAGAAACTTTTTCATGCTTTATGCCTACCTCTTGTAGTTG
TTGCAGAGCAAATATAAATTGTAATAAGATAGCTAGGCCTTGCAGAAACAAACAGA
AAAACTTAAAAAAAAATGATATAAGAGCTGGAGTCTAGTATTTATATGAATCTGTG
AGAGATAATTTTTTTGGTCTCACTGCAATGAACCAAAAGCGGCTGAGTTTGGTTTT
TAATTGTAGCCATGTATTGAAGGCATCTTTTTGACCAACTCTTGTTGGTTCTGTCT
TGAACCATTGTTAATCACTGTGCTGTAATTAGTATAGCTAAATCTTTTCCTTCCTT
GCTCCTCCCCCAGCCCACCCCGTCTTCCCTTAACATTTTTTCAGGGGGGGTTGGGA
GTGGTTTCATTTTAATGTGAGTGGATGTTTTGATAGTTGTAAGGAAAAAATGCATT
TCAGACACATTTCACACATGAGCTATTTTCTTACACAGTATGTCTTATTGGTAATA
AGAATGTAATTCAT


Sequence ID 722

CNTTCCNTAAGAATACAAAAAATTAGCTGGGCGTGGTGGCAGGCGCCTGTAATCCC
ATCTACTCAGGAAGCTGAGGCTGGAGAATCGCTTGAACCCGGGAGGCGGAGGTTGC
AGTGAGCAGAGATCACGCCACTGCAGTCCAGCCTGGGCAACAGTGCGAGACTCTGT
CTCAAAAAAAAAATAAATAAATTACCTGGGTGTGGCAGCGCGTGCCTGTAATCCCA
GCTACCCAGGAGGCTGAGGCAAGAGAACTGCTTGAACCCAGGAGGCAGAGGTTGCA
TGGAGCTGAGATGGCGCCACTGCACTCCAGTCTGGTGACAGAGTGAG


Sequence ID 724

CTCTCTACTAAAAATACAAAAATTAGCTGGGCACGGNGGTGCATGCCTGTAAACCC
AGCTACCAGGTACTCGGGAGGCTGAGGCAGGAGAATCGCTTGAACCAGGGAGTCGG


AGGTTGCGGCGAGCTGAGATCATGCCACTGCACTGCGGCCTGGAGACAAGAGCAAG
ACTCCGTCTCAAAAAAAAAAAAAAAAAAAAAAAAAAAAAGACNTCACCTAATTGCAG
NGNGNGGACCTTATTTGGCTNTTAATTCAAACTATTAAAAATGTGAACN


206

Sequence ID - 726                              nt:        260

CGGGGTCTGTACCGGGCTGGCCTGTGCCTATCACCTCTTATGCACACCTCCCACCC

CCTGTATTCCCACCCCTGGACTGGTGGCCCCTGCCTTGGGGAAGGTCTCCCCATGT

GCCTGCACCAGGAGACAGACAGAGAAGGCAGCAGGCGGCCTTTGTTGCTCAGCAAG

GGGCTCTGCCCTCCCTCCTTCCTTCTTGCTTCTCATAGCCCCGGTGTGCGGTGCAT

ACACCCCCACCTCCTGCAATAAAATAGTAGCATCGG


Sequence ID 727

CTGAGTNTAGAAATGATGCCATTAATACTGATTGCAAAAACATTACAACTCAGTAC

TGCAGCTTTCATTCAAATAGGTTATATGTATAAACTGAGTTCAACAATATTGTATT

TGAGATGGTAAAGTTAAAGAAATGCAATAATGTAAATAATACTTAAGAAAATAAGA

TCTCAGGAAACTGTATATACTCTGTACTTTTATGCAACTTTATCAGATCATTTCAG

TATATGCATCAAGGATATAGTGTATATGACATGAACTTTGAGTGCAAAAACTGTAC

TATGTACCTTTTGTTTATTTTGCTGTCAACATCTAAATAAAGGTTTTTTTGTTTGT

TTTTTGTTTTTTTAATTGTTTTGTTTTAAAGATTGTTTTAATTAATTAAAAAATTA

ATTGTTTTAATTAAACAATTGTTTAATTGTTTTAAAGTCGCCAGGCTGAGGCAGGT

GAATCACAAGCTTAGGAGTTGGAGGCTAGCCTGCCAACATGGTGAAACCCCGTCTC

TACTAAAAATACAAAAAAATTAACTGGGTGTGGG


Sequence ID 728

CCCATCTGCACCAGTACACAGGCAGGCATTATCATTCTTCACCTACTTTTTAAATA

GTGGCAACTTGGGATTCATTCTGGTGATTCTGAACCTTGCCTCATAGCTTAAAGTA

TAAAAAAGATTCAAGAGCAGTGAGGTTTGTTCTTTCCAGTGAATGGTGGACTGAGT

GGTGCGAGGTGGAGGGCTAACAAGAGGAAAGAACTACATTCTTCAGAATACAGTGA

TGAAAATTCATTTTGAAACTCAAATATTTTCATTTTGGATATTCTCCTGTTTTTAT

TAAACCAGTGATTACACCTGGCCATCCCTCTAAATGTTCTAGGAAGGCATGTCTAT

TGTGATTTTGATGAAGACAGAATTATTTTTCTCTGTAGAAACACAGATACCACTTT

ATCAGGGGAAGTTAGTCAAATGAAATGGAAATTGGTAAATGGACAAAAGCTAGCTA

GTAAAAAGGACGACCCAGCAACATGCTTTAACCCCATTGTATGTTTGTGGAAAGAG

CATAGTTTAACATCTTGAGAAATTTGGGACATAAAAGTTTTCATNGGTAGACAGTT

CATGGCAGTATATGAATTGACATAATGGAAATAATCTGATTTTATTTTTACAACTA

ACATCCTTTCCCC

Sequence ID - 736                           nt:        641

GGAATTCCAAGTGCTTGGGGATAATGATACCTCTGACCTTTCTTCCTTTTGGGAAG

TACTTGAGTGTGCAGCTGCATGAGGCCTCAGCAGGAGAGAGATTTTAGGTCCAAGA

AGCTATACCAGTAGGACAAGGCAGGAAAATACTACACTTTCAGGATCAAGCCCCTC

TGACTCTCATTTGGAAACTGGATGTTTGCTAAGCACCTGCTTCTTAAGGATGCCGA

GGGATTTAATGATACTCCCAGAAACCTGGAGAGATTAATGGGGCCTATGGAGAAGT

GCTCTGAACTCAGTGTTGGGACTTGAATAAAATTAACCATTGTCATGTTTTCAGAA

CAACTAAGCTGTTTTATATTTCATGTGCATGAAAGCCCTAGAACTAAGTTGTGTTA

TTTCCAGAAATGAAATAGATCCCACAGTTAGATGATGTGGCCATTAGGAAGTACCA

AATTTATAAAAATCACTGGAGGTCTGTCTGAGCAGTACCTAATAAAATATAGTATA

CTGAAAGTGAACAGATACTTTGTCTCTTTCTTTGGCTGCTTGATCTTTATCTGTGT

CTGCCGTACAGTGCACCCTTAAAGTATTCTACACCAGTGCTTCTCAAACTGGAAAT

GTGCATGTAAGTCACCCANGGGTCT


Sequence ID 739

TGCATGCCCATAGTCCCAGCTATTTGGGAGGCTGAGGCAGGAAAATCGCTTGAACC

CGGGAGCCAGAGGTTGCAGTGAGCCGAGATCGCACTCCAGCTTGGCGACAGAACAA

GACTCTGTCTCAAAAAAAAAAAAAAAAGAAATCTTGGGATCCTGAACCCCTTACTC

GAAGGGCTAAGGTAGCATCTCAGCATGTCTTATTCGAGACTTCGTANAACCAGACC

TGCTGTTTGTAGATGTTAATTAATCAAACCTTTCTCTACTCATTCTGGACCAGTTA

AGGTTTTCTCCTTCTCCGTATGAGTTTTGATTTTCGTCCTCCTTGGTTGGAGATCA

CACTTTGGTCTGCTGCTAAGTTGGATGCCTCCCACTGTCTTTCCCTAAGTCTAGGG

CTTCANACCCCAGTGTGGGGAGAGGGACTTTCGTTTCCTGCCCCTCACCACATCAG

ACACAGGCAGGCAAGAATAAGATGGCCAAAAGGCCGATGAACTTCTTGACCTAGCC

TGGGACATTACCTGTTACTAGGTGGACTTCACTGCCTGTGAATGGAAGCTGAAGGG

CTGTTTTTTTGGTTTGTATTTGGACAGGCCAGGCTTANAGAGGGAGAGAACTGGGC

TACTCTTCAGCAGTGATCTTTAAAATGCC

Sequence ID 747

CAGAGTGCAAGACGATGACTTGCAAAATGTCGCAGCTGGAACGCAACATAGAGACC
ATCATCAACACCTTCCACCAATACTCTGTGAAGCTGGGGCACCCAGACACCCTGAA
CCAGGGGGAATTCAAAGAGCTGGTGCGAAAAGATCTGCAAAATTTTCTCAAGAAGG
AGAATAAGAATGAAAAGGTCATAGAACACATCATGGAGGACCTGGACACAAATGCA
GACAAGCAGCTGAGCTTCGAGGAGTTCATCATGCTGATGGCGAGGCTAACCTGGGC
CTCCCACGAGAAGATGCACGAGGGTGACGAGGGCCCTGGCCACCACCATAAGCCAG
GCCTCGGGGAGGGCACCCCCTAAGACCACAGTGGCCAAGATCACAGTGGCCACGGC

CACGGCCACAGTCATGGTGGCCACGGCCACAGCCACTAATCAGGAGGCCAGGCCAC
CCTGCCTCTACCCAACCAGGGCCCCGGGGCCTGTTATGTCAAACTGTCTTGGCTGT
GGGGCTAGGGGCTGGGGCCAAATAAAGTCTCTTTCCTC


Sequence ID - 757                              nt:      583
GAACCCTGCGGAGGGACTTCAATCACATCAATGTAGAACTCAGCCTTCTTGGAAAG
AAAAAAAAGAGGCTCCGGGTTGACAAATGGTGGGGTAACAGAAAGGAACTGGCTAC
CGTTCGGACTATTTGTAGTCATGTACAGAACATGATCAAGGGTGTTACACTGGGCT
TCCGTTACAAGATGAGGTCTGTGTATGCTCACTTCCCCATCAACGTTGTTATCCAG
GAGAATGGGTCTCTTGTTGAAATCCGAAATTTCTTGGGTGAAAAATACATCCGCAG
GGTTCGGATGAGACCAGGTGTTGCTTGTTCAGTATCTCAAGCCCAGAAAGATGAAT
TAATCCTTGAAGGAAATGACATTGAGCTTGTTTCAAATTCAGCGGCTTTGATTCAG
CAAGCCACAACAGTTAAAAACAAGGATATCAGGAAATTTTTGGATGGTATCTATGT
CTCTGAAAAAGGAACTGTTCAGCAGGCTGATGAATAAGATCTAAGAGTTACCTGGC
TACAGAAAGAAGATGCCAGATGACACTTAAGACCTACTTGTGATATTTAAATGATG
CAATAAAAGACCTATTGATTTGG

Sequence ID - 758                              nt:        424

CTTGGCTCCTGTGGAGGCCTGCTGGGAACGGGACTTCTAAAAGGAACTATGTCTGG
AAGGCTGTGGTCCAAGGCCATTTTTGCTGGCTATAAGCGGGGTCTCCGGAACCAAA
GGGAGCACACAGCTCTTCTTAAAATTGAAGGTGTTTACGCCCGAGATGAAACAGAA
TTCTATTTGGGCAAGAGATGCGCTTATGTATATAAAGCAAAGAACAACACAGTCAC
TCCTGGCGGCAAACCAAACAAAACCAGAGTCATCTGGGGAAAAGTAACTCGGGCCC
ATGGAAACAGTGGCATGGTTCGTGCCAAATTCCGAAGCAATCTTCCTGCTAAGGCC
ATTGGACACAGAATCCGAGTGATGCTGTACCCCTCAAGGATTTAAACTAACGAAAA
ATCAATAAATAAATGTGGATTTGTGCTCTTGT


Sequence ID - 764                              nt:        626

GATTTTTTTTTTTTTTTTGAGATGGAGTCTTTCTCTGTCGCCCAGGCTGGAGTGCA
GTGGTGAAATCTCGACTCACTGCAACCTCCGTCTCCTGGGTTCAAGCAATTCTCCT
GCCTCAGCCTCCTGAGTAGCTGGGATTACAGGCACCAGCCACCACGCCCGGCTAAT
TTTTGTATTTTTAGTAGAGACAGGTTTTCACCATGTTGGCTAGGCTGATTTTGAAC
TCATGACCCCAAGTGATCTGCCCGCCTCGGCCTCCCAAAGTGCTGGAATTACAGGT
GTGAGCTACCACTCCCAGCCAATGATTACATTTATAAGGTAAAATAACTTGTGCCA
ATCTGTACAAGTGAATTCAGATTTAAAATTTTAATTGTAAAAGATATCCAGGTGA
TATTTCTCCCTGAATAATTTAGTTTCCTTTTCTATTTCTTGATATAAAAGTACTCA
GCATTGAAGTAATTGCTATCTTCACATTTCTTCCTATTTGAGCTGTCTAAATAAGT


AGTCCTACATATTTTCCCCCCAACACAAAAAACCCAGAAAAGAATTATTTTATACT
GGATTTTTTTGGTTGTAGCAGGAACCTAAAGGNGCCAATTGTAACATGCATGTTCT
TTTTGGCAAA

Sequence ID 766

GTCCATCCTGCAGGCCACAAGCTCTGGATGAGGAACTTGAGGCAAGTCACCAGCCC
CTGATCATTTCGCCTAAAAGAGCAAGGACTAGAGTTCCTGACCTCCAGGCCAGTCC
CTGATCCCTGACCTAATGTTATCGCGGAATGATGATATATGTATCTACGGGGGCCT
GGGGCTGGGCGGGCTCCTGCTTCTGGCAGTGGTCCTTCTGTCCGCCTGCCTGTGTT
GGCTGCATCGAAGAGTAAAGAGGCTGGAGAGGAGCTGGGCCCAGGGCTCCTCAGAG
CAGGAACTCCACTATGCATCTCTGCAGAGGCTGCCAGTGCCCAGCAGTGAGGGACC
TGACCTCAGGGGCAGAGACAAGAGAGGCACCAAGGAGGATCCAAGAGCTGACTATG
CCTGCATTGCTGAGAACAAACCCACCTGAGCACCCCAGACACCTTCCTCAACCCAG
GCGGGTGGACAGGGTCCCCCTGTGGTCCAGCCAGTAAAAACCATGGTCCCCCCACT
TCTGTGTCTCAGTCCTCTCAGTCATCTCGAGCCTCCGTTCAAAATGATCATCATCA
AAACTTATGTGGCTTTTTGACCTTTGAATAGGGAATTTTTTAAAATTTTTTAAAAA
TT

Sequence ID 768

CCAGCGCAGGGGCTTCTGCTGAGGGGGCAGGCGGAGCTTGAGGAAACCGCAGATAA
GTTTTTTTCTCTTTGAAAGATAGAGATTAATACAACTACTTAAAAAATATAGTCAA
TAGGTTACTAAGATATTGCTTAGCGTTAAGTTTTTAACGTAATTTTAATAGCTTAA
GATTTTAAGAGAAAATATGAAGACTTAGAAGAGTAGCATGAGGAAGGAAAAGATAA
AAGGTTTCTAAAACATGACGGAGGTTGAGATGAAGCTTCTTCATGGAGTAAAAAAT
GTATTTAAAAGAAAATTGAGAGAAAGGACTACAGAGCCCCGAATTAATACCAATAG
AAGGGCAATGCTTTTAGATTAAAATGAAGGTGACTTAAACAGCTTAAAGTTTAGTT
TAAAAGTTGTAGGTGATTAAAATAATTTGAAGGCGATCTTTTAAAAAGAGATTAAA
CCGAAGGTGATTAAAAGACCTTGAAATCCATGACGCAGGGAGAATTGCGTCATTTA
AAGCCTAGTTAACGCATTTACTAAACGCAGACGAAAATGGAAAGATTAATTGGGAG
TGGTAGGATGAAACAATTTGGAGAAGATAGAAGTTT

Sequence ID 773

GAGGAAAGGGGAGTTAATATTTAGTGGACAGAATTTCAGTTTTACAGATGAAAAGA
GTTCTGGAGATAGACGGTGTTGATAGTTGCACAGCAGTGTGAATGTGCTCATTGTT
ACCGAACTTAAAAATGTTTAACATAGTATTATGTGATTTTTATTTTGCCACTTAAA
AAAAAGAATGAAGTACTGATACATGCTACAACATGGGTGAGCTTTAAATACATTC
TGCTCAGTGAAATAAGCCAGATGCAAAAGATCACATATTATATAATCCACTTATAC

GAGATACCTAGAATAGGCAAATTCATAGAGACAGAAAGTAGAATAGTGGTTCCCAG
GGGCTGGGGACAAGGGGGCAGTGAGAGATTGAGAGTTATTATTAATGCGTACAGAG
TTTCAGTTTGGGCTGATAAAAAAGTTCTGAAGATGGATGGTGATGATGGTTGTACA
TCAATGTGAGTGTAATTACCGCCACTGAACTGCCCTTAAAAACGTTTAAAAGAGTA
AATTTTATGTTGNGTATATTTTACCATAAT


Sequence ID 776
TTTTTTTTTCATAAGAGGCAAGTACAAGAAAAAGCTTAATTACTTTAACTTCTAAG
TAGTTTGGAATCTAAATAAATAGGAGTTACCAAATATATGCGCTTCTGTGAATAGT
TTTCCCCCACATGTTTATTTATATTTTTGCATCTCATCAAACCTAACAGATTCTAA
AGTCTCTGGTGATAATGACAATATCTGCTACGGAGAGACTAGCCTGGGGGAAGAGG
ATCTCCCTGAACAAGGATAGCGGAGTTGCTGCAGCTTTCAAATGAAGCTGGACATT
TAGCTGCGGGGGTAGCACCCTTTGATCAAGGCAGCCCAAAGATGAGTTTCAGGGAT
GGGACTGACAGAAGAGAAAAGTTCTTCCCAGCCCTTTCTACTTTTTCTCTTTGTTT
CTCAGGCTTCTGGCCGTCTTCAGTTTTCACAAGTTTCACTCTCAACCCTAAACAGT
ACTTCTGTGAAGTACCCTTTGGCCCCTCGTTTTCAGCTCCTAAACTCACCTGGAAA
TAGATGTCAATCTAATTTTGGGTCTGACTAGTGCAGTAGGCATTTTTGGTGA


Sequence ID 782
CTCACACAGAACAAAAATGAATGAGTGTGGCTGTGTGCCACTATCACTGTGTCTAC
AAAAACAGCCAGTGGGCCTGATTTGGCCCTTGGCTGCAGTGCGCCCGTCTCTGTTT
TTGAGGAATAAAATCGCATCATTTCATATGGCTAATGCAATTTTTTTCCCATCTGG
AAGCAACATCTGATTGGACTCATCTTGTATGGTGCTTGTTACAGTCTCTGTAAATG
GGAGAGGGTCCGAGAATAGCTCTTCCTGTTTTCATCAGGACTGTTTTTAGGGATGG
CAAAGAAGTCAGTGTGTCCAGCCTGTGTCCTCCTCACCACGTGGCTGATTCCTGAA
TCTGCATGTGCANCACNTGCCGTTGTCTGGGCATGATCTGTGTGA

Sequence ID - 785               nt:      556

CTTTTCTCTGGGTATAGATTTACCCTAGCACCTATCTCATTATATTGAATTTTCCA
GCATATTTAAATAAACTATTAATTAGTCACACTATTTCTTAAAAGTCACACTATCA
ACTAATCGTGACCGCAATTATCTAGGGGTGATAATCTGCTGAGTCTACTCTTTAAA
TACACTGGGACCCAGCATATTGAGTTATATTGGCACAGAAACTTCACTCTGGGTAT
AGATTTACCCTAGTACCTTGCCGGCAGGATCCTATTATTCATGGTTGTACAAGCAA
GGTTCAGGGAAGAGGCTGGCACAGAGAAGGTACCTGGTAACTGTTGTTTGAGGCTG
AATTCAGCTCAACTCAGCTCCAGTAGAGATGGTGTCCCCTTCTCTACCGTGTTGAG
ATAGTGTGCAGTCCCTTCCTAAGGGCTGTTACCCACCGCAATAGGACTTGTCAGCT
TCAACTTTTAAATTTCTCTGCTCCCGCTGGGACCCACCCGCTTCAAAAATCATCAT


GGNGGNTTTAGCACCAATTTAGTAAACACAAACTGTCTGAAATATTTTGGAT


Sequence ID 796

GAACATTCAAGATAGTGAGAGGAAGAAAAAGATATGGCTGTACGGGACCGAGGTCT
CTTCTATTATCGCCTCCTCTTAGTTGGCATTGATGAAGTTAAGCGGATTCTGTGTA
GCCCTAAATCTGACCCTACTCTTGGACTTTTGGAGGATCCGGCAGAAAGACCTGTG
AATAGCTGGGCCTCAGACTTCAACACACTGGTGCCAGTGTATGGCAAAGCCCACTG
GGCAACTATCTCTAAATGCCAGGGGGCAGAGCGTTGTGACCCAGAGCTTCCTAAAA
CTTCATCCTTTGCCGCATCAGGACCCTTGATTCCTGAAGAGAACAAGGAGAGGGTA
CAAGAACTCCCTGATTCTGGAGCCCTCATGCTAGTCCCCAATCGCCAGCTTACTGC
TGATTATTTTGAGAAAACTTGGCTTAGCCTTAAAGTTGCTCATCAGCAAGTGTTGC
CTTGGCGGGGAGAATTCCATCCTGACACCCTCCAGATGGCTCTTCAAGTAGTGAAC
ATCCAGACCATCGCAATGAGTAGGGCTGGGTCTCGGCCATGGAAAGCATACCTCAG
TGCTCANGATGATACTGGCTGTCTGTTCTTAACAGAACTGCTATTGGAGCCTGGAA
ACTCAGAATGCAGATCTTTTGTGAACAAAATGAAGCAAGAACCGGAGACNCTGAAT
AGTTTTATTTCTGTATTAAAAACTGNGATTGGAACAATTGAAGA

Sequence ID 801

CCACTCCACCTTACTACCAGACAACCTTAGCCAAACCATTTACCCAAATAAAGTAT

AGGCGATAGAAATTGAAACCTGGCGCAATAGATATAGTACCGCAAGGGAAAGATGA

AAAATTATAACCAAGCATAATATAGCAAGGACTAACCCCTATACCTTCTGCATAAT

GAATTAACTAGAAATGAGGATTCTGACCTTGACTTTGATATCAGCAAATTGGAACA

GCAGAGCAAGGTGCAAAACACAGGACATGGAAAACCAAGAGAAAAGTCCATAATAG

ACGAGAAATTCTTCCAACTCTCTGAAATGGAGGCTTATTTAGAAAACAGAGAAAAA

GAAGAGGAACGAAAGATGATAATGATGATGAGTCAGGTAAAAGTTCCAGAAATGT

GAACAACAAAGATTTTTTTGATCCAGTTGAAAGTGATGAAGACATAGCAAGTGATC

ATGATGATGAGCTGGGTTCAAACAAGATGATGAAATTGCTGAAGAAGAAGCAGAAG

AAGGAAGCATTTCTGAAATATGAATGAAAAAAATTACATCTTTAGAAAAAGAGTTA

TTAGAAAAAAGCCTTGGCAGCCGTCNGGGGGAAGTGACGCACAGAAGAGACCAGAG

AATAGCTTCCTGGANGAGACCCTGCACTTTACCCATGCTGCTGGATGG

Sequence ID - 808                          nt:      641

CCGGGTTTTAGTATTTAACCAAGAGCCTTTTAAATATTGAAAACCCATAGTTCAGA

AAATGTTAGTATTGCTGCCCTTCTTCACATAAATTTTTTTTTAAATTATACTATTA

TTTTGCTTAATTTTATATTGGGTTAAAACAACCTTCAAGAAGGTTAACTAGGAAAG

AAGACCTTTTTGTTTTATTTTTACTATTTATATATAGAAGACAAATCAGCATTTGG

TGATAGTTTTACATGACCAGTTATCAAACGGTCATAGTATGAAGTGTGCAGTTGTT

CATTATTAGTAAATTATGTTTGATTTTTAAACTATTTAGTACTAATAGTTGAGATG

AAAACTGAAGAAAAATGCCAATGTGACGTTTGTGTATAGCTAGCCTTAAAAAACTT

CCCATGTTTTTAGGTGACTTTTTTTCCCCCTCTTAGTACTCTGGAGAAACAATGAAG

ATGGGCCATCTCAATTCCAGATGTAAACAAAAAGTAATTTTTATTTCAACATTTAA

TGTAACTGCTATTATTGNGGATTCTTGNCTTGNGTATTTTCTTTCCCTTATTCAAG

TAATATAGAATAACTTTCCTTAAAATGATTTGATCCAAGATACGTCATTTCTGTAT

TGGCAAAATGCCNCTATTAAAGTGT

Sequence ID - 814                          nt:      132

GTTAAAGTGATACATTTTTATACCAAATGTGTTTATTTTTTTGTGCAAGTAATCCT

TAAAATTGCAATTGTATTAGGTGTTAAAATAAAGTTTTTAAAAAATTAAAAAAAAA

AAAAAAAAAAAAAAAAAAAA

Sequence ID 817

GACAACCTTAGCCAAACCATTTACCCAAATAAAGTATAGGCGATAGAAATTGAAAC
CTGGCGCAATAGATATAGTACCGTAAGGGAAAGATGAAAAATTATAACCAAGCATA
ATATAGCAAGGACTAACCCCTATACCTTCTGCATAATGAATTAACTAGAAATAACT
TTGCAAGGAGAGCCAAAGCTAAGACCCCCGAAACCAGACGAGCTACCTAAGAACAG
CTAAAAGAGCACACCCGTCTATGTAGCAAAATAGTGGGAAGATTTATAGGTAGAGG
CGACAAACCTACCGAGCCTGGTGATAGCTGGTTGTCCAAGATAGAATCTTAGTTCA
ACTTTAAATTTGCCCACAGAACCCTCTAAATCCCCTTGTAAATTTAACTGTTAGTC
CAAAGAGGAACAGCTCTTTGGACACTAGGAAAAAACCTTGTAGAGAGAGTAAAAAA
TTTAACACCCATAGTAGGCCTAAAAGCAGCCACCAATTAAGAAAGCGTTCAAGCTC
AACACCCACTACCTAAAAAAATCCCAAACATATAACTGAACTCCTCACACCCAATT
GGACCAATCTATCACCCTATAGAAGACTAATGTTAGTATAAGTAACATGAAAACAT
TCTTCTNCGCATAAGCCTGCGTCAGATTAAAACACTGAACTGACAATTAA


Sequence ID - 821                    nt:      370    .

AAAGAGCTCCCAAATGCTATATCTATTCAGGGGCTCTCAAGAACAATGGAATATCA
TCCTGATTTANAAAATTTGGATGAAGATGGATATACTCAATTACACTTCGACTCTC
AAAGCAATACCAGGATAGCTGTTGTTTCANAGAAAGGATCGTGTGCTGCATCTCCT
CCTTGGCGCCTCATTGCTGTAATTTTGGGAATCCTATGCTTGGTAATACTGGTGAT
AGCTGTGGTCCTGGGTACCATGGCTGGTTTCAAAGCTGTGGAATTCAAAGGATAAA
TTAATGAAGAAAACAAGCGGAGCTGAAGAAGAAAGTACAATATGGTGCTGTCTTCC
TAATGAAATAAATTCACTAAATGGACATTAAAAA


Sequence ID 825

AGACTCGAGCAAGCTTATGCATGCATGCGGCCGCAATTCGAGCTCGGCCACTTGGC
CAATTCGCCCTATAGTGAGTCGTATTACAATTCACTGGCCGTCGTTTTACAACGTC
GTGACTGGGAAAACCCTGGCGTTACCCAACTTAATCGCCTTGCAGCACATCCCCCT
TTCGCCAGCTGGCGTAATAGCGAAGAGGCCCGCACCGATCGCCCTTCCCAACAGTT
GCGCAGCCTGAATGGCGAATGGAAATTGTAAGCGTTAATATTTTGTTAAAATTCGC
GTTAAATTTTTGTTAAATCAGCTCATTTTTTAACCAATAGGCCGAAATCGGCAAAA
TCCCTTATAAATCAAAAGAATAGACCGAGATAGGGTTGAGTGTTGTTCCAGTTTGG
AACAAGAGTCCACTATTAAAGAACGTGGACTCCAACGTCAAAGGGCGAAAAACCGT
CTATCAGGGCGATGGCCCACTACGTGAACCATCACCCTAATCAAGTTTTTTGGGGT
CGAGGTGCCGTAAAGCACTAAATCGGAACCCTAAAGGGAGCCCCCGATTTAAAGCT
TGACGGGGAAAGCCGGCGAACGTGGCGAGAAAGGAAGGGAAAAAAGCCAAANGGAG
CCGGCGCTAGGGCCTGGCAAGTGTACGGGCACGCTGCGCGTAACCACCCACACCCC
GCCGNGCTTAATGCCCCNTTCAGGGCGCGTNCTGATGCCGNATTTTNTCTTACNCA
TNTGTGCNGGNTT


Sequence ID 833
TAAAATAATGGCAAAAAACAAACAAAAAACAAGTTCTCTAAACAGAAAGGAAATTA
CTAAAGAAGGAATCTTGAAATAACAGGAAAGAGGAAATACCACAGTAGGCAACATT
ATGGGTAAATAAAACAGACTTTCCTTCTTTAGTTTCCTAAAATATGTTTGATGATT
AATGCAAAAATTACAATATTTTCTTATGTAGCACTAAAGGTATGTAGAGAAAATAT
TTAAGATAATTGTACTGTAAGCGGGAGATGACAGTGACATAAAGGCAACGTTTTTA
TACTTCACTCAAACTTTATGTATTAATGTAATCCATAAAGCAACCAAAAAAGCTAT
ACTAAGTACATTCAAAAACACAATAGATAAACCAAACAAAATTCTAAAGGATGTAC
AAGTAACCCACTGGAAGCTGCAAAAAATGTAAACAGAAACTAAAAACAGAGAATAA
ATGAAAAATTAAAAACGAAATGGCAGACTTAGGCCCTAATATACAAATTATCACAT
TAAATATAAATGGTCTAAATACACCAACTGTAAGACAGAGATTAGCAAAGTCGATT
TAAAAACATGACTCAACTACGTGCTGTCTACAAGAAACTCACTTCAAATATACCAA
GATAGGAAGGTTGAAAGTAAAACGATGGAAAAGATGTATCATGTGAACATTAATC
AAAGGAAAGCAGGGGTGGCTATATTAACATCAGGTAAAATAAACTTT


216

Sequence ID - 837                    nt:        603

TGAGGNTGGTCATGATGCANAAGCTACTCAAATGCAGTCGGCTTGTCCTGGCTCTT
GCCCTCATCCTGGTTCTGGAATCCTCAGTTCAAGGTTATCCTACGCGGAGAGCCAG
GTACCAATGGGTGCGCTGCAATCCAGACAGTAATTCTGCAAACTGCCTTGAAGAAA
AAGGACCAATGTTCGAACTACTTCCAGGTGAATCCAACAAGATCCCCCGTCTGAGG
ACTGACCTTTTTCCAAAGACGAGAATCCAGGACTTGAATCGTATCTTCCCACTTTC
TGAGGACTACTCTGGATCAGGCTTCGGCTCCGGCTCCGGCTCTGGATCAGGATCTG

GGAGTGGCTTCCTAACGGAAATGGAACAGGATTACCAACTAGTAGACGAAAGTGAT
GCTTTCCATGACAACCTTAGGTCTCTTGACAGGAATCTGCCCTCAGACAGCCAGGA
CTTGGGTCAACATGGATTAGAAGAGGATTTTATGTTATAAAAGAGGATTTTCCCAC
CTTGACACCAGGCAATGTAGTTAGCATATTTTATGTACCATGGNTATATGATTAAT
CTTGGGACAAAGAATTTTATAGAAATTTTTAAACATCTGAAAA

Sequence ID - 839                    nt:        71

ATTTATCTAATATTTGGTTTAATAAAATGTGAATAATGAAAAAAAAAAAAAAAAAA
AAAAAAAAAAAAAAA

Sqeuence 849                    nt:        622

TTTTTTTTTATTTTTTGAGAATGGAGTCTTGCTCTGCCGTCCAGGCTAGAGTTCAG
TGGTGCGATCTCAGCTCACTGCCACCTCACCTCCTAGGTTCCAGAGATTCTTGTGC
TTCAGCCTCCTCAGTAGTTGAGAATACAGGAACACGCCACCACGCCTAGCTAATTT
TTGTATTTTTAGTAGAGATGGGGTTTCACCATGTTGGCCAGGCTGGTCTCAAACTC
CTGGCCTAAGTGACCCACCTGCCTCAGCCTCCCAAAGTGCTGGGATTATAGGCGTG
AGTCATTGTCCCCAGCCGGATGTTTTCATCTTGATTTGCCTTAGTTTCTAAATCTC
ATCCTCTCCATTTTCTCCTGTTAGTAGTCACAGAGAACCAAATTCTGTCAAGTTAT
GAAACTAAAGTCTCTCTTCCACAAGTCTTCCTGTGTTCTGCCTCAAGTGAACTTGA
AAGAACATCAGTTTGTGGGAAGGTTGAAGACCGAATGATCTGCTGGGAAATCACTG
AGGCATTGCCATTCTCTTGAGGAATTTCATTTTCATCGAAGTTTCGGTTTATATCC
CTTTCTTGGTGAGTACTATTGCTGTTATGTAAATTAAATGAGTCGTCATCCTTCTT
NTGAGC

Sequence ID - 860                                      nt:        501

GTGAAATCACTTTCATGGATTATTAATGGATTTAAGAGGGCATCAATCAGCTCAAC

TCAAGATTTCATAATCATTTTTAGTATTTAGATTGTGCCTCAAAGTTGTAGTACCT

CACAATACCTCCACTGGTTTCCTGTTGTAAAAACCTTCAGTGAGTTTGACCATTGT

GCTCTTGGCTCTTGGGCTGGAGTACCGTGGTGAGGGAGTAAACACTAGAAGTCTTT

AGTACAAAACTGCTCTAGGGACACCTGGTGATTCCTACACAAGTGATGTTTATATT

TCTCATAAAGAGTCTTCCCTATCCCAAGGTCTTCATGATGCCAGTAGCCATATATG

ATAAATTATGTTCAGTGATAACTTAGTTATCAGAAATCAGCTCAGTGGTCTTCCCC

GCCATGATTCACATTTGATGAGTTTTTAAAAATCAAAGTGATTTTGAAAATCTCTA

ATGGCTCAGAAAATAAAAACATCCAGTTTGTGGATGACTATATTTAGATTTCT


Sequence ID 864

TTGTGTTTTTAGGACTCCTTATCTAAATTAAGGCAGAGAAGTTACAGTATTTATAT


CTGCATTAAATCTCAATTCCAGAAAAACCTTTTGAAAAATTATTTAATCCTCTGGA

AACTATTGATATGATACAGGAGAAATTTTCAGAAGTTTATTGAATAATTTAATATC

ATTTAATAGGACACTCTGGCTTGTATATAAGCAGATACGTTACTCAGACTTCTTGG

CTGTACTCTAAAATAATATATGTACTAGTCTCCTAAATATTACTAGCTCACCTTTC

AAAATGCATACTAATATTTCAATGTCTTTCTTCAATTTGAAAAGCTCTTGAATATC

TACTTGTGATAGCCCTAAGAGCTGAGATAATTATTTCCAGGAGGTTGAATCCCTGA

TTCTTAACTGTTCAGCAATGCATAAGCAAGAGAGAATATGACATAAGAGGACCATT

TCTACATTAGCCATTTTTTTTCACAAGATACCTATGTGAATACAGGGCACCTGGGA

GGGTAAGTGGAGGACTATTTCTAACTATATTTATAAGCACATACTGATATTGGTGA

ATCAAAACCTACAGCAGTGCTTCTCAGATGGGAAGGGAGACAATGTGTAAGGAGAT

CAGGAATTCATTAG


Sequence ID - 865                                      nt:        122

CCANAATCCACTCTCCAGTCTCCCTCCCCTGACTCCCTCTGCTGTCCTCCCCTCTC

ACGAGAATAAAGTGTCAAGCAAGAAAAAAAAAAAAAAAAAAAAAAAAAAAAAAAAA

AAAAAAAAAA

Sequence ID 867

TTTTTTTTTTTTTTTTTTTTCAGAGTCACAGATATTGTATAGCTGAGGTAAGCATTT
TACAACTTTTCAGACACAAGTAAGTACATAAATATTATTTTACAACCAACAATNTT
TAATATTTCCACATTGAANAATAGATGTGATAATTAAATCTTTTATAAGGTTTTAA
AAAGACATGAAACATAAACCTAATTATACATAAAAGAAAGAATTTTAAACAAGAG
CTTATTGNGATGACATTACTCATAACTTTTACCTTTAAAACCTTTTCTTGGGTAGC
TATTCAAAAGTAAAGACCACAAGTTTTGTTGCCCANATTTCTTATGTTTNGTATAT
TTAAGCTCTTTATTTATTGAACAGATGNGTCATTAATTCATTNGGAGCATTACTAT
TATCAGTAAAATTTGATTTTTTTTTCCCCTCAGTCATAGGTAAATCAGCTCCACCT
GGAATTTCTAAGGACCCAGTTTTAGTCAATATTTTCAAGTAATCATGACCTCAGAA
ATAGTCTTAATTAAGATAACAAATATTAGCCATCAAAATGGAACCAAGACAAGATT
CTAATGTTTGTAAACAGTCAATCCATATTTATGAATATTAGCATATATTGGNGAAT
AGTTAAGGCAAAAGGGTCTAGCAG


Sequence ID - 869                              nt:      667
TTGTGTTTTTAGGACTCCTTATCTAAATTAAGGCAGAGAAGTTACAGTATTTATAT
CTGCATTAAATCTCAATTCCAGAAAAACCTTTTGAAAAATTATTTAATCCTCTGGA
AACTATTGATATGATACAGGAGAAATTTTCAGAAGTTTATTGAATAATTTAATATC
ATTTAATAGGACACTCTGGCTTGTATATAAGCAGATACGTTACTCAGACTTCTTGG
CTGTACTCTAAAATAATATATGTACTAGTCTCCTAAATATTACTAGCTCACCTTTC


AAAATGCATACTAATATTTCAATGTCTTTCTTCAATTTGAAAAGCTCTTGAATATC
TACTTGTGATAGCCCTAAGAGCTGAGATAATTATTTCCAGGAGGTTGAATCCCTGA
TTCTTAACTGTTCAGCAATGCATAAGCAAGAGAGAATATGACATAAGAGGACCATT
TCTACATTAGCCATTTTTTTTCACAAGATACCTATGTGAATACAGGGCACCTGGGA
NGGTAAGTGGAGGACTATTTCTAACTATATTTATAAGCACATACTGATATTGNTGA
ATCAAAACCTACAGCAGTGCTTCTCAGATGGGAAGGGAGACAATGTGTAAGGAGAT
CAGGAATTCATTAGTCACCTTTCAGATGGTTTAATGCATACAGCTGTACCG


219

Sequence ID 870

GGAGTTTGAGCAGATCCTTCAGGAGCGGAATGAACTCAAAGCCAAAGTGTTCCTGC
TCAAGGAGGAACTGGCCTACTTCCAGCGGGAGCTGCTCACAGACCACCGGGTCCCC
GGCCTTCTGCTCGAGGCCATGAAGGTGGCTGTCCGGAAGCAGCGGAAGAAGATCAA
GGCCAAGATGTTAGGGACACCAGAGGAAGCAGAGAGCAGTGAGGATGAGGCTGGCC
CATGGATCCTGCTCTCCGATGACAAGGGAGACCATCCCCCACCCCCGGAGTCCAAA
ATACAGAGTTTCTTTGGCCTATGGTATCGGGGTAAAGCTGAATCCTCTGAGGATGA
GACCAGCAGCCCTGCACCCAGCAAGCTAGGGGGAGAAGAGGAGGCCCAACCACAGT
CTCCAGCTCCTGATCCGCCCTGTTCTGCCCTCCACGAACACCTTTGTCTGGGGGCC
TCAGCCGCCCCAGAGGCCTGACTTAGGGGTCTGGCTGTGGAAGGATGTGTGGCCTC
AAATGAGGACAGGGCTCCCGCCTTCACAGCCCTCGCCAGGGGTCTGCCCCAATCCT
GGCCTGCATCAGGCAAGGACGGGGTCTCAGC

Sequence ID - 871                                    nt:       642

GCAAGTCTTCAGTATGTACATTTATCCCCTAGAAGAAGAAAAATTAGTTGTGCATG
AAAAAGAAACATTAACTGCAAAGCTAAATGCTCACACTCTAAATCAGTGCTCTCCA
AAGTACAGCAGGCGGGAAAAGAAAATGGTAGATTTTTTTCTTCCAATTACTTTAAC
TTATTCTTTTTAATGGACACTTCATACATAAATATATTCACAATATATTAATATAT
ACATAATGTATAAGCATACATATTGAATGTGCAGTCAAAAAATGTACTAATGGAAT
GCTCTACCAAAACAAGTTCACGTTCATCTGTAAAATGGGAATAATATTTTTAAAAG
GCATACAGTCTGAACATTTTTAGATTATTCATAAAATCTATTCAGAAAGTTAAACT
AAAAAATTTAACGTATGCCTATAACAAATTTTGTACTTAATGTAATTGNTTTTCAT
CCTGAGATCTAATATCCTCGTTTTTAAGTAGAGCCACTTGTTTGCTACAGTTTAGT
CAAAACGTTAACATTAGATGGGTAAAGTAATATGAAATCTTTCTACTACTCCAAAA
TAGAAAACAGAACATTAAAAAGATAAAAATTCAAACATACTTACCAGTAGATTTTC
AACTGNGCAAAAGCTCATTGCATGGG

Sequence ID 873

GTTTTCCACCGTGAAGAGAACATTTCCTCTGGGAATGACAAAGCCCTCAGGAACNG

CTTTTATTTCTATTGGAAGATGCCCATCATACTTCTGGCAGGATAAAATGATAAAT
TTATTTATTCAACAGATGATACTCAATTCCCTGCTGTTTTACTAAAGGTTCTTTAC
GTTTTATAGAAGCTAAATTTACTGTCATAGAAATTGCAATTGTAGATGTTACTGTA
ATCTAGTCAGAATATCCTTATCCTTCTAAAATAAAACTAGTTAAAATTATTAACAT
ACGTACTGATATTAATTTTTAAGTTTAATGCTGCCACGTGCTTCTGCTAAGAACAT
TTATCACTACAAGTGGCAGAAAATTCCAAACTCATCAAAACCAAACTGTTGCTTCT
TCCCTGCTTTTTCAGAAAATGAGAAAGGATGACTTTATTCCAACATATTCTAAAAG
TATTCCAAGAACACTACCTTTATTCTAAATTCGTTATTTTCACAAAATAAAGGCTG
CAGATTGAAAGATAAAGGATTGCTATTAAAGAACAAAAGAAAACAAAACCGAGAGA
GAAGGAGAGCTAGGGAAATCCCTGCANAANAACCGAATANGGTCCCTCTATTCTGG
GCCGGGGCCTGAAACTATGAAACAGGCCAACACAGAATCTTGGCA


Sequence ID 875
CCTCTGACTCGCTCAGCTCACCCACGCTGCTGGCCCTGTGAGGGGGCAGGGAAGGG
GAGGCAGCCGGCACCCACAAGTGCCACTGCCCGAGCTGGTGCATTACAGAGAGGAG
AAACACATCTTCCCTAGAGGGTTCCTGTANACCTAGGGAGGACCTTATCTGTGCGT
GAAACACACCAGGCTGTGGGCCTCAAGGACTTGAAAGCATCCATGTGTGGACTCAA
GTCCTTACCTCTTCCGGAGATGTAGCAAAACGCATGGAGTGTGTATTGTTCCCAGT
GACACTTCANAGAGCTGGTAGTTAGTAGCATGTTGAGCCAGGCCTGGGTCTGTGTC
TCTTTTCTCTTTCTCCTTAGTCTTCTCATAGCATTAACTAATCTATTGGGTTCATT
ATTGGAATTAACCTGGTGCTGGATATTTTCAAATTGTATCTAGTGCAGCTGATTTT
AACAATAACTACTGTGTTCCTGGCAATAGTGTGTTCTGATTAGAAATGACCAATAT
TATACTAAGAAAGATACGACTTTATTTTCTGGTAGATAGAAATAAATAGCTATAT
CCATGTACTGNAGTTTTTCTTCAACATCAATGGTCATTGNAATGTTACTGATCATG
CATTGGTGAGGNGGTCTGAATGTTCTGACATTAACAATTTTCCAT


Sequence ID - 876                    nt:      115
AAACTTTTGTGGCAACAGTGCACTAATTTGGATAATGTTTGTTCCCAATAAATTAA
GAGCCAAATTGTAAAAAAAAAAAAAAAAAAAAAAAAAAAAAAAAAAAAAAAAAAAA
AAA

Sequence ID - 878                    nt:      634

GCCAGGCTTTGTGAATTACAGGACATTTGAGACAATCGTGAAACAGCAAATCAAGG
CACTGGAAGAGCCGGCTGTGGATATGCTACACACCGTGACGGATATGGTCCGGCTT
GCTTTCACAGATGTTTCGATAAAAAATTTTGAAGAGTTTTTTAACCTCCACAGAAC
CGCCAAGTCCAAAATTGAAGACATTAGAGCAGAACAAGAGAGAGAAGGTGAGAAGC
TGATCCGCCTCCACTTCCAGATGGAACAGATTGTCTACTGCCAGGACCAGGTATAC

AGGGGTGCATTGCAGAAGGTCAGAGAGAAGGAGCTGGAAGAAGAAAAGAAGAAGAA
ATCCTGGGATTTTGGGGCTTTCCAATCCAGCTCGGCAACAGACTCTTCCATGGAGG
AGATCTTTCAGCACCTGATGGCCTATCACCAGGAGGCCAGCAAGCGCATCTCCAGC
CACATCCCTTTGATCATCCAGTTCTTCATGCTCCAGACGTACGGCCAGCAGCTTCA
AAAGGCCATGCTGCAGCTCCTGCAGGGACAAGGACACCTACAGCTGGCTCCTGAAG
GAGCGGAGCGACACCAGCGACAAGCGGAAGTTNCTGAAGGAGCGGCTTGCACGGCT
GACGCAGGCTCGGCGCCG

Sequence ID 879

GTTGCCGGGTCCTGTGATAACTCTGTTTAACATTTTGAGGAACTGTTGAATGGTTT
TTCACAGCAGCTGCCTCATTTTTTATTCCCATCAGCAGTACTTCTTGGTTCTAATA
CCTCCACGTTCTCGCCAACACTTGTTGTTGTCTGTAATTTCGTTGTTAGCCATCCC
AGTGGGGATGAAGTAGTATCTTACTGTGGTTTTCAGTTGCGTTTCCCTGATAATTA
ATGATGGTGAACATCTTTTCATGTTCTTGTTGGCCATTTGTATGTCTTCTTGGGAA
AAAAAAAATGTCTGTTCAAATCCTTTACAAAGTATTTATTTTTTATGTCAACAATA
TAACCACTCAGTACACTGCTTTTTANACAATGATCTTTTAAAGGTTTGTTTACAAC
ATTTAGCACTTGAAATTTTAAGGTTATGCCCTCAAAAAAATTGCTGAGGGAGCTAA
GCTATGAAGATGCAAAGGCATAANAATTATACAATGGACTTTGGGGGAATCCAGGG
AAAGGGTGGGAGGGGGGTGANGGA

Sequence ID 881

TCGACTCTGATTTTTTTTTCTCCTTCCTCGCAGCCGCGCCAGGGAGCTCGCGGNGC
GCGGCCCCTGTCCTCCGGCCCGAGATGAATCCTGCGGCAGAAGCCGAGTTCAACAT
CCTCCTGGCCACCGACTCCTACAAGGTTACTCACTATAAACAATATCCACCCAACA
CAAGCAAAGTTTATTCCTACTTTGAATGCCGTGAAAAGAAGACAGAAAACTCCAAA
TTAAGGAAGGTGAAATATGAGGAAACAGTATTTTATGGGTTGCAGTACATTCTTAA
TAAGTACTTAAAAGGTAAAGTAGTAACCAAAGAGAAATCCAGGAAGCCAAAGATG
TCTACAAAGAACATTTCCAAGATGATGTCTTTAATGAAAAGGGATGGAACTACATT
CTTGAGAAGTATGATGGGCATCTTCCAATANAAATAAAAGCTGTTCCTGAGGGCTT
TGTCATTCCCAGAGGAAATGTTCTCTTCACGGTGGAAAACACAGATCCAGAGTGTT
ACTGGCTTACAAATTGGATTGAGACTATTCTTGTTCAGTCCTGGTATCCAATCACA
GTGGCCACAAATT

Sequence ID 883

TCATTTACATTAATACTCAAAACTGCTCGATTAAGCAGGTGCTGTTCTTATCGCCA
TTTTGCATATGATGAGAAAGGGTAAGGTCACCCAGCTAGTATTTGGCTCACAGCAG
GCCTTAAGACTTGGTTTGTGTGACTCATCAGTCCACGCTCCTAAAACCACTAAGTT

GTTCTACCCTTTAATGTTGAATTAACATTGGATAGTGTTCAAGTTTANATGGGTGG
GTGAGGGCCCAAGGACCTTTCAAACTCAGATCTCTTATTTAATAACCTGGTCCCAG
ATCCATTCCTCTGTCGAAGAGGAAGTCATCCTTCAGTGGCTATTCATTGTGGGGTT
AAGAGCGCAGACTATGAATTCAGTCTTTTTGGGTCCCAGTTTGCCAGACCTTGAGT
GAGTGCCCCGAGTTTACTTACTTGTAAAGGTAGGTGGAGGTAATATAATTAAATAA
ACTTAAAAAACTAATTAAAAACAAACAAATGAACTAAGGTCTTAGGATATCTGGC
GTCTATTTTGCGCCAAATCACATAATGTCTATTGTTGTGTGTTGGACTATAGGATT
GTCCTTTAACAGGGAAGGGTTTATTTCTGTAATCAAGTCTGTCAATATTATGACCA
TGTTGATAATAGCTACCTTTAATTGAGGGCTTCCATGTGCCAA

223

Sequence ID 885

TCAGTGGAAAAGGGCAGGTTGAATCAAGGTGAATCAATCTGAAATTGAGCACACCT
GCCTGCCATCGCTGTTCCTTCAACTGAGTGCTGCACATCATGGGCTCTGTCTGTGA
GAGAAAAATCCCGGTGCTTGGTGTCCTTGCATGACATGGAGTTTTGCATGTAGATC
AATTTAAAATGTACCTCTTGTTTACATAATTTGCATAATTTTAAAAGATAATGTTG
CCAAACTTTGGAAATGTTAATGTTCANACTGAAAATCTCCACTACATGTAACTTTC
TTCCTCTGGATCAGTGGCATGGCTTATAATCCCAGCCAGTGGTTTGAACTGTTCCA
GTGTCAACTGCCATGTGCTCTGCTTCAAGGGGGAACTAGCCTTTTGTGAATTTTTT
GTACATAAGTATTTGTTACAAATATTTTAGCAAATGCTTTCTATTTCTCTTGCTTG
TGCATATCTTGGCTGGCGTTACAGAAAAATAGTGTAAACATTATTTCCTTACCGGG
GAATGAGGGTTTT

Sequence ID 887

AGCACCTGGCACAGAGTAGTAGCTAACACAGATGTTAATTTTGCTGCGTCAAATGT
TTTCACTTTGAATCTCTCTTGAGTATTGTTCTCCTTATTGATTACATGATGACATC
CTGTTTTCTCTCCCTGACCTTTACTGTTTGTTTAGAAAAAAAAAAAAAAAAAAAAA
AAAAAA

Sequence ID 889

CAGAGAGCTTGTTCCCTCCCTCCCTGTGCATGCAAACAAGAGGGCATGGGAGCACA
CAGAGAGATGGCAGCCACCTACAAGCCAAGAGGAGAAGCCTCACAATCAAACTCTC
GCTGCTGGCGAGAGTCTTGGACTCTGTCTTGGACTTCCAGCCTCCAGACTGTGAGA
AACAAATTTCTGTTGTTTCAGCTTCTCAGTCTCTGGTGTTTTGTTATTGCAGCCTG
AGAACACAGCTGTACNATTATNAGGGAAACAGAAAACACTGATACTTAACAATGCT
AATGCAATTATTTATTTGCTTTTCAGTCTCTACAAAACGTTCTAAAACACTAATCT
AAATATTAACAGTAAAATATTTGCATAACTAATGGAAACTAAGAAATCATATGACC
AATATTTCACTTATTGGTAATCTTACTCTACTGATTTCCCCCCAGACTGTGATTTT

TGAACTTCCTTGCCTTTCTCCTGTCTTTCTGNGTTTATTCATGGAATTCCAGTTAT
CTGGGCTTGAAATTGCAGGCTCTCCTAACTTAAGCAAAATCTGACAGATCAGCAAA
ATGAGATAAATGTTTCTTTTTTCTTTCTGACTGCATTAAATCAGATACAACTCAGC
ATTAAAAAGCTATCTTTGNAAAATGNTGGTACTAATAAATTAGTCTTA

Sequence ID 890

CCAGTTCCACATTCAGTGAAGTCATGAACTTGAAATTGGCCATGATCAAAAAGTAT
TTAAATCACAGAAGTTGCAAATGCCACAAATCAAGGTCTTTTTCTCTTGGAGAACC
TGTTAAACATTTACCAACTCACGACCGCCATGCACCCAATACTGCAATAGGTCTAT
AGATGCAGATACTGTCTCCATGAATCTTATAGGCTAGAAAGGAAATAGATAAGTAG
TCCTACCAGAAGAACATGATGAAGGCATTTGTGGTAAACAGAATGATGGCCCCCCA
AAGATGTCCACATCCTAATCCCTGAAGCCTATGAATATACTACTTTACTTGGCAAA
AGGGACTTTGCCACAGGTTTTTAATTAAGGACCTTGAAATAGAGAGATTATCCTGG
ATAATCCAGATGGCCCCAGTGTAATCCCAAGGGTCCTCACAAAGGGTAGGAAGGAG
AGCCAGAGTCAGAGAAGGAGACGTAGCAATGGAGGCAGAGGTCANAGAGAGATCTG
CAGATGCTGCTGTGTTGGCTTTGAAAATGAGGAATGCAGGTGACCTCAANGNGCTA
GATGATGCAAGGAAACAAATAATCTCCTATGAACCCTAGGATGGGCATTATTATGA
GTCCTATTTTATAAACAAGGAACTGACNTCCAGAAAGATAAATGC


Sequence ID - 891                         nt:      626

GGCAGAGGTTGCAGTGAACTGAGATCATGCCATTGCAATCCAGCCTGGGCAACANG
AGTGAGACTCCATCTCAAAAAAAAAAAAAAAAAAGACAAGAGTNTCCACTCTAAACA
CTTNTATTCAACATAGTCCTGAAAGTCGTAGCCACAGCAATTTAACAAGATAAAGC
AATAAAATGTATTCAAATAGAAAAAGAGGAAGTCAAATTATCTTCACTGGNGATAT
AATTCTCTACCTGGGAAACTTCACCGAAAAGATTTCACCAAAAGATTTCTAAGCC
TAAATAATGACTTCAGCAAAGTCTCACCATACAAAATCAACATACACAAATGAGTA
GCATTTCTGTGCACCAATAATATTCAAGCTGAGAAAAAAGAACATGGTTCTATTT
ACAATAGCTACAAACAAAAAAATATGTACCTAGTAATACATTAAATCAAGGNGGTA
AAATATCTNTACAACAAGAACTACAAAACTGCTGAAAAAAAATAGAGACACGCAAA
TAAGTAAAAAGGCACTCCATGCTCATGAATTTAAAGAATCAATATAATTAAAATGT
CCGNGCTGCCTAAAGCAACTTACAGATTAAAGGCTATTTCTCTCAAACTATAAATG
CACCTTTTTA


Sequence ID - 893                         nt:      585

GTCATTGCTGGGTGGCGCCAGCCCTCAGACTTGCCTCTTTGCAGTAGGAAGAAGGC
CTCCCCACATACCTTCCCACACTCATCACCTTAAGCCAGACTCGGTGTCCAGTGAA
TATGACCATCTCTTGCCCATTTTCTAATGAGTGTTTTCATTAATGAGTTATAAGAA

225

TGTGGTGGGTAAATCTATGGGCTTTGAACTAGTGAATCAACTTGGTTTCAGAATCT
GGCACTGCTACTTACTAGTGAATTTAAGCAAGTTATTTCACCTTTCAGAGTGTCAG
TTCCCTCATGCATACAAGGAAGATAAAAAATAATGTNTACNAAAGTATTGGAGTAA
TTAATACATGGAGAACTACATGTAAAGCGTTTAGCATGATGTCTGACATATTAAGC
ATCCAATATTAGTNGCTTGCAGAATTATTAGTAAAAGAGATTGCTTCTGAAAGCCA
TTCCAATTCTTAAATTTTATAATGCCACATTTGAGGTCACCTGAAGTCGTGTATAA
CATGTGTACATTTTTGCGATTTATTTTTTCAATTCCCANATTAAAGGCATAGAGAT
ATCCTAGCNANGGACTCCAAGTGTG


Sequence ID - 895                        nt:        560
GTAATTGCAGCCTGGGCAACGGAGTGAGAGACTGTCTCAGGAAAAAAAAAAGAAAA
AAAACTACTGAGGTAGTTGAATATATCCTCCATTCCCCATTTGTGGATTAGTTAGT
AAATGGGGCATCTTAGGGTTTAAATATGTCCAGGGTCACTGAGGATCAGATCCTAG
GGTTCCTTTGACTCAAGGCTTTTGTCTCAGCAAAACGTCACCTTCCAGCAGGAAGG
CTTTCTCAGGCAAGTAGCAGGGTGGCTACTATGTATCGCTTCTTTATTTTTTCTTT
TTTAAAATAATGCAGGCACCGTGCGCATAATTTAAAAAATCAGTGCTAAAACCCTT
AAAAAAAAAAAGCTGTTCTCATCTCCTGTCTTTCTTTTTTTTTTCTTTTTATTTTT
TTCTTTTATTATTATTATACTTTAAGTTTTAGGGTACATGTGCACAACGTGCAGGT
TTGTTACATATGTATACATGTGCCATGTNGGTGAGCTGCACCCATTAACTCGTCAT
TTAGCATTAGGTATATCTCCTAATGCTATCCCTCCCCCCTCCCCCCTTTTTTTTTT


Sequence ID 896
GGGAATGTCTTAGGCACTGGGACTGTAAGTGCAAAGACCCTGTGGCACAAGGGAAT
GTTAATTATCTACCTTTCANAAACTGGAANAAGGCCTAGCCTAGAGCATTGAAAAC
AATAAGGGAAAGGAGGAGTAAGGCTGGANAGATAGGAATGGTTTAAAGTCTTTGTT
AAAAATTTTTTTAAAAAAATCTTTATCACAAGAAGAGGATTGGCNTGATCAAATTT
GACTTTTAAAAANATTACTTGGGTTGGGCATGATCAAATACTACTTAGGGAGATTA
GTTTANATGATAATGGCATTCTGGACCANAGTGGAGTCAGAGGTGAAAAGAGGTAG
ATATTCCANAATTGAGGGATTTGTGAGGTGAAATCATTTGTTACAGATATTAAAGG
ATAAGGAGCTTTGTCAAAGGGGATCTTAAGTTTCTGGTATGGTAACTGGGTTAGAG
AGCCCTGGAACATGACCAGCTTTAAGGGAAGAGAGCTTGAGCTCTGTTCTTGTTAA
GCTCAGTTTGAGATCTTTGTGGAATCAAGTGGAGAGGTCTAAGCAGGGAACTGGCT
TGGCTAGGCTGTAAAGATGAATCTGAGAGTCCCAAGAATATGGTAATTATTAATAA
AAGCCTTAGGTANATGAAATTGTTTTGGG

Sequence ID - 897                      nt:      509

GCAAATCTACACATTTGATTAAATGATAGGGAACTATGCACACACATAATACATAT

AATGCTAGTTTCTTGGTTTTGATATTGTACCATAGTTATGTAAGATGTAACCATTG
GGGGAAACTGGGTGAAGGCTACATGAGACCTCTCTGTACTTAATCTTTGCAACTTA
TGTGAATCTATAATTATTCCAAAATAAAAAGTTTTAAAGAACCTAAGTATCCTTAT
TACTGAGGGTCATCGTGCTAGACAGCAAGGTTGGGCCAGAGCTTCTAGTTATTTAA
AATACTAAATACCAGCCTGGGCAACATAGCAAGACCCTGCCTCTACAAAAAGCAAA
AAAATTAGCTGGGCATGGTGGTACATGCCTGTGGTCCTAGTTACTCTTGGAGGAGT
CTGAGGTGGGGAGCTTGAGCCTAGGAGTTTGAGGCCGCAGTGAGCCTTGATTGTGT
CTCTGTACTCCAGTCTGGGCCACAGAGCAAGACCCGGTCTCTAAAAATAAATAAAT
AAATA

Sequence ID 898

ANTGCACTCCAGCTTGGTGACAGAGGGAGACTCCATNTTAAAAAAAAAAAAAAAAA
AAAAAAGGGAGTAGCTTGAAGCCACATAGTAGTTAGTGGTAAAGGCCACCCCTTT
TCCCACAACTCACACCAGCACCACAAGCTAGCCTTTNTAATTTCCAAGCCAGTGCC
CTTTCAACGCACACACCCCTGTGTCAGTTCCCTTTCTGCTGCAAGCTCTCTGGAGG
CAGATACTGTTGAGTCCCTGGCCTGCCTATGAGAACGGCTCATGATCTCTATTTCT
TCTGCTTAATGACCATCTCGAAGTAACAAGTTTAGCCTAAAATAAACTTGCTAAGT
TAGCAAAGGAAGTCCTTAGCAGCCACCATTTCTCGATTCCTCCATCACCTCCCCTG
CCCCTCAACTCCCTCATTTCTCCCAAGATATGGGCTCCAGGCTGGGCGCGGTGGCT
CACGCCTATAATCCTAGCACTTTGGGAGGCTGAGGTGAGCAGATCACTGAGGTCAG
GAGTTCG

Sequence ID 899
TCNTTCGGAACGCGCC

Sequence ID 900

```
CTGGAGGGATGGGTAGGATTTTGACAAGAGTGGTTGAAGGTATTCTAATTCACTTA
GTACCTACATGTGCGAGGCAGCATGAAGGCAAAAAAGCCTGGGGCATGTTCAGAGA
ATAGCAAGTATTCTAGTTTGAGTGGCACCTGGTACGTATATAAGGGAATAGTAAAA
GATCTGGCTGGAAAGGAAAAGTAGGGGCAGGTTACGAAGGACCTCTGAAAGTCAGA
CTGTGGAACTGGAACTTTTATCAGGAAGCAGTAGTTAGTTTTTTTCAAGCAAAAGCT
AATTAGAGTTGATATTTAGGAGGATGAATCTAACAGTTGTGTGCAAGGATGCCTTC
AAACTGAGTGAGACTAGTACTGGAGACTGGTTAAGAGACTACAACAATAACCTGAG
TAAGAATTAATACAGGCCTGACCTAGTTTTGAGTGAGTAGGATTGGAAACAAGAGT
TTTAGGTATTATAGGATTTATGCATATAAAATGGACTTGACAGAACTTGAAGAAAG
AGAAAGTGTCAAAAGGACACAGAAAGTGAGGCAGGATATCTTACAATGTTAAAGGA
AAGGAATAATAGAAGTTAC
```

Sequence ID 903

```
GGAAACATAAGCTTGTTTCAGTACACTCACGCTGTAGATTAATTCTGATATTACAT
ATCTCCATCAGACTTTGTACCCTCTCTCTTCCATCCCTTACCCTTACCGATTAGGT
TGGTATTACCTAAAAATCCATAGAAAATGTCCAGGTGAATTGCCTTATGCTTTCTA
CCCCATAAGGTATAATT
```

Sequence ID 904

```
CTCTGTGGTGTGAGAACACAGTGGGTGACCAAGGCTTTCCAGATGAACCCAAGGAA
AGTGAAAAAGCTGATGCTAATAACCAGACAACAGAACCTCAGCTTAAGAAAGGCAG
CCAAGTGGAGGCACTCTTCAGTTATGAGGCTACCCAACCAGAGGACCTGGAGTTTC
AGGAAGGGGATATAATCCTGGTGTTATCAAAGGTGAATGAAGAATGGCTGGAAGGG
GAGTGCAAAGGGAAGGTGGGCATTTTCCCCAAAGTTTTTGTTGAAGACTGCGCAAC
TACAGATTTGGAAAGCACTCGGAGAGAAGTCTAGGATGTTTCACAAACTACAAAGC
TGAAGAAAATGAAGCCCTATTACTTGTTTGTAAGATTTAGCACCCTTCTGCTGTAT
ACTGTACTGAGACATTACAGTTTGGAAGTGTTAACTATTTATTCCCTGTTAAAATT
TAACCTACTAGACAATGATGTGAGTACCCAGGATGATTTCCTGGGGCACAGTGGGT
GAGGAGATGGGGACAGGTGAATGGAGGAGTTAGGGGAGAGGAAAAGTGGATGGAAG
TGTCTGGAAAGGGCACCAAAAAAGTCTTCCAGGTCTGATCCTGTTTCTTGCTCTGA
GTGCTAGCTACCACTGTGTCACACTGTAACATN
```

Sequence ID - 905                              nt:      655

CTCAGCTCTTGCCTGGTCACCTTGTGGCTTTTACCATCCTCATCCCCTGTGCCACC

CACATCCTGCCACTTCTGCATGGAGTTGGGGTGGGGCCATTGGAGAAAAGAGGTTA

AACAAGCAGTAATTTACTTGAGTACAGTCTTTGAGCCAATGAAATGCCAGTCATCA

TTTCCCAGGGGTACTTGTCATCTTGTCAACAACCCGCTGATAATGCTCCTTCAATG

TGAATAGCAAAAGTAGGGAGAGACGCTGAATGAAGAAGATGCCTACCCCTCAGGAA

GACTGCTGTCCGCCTCCAGGCCTGCATGCACACACCCATGCCCACCTGCACCCCCA

GCACCACGCCCACACTCACTCGCACACACCCACATGCCAGTGTTTTGGGGTTGGCA

GCCTGGACACTGCTGAGGCAAACACAAGTCATCAAGCATAATTCTCATTCTCTCCT

TCTGTCTCTGTTTTAGTTACAGGAATTTGGTCAGTTTAGAGGATTTAATAAGTCCG

TGGAAAATTTGTTTCTGTCTCTTGCTACCCACGTGAAAAGTAAGTGCATGCTTCAT

GATGTGTTTTCCCACTACCTTCCAGGCCAGCCGAGCCCACTGGCCANGGCCTGGCC

CGGTGACCTCGGTTGACACTGTCCTCANGCCACTCACTT


Sequence ID 906

CAGAATTTCATGTTTATGCTGCACAAGGCCTGTATTTTATAATGGTGGCTCTTTTG

GACGATGACTTCCTCGATGGTGAAACTTCCAGTAATCTCCCTCATCATACTGAAAT


GATATCAGTATATCATCAGAACACCATGGAGCTTGTCATTTGAGGGACACAGCTTG

CTTGTGTGCTTGGGAAAGAAGAGGTTTAGCATGGTTTCAGGTCAGTGATGAGTCCA

ATGATCTCTGCAAGTTCCCTTAGCTCTGANAATTCTGATGTCATATGCACTTCTGC

CGCCAGAGTTGCTGCTTACTGGATGCGTAAGAAGAAAAGAAAAAAAAAAAAAAA

Sequence ID - 907                    nt:      582

CTTCCATTGGGGGTAAAGATCAAACTTTAGGCGAGCCAGGTCTGTATCTCCATTCC

TGTCTCTGACTGCTTCCCTGTAGGGATTGTCTGCAAGCGCACACCTGCATTTTCTT

GTCCACAAGTCTATGCTCTAACTCTGTCACCTGCATGGCTGCAAATTAGCTTCCTT

CTTCCTGCCCTCTTCTCTCTAGCTTGGATTTTGAATTTGAATGGCAGGCATGGGAT

GTCCGTGTGTGTGTACTGCTGATGTGTACAGCCGCTTGTTAGCGCTCTCATTGTCT

TCAAATGTAAGTCATTTTGGCTGGGTGCGGTGGCTCATGCGTATAATCCCACGCTT

TGGGAGGCTGAGGTGAGCTGATCATTTGAGGTTAGGAGTTCGAGACCAGCCTGGCC

AACATGGCAAAACTCCATCTCTACCAAAAATACAAAAATTAGCTGGGTATGGTAGT

GCACGCCTGTAATCCCAGCTACTTGGAATGCTGAAGCAGGAGAATTGCCTGAACCC

ANGAGGCGGAGGTTGCGGTGAGCCAAGATCACGCCACTGCACTCCAACCTGGGTGA

CAGAGCAAGGCTGTGTCTCAAA

Sequence ID 908

ACCTGACTTCAAACTATACTACGAGGCTACAGTAATCAAAACAGCATGGTACTAGT

ACAAAAACAGACCAATGGAACAGAATAGAGATCTCAGAAATAAAACTGCACATCTA

CAACCATCTGATCTTCAACAAACCTGACAAAACGAGCAATGGGGAAAGGATTCCCT

ATTTAATAAATGGTGCTGGGAGAACTGGCT-AGCCATGTGCAGAAAATTGAAACTG

GACCCCTTCCTTACACCTTATACAAAAATTAACTCAAGATGGATTAAAGACTTAAA

TGTAGAACCCAAAACGATAAAAACCCTAGAAGAAAATCTAGGCAATATCATTAAGG

ACATAGACATGGGCAAAAATTTCATGATGAAAACATCAAAAGCAATGGCAACAAAA

GCAGAAACTGACAAATGGGCTTCTGCACAGCAAAAGAAACTATCGTCAGAGTGAAC

AGACAACCTACAGAATGGGAGACAGTTTTTGCAATCTATCCATCTGACAAAAGTCT

AATATCCAGAATCTACAAGGAATTTAA

Sequence ID 910

CAAAAAACAAGAATTACCCGGGCTTGGTGGTGCATGTCTGTAGTCCTATCTACTCA

GGAGGCTGAGGCTGAAGGATCACTTGAGCCCAGGAGTTTGAGGCTGCAGTGAGTGA

GCCATGATCATGCCAGTGTACTCCAGCCTTGGCAGACTGAGCAAAACTTGGTCCCT

CGCAAAATGTTGAAGCCCAGTTTTTCACTATTAACCTGTATTTCAGTTTCCCCATGC

TAACTTTGAAACACTGGGGCTGGCCTGAGGGTATAAAGGCTTATTCAAACTCAGTA

ATTTAAACTTAAAATCCTAAGGAACTTCAAAAAGTGTAATCTAGTCCAAATGGGGC

```
ATCAATTCTAAAGCATTTGCTTGTTTGAGCAGATTTTCTGTGTCTGAGGTATATAG
ATAACTTATCTTTTTATGACTAAATCCAAGTCCTTAGTTCCTGTTGGAATTCAAAA
TCATATTTAAAAATTGATGCTTTGTTCTATAATTAATGCTTTGATTGTATAAATAA
TAAGTATTCTTCCAAATCCCTTTTTACAGATGATGATTCTGATACCGAGACGTCAA
ATGACTTGCCAAAATTTGCAGATGGAATCAAGGCCNGAAACAGAAATCAGAACTAC
CTGGNTCCCAGTCCTGTNCTTAAAATTCTAACTCGAC
```


Sequence ID - 911                              nt:      595

```
GAGGGTGTAGAAGAGAAGAAGAAGGAGGTTCCTGCTGTGCCANAAACCCTTAAGAA
AAAGCGAAGGAATTTCGCAGAGCTGAAGATCAAGCGCCTGAGAAAGAAGTTTGCCC
AAAAGATGCTTCGAAAGGCAAGGAGGAAGCTTATCTATGAAAAANCAAAGCACTAT
CACAAGGAATATAGGCAGATGTACAAANCTGAAATTCGAATGGCGAGGATGGCAAG
AAAAGCTGGCAACTTCTATGTACCTGCAGAACCCAAATTGGCGTTTGTCATCAGAA
TCAGAGGTATCAATGGAGTGAGCCCAAAGGTTCGAAAGGTGTTGCAGCTTCTTCGC
CTTCGTCAAATCTTCAATGGAACCTTTGTGAAGCTCAACAAGGCTTCGATTAACAT
GCTGAGGATTGTAGAGCCATATATTGCATGGGGGTACCCCAATCTGAAGTCAGTAA
ATGAACTAATCTACAAGCGTGGTTATGGCAAAATCAATAAGAAGCGAATTGCTTTG
ACAGATAACGCTTTGATTGCTCGATCTCTTGGTAAATACNGCATCATCTGCATGGA
GGATTTGATTCATGAGATCTATACTGTTGGAAAAC
```


Sequence ID - 912                              nt:      651

```
CATTTCCAGAGTTTATGTGAATTGAATTGAACTATGGTTTTATGTTACTGTCAGTA
GAATGAAGTACGAATATTTGAAAAATACACCTTCAACTTCAAAGTGATTCTTGACA
AAAATTATAAGGAATCATTTTGGACACATTTTCTGGTAGAGCCTTGTAAAAATTAA
AACCAAGTGTTGTTTTCAAGAAGAACTGTAATACATAATCAGGAATTTGAGTAGGG
AGATTATTTTGTTATTTAAAATTAAAGTGGCTGTGTAGTTTTAACTTTAGTATTGC
AGGTAGAGTAAGCTTACATGATAACAAAAATCTTGGTCTTAGTGACTTAATGATTC
TGATATTTATTGATTGATTGGTTATCATTCCAAATATTTTAAAAGATAATAGCTGG
CTGGGTGCGGTGGCTCATGCCTGTAATCCCAGCACTTTGGGAGGCCAGGACGGGCG
GATCACGAGGTCAGGAGATCAAGACCATCCTGGCTAACACGGTGAAACCCCGTCTC
TACTAAAAATCAAAAAATTAGCCGGGTGTAGTGGCGGGCACCTGTAGTCCCAGCTA
CTCAGGAGGCTGAGGCAGGAGAATGGCATGAACCTGGGAGGCGGAGCTTGCAGTGA
GCTGAAATCGTGCCACTGCCTCCACCTGGCGACAA
```

Sequence ID 913

GTGAGGTGGGGACTTCATTCATTGTCCTATTTCTATCTCCACTTTGTGCCTGGAGA
GCTTTCAGGGGAGGTGGAGGAGGAGGGTCTGCCAAGCTACTGCAACATCTGTCACC

CACTATACCCAGTTACTTGGGGGAGGACAGACACTGTGGTGTCATTAAAGTTGTTT
GAACCAAAGTGGCGGCTGCATCTTTGTCCCGATGCTAGCCGTGCCGGTCTCCCATC
ATCCGCTCGCCCTCCTTTNCCCTGGGCTGCGCCCACTTGTCTTCCTGGATATTTGG
GGGTGACTCGCCATGCTTGGCACCCTCTGCTTCCTGGTGCTGCTCTGACTCGAAGA
CGGGACAGTCCCTGGTGCACATCCAGGGAAGAGGAGTGTCGGTAGTTCTTGCAGTA
GGCACTTTATCAGGACCTGACCTGTTGCTGGGTGATTTTAGTCTCTACAAACAGAA
AGCGTTTCAAAGCGTCAGCTGTGGGAGCAGAGTGACCCTTTGCTGATGCTGGGGGG
AGGGGATCTAAATCCTCATTTATCTCT

Sequence ID 914

GGCGCCTGCTGGAGGAGGAGAGAGCTCTGCTGGCATGAGCCACAGTTTCTTGACTG
GAGGCCATCAACCCTCTTGGTTGAGGCCTTGTTCTGAGCCCTGACATGTGCTTGGG
CACTGGTGGGCCTGGGCTTCTGAGGTGGCCTCCTGCCCTGATCAGGGACCCTCCCC
GCTTTCCTGGGCCTCTCAGTTGAACAAAGCAGCAAAACAAAGGCAGTTTTATATGA
AAGATTANAAGCCTGGAATAATCAGGCTTTTTAAATGATGTAATTCCCACTGTAAT
AGCATAGGGATTTTGGAAGCAGCTGCTGGTGGCTTGGGACATCAGTGGGGCCAAGG
GTTCTCTGTCCCTGGTTCAACTGTGATTTGGCTTTCCCGTGTCTTTCCTGGTGATG
CCTTGTTTGGGGTTCTGTGGGTTTGGGTGGGAAGAGGGCCATCTGCCTGAATGTAA
CCTGCTAGCTCTCCGAAGCCCTGCGGGCCTGCTTGTGTGAACCGTGTGGACAGTGG
TGGCCGCGCTGTGCCTGCTCGTGTTGCCTACATGTCCCTGGCTGTTGAGGCGCTGC
TTTAACCTGCACCCCTNCCTTG-CTCATANATGCTCCTTTTGA

Sequence ID - 915          nt:     230

TTTGAGACCAGCCTAGCCAACATGGTGAAACCCCATCTCTACTAAAAATACAAAAA
TTAGCCGGGCGTGGCGGCACATGCCTATAATCCCACTTACTTGGGAGGCTGANGTA
GGAGAATCGCTTGAACCCANANAGGCAGAGTTTGCAGTGAGCCGAGATTGTGCCAT
TGCACTCCAGCCTGGGCGACAGAGCGAGACTCCATCTAAAANAAAATAAATGAATA
AAATAA

Sequence ID 917

NNCAGATTTTTTTTTTTTTTTCAGNGTTAGACCATCTTTCAATTCCTGGAACAAAC
TTAACTTTCCATGATATGTATTTTTTATACATTGCTGGATTTTATTTGCTAATATT
TTACTTAGGATTTAATTTTCTAAGTNGACCTATAATTNTCCTGTATAAAATTGCAT
TTGTCACATTTTAGTATCAAGGTTGTCCTANCNCCATGAAATGGATTTANAATGGT
TTATGTAANATAAAGTACATTTCTTCTAAAGGTTTGNGTGGATTAACTTTCAAATC
TGCCANAGNGNGTTTTTTTCCTTTTTTTTTTTTTTTCATTTNAAGGGAGNGCAAGT
ANCTTTTCAAATNCTGATTTAATTTTTAAAATATTTNCAAGTNTNTTTANAGTTTT


TATTTNTTNTNGAANGTTAACATTTTTATANAAAANGGTNTTATCTTTTTAAATTC
TTTGACATCAGTTTCTTCANAATTCCTTCTTTTAA


Sequence ID 926

GTCATATCTCTTCCCAGGGAAAGCAGGAGCCCTTCTGGAGCCCTTCAGCAGGGTCA
GGGCCCCTCGTCTTCCCCTCCTTTCCCAGAGCCATCTTCCCAGTCCACCATCCCCA
TCGTGGGCATTGTTGCTGGCCTGGCTGTCCTAGCAGTTGTGGTCATCGGAGCTGTG
GTCGCTACTGTGATGTGTAGGAGGAAGAGCTCAGGTAGGGAAGGGGTGAGGGGTGG
GGTCTGGGTTTTCTTGTCCCACTGGGGGGTTTCAAGCCCCAGGTAGAAGTGTTCCCT
GCCTCATTACTGGGAAGCAGCATCCACACAGGGGCTAACGCAGCCTGGGACCCTGT
GTGCCAGCACTTACTCTTTTGTGCAGCACATGTGACAATGAAGGACGGATGTATCA
CCTTGATGGTTGTGGTGTTGGGGTCCTGATTTCAGCATTCATGAGTCAGGGGAAGG
TCCCTGCTAAGGACAGACCTTAGGAGGGCAGTTGGTCCAGGACCCACACTTGCTTT
CCTCGTGTTTCCTGATCCTGCCTTGGGTCTGTAG


Sequence ID 938

TGGCCATCCTTTTCCCCCCAAACACACCCCCTTAACCTATCTCTTGGGACTTAGCC
CGACCCTCCCTCTCATTTCCCATTAAGTCTGAGAGGCAAGAGCTAGGTTAGGCAAG
GAGGTGGTTGGCCAGAGATGGGGAACAGCCAGGTGCCCCAGTCCTCTGATTTTTCC
TCCATCCTGCTTACCACCTCCCTGGGTACTTACAGCCTTCTCTTGGGAACAGCCGG
GGCCAGGACTGGGTCACCTATGAGCTGAATCAGCATCTCCTCCTGAGTCCCAGGGC
CCCTGCAGTTCCCAGTCTCTTCTGTCCTGCAGCCCTTGCCTCTTTCCCACAGGTTC
CACTTTATATCCACCTTTTCCTTTTGTTCAATTTTTATTTTTATTTTTTTTATTAT
TAAATGATGTGGTCTATGGAAAAAAAAATAAAAATCTGACTTAGTTTT

Sequence ID - 939                         nt:      513

```
GGAACCCAGTGTATTACCTGCTGGAACCAAGGAAACTAACAATGTAGGTTACTAGT
GAATACCCCAATGGTTTCTCCAATTATGCCCATGCCACCAAAACAATAAAACAAAA
TTCTCTAACACTGCAAAGAGTGAGCCATGCCTGTTAACACTGTAAAGAATGTAACA
TGTGGGGGACACACAGGGGCAGATGGGATGGTTTAGTTTAGGATTTTATTAGTGCA
TGCCCTACCCTCTGGGGGAACGTCCCATCTGAGGTTTTCTTCTCGGTGGGGGGATT
TAACTTCTGTCCTAGGGAAAACAGTGTCTGATGAGGAGTGTTTCCAACACAGGCTA
CATGAATTCCCCTATACCAGTGCGAAAGCAGCCAGGAGTCCCCGTTGGAAAAGAAC
AATGCCACTCTCTTTTATGTATCTTGGTTCTGCAACTCATTTGTTGTAAGTAGGGT
TAATCGAGTATCAGGTTCACAGTATCCTGCCCTTATTATTTTATGATTCACTGACT
CAAGTTCCA
```

Sequence ID 947

```
GAGAGTGAAAAAATTCTGGTACAAATTGGGAAATTAGTATATAACAACATAGTGTT
AAATTCAATGGGAAAGTTTAATAAGAGGATTTGGTATCAACTGGCTGTCCAAAGA
TAAAAATGGACCGTCCTATCACATACAAAATTGTTTTTTAGATAAAGATTTAAATA
CAGGCACTCCTTCATTTGCGTGGTGCACCTTGAGGTGTTGCAGAAATGATGAGAGC
TGAAACTGCAAAGCAATTTTAATACTTTATCTGTTGGAAATCTTATAGTTTTCCTG
TGACCGTTAAAATTTTCATTAAACTATTAAAAACACCCATGACTGGTCACAAATGT
ATTGGGAAATGGAAAGAATTAATACACTAAAAATACAAAAAATAGAAAATATTTA
AAATTATCTAAAAATTTGAAACATTAGAAAAATTGAGAACTAGGCAGGGCGTGGTG
GCTCACATCTGTAATTTTAGCCCTTTGGGAGGCTGANGCAGGTGGATCACCTGANG
TCAGGAGTTCGAGACCAGCCTGCCAACGTGGGGAAACCCCGTCTCTACTGAAAATA
CAAAAATTANCCGGGCATGGTGGCACAAGCCTGTAATNCTTGCTNACCAGGANGCT
GAGGCAGGAGAATCACTTGAACCCANGANG
```

Sequence ID 949

```
GTTTCACATGAGAAGGTAGTATTATGTACAGTGACCTTGTTTAAAGTGTCNGTTTA
ATGTTACCACTAAGGCCCTGCCCCAGCTTTATCACCTGAGCACTAACAAGTGCTGT
GTGGAGTTCAGTCCATGCTGGTAACTNTTGAGTATTCAGTGGGTCTTTTAACAATT
ACCACCGTGGAGGANANAGCAAGGAAGAGAAATGCTGTGATCTTTTNCTGTTTTTA
ATTAGNGAAAGAGGGATTANATTAAACAAATGTTACAGAGNTGTGACTNTGATCCC
CCAGNGGTAAGCAATAATTGTANAGACTGGATTTNANAAGCCCTGAGAGTTTATTT
TCAACCTATNTATTATAGNNCAATCC
```

Sequence ID 1028

ACAAGGCTTGGGGGCTGGACTCCCTCTACTGCCTCTGGCCATACCCCCTCCTGGAG
ATGGGGTCAAGGCACCAGGACTGA

Sequence ID - 1056                                    nt:        435

TCGCTTGTAAAGCCTGAGACAGCTGCCTGTGTGGGACTGAGATGCAGGATTTCTTC
ACACCTCTCCTTTGTGACTTCAAGAGCCTCTGGCATCTCTTTCTGCAAAGGCATCT
GAATGTGTCTGCGTTCCTGTTAGCATAATGTGAGGAGGTGGAGAGACAGCCCACCC
CCGTGTCCACCGTGACCCCTGTCCCCACACTGACCTGTGTTCCCTCCCCGATCATC
TTTCCTGTTCCAGAGAAGTGGGCTGGATGTCTCCATCTCTGTCTCAACTTCATGGT
GCGCTGAGCTGCAACTTCTTACTTCCCTAATGAAGTTAAGAACCTGAATATAAATT
TGTTTTCTCAAATATTTGCTATGAAGGGTTGATGGATTAATTAAATAAGTCAATTC
CTGGAAGTTGAGAGAGCAAATAAAGACCTGAGAACCTTCCAGA

Sequence ID 1071

NGATATAGTNCCGCATGGGAAAGATGANCAGGTATAACCNAGCNTNATATAGCAAG
GACTAACCCCCCTGCCTTCTGCATAATGAATTAACTAGAAATAACTTNGCAAGGAG
AGCCAAAGCTAAGACCCCNGAAACCAGACGAGCTACCTAAGAACAGNTAAAAGAGC
ACACCCGTCTATGTAGCAAAATAGTGGGAAGATTTATAGGTAGAGGCGACAAACCT
ACCGAGCCTGGTGATAGCTGGTTGTCCAAGATAGAATCTTAGTTCAACTTTAAATT
NGCCCACAGAACCCTCTAAATCCCCTTGTAAATTTAACTGTTAGTCCAAAGAGGAA
CAGCTCTTTGGACACTAGGAAAAAACCTTGTAGAGAGTAAAAAATTTAACACCC
ATAGTAGGCCTAAAAGCAGCCACCAATTAAGAAAGCGTTCAAGCTCAACACCCACT
ACCTAAAAAATCCCAAACATATAACTGAACTCCTNACACCCAATTGGACCAATCTA
TCACCCTATAGAAGAACTAATGTTAGTATAAGTAACATGAAAACATTCTCCTCCGC
ATAAGCCTGCN

Sequence ID - 1074                          nt:      689

GGGAGGCGGAGGCTGCAGTGAGCTGAGATCGTGCCACTTCATTCCAGCCTGGGCAA
CAAAGCGAACTCTGTCTCAAAAAAAAAAAAAAAAAAAAAATTTGTTGACTGTTGTAA
TTTAAAGCTTGTCATTTTTTATTTAGTAATAACACTCATTAGTGTAGTATCTATGA
TGAACCAGGTTCTGCACAAAGTACCTTATGTTCATGGCCTCATATCGTCTTCTCCA
AAACTCTGCAAGATAGGATTCATCACCACTTATAGGGAGAGATCTGAAAGTTTAAA
ATTGTACCCAAGGTCACACAGCTGGTAAGTGCCAGAGCTGGGATTCCGTAGGGTGT
TCANAGTGCCTCTCCTGCCGTAGGCTTATCACAAAAAGTCAAAGTTTGGTCATAAT
AAAGCCTGAAGTTTGGCAGGATTTAAAAATAGTCACCANACTTTTGAGTTGGAGCA
TCCCACCTCACTGCTGTTCACCTTCTGTGGCAGGGAGAGTCATCATTTCCATTTCA
GCTTGTGGAATATCTTGTCATTAACATTCTCATGCAAAAGCCATTTTATGGTGCCC
AATGAANATGGTTAAGCTACTGCCCCAAGCCTNTGGAAGCCTTCCTAATTTTGGAC
TTGCACTATGCAAATTGNATAATATTTTCTCTACCCTAAGCCAAATATTTTCTTCA
CTTTTCATTCATTCTAC


Sequence ID 1081

CGCCGCCGCGCCGCCGTCGCTCTCCAACGCCAGCGCCGCCTCTCGCTCGCCGAGCT
CCAGCCGAAGGAGAAGGGGGGGTAAGTAAGGAGGTCTCTGTACCATGGCTCGTACAA
AGCAGACTGCCCGCAAATCGACCGGTGGTAAAGCACCCAGGAAGCAACTGGCTACA
AAAGCCGCTCGCAAGAGTGCGCCCTCTACTGGAGGGGTGAAGAAACCTCATCGTTA
CAGGCCTGGTACTGTGGCGCTCCGTGAAATTAGACGTTATCAGAAGTCCACTGAAC
TTCTGATTCGCAAACTTCCCTTCCAGCGTCTGGTGCGAGAAATTGCTCAGGACTTT
AAAACAGATCTGCGCTTCCAGAGCGCANCTATCGGTGCTTTGCAGGAGGCAAGTGA
GGCCTATCTGGTTGGCCTTTTTGAAGACACCAACCTGTGTGCTATCCATGCCAAAC


GTGTAACAATTATGCCAAAAGACATCCAGCTAGCACGCCGCATACGTGGAGAACGT
GCTTAAGAATCCACTATGATGGGAAACATTTCATTCTC


Sequence ID - 1083                          nt:      198

GCGCGTCGACTTTGTTTAGACATTGAATGACTTTGTTAAAGGCACAATTAATCACA
TTGGTTGTACTCTGNNGACAGCCTTCTTTAAAAAAAAAAATAAACAATTTAAAACAA
AAAAAAAAAAAAAAAAAAAAAAAAAAAAAAAAAAAAAAAAAAAAAAAAAAAAAAAAA
AAAAAAAAAAAAAAAAAAAANTTTTAACC


236

Sequence ID - 1084                    nt:      198

GCGCGTCGACTTTGTTTAGACATTGAATGACTTTGTTAAAGGCACAATTAATCACA
TTGGTTGTACTCTGNNGACAGCCTTCTTTAAAAAAAAAATAAACAATTTAAAACAA
AAAAAAAAAAAAAAAAAAAAAAAAAAAAAAAAAAAAAAAAAAAAAAAAAAAAAAAA
AAAAAAAAAAAAAAAAAAAANTTTTAACC


Sequence ID - 1099                    nt:      561

TGCATGCTTGTGGATTGGAAAAACTTTGGAGACTGATTACTTTTCATTATATATGT
GTCACAGTGAAACAGCTTTTATGTGTCATGTAAGATTACTGCTTGCCTCTCTAAGG
AAGGTCGTGACTGTTTAAATAGACGGGCAAGGTGGAACCTTTTGAAAGATGAGCTT
TTGAATATAAGTTGTCTGCTAGATCATGGTTTGTATTGAACTAACAAGGTTTGCAG
ATCTGCTGACTTATATAAAGCTTTTTGATTCCTACTAAGCTTTAAGATTTAAAAAA
TGTTCAATGTTGAAATTTCTGTGGGGCTCTATTTTTGCTTTGGCTTTCTGGTGAGA
GAGTGAGGAAGCATTCTTTCCTTCACTAAGTTTGTCTTTCTTGTCTTCTGGATAGA
TTGATTTTAAGAGACTAAGGGAATTTACAAACTAAAGATTTTAGTCATCTGGTGGA
AAAGGAGACTTTAAGATTGTTTAGGGCTGGGCGGGGTGACTCACATCTGTAATCCC
AGCACTTTGGGAGGCCAAGGCAGGCAGAACACTTGAAGGAGTTCAAGACCAGCGTG
G


Sequence ID 1109

TTTGNCGGTNTTGGANNNNNANAANTTTCTTCCANNCNTNACNTNTTGGTGGNCTA
AATTAANATGGNTTTNGNGGGTTCNTTNCTNNNTNNNNCATGGGANANAATTNATT
NTCNTNCNNNTTCCTTNNCCCTNAANCTACCTTCCCCCNATTTTCTCCCCTNTTCN
TNAATTANCATCCTCTCCNCNTANNTCNANACNTTAATGGCAANACTATCTAATAN
CNANNATAANANCTCCTGTNNNCCACATNTCTTATTNNNCGCNNCANGTTNCANNC
CCNCAGAGTNAACTCATCCTCNNCNNAANTTCATATCGTGNNCTNTNNNCNNTNGC
GCGANATATTAANNANACCNGTANNTNNNANACANNANNTNNGNAANAANCCTTCT
NANNTTTTAGCNTCNNGCNNTAACNNNNNTCTTNGTGNNNNCNCAGCTTTCNCNNC


ATNATNCTNCNNCGAANTNTCANNCNTCTCCNCTTNAATGNNTTCCCATGNATTAA
NTNCCTCGNNNANAGCACTATCGTNNNNGAGNNNATTATNGNCNNTTTACNTCATG
TGGTCCANTNNCGTTNGNCGCNNNNAATNTTCGTNNNNCNN

Sequence ID 1118

GGATTTTAGAGGAAGGCGCTNGGTTACATTGGAGAACTGGAGTGGTCTGGAGTTCC
ACGGTGTAGTGGACCAGAGGCCACCTCTCCTGGGCTTCTCAGTGTCTCGCCGGCGG
GGTTCGGCCTGAGCTGGATTGACATAGCCCTTGGCGGATTTAAACAACCTAAACAT
TAAGCAGTACAGCTGCCTCAAACCTTTGGGATTTTCAGAATGACTGACACTGCCGA
AGCTGTTCCAAAGTTTGAAGAGATGTTTGCTAGTAGATTCACAGAAAATGACAAGG
AGTATCAGGAATACCTGAAACGCCCTCCTGAGTCTCCTCCAATTGTTGAGGAATGG
AATAGCANAGCTGGTGGGAACCAAAGAAACAGAGGCAATCGGTTGCAAGACAACAG
ACAGTTCAGAGGCAGGGACAACAGATGGGGGTGGCCAAGTGACAATCGATCCAATC
AGTGGCATGGACGATCCTGGGGTAACAACTACCCGCAACACAGACAAGAACCTTAC
TATCCCCAGCAATATGGACATTATGGTTACAACCAGCGGCCTCCTTACGGTTACTA
CTGATAGAAATGTTGGCAGCTTTTAGTAAAAGCATTTACTCTGTTACCATGAGAAA


Sequence ID 1125

NGACTGGCTCCCGAAAAGAAGGGTGGCGAGAANAAAAAGGGCCGTTCTGCCATGGA
CGAAGTGGTAACCCGCGAATACACCATCAACATTNACAAGCGCATCCATGGAGTGG
GCTTCAAGAANCGTGCACCTCGGGCACTCAAAGAGATTCGGAAATTTGCCATGAAG
GAGATGGGAACTCCATATGTGCGCATTGACACCAGGCTCAACAAANCTGTCTGGGC
CAAAGGAATAAGGAATGTGCCATACCGAATCCGTGTGCGGCTGTCCANAAAACGTA
ATGAGGATGAAGATTCACCAAATAAGCTNTATACTTTGGTTACCTATGTACCTGTT
ACCACTTTCAAAAATCTACAGACAGTCAATGTGGATGANAACNAATCGCTGATCGT
CAGATCAAANAAANT


Sequence ID - 1139                    nt:      503

CAGCACTGCCAGTGGAGATGGGCGTCACTACTGCTACCCTCATTTCACCTGCGCTG
TGGACACTGAGAACATCCGCCGTGTGTTCAACGACTGCCGTGACATCATTCAGCGC
ATGCACCTTCGTCAGTACGAGCTGCTCTAAGAAGGGAACCCCCAAATTTAATTAAA
GCCTTAAGCACAATTAATTAAAAGTGAAACGTAATTGTACAAGCAGTTAATCACCC
ACCATAGGGCATGATTAACAAAGCAACCTTTCCCTTCCCCCGAGTGATTTTGCGAA
ACCCCCTTTTCCCTTCAGCTTGCTTAGATGTTCCAAATTTAGAAAGCTTAAGGCGG
CCTACAGAAAAAGGAAAAAAGGCCACAAAGTTCCCTCTCACTTTCAGTAAAAATA
AATAAAACAGCAGCAGCAAACAAATAAAATGAAATAAAAGAAACAAATGAAATAAA
TATTGTGTTGTGCAGCATTAAAAAAAATCAAAATAAAAATTAAATGTGAGCAAAG

Sequence ID - 1148                  nt:      587

TGAAAAATAAAGTTTTTATGTATATTCTACATATGTATATGTTGGTAGAAAGCAAA
AACGCTAGGTAAAAATAAATGTAATACAATTTTAGCTATGAACCAAAAAACCATTT
GTGGTGTGGATGCAAGAAAGTCTGGATGGGTGCAGAGTTCTCCATGTTTCACTTCT
GACATTTGAAAATACGCAGTTTGCATTTGATACGTCAAATGTTATTTTTAAGAAAA
CCAATAAAATCATTAAAACCGAAAAGGCAGTTTTGCTTGTTTTTACCTTAGTTGGA
GTTATCTGCAATTGCCGTATTAGTGTTTTAAGGAACTTGTAAGTAAGCTCCTTAGT
CCCCTTTAGAGCTACGAAACATGTCAATTTTACTTTTCTCCAGCTTTTTGGAATCT
TATCTAAATTACCATGTAGAGTTCTGCATAGCTTCAAATTCTCTTAGCCAATGTGG
TCTGTAAGTGTCTATCGATGAATTTCACCGTTAATTGCCGTAGTATACTGTCCTGT
ACCGGATGTGAAGAGGAGCAACTCTGCACAGTGCACTGGTTGCTCCCATGGTAGGA
ANGAATGGCTTATCAATGGTCGGATTT

Sequence ID - 1160                  nt:      650

GGAGGATGGAGCAGTGAGCGGGTCTGGGCGGCTGCTGGCAGCGCCATGGAGACGGT
ACAGCTGAGGAACCCGCCGCGCCGGCAGCTGAAAAAGTTGGATGAAGATAGTTTAA
CCAAACAACCAGAAGAAGTATTTGATGTCTTAGAGAAACTTGGAGAAGGGTGAGTG
TAAAGAAACTATAGGTAGGTCATTGGGTCCCAGTCTTTTTCCTGCCCCAGAAGAAG
CAGAAGGATATGAACCTTTCAGCATTGTTCTAGGTGGGGTGGAAGGTAAATTTACA
GCTTGTGATGTCCTTCTTCGCTTTACTCCAATCCCTATTATAGACAGATTTAGTGA
TTCCTGGTCTTTTTAACACGAAGAATATCTATTGTTTTCTCTTTTGTAGGATCTGT
ATGATTTTATCTACTTAACAGATAGCACTAATTAGATTAAAATTCTATAAGAAACT
TTTTAATTTGCTGTTCATAATTTCTGATTGGTATGCAATAACTGTTTCAATGAAAA
TCAATGTAATTTAGTATTTTAATATTTGCACCTTTGTGAAATATAGTAAATAAATT
AAGCACTATCACCACCTTCACAGCTACTTAGGAGATCCACAATCCTGGGTTGGGAG
CCAGTGGATTTCCTGAAACACAGATTTGTTAATG

Sequence ID - 1165                                      nt:        502

CTCAAGTGAATCCTGGCTTCTTGGAAGCGCTTGCCTAGACGAGACACAGTGCATAA
AAACAACTTTTGGGGGACAGGTATGTTTTCTTGCAGCTGCGGTTGTAAGGTCTTGG
CAAGACAAGCAGTGTGGCCAGAATTTTGAACTTCTGATGAATGTGTAATGCAAAGG
ACCTTGTACATTTTTTTTGTTTCAAGGTCCTCAAAATGAGCACATGAAGAGGTTGCT
GTGAAACTTTAAGTGGCCCTACTGCGCAGAAGCATTCAGATGTCACTTGATGATCT
GTAAGGGAACTTGCTGATTTGGGAATGTGCTTAGGGAACACACATTCCTTTTGACA
GGGTCTGTCACTGGGTGGGTGATGAATTATACAGATGACATGTGCTTTTTTTTCTT
TTTTCAACCTCAATGGTATTCCTACAGGAAATGGATAACCATTTTAACTGTATTTT
TTGCAGCCCGTACCTTCTTGGGAATACAATTGTCTAACTTTTTATTTTTGGTCT


Sequence ID - 1172                                      nt:        648

CCACAATAATAAGAGAAAAACAGGAGCAAAAGGATATACAAAACCACCAGAAAACA
AATAACAAAGTGACAGGAGTAAGTCCTTAACTGGCAATAATAACCATGAATCTAAA
TGGATTCCATTTCCCACTTAAAAGATAAAGACATGCTGAATGGATAAAAAGCTGTC
ACCCAGTTATATGCTGCCTACAACAAACTCACTTCACCTGTAAACATACATATGGA
TGGAAAGAGAAGGCATGGGAAAAGATACTCTACTCAAATGAAAACAAAAACCAAAC
AAAGGTGGCTATTCTTATATGAGATAATACAGACATTAAATCAAAAACTGGAAACA
AACACAAAGTCATTGTATAATGATGAATTCAATTATATCATGATGAATTCAATTAT
ATCCTCCTTCCTGATCAATTCAGAAAGGAGGATATAATCTTTTTAAATATATATAC
ACCCAACACCAGAGCATATAAATATGTAAAGGAAGATAAAGGGAGTCCTGTGATCA
AGAATAAATATAACAATTATAAATATTTTATCTAAAGTGATAGATAGACTGTAATA
CAATAATAGGGTGGTGACATTAACACCCCCTCTCACATTGGACTGATCATCTAGAA
GGGAGAAAAAGCTTTATGATTGGAAAAGCCAT

Sequence ID 1178

```
ATTGTGTTGGCCACCCGGGAATTCGCGGCCGCGTCGACCTACGCACACGAGAACAT
GCCTCTCGCAAAGGATCTCCTTCATCCCTCTCCAGAAGAGGAGAAGAGGAAACACA
AGAAGAAACGCCTGGTGCAGAGCCCCAATTCCTACTTCATGGATGTGAAATGCCCA
GGTGAGGAGACGGCTTGCTGTAGTGGGGAAAGCACTGGACCTCAACAGTTGGAAAA
TGTTGTAGTGTTAGCTGTCTCGTATCCTTGAAGCTGTGCAGCAGCTTCAGTTTCTT
CGCCTGTGGAAAATATTTTCCCTGATACTCTTAAAATTTGAATGTATGAGACTGGC
AAAGTTTTGCATCTTAGGAGGAGTGATTCATTTCACCGTGATCTCTCATCACATTT
CACATACAACCCCTACGTTTTTTTGTGTTGGGAAACAATGTAATGGATGATGAGTT
GGGCATAAGTGCAGGAAAGACGGGTGTAATAGAGGAAAAAAATGTTATCTGCTTTT
CTTTCAGGATGCTATAAAATCACCACGGTCTTTAGCCATGCACAAACGGTAGTTTT
GTGTGTTGGCTGCTCCACTGTCCTCTGCCAGCCTACAGGAGGAAAAGCAAGGCTTA
CAGAAGGATGTTCCTTCAGGAGGAAGCAGCACTAAAAGCACTCTGAGTCAANATGA
GTGGGAAACCATCTCAATAAACACATTTTGGAT
```

Sequence ID - 1180                              nt:       622

```
CTTTTCCTCCCGCTGTCCCCCACGGGAGGGGACTGCTCTCCCCCGCTGCATCCTTT
CTGTGAGGTACCTTACCCACCTCAGCACCTGAGAGGGTGAAATAGAATTCTAACCT
CGACATTCGGGAAGTGTTTTTGAGAAGTCTCGGTCGGTAAGGGAAGTCTTCCAAGT
CCGTGCAGCACTAACGTATTGGCACCTGCCTCCTCTTCGGCCACCCCCCAGATGAG
GCAGCTGTGACTGTGTCAAGGGAAGCCACGACTCTGACCATAGTCTTCTCTCAGCT
TCCACTGCCGTCTCCACAGGAAACCCAGAAGTTCTGTGAACAAGTCCATGCTGCCA
TCAAGGCATTTATTGCAGTGTACTATTTGCTTCCAAAGGATCAGGCCCTGAGAACA
```

```
ATGACCTTATTTCCTACAACAGTGTCTGGGTTGCGTGCCAGCAGATGCCTCAGATA
CCAAGAGATAACAAAGCTGCAGCTCTTTTGATGCTGACCAAGAATGTGGATTTTGT
GAAGGATGCACATGAAGAAATGGAGCAGGCTGTGGAAGAATGTGACCCTTACTCTG
GCCTCTTGAATGATACTGAGGAGAACAACTCTGACANCCACAATCATGAGGATGAT
GTGTTG
```

Sequence ID - 1181                              nt:       155

```
CGCCACTTATCCAGTGAACCACTATCACGAAAAAAACTCTACCTCTCTATACTAAT
CTCCCTACAAATCTCCTTAATTATAACATTCACAGCCACAGAACTAATCATATTAA
AAAAAAAAAAAAAAAAAAAAAAAAAAAAAAAAAAAAAAAAAAAAAA
```

Sequence ID 1182

CATTGTGTTGGCNCCCGGGAATTCGCGGCCGCGTCGACTTTTTTGTGTTGTTTGGAG
CAGAAATACTAAAGAAGATTCCGGGCCGAGTATCCACAGAAGTGGACGCAAGGCTC
TCCTTTGATAAAGATGCGATGGTGGCCAGAGCCAGGCGGCTCATCGAGCTCTACAA
GGAAGCTGGGATCAGCAAGGACCGAATTCTTATAAAGCTGTCATCAACCTGGGAAG
GAATTCAGGCTGGAAAGGAGCTCGAGGAGCAGCACGGCATCCACTGCAACATGACG
TTACTCTTCTCCTTCGCCCAGGCTGTGGCCTGTGCCGAGGCGGGTGTGACCCTCAT
CTCCCCATTTGTTGGGCGCATCCTTGATTGGCATGTGGCAAACACCGACAAGAAAT
CCTATGAGCCCCTGGAAGACCCTGGGGTAAAGAGTGTCACTAAAATCTACAACTAC
TACAAGAAGTTTAGCTACAAAACCATTGTCATGGGCGCCTCCTTCCGCAACACGGG
CGAGATCAAAGCACTGGCCGGCTGTGACTTCCTCACCATCTCACCCAAGCTCCTGG
GAGAGCTGCTGCAGGACAACGCCAAGCTGGTGCCTGTGCTCTCAGCCAAGGCGGCC
CAAGCCAGTGACCTGGAAAAAATCCACCTGGATGAGAAGTCTTTCCGTTGGTTGCA
CAACGAGGACCAGATGGCTGTGGAGAAG


Sequence ID - 1183                          nt:      479

CGTGGCAGCCATCTCCTTCTCGGCATCATGGCCGCCCTCAGACCCCTTGTGAAGCC
CAAGATCGTCAAAAAGAGAACCAAGAAGTTCATCCGGCACCAGTCAGACCGATATG
TCAAAATTAAGCGTAACTGGCGGAAACCCAGAGGCATTGACAACAGGGTTCGTAGA
AGATTCAAGGGCCAGATCTTGATGCCCAACATTGGTTATGGAAGCAACAAAAAAAC
AAAGCACATGCTGCCCAGTGGCTTCCGGAAGTTCCTGGTCCACAACGTCAAGGAGC
TGGAAGTGCTGCTGATGTGCAACAAATCTTACTGTGCCGAGATCGCTCACAATGTT
TCCTCCAAGAACCGCAAAGCCATCGTGGAAAGAGCTGCCCAACTGGCCATCAGAGT
CACCAACCCCAATGCCAGGCTGCGCAGTGAAGAAAATGAGTAGGCAGCTCATGTGC
ACGTTTTCTGTTTAAATAAATGTAAAAACTG

Sequence ID - 1185                               nt:        628
CTTTGATTACCTTTGAGTATTAGGTTGAAAGCTTCTCTGTGCTTGATTGAACATTG
TGATGATGTTGATTGGGTCATGTCAGATTTAGACAGTGTTGTGTTTAAGATAAATG
TTTAATGGCTCTTAGCAGTGTTCATGCCTCCCCTTTTCCCCTGATACTTTAAAAAC
AGAATATACAGAAAAGGGGAGTTGGGTGAAGAATCACCATATTCTCATTACCAGAG
TAGTGTCTACCAGCTGTTTTCACATTTTTCTGTTTCCTTCTGTCCTTGGAATCCTT
TTTTTAGATCCTTGTAATACTAGTAAAGATATTCCACTCTGTGTTGTAAGCATTTT
TCCATTTTGCTCCATGGTCTTCATAATGCCCTGTGGTCCTTTATTAAGGGGATGCA
CCATGTAGAGGTGAAAGGCTTTCCTTGACTTGGCCACCATTTCTGTATTTTCCTTA
GAGGAGGAGGTTTCCAACATTTCTTTTTTAGAGACAGAGTCTCGTTCTGACACGCA
GGCAGGAGTGCAGTGGCATGATAACAGCTCACTGCAGCCTCGAACTCCTGGGCTCA
AGTTATCCTCCCACCTCAGCTTCCTGAGTAGCTAGGACTGCAGGTGCCTGCCACCA
CACCCAGCTAAT


Sequence ID - 1186                               nt:        494
CAGCCCTCCGTCACCTCTTCACCGCACCCTCGGACTGCCCCAAGGCCCCCGCCGCC
GCCTCCAGCGCCGCGCAGCCACCGCCGCCGCCGCCGCCTCTCCTTAGTCGCCGCCA
TGACGACCGCGTCCACCTCGCAGGTGCGCCAGAACTACCACCAGGACTCAGAGGCC
GCCATCAACCGCCAGATCAACCTGGAGCTCTACGCCTCCTACGTTTACCTGTCCAT
GTCTTACTACTTTGACCGCGATGATGTGGCTTTGAAGAACTTTGCCAAATACTTTC
TTCACCAATCTCATGAGGAGAGGGGAACATGCTGAGAAACTGATGAAGCTGCAGAA
CCAACGAGGGTGGCCGAATCTTCCTTCAGGATATCAAGAAACCAGACTGTGATGAC
TGGGAGAGCGGGCTGAATGCAATGGAGTGTGCATTACATTTGGAAAAAAATGTGAA
TCAGTCACTACTGGAACTGCACAAACTGGCCACTGACAAAAATGAC

Sequence ID - 1188                    nt:      599

GGGAGACAAGCCCAGCCTTTCGGCGAGNATACGTCTAACCCTGTGCAACAGCCACT
ACATTACTTCAAACTGAGATCCTTCCTTTTGAGGGAGCAAGTCCTTCCCTTTCATT
TTTTCCAGTCTTCCTCCCTGTGTATTCATTCTCATGATTATTATTTTAGTGGGGGC
GGGGTGGGAAAGATTACTTTTTCTTTATGTGTTTGACGGGAAACAAAACTAGGTAA
AATCTACAGTACACCACAAGGGTCACAATACTGTTGTGCGCACATCGCGGTAGGGC
GTGGAAAGGGGCAGGCCANAGCTACCCGCAGAGTTCTCAGAATCATGCTGAGAGAG
CTGGAGGCACCCATGCCATCTCAACCTCTTCCCCGCCCGTTTTACAAAGGGGGAGG
CTAAAGCCCAGAGACAGCTTGATCAAAGGCACACAGCAAGTCAGGGTTGGAGCAGT
AGCTGGAGGGACCTTGTCTCCCAGCTCAGGGCTCTTTCCTCCACACCATTCAGGTC
TTTCTTTCCGAGGCCCCTGTCTCAGGGTGAGGTGCTTGAGTCTCCAACGGCAAGGG
AACAAGTACTTCTTGATACCTGGGATACTGTGCCCAGAG


Sequence ID 1189

GGGAGACAAGCCCAGCCTTTCGGCGAGATACGTCTAACCCTGTGCAACAGCCACTA
CATTACTTCAAACTGAGATCCTTCCTTTTGAGGGAGCAAGTCCTTCCCTTTCATTT
TTTCCAGTCTTCCTCCCTGTGTATTCATTCTCATGATTATTATTTTAGTGGGGGCG
GGGTGGGAAAGATTACTTTTTCTTTATGTGTTTGACGGGAAACAAAACTAGGTAAA
ATCTACAGTACACCACAAGGGTCACAATACTGTTGTGCGCACATCGCGGTAGGGCG
TGGAAAGGGGCAGGCCAGAGCTACCCGCAGAGTTCTCAGAATCATGCTGAGAGAGC
TGGAGGCACCCATGCCATCTCAACCTCTTCCCCGCCCGTTTTACAAAGGGGGAGGC
TAAAGCCCAGAGACAGCTTGATCAAAGGCACACAGCAAGTCAGGGTTGGAGCAGTA
GCTGGAGGGACCTTGTCTCCCAGCTCAGGGCTCTTTCCTCCACACCATTCAGGTCT
TTCTTTCCGAGGCCCCTGTCTCAGGGTGAGGTGCTTGAGTCTCCAACGGCAAGGGA
ACAAGTACTTCTTGATACCTGGGATACTGTGCCCAGAGCCTCGAGGAGGT

Sequence ID 1190

GTTTAAATTTGACAAACTAAAGCTNATNACTGCTATAAGAGTAATAACTGCTCATT
TTCCATAACTCATTCTTAAAGTTTTAGTAATGTAAAAGTTATTTTTTTGCAGTAAG
TTATAATGATAGAAGCTTACATGTTTTTTCATGCCTCATCTGTTTCCCCTTAAAAC
TATAATTATCAGTAAAGTCCTGTGGTATTTTTCAATTTGTAAGAAACTAGGCTATA
TATACATTGGGAAAAACAGCCTTCATTTGTCAATGCACTAGTGTTCCAAAGGTTTC
TGGTAATTGTGTGCTATTGCTTTTTGTTGACTTGCAAAAAAAAAAAAAAAAAAATT
ACTATGACTTGNGGTAGCCCTGCAACCTTCGGAAGTGCTTAGCCCAGTCTGACCAT
ACATTTATATTTANAATGCTTAGGTAAATAAATAATATGCCTAAACCCAATGCTAT
AAGATACTATATAATATCTCATAATTTTAAAAATCACTGTTTTGTATAATAATAAA
ACAAGGCAGGCAAGCTGTTCTACAATGACTGTTGGTAAGGGTGCTGAGGAAGAAAA
ACAAACAATCTTGATTCAGGGATAGTGAATAGACAAAAAATGTCCTAATCAATGAA
GCTGTGTGATGATTCTGATTGACAGAGA

Sequence ID 1191

GTGCAAAGTGTTATATCCACTTTCAACAAAGAGAGAAGCTGAAAAGCTAACCCAAT
GTTAATTTTGGATCACACACATTCAGTGTAGACTTTAAGATTTTACTTCTGTTGGA
GTAGCTATATTATTTCTAGTTAAAAAACTCTCTATATACATATTTATTTGTTTTTC
TACTTGTTTAATATTTTTCTCTTCCAATTAGGAACTCAATATGGAATAAAAAATAT
TTAAATGTATTTTACTCAAACGTGTGTGTATATATGTTTGTGTGCATGATAAGGAG
AGTGAGAGCAAGAGTAAGAGAGAGAGAGCACGCATAGATGGAAGCACACATTTAAT
GTCTATGAAATGAGAAACATTAAGGCTAAGATATTTTTCCTTCTGAACTAGCAGA
TTGTATCAATGGCTGGTCACTTAAATTAATCAGTTTGTAAAGATATTTAAAAGGTA·
TGTCTACCTTCTTGCAATTAATTTGATTATGTTCTAATGGCATGGCAAGAGAAATG

AAAGAAGATAACTAAAAGTTAAAAGTCGTTGCATGTTTTTGTTGCAGCATACCCTT
CTTTCAGGCTACCGAATAACCTTGATTGACATTGGATTAGTAGTAGAATACCTCAT
TGGTAGAGCATATCGCAGCANCTACACTAGAAAACAT

Sequence ID 1192

GTCTGGAACTCCAGACCTCAGGTGATACCCCTGCCTCAGCCTCCCAATGTGCTGGG
ATTACAGCTGTGAAGCCACCGCGCCCGGCTGCTGTGATAGTTGAGATGTAAACCAA
AAATAAAATTCTAAGCCACCCAATCCGACTGAATGGACCCTTCCTGTTGAGCAAGG
ACATTCCAAAGTAAACTGAAAAGACCAGCTTAGGCCATGATGGGAAGGGGAGGTGT
CAACATGCCTCATTCTACCTTCCTCCCTCTGGAATCCAGACACAACTGACCAGCAT
TAACATTAAAACAGAGATCTTAAGCTGGGCACGGTGGCTCATGCCTGTAATCCCAG
CACTTTGGGAGGCCAAGGTGGGATCACCTGAGGTCGGAAGTTCAAGACCAGCCTGG
CCGGTATGGTGAAGCCATGTCTCTACTGAAAATGCAAAATTGGCCGGACATTGTGG
TGCA

Sequence ID 1193

TNCNTTTTTTTTCCCNCGGGAAAGCGCGCCATTGTGTTGGTCCCCGGGAATTCGCG
GCCGCGTCGACGAGAAATGGCTTGAACCCAGTAGGCAGAGGTTGTAGTGAGCCCAG
AATNGGNCACCTGCACNTTTANCCNTGGGTGACAAAANTGAAAACTTTGTCTNAAA
AAAAAAAAAAAAAATTTTAANTNAAATNAAAAANCCTTTNCNTTNTTTTTNAAAN
NGGGGGGGGNNTTTTTNGGGGNTTNGNNNTGGTAAAAANTNNNTTTTTTTTTTTTTA
GGGGCCNANNCCCCNTTTTTANAAAANCCNGNTTTTTNAAAAAANTTTTTTTNCCCNCN
NTTNGGGGGGGGGGNTTTTTNANCNNTNTTNGGGGGGGGNNCCCCTNTTANNACCNNC
AAANTTTTTANTTTTTTTGNNNAANNNCCCCCTTTTTTTNNTTTTTTTTTGNGGGGGGG
GGGNNGCCCCCNNCCTTTNGGGGGGGGGGGNTTNNGNAAAANNACTTTTNAAAANNA
AGGGNNGGGGGNANATNNCCCCCCCNGGNTTTTTTTTTTTAAAAANTNAANNGGGGG
GGGNNNCTNANTNGGGGGCNCCCANNGGGGGGNTTANAANNATTTTCTNCCCAAACCC
CCNGNTTTTATNNCCCCCCCCCCCCNCNNNNGAANGGGNGGNCCNTTTTTTTTTATT
TTTNNGGNGGGNAAAAAANTTTNAAAAANNANNATNTTTTTTCCCCCCCCCCCCCNC
TTTTNGGNAAANCCNNGGGGGGGNTCCTTTTTNAAANNNNCCCCCAAAAAAAAANTTT
TTTTNTTNTNTTTTTCTCTNGGGGNCCNNANTTNTANANTTTTNCNCCNAAAAAAAA
ANGGGNCCCCTTTTTTTTNCNGGNNGGNNCCCAAAANNTTTTTTTTTNAAAAAAAAAA
AAAA

Sequence ID 1195

GTTCGTGACNTTCGGAGCTACCTGACAGAGCAGAGTCAACCAGGNTCTGCCCAAAG
AGAGTGTTAGGCCTGAGCTTGAGAGCCCTGGAGAGACGTGTGCACAAAATGTGACC

TGAGGCCCTAGTCTAGCAAGAGGACATAGCACCCTCATCTGGGAATAGGGAAGGCA
CCTTGCAGAAAATATGAGCAATTTGATATTAACTAACATCTTCAATGTGCCATAGA
CCTTCCCACAAAGACTGTCCAATAATAAGAGATGCTTATCTATTTTA

Sequence ID - 1196                              nt:      412
GTCGACGCGGCCGCGGTCGCTGGAGNCGATCAACTCTAGGCTCCAACTCGTTATGA
AAAGTGGGAAGTACGTCCTGGGGTACAAGCAGACTCTGAAGATGATCAGACAAGGC
AAAGCGAAATTGGTCATTCTCGCTAACAACTGCCCAGCTTTGAGGAAATCTGAAAT
AGAGTACTATGCTATGTTGGCTAAAACTGGTGTCCATCACTACAGTGGCAATAATA
TTGAACTGGGCACAGCATGCGGAAAATACTACAGAGTGTGCACACTGGCTATCATT
GATCCAGGTGACTCTGACATCATTAGAAGCATGCCAGAACAGACTGGTGAAAAGTA
AACCTTTTCACCTACAAAATTTCACCTGCAAACCTTAAACCTGCAAAATTTTCCTT
TAATAAAATTTGCTTGTTTT

Sequence ID 1197
CCGCCAACATGGGCCGCGTTCGCACCAAAACCGTGAAGAAGGCGGCCCGGGTCATC
ATAGAAAAGTACTACACGCGCCTGGGCAACGACTTCCACACGAACAAGCGCGTGTG
CGAGGAGATCGCCATTATCCCCAGCAAAAAGCTCCGCAACAAGATAGCAGGTTATG
TCACGCATCTGATGAAGCGAATTCAGAGAGGCCCAGTAAGAGGTATCTCCATCAAG
CTGCAGGAGGAGGAGAGAGAAAGGAGAGACAATTATGTTCCTGAGGTCTCAGCCTT
GGATCAGGAGATTATTGAAGTAGATCCTGACACTAAGGAAATGCTGAAGCTTTTGG
ACTTCGGCAGTCTGTCCAACCTTCAGGTCACTCAGCCTACAGTTGGGATGAATTTC
AAAACGCCTCGGGGACCTGTTTGAATTTTTTCTGTAGTGCTGTATTATTTTCAATA
AATCTGGGACAA

Sequence ID 1198
CAGAGGTGGGAGGATTGCTTCAGTTCAAGAGTTTGAGACCAGCCTGGGTAACATGG
CGAAACCCTGTCTTTACAAAAAATGCAAACCTTTGCCGCATGTGTTGGGGTGCGCC
TGTAGTCCCAGCTTCTCGGGAGGCTGAGGTGGGGGGACCACCTGAGCCATGGAGGT
TGAGGCTGCAGTGAGCCGTGATACCACCACTGTACTCTAGCCTGGGCCATAGAGTG
AGACACCCTGCCTCAGAAATA

Sequence ID - 1199                                  nt:       439
CCCATCCCCTCGACCGCTCGCGTCGCATTTGGCCGCCTCCCTACCGCTCCAAGCCC
AGCCCTCAGCCATGGCATGCCCCCTGGATCAGGCCATTGGCCTCCTCGTGGCCATC
TTCCACAAGTACTCCGGCAGGGAGGGTGACAAGCACACCCTGAGCAAGAAGGAGCT
GAAGGAGCTGATCCAGAAGGAGCTCACCATTGGCTCGAAGCTGCAGGATGCTGAAA

TTGCAAGGCTGATGGAAGACTTGGACCGGAACAAGGACCAGGAGGTGAACTTCCAG
GAGTATGTCACCTTCCTGGGGGCCTTGGCTTTGATCTACAATGAAGCCCTCAAGGG
CTGAAAATAAATAGGGAAGATGGAGACACCCTCTGGGGGTCCTCTCTGAGTCAAAT
CCAGTGGTGGGTAATTGTACAATAAATTTTTTTTTGGTCAAATTTAA


Sequence ID - 1200                                  nt:       526
CTGGAGACGACGTGCAGAAATGGCACCTCGAAAGGGGAAGGAAAAGAAGGAAGAAC
AGGTCATCAGCCTCGGACCTCAGGTGGCTGAAGGAGAGAATGTATTTGGTGTCTGC
CATATCTTTGCATCCTTCAATGACACTTTTGTCCATGTCACTGATCTTTCTGGCAA
GGAAACCATCTGCCGTGTGACTGGTGGGATGAAGGTAAAGGCAGACCGAGATGAAT
CCTCACCATATGCTGCTATGTTGGCTGCCCAGGATGTGGCCCAGAGGTGCAAGGAG
CTGGGTATCACCGCCCTACACATCAAACTCCGGGCCACAGGAGGAAATAGGACCAA
GACCCCTGGACCTGGGGCCCAGTCGGCCCTCANAGCCCTTGCCCGCTCGGGTATGA
AGATCGGGCGGATTGAGGATGTCACCCCCATCCCCTCTGACAGCACTCGCAGGAAG
GGGGGTCGCCGTGGTCGCCGTCTGTGAACAAGATTCCTCAAAATATTTTCTGTTAA
TAAATTGCCTTCATGTAAACTG

Sequence ID - 1201          nt:       613

CTTAAGTATGCCCTGACAGGAGNATGAAGTAAAGAAGATTTGCATGCAGCGGTTCA
TTAAAATCGATGGCAAGGTCCGAACTGATATAACCTACCCTGCTGGATTCATGGAT
GTCATCAGCATTGACAAGACGGGAGAGAATTTCCGTCTGATCTATGACACCAAGGG
TCGCTTTGCTGTACATCGTATTACACCTGAGGAGGCCAAGTACAAGTTGTGCAAAG
TGAGAAAGATCTTTGTGGGCACAAAAGGAATCCCTCATCTGGTGACTCATGATGCC
CGCACCATCCGCTACCCCGATCCCCTCATCAAGGTGAATGATACCATTCAGATTGA
TTTAGAGACTGGCAAGATTACTGATTTCATCAAGTTCGACACTGGTAACCTGTGTA
TGGTGACTGGAGGTGCTAACCTAGGAAGAATTGGTGTGATCACCAACAGAGAGAGG
CACCCTGGATCTTTTGACGTGGTTCACGTGAAAGATGCCAATGGCAACAGCTTTGC
CACTCGACTTTCCAACATTTTTGTTATTGGCAAGGGCAACAAACCATGGATTTCTC
TTCCCCGAGGAAAGGGTATCCGCCTCACCATTGCTGAAGAGAGAGACAAAAGA


Sequence ID 1202

GGAATTCGCGGCCGCGTCGACCTCTGCTCGAATTGACAGAAAAGGATTCTGTGAAG
AGTGATGAGATTTCCATCCATGCTGACTTTGAGAATACATGTTCCCGAATTGTGGT
CCCCAAAGCTGCCATTGTGGCCCGCCACACTTACCTTGCCAATGGCCAGACCAAGG
TGCTGACTCAGAAGTTGTCATCAGTCAGAGGCAATCATATTATCTCAGGGACATGC
GCATCATGGCGTGGCAAGAGCCTTCGGGTTCAGAAGATCAGGCCTTCTATCCTGGG
CTGCAACATCCTTCGAGTTGAATATTCCTTACTGATCTATGTTAGCGTTCCTGGAT


CCAAGAAGGTCATCCTTGACCTGCCCCTGGTAATTGGCAGCAGATCAGGTCTAAGC
AGCAGAACATCCAGCATGGCCAGCCGAACCAGCTCTGAGATGAGTTGGGTAGATCT
GAACATCCCTGATACCCCAGAAGCTCCTCCCTGCTATATGGATGTCATTCCTGAAG
ATCACCGATTGGAGAGCCCAACCACTCCTCTGCTAGATGACATGGATGGCTCTCAA
GACAGCCCTATCTTTATGTATGCCCCTGAGTTCAAGTTCATGCCACCACCGACTTA
TACTGAGGTGGATCCCTGCATCCTCAACAACAATGTGCAGTGAGCAT

Sequence ID - 1203                          nt:        692
TGCAGAGGGGTCCATACGGCGTTGTTCTGGATTCCCGTCGTAACTTAAAGGGAAAC
TTTCACAATGTCCGGAGCCCTTGATGTCCTGCAAATGAAGGAGGAGGATGTCCTTA
AGTTCCTTGCAGCAGGAACCCACTTAGGTGGCACCAATCTTGACTTCCAGATGGAA
CAGTACATCTATAAAAGGAAAAGTGATGGCATCTATATCATAAATCTCAAGAGGAC
CTGGGAGAAGCTTCTGCTGGCAGCTCGTGCAATTGTTGCCATTGAAAACCCTGCTG
ATGTCAGTGTTATATCCTCCAGGAATACTGGCCAGAGGGCTGTGCTGAAGTTTGCT
GCTGCCACTGGAGCCACTCCAATTGCTGGCCGCTTCACTCCTGGAACCTTCACTAA
CCAGATCCAGGCAGCCTTCCGGGAGCCACGGCTTCTTGTGGTTACTGACCCCAGGG
CTGACCACCAGCCTCTCACGGAGGCATCTTATGTTAACCTACCTACCATTGCGCTG
TGTAACACAGATTCTCCTCTGCGCTATGTGGACATTGCCATCCCATGCAACAACAA
GGGAGCTCACTCAGTGGGTTTAATGTGGTGGATGCTGGCTCGGGAAGTTCTGCGCA
TGCGTGGCACCATTTCCCGTGAACACCCATGGGAGGTCATGCCTGATCTGTACTTC
TACAGAGATCCTGAAGAGAT


Sequence ID 1204
TTTTTTTTTTTTTTCCTGCGGGAAAGCGCGCCATTGTGTTGGTACCCGGGAAATTCG
CGGCCGCGTCGACACAGGCCCCAGCATCAAGATCTGGGATTTAGAGAGGAAAGATC
ATTGTAGATGAACTGAAGCAAGAAGTTATCAGTACCAGCAGCAAGGCAGAACCACC
CCAGTGCACCTCCCTGGCCTGGTCTGCTGATGACACAGGTTGGGCNGGNNCNCNGG
GGNGGNNNNGNNNNGCNGNNGGNNCNGNNNNCNNNNNGCNNNNGNNNNTNNNCNNN
GNNCNNNNNNNNNNNNNNNNNNNGNTCNNGNNGCNGGGGCCNGGNCGNCGCGGNCGCG
NNTNNNNGGGTNCNNNCNCNNNGGCGCGC


Sequence ID 1205
CAGACTCTGACCCAGCCTCAGTCCTAACTCCTGGGGCTGGGCTGAGGGGAACAAGC
ATTTGCTGAAACTTGAAAAAACAAAGCAAATCAAAAACAGGAAAAAATTGTACCTG
GTACTTTTTTTTAGAAAAAAAGATTAAAAAAGAAAGAATAAATTCTTGTTTGGAAA
CTTGAAAAAAAAAAAAAAAAAAAAAAAAAAAAAAAAAAAAAAAAAAAAAAAAAAAAA
AAAAAATTTTAAACTC


TNNNNNTNNCNNCNANTAANNCANNTCNANNNNANNNAATTACTTNNANGTNNNTC
ACN

Sequence ID - 1207                         nt:        642

ACGAGAAGCCAGATACTAAAGAGAAGAANCCCGAAGCCAAGAAGGTTGATGCTGGT
GGCAAGGTGAAAAAGGGTAACCTCAAAGCTAAAAAGCCCAAGAAGGGGAAGCCCCA
TTGCAGCCGCAACCCTGTCCTTGTCAGAGGAATTGGCAGGTATTCCCGATCTGCCA
TGTATTCCANAAAGGCCATGTACAAGAGGAAGTACTCAGCCGCTAAATCCAAGGTT
GAAAAGAAAAAGAAGGAGAAGGTTCTCGCAACTGTTACAAAACCAGTTGGTGGTGA
CAAGAACGGCGGTACCCGGGTGGTTAAACTTCGCAAAATGCCTAGATATTATCCTA
CTGAAGATGTGCCTCGAAAGCTGTTGAGCCACGGCAAAAAACCCTTCAGTCAGCAC
GTGAGAAAACTGCGAGCCAGCATTACCCCCGGGACCATTCTGATCATCCTCACTGG
ACGCCACAGGGGCAAGAGGGTGGTTTTCCTGAAGCAGCTGGCTAGTGGCTTATTAC
TTGTGACTGGACCTCTGGTCCTCAATCGAGTTCCTCTACGAAGAACACACCAGAAA
TTTGTCATTGCCACTTCAACCAAAATCGATATCAGCAATGTAAAAATCCCAAAACA
TCTTACTGATGCTTACTTCAAAAAGA


Sequence ID 1208

CCCTATACCTTCTGCATAATGAATTANCTAGAAATAACTTTGCAAGGGAGAGCCAA
AGCTAAGACCCCCGAAACCAGACGAGCTACCTAAGAACAGCTAAAAGAGCACACCC
GTCTATGTAGCAAAATAGTGGGAAGATTTATAGGTAGAGGCGACAAACCTACCGAG
CCTGGTGATAGCTGGTTGTCCAAGATAGAATCTTAGTTCAACTTTAAATTTGCCCA
CAGAACCCTCTAAATCCCCTTGTAAATTTAACTGTTAGTCCAAAGAGGAACAGCTC
TTTGGACACTAGGAAAAAACCTTGTAGAGAGAGTAAAAAATTTAACACCCATAGTA
GGCCTAAAAGCAGCCACCAATTAAGAAAGCGTTCAAGCTCAACACCCACTACCTAA
AAAATCCCAAACATATAACTGAACTCCTCACACCCAATTGGACCAATCTATCACCC
TATAGAAGAACTAATGTTAGTATAAGTAACATGAAAACATTCTCCTCCGCATAAG


Sequence ID - 1209                         nt:        620

CTCTCCTGTCAACAGCGGCCAGCCTCCCAACTACGAGAATGCTCAAGGAGGAGCAG
GAAGTGGCTATGCTGGGGGCGCCCCACAACCCTGCTCCCCCGACGTCCACCGTGAT
CCACATCCGCAGCGAGACCTCCGTGCCCGACCATGTCGTCTGGTCCCTGTTCAACA
CCCTCTTCATGAACACCTGCTGCCTGGGCTTCATAGCATTCGCCTACTCCGTGAAG
TCTAGGGACAGGAAGATGGTTGGCGACGTGACCGGGGCCCAGGCCTATGCCTCCAC
CGCCAAGTGCCTGAACATCTGGGCCCTGATTTTGGGCATCTTCATGACCATTCTGC
TCGTCATCATCCCAGTGTTGGTCGTCCAGGCCCAGCGATAGATCAGGAGGCATCAT
TGAGGCCAGGAGCTCTGCCCGTGACCTGTATCCCACGTACTCTATCTTCCATTCCT

CGCCCTGCCCCCAGAGGCCAGGAGCTCTGCCCTTGACCTGTATTCCACTTACTCCA
CCTTCCATTCCTCGCCCTGTCCCCACAGCCGAGTCCTGCATCAGCCCTTTATCCTC
ACACGCTTTTCTACAATGGCATTCAATAAAGTGTATATGTTTCTGGTGCTGCTGTG
ACTT


Sequence ID 1210
TTCGTAATTAGAATACTGTTTGGACTTGCTCAACAAGCACCTTATCTTAACAAAAA
GTAACTTATAGAAAAGGGAGACATTCATTTAACTTCAAGCCCATATTATTCTTAAA
AGCTGACTCTTGAAATAGTATTTATTGAGTCATAGTGGAGTCATGGGACTTTTTAA
GGGCCGGAAGGGACTATTTAGATCATCCAGTCCCACCCTGTCATTTTATGGAGGAG
GAAACTGAGGCCTAGATAAGATAACCAGTTAGTGGGTCCACTGACCTTTAGGACAG
TAGTCTATCCGTAAGAGACAACATGGAGAAAGAAATACAACGTTTTTATAGTGAAT
TATCATCTTACAAAGAATATTCTTCCCATATCGCACTTTTAAAAAGTGGGTACCTT
AGTCAAATAGGAGAAAAACCACTTGAGTAGTTTCATCCTCAGGTTTTAGGTGAGG
AAACTGATACTCAGATTAAATAACTTTAAGCACACAGAGCCTGAATGATAGTCTTA
TTTGAGCTCATCTGTGCTTTTAATGTGTACTACGTTAGGTGTTTTCACTTGCATTT
CCTTTAGTCTTATTTGAGCTCATCTGTGCTTTTAATGTGTACTACGTTAGGTGTTT
TCACTTGCATTTCCTTGTTTGACGTTGACAATAAATCGTGAAGCTGCCTTATCTAA
GGAAGTCCTAAAGTAAATCATTGGAACACA


Sequence ID 1211
CCATTGTGTTGGNACCCGGGAATTCGCGGCCGCGTCGACGGAGTTTTACCTTATTA
CACTTTAATCTCTGGATTTACCCCATCTCATTTCTCTTTTAGGAAAACTGTTTGTA
TGTGGTGGCTTTGATGGTTCTCATGCCATCAGTTGTGTGGAAATGTATGATCCAAC
TAGAAATGAATGGAAGATGATGGGAAATATGACTTCACCAAGGAGCAATGCTGGGA
TTGCAACTGTAGGGAACACCATTTATGCAGTGGGAGGATTCGATGGCAATGAATTT
CTGAATACGGTGGAAGTCTATAACCTTGAGTCAAATGAATGGAGCCCCTATACAAA
GATTTTCCAGTTTTAACAAATTTAAGACCCTCTCAAACTAACAGGCTTAGTGATGT
AATTATGGTTAGCAGAGGTACACTTGTGAATAAAGAGGGTGGGTGGGTATAGATGT
TGCTAACAGCAACACAAAGCTTTTGCATATTGCATACTATTAAACATGCTGTACAT
ACTTTTTGGGTTTATTTGGAAAGGAATGCAAAGATGAAGGTCTGTTTTGTGTACTT
TTAAGACTTTGGTTATTTTACTTTTTTGGAAAAGAATAAACCAAGAATTGATTGGGC
ACATCATTTCAAGAAG

Sequence ID - 1212                          nt:      374

AGAGCAGCAGCCATGGCCCTACGCTACCCTATGGCCGTGGGCCTCAACAAGGGCCA

CAAAGTGACCAAGAACGTGAGCAAGCCCAGGCACAGCCGACGCCGCGGGCGTCTGA
CCAAACACACCAAGTTCGTGCGGGACATGATTCGGGAGGTGTGTGGCTTTGCCCCG
TACGAGCGGCGCGCCATGGAGTTACTGAAGGTCTCCAAGGACAAACGGGCCCTCAA
ATTTATCAAGAAAAGGGTGGGGACGCACATCCGCGCCAAGAGGAAGCGGGAGGAGC
TGAGCAACGTACTGGCCGCCATGAGGAAAGCTGCTGCCAAGAAAGACTGAGCCCCT
CCCCTGCCCTCTCCCTGAAATAAAGAACAGCTTGACAG

Sequence ID - 1213                          nt:      567

GAATTATTGACTTTGAATTGCATTTCAGTACCATGAAGTCAAAGTCAGTGGTGTAT
TTGCTCATTTGTTCATTCTTTCTTTTCCACCAACATTACTGCCTGCAGAGCCAGAG
GTGAGTGCAGAAATCCTGTCAATTCGTCACTTGTGGACAACCTGCAGCTTGCCACA
GCCTACAGTTCCACCACTGTGACCTCTGAAAACCTCCTGAACAAAAGGAAGGAGAC
TTGGAAATCCTGAATGGGCTTGGAGACATTAAGGGAGAACTGCCTCCCTGGACCAA
GGCAGAATTCAATAGAACCAGCAAGAAATTTTCCTATGAATGGGAAAGCAGGTGGC
AGGGGGCAGGGGTGGAAAAGCTTTGTACAGGAATTGTGGAAAAGCTTTTGCATTAT
CTCTAGTCTGAAAGTCACATTTCTCAGTTCCTTTCCACTCTCTTCTGTCAACTTGC
TGTGAGTAAATGACATCTGTCACCTGTGACACGGGCCAGGGACTATCACCATATGG
CCCCCACACATTATCTAGTACCAGCCTGCCTGGGCCATGCCTTTTCCAGTCACTGT
ACCAGCC

Sequence ID - 1214                    nt:       620
CTCTCCTGTCAACAGCGGCCAGCCTCCCAACTACGAGAATGCTCAAGGAGGAGCAG
GAAGTGGCTATGCTGGGGGCGCCCCACAACCCTGCTCCCCCGACGTCCACCGTGAT
CCACATCCGCAGCGAGACCTCCGTGCCCGACCATGTCGTCTGGTCCCTGTTCAACA
CCCTCTTCATGAACACCTGCTGCCTGGGCTTCATAGCATTCGCCTACTCCGTGAAG
TCTAGGGACAGGAAGATGGTTGGCGACGTGACCGGGGCCCAGGCCTATGCCTCCAC
CGCCAAGTGCCTGAACATCTGGGCCCTGATTTTGGGCATCTTCATGACCATTCTGC
TCGTCATCATCCCAGTGTTGGTCGTCCAGGCCCAGCGATAGATCAGGAGGCATCAT
TGAGGCCAGGAGCTCTGCCCGTGACCTGTATCCCACGTACTCTATCTTCCATTCCT
CGCCCTGCCCCCAGAGGCCAGGAGCTCTGCCCTTGACCTGTATTCCACTTACTCCA
CCTTCCATTCCTCGCCCTGTCCCCACAGCCGAGTCCTGCATCAGCCCTTTATCCTC
ACACGCTTTTCTACAATGGCATTCAATAAAGTGTATATGTTTCTGGTGCTGCTGTG
ACTT


Sequence ID 1215
CACAAGATAGAATGGTAAAAAAAAAAAAAAAAAAAAAAAAAAAAAAAAAAATTTTAA
GTGACAGTGCCATAGTTTGGACAGTACCTTTCAATGATTAATTTTAATAGCCTGTG


AGTCCAAGTAAATGATCACTTTATTTGCTAGGGAGGGAAGTCCTAGGGTGGTTTCA
GTTTCTCCCAGACATACCTAAATTTTTACATCAATCCTTTTAAAGAAAATCTGTAT
TTCAAAGAATCTTTCTCTGCAGTAAATCTCGCAGGGGAATTTGCACTATTACACTT
GAAAGTTGTTATTGTTAACCTTTTCGGCAGCTTTTAATAGGAAAGTTAAACGTTTT
AAACATGGTAGTACTGGAAATTTTACAAGACTTTTACCTAGCACTTAAATATGTAT
AAATGTACATAAAGACAAACTAGTAAGCATGACCTGGGGAAATGGTCAGACCTTGT
ATTGTGTTTTTGGCCTTGAAAGTAGCAAGTGACCAGAATCTGCCATGGCAACAGGC
TTTAAAAAAGACCCTTAAAAAGACACTGTCTCAACTGTGGTGTTAGCACCAGCCAG
CTCTCTGTACATTTGCTAGCTTGTAGTTTTCTAAGACTGAGTAAACTTCTTATTTT
TAGAAAGTGGAGGTCTGGTTTGTAACTTTCCTTGTACTTAATTGGGTAAAAGT

Sequence ID - 1216                    nt:        484

CAACCTTAGCCAAACCATTTACCCAAATAAAGTATAGGCGATAGAAATTGAAACCT
GGCGCAATAGATATAGTACCGCAAGGGAAAGATGAAAAATTATAACCAAGCATAAT
ATAGCAAGGACTAACCCCTATACCTTCTGCATAATGAATTAACTAGAAATAACTTT
GCAAGGAGAGCCAAAGCTAAGACCCCCGAAACCAGACGAGCTACCTAAGAACAGCT
AAAAGAGCACACCCGTCTATGTAGCAAAATAGTGGGAAGATTTATAGGTAGAGGCG
ACAAACCTACCGAGCCTGGTGATAGCTGGTTGTCCAAGATAGAATCTTAGTTCAAC
TTTAAATTTGCCCACAGAACCCTCTAAATCCCCTTGTAAATTTAACTGTTAGTCCA
AAGAGGAACAGCTCTTTGGACACTAGGAAAAAACCTTGTAGAGAGAGTAAAAAATT
TAACACCCATAGTAGGCCTAAAAGCAGCCACCAATT


Sequence ID 1217

GACAGGCGGGGGCCCAGCGGCCGGGTGAAGGCCGGGTGGCTCTGTGAATCAAAGGA
GAGTCCCAGAAAACCTGTGACTGTTGAAGAAAATTCATCTGTGAATTTTTATATTC
AAGGAGTCAGTATTTATATTCATCTTTTAAACTGGGAAGATTTATATTTTACTTTA
AAACTTCTTGATAATAATTTACAATGAATGGACACAGTGATGAAGAAAGTGTTAGA
AACAGTAGTGGAGAATCAAGGTAAGTAAGCACTTTGTTATCAATTGTTTACTATGA
AGAGAGTTGAAAACTTGACTTTTTTCTTTATTGTTATTGTTGTTATTTAGTTTTCC
TCATAGGTAGCAGAGTTTTCAGGTTTTCCTCTTAGCTATCCAAATACTAAAAAAAT
TCTGATATACGAACCTTTTTTCATAATACAGGTTTTAATTATATTTTTCATTCAGA
TACACAGTAGATCTTAAATATAGAAAGTTTTTGTTTACTTAAATCTATTTGGAAGT
TTATATTTGAGCTAATAATTAAGCTGGAGCATGTATAATAGATTTAAATTGTTTTG
ACTGTTAGTGAAATTT


Sequence ID 1218

CTCACTTGGTGGGTGAGCCTCCAATGACTACACCCAAGGAGGATTTAACACAGGGA

TTTTATGACTTGCAACAAGTCAGGAGGACATGGGGTTGGGGTAGTTCAGCAGTGCC
TGTCTGAACAAAGGTGAAAATTGGGCTTTTATTGGGCTGATCAAGGGGGAGTAAAG
GCAGCCAGGAGCAGTCGCCTGTCATGCTTCTACCTATATTGCATGTATAGAAAAGG
GAAAATAAACTCCTTCCTGGGCAGGGTTTTAGTATGCTAAGGAGGGGAGTTATTCA
ACTTCAATCCAACTCAAGCATCAGCATTGCTGCGTCCATCCCAGTTTTGTTTTGCT
GGGGCTGAACTTCTTCCTATAACTTTTTGAAACAACAAGAACTCAAGGTGTGACAG
TTACAAGTGGGCCCTTTTTCACAGTGTGTACCTAAACACGTGAGGACCCTGGATTA
CAGAATGACAGACTCGAAGTGACTCAAGTTCCGGTTGTTCATCTTTAGATGGTAAA
GATGGCTGTACGTACTATCCTTGCTTATTTCCAATCTATTGTTTAAACTCTTGTAT
ATGTAATACCGCAGAGGCTAGAGATACAACCTTTGACCAAATGAGTGAATTCAAGT
AATCCATTACTAATGTGATCTGGAAACAAACATGGTGTTGAATGTGCATATGT

Sequence ID - 1219                         nt:     559
CTTGGCAGCTCCGTTATGTGCCCAGCTCTTTGCAAGGGCATACTGGGAAATGAGTG
GAGATAAAGGACCCAATCATAAGCATTTTACAGTATGGATACCCCATTTTAAAAAG
GTAAACTGAGGCACAATGCAATTTTTTTTTTTTTTTAAGGAGTTTATTTGAGCAAA
CAGTGATTCATGAATCAGGCAGCACCAAACCAGAAGGAGGCTTTGCTGAANAAGGA
TGAGGGACAAGCATTTATAAAGTGAATGTAGATGTAATACAAAGAAAATATTTGAA
CCGGGTGCGGTGGCTTACACTTGTAATCCCAACACTTTGGGAGGCCAAGGCGGGCA
GATCACAAGATCAAGAGATCGAGACCATCCTGGTCAACATGGTGAAACCCCATCTN
TACTAAAAAATACAAAAATTANCTGGGCGTGGTGGTGCGTGCCTGTAGTCCCAGCT
ACTTGGGCGGCTGAGGCAGGANAATTGCTTGAACCCGGGAGGTGGAGGTTGCAGTA
AGCCGAGATTGCACCATTGCACTACTCCAGCCTGGTGACAGAGAGAGACTCCATC

Sequence ID 1220

GANNNGTGCGATANNATGNNTGTCTTTTTTTTTAAAGTNTTTCNNATNGNAGNGAAN
CCCCCNNANNTNNCATAANGAGAGATNACTACNGTACANATAGNGNCANACNGATA
GTAGTANCAANATTGTNTTAGCTANATNANTCAATAGATATCNAGATANAANAANA
NCNNGGATATACAGCGATGTNTNANNGGNNNNNNNNANGGAACGAACATCNACNTTA
ANNATAAGCTNGNGGAGAGAGACANGTANGTTATANANNAGAATNGNAGTAGGNGT
GATCATAATAGNNNNNANNTANTATATANGATNTTANTGNNCTNTNNTNNGTTTAT
CNNNAATNTCTATNCTNGAGAGNAGCNNNATNNNNAGGCGANGANATTGGGNNNTN
CTCNTNATAGANANCTGGTGTCNNANAANTACNTCATCTATTNANCTCTCACNANA
TGGNANNATANAGNAGNGNNNTNNANAGGANTANGCATAGNGNNTNNCTNAAACAA
AANNNATAAGANNTCTCGNNAANANGGGCCTNTNNTNTAGCGAGGNNTTANTTTNT
ATANTTNTTCNCTCTTNNAATANNTANGATANATGANCTNGNNGTGATANATANNN
NNTACNGTNAANNTNTANTCNTATAATAGATANAAATATAGGATNTTNCTCTGGCN

GGTNGAANANTTNNTNCNNTTTNAATAATGNTGTTAGNGACNGNGNTNTNANANNN
NNTTAGAAAGGTACTCTATATACTNNTATGNTNCGGCNNATAATANAACAGATGTT
TGTATNAATATNAAANAAGGTCNNTTTCGNCAAGAGAANNNTGNCTGGTNATAGAA
TTAGCATAANTTANNTANTATGATNNANTNNTNCTACNANTNTTAGCNNTTNGCAG
NAGTCATTNNGNATNTATNNNGNNTANTAGTNANTTGGGNCTNNTNCAGANTATAT
TNTGNGAANATGAANNTACGNANTCCTNNGNANTATNATNNTGANTANGANAANCN
ANANNTNTTNTANNANTGNCTATANATTGCCNNGATANATTNTNNNAATGAANCGA
TAGCCCGCNCTAAGGANNTNNGTNANNTAAANNTCTCAGATAANNTACNTNTTNNT
TATTAANCNANNATCACANTATANCNGNGACANNNGCGANANTATATGTATGNNAN
TATNACNGNTCCNNNCCGNGAANNTANTCNTANNAGGCATTCNGNNGAGCTNTTCT
NCTAGACNATTTNNANTGAAANNATGCNGNNAAAAACGACNNNCTTNAANTTNTGT
CTACANTCCGCNNTNTTTNTACAGATNGCAGNTAAGNNNANTNANNGCTCTCANCT
NGCTNNNACT

Sequence ID - 1221                              nt:        741

AAGCAGAANTNTCTCTAAAAACATTATCTCCTTAAAATCTTGAGGTGCATATNAGA
GCCACAGGCAATCTCTGACATATAAAATTGCAGTACAGGCCTTTCAAATTTGGCAT
TTCACTGGTACAATACAACAACCAAGATATATAATAACTGTACAGTGCCTAGACAT
TCCAGTAAGAACCATTATTTTCTTTAATGTAGAATGATTAATACATATTCTACAAG
GGGCAGTAAGGTTAGTAATTCTATAGGGTATGTCCCGACATAATTTTCAAATTGTA
CAATAACACAAACAACTTTGTTAAGGCCATGTTTTATTTGCTGATTAATGGACAAA
AGGCAATGTAATTTATTTTCAAGTATTTTCTTGAAAGTCTGTGCTCATAAAAATCA
TGAAAGTTGGAAAGACTGTTAAATCACTGAAACTTCAAATATATCTTACACAATC
TTGTTTGTACAAAAATACAAGTTAAATATAAACATAAAGCAATCATGGTAATTTTA
TGCAAATCTGTTTTATGTGATCATCAGTTATATATAAAGTTTCTCAGTTCTGTTA
TTTGTGAAAGATCAATACCAGATTGAATGACTACCTATTGGCAAAGGGCCCTAAA
AAGCTTACTTTAGCACTCATCTTTTACATGGTTAAATGCATTTCCTAATTTGAGAT
CACCTAAACACTGGAAAAGAAAAAAAATGAAAGGGCAGTATGTCCATAAACCAACA
AATAATTTGGCTG

Sequence ID - 1224                              nt:        485

CGAAATTTCCTTGTGACACAGAGGAAGGGCAAAGGTCTGAGCCCAGAGTTGACGGA
GGGAGTATTTCAGGGTTCACTTCAGGGGCTCCCAAAGCGACAAGATCGTTAGGGAG
AGAGGCCCAGGGTGGGGACTGGGAATTTAAGGAGAGCTGGGAACGGATCCCTTAGG
TTCAGGAAGCTTCTGTGCAAGCTGCGAGGATGGCTTGGGCCGAAGGGTTGCTCTGC
CCGCCGCGCTAGCTGTGAGCTGAGCAAAGCCCTGGGCTCACAGCACCCCAAAAGCC
TGTGGCTTCAGTCCTGCGTCTGCACCACACAATCAAAAGGATCGTTTTGTTTTGTT

TTTAAAGAAAGGTGAGATTGGCTTGGTTCTTCATGAGCACATTTGATATAGCTCTT
TTTCTGTTTTTCCTTGCTCATTTCGTTTTGGGGAAGAAATCTGTACTGTATTGGGA
TTGTAAAGAACATCTCTGCACTCAGACAGTTTACAGA

Sequence ID 1226

GGTTTTTATACTTGCCATGAAACTGTTCTTTGGGATATTATTTTGTTCAGGTTCCC
CACTTGGACAGCAGAGGGGGTGACTCTGCCCATCCCTGCCACTGGTAGCCAGGCGG
GCAATGTCTGCTAGCAGTCTGCTTCTGTCTGAACTCAGCCAGCAGAGGCAAACTCC
CGGTTCCCCGAGAAACACTCTGAAGGCAGGGTGGGTGACTCCACCCACCACCGCCT
CTCCTAGCCATGCAGGCCATGTCTGCTAGAGCTTCCAGCGCAGTGGTCCTAATTCT
GTCTGAATCCGGCTGAGGGGTGCAGCCTCCTGTTACTGCCCAGGGAAACACCCAGA
TGGCAGGGTGGGTGACTCCAACCACCTCTGCCTGTGGTAGCCAGATGGGCCACACC
TGCTAGAGCTTCCAGCCCAGCAGTCCCGCTACTCTGTGGGTGGGTGCCATCCCCTG
TTCCTCTGGGAAGCACCCAGACAGCTGATTACGTGACCCCACCCACTTCTGCAGAT
CCTAGCTGAGCAGGACTTGCTGGTTTGGACAATGCCCAAGCAGGGAAGAGCCCTCA
TTCTCTTATCACTGACAGAGGTGAGATGTCCGANTTTGTANGCTGGTGGAGGAGTG
AGGTGGAGGAGGTATGCCTCT


Sequence ID 1228

GTTATTCAGGTATCCATCAAAATTTTATAAGAGGGCCGGAAACATCGGCTCACACC
TGTAATCCCAGCACTTTGGGAGGCTGAGGCAGGTGGTTCACTTGAGGTCAGGAGTT
CGAGACCAGCCTGGCCAACATGGCAAAACCCCGTCACTATTAAAAATACAAAACAT
TAGCTGGGTGTAGTGGCAGGTGCCTGTAATCCCAGCTATTCGGGAGGCCTAGGAAG
GAAAATGGCTTGAACCTGGGGGTGGAGGTTGGAGTGAGGCAAGATCACACCACTGC
ACTCCAGCCTGGGCGACAGAGCGAGACTCCATCTCAAAAGAAGAAAAAAAAAACAA
CAAAAAAACCTTTATCAGATTATCAGAGGTTATCACTACAGAGGGAGGTAAAATTG
GAGGGAAAAGGGTACAAATTTATTTCAC


Sequence ID - 1230                                    nt:      741

AAGCAGAANTNTCTCTAAAAACATTATCTCCTTAAAATCTTGAGGTGCATATNAGA
GCCACAGGCAATCTCTGACATATAAAATTGCAGTACAGGCCTTTCAAATTTGGCAT
TTCACTGGTACAATACAACAACCAAGATATATAATAACTGTACAGTGCCTAGACAT
TCCAGTAAGAACCATTATTTTCTTTAATGTAGAATGATTAATACATATTCTACAAG
GGGCAGTAAGGTTAGTAATTCTATAGGGTATGTCCCGACATAATTTTCAAATTGTA
CAATAACACAAACAACTTTGTTAAGGCCATGTTTTATTTGCTGATTAATGGACAAA
AGGCAATGTAATTTATTTTCAAGTATTTTCTTGAAAGTCTGTGCTCATAAAAATCA
TGAAAAGTTGGAAAGACTGTTAAATCACTGAAACTTCAAATATATCTTACACAATC

TTGTTTGTACAAAAATACAAGTTAAATATAAACATAAAGCAATCATGGTAATTTTA
TGCAAATCTGTTTTATGTGATCATCAGTTATATATAAAAGTTTCTCAGTTCTGTTA
TTTGTGAAAAGATCAATACCAGATTGAATGACTACCTATTGGCAAAGGGCCCTAAA
AAGCTTACTTTAGCACTCATCTTTTACATGGTTAAATGCATTTCCTAATTTGAGAT
CACCTAAACACTGGAAAAGAAAAAAAATGAAAGGGCAGTATGTCCATAAACCAACA
AATAATTTGGCTG

Sequence ID - 1231                                      nt:      203

TTGAGGAAGGGTCTACTGTCTTTTTAAATGGCACAATTTTAAGAGGTTTGAGAGGT
ACAGTCCCTTAACCTGCCACGGGAGAGGGGCCCCCAAACTTTCTTCCCCCCACACT
TCTGGTTTTCTGTGTGGAGGGGGAGCAGGGATATCTAAGCTGTGGTGTGAAAGGGT
AGGAGAGATGCTGGAGGTGGGGGTGCTGTGTTCTA

Sequence ID 1239

TTTCCTCGGGAAGCGCGCCATTGTGTTGGTACCCGGGAATTCGCGGCCGCGTCGAC
ATTTTTTTTTTTTTTTTTTTTTTTAGAATGATTAACAATTTATTGAGTTTTATTTATC
TACAAAAATATAGCAATACAGNGAACTTCACCAAATCCTAAATATTCAGTACCTGA
ACTGGCTACAACACCGNGTGCACACCCAGTTCCTGCAGAATCTCTTGCAGATATGG
GAGAGTCAGCCAGTGAAAAGATCCATTTCTTGGGAATCCTTGTCAACAAGACCAGT
TCAGAAATCCAGGATATATAGAAGCCTACTGTAATTTAAAAACAGTAACAAAAACC
CCAACAAAACCCAAATCAACAAAGACCAAGATAAAGGNGTGATAAACATTAATTGT
AATGGTTTTCCTTTACATGCAATACATGCATTTTAAAATCACTAAGAAACACGAAA
TTTTGTAGAGCAAAGTTTGNGTTTCACGTAAGTGCAAATGAATATATATTTTATTT
TTTATACTATTAAATTATATATATTTTTTCCATACAAAAGCACACAGTGTTAATCT
ATAAAATGACATCCAAGTGGATGATGATTGTTTTTGCATGTCCCCCTGCTTAGATT
TTTTTAAAATATATAGTCAAAAATTAACATCCTTCTTTAAAAATACAGAAGGGAAA
AANGGGCAAAAAAAAAAATCTAGACTCGAGCAAGCTTATGCATGCATGCGGCCGCA
ATTCGANCTCGGNCGACTTGGCCAATTCGCCCTATAGNGAGTCGNATTACAATTCA
CTGGGCCGNCGNTTTACAACGTCGNGACTGGGAAAACCCTGGCGTTACCCNNCTNA
TCGNCTTGNAACAATNCCCNTTTNGCCAGNGGGG

Sequence ID 1255

TCACTTCGTATNGAANCTGTTTGGACTTGCTCAACAAGACCTTATCTTAACAAAAA
GTAACTTATAGAAAAGGGAGACATTCATTTAACTTCAAGCCCATATTATTCTTAAA
AGCTGACTCTTGAAATAGTATTTATTGAGTCATAGTGGAGTCATGGGACTTTTTAA
GGGCCGGAAGGGACTATTTAGATCATCCAGTCCCACCCTGTCATTTTATGGAGGAG
GAAACTGAGGCCTAGATAAGATAACCAGTTAGTGGGTCCACTGACCTTTAGGACAG

TAGTCTATCCGTAAGAGACAACATGGAGAAAGAAATACAACGTTTTTATAGTGAAT
TATCATCTTACAAAGAATATTCTTCCCATATCGCACTTTTAAAAAGTGGGTACCTT
AGTCAAATAGGAGAAAAAACCACTTGAGTAGTTTCATCCTCAGGTTTTAGGTGAGG
AAACTGATACTCAGATTAAATAACTTTAAGCACACAGAGCCTGAATGATAGTCTTA
TTTGAGCTCATCTGTGCTTTTAATGTGTACTACGTTAGGTGTTTTCACTTGCATTT
CCTTTAGTCTTATTTGAGCTCATCTGTGCTTTTAATGTGTACTACGTTAGGTGTTT
TCACTTGCATTTCCTTGTTTGACGTTGACAATAAATCGTGAAGCTGCCTTATCTAA
GNAGTCCTAAAGTAAATCATTGGAACACATGTANCCAGTTTGTTGTTTTTATTTGC
CAGGTNTCAAATATAACTGAAAACCCATGCTAACTGACTNATTTTAAAAGNTGTNT
GGGGCATGAAANGATTGCTCTGCCTGGGCGGGNGGTTNANCCTGNGTCCCCCNTTT
NGGAGNCCACCCANGANGCGATATTTNAGGGNNGATTCNAAACCCCTGGCACGNGN
NAACCCCNTTTTTAAANANAAAANANCGGNNG

Sequence 1256

TTGTGTTGGTACCCGGGAATTCGCGGCCGCGTCGACGGAGTTTTACCTTATTACAC
TTTAATCTCTGGATTTACCCCATCTCATTTCTCTTTTAGGAAAACTGTTTGTATGT
GGTGGCTTTGATGGTTCTCATGCCATCAGTTGTGTGGAAATGTATGATCCAACTAG
AAATGAATGGAAGATGATGGGAAATATGACTTCACCAAGGAGCAATGCTGGGATTG
CAACTGTAGGGAACACCATTTATGCAGTGGGAGGATTCGATGGCAATGAATTTCTG
AATACGGTGGAAGTCTATAACCTTGAGTCAAATGAATGGAGCCCCTATACAAAGAT
TTTCCAGTTTTAACAAATTTAAGACCCTCTCAAACTAACAGGCTTAGTGATGTAAT
TATGGTTAGCAGAGGTACACTTGTGAATAAAGAGGGTGGGTGGGTATAGATGTTGC
TAACAGCAACACAAAGCTTTTGCATATTGCATACTATTAAACATGCTGTACATACT
TTTTGGGTTTATTTGGAAAGGAATGCAAAGATGAAGGTCTGTTTTGTGTACTTTTA
AGACTTTGGTTATTTTACTTTTTTGGAAAAGAATAAACCAAGAATTGATTGGGCACA
TCATTTCAAGAAGTCCCCTCTCCTCCACATTTGTTTTGCCAATTTGCACATTAAAT
GACTCTTCCCTCAAATGTGTACTATGGGGTAAAAGGGGTAGGGNTTAAANATGTAA
ACAGTTGGGTTTTTTAAGGGNCCTTTTTCATAACTGGAACACTCTNTACAAGGNTN
CTTNTTAAATAAATAACTTGACTTTTTTGTTTTNTAAANGNANCTTCNTGCTTCCA
TAAAAAAAAAAATTTAANTNGNCANCTNTGCTGCTGCGNCCANTTNGCTNGNCCNT
GGCATTCCCTAGGGANGNTNAATANTGGCNNNTTAACNNGGCNGNAACNNNNNCCA
NT


Sequence ID 1331

GGGCGATGCATGCTTTATTAAGGCTCTTGTTTCACCTGGCAGTGTACTGTATCAAC
GTATAATACAGAAAAAAAATCTCTTTAAGGTCCTCCTTCACAAAGACATAGAGTGA
AACTCCCTTTACATGTCAGTATTTGTTCAACACTTTAGGCAACTTGACTGTCAGTG

TTAAAATGGAAAACAGGAAAATGGAAAAATCTGACCAATTCTGCCACCTTGAGACT
TTCATATAGACCTTGCACAACAATTGTATAGATCACACACCGGCTGTATTTAATAT
GTAACATTTTCACACATATTAAAGATACAGAAGTATTAAAAAACCCCCAATGTTAA
TGTATTTGCTTAAAAGGCACAAGTTTCACATATCTGTCTAGCTATCTGTTGGTAAT
ACAGAAGTATACTACTTTTTTAAAAAAGTGGGCAGAATTCTTGTGTATGTATATT
TGTGTGTACAGTATGTGTATGTGTGTATATATATATATTATATATATAGATAATAT
ATAAATATTTTTTTTAAGGAGAAACTAGAATGTTTAGCTAGAAAATTCCACAGCCT
GTGAAGAAATATTTCAAAATGGCCATAAAGGAGGTAAAAATGAAAACCATAACCTA
ACTTTTATAGAGGCTTTATCTTTAATTTAACGATGTGCGGAGGACTTTCTTGCTTG
AATCTGTTCCGGGCTGTCTGCTCTGTCCATCAAATGGGCAGGTCTGGGAATGAGGC
ACCTTCGGCCGTTCAGAAGTGGCCTGAACAGAATGCTGGACCCAGGCTGGACTCG
GAC


Sequence ID 1332
CAAACCTGCATGTTCTGCACATGTATCCAGGAACTTAAAAAAAAAAAAAGATAGTT
TGTGTGTCTTAATTGAATAATAGTAGATTTATAGATTAAAGATCTATGGGTTTTTA
ATATGGATTAGAAATCTGTGGGTTTTTGATATGGATTAGAAATCTGTGGGTTTTTA
ATATGGATTGGAAATCTGTGGGTTTTTAATATGGATTAAAAAACATCTGTGGGTTT
TTAATATGGATTAAACATCTGTGGGTTTTTAATATGGATTAAACATCTGGGTTTTT
AATATGGATTAAACATCTGTGGGTTTTTAATATGGGTTAAAAATCAAAGAAAATG
AACTATTTGCTCCAGTGCAGGAAAATACAGGCAATACTGGATACAATTAGATGGTC
AGGAGCGATAACCCGGTTGCCATTGTTTGAAGAAGAGAATAAGGTGCTAGCATTCC
TATCCGTAGATAATTTGACAGCTAGGAAATAGGGGGAGTCTTCTATGTAGTTAGTG
AAGGCTAAATGAACTATTATATGCAGTTATCGTAGAAGAGTACTCAAAAAAATCTG
TAAAAAATAAAGAAAGGCCGGGCGCGGTGGCTCACGCCTGTAATCCCAGCACTTTG
GGAGGCCGAGGCGGGTGGATCATGAGGTCAGGAGATCGAGACCATCCTGGCTACCA
NGGTGAAACCCCCGTCT

Sequence ID 1335

CAAGACTCCATCTCAAAAAAAAAAAAAATCTACAGTGCTGAGTATATAAAATTAT
TAACACATTTCACAACAATATGTGTTTGTGGAGTTAAATATTTTTTGTCTTTAAAA
CAGGTAATTTTAGTGCATACTTAATTTGATGATTAAATATGGTAGAATTAAGCATT
TTAAATGTTAATGTTTGTTACATTGTTCAAGAAATAAGTAGAAATATATTCCTTTG
TTTTTTATTTAAATTTTTGTTCCTCTGTAAACTAAAAGAACACGAAGTAATTGGTC
ACAATTACTGGTGTTTAACTGCCAAATATGGGTAAATAAGGGAAAATTTTGTTTAA
TATTTAGTCCTTCTGAGATGGCTTGAATATTTGAATTTTGTTGTACGTCTATACTG
GGTAGTCACAAGTCTTATAAACACTTTAGAGGAAAGATGGATTTCAGTCTGTATTT


TTAAACATCATTTATTTTAAATCTGGTGCTGAAAAATAAGAAAAAAATTAAACTGC
ATTCTGCTGTTCTTCTTTAGAAGCATTCCTGCGTAAATACTGCTGTAATACTGTCA
TGCAAAGTGTATCCTTTCTTGTCGTATCCTTTTTGGGGCAGTGTTTTTTTGTTTTT
TTCCTAGAAATGTTTGTCCTTCCCCCACCTGTTGATCCAGGTTAAGGAATACTTTT
TTACACTTTATTCAAA


Sequence ID 1336

CTTTTCCTCCCGCTGTCCCCCACGGAGGGGACTGCTCTCCCCCGCTGCATCCTTTC
TGTGAGGTACCTTACCCACCTCAGCACCTGAGAGGGTGAAATAGAATTCTAACCTC
GACATTCGGGAAGTGTTTTTGAGAAGTCTCGGTCGGTAAGGGAAGTCTTCCAAGTC
CGTGCAGCACTAACGTATTGGCACCTGCCTCCTCTTCGGCCACCCCCCAGATGAGG
CAGCTGTGACTGTGTCAAGGGAAGCCACGACTCTGACCATAGTCTTCTCTCAGCTT
CCACTGCCGTCTCCACAGGAAACCCAGAAGTTCTGTGAACAAGTCCATGCTGCCAT
CAAGGCATTTATTGCAGTGTACTATTTGCTTCCAAAGGATCAGGCCCTGAGAACAA
TGACCTTATTTCCTACAACAGTGTCTGGGTTGCGTGCCAGCAGATGCCTCAGATAC
CAAGAGATAACAAAGCTGCAGCTCTTTTGATGCTGACCAAGAATGTGGATTTTGTG
AAGGATGCACATGAAGAAATGGAGCAGGCTGTGGAAGAATGTGACCCTTACTCTGG
CCTCTTGAATGATACTGAGGAGAACAACTCTGACAACCACAATCATGAGGATGATG
TGTTGGGGTTTCCCAGCAATCAGGACTTGTATTGGTCAGAGGACGATCAAGAGCTC
ATAATCCCATGCCTTGCGCTGGTGAGAGCATCCAAAGCCTGCCTGAAGAAAA

Sequence ID 1337

CAAGAACTCTGGGACATTTGCAAAGGGTATGGCATATGTGTAATGGGAATACCAGA
GGAGAGGAAAGACAGGAAGTCAAAAAAAGAATTTTTCCAAATTAATGATAGGTTCC
AAACCACAGATGCAGGAAGCTTAAACACCAACAGGATAAATAAAACAAAATCTACG
CTTAAGCATATCATACTTAACCTGCAGAAAATTACAGACAAAGAAAAAACACCAGA
GGGGAAGCTGGCAGAAACATACCACCTATAGCGGAAGAAGAATAAGAATTACATCA
GACTTCCCTTCAGAAATCTTGCAAACAAAAAGATGTAGCACAATATTTAAAGTATT
AAAGGAGGCCGGGCCCGGTGGCTCGGGCCTGTAATCCTAACACTTTGGGAGGCTGA
GGCAGGAGGACCATGAGGTCAGGAGATCGAGACCATCCTGGTGATGGTGATACCCC
ATCTCTACTAAAAATACAAAAAATTAACCGGGCATGGTGACACGCACCTGTAATCC
CAGCTACTTGGGAGGCTGAAGCAGGAGAATCGTTTGAGCCCAGGAGGTGGAGGTTG
CAGTGAGCCGAGATCACATCACTGCACGCCTGGGCAACAGAGCGAGACTCCATCTC
AAAAAA


Sequence ID 1338

CGACCCGTTTTAGTCAGGATGGTCTCGATCTCCTGACCTCGTGATCCGCCTGCCTC


GGCCTCCCAAAGTGCTGGGATTACAGGCGTGAGCCACCGCGCCCGGCGTAAATCAG
GTTTTTTAAATGTTTGCCAAACCTTATCACTGACTTTTATAACAAAATTATTTACT
ATAATCATTAGGGAATATTTAAGTTCTGCTAATACTTAAAATTGCAGAGTGCTAAA
ACCAGCAGTGAGTTTAGAATCAAGCTAAGCTTTATTGTTGCTACTATTTGAGGCAT
ATTAGTTGACTGGTGTTCATATGCAAGGCAGTCTACTGGGTGCAACAAGGGTTAGA
AGGATATTTTTAAAÀAACTGACCCTATTCTCAGGATGAAAATAATACACTAGTAAT
AGTCTGCTCTGTTGGTTAACTCCTCGTAAGGAGGTACAATTAAAATGCTGTAGTGT
TGCAAGGGAAGGAGAGGAAGAATCATATTCCTTCACTAGCAGGATCAAGAAAGCTT
TTATAGAAATATACAAAATCTTCACTTCTTGAAGGATTGGTAAAATTTAATAGCCA
ACATTGGGCACTTATTCATTCTCTGAGTAAATATTTATTGCATGCTTATCTTGTAT
CAAGCATTGTGATGAAAGCACAAGAATGAAAGAGGAGGGAGAATGTTTAGAGAATA
AGGGCTGAAACACAGATTTTGTAGGGAGCGTAGGGGAGACTGANAAGACAGGTTCA
GGTTAGTAAGGGCGCTCATATTTTGACCCTGAATGTTAACTATGTGCACATCATGC
TAGCTATTCTAAATCAGGCATTTTCAAATGGAAGCAGGCACTGACATTTT

Sequence ID 1344
CGTGAAGGGTCTTTATGTATTAGTATTAGAGTGATCTTTTGATTATTTTCCTCACT
ATAAGGAAATTATTTCCTCAGGATGAGCTGCCATAACATTCCACTGTCTGATGGCA
ATTTTAAAGCCTGAAATTGAAGCCCATGGCTAGGCTATGAGAACCCTAGTTCGTAT
AGTAAAGTTGATATCTTCTGGATGTATACTAATTTTAGGCTTTATTTTAAAACTGC
TGGAAACTGAAACTTAGACAAAAGTATTTTCAGGACATCATTTACAATGTTTAGCC
CTAAAGAGTCAAGCTGTGGGATTCTGAGTCTTTCATATGTTACAGCAGAAACTTAA
AAGCAAGAGGAAATTGGCTGGGCACAGTGGCTCTGTAATCCCAGCACTTTGGGAGG
CTGAGGTGGGTGGATCATGAGGTCAAGAGATTGAGACCATCCTAGCCAACATGGTG
AAACCCCATCTCTACTAAAAATACAAAAATTAGCTGGGCGTGGTGGCACACGCCTG
TAATCCCAGCTAGTCAGGAGGCTGAGGCAGGAGAATATCTTGAACTTGGGAGGCAG
AGGTTGCAGTGAGCCAAGATTACATCACTGCACTCCAGCCTGGTGACAGAGCGAGA
CTCCGACT


Sequence ID 1348
CTGAAACTGCACTGAACCCACAGGTAGGTTACATCACAGGACAGAAATCTGAGGAG
CTGGAGAAAGCAAAAGAATAAAGGATGGGCTGACACCAGAAGGAATTAAAGGAATT
TTTATACTGAACTTCAATTACTTGTTCATTTGAAGTTTGTTTTTTTAATGAACGTT
TTTGCTGTTACTTAAATATAGTGTTTTGAAAGTGTTTCAAATGTATTCAAGTTGGG
ATTTTCCATATTTTACTACAGTTCTGTCTTAGTATGTTCACCATAAAACACTTATC
ATTAAAGCTCACAAAGTGCTTTTTTTGTAATATGAGGATAAAATGAAGCCATATAAG
AATTTTTTTATATCTGTACATTTAACCCACATTTGAGCTTTAGCCAAAATATATAG


CTTTTTTTTTTTCTGACCTGGCCAACGTATTATCCAGCAAACATCAACTGAAGCAAT
ATGGAAACACTTCCAAATGTTTGCCAATAATGCTATTAAGTGACTGATGTCAACAT
TAGTTACATGGCAAACTAAAGAGGCATTATACATTTTTAAAACACACTAACATATA
ACTGTAGATAATGTAAGGTTTATTTATATGCATATTTCATAGTATATTTAAATGTT
TAAATATAAAAAAGGGTTTTTAAACACTTTTAATTTTTATCTTTGATTTTTTTTAT
TGATATCTCTTTCCAGGCTACTAATAAAATTGCCAGAACTAAACTATCAGGTAAAG
GTTAAGGCATCAATTGACAAGTAAGTTTTCTAATTTCGTTTTGAATTACAATTCCA
AATGTAAGACTTTTAAAAATGAATGGCCTTTATTTTATAGAATAATTTTGACCTTT
TAAATTTACTTATCTAACATTATATAATGAATGTACTTCAAATATTTGACTTTGAA
GTCAACATTAACAAATTCATGGATCCTAATTAAAATTTACTATAAAACTGGAATCA
TTTATTACTTCCTT

Sequence ID 1351

TTTTTTTTTTTTTAAAAGAGATGGGTTCTCACTATGTTGCCCATAATGTTTATGAG
ATTAAGTTCATCTTTTTTATCTGAGTAGTATTTTATTGTATGAATATACCACCATT
TATTTATCTGTTGGTTATTTCCAGTTTTGGGCTATAATCCAAAATGCTTTTTTCAA
ACAATAGGCTATATATCATTAATGTCCGTTTATCAGCAGTATAAAATATCTTACCA
TAAATATTAATAAAAGAAGCATTCATATATAAAATATAGATATTTCAAACCCTACA
GAGGGCCTTTTAATGATTAAATATTTTGTCCTTACAAAAAGGTCCAGGTAATTACA
CCCATGAGGTTAACCTGCCTTAGTGCAGGACTTAAAATAAGGCTTCTCCTGCCATC
TCTCTCCATTTGTAGAATGTGAAATTCTTTAAAATGCATCCTATATTAGGAATACT
ATAGCTGTGCACTGGTGTTTGTTCTCTTCTTTAAACTCGGGACCGTATATATCTGC
TCAAATTGCCCAAGTATACATATGCTGCACTCCATCAAGTGTCAGGCCACATTCTA
TCAGCACAGCGTGACTGCCTATCAGTGACAATATAAGTGAGCTCTATTTGGATCCC
TCTTACCCTACCTTTTATATTTATGACAGCATTATCATAAAACTCCAATATTCTTC
AATAACTTACATGTTTGTTGTAGGATAAAATTATTACCCTCAATGAACTACAT


Sequence ID 1352

ACCAGCTTCTTCACAGGTTCCACGAGTCATGTCAACACAGCGTGTTGCTAACACAT
CAACACAGACAATGGGTCCACGTCCTGCAGCTGCAGCCGCTGCAGCTACTCCTGCT
GTCCGCACCGTTCCACAGTATAAATATGCTGCAGGAGTTCGCAATCCTCAGCAACA
TCTTAATGCACAGCCACAAGTTACAATGCAACAGCCTGCTGTTCATGTACAAGGTC
AGGAACCTTTGACTGCTTCCATGTTGGCATCTGCCCCTCCTCAAGAGCAAAAGCAA
ATGTTGGGTGAACGGCTGTTTCCTCTTATTCAAGCCATGCACCCTACTCTTGCTGG
TAAAATCACTGGCATGTTGTTGGAGATTGATAATTCAGAACTTCTTCATATGCTCG
AGTCTCCAGAGTCACTCCGTTCTAAGGTTGATGAAGCTGTAGCTGTACTACAAGCC
CACCAAGCTAAAGAGGCTGCCCAGAAAGCAGTTAACAGTGCCACCGGTGTTCCAAC


TGTTTAAAATTGATCAGGGACCATGAAAAGAAACTTGTGCTTCACCGAAGAAAAAT
ATCTAAACATCGAAAAACTTAAATATTATGGAAAAAAACATTGCAAAATATAAAA
TAAATAAAAAAGGAAAGGAAACTTTGAACCTTATGTACCGAGCAAATGCCAGGTC
TAGCAAACATAATGCTAGTCCTAGATTACTTATTGATTTAAAA

Sequence ID 1353
ACATTCTGGAAAAGGCAAAAGGGAGGAAGAACTGATTAGTGGTTAGCCCAGGGGTTA
GAGTTGGGGAGAGGATATAATGAGGGAACTTTTGTGGATTCTGTACCATGATTATG
ATTACACAAACCTATGCATACATTGAAACACATAGAACTATACGTTGAAAAAAGTG
AATCTGCCTGTATGTAAATTTAAAAGAAAAATATTTTTTTAAAAAAACAGATGCTT
CTTAACACATTATCATCTATGTCAGTTTAACAGTTAGTAGACTTAGGCCAGGTGTC
ATGGCTCACTCCTGTAATCCCAGTGCTTTGGGAGTCTGAGGTGGGACGATCTCTTG
AGACTAGGAGGGAGTTTGAGACAAACCTAGGCAATGTAATGAGACTCTTTCTCTAC
AAAAAATTTTAAAGTTATCTGGACATGGTGGTGCCTGCCTGTAGTCCCAGCTACTT
GGGAGGCTGAGGTGGGAGGATTCCTTGAGCCCAGAAGTTCAAGGCTACAGTGTGCT
ATGATAGAGCCACTGCACTCCAGCCTGGGCAACCAGGTGAGACCTTGTCTCTAAAA
TGAATAAATAAAT

Sequence ID 1355
TGGTCTTTCACCCAGCCAGGGAGAAGGTTCTTCGCTCAGTATGAAGAAAAGCAACC
CAAAACTCTCAATCTGATTTGTTTTTGTTTATGTCGATGCCCTGTAGTTTGAAAGT
GAAGTAAAGATTTAGAATTCACCTAAGTCCAAAGGAAAACACGTGGTTTTTAAAGC
CATTAGGTAAAAAAAGTTCTCAATAAAGGCATTACAATTTTTTAGGTTTAGAAAGA
TGGACTTTTCTGATAAATCTTGGCAGACATCTAAAAAAAAAAACCATATTTTTCACA
AGAAAATGCAAGTTACTTTTTTTGGAAATAATACTCACTGATTATGGATAAAATGG
AATATTTTCAGATACTATATTGGCTGTTTCAAAATAGTACTATTCTTTAAACTTGT
AATTTTTGCTAAGTTATTTGTCTTTGTTGTATCTATAAATATGTAAAAAATATTTA
AATAGATGTACCTGTTTTGCTTTCACACTTAATAAAAAATTTTTTTTTGT

Sequence ID 1359
CGGGATCCCTAGTATAACACATTCAGTGTTCCCCTTTCAGTCTTACTACTTTGACC
GCGATGATGTGGCTTTGAAGAACTTTGCCAAATACTTTCTTCACCAATCTCATGAG
GAGAGGGAACATGCTGAGAAACTGATGAAGCTGCAGAACCAACGAGGTGGCCGAAT
CTTCCTTCAGGATATCAAGAAACCAGACTGTGATGACTGGGAGAGCGGGCTGAATG
CAATGGAGTGTGCATTACATTTGGAAAAAATGTGAATCAGTCACTACTGGAACTGC
ACAAACTGGCCACTGACAAAAATGACCCCCATGTGAGTATTGGAACCCCAGGAAAT
AAATGGAGGAAATCATTTGCCTTAGGGATTGGGAAAGCTGCCCACTAACTGTCTTC

CCCATTGTTTTGCAGTTGTGTGACTTCATTGAGACACATTACCTGAATGAGCAGGT
GAAAGCCATCAAAGAATTGGGTGACCACGTGACCAACTTGCGCAAGATGGGAGCGC
CCGAATCTGGCTTGGCGGAATATCTCTTTGACAAGCACACCCTGGGAGACAGTGAT
AATGAAAGCTAAGCCTCGGGCTAATTTCCCCATAGCCGTGGGGTGACTTCCCTGGT
CACCAAGGCAGTGCATGCATGTTGGGGTTTCCTTTACCTTTTCTATAAGTTGTACC
AAAACATCCACTTAAGTTCTTTGATTTGTACCATTCCTTCAAATAAAGAAATTTGG
TACC


Sequence ID 1360


TGCGCAGACCAGACTTCGCTCGTACTCGTGCGCCTCGCTTCGCTTTTCCTCCGCAA
CCATGTCTGACAAACCCGATATGGCTGAGATCGAGAAATTCGATAAGTCGAAACTG
AAGAAGACAGAGACGCAAGAGAAAAATCCACTGCCTTCCAAAGAAACGATTGAACA
GGAGAAGCAAGCAGGCGAATCGTAATGAGGCGTGCGCCGCCAATATGCACTGTACA
TTCCACAAGCATTGCCTTCTTATTTTACTTCTTTTAGCTGTTTAACTTTGTAAGAT
GCAAAGAGGTTGGATCAAGTTTAAATGACTGTGCTGCCCCTTTCACATCAAAGAAC
TACTGACAACGAAGGCCGCGCCTGCCTTTCCCATCTGTCTATCTATCTGGCTGGCA
GGGAAGGAAAGAACTTGCATGTTGGTGAAGGAAGAAGTGGGGTGGAAGAAGTGGGG
TGGGACGACAGTGAAAT


Sequence ID 1361

TATAAATACACTCCGGGATGATTTACCCCCGGAGGTCAGCTAGTAAAATACATGAG
TAGAATTCCTTAAAGTATGTGATAATTGCTCATCACTATCCAAGTGTGACATAAAT
CATAAAAAGAATTGACAAAATCAGGGTCGCAAAGAGAATTGAAAAAAATCTGTCAC
AACCAAAATTTAAATTGACCTCTGTCCTAGAGTATGAGAGCCACACTGAACAGAAA
AACCAGATAAATCTTTTATAAAATATTCATTTGCAGCCCCATTAACGTTGCTTGTC
ACCCCACCTCCCCATGTCCTTGGACAAACTGAATGTATAGTAACATCATCCCAGGC
CAGGCGCGGTGGCTCATGCCTGTAATCCCAGCACTTTGTGAGGCTAAGGCAGGCAG
ATCAGGAGGTCAGGAGTTCAGGACCAGCCTGGCCAAAAAGGTGAAACTCCGTCTCT
ACTAACAATACAAAAATTAGCTGGGTGCGGTAGTAGGCGCCTGTAATCCCAGCTAC
TCGGGAGGCTGAGGCAGGAGAATTGCTCAAACCCGGAAGGTGGAGGTTGCAGTGAG
CTGAGATCGTGCCACTGCACTCCAGCCTGGGTGACAGAGCAAGACTCTGTCTCGGG
GAGGGGGGTGGCGGAGATAAAGAAATAACATCATCTTATACTGTCAAGCTCAAGGT
GTCTGCAGCCTTATCTTCAGGGGAAGTTGTGTCTTTCTCAGGGAAGATACAGATTT
CAATTTAGAGCAAGACAGAGAGAAGTTACATTCAGAGAGGAAAATGCAGTAGTCTA
ACTG

Sequence ID 1364

GCGGCCGCGCTCTTTTTCAATTTTTTAAAAAGAAGTTTGTTTTTCCATTTCAGTAATTT
CTGCTTTGATCTTCCTTATGTCCTCCTATTGAGTTGATCAGCTTTCTTTATTCTTG
CCTTTTCTCCTCTGTGTGCCCTTTCTATTAACGTATTTACCCTTAGGCTGGGCACA
ATGGCTGATGCCTGTAATCCCTGCACTTTGGGAGGCCGAGGCAGGTGGATCACCTA
AGGTCAGGAGTTCAAGACCAGCCTGGCCAACATGGTGAAACCTGGTCTCTACTAAA
AACACAAAAATTAGCCAGGCATGGTGGTGTGCACCTGTAATCCCAGCTACTCAGGA
GGCTGAGGCAGGAGAATTGCTTGAACCTGGGAGGCGGAGATTGTGCCAAAGCACTC
CAGCCTGGGCAACAAAATGAGACTTTGTGTC

Sequence ID 1365

CACCAGGCTGTCTTCAGATACTTCATACAGAAATGAGCCTCCCTGTGGGGTCCTCT
TCCCTCCTTCAGCCTGTCCATCAACACAGCATTGCGGGATCCTTACCATGGCATCC
AGCCCTGGAGATGCTTCAGGAAAGTTGCAGGTCCATGCTGCAGGACAGGCTCAGAT
CAGCAGAGACGCATCTCACATCGGGCTGTGAAATTCAAGTTGAGCTGCAATTGGCA
ATGAGAA

Sequence ID 1366

GTTATTCACTGAGACCGTGCCCCGGTTATGAGGTTGTACCAGAAAGCAAGTATTCA
CTATGCACACTATTCACCGCTCACCCTAGCATTGAAGCCAGCCTGTAGCCTGAAAG
CCTTTGCTTTGAGGGCAGGTCTTTCCCCAAAATGCAGACACGAAGGTGCAAAGTGA
AGCTGCCAGTCTTGCAAAAGATGTAACTTGTCACGAAGGCCACGAGTGGCAGGGAG
AGCTGTCCCACATTTGCGGAAGTGGCTATGTGAGGACGGGGGAGGCGGGTCCCTTA
GAGATGAGACAATCATAAGGGGAGATATCAGAGAAAATCGTAAGGGGAGCAGATGG
TTGTCAAGAGAATAGGCTGACCATCGAAGGACTGGCAGAAGCTTTCAGAAAACCAC
TGGACGGCTGGGCACAGTGGCTTAGGCCTGTAATCCCAGCACTTTGGGAGGCTGAC
GCAGGTGAATCACTTGAGGTCAGGAGTTCCAGACCAGCCTGGCCAACATGGTGAAA
CCCCATCTCTACAGAAAATATAAAAATTAGCCAGGCGTGGTGGCACAAGCCTAGAA
TCCCAGCTACTTGGGAGGCTGAGGCAGGCGAATGGCTTGAACCCAGGAGTCAGAGG
CTGCAGTGAGTCGAGATTGTTCCACTGCACTCCAGCCTGGGTGACAGTGCAAGACT
CCTTCCAAAAAAAAA

Sequence ID 1367

TTCGTGAGTGATGGCGTCCCGGGTTGCTTGCCGGTGCTGGCCGCCGCCGGGAGAGC
CCGGGGCAGAGCAGAGGTGCTCATCAGCACTGTAGGCCCGGAAGATTGTGTGGTCC
CGTTCCTGACCCGGCCTAAGGTCCCTGTCTTGCAGCTGGATAGCGGCAACTACCTC
TTCTCCACTAGTGCAATCTGCCGATATTTTTTTTTGTTATCTGGCTGGGAGCAAGA


TGACCTCACTAACCAGTGGCTGGAATGGGAAGCGACAGAGCTGCAGCCAGCTTTGT
CTGCTGCCCTGTACTATTTAGTGGTCCAAGGCAAGAAGGGGGAAGATGTTCTTGGT
TCAGTGCGGAGAGCCCTGACTCACATTGACCACAGCTTGAGTCGTCAGAACTGTCC
TTTCCTGGCTGGGGAGACAGAATCTCTAGCCGACATTGTTTTGTGGGGAGCCCTAT
ACCCATTACTGCAAGATCCCGCCTACCTCCCTGAGGAGCTGAGTGCCCTGCACAGC
TGGTTCCAGACACTGAGTACCCAGGAACCATGTCAGCGAGCTGCAGAGACTGTACT
GAAACAGCAAGGTGTCCTGGCTCTCCGGCCTTACCTCCAAAAGCAGCCCCAGCCCA
GCCCCGCTGAGGGAAGGGCTGTCACCAATGAGCCTGAGGAGGAGGAGCTGGCTACC
CTATCTGAGGAGGAGATTGCTATGGCTGTTACTGCTTGGGAGAANGGCCTAGAAAG
TTTTGCCCCCGCTGCGGCCCCAGCANAATCCAGTGTTGCCTGTGGCTGGAGAAAGG
AATGTGCTCATCACCAGTGCCCTCCNTTACGTCAACAATGTCCCCCACCTTGGGAA
CATCATTGGTTGTGTGCTCAGTGCCCGATGTCTT


Sequence ID 1368

CAGTGAGCCAAGATCACACCACTGCACTCCAGCCTGGACAACAGAACGAGACTCCA
TATCAAAAAAATTAAATTAAAATATAATAAATTTCTTGCCGGGCGCAGTGGCTCAC
ACCTGTAATCCCAGCACTTTGGGAGGCCGAGGTGGGCGGATCACGAAGTCAGGAGA
TTGAGACCATCCTGGCTAATACAGTGAAACCCCGTCTCTACTATAAATACAAAAAA
TTAGCTGGGCATGGTGGCGGGCGTCTGTAGTCCCAGCTACTCAGGAGTCTGAGGCA
GGAGAATGGTGTGAACCCGGGAGGCGGAGCTTGCAGTGAGCCGAGATCGTGCCACT
GCAATCCAGCCTGGGCAGCAGAACGAGACTCCATCTCAAATAAATAAATAAATAAA
ATGAATTTCAGCTAGAAGAGCCTTATTCCATTTTCCTTTTTATTAAACATCTGGCA
TAAGTTGGTAAGTATGTGAAGTTTATCATATATTCTTATGCGAATTATTATTTTCG
CCTTTTTTTTTATAATTCTGTCTGGGATTTGAATAGTAGAGTTTGAATTCAGGAAG
GACACCTGTGATAGGACAATAAAAT

Sequence ID 1369
CTGATTGCAAAAACATTACAACTCAGTACTGCGGCTTTCATTCAAATAGGTTATAT
GTATAAACTGAGGTTCAACAATATTGTATTTGAGATGGGAAAGTTAAAGAAATGCA
ATAATGTAAATAATACTTAAGAAAATAAGATCTCAGGAAACTGTGTATACTCTGTA
CTTTTATGCAACTTTATCAGATCATTTCAGTATATGCATCAAGGATATAGTGTATA
TGACATGAACTTTGAGTGCAAAAACTGTACTATGTACCTTTTGTTTATTTTGCTGT
CAACATCTAAATAAAGGTTTTTTTG

Sequence ID 1370
CGAAAGGACTACAGAGCCCCGAATTAATACCAATAGAAGGGCAATGCTTTTAGATT
AAAATGAAGGTGACTTAAACAGCTTAAAGTTTAGTTTAAAAGTTGTAGGTGATTAA

AATAATTTGAAGGCGATCTTTTAAAAAGAGATTAAACCGAAGGTGATTAAAAGACC
TTGAAATCCATGACGCAGGGAGAATTGCGTCATTTAAAGCCTAGTTAACGCATTTA
CTAAACGCAGACGAAAATGGAAAGATTAATTGGGAGTGGTAGGATGAAACAATTTG
GAGAAGATAGAAGTTTGAAGTGGAAAACTGGAAGACAGAAGTACGGGAAGGCGAAG
AAAAGAATAGATAAGATAGGGAAATTAGAAGATAAAAACATACTTTTAGAAGAAAA
AAGATAAATTTAAACCTGAAAAGTAGGAAG

Sequence ID 1371

GTCCAGNAGAAAGTTCAGTGACTTGTCCAGAGCTGCAGGTCTTAAGAGGCTGAAAT
CTCGCCTCTGCCTCGAGGCTGCGGTTCCACTGACCCATACTACTTGCCTTCAGGAA
AGAGAAATGGTGTAGGAAGGCTGTGGATGAAGACGCTTACATTCATGAAGGATTTG
GATAGGCGAACATGAGCTTTTCCACCAAATTTCAGAATTTTAAGAAATGCCTTAAA
TTATTTCTTAAAAATCAATTTGGGGCAGACGAGAAGTTCTGATAATAGTTTTTAGG
GAACATGATAAAATTCTGACCTTAGAAGTGGTATACCAGTTTGAGAAGAAGAACAA
GCTATAAACGGTGTAGATAACATTCACGGCTATTTAAGAAAGAGTTACTAAGGGAA
ACCAGAATGACTTAAGAGTGTTACTCTTCTTTTTCTGAGAGAACAATAGCATCATC
TCAGAAAGCCTTTCATGCCATTAATAGGTAAGAATCTGGGCTTCTTGGACCATGGG
TTAGACTTTCTTACAAAACCATAATATGCATTTCCTAGCAAAATTTATGCTATTAC
ATTTCCTTATCTCAACAAAGACTGGTAAATTCAGTACTTATTCCTCAATTTTCCTA
CCCTTAAAATGGGGATATTCTGCCTCTCCAAGGAATGCTGGGAACAAGCAAGTCCT
CATGTTAGGGGTCTTTGAGTTTTCATGGAAGTTTAGGTTATTTATATGATGACATA
GTTGTCAACTTACTTTCAGGATGGACTTTTCTTTTGTGAGTTTGTGACCTAAATAC
AATAGTTGTTATGCATGTCCAGTTTATGGAAGTACCACTGCAATANCAG


Sequence ID 1372

CAGTGCAGCCAAGTATCACACCACTGCACTCCAGTCCTGGACAACAGAAACGANTA
CTCCATATCAAAAAAATTAAATTAAANGATAATAAATTTCTTGCCGGGCGCAGTGG
CTCACACCTGTAATCCCAGCACTTTGGGAGGCCGAGGTGGGCGGATCACGAAGTCA
GGAGATTGAGACCATCCTGGCTAATACAGTGAAATCCCCGTCTCTACTATAAATAC
AAAAAATTAGCTGGGCATGGTGGCGGGCGTCTGTAGTCCCAGCTACTCAGGAGTCT
GAGGCAGGAGAATGGTGTGAACCCGGGAGGCGGAGCTTGCAGTGAGCCGAGATCGT
GCCACTGCAATCCAGCCTGGGCAGCAGAACGAGACTCCATCTCAAATAAATAAATA
AATAAAATGAATTTCAGCTAGAAGAGCCTTATTCCATTTTCCTTTTTATTAAACAT
CTGGCATAAGTTGGTAAGTATGTGAAGTTTATCATATATTCTTATGCGAATTATTA
TTTTCGCCTTTTTTTTTTATAATTCTGTCTGGGATTTGAATAGTAGAGTTTGAATTC
AGGAAGGACACCTGTGATAGGACAATAAAATCTA


Sequence ID 1374

GAAAGCACATATGATATACATGTGTGTCATATGTATTATTTTGTTTGCCATCTGAG
TCTTCAAAATTTGTTACAGAATACCTGCATATTAATATTTCAAGGTATGGATTAAT

Sequence ID 1378

CTGAGTATTAACTAAAAAAAAAAAAAAAAAAAAAAAAAAAA

Sequence ID 1380

CCAAACCCAACTGGTCCAGTAGGATACTCACCTTACAGGGGGCGTCTCAAGAGTCT
CACAGTTCCCTTGGGTCTTAAGAGACTCACTGTTGGACCAGGCGTGGTGACTCACG
CCTGTAAAACCAGCACTTTGGGAGGCCGAGGCGGGCGGATCAGTTGAGGTCAAGAG
TTCAAGACCAGCCTGACCAAGGTGCTGAAACCCCGTCTCTACTAAAAATACAAAAA
TTAGCCAGGCATGGTGGTGTGCGCCTGTAATCCCAGCTACTCCAGAGGCTGAGGCA
GGAGAATCTCTTGAACCCAGGAGGTGGAGGTTGCAGTGAGTCGAGATCATGCCACT
GCACTCCAGCCTGGGTGACAGAGCGAGACTCCGTCTTAGAAAAAAAAAAAAAAAAA
AAAAGAACCTCACAGTTCAGCAGGGTTCTAGCATGAGACAATGAGGACAAGGGTAG
GTGAGCAGGTGGAAAGAGTGAGAACAGGTCAATTGTGATGGAGAAAATAATAAAGA
CAGAAAAGGCAGAAGACTGCCTGGCAGAAGACCTGTCCCAGCAGATACAAAAATAC
AGACAACAGGAGCCAGCATAGACCCTTGACCTGTGTAAGTCTTTCTCAGGCCTTCT
TTTAAGTAGAAACATGCCTTTGAAAAAAGTTTTAATAAACAGGAAAATCATAAAT
CCCTATTTACATAAATAATATATCCTGGTCTTATTCTTAAAACCATTGATTTTTCA
CGGCTCATTAANAAAGCTGGGCGAGGTGGCTCACGCCCGTCATCCTAGCACTTTGG
GAGGCCGAGGCGGGCANATCACAAGGTGAGGAGTTGGGAGACCAGCCTGACCAACA
CGGTGAAACCCAGTCTCTACTAAAAATACAAAAATTANCTGGGGGTGGTGGTGTGT
GCCTGTAATCCAAGCTACTCGGGAGGCTGAGGCAGGA

Sequence ID 1382

CTTACTACCTCCAACATGAAACAAGCAGCCCCGCACTTCTCGAAGGTCTGAGTTAC
TTGGAATCGTTTTACCACATGATGGACAGAAGGAATATTTCAGATATCTCTGAAAA
CCTCAAGCGTTACCTTCTTCAGTATTTTAAGCCAGTGATTGACAGGCAAAGCTGGA
GTGACAAGGGCTCAGTCTGGGACAGGATGCTCCGCTCGGCTCTCTTGAAGCTGGCC
TGTGACCTGAACCATGCTCCTTGCATCCAGAAAGCTGCTGAACTCTTCTCCCAGTG
GATGGAATCCAGTGGAAAATTAAATATACCAACAGATGTTTTAAAGATTGTGTATT
CTGTGGGTGCTCAGACAACAGCAGGATGGAATTACCTTTTAGAGCAATATGAACTG
TCAATGTCAAGTGCTGAACAAAACAAAATTCTGTATGCTTTGTCAACGAGCAAGCA
TCAGGAAAAGTTACTGAAGTTAATTGAACTAGGAATGGAAGGAAAGGTTATCAAGA
CACAGAACTTGGCAGCTCTCCTTCATGCGATTGCCAGACGTCCAAAGGGGCAGCAA

```
CTAGCATGGGATTTTGTAAGAGAAAATTGGACCCATCTTCTGAAAAAATTTGACTT
GGGCTCATATGACATAAGGATGATCATCTCTGGCACAACAGCTCACTTTTCTTCCA
AGGATAAGTTGCAAGAGGTGAAACTATTTTTTGAATCTCTTGAGGCTCAAGGATCA
CATCTGGATATTTTTCAAACTGTTCTGGAAACGATAACCAAAAATATAAAATGGCT
GGAGAAGAATCTTCCGACTCTGAGGACTTGGCTAATGGTTAATACTTAAATGGTCA
ATAGAAAAGTAGGCTGGGCGCGGTGGCTCACGCCTGTAATCCCAGCACTTTGGGA
```

Sequence ID 1387

```
AAAATTTTTTTTTTTTTTTTTTTTTTTTTTTTTTTTTTTTTTTTTTTTTTTTTTTT
TTTTTTTTTTTTTTTTTTTTTTTTTTTTTTTTTTTTTTTTTTTTTTTTTTTTTCAGT
GTTAAAGTAGGTTTGTCGACGCGGCCACGAATTTCCCGGGGACCAA
```

Sequence ID 1389

```
TTTTTTTTTTTTTTTGGGAGTCAGTTTTCTTTTCTTTTCTTTCTTTTTTTTTTTTT
GNTTTTCGGAAACGGAGTCTCGCTTTCTCGCCCACTCTGGAGTGGNGCAGTGGGGN
GGTCTCAGCTCACCACAGCCTCCACCTCCTGGGCCCAAGCGATCCTNTCACCTCAG
CCTCCTGCGTAGCTGGGACTACAGGCGTGCACCACCATTCCCAGGTAATTTTTGTA
TTTTTTGTANANACAGGGTTTCACTGTTGTTGCCCAGGCTGGTCTCGAACTCCTGC
TTCAGTCTGCCANAATGCTGGATTCTAGGCGTGAGCCACCGNGCCTGGCCCAAAAG
TTACTTTTCTTACAGAAGCAAAGCTTTAATGCATTTTACTGAATGCTTATAGCTTT
GTAGATACTGAAAAGAGTATGAGCGTCACATACAGACACATNTAACAGCACTGCCT
CCAACCAGCCCCTACCCACTGGTCAGGNGAGTAANAATCAAAATTCTTTTCTGNGA
GTGGAACGGAAATTTCATCTCTCCTCCTCAGGCAAGTAGTTAANAGGCTGGNGGGA
GTCATGGCCCCATTTTGTTCAAAATACAAGCTCCACAGGAACAAAAGGCTGAACTG
CTCACCTCCCAACTGATGAACCTCGTCTTTGTTCCATGTCAAAGGGGCCTTTGTGT
TACTGCAGCAGAAACTCCAGCTATCAAACCATCAGGCACCAAAAGTAAAACTCCTT
TCTCTAAAAAGACCTCTCTTTACCTGAGCCTTTCAATGCATCTTTGCCCCCANATA
ATCCTGGATGAGATAATCCCCAGAGGAANACCAGCGCTTGCCTAGTGAAATTATAC
TATGAGACAAGGGTAAAAGACCTCAAANACCGGGTTGGCAGGTAAGGGAGTAGGGN
```

Sequence ID 1390

TCNGTGGCACCCGTTTCCGGCACCTTCAGACTCTGAAGAGCCACCTGCGAATCCAC

ACAGGAGAGAAACCTTACCATGTACGTAAGCCTCTTGAGGCCGCTCTCTGACCTGC

GGGGATGTGGAGGGCAGGGAAGGAGGTGGAGCGCAGGGAAGGAGGTGGAGCAGGGA

GGCAGTGGAACTGTTTGCTCCCATCTCAAGCACACAGTGGGGCAACCACTACGCTA

ATGGTTGGAAGACCTAGATCTGGGCCCAATGGCCAGACACCCTGCTTGACCTTGGC

CCAAGCATTAGGGGACTCATCTTTAAAATGAGGGTATGGGACTAGATGATCTGGGC

CTTAGGAGAGGAGT

Sequence ID 1391

CGGCTNCTACCCTGCGGAGATCACACTGACCTGGCAGTGGGATGGGGAGGACCAAA

CTCAGGACACCGAGCTTGTGGAGACCAGGCCAGCAGGAGATGGAACCTTCCAGAAG

TGGGCAGCTGTGGTGGTGCCTTCTGGAGAAGAGCAGAGATACACGTGCCATGTTCA

GCACGAGGGGCTGCCGGAGCCCCTCACCCTGAGATGGAAGCCGTCTTCCCAGCCCA

CCATCCCCATCGTGGGCATCGTTGCTGGCCTGGCTGTCCTGGCTGTCCTAGCTGTC

CTAGGAGCTATGGTGGCTGTTGTGATGTGTAGGAGGAAGAGCTCAGGTGGAAAAGG

AGGGAGCTGCTCTCAGGCTGCGTCCAGCAACAGTGCCCAGGGCTCTGATGAGTCTC

TCATCGCTTGTAAAGCCTGAGACAGCTGCCTGTGTGGGACTGAGATGCAGGATTTC

TTCACACCTCTCCTTTGTGACTTCAAGAGCCTCTGGCATCTCTTTCTGCAAAGGCA

TCTGAATGTGTCTGCGTTCCTGTTAGCATAATGTGAGGAGGTGGAGAGACAGCCCA

CCCCCGTGTCCACCGTGACCCCTGTCCCCACACTGACCTGTGTTCCCTCCCCGATC

ATCTTTCCTGTTCCAGAGAAGTGGGCTGGATGTCTCCATCTCTGTCTCAACTTCAT

GGTGCGCTGAGCTGCAACTTCTTACTTCCCTAATGAAGTTAAGAACCTGAATATAA

ATTTGTTTTCTCAAATATTTGCTATGAAGGGTTGATGGATTAATTAAATAAGTCAA

TTCCTGGAAGTTGAGAGAGCAAATAAAGACCTGAGAACCTTCCANAATCCG

Sequence ID 1392

TGAAACAAAATGAATTTNTATGGGTAAGAGAGGGTAATATTTTAGAGTTGTGTTAC
AAAACTACAAATTTTTATTAAATTAATAAATCAGAATACTAAATCCATGTGTTTTT
TTCTTTCTTAAAAAATATCTTTTGGCTGGGCACGGTAGCTCATGGCTGTAATCCCA
GCACTTTGGGAGGCTGAGGTGGGTGGATCGCCTGATGTCAGGAGTTCAAGACCAGC
CTGGTCAACATGTTGAAACCCCATCTCTACTAAAAATATAAAAATTAGCCGGTGTG
GTGGTGGGCGCCTGTAATCCCAGCTACTCAGGAGGCTAAGGCAGGAGAATTGCGTG
AACCCAGGAGTTCAGTGATGTAGCGGGGAGCTGAGATTGTGCCACTACACTCCAGC
CTGGATGACAGAGTGAGACTCCATCTCAAAAAAAAAAAAAAAAAA

Sequence ID 1394

GCATAATGTGAGGAGGTGGAGAGACAGCCCACCCCCGTGTCCACCGTGACCCCTGT
TCCCATGCTGACTTGTGTTTCCTCCCCAGTCATCTTTCCTGTTCCAGAGAGGTGGG
GCTGGATGTCTCCATCTCTGTCTCAACTTTATGTGCACTGAGCTGCAACTTCTTAC
TTCCCTACTGAAAATAAGAATCTGAATATAAATTTGTTTTCTCAAATATTTGCTAT
GAGAGGTTGATGGATTAATTAAATAAGTCAATTCCTGGAATTTGAGAGAGCAAATA
AAGACCTGAGAACCTTCCAGAAAAAAAAAAAAAAAAAAAAAAAAAAAAAAAAAAAAA
AAAAAAAAAAAAAAAAAAAAAAAAAAAAAAA

Sequence ID 1395

CTTACCATGTCAGTGCACAGAAATGCTGTCTTGGGATGTAGGAAAAATAAATCCAC
AAAAGCTACCAAGTTTGAAGGGGACCATGAGTCTTCAGGCTGGAGCTTCCAAACCA
GATGAAAACCCCACAATTAACCTGCAGTTTAAGATCCAGCAGCTGGCCATTTCTGG
ACTCAAGGTGAATCGTCTGGATATGTATGGAGAAAAGTACAAACCCTTTAAGGGCA
TAAAATACATGACCAAAGCTGGGAAGTTCCAAGTTCGAACCTGAAGGGAGCATTTG
CTGAGGGAATAGTCTTGCACATTTTTTCATTTCTTACTTGTCTAAAAGTAAAAAAA
AATATCAGCCTGTCTCCTAGGTCAGTCCCCTCCTGGACCCACCCGCTCCCTTTTTT
CCTTAGCCTTCAGTGCCATGGAACTAATCAAGGGAGGAAAAGGTCACCAGGGAGAA
CTGGACAGAACTGAAACACAGCAACACCAGTTCTCAAGGACAAGGTGTGTGATGGG
GGTAGGAAGCTTGGTGCTTATGTAACCATTTTAAACGTGGTTTCTATAGGAAAGAC
CAACATTTGTTTAGCTTGCTTGGCTTTAATTATCTAAAGCCAATGAAAGACTTCTT
TGTTGATTTTTTAAGATAGAAAGATT

Sequence ID 1396

CAAACACTATGTTATTTTATGAANAAGACTTGAACATCTATGGATTTTGGTATTTG
CAAGGGGTGAATGGGGTATTTGCAAGCAGTGAATGAGGAGGCCTGGAACCAATCTT
CTGCTGATATTGAGGCACAACTGAAAAAGGTATATTACTTAAATCTCTTATTGTAT
TGTAAACTGTATAAGTAATGAAATTAAAAGGCAGAAATTGTCAGACTGAATAAAAT
GAAAAGACCAAACAATATGCTGCTTACAAGAAACACAATTCAAATATAAGGACACA
ATTAGTTTAAAGGAAAAGAACTGGAAAAGATATACCATGATAACACAAGTCAGAAG
AAAGCTGCTGTGGATATATTAATATGAGATGTAGATTTCAGAGCAGTGAATATTGC
CAGGCATAAAGAAAGTTATTACATAATAATTAAGGTATCAGTTCATCAAGAAGATG
TAATAACCCTAAGTATTTATACAACTAATATCAGAGCTTCAAAATACATGAAGCAA
AAACCAGTGGAATTGATAGGAGAAACACACAATTACACAATTATAGTCAGAATTTT
CAACATATCTTTCTCAATGGAGAAACAACTAGACAGGAAATCATTAAGGATATAG
ATGATTTAAATTATATGATCAACTACCTGGACGTAATTGGCATTTATGGAACACTG
CACCACCAACAGCAGAGTACATATTATTTTCAAGTACACAGAAAACAGTTACCAAT
ATAGACCATTTTCTGGGTCATAAAACACATCTCAATAAATGTAAAACAATTAATGT
TATATAAAGTATGTGCTCTGACCNCAAAGGAATTAGAGATCAATAAAAGAACATCT
TTGAAAAATCTCACNTATTTAAAAACTAATAACTCACTTCTAAATAACTCCTGTNT
CAAGAGAATNAAANGG

Sequence ID 1397

CCCAGCCTCACTGCGCCCCGTCAGGCCAGGCAGCTGCCCTCAGGGTCTGCCAAGGT
GGGGGTCAAGGGCCATGGGGGCAGGTAGCTCTGCCTGCAAAGCCCACAAGCATGTC

278

AGATCACCTGGGCTGCAGACAGACAAACACCTGAGCTGTTCTGAATACCTTCAGGT
TCCTGGCCTCGCTGAGCAAGTGCAGAAATTTTTACCTTCAAGGATCAGGGTTTTTC
TGTTTGTTTGTTTTTTAACACACACATATGTGAACAAAGAGTATGCGTTTGTACTG
GCAGAAGAAGCGTCTGGTAAGACAACCAGCAAGTTAACAATGGTCACCTCCAGAAA
TGGGCTGGGTAAACCAAAGAATTTTTTTGTTTTTGTTTTTTTTTGAGTCAGGGTCTA
GCTCTGTCACCCAGGCTGGAACGCACTGGTGTGATCACGGCTCACTGCAGCCTTGA
CCTCCCTGGCTCAAGCAATCCTCCCAGCTCAGCCTCCTGAGTCGTTGGGACTACAG
GCACGTGCCACCACGCCTGACACATTTTTTAAATTTTTGTAGAGACAGTGTTTCAC
CATGTTGCCCAGGCAGGTCTCAAACTCCTGGGCTCAAGTGGTCCTCCAGCTTCAGC
CTCCCAAAGTGCTAGGATTATAGGTGTGAGCCACAGTGCCCAGCCCCGTAGTGGAG
AATTTCTGTTGAATGAACCAAAAGCAACTGCCAACCTCTCCATGCACCATGTGTTT
CAGAGGAGAAAGCACAGTGAAGAATGCAGTGTGTTCTGAGGTCCTGTCACCCCTGA
GGCTGTGTGTGTCCTTTGCCAAATTAAAGAGTCTTACTGAATGCGGTGCATCCAGG
AGACAGGCCNAGGTTTGGACTGGTAAAAAAAAA


Sequence ID 1399
CAGACACCTGGNAGAACGGGAAGGAGACGCTGCAGCGCGCGGACCCCCCAAAGACA
CATGTGACCCACCACCCCATCTNTGACCATGAGGCCACCCTGAGGTGCTGGGCCCT
GGGCTTCTACCCTGCGGAGATCACACTGACCTGGCAGCGGGATGGCGAGGACCAAA
CTCAGGACACCGAGCTTGTGGAGACCAGACCAGCAGGAGACAGAACCTTCCAGAAG
TGGGCAGCTGTGGTGGTGCCTTCTGGAGAAGAGCAGAGATACACATGCCATGTACA
GCATGAGGGGCTGCCGAAGCCCCTCACCCTGAGATGGGAGCCATCTTCCCAGTCCA
CCGTCCCCATCGTGGGCATTGTTGCTGGCCTGGCTGTCCTAGCAGTTGTGGTCATC
GGAGCTGTGGTCGCTGCTGTGATGTGTAGGAGGAAGAGTTCAGGTGGAAAAGGAGG
GAGCTACTCTCAGGCTGCGTCCAGCGACAGTGCCCAGGGCTCTGATGTGTCTCTCA
CAGCTTGAAAAGCCTGAGACAGCTGTNTTGTGAGGGACTGAGATGCAGGATTTCTT
CACGCCTCCCCTTTGTGACTTCAAGAGCCTCTGGCATCTCTTTCTGCAAAGGCACC
TGAATGTGTCTGCGTCCTTGTTAGCATAATGTGAGGAGGTGGAGAGACAGCCCACC
CTTGTGTCAACTGTGACCCCCTGTTCCCATGCTGACCTGTGTTTCCTCCCCAGTCA
TCTTTTTTGTTCNCAATAGGTGGGGCCTGGATGTCTCCATCTCTGTNTCA

Sequence ID 1440

TTATAAGGTACTTTTAAGGTATTTTAGTTGTCTTAGTCTATATTTCTGTACTCACC
TTTCTTTATCCACTCATCAGTTGATGGGCATGTAGGTTGGTTCCATATCTTTGCAA
TTCTGAATTGTGCTGTGATCAGGTGTCTTTTTAGTATAATGATTTACTCTCCTTTG
GGTAGATACCCAGTAGTGGGATTGCTGGATCGAATGGTTTTTATAATTTTCTATTT

TACCACAGTTTCTCTCTGCATTTTTCCTCTTTGACCACTAACCATGTGAAATTCTC
ATATTGACCTTTATAATGATCATGAACTCTTAGTATCATTGGGAAGGCCACATTTG
CCACTTATGATTGTAAACCTTATCCTCCATTTTTCCTGTTATTGTTGGTGCAAAAA
GCACCTATTATACCAGGACTTTAAAAATCAGTCTGATAAGTCTTTGATAAGTCTAA
TAATAATAACTGATAAGTCCATTGAATTTGCTTCTGATTACTTTTTCTTTAGTAGC
TAAACATGTATGTACTCCTATGATTACAATGAACACTCCTCTCCATTTAAATTAAT
TATTTACATTGATGAAATAGCAAAATGTTAATGACTAAATACTGTCTTGGTTTTTT
CGTTCCAGGTCAGTCAATATTAACTTCTTATAATTTTCTTTTTTTTCTTT

Sequence ID 1447

GCAAGGACTAACCCCTATACCTTCTGCATAATGAATTAACTAGAAATAACTTTGCA
AGGAGAGCCAAAGCTAAGACCCCCGAAACCAGACGAGCTACCTAAGAACAGCTAAA
AGAGCACACCCGTCTATGTAGCAAAATAGTGGGAAGATTTATAGGTAGAGGCGACA
AACCTACCGAGCCTGGTGATAGCTGGTTGTCCAAGATAGAATCTTAGTTCAACTTT
AAATTTGCCCACAGAACCCTCTAAATCCCCTTGTAAATTTAACTGTTAGTCCAAAG
AGGAACAGCTCTTTGGACACTAGGAAAAAACCTTGTAGAGAGAGTAAAAAATTTAA
CACCCATAGTAGGCCTAAAAGCAGCCACCAATTAAGAAAGCGTTCAAGCTCAACAC
CCACTACCTAAAAAATCCCAAACATATAACTGAACTCCTCACACCCAATTGGACCA
ATCTATCACCCTATAGAAGAACTAATGTTAGTATAAGTAACATGAAAACATTCTCC
TCCGCATAAGCCTGCGTCAGATTAAAACACTGAACTGACAATTAACAGCCCAATAT
CTACAATCAACCAACAAGTCATTATTACCCTCACTGTCAACCCAACACAGGCATGC
TCATAAGGAAAGGT

Sequence ID 1448

GGCCACCGGGTGCAAGGTCAGGGCTGGGGTGGAGGCTGGGAAGCCCAGGGCTTGGC
CCACTGTGGCCGCCTTGTGTGGTCACTGCTTTCCTGGGCCTGCTGTGAGCTCCCTC
TAGGACCCCAGGCCTGTCTGGTGGGTCACTGTGACCACCACCTTGCACAGCACCTG
GCGCGTGGCAGGTGCTCAAACATTACTTGTTTCGGAATGAACTTCATCTTGCTCTT
GGCTTTTTGACTAATGCTGTGGAACATCTGACTAATTAGTGACTCTTTGGGGCCCC
CAGTTTCCCAGCTATAAAGTGGTAATATTAAGATAATAATTCGGCCGGGCGCGGTG
GCTCACGCCTGTAATCCCAGCAGCACTTTGGGAGGCCGAGGTGGGCAGATCACGAG
GTCAGAAGATCGAGACCATCCTGGCTAACACGGTGAAACCCCATCTCTACTAAAAA
TACAAAAAATTANCCGGGCGTGGTGGCGGGCGCCTGTAGTCCCAGCTACTCANGAG
GCTGANGCAGGAGAATGGTGTGAACCCGGGAGGCAGAGGTTGCAGTGAACCAAGAT
CGNNCCACTGCACTCCAGCCTGGGCAACAGAGCGAGACTCCATCTTAAAAAA

Sequence ID 1449

AATCAGGGCCGCAGTGTGTTCTGCGCCTGCCCAGAGCTGACTCCTGATTTAACCGC
TGGCGTAACCGCGGGTTGCACGCATGCGTGCTGAAAAGCCTTTCACCCTCACGTGG
TTTCTTTTTTAACCAGTCATCAAGCGAGGCTCGCGCGCAGGCCCCGCGTTGGAAAA
TGGCGGGGAAGCTGAAACCTCTGAATGTGGAGGCGCCAGAAGCTGCTGAGGAGGCT
GAAGGTAGTGAGGGCAAGTGGGCTGCACTCCTTTCTCTCCAACCAGGGCAGAAAGG
AGGGAGGATTCGTCCCATTACAATAATGAAATAATGATATTCTAATTTTTTTAAAT
AAAATGTTAAGCCTTTTGTTATTGAA

Sequence ID 1450

GGAAANCATGAGGCTTCGGGAGCCGCTCCTGAGCGGCAGCGCCGCGATGCCAGGCG
CGTCCCTACAGCGGGCCTGCCGCCTGCTCGTGGCCGTCTGCGCTCTGCACCTTGGC
GTCACCCTCGTTTACTACCTGGCTGGCCGCGACCTGAGCCGCCTGCCCCAACTGGT
CGGAGTCTCCACACCGCTGCAGGGCGGCTCGAACAGTGCCGCCGCCATCGGGCAGT
CCTCCGGGGAGCTCCGGACCGGAGGGGCCCGGCCGCCGCCTCCTNTAGGCGCCTCC
TCCCAGCCGCGCCCGGGTGGCGACTCCAGCCCAGTCGTGGATTCTGGCCCTGGCCC
CGCTAGCAACTTGACCTCGGTCCCAGTGCCCCACACCACCGCACTGTCGCTGCCCG
CCTGCCCTGAGGAGTCCCCGCTGCTTGGTAAGGACTCGGGTCGGCGCCAGTCGGAG
GATTGGGACCCCCCCGGATTTCCCCGACAGGGTCCCCCANACATTCCCTCAGGCTG
GCTCTTCTACGACAGCCAGCCTCCCTCTTCTGGATCAGAGTTTTAAATCCCANACA
GAGGCTTGGGACTGGATGGGAGAGAAGGTTTGCGAGGTGGGTCCCTGGGGAGTCCT
GTTGGAGGCGTGGGGCCGGGACCGCACAGGGAAGTCCCGAGGCCCCTCTAGCCCCA
AAACCANAGAAGGCCTTGGAGACTTCCCTGCTGTGGCCCGAGGCTNAGGAAGTTTT
GGAGTTTTGGGTCTGCTTANGGCTTCNAGCAGCCTTGCACTGAGAACTTTGGTAGG
GACCTCGAGTAATCCACTCCNTTTTNGGGACTGACGTGAGGCTCCCGGTGGGGAAA
GANACTGACCTNTC

Sequence ID 1453

CCGACCTGTCTCGCTCCGTGGCCTTAGCTGTGCTCGCGCTACTCTCTCTTTCTGGC
CTGGAGGCTATCCAGCGTACTCCAAAGATTCAGGTTTACTCACGTCATCCAGCAGA
GAATGGAAAGTCAAATTTCCTGAATTGCTATGTGTCTGGGTTTCATCCATCCGACA
TTGAAGTTGACTTACTGAAGAATGGAGAGAGAATTGAAAAAGTGGAGCATTCAGAC
TTGTCTTTCAGCAAGGACTGGTCTTTCTATCTCTTGTACTACACTGAATTCACCCC
CACTGAAAAAGATGAGTATGCCTGCCGTGTGAACCATGTGACTTTGTCACAGCCCA
AGATAGTTAAGTGGGATCGAGACATGTAAGCAGCATCATGGAGGTTTGAAGATGCC
GCATTTGGATTGGATGAATTCCAAATTCTGCTTGCTTGCTTTTTAATATTGATATG
CTTATACACTTACACTTTATGCACAAAATGTAGGGTTATAATAATGTTAACATGGA
CATGATCTTCTTTATAATTCTACTTTGAGTGCTGTCTCCATGTTTGATGTATCTGA

282

GCAGGTTGCTCCACAGGTAGCTCTAGGAGGGCTGGCAACTTAGAGGTGGGGAGCAG
AGAATTCTCTTATCCAACATCAACATCTTGGTCAGATTTGAACTCTTCAATCTCTT
GCACTCAAAGCTTGTTAAGATAGTTAAGCGTGCATAAGTTAACTTCCAATTTACAT
ACTCTGCTTAGAATTTGGGGGAAAATTTAGAAATATAATTGACAGGATTATTGGAA
ATTTGTTATAATGAATGAAACATTTTTGTCATATAAGATTCATATTTACTTCTTAT
ACA


Sequence ID 1454

TAAATAGGGAATCCTTTCCCCATTGCTTGTTTTTCTCAGGTTTGTCAAAGATCAGA
TAGTTGTAGATATGCGACGTTATTTCTGAGGGCTCTGTTCTGTTCCATTGATCTAT
ATCTCTGTCACATGCACACGTATGTTTGTTGTGGCACTATTCACAGTGGCAAAGAC
TTGGAACCAACCCAAATGTCCAACAATGATAGACCGGGTTAAGAAAATGCGGCACA
TATACACCATGGAATACTATGTAGCCATAAAAAATGATGAGTTCGTGTCCTTTGTA
GGGACATGGATGAAATTGGAAATCATCATTCTCAGTAAACTATCGCAGGAACAAAA
AACCAAACACTGCATATTCTCACTCATAGGTGGGAATTGAACAGTGGGAACACATG
GACACAGGAAGGGGAACATCACACTCTGAGGACTGTTGTGGGGTGGGGGGAGGGAG
GAGGGATAGCATTGGGAGATATACCTAGTGCTGGATGACGAGTTAGTGGGTGCAGC
GCACCAGCATGTCACATGTATACATATGTAACTAACCTGCACATTGTGCACATGTA
CCCTAAAACTTAAGGTAT


Sequence ID 1456

CCGCAACAAACACGGGAGTGCAGATATCGCTGCGATGGGCTGATTTCCTTTATTTG
GGTATATACCCAGCAGTGGGATTGCTGGATTGTATGGTAGCTCTATTAGTTTTTTG
AGGAACCTCCAAACTGTTCTNCATAGTGGTTGTACTCATTTACATTCCCACTGTGA
ACCCTGAAAATTTGAGGCAGGTCTCAGTTAAATTAGAAAGTTGATTTTGCCAAGTT
GGGGACACGCACTCGTGACACAGCCTCAGGAGGAACTGATGACATGTGCCCAGGTG
GTCAGAGCACAGCTTGGTTTTATACATTTTAGGGAAACCTGAGCCATCAATCAACA
TACGTAAAATGGGCCGGGCACAGCAGCTCAAGCTGTAATCCCAGCACTCTGGGAGG
CCGAGGCGGGTGGATCACTTGAGGTCAGGAGTTCGAGACCAGCCTGGCCAACATGG
TGAAACCCCGTCTCTATTAAAAATACAAAGCTTAGCTGGATGTGGTGGCGCATGCC
TGTAGTCCCAGCTGCTCTAGGAGGCTGAGGCATGAGAATTGCTTGAACCTGGGAGG
CAGAGGCTGCAGTGAGCCGAGATCGAGCCACTATACTCCAGCCTGGTCAACAGAGT
GAGACCCTGTCT

Sequence ID 1460

CCACAACTGTGTTCACTAGCAACCTCAAACAGACACCATGGTGCACCTGACTCCTG

AGGAGAAGTCTGCCGTTACTGCCCTGTGGGGCAAGGTGAACGTGGATGAAGTTGGT
GGTGAGGCCCTGGGCAGGCTGCTGGTGGTCTACCCTTGGACCCAGAGGTTCTTTGA
GTCCTTTGGGGATCTGTCCACTCCTGATGCTGTTATGGGCAACCCTAAGGTGAAGG
CTCATGGCAAGAAAGTGCTCGGTGCCTTTAGTGATGGCCTGGCTCACCTGGACAAC
CTCAAGGGCACCTTTGCCACACTGAGTGAGCTGCACTGTGACAAGCTGCACGTGGA
TCCTGAGAACTTCAGGCTCCTGGGCAACGTGCTGGTCTGTGTGCTGGCCCATCACT
TTGGCAAAGAATTCACCCCACCAGTGCAGGCTGCCTATCAGAAAGTGGTGGCTGGT
GTGGCTAATGCCCTGGCCCACAAGTATCACTAAGCTCGCTTTCTTGCTGTCCAATT
TCTATTAAAGGTTCCTTTGTTCCCTAAGTCCAACTACTAAACTGGGGGATATTATG
AAGGGCCTTGAGCATCTGGATTCTGCCTAATAAAAAACATTTATTTTCATTG

Sequence ID 1490

ATGGGCATCTCTCGGGACAACTGGCACAAGCGCCGCAAAACCGGGGGGCAAGAGAAA
GCCCTACCACAAGAAGCGGAAGTATGAGTTGGGGCGCCCAGCTGCCAACACCAAGA
TTGGCCCCCGCCGCATCCACACAGTCCGTGTGCGGGGAGGTAACAAGAAATACCGT
GCCCTGAGGTTGGACGTGGGGAATTTCTCCTGGGGCTCANAGTGTTGTACTCGTAA
AACAAGGATCATCGATGTTGTCTACAATGCATCTAATAACGAGCTGGTTCGTACCA
AGACCCTGGTGAAGAATTGCATCGTGCTCATCGACAGCACACCGTACCGACAGTGG
TACGAGTCCCACTATGCGCTGCCCCTGGGCCGCAAGAAGGGAGCCAAGCTGACTCC
TGAGGAAGAAGAGATTTTAAACAAAAAACGATCTAAAAAAATTCAGAAGAAATATG
ATGAAAGGAAAAAGAATGCCAAAATCAGCAGTCTCCTGGAGGAGCAGTTCCAGCAG
GGCAAGCTTCTTGCGTGCATCGCTTCAAGGCCGGGACAGTGTGGCCGAGCAGATGG
CTATGTGCTAGAGGGCAAAGAGTTGGAGTTCTATCTTAGGAAAATCAAGGCCCGCA
AAGGCAAATAAATCCTTGTTTTGTCTTCACCCATGTAATAAAGGTGTTTATTGTTT
TTGTT

Sequence ID 1491

CTTNCACATACTGATTGATGTCTCATGTCTCTCTAAAATGTGTAAAACCAAGCTGT
GCCCCAACCACCTTGGGNACATGTGGNGAGGACCTCCTGAGGCTGTGTCATGGGCA
CACCTTAACCCTGGGAAAATAAACTTTCTAAACTGACTTGAGAGCTGTCTCAGATA
TTCTGAGCTTACAGTTATTGTGAAATCATTTTAATTATAAATTAAGTGGAGATTTA
CTTAAAATCATGTGTAGAAGTAGCCTGTGATATAGTCCTAGATACATACATTATCA
TCTTATGTATCTTCCCTCCCTCTTCCAGGTTCTGATAAAAACAGATGAAATCTGAA
AGACCATGACAGTAGTATTTTGAAAATGACAGTATTTGAAATTAAAAAATTGTAAA
AGTGTTCTGTTCTATCACTGCCAAAGGATAAGTTACAAATTGGTTCTTGGAACGTA
ATATGTACTATGTGCTTGCTATTTAATAATTTACCAGTCTTAGTCTTTTTTATTCA
GACTAATTTTACCTTTTTTTAACCTATGACTCTTTAGTTATAGTAGTACAAAAAAG


TAGTTTTAGTTATAGTTTTAGTTGTAGTACAAAAAAGCATTTTCTGTAAGCTTAAT
TTCTTTCCCCTTCCCGCTTTCCCAGTCAGATGACTTTAGTGATTTGGAGTTGTGTG
CTTTATAAGTGCATTCCTCAGAGGACTTAATATTACTAAGATTTTAGCAACNCTGA
AATATGTT


Sequence ID 1492

TGTNCCTGTAGTCCTGTGTGGGAGGATTGCCTGAGCCTAGGAGCTCAAAGTTGCAG
TGAGCCCAGATCGNGNCATTGCAGTCCAGCCTGGGTGACAGAGTGAGACCCCATGT
CAAAAAAAAAAAAACAAAAAACAGGGGCCTGCCTCANCCAGCAGGTGAGGTCTGCC
ACTGAGAGCACTTCTAGCAGCAGGAACAGCCTCCACCCCCACACTGCAATCAAGTT
TTTTGGGTCAGCCTTAGGAGCTAANAAAGGGCCTAGTTTGNCTAAATAGCAGGAGT
TATATCCAGGGATCTTCAGGCCCAGGAATGCTAATGAGTAGGCATTCCATGGGCCC
TGGGAATGGCTTTGTGTGCCANAAATGATGGCCACAAAGGCCTTGCTGCCTTTTTT
CAAAATGGCTGCATCCAGCTGAGTGCTCTCTGCCAAAGGGGANAANAAAATAAGTC
TCCAGTGCATTTAGATTGGTCTCTCATCATCTCTCTCCTTTTTGTTTTTATTAGTC
TCCTTAACCAAAACTGCCAAGAAAGGCTTGGAATTGAAACAAAACCTGATANAANA
GGTAAGAGGTTGTTCTTTT

Sequence ID 1493

TGTNTCAAAAAAAAAAAAAAGAACGGNAATGTACTGGAGATGTATTTGATAACCAA
GGNTTTAGGTAAATTTTCACCAGTATTAGTTNTATTTGCAAACTGAAAAATGTTGT
AGGCTTAATATAAAATAACCACATTAGTGAACATTATATCTCTTAGAAGAAAGGCC
ATATTTTGCTCCTGCTTCTGTAAAAATATTATTTGTTTGAAGGGGAAATAATGGTA
GTGTGACCTTTCACTTAATTCCTACTCCCTTAATGTGAGAGAGACAAAATGAGCTG
AAGAAGGAAAATTCTGGAGTTACACTCCACAACCTTGAACATACTGACGGACATCT
CTGTTTTGACAACGATTTCTCCATGCCACCCATGCTNTAATGCCTTGTGGATCACG
GACAACCCTCTTTGCACAAGCTACAGCATCAGCGATGTTATCTTGCAGCAAAGCAC
TGCAGGATAAATGACAGGCATTAACTGCTCCTGGGGTTTTGCCATCATTACACCAG
TAGCGGCTATTGATCTGAAATATCCCATAATCAGTGCTTCTGTCTCCAGCATTGTA
GTTTGTAGCTCGTGTGTTGTAACCACTCTCCCATTTGGCCAAACACATCCAGTTTG
CTAGGCTGATTCCCCTGTAGCCATCCATTCCCAATCTTTTCAGAGTTCTGGCCAAC
TCACACCTTTCAAAGACCTTGCCCTGGACCGTAACAGAAAGGAGGACAAGCCCCAG
AACAATGAGAGCCTTCATGTTGAC


Sequence ID 1494

TTGGTACCCGGGAAATTCTTTGCCGCGTCGACGGCCGGTGAGGCAGATCACCTGAG
CCCAGGAGTTCAGGACCAGCCTGGGCAGCATACCGGGATTCCATCTNNACTAAAAA


CAGTAGGCTGGGTGTGGTGGCTCATGTCTGTAAGCTCAGGACTTTGGAAGGCCAAG
ATGGGAGGATCACTTGAGCCTGGGAGTTTGACACCAGCTTGAGCATCGTAGCCAGG
CCCTGACTCTACAAAAAAGTGAAATAATTAGCCGAGTGTGGTGGTTCACACCTGTA
ATCCCAGCTGCTCAGGAGGCTGAGGTAGGAGAATCATTTGAACCCGGGAGGTGGAG
GTTGCAGTTAGCCGAGATCACGCCATTGCACTCCGGCCTGGGCGATAAAGCGAGAC
TCTGTCTCAAAAAAAAAAAAAA

Sequence ID 1495

```
ATTCGGGCCGAGATGTCTCGCTCCGTGGCCTTAGCTGTGCTCGCGCTACTCTCTCT
TTCTGGCCTGGAGGCTATCCAGCGTACTCCAAAGATTCAGGTTTACTCACGTCATC
CAGCAGAGAATGGAAAGTCAAATTTCCTGAATTGCTATGTGTCTGGGTTTCATCCA
TCCGACATTGAAGTTGACTTACTGAAGAATGGAGAGAGAATTGAAAAAGTGGAGCA
TTCAGACTTGTCTTTCAGCAAGGACTGGTCTTTCTATCTCTTGTACTACACTGAAT
TCACCCCCACTGAAAAAGATGAGTATGCCTGCCGTGTGAACCATGTGACTTTGTCA
CAGCCCAAGATAGTTAAGTGGGATCGAGACATGTAAGCAGCATCATGGAGGTTTGA
AGATGCCGCATTTGGATTGGATGAATTCCAAATTCTGCTTGCTTGCTTTTTAATAT
TGATATGCTTATACACTTACACTTTATGCACAAAATGTAGGGTTATAATAATGTTA
ACATGGACATGATCTTCTTTATAATTCTACTTTGAGTGCTGTCTCCATGTTTGATG
TATCTGAGCAGGTTGCTCCACAGGTAGCTCTAGGAGGGCTGGCACCTTAGAGGTGG
GGAGCAGAGAATTCTCTTATCCAACATCAACATCTTGGTCAGATTTGAACTCTT
```

Sequence ID G6

```
GGATTTTTGGTCCGCACGCTCCTGCTCCTGACTCACCGCTGTTCGCTCTCGCCGAG
GAACAAGTCGGTCAGGAAGCCCGCGCGCAACAGCCATGGCTTTTAAGGATACCGGA
AAAACACCCGTGGAGCCGGAGGTGGCAATTCACCGAATTCGAATCACCCTAACAAG
CCGCAACGTAAAATCCTTGGAAAAGGTGTGTGCTGACTTGATAAGAGGCGCAAAAG
AAAAGAATCTCAAAGTGAAAGGACCAGTTCGAATGCCTACCAAGACTTTGAGAATC
ACTACAAGAAAAACTCCTTGTGGTGAAGGTTCTAAGACGTGGGATCGTTTCCAGAT
GAGAATTCACAAGCGACTCATTGACTTGCACAGTCCTTCTGAGATTGTTAAGCAGA
TTACTTCCATCAGTATTGAGCCAGGAGTTGAGGTGGAAGTCACCATTGCAGATGCT
TAAGTCAACTATTTTAATAAATTGATGACCAGTTGTTAAAA
```

Sequence ID - 61    nt: 362

```
CTTATTGAAAATTTTACTAATTTCTTACTTTTTAGGTTTTAGGAGAATACTTTTGGA
TAATTGACTAGCCTCACATTATATTGATAGAGGTTCTTGAAAACTTTAATGCCAAT
TCATGTATCTTATGACTAAAATAGATAATCCATTTAGAAATTTAAGTCATTCTTGC
GTGCTTGATATGTGTCAGCACTATCCAAGTTGCTAGGGGATACAATGGTGAAGTG
AAAATATCAGCTAGGTGCCGGTGGCTCACACCTGTTATCCCAACAGTTTGGGAGG
CCAGGGTGGGAGGATCACTCAAGCACANGCGTTTCACACCAGCCTGGACAACAT
ACAAGACCCCATCTTTACCAAAAGTTAAG
```

Sequence ID - 490        nt: 382

```
TTTTCTTAGAACTTTATTTTTTCTGGCCAGGCGCAGTGGCTCACACCTGTAATCCC
AGCACTTTGGGAGGCCAAGGCAGGTCGATCACCTGAGGTCAGGAGCTCAAGACC
AGCCTGGCCAACATGGTGAAACCCTGTCTCTACTAAAAATACAAAAATTAGCTGG
GCGTGGTGGCGCATGCCTGTAATCCCANCTACTCAGGAGGCTGAGGCAGGAGAA
TTGTTTGAACCCGGGAGGCGGAGGTTGCANTGAGCCGAGATTGCGCCACTGCACT
CCAGCCTGGGCAACAGAGCGAAACTCCATCTCAAAAAAAAAAAAAAAAAACAAC
CTTTATTTTTTCTGATTTTAAAAGTAATAACTAGTTTGTAGAAACATTAAAAGT
```

Sequence ID – 892      nt: 559

```
TCTTTCGGAAGCGCGCCTTGTGTTGGTACCCGGGAATTCGCGGCCGCGTCGACGC
GGTCGTAAGGGCTGAGGATTTTTGGTCCGCACGCTCCTGCTCCTGACTCACCGCT
GTTCGCTCTCGCCGAGGAACAAGTCGGTCAGGAAGCCCGCGCGCAACAGCCATG
GCTTTTAAGGATACCGGAAAAACACCCGTGGAGCCGGAGGTGGCAATTCACCGA
ATTCGAATCACCCTAACAAGCCGCAACGTAAAATCCTTGGAAAAGGTGTGTGCTG
ACTTGATAAGAGGCGCAAAAGAAAAGAATCTCAAAGTGAAAGGACCAGTTCGAA
TGCCTACCAAGACTTTGAGAATCACTACAAGAAAAACTCCTTGTGGTGAAGGTTC
TAAGACGTGGGATCGTTTCCAGATGAGAATTCACAAGCGACTCATTGACTTGCAC
AGTCCTTCTGAGATTGTTAAGCAGATTACTTCCATCAGTATTGAGCCAGGAGTTG
AGGTGGAAGTCACCATTGCAGATGCTTAAGTCAACTATTTTAATAAATTGATGAC
CAGTTGTTT
```

Sequence ID - 77        nt: 464

```
GCGGCTGCTGTTGGTTGGGGGCCGTCCCGCTCCTAAGGCAGGAAGATGGTGGCCG
CAAAGAAGACGAAAAAGTCGCTGGAGTCGATCAACTCTAGGCTCCAACTCGTTAT
GAAAAGTGGGAAGTACGTCCTGGGGTACAAGCAGACTCTGAAGATGATCAGACA
AGGCAAAGCGAAATTGGTCATTCTCGCTAACAACTGCCCAGCTTTGAGGAAATCT
GAAATAGAGTACTATGCTATGTTGGCTAAAACTGGTGTCCATCACTACAGTGGCA
ATAATATTGAACTGGGCACAGCATGCGGAAAATACTACAGAGTGTGCACACTGG
CTATCATTGATCCAGGTGACTCTGACATCATTAGAAGCATGCCAGAACAGACTGG
TGAAAAGTAAACCTTTTCACCTACAAAATTTCACCTGCAAACCTTAAACCTGCAA
AATTTTCCTTTAATAAAATTTGCTTG
```

## Claims

1. A set of less than 1000 oligonucleotide probes, wherein said set comprises the oligonucleotides as described in Table 2b for which sequences are provided having the sequences as set forth in sequence No. 61, 77, 93, 108, 110, 192, 250, 308, 309, 310, 321, 327, 338, 339, 360, 361, 364, 365, 368, 378, 380, 381, 382, 384, 390, 391, 397, 398, 401, 403, 406, 411, 412, 413, 414, 415, 416, 418, 421, 423, 424, 428, 434, 436, 438, 441, 442, 450, 452, 453, 458, 460, 463, 464, 469, 471, 473, 474, 475, 476, 477, 478, 479, 482, 483, 485, 487, 488, 489, 492, 493, 494, 495; 503, 504, 505, 506, 507, 508, 509, 510, 512, 513, 515, 518, 519, 521, 523, 524, 526, 527, 529, 530, 532, 534, 560, 562, 564, 565, 566, 567, 568, 570, 571, 572, 575, 576, 578, 579, 580, 583, 585, 589, 591, 592, 593, 594, 596, 598, 600, 601, 605, 607, 610, 612, 613, 614, 615, 617, 618, 619, 622, 624, 628, 629, 630, 631, 632, 633, 634, 635, 636, 637, 638, 639, 643, 644, 645, 649, 651, 656, 658, 660, 661, 663, 665, 672, 673, 675, 679, 682, 683, 684, 685, 687, 688, 689, 691, 693, 696, 697, 699, 701, 702, 705, 706, 707, 708, 709, 711, 714, 718, 720, 721, 722, 724, 726, 736, 739, 747, 757, 758, 764, 766, 768, 773, 776, 782, 785, 796, 801, 808, 814, 817, 821, 825, 833, 837, 839, 849, 860, 864, 865, 867, 869, 870, 871, 873, 875, 876, 878, 879, 881, 885, 887, 889, 891, 892, 893, 895, 897, 899, 903, 904, 905, 906, 907, 908, 910, 911, 912, 915, 917, 926, 938, 939, 947, 949, 1028, 1056, 1071, 1074, 1081, 1083, 1084, 1099, 1109, 1118, 1125, 1139, 1148, 1160, 1165, 1172, 1178, 1180, 1181, 1182, 1183, 1185, 1186, 1188, 1189, 1190, 1192, 1193, 1195, 1196, 1197, 1198, 1199, 1200, 1201, 1202, 1203, 1204, 1205, 1207, 1208, 1209, 1210, 1211, 1212, 1213, 1214, 1215, 1216, 1217, 1218, 1219, 1220, 1221, 1224, 1226, 1228, 1230, 1231, 1239, 1331, 1332, 1335, 1336, 1337, 1338, 1344, 1348, 1351, 1352, 1353, 1355, 1360, 1361, 1364, 1365, 1366, 1368, 1369, 1370, 1371, 1372, 1374, 1378, 1380, 1382, 1387, 1389, 1390, 1391, 1392, 1394, 1395, 1396, 1397, 1399, 1440, 1448, 1453, 1456, 1460, 1495 and g6

or a set in which one or more of said oligonucleotides is substituted, wherein each oligonucleotide which is substituted is substituted by a part of said oligonucleotide which part is 15-200 bases in length, or by an oligonucleotide with a complementary sequence to said oligonucleotide.

2. A set of less than 1000 oligonucleotide probes, wherein said set comprises the oligonucleotides as described in Table 4b for which sequences are provided having the sequences as set forth in sequence No. 299, 300, 302, 304, 306, 308, 309, 310, 311, 313, 314, 315, 316, 321, 322, 323, 324, 325, 326, 327, 328, 330, 331, 335, 337, 338, 339, 360, 361, 363, 364, 365, 366, 368, 369, 370, 371, 373, 374, 378, 380, 381, 382, 383, 384, 386, 387, 388, 389, 390, 391, 394, 395, 396, 397, 398, 399, 400, 401, 402, 403, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 438, 441, 442, 446, 447, 448, 450, 452, 453, 454, 458, 459, 460, 461, 462, 463, 464, 469, 471, 472, 473, 474, 475, 476, 477, 478, 479, 481, 482, 483, 484, 485, 487, 488, 489, 490, 491, 492, 493, 494, 495, 496, 497, 499, 500, 502, 503, 504, 505, 506, 507, 508, 509, 510, 512, 513, 515, 518, 519, 521, 523, 524, 525, 526, 527, 529, 530, 532, 533, 534, 560, 561, 562, 563, 564, 565, 566, 567, 568, 570, 571, 572, 574, 575, 576, 577, 578, 579, 580, 581, 582, 583, 585, 586, 587, 588, 589, 590, 591, 592, 593, 594, 595, 596, 597, 598, 599, 600, 601, 602, 603, 605, 606, 607, 609, 610, 611, 612, 613, 614, 615, 617, 618, 619, 621; 622, 624, 625, 626, 627, 628, 629, 630, 631, 632, 634, 635, 636, 637, 638, 639, 641, 642, 643, 644, 645, 646, 647, 648, 649, 650, 651, 652, 653, 654, 655, 656, 657, 658, 660, 661, 663, 665, 666, 669, 670, 671, 672, 673, 674, 675, 676, 679, 682, 683, 684, 685, 686, 687, 688, 689, 690, 691, 692, 693, 694, 696, 697, 698, 699, 700, 701, 702, 703, 704, 705, 706, 707, 708, 709, 710, 711, 713, 714, 717, 718, 719, 720, 721, 722, 724, 726, 727, 728, 870, 871, 873, 878, 879, 883, 885, 887, 889, 890, 892, 893, 895, 896, 897, 898, 899, 900, 903, 904, 905, 906, 907, 908, 910, 911, 912, 913, 914, 915, 1178, 1180, 1181, 1182, 1183, 1185, 1186, 1188, 1189, 1190, 1191, 1193, 1200, 1332, 1336, 1337, 1348, 1351, 1353, 1355, 1359, 1361, 1364, 1365, 1366, 1367, 1368, 1369, 1370, 1372, 1374, 1382, 1387, 1389, 1390, 1391, 1397, 1399, 1440, 1447, 1448, 1449, 1450, 1453, 1454, 1490, 1491, 1492, 1493, 1494 and 1495

or a set in which one or more of said oligonucleotides is substituted, wherein each oligonucleotide which is substituted is substituted by a part of said oligonucleotide which part is 15-200 bases in length, or by an oligonucleotide with a complementary sequence to said oligonucleotide.

3. A set of oligonucleotide probes as claimed in claim 1 or 2, wherein each of said oligonucleotide probes is from 15 to 200 bases in length.

4. A set of oligonucleotide probes as claimed in any one of claims 1 to 3, wherein said probes are immobilized on one or more solid supports.

5. A set of oligonucleotide probes as claimed in claim 4, wherein said solid support is a sheet, filter, membrane, plate or biochip.

6. A kit comprising a set of oligonucleotide probes as defined in claim 4 or 5 immobilized on one or more solid supports.

7. A kit as claimed in claim 6 wherein said probes are immobilized on a single solid support and each unique probe is attached to different region of said solid support.

8. A kit as claimed in claim 6 or 7 further comprising standardizing materials.

9. The use of a set of probes as described in any one of claims 1 to 5 or a kit as described in any one of claims 6 to 8 to determine the gene expression pattern of a cell in a blood sample which pattern reflects the level of gene expression of genes to which said oligonucleotide probes bind, comprising at least the steps of:

a) isolating mRNA from said cell, which may optionally be reverse transcribed to cDNA;
b) hybridizing the mRNA or cDNA of step (a) to a set of oligonucleotides or a kit as defined in any one of claims 1 to 8; and

c) assessing the amount of mRNA or cDNA hybridizing to each of said probes to produce said pattern.

**10.** A method of preparing a standard gene transcript pattern characteristic of breast cancer or Alzheimer's disease or a stage thereof in an organism comprising at least the steps of:

a) isolating mRNA from the cells of a blood sample of one or more organisms having breast cancer or Alzheimer's disease or a stage thereof, which may optionally be reverse transcribed to cDNA;
b) hybridizing the mRNA or cDNA of step (a) from an organism with breast cancer or a stage thereof to a set of oligonucleotides or a kit as defined in any one of claims 1 or 3 to 8 specific for breast cancer or a stage thereof in an organism and sample thereof corresponding to the organism and sample thereof under investigation or hybridizing the mRNA or cDNA of step (a) from an organism with Alzheimer's disease or a stage thereof to a set of oligonucleotides or a kit as defined in any one of claims 2 to 8 specific for Alzheimer's disease or a stage thereof in an organism and sample thereof corresponding to the organism and sample thereof under investigation; and
c) assessing the amount of mRNA or cDNA hybridizing to each of said probes to produce a characteristic pattern reflecting the level of gene expression of genes to which said oligonucleotides bind, in the sample with breast cancer or Alzheimer's disease or a stage thereof.

**11.** A method of preparing a test gene transcript pattern comprising at least the steps of:

a) isolating mRNA from the cells of a blood sample of said test organism, which may optionally be reverse transcribed to cDNA;
b) hybridizing the mRNA or cDNA of step (a) to a set of oligonucleotides or a kit as defined in any one of claims 1 or 3 to 8 specific for breast cancer or a stage thereof in an organism and sample thereof corresponding to the organism and sample thereof under investigation or to a set of oligonucleotides or a kit as defined in any one of claims 2 to 8 specific for Alzheimer's disease or a stage thereof in an organism and sample thereof corresponding to the organism and sample thereof under investigation; and
c) assessing the amount of mRNA or cDNA hybridizing to each of said probes to produce said pattern reflecting the level of gene expression of genes to which said oligonucleotides bind, in said test sample.

**12.** A method of diagnosing or identifying or monitoring breast cancer or Alzheimer's disease or a stage thereof in an organism, comprising the steps of:

a) isolating mRNA from the cells of a blood sample of said organism, which may optionally be reverse transcribed to cDNA;
b) hybridizing the mRNA or cDNA of step (a) to a set of oligonucleotides or a kit as defined in any one of claims 1 or 3 to 8 specific for breast cancer or a stage thereof in an organism and sample thereof corresponding to the organism and sample thereof under investigation or to a set of oligonucleotides or a kit as defined in any one of claims 2 to 8 specific for Alzheimer's disease or a stage thereof in an organism and sample thereof corresponding to the organism and sample thereof under investigation;
c) assessing the amount of mRNA or cDNA hybridizing to each of said probes to produce a characteristic pattern reflecting the level of gene expression of genes to which said oligonucleotides bind in said sample; and
d) comparing said pattern to a standard diagnostic pattern prepared as described in claim 10 using a sample from an organism corresponding to the organism and sample under investigation to determine the degree of correlation indicative of the presence of breast cancer or Alzheimer's disease or a stage thereof in the organism under investigation.

**13.** A method as claimed in any one of claims 10 to 12 wherein said mRNA or cDNA is amplified prior to step b).

**14.** A method as claimed in any one of claims 10 to 13 wherein the oligonucleotides and/or the mRNA or cDNA are labelled.

**15.** A method as claimed in any one of claims 10 to 14 wherein said pattern is expressed as an array of numbers relating to the expression level associated with each probe.

**16.** A method as claimed in any one of claims 10 to 15 wherein said organism is a eukaryotic organism, preferably a mammal.

**17.** A method as claimed in claim 16 wherein said organism is a human.

**18.** A method as claimed in any one of claims 10 to 15 wherein the data making up said pattern is mathematically projected onto a classification model.

**19.** A method as claimed in any one of claims 10 to 18 wherein the cells in the sample are not disease cells, have not been in contact with such cells and do not originate from the site of the disease or condition.

**20.** A method of identifying probes useful for diagnosing or identifying or monitoring breast cancer or Alzheimer's disease or a stage thereof in an organism, comprising the steps of:

a) immobilizing a set of oligonucleotide probes, as described in claim 1 for breast cancer or claim 2 for Alzheimer's disease on a solid support;
b) isolating mRNA from a blood sample of a normal organism (normal sample), which may optionally be reverse transcribed to cDNA;
c) isolating mRNA from a sample from an organism, corresponding to the sample and organism of step (b), which is known to have breast cancer or Alzheimer's disease or a stage thereof (diseased sample), which may optionally be reverse transcribed to cDNA;
d) hybridizing the mRNA or cDNA of steps (b) and (c) from said organism with breast cancer or Alzheimer's disease to said set of immobilized oligonucleotide probes of step (a) for breast cancer or Alzheimer's disease, respectively; and
e) assessing the amount of mRNA or cDNA hybridizing to each of said oligonucleotide probes to determine the level of gene expression of genes to which said oligonucleotide probes bind in said normal and diseased samples to generate a gene expression data set for each sample;
f) normalizing and standardizing said data set of step (e);
g) constructing a calibration model for classification, preferably using the statistical techniques Partial Least Squares Discriminant Analysis (PLS-DA) and Linear Discriminant Analysis (LDA);
h) performing JackKnife analysis and identifying those oligonucleotide probes which are required for classification of said disease and normal samples into their respective groups.

**Patentansprüche**

**1.** Satz von weniger als 1000 Oligonukleotidsonden, wobei dieser Satz die in Tabelle 2b beschriebenen Oligonukleotide umfasst, für die Sequenzen mit den in Sequenz-Nr. 61, 77, 93, 108, 110, 192, 250, 308, 309, 310, 321, 327, 338, 339, 360, 361, 364, 365, 368, 378, 380, 381, 382, 384, 390, 391, 397, 398, 401, 403, 406, 411, 412, 413, 414, 415, 416, 418, 421, 423, 424, 428, 434, 436, 438, 44.1, 442, 450, 452, 453, 458, 460, 463, 464, 469, 471, 473, 474, 475, 476, 477, 478, 479, 482, 483, 485, 487, 488, 489, 492, 493, 494, 495; 503, 504, 505, 506, 507, 508, 509, 510, 512, 513, 515, 518, 519, 521, 523, 524, 526, 527, 529, 530, 532, 534, 560, 562, 564, 565, 566, 567, 568, 570, 571, 572, 575, 576, 578, 579, 580, 583, 585, 589, 591, 592, 593, 594, 596, 598, 600, 601, 605, 607, 610, 612, 613, 614, 615, 617, 618, 619, 622, 624, 628, 629, 630, 631, 632, 633, 634, 635, 636, 637, 638, 639, 643, 644, 645, 649, 651, 656, 658, 660, 661, 663, 665, 672, 673, 675, 679, 682, 683, 684, 685, 687, 688, 689, 691, 693, 696, 697, 699, 701, 702, 705, 706, 707, 708, 709, 711, 714, 718, 720, 721, 722, 724, 726, 736, 739, 747, 757, 758, 764, 766, 768, 773, 776, 782, 785, 796, 801, 808, 814, 817, 821, 825, 833, 837, 839, 849, 860, 864, 865, 867, 869, 870, 871, 873, 875, 876, 878, 879, 881, 885, 887, 889, 891, 892, 893, 895, 897, 899, 903, 904, 905, 906, 907, 908, 910, 911, 912, 915, 917, 926, 938, 939, 947, 949, 1028, 1056, 1071, 1074, 1081, 1083, 1084, 1099, 1109, 1118, 1125, 1139, 1148, 1160, 1165, 1172, 1178, 1180, 1181, 1182, 1183, 1185, 1186, 1188, 1189, 1190, 1192, 1193, 1195; 1196, 1197, 1198, 1199, 1200, 1201, 1202, 1203, 1204, 1205, 1207, 1208, 1209, 1210, 1211, 1212, 1213, 1214, 1215, 1216, 1217, 1218, 1219, 1220, 1221, 1224, 1226, 1228, 1230, 1231, 1239, 1331, 1332, 1335, 1336, 1337, 1338, 1344, 1348, 1351, 1352, 1353, 1355, 1360, 1361, 1364, 1365, 1366, 1368, 1369, 1370, 1371, 1372, 1374, 1378, 1380, 1382, 1387, 1389, 1390, 1391, 1392, 1394, 1395, 1396, 1397, 1399, 1440, 1448, 1453; 1456, 1460, 1495 und g6 gezeigten Sequenzen angegeben sind, oder Satz, in dem eines oder mehrere der Oligonukleotide substituiert ist, wobei jedes substituierte Oligonukleotid durch einen 15-200 Basen langen Teil des Oligonukleotids oder durch ein Oligonukleotid mit einer zu diesem Oligonukleotid komplementären Sequenz substituiert ist.

**2.** Satz von weniger als 1000 Oligonukleotidsonden, wobei dieser Satz die in Tabelle 4b beschriebenen Oligonukleotide umfasst, für die Sequenzen mit den in Sequenz-Nr. 299, 300, 302, 304, 306, 308, 309, 310, 311, 313, 314, 315, 316, 321, 322, 323, 324, 325, 326, 327, 328, 330, 331, 335, 337, 338, 339, 360, 361, 363, 364, 365, 366, 368, 369, 370, 371, 373, 374, 378, 380, 381, 382, 383, 384, 386, 387, 388, 389, 390, 391, 394, 395, 396, 397, 398, 399, 400, 401, 402, 403, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 438, 441, 442, 446, 447, 448, 450, 452, 453, 454, 458, 459, 460, 461, 462, 463, 464, 469, 471, 472, 473, 474, 475, 476, 477, 478, 479, 481, 482, 483, 484, 485, 487, 488, 489, 490, 491, 492, 493, 494, 495, 496, 497, 499, 500, 502, 503, 504, 505, 506, 507, 508, 509, 510, 512, 513, 515, 518, 519, 521, 523, 524, 525, 526, 527, 529, 530, 532, 533, 534, 560, 561, 562, 563, 564, 565, 566, 567, 568, 570, 571, 572, 574, 575, 576, 577, 578, 579, 580, 581, 582, 583, 585, 586, 587, 588, 589, 590, 591, 592, 593, 594, 595, 596, 597, 598, 599, 600,601,602,603, 605, 606, 607, 609, 610, 611, 612, 613, 614, 615, 617, 618, 619, 621, 622, 624, 625, 626, 627, 628, 629, 630, 631, 632, 634, 635, 636, 637, 638, 639, 641, 642, 643, 644, 645, 646, 647, 648, 649, 650, 651, 652, 653, 654, 655, 656, 657, 658, 660, 661, 663, 665, 666, 669, 670, 671, 672, 673, 674, 675, 676, 679, 682, 683, 684, 685, 686, 687, 688, 689, 690, 691, 692, 693, 694, 696, 697, 698, 699, 700, 701, 702, 703, 704, 705, 706, 707, 708, 709, 710, 711, 713, 714, 717, 718, 719, 720, 721, 722, 724, 726, 727, 728, 870, 871, 873, 878, 879, 883, 885, 887, 889, 890, 892, 893, 895, 896, 897, 898, 899, 900, 903, 904, 905, 906, 907, 908, 910, 911, 912, 913, 914, 915, 1178, 1180, 1181, 1182, 1183, 1185, 1186, 1188, 1189, 1190, 1191, 1193, 1200, 1332, 1336, 1337, 1348, 1351, 1353, 1355, 1359, 1361, 1364, 1365, 1366, 1367, 1368, 1369, 1370, 1372, 1374, 1382, 1387, 1389, 1390, 1391, 1397, 1399, 1440, 1447, 1448, 1449, 1450, 1453, 1454, 1490, 1491, 1492, 1493, 1494 und 1495 gezeigten Sequenzen angegeben sind, oder Satz, in dem eines oder mehrere der Oligonukleotide substituiert ist, wobei jedes substituierte Oligonukleotid durch einen 15-200 Basen langen Teil des Oligonukleotids oder durch ein Oligonukleotid mit einer zu diesem Oligonukleotid komplementären Sequenz substituiert ist.

3. Satz von Oligonukleotidsonden nach Anspruch 1 oder 2, wobei jede der Oligonukleotidsonden 15 bis 200 Basen lang ist.

4. Satz von Oligonukleotidsonden nach einem der Ansprüche 1 bis 3, wobei die Sonden auf einem oder mehreren festen Trägern immobilisiert sind.

5. Satz von Oligonukleotidsonden nach Anspruch 4, wobei der feste Träger ein Blatt, ein Filter, eine Membran, eine Platte oder ein Biochip ist.

6. Kit, umfassend einen Satz von Oligonukleotidsonden nach Anspruch 4 oder 5, der auf einem oder mehreren festen Trägern immobilisiert ist.

7. Kit nach Anspruch 6, wobei die Sonden auf einem einzigen festen Träger immobilisiert sind und jede spezifische Sonde an einen anderen Bereich des festen Trägers gebunden ist.

8. Kit nach Anspruch 6 oder 7, ferner umfassend Standardisierungsmaterialien.

9. Verwendung eines Satzes von Sonden nach einem der Ansprüche 1 bis 5 oder eines Kits nach einem der Ansprüche 6 bis 8 zum Bestimmen des Genexpressionsmusters einer Zelle in einer Blutprobe, wobei das Muster das Genexpressionsniveau von Genen, an welche die Oligonukleotidsonden binden, widerspiegelt, umfassend zumindest die Schritte:

   a) Isolieren von mRNA aus der Zelle, die gegebenenfalls in cDNA revers transkribiert werden kann,
   b) Hybridisieren der mRNA oder cDNA aus Schritt (a) mit einem Satz von Oligonukleotiden oder einem Kit nach einem der Ansprüche 1 bis 8 und
   c) Evaluieren der Menge an mRNA oder cDNA, die mit den einzelnen Sonden unter Bildung des Musters hybridisiert.

10. Verfahren zum Erzeugen eines Standard-Gentranskriptmusters, das für Brustkrebs oder Morbus Alzheimer oder ein Stadium davon in einem Organismus typisch ist, umfassend zumindest die Schritte:

    a) Isolieren von mRNA aus den Zellen einer Blutprobe eines oder mehrerer Organismen mit Brustkrebs oder Morbus Alzheimer oder einem Stadium davon, die gegebenenfalls in cDNA revers transkribiert werden kann,
    b) Hybridisieren der mRNA oder cDNA aus Schritt (a) von einem Organismus mit Brustkrebs oder einem Stadium davon mit einem Satz von Oligonukleotiden oder einem Kit nach einem der Ansprüche 1 oder 3 bis 8, das für Brustkrebs oder ein Stadium davon in einem Organismus und einer Probe davon, welche dem zu untersuchenden Organismus und der Probe davon entspricht, spezifisch ist, oder Hybridisieren der mRNA oder cDNA aus Schritt (a) von einem Organismus mit Morbus Alzheimer oder einem Stadium davon mit einem Satz von Oligonukleotiden oder einem Kit nach einem der Ansprüche 2 bis 8, das für Morbus Alzheimer oder ein Stadium davon in einem Organismus und einer Probe davon, welche dem zu untersuchenden Organismus und der Probe davon entspricht, spezifisch ist, und

c) Evaluieren der Menge an mRNA oder cDNA, die mit den einzelnen Sonden unter Bildung eines typischen Musters hybridisiert, welches das Genexpressionsniveau von Genen, an welche die Oligonukleotide binden, in der Probe von Brustkrebs oder Morbus Alzheimer oder ein Stadium davon widerspiegelt.

11. Verfahren zum Erzeugen eines Test-Gentranskriptmusters, umfassend zumindest die Schritte:

a) Isolieren von mRNA aus den Zellen einer Blutprobe des Testorganismus, die gegebenenfalls in cDNA revers transkribiert werden kann,

b) Hybridisieren der mRNA oder cDNA aus Schritt (a) mit einem Satz von Oligonukleotiden oder einem Kit nach einem der Ansprüche 1 oder 3 bis 8, der/das für Brustkrebs oder ein Stadium davon in einem Organismus und einer Probe davon, welche dem zu untersuchenden Organismus und der Probe davon entspricht, spezifisch ist, oder mit einem Satz von Oligonukleotiden oder einem Kit nach einem der Ansprüche 2 bis 8, der/das für Morbus Alzheimer oder ein Stadium davon in einem Organismus und einer Probe davon, welche dem zu untersuchenden Organismus und der Probe davon entspricht, spezifisch ist, und

c) Evaluieren der Menge an mRNA oder cDNA, die mit den einzelnen Sonden unter Bildung des Musters hybridisiert, welches das Genexpressionsniveau von Genen, an welche die Oligonukleotide binden, in der Testprobe widerspiegelt.

12. Verfahren zur Diagnose oder zum Erkennen oder zum Verfolgen von Brustkrebs oder Morbus Alzheimer oder einem Stadium davon in einem Organismus, umfassend die Schritte:

a) Isolieren von mRNA aus den Zellen einer Blutprobe des Organismus, die gegebenenfalls in cDNA revers transkribiert werden kann,

b) Hybridisieren der mRNA oder cDNA aus Schritt (a) mit einem Satz von Oligonukleotiden oder einem Kit nach einem der Ansprüche 1 oder 3 bis 8, der/das für Brustkrebs oder ein Stadium davon in einem Organismus und einer Probe davon, welche dem zu untersuchenden Organismus und der Probe davon entspricht, spezifisch ist, oder mit einem Satz von Oligonukleotiden oder einem Kit nach einem der Ansprüche 2 bis 8, der/das für Morbus Alzheimer oder ein Stadium davon in einem Organismus und einer Probe davon, welche dem zu untersuchenden Organismus und der Probe davon entspricht, spezifisch ist, und

c) Evaluieren der Menge an mRNA oder cDNA, die mit den einzelnen Sonden unter Bildung eines typischen Musters hybridisiert, welches das Genexpressionsniveau von Genen, an welche die Oligonukleotide binden, in der Probe widerspiegelt, und

d) Vergleichen dieses Musters mit einem Standard-Diagnose-Muster, das nach Anspruch 10 unter Verwendung einer Probe von einem Organismus, welcher dem zu untersuchenden Organismus und der zu untersuchenden Probe entspricht, erzeugt wurde, um den Grad der Übereinstimmung zu bestimmen, der auf das Vorhandensein von Brustkrebs oder Morbus Alzheimer oder einem Stadium davon im zu untersuchenden Organismus hinweist.

13. Verfahren nach einem der Ansprüche 10 bis 12, wobei die mRNA oder cDNA vor Schritt (b) amplifiziert wird.

14. Verfahren nach einem der Ansprüche 10 bis 13, wobei die Oligonukleotide und/oder die mRNA oder cDNA markiert sind.

15. Verfahren nach einem der Ansprüche 10 bis 14, wobei das Muster als eine Reihe von Zahlen ausgedrückt wird, die sich auf das zu jeder Sonde gehörende Expressionsniveau beziehen.

16. Verfahren nach einem der Ansprüche 10 bis 15, wobei der Organismus ein eukaryotischer Organismus, bevorzugt ein Säuger, ist.

17. Verfahren nach Anspruch 16, wobei der Organismus ein Mensch ist.

18. Verfahren nach einem der Ansprüche 10 bis 15, wobei die Daten, aus denen das Muster besteht, mathematisch auf ein Klassifikationsmodell übertragen werden.

19. Verfahren nach einem der Ansprüche 10 bis 18, wobei die Zellen in der Probe keine Zellen der Krankheit sind, nicht in Kontakt mit solchen Zellen gestanden haben und nicht vom Ort der Krankheit oder des Zustands stammen.

**EP 1 565 574 B1**

**20.** Verfahren zum Identifizieren von Sonden, welche für die Diagnose oder das Erkennen oder das Verfolgen von Brustkrebs oder Morbus Alzheimer oder eines Stadiums davon in einem Organismus verwendbar sind, umfassend die Schritte:

a) Immobilisieren eines Satzes von Oligoriukleotidsonden, wie in Anspruch 1 für Brustkrebs oder wie in Anspruch 2 für Morbus Alzheimer beschrieben, auf einem festen Träger,

b) Isolieren von mRNA aus einer Blutprobe eines normalen Organismus (Normalprobe), die gegebenenfalls in cDNA revers transkribiert werden kann,

c) Isolieren von mRNA aus einer Probe von einem Organismus, welche der Probe und dem Organismus aus Schritt (b) entspricht, der bekanntermaßen Brustkrebs oder Morbus Alzheimer oder eine Stadium davon (pathologische Probe) aufweist, die gegebenenfalls in cDNA revers transkribiert werden kann,

d) Hybridisieren der mRNA oder cDNA aus den Schritten (b) und (c) von dem Organismus mit Brustkrebs oder Morbus Alzheimer mit dem Satz immobilisierter Oligonukleotidsonden für Brustkrebs beziehungsweise Morbus Alzheimer aus Schritt (a) und

e) Evaluieren der Menge an mRNA oder cDNA, die mit den einzelnen Oligonukleotidsonden hybridisiert, zum Bestimmen des Genexpressionsniveaus von Genen, an welche die Oligonukleotidsonden binden, in den Normal- und pathologischen Proben, um einen Satz von Genexpressionsdaten für jede Probe zu erstellen,

f) Normalisieren und Standardisieren des Datensatzes aus Schritt (e),

g) Erstellen eines Kalibriermodells zur Klassifizierung, vorzugsweise unter Verwendung der statistischen Verfahren "Partial Least Squares"-Diskriminanzanalyse (PLS-DA) und Lineare Diskriminanzanalyse (LDA),

h) Durchführen einer JackKnife-Analyse und Identifizieren derjenigen Oligonukleotidsonden, die zur Klassifizierung der pathologischen und der Normalproben in ihre entsprechenden Gruppen notwendig sind.

**Revendications**

**1.** Une série de moins de 1000 sondes oligonucléotidiques, ladite série comprenant les oligonucléotides tels que décrits au tableau 2b, dont les séquences sont telles qu'exposées à la séquence n° 61, 77, 93, 108, 110, 192, 250, 308, 309, 310, 321, 327, 338, 339, 360, 361, 364, 365, 368, 378, 380, 381, 382, 384, 390, 391, 397, 398, 401, 403, 406, 411, 412, 413, 414, 415, 416, 418, 421, 423, 424, 428, 434, 436, 438, 441, 442, 450, 452, 453, 458, 460, 463, 464, 469, 471, 473, 474, 475, 476, 477, 478, 479, 482, 483, 485, 487, 488, 489, 492, 493, 494, 495; 503, 504, 505, 506, 507, 508, 509, 510, 512, 513, 515,518, 519, 521, 523, 524, 526, 527, 529, 530, 532, 534, 560, 562, 564, 565, 566, 567, 568, 570, 571, 572, 575, 576, 578, 579, 580, 583, 585, 589, 591, 592, 593, 594, 596, 598, 600, 601, 605, 607, 610, 612, 613, 614, 615, 617, 618, 619, 622, 624, 628, 629, 630, 631, 632, 633, 634, 635, 636, 637, 638, 639, 643, 644, 645, 649, 651, 656, 658, 660, 661, 663, 665, .672, 673, 675, 679, 682, 683, 684, 685, 687, 688, 689, 691, 693, 696, 697, 699, 701, 702, 705, 706, 707, 708, 709, 711, 714, 718, 720, 721, 722, 724, 726, 736, 739, 747, 757, 758, 764, 766, 768, 773, 776, 782, 785, 796, 801, 808, 814, 817, 821, 825, 833, 837, 839, 849, 860, 864, 865, 867, 869, 870, 871, 873, 875, 876, 878, 879, 881, 885, 887, 889,891, 892, 893, 895, 897, 899, 903, 904, 905, 906, 907, 908, 910, 911, 912, 915, 917, 926, 938, 939, 947, 949, 1028, 1056, 1071, 1074, 1081, 1 083, 1084, 1099, 1109, 1118, 1125, 1139, 1148, 1160, 1165, 1172, 1178, 1180, 1181, 1182, 1183, 1185, 1186, 1188, 1189, 1190, 1192, 1193, 1195, 1196, 1197, 1198, 1199, 1200, 1201, 1202, 1203, 1204, 1205, 1207, 1208, 1209, 1210, 1211, 1212, 1213, 1214, 1215, 1216, 1217, 1218, 1219, 1220, 1221, 1224, 1226, 1228, 1230, 1231, 1239, 1331, 1332, 1335, 1336, 1337, 1338, 1344, 1348, 1351, 1352, 1353, 1355, 1360, 1361, 1364, 1365, 1366, 1368, 1369, 1370, 1371, 1372, 1374, 1378, 1380, 1382, 1387, 1389, 1390, 1391, 1392, 1394, 1395, 1396,1397, 1399, 1440, 1448, 1453, 1456, 1460, 1495 et g6
ou une série, dans laquelle un ou plusieurs desdits oligonucléotides sont substitués, chaque oligonucléotide qui est substitué, étant substitué par une partie dudit oligonucléotide, partie, qui présente une longueur de 15 à 200 bases, ou par un oligonucléotide avec une séquence complémentaire audit oligonucléotide.

**2.** Une série de moins de 1000 sondes oligonucléotidiques, ladite série comprenant les oligonucléotides tels que présentés au tableau 4b, dont les séquences sont telles qu'exposées à la séquence n° 299, 300, 302, 304, 306, 308, 309, 310, 311, 313, 314, 315, 316, 321, 322, 323, 324, 325, 325, 327, 328, 330, 331, 335, 337, 338, 339, 360, 361, 363, 364, 365, 366, 368, 369, 370, 371, 373, 374, 378, 380, 381, 382, 383, 384, 386, 387, 388, 389, 390, 391, 394, 395, 396, 397, 398, 399, 400, 401, 402, 403, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 438, 441, 442, 446, 447, 448, 450, 452, 453, 454, 458, 459, 460, 461, 462, 463, 464, 469, 471, 472, 473, 474, 475, 476, 477, 478, 479, 481, 482, 483, 484, 485, 487, 488, 489, 490, 491, 492, 493, 494, 495, 496, 497, 499, 500, 502, 503, 504, 505, 506, 507, 508, 509, 510, 512, 513, 515, 518, 519, 521, 523, 524, 525, 526, 527, 529, 530, 532, 533, 534, 560, 561, 562, 563, 564, 565, 566, 567, 568, 570, 571, 572, 574, 575, 576, 577, 578, 579, 580, 581, 582, 583, 585, 586, 587, 588, 589, 590, 591, 592, 593, 594, 595, 596, 597, 598, 599, 600, 601, 602, 603, 605, 606, 607, 609, 610, 611, 612, 613, 614, 615, 617, 618, 619, 621, 622, 624, 625, 626, 627, 628, 629, 630, 631, 632, 634, 635, 636, 637, 638, 639, 641, 642, 643, 644, 645, 646, 647, 648, 649, 650, 651, 652, 653, 654, 655, 656, 657, 658, 660, 661, 663, 665, 666, 669, 670, 671, 672, 673, 674, 675, 676, 679, 682, 683, 684, 685, 686, 687, 688, 689, 690, 691, 692, 693, 694, 696, 697, 698, 699, 700, 701, 702, 703, 704, 705, 706, 707, 708, 709, 710, 711, 713, 714, 717, 718, 719, 720, 721, 722, 724, 726, 727, 728, 870, 871, 873, 878, 879, 883, 885, 887, 889, 890, 892, 893, 895, 896, 897, 898, 899, 900, 903, 904, 905, 906, 907, 908, 910, 911, 912, 913, 914, 915, 1178, 1180, 1181, 1182, 1183, 1185, 1186, 1188, 1189, 1190, 1191, 1193, 1200, 1332, 1336, 1337, 1348, 1351, 1353, 1355, 1359, 1361, 1364, 1365, 1366, 1367, 1368, 1369, 1370, 1372, 1374, 1382, 1387, 1389, 1390, 1391, 1397, 1399, 1440, 1447, 1448, 1449, 1450, 1453, 1454, 1490, 1491, 1492, 1493, 1494 et 1495

ou une série, dans laquelle un ou plusieurs desdits oligonucléotides sont substitués, chaque oligonucléotide, qui est substitué, étant substitué par une partie dudit oligonucléotide, partie, qui présente une longueur de 15 à 200 bases, ou par un oligonucléotide avec une séquence complémentaire audit oligonucléotide.

3. Une série de sondes oligonucléotidiques suivant la revendication 1 ou 2, dans laquelle chacune desdites sondes oligonucléotidiques présente une longueur de 15 à 200 bases.

4. Une série de sondes oligonucléotidiques suivant une quelconque des revendications 1 à 3, dans laquelle lesdites sondes sont immobilisées sur un ou plusieurs supports solides.

5. Une série de sondes oligonucléotidiques suivant la revendication 4, dans laquelle ledit support solide est une feuille, un filtre, une membrane, une plaque ou une biopuce.

6. Un kit comprenant une série de sondes oligonucléotidiques, telle que définie par la revendication 4 ou 5, sondes oligonucléotidiques, qui sont immobilisées sur un ou plusieurs supports solides.

7. Un kit suivant la revendication 6, dans lequel lesdites sondes sont immobilisées sur un unique support solide et dans lequel chaque sonde unique est fixée à une région différente dudit support solide.

8. Un kit suivant la revendication 6 ou 7 comprenant en plus des matériaux d'étalonnage.

9. Utilisation d'une série de sondes, telle que décrite par une quelconque des revendications 1 à 5, ou un kit, tel que décrit par une quelconque des revendications 6 à 8, servant à déterminer le profil d'expression génétique d'une cellule dans un échantillon de sang, profil qui réflète le niveau d'expression génétique de gènes, auxquels lesdites sondes oligonucléotidiques s'attachent, utilisation comprenant au moins les étapes suivantes :

a) Isolement des ARNm de ladite cellule, ARNm, qui peuvent optionnellement faire l'objet d'une transcription inverse en ADNc.
b) Hybridation des ARNm ou ADNc de l'étape a) avec une série d'oligonucléotides ou un kit, tels que définis par une quelconque des revendications 1 à 8; et
c) Evaluation de la quantité des ARNm ou ADNc hybridés avec chacune desdites sondes pour produire le profil.

10. Méthode d'élaboration d'un profil de transcription génétique caractéristique du cancer du sein ou de la maladie d'Alzheimer ou d'un stade de ceux-ci dans un organisme comprenant au moins les étapes suivantes :

a) Isolement des ARNm des cellules d'un échantillon de sang d'un ou de plusieurs organismes atteints du cancer du sein ou de la maladie d'Alzheimer ou d'un stade de ceux-ci, ARNm, qui peuvent optionnellement faire l'objet d'une transcription inverse en ADNc ;
b) Hybridation des ARNm ou ADNc de l'étape (a) d'un organisme atteint du cancer du sein ou d'un stade de celui-ci avec une série d'oligonucléotides ou un kit, tels que définis par une quelconque des revendications 1 ou 3 à 8, spécifiques du cancer du sein ou d'un stade de celui-ci dans un organisme et échantillon de celui-ci correspondant à l'organisme et échantillon de celui-ci en cours d'étude ou hybridation des ARNm ou ADNc de l'étape a) d'un organisme atteint de la maladie d'Alzheimer ou d'un stade de celle-ci avec une série d'oligonucléotides ou un kit, tels que définis par une quelconque des revendications 2 à 8, spécifiques de la maladie d'Alzheimer ou d'un stade de celle-ci dans un organisme et échantillon de celui-ci correspondant à l'organisme et échantillon de celui-ci en cours d'étude; et
c) Evaluation de la quantité des ARNm ou ADNc hybridés avec chacune desdites sondes, pour produire un profil caractéristique réflétant le niveau d'expression génétique de gènes, auxquels lesdits oligonucléotides s'attachent dans l'échantillon avec le cancer du sein ou avec la maladie d'Alzheimer ou avec un stade de ceux-ci.

**11.** Méthode d'élaboration d'un profil test de transcription génétique comprenant au moins les étapes de :

a) Isolement des ARNm des cellules d'un échantillon de sang dudit organisme de test, ARNm, qui peuvent optionnellement faire l'objet d'une transcription inverse en ADNc ;
b) Hybridation des ARNm ou ADNc de l'étape (a) avec une série d'oligonucléotides ou un avec kit, tels que définis par une quelconque des revendications 1 ou 3 à 8, spécifiques du cancer du sein ou d'un stade de celui-ci dans un organisme et échantillon de celui-ci correspondant à l'organisme et échantillon de celui-ci en cours d'étude ou avec une série d'oligonucléotides ou avec un kit, tels que définis par une quelconque des revendications 2 à 8, spécifiques de la maladie d'Alzheimer ou d'un stade de celle-ci dans un organisme et échantillon de celui-ci correspondant à l'organisme et échantillon de celui-ci en cours d'étude; et
c) Evaluation de la quantité des ARNm ou des ADNc hybridés avec chacune des sondes pour produire un profil caractéristique réflétant le niveau d'expression génétique de gènes, auxquels lesdits oligonucléotides s'attachent, dans ledit échantillon test.

**12.** Méthode de diagnostique ou d'identification ou de monitorage du cancer du sein ou de la maladie d'Alzheimer ou d'un stade de ceux-ci dans un organisme, comprenant les étapes de :

a) Isolement des ARNm des cellules d'un échantillon de sang dudit organisme, ARNm, qui peuvent optionnellement faire l'objet d'une transcription inverse en ADNc ;
b) Hybridation des ARNm ou ADNc de l'étape (a) avec une série d'oligonucléotides ou avec un kit, tels que définis par une quelconque des revendications 1 ou 3 à 8, spéficiques du cancer du sein ou d'un stade de celui-ci dans un organisme et échantillon de celui-ci correspondant à l'organisme et échantillon de celui-ci en cours d'étude ou avec une serie d'oligonucléotides ou avec un kit, tels que définis par une quelconque des revendications 2 à 8, spécifiques de la maladie d'Alzheimer ou d'un stade de celle-ci dans un organisme et échantillon de celui-ci correspondant à l'organisme et échantillon de celui-ci en cours d'étude;
c) Evaluation de la quantité des ARNm ou ADNc hybridés avec chacune desdites sondes pour produire un profil caractéristique réflétant le niveau d'expression de gènes, auxquels lesdits oligonucléotides s'attachent dans ledit échantillon ; et
d) Comparaison dudit profil à un profil diagnostique standard élaboré comme décrit à la revendication 10, en utilisant un échantillon correspondant à un organisme et un échantillon en cours d'étude pour déterminer le degré de corrélation indicateur de la présence d'un cancer du sein ou de la maladie d'Alzheimer ou d'un stade de ceux-ci dans l'organisme en cours d'étude.

**13.** Méthode suivant une quelconque des revendications 10 à 12, dans laquelle lesdits ARNm ou ADNc sont amplifiés préalablement à l'étape b).

**14.** Méthode suivant une quelconque des revendications 10 à 13, dans laquelle les oligonucléotides et/ou les ARNm ou ADNc sont marqués.

**15.** Méthode suivant une quelconque des revendications 10 à 14, dans laquelle ledit profil est exprimé sous forme d'un réseau de numéros relatif au niveau d'expression associé à chaque sonde.

**16.** Méthode suivant une quelconque des revendications 10 à 15, dans laquelle ledit organisme est un organisme eucaryote, de préférence un mammifère.

**17.** Méthode suivant la revendication 16, dans laquelle ledit organisme est un humain.

**18.** Méthode suivant une quelconque des revendications 10 à 15, dans laquelle les données constituant ledit profil sont projetées mathématiquement sur un modèle de classification.

**19.** Méthode suivant une quelconque des revendications 10 à 18, dans laquelle les cellules dans l'échantillon ne sont pas des cellules pathogéniques, ni ont été en contact avec ce genre de cellules, ni proviennent du siège de la maladie ou de l'état pathogénique.

**20.** Méthode d'identification de sondes servant à diagnostiquer ou à identifier

ou à monitorer le cancer du sein ou la maladie d'Alzheimer ou un stade de ceux-ci dans un organisme, comprenant les étapes de :

a) Immobilisation d'une série de sondes oligonucléotidiques, telles que décrites dans la revendication 1 pour le cancer du sein ou dans la revendication 2 pour la maladie d'Alzheimer, sur un support solide ;

b) Isolement des ARNm d'un échantillon de sang d'un organisme normal (échantillon normal), ARNm, qui peuvent optionnellement faire l'objet d'une transcription inverse en ADNc ;

c) Isolement des ARNm d'un échantillon provenant d'un organisme correspondant à celui-ci et d'un organisme de l'étape (b), dont on sait, qu'il est atteint d'un cancer du sein ou de la maladie d'Alzheimer ou d'un stade de ceux-ci (échantillon pathogénique), ARNm, qui peuvent optionnellement faire l'objet d'une transcription inverse en ADNc ;

d) Hybridation des ARNm ou ADNc des étapes (b) et (c ) dudit organisme avec le cancer du sein ou avec la maladie d'Alzheimer avec ladite série de sondes oligonucléotidiques immobilisées de l'étape (a) respectivement pour le cancer du sein ou pour la maladie d'Alzheimer ; et

e) Evaluation de la quantité des ARNm ou ADNc hybridés avec chacune desdites sondes oligonucléotidiques pour déterminer le niveau d'expression génétique de gènes, auxquels lesdites sondes oligonucléotidiques s'attachent dans lesdits échantillons normal et pathogénique pour générer un ensemble de données d'expression génétique pour chaque échantillon ;

f) Normalisation et standardisation dudit ensemble de données de l'étape (e);

g) Elaboration d'un modèle d'étalonnage pour la classification en utilisant de préférence l'analyse discriminante PLS et l'analyse discriminante linéaire (LDA) comme techniques statistiques;

h) Réalisation d'une analyse jackknife et identification de celles des sondes oligonucléotidiques, qui sont nécessaires pour la classification desdits échantillons pathogénique et normal dans leur groupe respectif.

# FIG. 1

Effect of Direct Standardization (DS) on the Alzheimer
Data measured in two different series of experiments

RESULT2, X-expl: 25%, 12%

RESULT1, X-expl: 24%, 20%

# FIG. 1 CONT'D

# FIG. 2

Projection of normal (including benign) and breast cancer samples onto a
classification model generated by PLSR-DA using the data of 44 informative genes

PC = Principal Components
N = normal
C = breast cancer patients

EP 1 565 574 B1

# FIG. 3

Projection of individuals with and without Alzheimer's disease onto a
classification model generated by PLSR-DA using 182 informative genes.

PC = Principal Components
N = Non –Alzheimer's disease
A = Alzheimer's disease

# FIG. 4

Projection of normal (women with suspected mammogram but no breast cancer) and breast cancer samples onto a classification model generated by PLSR-DA using the data of 719 cDNAs

PC = Principal Components
N = normal
B = breast cancer patients

# FIG. 5

Prediction of normal (women with suspected mammogram but no breast cancer) and breast cancer samples based on 3 principal components using the data of 719 cDNAs

EP 1 565 574 B1

# FIG. 6

Projection of normal (women with suspected mammogram but no breast cancer) and breast cancer samples onto a classification model generated by PLSR-DA using the data of 111 cDNAs already on file

PC = Principal Components
N = normal
B = breast cancer patients

# FIG. 7

Prediction of normal (women with suspected mammogram but no breast
cancer) and breast cancer samples based on 4 principal components using
the data of 111 cDNAs already on file

EP 1 565 574 B1

# FIG. 8

Projection of normal (women with suspected mammogram but no breast cancer)
and breast cancer samples onto a classification model generated by PLSR-DA
using the data of 345 sequenced cDNAs for breast cancer

PC = Principal Components
N = normal
B = breast cancer patients

# FIG. 9

Prediction of normal (women with suspected mammogram but no breast cancer) and breast cancer samples based on 3 principal components using the data of 345 sequenced cDNAs

EP 1 565 574 B1

# FIG. 10

Projection of non-alzheimer and alzheimer samples onto a classification
model generated by PLSR-DA using the data of 520 sequenced cDNAs

PC = Principal Components
A= non-alzheimer
AD = alzheimer patients

# FIG. 11

Prediction of non-alzheimer and alzheimer samples based on 4
principal components using the data of sequenced cDNAs

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02059271 A
- WO 9849342 A

**Non-patent literature cited in the description**

- **Alon et al.** *PNAS,* 1999, vol. 96, 6745-6750
- **Golub et al.** *Science,* 1999, vol. 286, 531-537
- **Alizadeh et al.** *Nature,* 2000, vol. 403, 503-511
- **Bittner et al.** *Nature,* 2000, vol. 406, 536-540
- **Whitney et al.** *PNAS,* 2003, vol. 100 (4), 1896-1901
- **Sherlock et al.** *Current Opinion in Immunology,* 2000, vol. 12, 201-205
- **Thompson et al.** *Nucl. Acids Res.,* 1994, vol. 22, 4673-4680
- **Sambrook.** Molecular Cloning : A laboratory manual. Cold Spring Harbor Laboratory Press, 1989
- **Lockhart et al.** *Nat. Biotech.,* 1996, vol. 14, 1675-1680
- **Schena et al.** *Science,* 1995, vol. 270, 467-470
- **Maier E et al.** *Nucl. Acids Res.,* 1994, vol. 22, 3423-3424
- **Bernard et al.** *Nucl. Acids Res.,* 1996, vol. 24, 1435-1442
- **Richmond ; Somerville.** *Current Opin. Plant Biol.,* 2000, vol. 3, 108-116
- **Finkelstein et al.** Methods of Microarray Data Analysis. Kluwer Academic, 2001, 57-68
- **Yang et al.** Optical Technologies and Informatics. Proceedings of SPIE, 2001, vol. 4266, 141-152
- **Dudoit et al.** *J. Am. Stat. Ass.,* 2000, vol. 97, 77-87
- **Newton et al.** *J. Comp. Biol.,* 2001, vol. 8, 37-52
- **Jollife.** Principal Component Analysis. Springer-Verlag, 1986
- **Jackson.** A User's Guide to Principal Components. Wiley, 1991
- **Wang ; Kowalski.** *Anal. Chem.,* 1991, vol. 63, 2750
- *J. Chemometrics,* 1991, vol. 5, 129-145
- **Eisen et al.** *PNAS,* 1998, vol. 95, 14863-14868
- **Perou et al.** *Nature,* 2000, vol. 406, 747-752
- **Ross et al.** *Nature Genetics,* 2000, vol. 24 (3), 227-235
- **Herwig et al.** *Genome Res.,* 1999, vol. 9, 1093-1105
- **Tamayo et al.** *Science, PNAS,* 1999, vol. 96, 2907-2912
- **Tavazoie et al.** *Nature Genetics,* 1999, vol. 22 (3), 281-285
- **Hastie et al.** *Genome Biology,* 2000, vol. 1 (2
- **Tibshirani et al.** *Tech repot Univ Stanford.,* 1999
- **Lazzeroni.** *Stat. Sinica,* 2002, vol. 12, 61-86
- **Alter et al.** *PNAS,* 2000, vol. 97 (18), 10101-10106
- **Brown et al.** *PNAS,* 2000, vol. 97, 262-267
- **Nguyen ; Rocke.** *Bioinformatics,* 2002, vol. 18, 39-501216-1226
- **Dudoit et al.** *J. Am. Stat. Ass.,* 2002, vol. 97, 77-87
- **Kerr et al.** *PNAS,* 2000, vol. 98, 8961-8965
- **Park et al.** *Pacific Symposium on Biocomputing,* 2002, 52-63
- **Indahl et al.** *Chem. and Intell. Lab. Syst.,* 1999, vol. 49, 19-31
- **Duda ; Hart.** Classification and Scene Analysis. Wiley, 1973
- **Westad ; Martens.** *J. Near Inf. Spectr.,* 2000, vol. 8, 117-124
- **Marteus ; Naes.** Multivariate Calibration. John Wiley & Sons, Inc, 1989